# EUROPEAN PATENT APPLICATION

(11) **EP 4 692 330 A1**
(43) Date of publication of application: **11.02.2026**
(21) Application number: 24774127.5
(22) Date of filing: 20.03.2024
(51) Int. Cl.: C12N 5/10, C12N 5/078, A61P 35/00, A61K 35/17, C12N 15/113

(54) **MODIFIED CELL AND USE THEREOF**

(30) Priority: 21.03.2023 WO PCT/CN2023/082715; 21.03.2023 WO PCT/CN2023/082718; 21.03.2023 WO PCT/CN2023/082719; 21.03.2023 WO PCT/CN2023/082721; 21.03.2023 WO PCT/CN2023/082723; 09.05.2023 WO PCT/CN2023/092923; 31.05.2023 WO PCT/CN2023/097386
(71) Applicant: Suzhou Grit Biotechnology Co., Ltd., Shanghai 200120 (CN); Shanghai Grit Biotechnology Co., Ltd., Shanghai 200120 (CN); Beijing Grit Biotherapeutics Co., Ltd., Shanghai 200120 (CN)
(72) Inventor: LIU, Yarong, Shanghai 200120 (CN); SUN, Jingwei, Shanghai 200120 (CN); SHENG, Yao, Shanghai 200120 (CN)
(74) Representative: Fish & Richardson P.C.
(86) International application number: PCT/CN2024/082588
(87) International publication number: WO 2024/193562

(57) **Abstract**

The present invention belongs to the field of biomedicine, and provides a modified cell and uses thereof, and in particular relates to a method for culturing cells that comprises reducing the expression and/or attenuating the activity of target genes in the cells. The present invention also relates to methods for using the cultured cells to prevent and/or treat tumors.

## Description

### TECHNICAL FIELD

The present invention relates to the field of biomedicine, and in particular to a modified cell and uses thereof.

### BACKGROUND

Currently, immunotherapy is an effective method for treating patients with poor prognosis. However, the immune cells used in immunotherapy have problems such as weak cell function or weak proliferation and survival after infusion. Therefore, how to provide a modified immune cell and a robust and reliable immune cell culture method is an urgent issue to be solved.

### SUMMARY

The present invention provides a method for culturing cells, which has one or more of the following advantages: enhanced target cell killing ability, enhanced cell proliferation ability, enhanced cytokine release ability, increased proportion of activated cells, reduced proportion of regulatory cells, reduced proportion of exhausted cells, increased proportion of central memory cells and/or naive cells, reduced proportion of apoptotic cells and increased proportion of stem cell-like cells.

In one aspect, the present invention provides a method for culturing cells, the method comprising: reducing the expression and/or attenuating the activity of a family member and/or a functionally active fragment thereof provided by the present invention in the cell.

In another aspect, the present invention provides a cell obtained by the method of the present invention.

In another aspect, the present invention provides a pharmaceutical composition comprising the cell of the present invention, and optionally a pharmaceutically acceptable carrier.

In another aspect, the present invention provides a method of influencing cell growth, comprising administering the cells of the present invention and/or the pharmaceutical composition of the present invention.

In another aspect, the present invention provides use of the cell of the present invention and/or the pharmaceutical composition of the present invention in the preparation of a medicament for preventing and/or treating a disease and/or symptom.

Those skilled in the art can easily discern other aspects and advantages of the present invention from the detailed description below. In the detailed description below, only exemplary embodiments of the present invention are shown and described. As will be appreciated by those skilled in the art, the content of the present invention enables those skilled in the art to make changes to the disclosed specific embodiments without departing from the spirit and scope of the invention to which the present invention relates. Accordingly, the descriptions in the drawings and specification of the present invention are merely exemplary and not restrictive.

### BRIEF DESCRIPTION OF THE DRAWINGS

The features and advantages of the invention can be better understood by referring to the exemplary embodiments and drawings described in detail below. The drawings are briefly described as follows:
FIG. 1A shows the human RASA2 gene editing target region relative to the start codon provided by the present invention, for example, it may be a continuous region with about 3 or more transcription factor binding numbers; and it may be an exon region of the gene or an intron region about 20 bp away from the exon.
FIG. 1B shows the human FIBP gene editing target region relative to the start codon provided by the present invention, for example, it may be a continuous region with about 3 or more transcription factor binding numbers; and it may be an exon region of the gene or an intron region about 20bp away from the exon.
FIG. 1C shows the human MED12 gene editing target region relative to the start codon provided by the present invention, for example, it may be a continuous region with about 3 or more transcription factor binding numbers; and may be an exon region of the gene or an intron region about 20 bp away from the exon.
FIG. 1D shows the human TIGIT gene editing target region relative to the start codon provided by the present invention, for example, it may be a continuous region with about 3 or more transcription factor binding numbers; and it may be an exon region of the gene or an intron region about 20 bp away from the exon.
FIG. 1E shows the human BRD4 gene editing target region relative to the start codon provided by the present invention, for example, it may be a continuous region with about 3 or more transcription factor binding numbers; and may be an exon region of the gene or an intron region about 20 bp away from the exon.
FIG. 1F shows the human IKZF1 gene editing target region relative to the start codon provided by the present invention, for example, it may be a continuous region with about 3 or more transcription factor binding numbers; and may be an exon region of the gene or an intron region about 20 bp away from the exon.
FIG. 1G shows the human ADNP gene editing target region relative to the start codon provided by the present invention, for example, it may be a continuous region with about 3 or more transcription factor binding numbers; and it may be an exon region of the gene or an intron region about 20 bp away from the exon.
FIG. 1H shows the human NFKBIA gene editing target region relative to the start codon provided by the present invention, for example, it may be a continuous region with about 3 or more transcription factor binding numbers; and may be an exon region of the gene or an intron region about 20 bp away from the exon.
FIG. 1I shows the human PTPN6 gene editing target region relative to the start codon provided by the present invention, for example, it may be a continuous region with about 3 or more transcription factor binding numbers; and it may be an exon region of the gene or an intron region about 20 bp away from the exon.
FIG. 1J shows the human TNIP1 gene editing target region relative to the start codon provided by the present invention, for example, it may be a continuous region with about 3 or more transcription factor binding numbers; and it may be an exon region of the gene or an intron region about 20 bp away from the exon.
FIG. 2A shows that RASA2 gene-edited T cells in the unstimulated group can have significant expansion capacity.
FIG. 2B shows that RASA2 gene-edited T cells in the CD3 antibody stimulated group can have significant expansion capacity.
FIG. 2C shows that RASA2 gene-edited T cells have a lower proportion of exhausted T cells.
FIG. 2D shows that the RASA2 gene-edited T cells in the unstimulated group had a higher cytokine expression ratio.
FIG. 2E shows that the RASA2 gene-edited T cells in the stimulated group had a higher cytokine expression ratio.
FIG. 3A shows that FIBP gene-edited T cells in the unstimulated group can have significant expansion capacity.
FIG. 3B shows that FIBP gene-edited T cells in the CD3 antibody stimulated group can have significant expansion capacity.
FIG. 3C shows that FIBP gene-edited T cells have a higher proportion of central memory T cells.
FIG. 3D shows that FIBP gene-edited T cells have a lower proportion of exhausted T cells.
FIG. 3E shows that the FIBP gene-edited T cells in the unstimulated group had a higher cytokine expression ratio.
FIG. 3F shows that the FIBP gene-edited T cells in the stimulated group had a higher cytokine expression ratio.
FIG. 4A shows that MED12 gene-edited T cells in the unstimulated group can have significant expansion capacity.
FIG. 4B shows that the MED12 gene-edited T cells in the CD3 antibody stimulated group can have significant expansion capacity.
FIG. 4C shows the cytotoxicity of MED12 gene-edited T cells to target cells.
FIG. 4D shows that T cells after MED12 gene editing have a higher proportion of central memory T cells.
FIG. 4E shows that T cells after MED12 gene editing have a lower proportion of exhausted T cells.
FIG. 4F shows that the MED12 gene-edited T cells in the unstimulated group had a higher cytokine expression ratio.
FIG. 4G shows that the MED12 gene-edited T cells in the stimulated group had a higher cytokine expression ratio.
FIG. 5A shows that TIGIT gene-edited T cells in the unstimulated group can have significant expansion capacity.
FIG. 5B shows that TIGIT gene-edited T cells in the CD3 antibody stimulated group can have significant expansion capacity.
FIG. 5C shows that T cells after TIGIT gene editing have a higher proportion of central memory T cells.
FIG. 5D shows that T cells after TIGIT gene editing have a lower proportion of exhausted T cells.
FIG. 5E shows that TIGIT gene-edited T cells in the unstimulated group had a higher cytokine expression ratio.
FIG. 5F shows that TIGIT gene-edited T cells in the stimulated group had a higher cytokine expression ratio.
FIG. 6A shows that BRD4 gene-edited T cells in the unstimulated group can have significant expansion capacity.
FIG. 6B shows that BRD4 gene-edited T cells in the CD3 antibody stimulated group can have significant expansion capacity.
FIG. 6C shows that BRD4 gene-edited T cells have a higher proportion of central memory T cells.
FIG. 6D shows that BRD4 gene-edited T cells have a lower proportion of exhausted T cells.
FIG. 6E shows that the BRD4 gene-edited T cells in the unstimulated group have a higher cytokine expression ratio.
FIG. 6F shows that the BRD4 gene-edited T cells in the stimulated group have a higher cytokine expression ratio.
FIG. 7A shows the increase fold of IKZF 1 gene-edited TILs in the unstimulated medium group.
FIG. 7B shows the increase fold of IKZF1 gene-edited TILs in the TransACT stimulated group.
FIG. 7C shows the cytotoxicity of the IKZF1 gene-edited T cells derived from the first donor to target cells.
FIG. 7D shows the cytotoxicity of the IKZF1 gene-edited T cells derived from the second donor to target cells.
FIGs. 7E, 7F, 7G, and 7H show the proportion of exhausted T cells in TILs after IKZF1 gene editing.
FIG. 7I shows the expression ratio of stem T cells in TILs after IKZF1 gene editing.
FIG. 7J shows the cytokine expression ratio in TILs after IKZF1 gene editing in the unstimulated group.
FIG. 7K shows the cytokine expression ratio of TILs after IKZF1 gene editing in the stimulated group.
FIG. 7L shows the results of serial killing by TCR-T cells without editing or knocking out the IKZF1 target gene.
FIG. 7M shows the cytokine release of TCR-T cells without editing or knockout of IKZF1 target gene using a CBA kit.
FIG. 8A shows the increase fold of TILs in the unstimulated medium group after gene editing in combination with IKZF1.
FIG. 8B shows the increase fold of TILs in the TransACT stimulated group that were gene-edited in combination with IKZF1.
FIGs. 8C, 8D, and 8E show the cytotoxicity of TILs gene-edited in combination with IKZF1 to target cells.
FIGs. 8F, 8G, 8H, 8I, and 8J show the cytokine expression ratios of TILs in the unstimulated group that were gene-edited in combination with IKZF1.
FIGs. 8K, 8L, 8M, 8N, 8O, 8P, and 8Q show the cytokine expression ratios of TILs gene-edited in combination with IKZF1 in the stimulated group.
FIG. 9A shows the increase fold of TILs edited with the target gene of the present invention in the unstimulated group.
FIG. 9B shows the increase fold of TILs edited with the target gene of the present invention in the TransACT stimulated group.
FIG. 9C shows the cytotoxicity of the TILs edited with the target gene derived from donor 812 to target cells in the present invention.
FIG. 9D shows the cytotoxicity of the TILs edited with the target gene derived from donor 107 to target cells in the present invention.
FIG. 9E shows the proportion of central memory T cells in TILs after target gene editing in the present invention.
FIG. 9F shows the proportion of naive T cells in TILs after target gene editing in the present invention.
FIGs. 9G, 9H, 9I and 9J show the proportion of exhausted T cells in TILs after target gene editing in the present invention.
FIG. 9K and FIG. 9L show the proportion of stem T cells in TILs after target gene editing in the present invention.
FIG. 9M and FIG. 9N show the cytokine expression ratios in TILs after target gene editing in the unstimulated group in the present invention.
FIGs. 9O, 9P and 9Q show the cytokine expression ratios of TILs in the stimulated group after target gene editing in the present invention.
FIG. 9R shows the results of multiple rounds of killing by TCR-T cells that were not edited or knocked out in the targets of the present invention.
FIG. 9S shows the cytokine release of TCR-T cells that were not edited or knocked out in the targets of the present invention using a CBA kit.
FIG. 10A shows the increase fold of TILs edited by the target combination genes in the present invention in the unstimulated group.
FIG. 10B shows the increase fold of TILs edited by the target combination genes in the present invention in the TransACT stimulated group.
FIGs. 10C, 10D, 10E and 10F show the cytotoxicity of TILs edited by the target combination genes to target cells in the present invention.
FIGs. 10G, 10H, 10I, 10J and 10K show the cytokine expression ratios of TILs edited by the target combination genes in the present invention in the unstimulated group.
FIGs. 10L, 10M, 10N, 10O and 10P show the cytokine expression ratios of TILs edited by the target combination genes in the present invention in the stimulated group.
FIG. 10Q shows the cytokine release of TILs edited by the target combination genes in the present invention in the transACT stimulated group.
FIG. 10R shows the cytokine release of TILs after 24-hour co-culture with target cells A375, wherein the TILs are gene-edited with the target combination genes in the present invention.
FIG. 11A shows that RASA2 gene-edited TCR-T cells in the TransACT stimulated group can have significant expansion capacity.
FIG. 11B and FIG. 11C show the cytotoxicity of RASA2 gene-edited TCR-T cells to target cells.
FIGs. 11D, 11E, 11F, and 11G show the cytokine release of TCR-T cells without editing or knockout of RASA2 target gene using a CBA kit.
FIG. 12A shows that IKZF1 gene-edited TCR-T cells in the TransACT stimulated group can have significant expansion capacity.
FIG. 12B and FIG. 12C show the cytotoxicity of IKZF1 gene-edited TCR-T cells to target cells.
FIGs. 12D, 12E, 12F, and 12G show the cytokine release of TCR-T cells without editing or knockout of the IKZF1 target gene using a CBA kit.
FIG. 13A shows that TNIP1 gene-edited TCR-T cells in the TransACT stimulated group can have significant expansion capacity.
FIG. 13B and FIG. 13C show the cytotoxicity of TNIP1 gene-edited TCR-T cells to target cells.
FIGs. 13D, 13E, 13F, and 13G show the cytokine release of TCR-T cells without editing or knockout of TNIP1 target gene using a CBA kit.

### DETAILED DESCRIPTION

The following describes embodiments of the present invention by means of specific examples. Those skilled in the art can easily understand other advantages and effects of the present invention from the contents disclosed in this specification.

### Definition of Terms

In the present invention, the term "CBL family member" generally refers to a family member protein having an SH3 domain or a functionally active fragment thereof. For example, a CBL family member may include CBLB. For example, the UniProt number of a CBL family member may be Q13191. The CBL family members of the present invention may also include functionally active fragments thereof, not limited to human and mammalian forms, conservative amino acid substitutions, glycoform modifications or variants, active fragments thereof, or substances containing the functionally active fragments thereof produced after processing and/or modification in cells. For example, the CBL family members of the present invention may contain functionally active fragments thereof and any other domains.

In the present invention, the term "STAT-induced STAT inhibitor (SSI) family member" generally refers to a family member protein having an SH2 domain or a functionally active fragment thereof. For example, a STAT-induced STAT inhibitor (SSI) family member may include SOCS1. For example, the UniProt number of a STAT-induced STAT inhibitor (SSI) family member may be 015524. The STAT-induced STAT inhibitor (SSI) family member of the present invention may also encompass its functionally active fragments, not limited to human and mammalian forms, conservative amino acid substitutions, glycoform modifications or variants, its active fragment, or a substance containing the functionally active fragment produced after processing and/or modification thereof in a cell. For example, the STAT-induced STAT inhibitor (SSI) family member of the present invention may contain its functionally active fragments and any other domains.

In the present invention, the term "peptidase C64 family member" generally refers to a family member protein or a functionally active fragment thereof having a ubiquitin binding domain. For example, a peptidase C64 family member may include TNFAIP3. For example, the UniProt number of a peptidase C64 family member may be P21580. The peptidase C64 family member of the present invention may also encompass its functionally active fragments, not limited to human and mammalian forms, conservative amino acid substitutions, glycoform modifications or variants, its active fragments, or substances containing the functionally active fragments produced after processing and/or modification thereof in cells. For example, the peptidase C64 family member of the present invention may contain its functionally active fragments and any other domains.

In the present invention, the term "ZC3H12 family member" generally refers to a family member protein or a functionally active fragment thereof having a C3H1-type zinc finger domain. For example, a ZC3H12 family member may include ZC3H12A. For example, the UniProt number of a ZC3H12 family member may be Q5D1E8. The ZC3H12 family members of the present invention may also include functionally active fragments thereof, not limited to human and mammalian forms, conservative amino acid substitutions, glycoform modifications or variants, active fragments thereof, or substances containing the functionally active fragments thereof produced after processing and/or modification thereof in cells. For example, the ZC3H12 family members of the present invention may contain functionally active fragments thereof and any other domains.

In the present invention, the term "IKAROS zinc finger protein family member" generally refers to a family member protein having a zinc finger domain or a functionally active fragment thereof. For example, a IKAROS zinc finger protein family member may include IKZF1. For example, the UniProt number of a IKAROS zinc finger protein family member may be Q13422. The IKAROS zinc finger protein family members of the present invention may also include its functionally active fragments, not limited to human and mammalian forms, conservative amino acid substitutions, glycoform modifications or variants, its active fragments, or substances containing the functionally active fragments produced after its processing and/or modification in cells. For example, the IKAROS zinc finger protein family members of the present invention may contain functionally active fragments thereof and any other structural domains.

In the present invention, the term "tumor necrosis factor alpha-induced protein 3 (TNFAIP3)" generally refers to an inhibitory molecule of a signaling pathway. For example, TNFAIP3 can ubiquitinate a signal transduction substance of the NF-κB pathway. For example, the UniProt accession number of TNFAIP3 can be P21580. In the present invention, TNFAIP3 can include unprocessed TNFAIP3, any form of processed TNFAIP3, a variant of TNFAIP3, or a substance containing a functionally active fragment of TNFAIP3.

In the present invention, the term "GTPase activating protein 1 family member" generally refers to a family member protein having a GTPase activation domain or a functionally active fragment thereof. For example, a GTPase activating protein 1 family member may include RASA2. For example, the UniProt number of a GTPase activating protein 1 family member may be Q15283. The GTPase activating protein 1 family member of the present invention may also encompass its functionally active fragments, not limited to human and mammalian forms, conservative amino acid substitutions, glycoform modifications or variants, its active fragments, or substances containing the functionally active fragments produced after processing and/or modification thereof in cells. For example, the GTPase activating protein 1 family members of the present invention may contain its functionally active fragments and any other domains.

In the present invention, the term "FGF binding protein family member" generally refers to a family member protein having an FGF binding domain or a functionally active fragment thereof. For example, a FGF binding protein family member may include FIBP. For example, the UniProt number of a FGF binding protein family member may be O43427. The FGF binding protein family members of the present invention may also encompass its functionally active fragments, not limited to human and mammalian forms, conservative amino acid substitutions, glycoform modifications or variants, its active fragments, or substances containing the functionally active fragments produced after processing and/or modification thereof in cells. For example, the FGF binding protein family members of the present invention may contain its functionally active fragments and any other domains.

In the present invention, the term "Mediator (MED) family member" generally refers to a family member protein or a functionally active fragment thereof having a CDK8 binding domain. For example, a Mediator (MED) family member may include MED12. For example, the UniProt number of a Mediator (MED) family member may be Q93074. The Mediator (MED) family members of the present invention may also include its functionally active fragments, not limited to human and mammalian forms, conservative amino acid substitutions, glycoform modifications or variants, its active fragments, or substances containing the functionally active fragments produced after processing and/or modification thereof in cells. For example, the Mediator (MED) family members of the present invention may contain its functionally active fragments and any other domains.

In the present invention, the term "PVR binding protein family member" generally refers to a family member protein having a PVR binding domain or a functionally active fragment thereof. For example, a PVR binding protein family member may include TIGIT. For example, the UniProt number of a PVR binding protein family member may be Q495A1. The PVR binding protein family members of the present invention may also include functionally active fragments thereof, not limited to human and mammalian forms, conservative amino acid substitutions, glycoform modifications or variants, active fragments thereof, or the substances produced after processing and/or modification thereof in cells. For example, the PVR binding protein family members of the present invention may contain functionally active fragments of the substance as well as any other domains.

In the present invention, the term "BET family member" generally refers to a family member protein having a bromodomain or a functionally active fragment thereof. For example, a BET family member may include BRD4. For example, the UniProt number of a BET family member may be O60885. The BET family members of the present invention may also encompass functionally active fragments thereof, not limited to human and mammalian forms, conservative amino acid substitutions, glycoform modifications or variants, active fragments thereof, or the substances produced after processing and/or modification thereof in cells. For example, the BET family members of the present invention may contain functionally active fragments of the substance as well as any other domains.

In the present invention, the term "activity-dependent neuroprotective protein family member" generally refers to a family member protein having a zinc finger domain or a functionally active fragment thereof. For example, an activity-dependent neuroprotective protein family member may include ADNP. For example, the UniProt number of an activity-dependent neuroprotective protein family member may be Q9H2P0. The activity-dependent neuroprotective protein family members of the present invention may also include its functionally active fragments, not limited to human and mammalian forms, conservative amino acid substitutions, glycoform modifications or variants, its active fragments, or a substance containing a functionally active fragment of an activity-dependent neuroprotective protein family member produced after processing and/or modification in a cell. For example, the activity-dependent neuroprotective protein family members of the present invention may contain functionally active fragments of the substance and any other domains.

In the present invention, the term "NF-kappa-B inhibitory protein family member" generally refers to a family member protein or a functionally active fragment thereof having an ankyrin repeat domain. For example, a protein tyrosine phosphatase family member may include NFKBIA. For example, the UniProt number of an NF-kappa-B inhibitory protein family member may be P25963. The NF-kappa-B inhibitory protein family members of the present invention may also encompass its functionally active fragments, not limited to human and mammalian forms, conservative amino acid substitutions, glycoform modifications or variants, its active fragments, or substances containing functionally active fragments of NF-kappa-B inhibitory protein family members produced after processing and/or modification in cells. For example, the NF-kappa-B inhibitory protein family members of the present invention may contain functionally active fragments of the substance and any other domains.

In the present invention, the term "protein tyrosine phosphatase family member" generally refers to a family member protein or a functionally active fragment thereof having a Src homolog (SH2) domain. For example, a protein tyrosine phosphatase family member may include PTPN6. For example, the UniProt number of a protein tyrosine phosphatase family member may be P29350. The protein tyrosine phosphatase family members of the present invention may also encompass its functionally active fragments, not limited to human and mammalian forms, conservative amino acid substitutions, glycoform modifications or variants, its active fragments, or substances containing functionally active fragments of protein tyrosine phosphatase family members produced after processing and/or modification in cells. For example, the protein tyrosine phosphatase family members of the present invention may contain functionally active fragments of the substance as well as other domains.

In the present invention, the term "A20 binding protein family member" generally refers to a family member protein having an A20 binding domain or a functionally active fragment thereof. For example, an A20 binding protein family may include TNIP1. For example, the UniProt number of an A20 binding protein family member may be Q15025. The A20 binding protein family member of the present invention may also include its functionally active fragments, not limited to human and mammalian forms, conservative amino acid substitutions, glycoform modifications or variants, its active fragments, or substances containing functionally active fragments of A20 binding protein family members produced after processing and/or modification in cells. For example, the A20 binding protein family members of the present invention may contain functionally active fragments of the substance and any other domains.

In the present invention, the term "immune cell" generally refers to cells involved in innate and adaptive immune responses. For example, lymphocytes (such as T cells (including thymocytes) and B cells), natural killer (NK) cells, NKT cells, macrophages, monocytes, eosinophils, basophils, neutrophils, dendritic cells and mast cells may be included but are not limited to. In some embodiments, the modified immune effector cells are T cells, such as CD4+T cells, CD8+T cells (also referred to as cytotoxic T cells or CTL), regulatory T cells (Treg), Th1 cells, Th2 cells, Th17 cells αβT cells and/or γδT cells. For example, the immune cells of the present invention also include immune cells derived from stem cell differentiation. For example, the immune cells of the present invention also include immune cells derived from pluripotent stem cell differentiation. For example, obtaining the stem cells of the present invention can be produced by induction. For example, the above-mentioned stem cells of the present invention can include induced pluripotent stem cells (iPSC).

In the present invention, the term "chimeric antigen receptor" (CAR) generally refers to an engineered antigen receptor. For example, CAR may contain an extracellular antigen binding domain fused to a cytoplasmic domain comprising a signaling domain via a hinge and a transmembrane domain. In some embodiments, the CAR extracellular domain can bind to an antigen expressed by a target cell in an MHC-independent manner, thereby causing cell activation and proliferation. In some embodiments, the extracellular domain of CAR can recognize a tag fused to an antibody or its antigen-binding fragment. For example, a single CAR construct can be made to target a variety of different antigens by replacing one antibody with another antibody. In some embodiments, the extracellular domain of CAR may contain an antigen-binding fragment derived from an antibody. Antigen binding domains that can be used in the present disclosure include, for example, scFv, antibodies, antigen-binding regions of antibodies, variable regions of heavy chains/light chains, and/or single-chain antibodies.

In the present invention, the term "T cell receptor" generally refers to an engineered antigen receptor. For example, a TCR may comprise a TCR alpha and/or TCR beta chain that has been isolated and cloned from a population of T cells that recognize a specific target antigen. For example, the TCR alpha and/or TCR beta genes (i.e., TRAC and TRBC) may be cloned from T cell populations isolated from an individual with a specific malignancy or isolated from humanized mice immunized using tumor-specific antigens or tumor cells. The engineered TCR can recognize antigens by the same mechanism as its endogenous counterpart (e.g., by recognizing its cognate antigen presented in the context of major histocompatibility complex (MHC) proteins expressed on the surface of target cells), which can lead to the activation and proliferation of TCR-engineered cells.

In the present invention, the term "gene regulation system" generally refers to a system that regulates the expression or activity of a target gene. For example, a gene regulation system may include gene regulatory molecules. For example, a gene regulation system may regulate the expression or activity of a gene, such as rendering the gene in an inactive or activated state, increasing or decreasing the amount of the gene, rendering the gene in a state of increased or decreased transcription, and/or rendering the transcription product of the gene in an inactive or activated state; for example, a gene regulation system may regulate the expression or activity of a gene, such as increasing or decreasing the amount of the expression product of the gene in individual cells and/or increasing or decreasing the number of cells expressing the expression product of the gene.

In the present invention, the term "guide nucleic acid molecule" generally refers to a nucleic acid molecule that can be used for gene editing. For example, a guide nucleic acid molecule can provide information for nucleotide insertion or deletion to guide the editing process. For example, a guide nucleic acid molecule can be a guide RNA or a gRNA. For example, "gRNA" can refer to an RNA molecule that binds to a Cas protein and targets the Cas protein to a specific location in a target DNA. For example, where hybridization between a gRNA and a DNA target sequence promotes the formation of a CRISPR complex, complete complementarity may not be required, for example, as long as there is sufficient complementarity to cause hybridization and promote the formation of a CRISPR complex.

In the present invention, the term "enzyme protein" generally refers to a protein with enzymatic activity. For example, an enzyme protein may refer to a Cas protein. For example, a Cas protein may contain at least one RNA recognition or binding domain that can interact with gRNAs. A Cas protein may also contain a nuclease domain (e.g., a DNase or RNase domain), a DNA binding domain, a helicase domain, a protein-protein interaction domain, a dimerization domain, and/or other domains. The nuclease domain may have catalytic activity for nucleic acid cutting. Cutting may include breaking of covalent bonds of nucleic acid molecules. A Cas protein may be a wild-type protein (i.e., proteins existing in nature), modified Cas proteins (i.e., Cas protein variants), or fragments of wild-type or modified Cas proteins. A Cas protein may also be an active variant or fragment of a wild-type or modified Cas protein. In the present invention, Cas proteins may include unprocessed Cas proteins, any form of processed Cas proteins, variants of Cas proteins, or substances containing functionally active fragments of Cas proteins.

In the present invention, the term "ribonucleoprotein complex" generally refers to a complex formed by a protein and a nucleic acid. For example, the protein in a ribonucleoprotein complex can have nuclease activity. For example, a ribonucleoprotein complex can cut the target sequence under the guidance of the nucleic acid therein. For example, a ribonucleoprotein complex can be a complex formed by a Cas protein and a gRNA.

In the present invention, the term "lipid nanoparticle (LNP)" generally refers to a lipid-nucleic acid particle or a nucleic acid-lipid particle. For example, LNP refers to a particle made of lipids (e.g., cationic lipids, non-cationic lipids, and conjugated lipids that prevent particle aggregation) and nucleic acids, wherein nucleic acids (e.g., mRNA, gRNA, siRNA, aiRNA, miRNA, ssDNA, dsDNA, ssRNA, short hairpin RNA (shRNA), dsRNA, self-amplifying RNA or plasmids, including plasmids from which interfering RNA or mRNA is transcribed) are encapsulated in lipids. For example, proteins can be encapsulated in LNPs, for example, Cas proteins known in the art can be encapsulated in LNPs. For example, the lipids in LNPs include (1) "simple lipids", which include fats and oils and waxes; (2) "complex lipids", which include phospholipids and glycolipids; and (3) "derived lipids" such as steroids. For example, the lipids in LNPs can also include lipid derivatives, such as lipids covalently or non-covalently bound to proteins or polypeptides. For example, the components in LNP may also include a polypeptide component, wherein the polypeptide component may replace one or more lipid components in traditional LNP to maintain or improve the delivery ability of LNP.

In the present invention, the term "exon" generally refers to a portion of a gene that can be expressed as a protein. For example, an exon can refer to a portion of a gene that has the ability to be expressed as a protein during protein biosynthesis. For example, splicing the exon sequence of a target gene can reduce the activity or function of the target gene.

In the present invention, the term "intron" generally refers to a segment in DNA that does not encode part or all of the expressed protein. Usually under endogenous conditions, introns are transcribed into RNA molecules, but they are spliced off from endogenous RNA before being translated into proteins. For example, editing by targeting the location of introns can reduce the activity or function of the target gene. For example, editing by targeting the junction of introns and exons, such as editing intron regions about 0 bp to about 100 bp upstream or downstream of exons, preferably about 0 bp to about 20 bp, can reduce the activity or function of the target gene.

In the present invention, the term "start codon" generally refers to a unit of adjacent nucleotides ('codon') on a gene that can define the start of protein synthesis (mRNA translation). For example, targeting the region 0 bp to 1500 bp upstream of the start codon, preferably 0 bp to 100 bp upstream of the start codon for editing can reduce the activity or function of the target gene.

In the present invention, the term "protospacer adjacent motif (PAM)" generally refers to a short sequence after a target sequence. For example, when Cas9 performs site-specific cleavage of a target DNA, the PAM sequence can be used to determine the location of the cleavage. For example, by determining the region of the PAM, a person skilled in the art can easily determine the appropriate target sequence location, and can easily design a gRNA sequence for cleaving the target sequence.

In the present invention, the term "reduced expression" generally refers to a decrease in the amount of expression of a product or its gene and/or a decrease in the proportion of cells capable of expressing the product (e.g., at least about 5-100%). For example, it may be that the amount of the product expressed by the gene in the cell is reduced or the proportion of cells containing the product expressed by the gene is reduced, or the proportion of cells secreting the product expressed by the gene is reduced. For example, the amount of knockout of the gene in the genome of the cell can be detected to indirectly indicate that the expression of the gene is reduced. For example, the proportion of cells in which the gene is knocked out in a cell population can be detected to indirectly indicate that the expression of the gene is reduced.

In the present invention, the term "activity" generally refers to the biological function of a substance. For example, the activity of a gene may refer to the transcription and/or translation state of the gene. For example, a reduction of the activity of a gene (e.g., at least about 5-100%) may mean that the transcription function of the gene is weakened, that the gene cannot be normally transcribed, or that the function of the transcription product of the gene is inhibited.

In the present invention, the term "CD80" generally refers to a cell stimulatory molecule. For example, CD80 can be a ligand of CD28. For example, CD80 can be found in GenBank Accession No. P33681. The CD80 proteins of the present invention can also include its functionally active fragments, not limited to substances containing functionally active fragments of CD80 produced after processing and/or modification in cells. For example, the CD80 of the present invention can contain functionally active fragments of CD80 and any other domains.

In the present invention, the term "CD86" generally refers to a cell stimulatory molecule. For example, CD86 can be a ligand of CD28. For example, CD86 can be found in GenBank Accession No. P42081. The CD86 proteins of the present invention can also include its functionally active fragments, not limited to substances containing functionally active fragments of CD86 produced after processing and/or modification in cells. For example, the CD86 of the present invention can include functionally active fragments of CD86 and any other domains.

In the present invention, the term "secreted" generally refers to a substance that can be located outside of a cell. For example, a secreted substance can be transported to the extracellular space of a cell after being synthesized inside the cell. For example, whether a substance is a secreted substance can be detected by enzyme-linked immunosorbent assay or other detection methods.

In the present invention, the term "T cell receptor" or "TCR" generally refers to a complex of membrane proteins that participate in the activation of T cells in response to the presentation of antigens. TCR can be responsible for recognizing antigens that are bound to major histocompatibility complex molecules. TCR can be composed of heterodimers of alpha (α) and beta (β) chains, or composed of gamma and delta (γ/δ) chains. TCR can exist in α/β and γ/δ forms, which are structurally similar, but have unique anatomical locations and functions. For example, TCR can be a TCR that is modified on any cell expressing TCR. For example, the type of TCR can be analyzed by TCR subtyping reagents.

In the present invention, the term "clonal diversity" generally refers to a substance having multiple clonal types. For example, the clonal diversity of TCR may mean that TCR may have different sequence structures and/or antigen recognition abilities. For example, the diversity of TCR is often distinguished by β-chain subtypes, which can include Vβ23, Vβ7.2, Vβ5.2, Vβ11, Vβ16, Vβ3, etc. When a T cell population has more β-chain subtypes, it can be considered that the T cell population has higher clonal diversity.

In the present invention, "CD4⁺ Cells" generally refer to CD4-positive cells, such as T cells. The terms "CD4⁺ cells" and "CD4 positive cells" can be used synonymously. These cells can be identified by methods known in the art, such as by staining the cells with fluorescently labeled antibodies against CD4 and using fluorescence activated cell sorting. For example, existing data can show that an increase in the ratio of CD4⁺ cells can increase the ability of the cell population to secrete IFN and/or TNF, and can improve the effect of the T cell population in promoting tumor suppression. For example, see Tay, RE, Richardson, EK et al. (2020). Cancer Gene Therapy, 1-13. However, there is a lack of a method for increasing the ratio CD4⁺ cells in the field. The present invention can provide a method for affecting CD4⁺ cell ratio.

In the present invention, "CD8⁺ Cells" generally refer to CD8-positive cells, such as T cells. The terms "CD8⁺ cells" and "CD8 positive cells" can be used synonymously. These cells can be identified by methods known in the art, such as by staining the cells with fluorescently labeled antibodies against CD8 and using fluorescence activated cell sorting.

In the present invention, the term "IC₅₀ value" or "IC50 value" generally refers to the concentration of the target substance required to achieve 50% inhibition of a biological process. The IC50 value can be converted to an absolute inhibition constant (Ki) using the Cheng-Prusoff equation (Biochem. Pharmacol. (1973) 22: 3099).

In the present invention, the term "K_{D} value" or "KD value" generally refers to the dissociation constant, which can be determined by surface plasmon resonance. Typically, surface plasmon resonance analysis uses a BIAcore system (Pharmacia Biosensor, Piscataway, NJ) to measure the real-time binding interaction between a ligand (a substance immobilized on a biosensor matrix) and an analyte (a substance in solution) by surface plasmon resonance (SPR). Surface plasmon analysis can also be performed with an immobilized analyte (a substance on a biosensor matrix) and a presented ligand.

In the present invention, the term "encoding" generally refers to the ability to directly or indirectly infer, from the structure or composition information of one molecule, the structure or composition information of another type of molecule related to it, according to essentially determined rules. For example, the nucleotide sequence can be inferred from the sequence of amino acids, such as according to the characteristics of transcribing complementary nucleic acids from deoxyribonucleic acid, including nucleic acids that can be translated into polypeptides. For example, deoxyribonucleic acid can encode RNA transcribed from deoxyribonucleic acid. Deoxyribonucleic acid can similarly encode polypeptides translated from RNA transcribed from deoxyribonucleic acid.

In the present invention, the term "small molecule compound" generally refers to peptides, peptide mimetics, amino acids, amino acid analogs, polynucleotides, polynucleotide analogs, nucleotides, nucleotide analogs, organic or inorganic substances with a molecular weight of less than about 10,000 g/mole (i.e., including heterologous organic substances and organometallic compounds), organic or inorganic substances with a molecular weight of less than about 5,000 g/mole, organic or inorganic substances with a molecular weight of less than about 1,000 g/mole, organic or inorganic substances with a molecular weight of less than about 500 g/mole, and salts, esters and other pharmaceutically acceptable forms of such drugs.

In the present invention, the term "NK cell" is also called "natural killer cell", which generally refers to a cell with large granules in the cytoplasm. NK cells develop from bone marrow lymphoid stem cells and can differentiate and develop depending on the bone marrow or thymus microenvironment. In the present invention, the proportion of NK cells in TIL cells can be changed by the method of the present invention.

In the present invention, the term "antibody" generally refers to an immunoglobulin or its fragment or derivative, covering any polypeptide comprising an antigen binding site, whether produced in vitro or in vivo. The term includes, but is not limited to, polyclonal, monoclonal, monospecific, multispecific, nonspecific, humanized, single-chain, chimeric, synthetic, recombinant, hybrid, mutated and transplanted antibodies. Unless otherwise modified by the term "complete", such as in "complete antibody", for the purposes of the present invention, the term "antibody" also includes antibody fragments, such as Fab, F(ab') ₂, Fv, scFv, Fd, dAb and other antibody fragments that retain antigen binding function (e.g., specific binding to CD3). Generally, such fragments should include an antigen binding domain. The basic 4-chain antibody unit is a heterotetrameric glycoprotein composed of two identical light (L) chains and two identical heavy (H) chains. IgM antibodies are composed of 5 basic heterotetrameric units and another polypeptide called a J chain, and contain 10 antigen binding sites, while IgA antibodies contain 2-5 basic 4-chain units that can be combined with J chains to form a multivalent combination. For IgG, the 4-chain unit is generally about 150,000 Daltons. Each L chain is connected to the H chain by a covalent disulfide bond, and the two H chains are connected to each other by one or more disulfide bonds depending on the isotype of the H chain. Each H and L chain also has a regularly spaced intrachain disulfide bridge. Each H chain has a variable domain (VH) at the N-terminus, followed by three constant domains (CH) for α and γ chains, and four CH domains for µ and ε isotypes. Each L chain has a variable domain (VL) at the N-terminus and a constant domain at its other end. VL corresponds to VH, and CL corresponds to the first constant domain (CH1) of the heavy chain. Specific amino acid residues are believed to form an interface between the light chain and the heavy chain variable domains. VH and VL pair together to form a single antigen binding site. The L chains from any vertebrate species can be divided into one of two clearly distinct types, called kappa and lambda, based on the amino acid sequence of their constant domains. Depending on the amino acid sequence of the heavy chain (CH) constant domain, immunoglobulins can be divided into different classes or isotypes. There are currently five classes of immunoglobulins: IgA, IgD, IgE, IgG, and IgM, with heavy chains designated α, δ, ε, γ, and µ, respectively.

In the present invention, the term "antigen-binding fragment" generally refers to one or more polypeptide fragments that have the ability to specifically bind to an antigen. In the present invention, the antigen-binding fragment may include Fab, Fab', F(ab)₂, Fv fragment, F(ab')₂, scFv, di-scFv and/or dAb.

In the present invention, the term "expression" generally refers to the transcription and/or translation process of the gene encoding the target polypeptide in the cell. The transcription level of the gene encoding the target polypeptide in the host cell can be determined by measuring the amount of the corresponding mRNA present in the cell. For example, the mRNA transcribed from the gene encoding the target polypeptide can be quantitatively measured by PCR or by RNA hybridization. The translation level of the gene encoding the target polypeptide can be measured by a variety of methods, such as by ELISA, by polypeptide biological activity test, or by protein blotting or radioimmunoassay. In the present invention, the term "expression" generally also refers to the transcription and/or translation process of the product. For example, the expression of a cytokine can be the process of a cell transcribing and/or translating the cytokine. For example, the expression of a cytokine can be determined by detecting the amount of the corresponding mRNA present in the cell or by detecting the amount of the cytokine produced by the cell, or both.

In the present invention, the term "stage" in "a stage of in vitro expansion", "a single stage of in vitro expansion", or "a first stage of in vitro expansion" generally refers to a period of expansion that TIL undergoes in vitro. In one embodiment, each stage can be divided by the change in the number of TIL cells. In one embodiment, when the number of TIL cells increases by at least about 1 time, it can be considered that the TIL cells have entered the next stage of in vitro expansion. In some embodiments, when the number of TIL cells increases by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times, it can be considered that the TIL cells have entered the next stage of in vitro expansion. In one embodiment, each stage can also be divided by the conditions of TIL cell culture. In one embodiment, when T cell activators and/or T cell growth factors are added or supplemented to the cell culture medium, it can be considered that the TIL cells have entered the next stage of in vitro expansion. In one embodiment, when the TIL cells are centrifuged and/or washed, it can be considered that the TIL cells have entered the next stage of in vitro expansion. In one embodiment, each stage can also be divided by the number of days of TIL cell culture. In one embodiment, after the TIL cells are cultured in vitro for about 1-100 days, for example, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, the TIL cells can be considered to have entered the next stage of in vitro expansion.

In the present invention, the term "first stage in vitro expansion" generally refers to the stage of expansion using T cell growth factors after obtaining primary TILs from tissues. In one embodiment, the tissue of the present invention can be selected from the following group: tumor tissues, tumor-associated lymph nodes with or without tumor metastasis, tumor metastatic lesions, fragments of paracancerous tissue, pleural effusion and/or peritoneal effusion. The pleural effusion of the present invention can be the pleural effusion of a patient with a metastatic cancer. In one embodiment, the expansion of the present invention may be an in vivo expansion performed autologously or allogeneically, or may be an in vitro expansion. The first stage of in vitro expansion of the present invention may also be referred to as the preREP (pre-rapid expansion) stage. For example, TILs derived from tumor tissue and not expanded in vitro may be referred to as a first TIL group. For example, TILs obtained by the first stage of in vitro expansion in the culture method of the present invention divided by the two-step method may be referred to as a second TIL group.

In the present invention, the term "second stage in vitro expansion" generally refers to the stage of expansion again after the tissue removed from the subject is expanded. In one embodiment, compared with the TIL expanded in vitro in the first stage, the number of TIL cells expaned in vitro in the second stage of the present invention increases, for example, it can increase by at least about 10 times (or at least about 20, 30, 40, 50, 60, 70, 80 or 90 times), or the number of cells in one embodiment can increase by at least about 100 times. In one embodiment, the second stage in vitro expansion can be different from the culture conditions of the first stage in vitro expansion, for example, the culture material added can be different. For example, the second stage in vitro expansion can also be referred to as the REP (rapid expansion) stage in the culture method of the present invention divided by the two-step method. For example, the TIL obtained by the second stage in vitro expansion in the culture method of the present invention divided by the two-step method can be referred to as a third TIL group.

In the present invention, the term "in vivo" generally refers to events occurring within the body of a subject.

In the present invention, the term "in vitro" generally refers to events that occur outside the body of a subject.

In the present invention, the term "ex vivo" generally refers to an event involving treatment or surgery on cells, tissues and/or organs that have been removed from the subject's body. In one embodiment, the cells, tissues and/or organs can be returned to the subject's body through surgery or treatment.

In the present invention, the term "secretion capacity" generally refers to the ability of a cell to express a polypeptide or protein and transfer the polypeptide or protein of the present invention to the extracellular environment.

In the present invention, the term "irradiation" generally refers to the treatment of a substance by radiation. For example, in one embodiment, irradiation may refer to the irradiation of a substance by X-rays, α-rays, β-rays, or γ rays.

In the present invention, the term "engineered cell" generally refers to a cell that has been genetically modified by adding additional genetic material in the form of DNA or RNA to the total genetic material of the cell. In one embodiment, the engineered cell can be genetically modified to express a TIL of a T cell activator and/or a T cell growth factor of the present invention.

In the present invention, the term "co-culture" generally refers to culturing two or more different populations of cells with a certain degree of contact between them. The "contact" of two or more different populations of cells of the present invention may be by direct contact in one embodiment, i.e., direct physical contact between cells of one population and cells of another population, or in one embodiment, indirect contact mediated by a shared culture medium. The shared culture medium of the present invention may contain metabolites produced and released by at least one population of co-cultured cells, and used to culture cells of another population.

In the present invention, the term "contact" generally refers to two or more different types of substances being in contact with each other in any order, in any manner, and for any duration. In one embodiment, direct contact may be used, for example, one or more feeder cells, T cell activators, and/or T cell growth factors may be added to the culture medium of TIL cells, for example, a culture medium containing one or more feeder cells, T cell activators, and/or T cell growth factors may be added to and/or replace the culture medium of TIL cells, for example, a culture medium containing one or more feeder cells, T cell activators, and/or T cell growth factors may be used for the culture of TIL cells; in one embodiment, indirect contact may be used, for example, metabolites produced and released by feeder cells may be used to culture TIL cells.

In the present invention, the terms "contacting simultaneously", "contacting together", "contacting at the same time as...", "concurrently" and "together" generally refer to administering two or more substances to a subject and/or a cell so that the substances are present in the subject and/or cell culture environment at the same time. Concurrent contact may include administering different compositions simultaneously, administering different compositions at different times, or administering a composition in which two or more active pharmaceutical ingredients are present. For example, "contacting simultaneously" in the present invention generally refers to contacting essentially simultaneously.

In the present invention, the term "expansion" generally refers to an increase in the number of cells by several times over a period of time. In one embodiment, the number of cells can be increased by at least about 3 times (or 4, 5, 6, 7, 8 or 9 times), in one embodiment, the number of cells can be increased by at least about 10 times (or 20, 30, 40, 50, 60, 70, 80 or 90 times), or in one embodiment, the number of cells can be increased by at least about 100 times. In the present invention, the term "expanded" generally refers to cells of the present invention undergoing one or more of the above-mentioned expansions.

In the present invention, the term "polymer" generally refers to a molecule consisting of separate chemical parts connected together, and the polymer parts of the present invention can be the same or different. In one embodiment, the term "polymer" can refer to separate chemical parts that are connected end to end to form a linear molecule, as well as separate chemical parts that are connected together in the form of a branched (such as "multi-arm" or "star") structure. In one embodiment, the polymers can include, for example, a polysaccharide, a dextran, a hydrogel, a polyethylene glycol, or a poloxamer. Poloxamer is a non-ionic triblock copolymer having a polyoxypropylene (poly (propylene oxide)) central hydrophobic chain and two polyoxyethylene (poly (ethylene oxide)) hydrophilic chains on the side. The substances included in the present invention can be formulated with any polymer described herein or known in the art, or administered together with them.

In the present invention, the term "chimeric antibody" generally refers to an antibody formed by fusing the variable region of a mouse antibody with the constant region of a human antibody, which can reduce the immune response induced by the mouse antibody. To establish a chimeric antibody, a hybridoma that secretes mouse-specific monoclonal antibodies can be established, and then the variable region gene can be cloned from the mouse hybridoma cells, and the constant region gene of the human antibody can be cloned as needed, and the mouse variable region gene and the human constant region gene can be connected into a chimeric gene and inserted into an expression vector, and the chimeric antibody molecule can be expressed in a eukaryotic system or a prokaryotic system.

In the present invention, the term "humanized antibody", also known as CDR-grafted antibody, generally refers to an antibody produced by transplanting mouse CDR sequences into human antibody variable region frameworks, i.e., different types of human germline antibody framework sequences, which can overcome the heterologous reactions induced by carrying a large amount of mouse protein components. Such framework sequences can be obtained from public DNA databases or published references including germline antibody gene sequences. For example, the germline DNA sequences of human heavy chain and light chain variable region genes can be found in the "VBase" human germline sequence database.

In the present invention, the terms "fully human antibody", "fully human antibody" or "completely human antibody", also known as "fully human monoclonal antibody", the variable region and constant region of the antibody thereof can be both human, eliminating immunogenicity and toxic side effects. The development of monoclonal antibodies has gone through four stages, namely: murine monoclonal antibodies, chimeric monoclonal antibodies, humanized monoclonal antibodies and fully human monoclonal antibodies. The antibody or ligand described in the present invention can be a fully human monoclonal antibody. The relevant technologies for the preparation of fully human antibodies can be: human hybridoma technology, EBV transformed B lymphocyte technology, phage display technology (phage display), transgenic mouse antibody preparation technology (transgenic mouse) and single B cell antibody preparation technology, etc.

In the present invention, the term "CDR" generally refers to one of the 6 hypervariable regions that mainly contribute to antigen binding in the variable domains of an antibody. One of the most commonly used definitions of the 6 CDRs can be provided by Kabat EA et al., Chothia et al. and MacCallum et al. As used in the present invention, the Kabat definition of CDR can be applied to CDR1, CDR2 and CDR3 of a light chain variable domain (CDR L1, CDR L2, CDR L3 or L1, L2, L3), and CDR1, CDR2 and CDR3 of a heavy chain variable domain (CDR H1, CDR H2, CDR H3 or H1, H2, H3).

In the present invention, the term "IL-2" or "IL2" generally refers to a T cell growth factor called interleukin 2, and includes all forms of IL-2, which may include in one embodiment human and mammalian forms, conservative amino acid substitutions, glycoform modifications or variants, or active fragments thereof. The GeneID encoding the IL-2 gene may be 3558.

In the present invention, the term "antigen presenting cell", "antigen presenting cell", or "APC" generally refers to an immune system cell, such as a helper cell (e.g., B cell, dendritic cell, etc.), which displays a foreign antigen in complex with a major histocompatibility complex (MHC) on its surface. T cells can recognize these complexes using its T cell receptor (TCR). The APC can process the antigen and present it to the T cell. In one embodiment, the antigen presenting cell can include cells selected from the following group: peripheral mononuclear cells, dendritic cells, and artificial antigen presenting cells.

In the present invention, the term "TIL characteristics" generally refers to the characteristics of TIL cells obtained by the culture method of the present invention. Changes in TIL characteristics may include: increased TIL cell number, increased viable cell ratio, increased survival ability, improved T cell subset ratio, increased cytokine secretion ability, improved in vitro tumor cell killing ability, improved in vivo tumor killing ability, increased T cell receptor (TCR) clonal diversity and increased TIL cell number in tissue, or any combination thereof. The changes of the present invention may be an increase or a decrease.

In the present invention, the term "survival" generally refers to the presence of cells in vitro and/or in a subject. For example, an increase in the survival ability of TIL cells may refer to an increase in the time that TIL cells exist in vivo. For example, an increase in survival ability may refer to an increase in the time that cells exist in a subject's tissues, such as a tumor, spleen, bone marrow, lung tissue, and blood. For example, an increase in survival ability may be an increase in the survival ability of TIL cells after IL-2 is removed from the culture medium.

In the present invention, the term "artificial antigen presenting cell" generally refers to an artificially constructed immune cell for presenting exogenous antigens. For example, the way of presenting exogenous antigens can be that the surface of the artificial antigen presenting cell contains a complex of exogenous antigens and major histocompatibility complex (MHC). In one embodiment, isolated artificial antigen presenting cells (aAPCs) can be included, which can include cells expressing HLA-A/B/C (the gene GeneID encoding it can be 3105, 3106 or 3107), CD64 (the gene GeneID encoding it can be 2209), CD80 (the gene GeneID encoding it can be 941), ICOS-L (the gene GeneID encoding it can be 23308) and CD58 (the gene GeneID encoding it can be 965), and can be modified to express more than one T cell activator.

In the present invention, the term "fusion protein" generally refers to a polypeptide or a protein containing an amino acid sequence of a first polypeptide or protein or its fragment, analog or derivative and an amino acid sequence of a heterologous polypeptide or protein (i.e., a second polypeptide or protein that is different from the first polypeptide or protein or its fragment, analog or derivative, or that is not normally a part of the first polypeptide or protein or a fragment, analog or derivative thereof). In some cases, the fusion protein may comprise a preventive or therapeutic drug fused to a heterologous protein, polypeptide or peptide, wherein the heterologous protein, polypeptide or peptide of the present invention may or may not be a preventive or therapeutic drug of a different type. For example, two different proteins, polypeptides or peptides having immunomodulatory activity may be fused together to form a fusion protein. In some cases, the fusion protein may retain or increase the activity compared to the activity of the original polypeptide or protein before fusion of the heterologous protein, polypeptide or protein.

In the present invention, the term "killing ability" generally refers to killing target cells by contacting the cells of the present invention with an effective amount of a substance. In one embodiment, the substance of the present invention may be a TIL cell. The killing of the present invention may include killing cells by itself or by promoting CDC, apoptosis, ADCC and/or phagocytosis of other cells or substances, or by a combination of two or more of these mechanisms.

In the present invention, the term "administering" or "administration" generally refers to delivering a substance to a subject in need thereof by any route known in the art. Pharmaceutical carriers and formulations or compositions are also well known in the art. Routes of administration may include: intravenous, intramuscular, intradermal, subcutaneous, transdermal, mucosal, intratumoral and/or mucosal.

In the present invention, the term "kit" generally refers to two or more components packaged together in a container, a receptacle or other container, one of which corresponds to the substance of the present invention. For example, it contains the TIL cells of the present invention.

In the present invention, the term "subject" generally refers to a cell or an animal, which may be a mammal, such as a human, a non-human primate (ape, gibbon, gorilla, chimpanzee, orangutan, macaque), livestock (dogs and cats), farm animals (poultry such as chickens and ducks, horses, cattle, goats, sheep, pigs) and experimental animals (mice, rats, rabbits, guinea pigs). Human subjects include fetuses, newborns, infants, adolescents and adult subjects. Subjects include animal disease models, such as tumor animal models, and other animal models known to those skilled in the art.

In the present invention, the term "feeder cell" generally refers to a cultured cell that can be used to support the growth of another target cell. For example, it can be grown in vitro and secrete at least one factor into the culture medium. In one embodiment, feeder cells can include an antigen presenting cell.

In the present invention, the term "specific binding" generally refers to a binding substance that recognizes a specific target substance, but does not substantially recognize or bind to other molecules in the sample. For example, if a binding substance can specifically bind to a specific target substance of the present invention from one species, the binding substance of the present invention can also specifically bind to a target substance of the present invention or a homologous target substance from one or more other species. This interspecies reactivity itself may not change the classification of the binding substance as specific. In some cases, a binding substance that specifically binds to a target substance can also bind to different allelic forms of the target substance.

In the present invention, the term "complete culture process" generally refers to the complete process starting from isolating cells from tumor tissue isolated from a patient, undergoing one or more expansions, and finally obtaining cells that can be administered to a subject.

In the present invention, the term "cell culture medium" generally refers to a nutrient solution in which cells, such as mammalian cells, are grown. The preparation of cell culture media is well known in the art. Typically, cell culture media include buffers, salts, carbohydrates, amino acids, vitamins and necessary trace elements. Cell culture media may or may not contain serum, peptone and/or protein. Cell culture media may be supplemented with additional components or components in increased concentrations, such as amino acids, salts, sugars, vitamins, hormones, growth factors, buffers, antibiotics, lipids, trace elements, etc., depending on the requirements of the cells to be cultured and/or the desired cell culture parameters.

In the present invention, the term "pharmaceutical composition" or "pharmaceutical preparation" generally refers to a preparation that allows the biological activity of the active ingredient to be effective and may contain no additional components that are unacceptably toxic to the subject to whom the preparation will be administered. This type of formulation is sterile. "Pharmaceutically acceptable" excipients (carriers, additives) are those excipients that can reasonably be administered to a subject mammal to provide an effective dose of the active ingredient employed.

In the present invention, the term "tumor infiltrating lymphocytes" or "TIL" generally refers to a population of cells originally obtained as leukocytes that have left the bloodstream of a subject and migrated into a tumor. TIL may include, but is not limited to, CD8⁺ cytotoxic T cells (lymphocytes), Th1 and Th17 CD4⁺ T cells, natural killer cells, dendritic cells and M1 macrophages. TIL can include primary TIL and secondary TIL. "Primary TIL" can be those TIL cells obtained from a subject's tissue sample, and "secondary TIL" can be any TIL group that has been expanded in the present invention. In some embodiments, the tumor infiltrating lymphocytes of the present invention may be not isolated or purified, or may be mutually infiltrated with tumor cells. In one embodiment, the TIL of the present invention may refer to a TIL population.

In the present invention, the term "central memory T cells" generally refers to T cells with long-term memory and capable of accepting antigen restimulation. Central memory T cells may have a CD45RO⁺ CD62L⁺ phenotype. For example, central memory T cells can be identified by CD45RO⁺ and CD62L⁺. Central memory T cells can have stronger anti-tumor growth ability than ordinary T cells.

In the present invention, the term "regulatory T cells" generally refers to a type of T cell subpopulation that controls autoimmune reactivity in the body. Regulatory T cells may have a CD4⁺ CD25⁺ Foxp3⁺ phenotype. For example, regulatory T cells can be identified by CD4⁺, CD25⁺ and Foxp3⁺. Regulatory T cells can have the ability to suppress the anti-tumor growth ability of T cells.

In the present invention, the term "activated T cells" generally refers to T cells that have been activated to have the ability to resist tumor growth. Activated T cells may have a PD-1⁺ (PD1⁺), LAG-3⁺ (LAG3⁺) or CD28⁺ phenotype. For example, activated T cells can be identified by PD-1⁺, LAG-3⁺ or CD28⁺. Activated T cells can have the ability to resist tumor growth.

In the present invention, the term "tumor-specific T cells" generally refers to T cells that can specifically fight tumor growth. Tumor-specific T cells may have a CD103⁺ CD39⁺ phenotype. For example, tumor-specific T cells can be determined by CD103⁺ and CD39⁺. Tumor-specific T cells can have more specific anti-tumor growth capabilities than ordinary T cells.

In the present invention, the term "stem cell-like T cells" generally refers to a type of T cells that can have the potential for self-proliferation and/or differentiation. For example, in the present invention, cells with differentiation potential and/or sustained proliferation ability can be considered as stem cell-like cells. For example, naive T cells (CD45RO⁻ CD62L⁺) can be considered as stem cell-like cells. For example, naive T cells can have a CD45RO⁻ CD62L⁺ phenotype. For example, stem cell-like T cells can be identified through CD45RO⁻ CD62L⁺. For example, stem cell-like T cells can be identified through CD39⁻ CD69⁻. For example, stem-like T cells can have a TCF1⁺ phenotype. For example, stem cell-like T cells can be identified through TCF1⁺. Stem cell-like T cells may have stronger and/or longer-lasting anti-tumor growth capabilities than ordinary T cells.

In the present invention, the term tumor "fragments" generally refers to tumor fragments formed by mechanical disruption, enzymatic hydrolysis and/or other disruption methods after tumor tissue is removed from a subject.

In the present invention, the term "composition" or "pharmaceutical composition" generally refers to a mixture of at least one cell and at least one and optionally more than one other pharmaceutically acceptable chemical components such as carriers, stabilizers, diluents, dispersants, suspending agents, thickeners and/or excipients.

In the present invention, the term "pharmaceutically acceptable carrier" generally refers to one or more non-toxic materials that do not interfere with the active ingredients. For example, a pharmaceutically acceptable carrier may not interfere with the biological activity of the active ingredient; for example, a pharmaceutically acceptable carrier may not interfere with the effectiveness of the biological activity possessed by the active ingredient. Such preparations may conventionally contain salts, buffers, preservatives, compatible carriers, and optionally other therapeutic agents. Such pharmaceutically acceptable preparations may also contain compatible solid or liquid fillers, diluents or encapsulating materials suitable for administration to humans. Other contemplated carriers, excipients and/or additives that may be used in the preparations described herein may include, for example, flavoring agents, antimicrobial agents, sweeteners, antioxidants, antistatic agents, lipids, protein excipients (such as serum albumin, gelatin, casein), salt-forming counterions (such as sodium), etc. These and other known pharmaceutical carriers, excipients and/or additives suitable for use in the formulations described herein are known in the art. In the present invention, "pharmaceutically acceptable carriers" can be understood as not including vectors of nucleic acid forms used in genetic engineering.

In the present invention, the term "functionally active fragment" generally refers to a fragment having a partial region of a full-length protein or nucleic acid, but retaining or partially retaining the biological activity or function of the full-length protein or nucleic acid. For example, a functionally active fragment can retain or partially retain the ability of the full-length protein to bind to another molecule.

In the present invention, the term "T cell activator" generally refers to a substance that binds to a corresponding binding receptor on a T cell and mediates a T cell co-stimulatory response. A T cell activator may be a substance other than an antigen receptor required for a T cell to produce an effective immune response. A T cell activator may refer to a T cell co-stimulatory molecule. For example, the T cell activator of the present invention may include any substance comprising a variant, homolog or functionally active fragment thereof. T cell activators may include but are not limited to MHC Class I molecules, TNF receptor protein, immunoglobulin-like protein, cytokine receptor, integrin, signal lymphocyte activation molecule (SLAM protein), NK cell activation receptor, BTLA (the GeneID of the gene encoding it may be 151888), Toll ligand receptor, OX40 (the GeneID of the gene encoding it may be 7293), CD2 (the GeneID of the gene encoding it may be 914), CD7 (the GeneID of the gene encoding it may be 924), CD27 (the GeneID of the gene encoding it may be 939), CD28 (the GeneID of the gene encoding it may be 940), CD30 (the GeneID of the gene encoding it may be 943), CD40 (the GeneID of the gene encoding it may be 958), CDS, ICAM -1 (the GeneID of the gene encoding it may be 3383), LFA-1 (CD11a/CD18) (the GeneID of the gene encoding it may be 3689), 4-1BB (CD137) (the GeneID of the gene encoding it may be 3604), B7-H3 (the GeneID of the gene encoding it may be 80381), ICOS (CD278) (the GeneID of the gene encoding it may be 29851), GITR (the GeneID of the gene encoding it may be 8784), BAFFR (the GeneID of the gene encoding it may be 115650), LIGHT (the GeneID of the gene encoding it may be 8740), HVEM (LIGHTR) (the GeneID of the gene encoding it may be 8764), KIRDS2 (the GeneID of the gene encoding it may be 100132285), SLAMF7 (the GeneID of the gene encoding it may be 57823), NKp80 (KLRF1) (the GeneID of the gene encoding it may be 51348), NKp44 (the GeneID of the gene encoding it may be 9436), NKp30 (the GeneID of the gene encoding it may be 259197), NKp46 (the GeneID of the gene encoding it may be 9437), CD19 (the GeneID of the gene encoding it may be 930), CD4 (the GeneID of the gene encoding it may be 920), CD8α (the GeneID of the gene encoding it may be 925), CD8β (the GeneID of the gene encoding it can be 926), IL-2Rβ, IL-2Rγ, IL7Rα (the GeneID of the gene encoding it can be 3575), ITGA4 (the GeneID of the gene encoding it can be 3676), VLA1 (the GeneID of the gene encoding it can be 3672), CD49a (the GeneID of the gene encoding it can be 3672), IA4 (the GeneID of the gene encoding it can be 3732), CD49D (the GeneID of the gene encoding it can be 3676), ITGA6 (the GeneID of the gene encoding it can be 3655), VLA-6 (the GeneID of the gene encoding it can be 3655), CD49f (the GeneID of the gene encoding it may be 3655), ITGAD (the GeneID of the gene encoding it may be 3681), CD11d (the GeneID of the gene encoding it may be 3681), ITGAE (the GeneID of the gene encoding it may be 3682), CD103 (the GeneID of the gene encoding it may be 3682), ITGAL (the GeneID of the gene encoding it may be 3683), CD11a (the GeneID of the gene encoding it may be 3683), LFA-1 (the GeneID of the gene encoding it may be 3683), ITGAM (the GeneID of the gene encoding it may be 3684), CD11b (the GeneID of the gene encoding it may be 3684), ITGAX (the GeneID of the gene encoding it may be 3687), CD11c (the GeneID of the gene encoding it may be 3687), ITGB1 (the GeneID of the gene encoding it may be 3688), CD29 (the GeneID of the gene encoding it may be 3688), ITGB2 (the GeneID of the gene encoding it may be 3689), CD18 (the GeneID of the gene encoding it may be 3689), LFA-1 (the GeneID of the gene encoding it may be 3689), ITGB7 (the GeneID of the gene encoding it may be 3695),NKG2D (the GeneID of the gene encoding it may be 22914), NKG2C (the GeneID of the gene encoding it may be 3822), TNFR2 (the GeneID of the gene encoding it may be 7133), TRANCE/RANKL (the GeneID of the gene encoding it may be 8600), DNAM1 (CD226) (the GeneID of the gene encoding it may be 10666), SLAMF4 (CD244, 2B4) (the GeneID of the gene encoding it may be 51744), CD84 (the GeneID of the gene encoding it may be 8832), CD96 (Tactile) (the GeneID of the gene encoding it may be 10225), CEACAM1 (the GeneID of the gene encoding it may be 634), CRTAM (the GeneID of the gene encoding it may be 56253), Ly9 (CD229) (the GeneID of the gene encoding it may be 4063), CD160 (BY55) (the GeneID of the gene encoding it may be 11126), PSGL1 (the GeneID of the gene encoding it may be 6404), CD100 (SEMA4D) (the GeneID of the gene encoding it may be 10507), CD69 (the GeneID of the gene encoding it may be 969), SLAMF6 (NTB-A, Ly108) (the GeneID of the gene encoding it may be 114836), SLAM (SLAMF1, CD150, IPO-3) (the GeneID of the gene encoding it may be 65 04), BLAME (SLAMF8) (the GeneID of the gene encoding it can be 56833), SELPLG (CD162) (the GeneID of the gene encoding it can be 6404), LTBR (the GeneID of the gene encoding it can be 4055), LAT (the GeneID of the gene encoding it can be 27040), GADS (the GeneID of the gene encoding it can be 9402), SLP-76 (the GeneID of the gene encoding it can be 3937), PAG/Cbp (the GeneID of the gene encoding it can be 55824), CD19a, a ligand that specifically binds to CD3, a ligand that specifically binds to CD28, a ligand that specifically binds to HVEM, a ligand that specifically binds to CD40L, a ligand that specifically binds to OX40, and a ligand that specifically binds to 4-1BB. The co-stimulatory intracellular signaling domain may refer to the intracellular portion of a T cell activator. The intracellular signaling domain may contain a complete intracellular portion of a molecule derived therefrom or a complete native intracellular signaling domain or a functional fragment thereof.

In the present invention, the term "T cell growth factor" generally refers to a biologically active polypeptide or small molecule compound that causes cell proliferation. For example, the T cell growth factors of the present invention may include any substance including its variants, homologues or functionally active fragments thereof. In one embodiment, the T cell growth factors can be selected from one or more of the following group: IL-2 (the gene encoding it may be 3558 in GeneID), IL-4 (the gene encoding it may be 3565 in GeneID), IL-6 (the gene encoding it may be 3569 in GeneID), IL-7 (the gene encoding it may be 3574 in GeneID), IL-10 (the gene encoding it may be 3586 in GeneID), IL-12 (the gene encoding it may be 3592 or 3593 in GeneID), IL-15 (the gene encoding it may be 3600 in GeneID), IL-21 (the gene encoding it may be 59067 in GeneID), TNF-α (the gene encoding it may be 100137091 in GeneID), interferon-γ (the gene encoding it may be 3458 in GeneID), GZMB (the gene encoding it may be 3002 in GeneID), CD107a (the gene ID of the gene can be 6499 in GeneID) and so on.

In the present invention, the term "substantially simultaneously" generally refers to that during a period of time during the contact process, TIL can be in contact with two or more substances at the same time, but may not be limited to TIL always being in contact with two or more substances at the same time during the entire contact process. In one embodiment, substantially simultaneously may mean that the TIL can be in contact with at least 10-95%, such as at least 10%, 20%, 30%, 40%, 50%, 60%, 70%, 75%, 80%, 85%, 90%, 95% of each of the two or more substances at the same time during a period of time.

In the present invention, the term "dendritic cell" generally refers to an antigen presenting cell present in vivo, in vitro, ex vivo or in a host or subject or derived from a hematopoietic stem cell or a monocyte. Dendritic cells and their precursors can be separated from various lymphoid organs such as spleen, lymph nodes, bone marrow and peripheral blood. The dendritic cells of the present invention can have characteristic morphology, such as thin layers (lamellipodia) extending in multiple directions of the dendritic cell body. Typically, dendritic cells can express high levels of MHC and costimulatory (such as B7-1 and B7-2) molecules. Dendritic cells can induce antigen-specific differentiation of T cells in vitro, and can trigger primary T cell responses in vitro and in vivo.

In the present invention, the term "in vitro expansion" generally refers to culturing to produce a change in the number of cells. The expanded cells may also produce changes in the number and/or proportion of cells, changes in secretion capacity, changes in killing capacity or changes in expression capacity, or any combination thereof. The changes of the present invention may be an increase or a decrease. In the present invention, in vitro expansion may be for the purpose of expansion; the operation steps performed on TIL cells in order to detect the function of TIL cells, such as detecting the ability of TIL cells to release cytokines (such as adding one or more substances to the culture medium of TIL cells to detect the ability of TIL cells to release cytokines) may not be encompassed in the in vitro expansion of the present invention.

In the present invention, the term "peripheral mononuclear cell" or "peripheral blood mononuclear cell" generally refers to a cell having a single nucleus in peripheral blood. For example, in the present invention, the peripheral blood mononuclear cells of the present invention may include lymphocytes, monocytes and/or dendritic cells.

In the present invention, the term "cytokine" generally refers to a protein released by a cell population that acts as an intercellular regulator on another cell. The cytokines of the present invention can be a lymphokine, a monocytokine, and a polypeptide hormone. The cytokines of the present invention can include interleukins (ILs) such as IL-1, IL-1α, IL-2, IL-3, IL-4, IL-5, IL-6, IL-7, IL-8, IL-9, IL-10, IL-11, IL-15, IL-21 and/or IL-12. In the present invention, the term cytokine can include proteins from natural sources or from recombinant cell culture, biologically active equivalents of native sequence cytokines, and functionally active fragments thereof.

In the present invention, the term "diameter" generally refers to the diameter of the cross section of the substance of the present invention. For example, when the substance of the present invention is not spherical, the term "diameter" generally refers to the maximum diameter and/or average diameter of the largest cross section of the substance of the present invention. The method for determining the diameter of the substance can be a method commonly used in the art, such as transmission electron microscopy.

In the present invention, the term "tumor" generally refers to any new pathological tissue proliferation. The tumor of the present invention may be benign or malignant. The tumor of the present invention may be solid or blood. The term "tumor" may be selected from one or more from the following group: melanoma, ovarian cancer, cervical cancer, lung cancer, bladder cancer, breast cancer, head and neck cancer, pancreatic cancer, liver cancer, gastric cancer, colorectal cancer and kidney cancer.

In the present invention, the term "tumor tissue" generally refers to a sample from a tumor in a subject, including any solid tumor and/or any tissue of a non-solid tumor in the subject.

In the present invention, the term "T cell subset ratio" generally refers to the ratio of different T cell subsets in TIL cells or TIL populations. For example, different T cell subsets of the present invention have different immune activities and/or differentiation abilities. For example, the T cell subsets of the present invention can be distinguished based on T cell surface markers. For example, central memory T cells can have a CD45RO⁺ CD62L⁺ phenotype. For example, naive T cells may have a CD45RO⁻ CD62L⁺ phenotype. For example, regulatory T cells may have a CD4⁺ CD25⁺ Foxp3⁺ phenotype. For example, activated T cells may have a CD25⁺, CD28⁺, PD-1⁺ or 41BB⁺ phenotype. For example, tumor-specific T cells may have a CD103⁺ CD39⁺ phenotype. For example, stem-like T cells can have a TCF1⁺ phenotype.

In the present invention, the term "TIL cell number" generally refers to the number of cells in the TIL cells of the present invention. In the present invention, the number of TIL cells may refer to the number of cells in the TIL population obtained at any stage of the present invention. For example, the number of TIL cells may refer to the number of cells of a first TIL population derived from a tumor tissue and not expanded in vitro. For example, the number of TIL cells may refer to the number of cells of a second TIL population expanded in vitro in the first stage. For example, the number of TIL cells may refer to the number of cells of a third TIL population expanded in vitro in the second stage. For example, the number of TIL cells may refer to the cells of the TIL finally obtained by any one of the culture methods of the present invention. In the present invention, the number of TIL cells can be measured by methods commonly used in the art, for example, including but not limited to manual cell counting with a cell counting plate and/or counting with an automatic cell counter.

In the present invention, the terms "about" and "approximately" generally refer to a statistically meaningful numerical range. Such a range can be within an order of magnitude of a given value or range, can include within 50%, preferably include within 20%, more preferably include within 10%, and most preferably include within 5%. The permissible variation encompassed in the term "about" or "approximately" may depend on the specific system under study, and can be easily understood by those of ordinary skill in the art.

In the present invention, the terms "above", "below", "at most" and "at least" include the present number.

### DETAILED DESCRIPTION OF THE INVENTION

### RASA2, FIBP, MED12, TIGIT, BRD4 Knockout

1. A method for culturing cells, the method comprising: reducing the expression and/or attenuating the activity of at least one family member selected from the group consisting of GTPase activating protein 1, FGF binding protein, Mediator (MED), PVR, BET family and/or its functionally active fragments in the cells.
2. The method of embodiment 1, wherein the cells comprise immune cells.
3. The method according to embodiment 2, wherein the immune cells comprise phagocytes, lymphocytes, neutrophils, eosinophils and/or basophils.
4. A method according to any one of embodiments 2-3, wherein the immune cells comprise monocytes, macrophages and/or dendritic cells.
5. A method according to any one of embodiments 2-4, wherein the immune cells are derived from immune cells differentiated from stem cells.
6. A method according to embodiment 5, wherein the stem cells comprise induced pluripotent stem cells (iPSCs), embryonic stem cells, bone marrow stem cells, umbilical cord blood stem cells and/or peripheral blood stem cells.
7. A method according to any one of embodiments 2-6, wherein the immune cells comprise B cells, T cells, natural killer cells and/or natural killer-like T cells (NKT).
8. A method according to any one of embodiments 2-7, wherein the immune cells comprise αβ T cells and/or γδ T cells.
9. A method according to any one of embodiments 2-8, wherein the immune cells comprise tumor infiltrating lymphocytes (TILs).
10. A method according to embodiment 9, wherein the TIL is TIL derived from tumor tissue, tumor-associated lymph nodes with or without tumor metastasis, tumor metastatic lesions, fragments of peritumoral tissue, pleural effusion and/or peritoneal effusion and/or TIL derived from recovery after cryopreservation.
11. The method of embodiment 10, wherein the volume of the fragments is from about 1 cubic millimeter to about 27 cubic millimeters.
12. The method according to any one of embodiments 2-11, wherein the immune cell contains an engineered immune receptor displayed on the surface of a cell.
13. A method according to embodiment 12, wherein the engineered immune receptor specifically binds to an antigen expressed on a target cell.
14. A method according to any one of embodiments 2-13, wherein the immune cell comprises a chimeric antigen receptor and/or a T cell receptor.
15. A method according to any one of embodiments 1-14, wherein the reduced expression and/or weakened activity of at least one family member selected from the group consisting of GTPase activating protein 1, FGF binding protein, Mediator (MED), PVR, BET family and/or their functionally active fragments comprises an effect selected from the following group: inhibition of GTPase, inhibition of binding to FGF, inhibition of CDK8 activation, inhibition of PVR binding, and inhibition of chromatin targeting function.
16. A method according to any one of embodiments 1-15, wherein the cells obtained by reducing the expression and/or attenuating the activity of at least one family member selected from GTPase activating protein 1, FGF binding protein, Mediator (MED), PVR, BET family, and/or its functionally active fragments show improved cell characteristics compared to cells in which the expression of at least one family member selected from GTPase activating protein 1, FGF binding protein, Mediator (MED), PVR, BET family, and/or its functionally active fragments is reduced and/or the activity is not changed.
17. A method according to embodiment 16, wherein the improved cell characteristics include one or more selected from the following groups: improved cell proliferation ability, increased proportion of live cells, improved proportion of cell subpopulations, increased cytokine secretion ability, improved in vitro tumor cell killing ability and improved in vivo tumor killing ability.
18. A method according to embodiment 17, wherein the improved proportion of cell subpopulations comprises one or more selected from the following group: an increased proportion of activated cells, a decreased proportion of regulatory cells, a decreased proportion of exhausted cells, an increased proportion of central memory cells and/or naive cells, a decreased proportion of apoptotic cells and an increased proportion of stem-like cells.
19. A method according to any one of embodiments 1-18, wherein the family members selected from GTPase activating protein 1, FGF binding protein, Mediator (MED), PVR, and BET family respectively contain a GTPase activating domain, an FGF binding domain, a CDK8 binding domain, a PVR binding domain, and a bromodomain.
20. A method according to any one of embodiments 1-19, wherein the family members selected from GTPase activating protein 1, FGF binding protein, Mediator (MED), PVR, and BET family respectively include RASA2, FIBP, MED12, TIGIT, and BRD4.
21. A method according to any one of embodiments 1-20, wherein reducing the expression and/or weakening the activity of a family member selected from GTPase activating protein 1, FGF binding protein, Mediator (MED), PVR, and BET family in the cell comprises introducing a gene regulatory system into the cell.
22. A method according to embodiment 21, wherein the gene regulatory system disrupts the family members selected from GTPase activating protein 1, FGF binding protein, Mediator (MED), PVR, and BET family at the DNA level; and optionally, in the cells the expression and/or activity of that selected from TNFAIP3, SOCS1, ZC3H12A, CBLB, FAS, IKZF1, LAG3, PD1, TIM3, ADNP, NFKBIA, PTPN6, BCL2L11, PTPN2, AFF3, AXL, NFE2L1, RARG, UBFD1, CRP, CYLD, GIF, KLF4, NDST1, NLRP1, SCGB1A1, ADCY7, ARIH2, CPT2, LNPEP, NOSIP, NPRL3, TANK, TRAF3, TSC1, ZBTB7B, ZC3H12D, RC3H2, and TNIP1 is reduced.
23. A method according to any one of embodiments 21-22, wherein the gene regulatory system comprises a guiding nucleic acid molecule and an enzyme protein.
24. A method according to embodiment 23, wherein reducing the expression and/or weakening the activity of a family member selected from GTPase activating protein 1, FGF binding protein, Mediator (MED), PVR, and BET family in the cell comprises: introducing into the cell a complex comprising the guide nucleic acid molecule and the enzyme protein, or a complex comprising the guide nucleic acid molecule and a nucleic acid encoding the enzyme protein, an LNP comprising a gRNA and a Cas protein, or an LNP comprising a nucleic acid encoding a gRNA and a Cas protein.
25. A method according to any one of embodiments 23-24, wherein the enzyme protein comprises a Cas protein, a Cas protein homolog, or a functionally active fragment thereof, preferably selected from Cas 9 and Cas 12.
26. A method according to any one of embodiments 23-25, wherein the guide nucleic acid molecule comprises a guide RNA (gRNA).
27. A method according to any one of embodiments 23-26, wherein the guiding nucleic acid molecule binds to the sequence of at least one family member selected from the group consisting of GTPase activating protein 1, FGF binding protein, Mediator (MED), PVR, and BET family.
28. A method according to any one of embodiments 23-27, wherein the guide nucleic acid molecule binds to a sequence consisting of about 15 to about 25 nucleotides upstream of the 5' end of the protospacer adjacent motif (PAM) selected from the following group: AGG, TGG, CGG and GGG, or binds to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, and NTN, wherein N is A, T, C or G, Y is T or C, V is A, C or G, and R is A or G.
29. A method according to any one of embodiments 23-28, wherein the guide nucleic acid molecule binds to a region or a fragment thereof defined by the genomic coordinates shown in Tables 1A-1E or Tables 2A-2E.
30. The method of any one of embodiments 23-29, wherein the guide nucleic acid molecule binds to at least one region or a fragment thereof selected from the group consisting of: SEQ ID NO: 1093-2184 (RASA2), SEQ ID NO: 2732-3278 (FIBP), SEQ ID NO: 4855-6430 (MED12), SEQ ID NO: 6988-7544 (TIGIT), SEQ ID NO: 10190-12834 (BRD4).
31. The method of any one of embodiments 23-30, wherein the guide nucleic acid molecule comprises a targeting domain comprising a sequence as shown in at least one of SEQ ID NOs: 1-1092, 29247-29248, 29292-29314 (RASA2), SEQ ID NOs: 2185-2731, 29249-29250 (FIBP), SEQ ID NOs: 3279-4854, 29251-29252 (MED12), SEQ ID NOs: 6431-6987, 29253-29254 (TIGIT), SEQ ID NOs: 7545-10189, 29255-29256 (BRD4).
32. A method according to any one of embodiments 1-31, wherein the proportion of cells expressing the target gene in the obtained cells is reduced and/or the expression level of the target gene in individual cells is decreased compared to cells in which the expression and/or activity of the family members selected from GTPase activating protein 1, FGF binding protein, Mediator (MED), PVR, and BET family are unchanged.
33. A method according to any one of embodiments 1-32, wherein the proportion of cells expressing the target gene in the cells obtained by reducing the expression and/or weakening the activity of the family members selected from GTPase activating protein 1, FGF binding protein, Mediator (MED), PVR, and BET family is less than about 95%.
34. A cell obtained by the method of any one of embodiments 1-33.
35. A pharmaceutical composition comprising the cell of embodiment 34, and optionally a pharmaceutically acceptable carrier.
36. A method of influencing cell growth, comprising administering the cell of embodiment 34 and/or the pharmaceutical composition described in embodiment 35.
37. Use of the cells described in embodiment 34 and/or the pharmaceutical composition described in embodiment 35 in the preparation of a drug for preventing and/or treating a disease and/or symptom.
38. A medicament for preventing and/or treating a disease and/or symptom, comprising the cell of embodiment 34 and/or the pharmaceutical composition of embodiment 35 as an active ingredient.
39. A method for preventing and/or treating a disease and/or a symptom, comprising administering the cell of embodiment 34 and/or the pharmaceutical composition of embodiment 35 to a subject in need.
40. The cell described in embodiment 34 and/or the pharmaceutical composition described in embodiment 35, which is used for preventing and/or treating diseases and/or symptoms.
41. The use according to embodiment 37, the medicament according to embodiment 38, the method according to embodiment 39, and/or the cell and/or the pharmaceutical composition for use according to embodiment 40, wherein the disease and/or symptom comprises a tumor.
42. The use according to embodiment 37, the medicament according to embodiment 38, the method according to embodiment 39, and/or the cell and/or the pharmaceutical composition for use according to embodiment 40, wherein the disease and/or condition comprises a solid tumor.
43. The use according to embodiment 37, the medicament according to embodiment 38, the method according to embodiment 39, and/or the cell and/or the pharmaceutical composition for use according to embodiment 40, wherein the disease and/or symptom comprises one or more selected from the following group: melanoma, ovarian cancer, cervical cancer, lung cancer, bladder cancer, breast cancer, head and neck cancer, pancreatic cancer, liver cancer, gastric cancer, colorectal cancer and kidney cancer.

The present invention provides a method for reducing the expression and/or attenuating the activity of at least one family member selected from the group consisting of GTPase activating protein 1, FGF binding protein, Mediator (MED), PVR, BET family and/or its functionally active fragments in the cells.

In one aspect, the present invention provides a method for culturing cells, comprising reducing the expression and/or weakening the activity of at least one family member selected from GTPase activating protein 1, FGF binding protein, Mediator (MED), PVR, BET family and/or its functionally active fragments in the cells.

For example, the cell can further comprise reduced expression and/or reduced activity of a gene, optionally selected from the group consisting of TNFAIP3, SOCS1, ZC3H12A, CBLB, FAS, IKZF1, LAG3, PD1, TIM3, ADNP, NFKBIA, PTPN6, BCL2L11, PTPN2, AFF3, AXL, NFE2L1, RARG, UBFD1, CRP, CYLD, GIF, KLF4, NDST1, NLRP1, SCGB1A1, ADCY7, ARIH2, CPT2, LNPEP, NOSIP, NPRL3, TANK, TRAF3, TSC1, ZBTB7B, ZC3H12D, RC3H2, and TNIP1.

For example, the GTPase activating protein 1 family member may comprise a GTPase activating domain. For example, the GTPase activating protein 1 family member may comprise RASA2.

For example, the FGF binding protein family member may comprise an FGF binding domain. For example, the FGF binding protein family member may comprise FIBP.

For example, the Mediator (MED) family member may comprise a CDK8 binding domain. For example, the Mediator (MED) family member may comprise MED12.

For example, the PVR family member may comprise a PVR binding domain. For example, the PVR family member may comprise TIGIT.

For example, the BET family member may comprise a bromodomain. For example, the BET family member may comprise BRD4.

For example, the target gene of the present invention can be a gene encoding a protein selected from the group consisting of GTPase activating protein 1, FGF binding protein, Mediator (MED), PVR, BET family and/or functionally active fragments thereof. For example, compared with cells in which the expression and/or activity of the target gene are not changed, the cells obtained by reducing the expression and/or weakening the activity of at least one target gene of the cell can show improved cell characteristics. In one embodiment, the cells in which the expression and/or activity of the target gene are not changed may refer to cells that are derived from the same donor and that have not had the expression and/or the activity of at least one target gene of the cell reduced. In one embodiment, the cells in which the expression and/or activity of the target gene are not changed may refer to cells that are derived from the same donor and that have not had the expression and/or the activity of other genes other than the target gene of the cell reduced (e.g., knocking out the other gene has substantially no effect on cell function) .

In one embodiment, the corresponding cells that have not had the expression and/or the activity of at least one target gene of the cell reduced may refer to cells that are isolated in the same manner from the same donor and that have not had the expression and/or the activity of at least one target gene of the cell reduced. In one embodiment, the corresponding cells that have not had the expression and/or the activity of at least one target gene of the cell reduced may refer to cells that are from the same tumor source of the same donor and that have not had the expression and/or the activity of at least one target gene of the cell reduced. In one embodiment, the corresponding cells that have not had the expression and/or the activity of at least one target gene of the cell reduced may refer to dividing cells from the same tumor source of the same donor into two groups, wherein one group of cells that have not had the expression and/or the activity of at least one target gene of the cell reduced may be the corresponding cells that have not had the expression and/or the activity of at least one target gene of the cell reduced. For example, the reduced expression and/or weakened activity of at least one target gene may mean that the target gene in a natural cell is in an expression state to a certain extent, and after the treatment of the present invention, the expression level of the target gene in the cell can be reduced, that is, the reduced expression level of the target gene can be such that the natural cell changes from expressing the target gene to basically not expressing the target gene or the amount of expression of the target gene is reduced.

For example, the cells include immune cells. For example, the cells include immune effector cells. For example, the cells include immune effector T cells, immune effector NK cells, immune effector NKT cells. For example, the cells include phagocytes, lymphocytes, neutrophils, eosinophils and/or basophils.

For example, the cells comprise monocytes, macrophages and/or dendritic cells.

For example, the cells of the present invention also include cells derived from stem cell differentiation. For example, the cells of the present invention also include cells derived from pluripotent stem cell differentiation. For example, obtaining the stem cells of the present invention can be produced by induction. For example, the above-mentioned stem cells of the present invention can include induced pluripotent stem cells (iPSC), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from reproductive tissue of a fetus. Exemplary pluripotent stem cells can also be generated from somatic cells by reprogramming them to a pluripotent state through the expression of certain transcription factors associated with pluripotency; these cells are referred to as "induced pluripotent stem cells" or "iPSCs."

For example, the cell comprises B cells, T cells, natural killer cells and/or natural killer-like T cells (NKT). For example, "unmodified cells" or "unmodified cells" may refer to cells or cell colonies in which the genome is not modified and does not comprise a gene regulatory system or comprises a control gene regulatory system (e.g., an empty vector control, a non-targeted gRNA, an interfering siRNA, etc.). For example, the cell comprises αβ T cells and/or γδ T cells. For example, the cell comprises tumor infiltrating lymphocytes (TIL). For example, the TIL is TIL derived from tumor tissue, tumor-associated lymph nodes with or without tumor metastasis, tumor metastasis lesions, fragments of para cancerous tissue, pleural effusion and/or peritoneal effusion and/or TIL recovered after cryopreservation.

For example, the TILs of the present invention may be TILs derived from tumor tissue, tumor-associated lymph nodes with or without tumor metastasis, tumor metastasis lesions, fragments of paracancerous tissue, pleural effusion and/or peritoneal effusion and/or TILs recovered after cryopreservation. For example, TIL of the present invention can be obtained by processing tumor tissue into tumor fragments. For example, the volume of tumor fragments of the present invention is about 1-27 cubic millimeters. For example, the volume of tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cell comprises an engineered immune receptor displayed on the cell surface. For example, the engineered immune receptor specifically binds to an antigen expressed on a target cell. For example, the cell comprises a chimeric antigen receptor and/or a T cell receptor.

In one aspect, the present invention provides a method for culturing tumor infiltrating lymphocytes (TIL), which may include: reducing the expression and/or activity of at least one family member selected from the group consisting of GTPase activating protein 1, FGF binding protein, Mediator (MED), PVR, and BET family and/or its functionally active fragments in the TIL.

For example, TILs derived from tumor tissue, tumor-associated lymph nodes with or without tumor metastasis, tumor metastatic lesions, fragments of paracancerous tissue, pleural effusion and/or peritoneal effusion and not expanded in vitro can be subjected to at least one stage of in vitro expansion, wherein, in at least one stage of the in vitro expansion, the expression and/or activity of at least one family member selected from the group consisting of GTPase activating protein 1, FGF binding protein, Mediator (MED), PVR, and BET family and/or its functionally active fragments can be reduced in the TILs.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion and a second stage of in vitro expansion, and in the second stage of in vitro expansion of the present invention, the expression and/or activity of at least one family member selected from GTPase activating protein 1, FGF binding protein, Mediator (MED), PVR, BET family and/or its functionally active fragments in the TIL can be reduced. For example, the TIL derived from tumor tissue, tumor-associated lymph nodes with or without tumor metastasis, tumor metastasis lesions, paracancerous tissue fragments, pleural effusion and/or peritoneal effusion of the present invention and not expanded in vitro can be subjected to a first stage of in vitro expansion and a second stage of in vitro expansion, and in the first stage of in vitro expansion of the present invention, the expression and/or activity of at least one family member selected from GTPase activating protein 1, FGF binding protein, Mediator (MED), PVR, BET family and/or its functionally active fragments in the TIL can be reduced.

For example, the TILs of the present invention that are derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion and a second stage of in vitro expansion, and in the first stage of in vitro expansion of the present invention, the expression and/or activity of at least one family member selected from GTPase activating protein 1, FGF binding protein, Mediator (MED), PVR, and BET family and/or its functionally active fragments in the TILs can be reduced, and in the second stage of in vitro expansion of the present invention, the expression and/or activity of at least one family member selected from GTPase activating protein 1, FGF binding protein, Mediator (MED), PVR, and BET family and/or its functionally active fragments can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the first stage of in vitro expansion of the present invention, the expression and/or activity of at least one family member selected from GTPase activating protein 1, FGF binding protein, Mediator (MED), PVR, BET family and/or its functionally active fragments in the TIL can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the second stage in vitro expansion of the present invention, the expression and/or activity of at least one family member selected from the group consisting of GTPase activating protein 1, FGF binding protein, Mediator (MED), PVR, and BET families in the TILs and/or its functionally active fragments can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the third stage of in vitro expansion of the present invention, the expression and/or activity of at least one family member selected from GTPase activating protein 1, FGF binding protein, Mediator (MED), PVR, BET family and/or its functionally active fragments in the TIL can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the first stage of in vitro expansion of the present invention, the expression and/or activity of at least one family member selected from GTPase activating protein 1, FGF binding protein, Mediator (MED), PVR, BET family and/or its functionally active fragments in the TILs can be reduced, and in the second stage of in vitro expansion of the present invention, the expression and/or activity of at least one family member selected from GTPase activating protein 1, FGF binding protein, Mediator (MED), PVR, BET family and/or its functionally active fragments can be reduced in the TILs.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the first stage of in vitro expansion of the present invention, the expression and/or activity of at least one family member selected from GTPase activating protein 1, FGF binding protein, Mediator (MED), PVR, BET family and/or its functionally active fragments in the TILs can be reduced, and in the third stage in vitro of expansion of the present invention, the expression and/or activity of at least one family member selected from GTPase activating protein 1, FGF binding protein, Mediator (MED), PVR, BET families and/or its functionally active fragments in the TIL can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not amplified in vitro can be subjected to a first stage of in vitro expansion, a second stage of in vitro expansion and a third stage of in vitro expansion, and in the second stage of in vitro expansion of the present invention, the expression and/or activity of at least one family member selected from GTPase activating protein 1, FGF binding protein, Mediator (MED), PVR, BET family and/or its functionally active fragments in the TILs can be reduced, and in the third stage of in vitro expansion of the present invention, the expression and/or activity of at least one family member selected from GTPase activating protein 1, FGF binding protein, Mediator (MED), PVR, BET family and/or its functionally active fragments can be reduced in the TILs.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the first stage of in vitro expansion of the present invention, the expression and/or activity of at least one family member selected from GTPase activating protein 1, FGF binding protein, Mediator (MED), PVR, BET family and/or its functionally active fragments in the TILs can be reduced, and in the second stage of in vitro expansion of the present invention, the expression and/or activity of at least one family member selected from GTPase activating protein 1, FGF binding protein, Mediator (MED), PVR, BET family and/or its functionally active fragments in the TILs can be reduced, and in the third stage of in vitro expansion of the present invention, the expression and/or activity of at least one family member selected from GTPase activating protein 1, FGF binding protein, Mediator (MED), PVR, BET family and/or its functionally active fragments in the TILs can be reduced.

For example, each stage of in vitro expansion can be divided by the change of TIL cell number, for example, when the number of TIL cells increases by at least about 1 time, it can be considered that TIL cells have entered the next stage of in vitro expansion. In some embodiments, when the number of TIL cells increases by at least about 1-1000 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, at least about 50 times, at least about 100 times, at least about 200 times, at least about 500 times, or at least about 1000 times, it can be considered that TIL cells have entered the next stage of in vitro expansion. For example, each stage of in vitro expansion can also be divided by the change of the conditions of TIL cell culture. For example, when cell activators and/or cell growth factors are added or supplemented to the cell culture medium, it can be considered that the TIL cells have entered the next stage of in vitro expansion. For example, when IL-2 is added or supplemented to the cell culture medium, it can be considered that the TIL cells have entered the next stage of in vitro expansion. For example, when one or more gene regulatory systems are added or supplemented to the cell culture medium, it can be considered that the TIL cells have entered the next stage of in vitro expansion. For example, when feeder cells are added or supplemented to the cell culture medium, it can be considered that the TIL cells have entered the next stage of in vitro expansion. For example, after the TIL cells are centrifuged and/or cell washed, it can be considered that the TIL cells have entered the next stage of in vitro expansion. For example, each stage can also be divided by the number of days of TIL cell culture. For example, after TIL cells are cultured in vitro for about 1-100 days, such as about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, the TIL cells can be considered to have entered the next stage of in vitro expansion.

For example, the reducing the expression and/or weakening the activity of a GTPase activating protein 1 family member in the cell comprises inhibiting the function of a GTPase.

For example, the reducing the expression and/or weakening the activity of a FGF binding protein family member comprises inhibiting the function of binding to FGF.

For example, the reducing the expression and/or weakening the activity of a Mediator (MED) family member in the cell comprises inhibiting the CDK8-activating function.

For example, the reducing the expression and/or weakening the activity of a PVR family member in the cell comprises inhibiting the function of inhibiting PVR binding.

For example, the reducing the expression and/or attenuating the activity of a BET family member in said cell comprises inhibiting the function of targeting chromatin.

For example, compared to cells in which the expression and/or activity of at least one family member selected from GTPase activating protein 1, FGF binding protein, Mediator (MED), PVR, and BET family is not changed, cells obtained by reducing the expression and/or attenuating the activity of at least one family member selected from GTPase activating protein 1, FGF binding protein, Mediator (MED), PVR, and BET family show improved cell characteristics.

For example, the improved cell properties include one or more selected from the following group: improved cell proliferation ability (i.e., cell number), increased proportion of live cells, improved cell subpopulation proportions, enhanced cytokine secretion ability, enhanced in vitro tumor cell killing ability, and enhanced in vivo tumor killing ability.

For example, the improved cell subpopulation ratio comprises one or more selected from the following group: an increased ratio of activated cells, a decreased ratio of regulatory cells, a decreased ratio of exhausted cells, an increased ratio of central memory cells and/or naive cells, a decreased ratio of apoptotic cells, and an increased ratio of stem-like cells.

For example, the improved cell number of the present invention refers to that compared to the cell number of the cells in which the expression and/or the activity of at least one family member selected from GTPase activating protein 1, FGF binding protein, Mediator (MED), PVR, BET family is unaltered, the cell number of the cells of the present invention in which the expression of at least one family member selected from the group consisting of GTPase activating protein 1, FGF binding protein, Mediator (MED), PVR, and BET family is reduced and/or the activity is weakened in at least one in vitro expansion stage can be increased by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the increased proportion of live cells can be expressed as an increase in cell survival rate. For example, the increased proportion of live cells in the present invention can mean that the proportion of live cells of the present invention in which the expression and/or activity of at least one family member selected from GTPase activating protein 1, FGF binding protein, Mediator (MED), PVR, BET family is reduced and/or the activity is weakened in at least one in vitro expansion stage can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the improved cytokine secretion capacity of the present invention may refer to the improved cytokine secretion capacity of the cell selected from the following group: IL-2, IL-6, CD107a, GZMB, TNF-α and IFN-γ. For example, compared to the cell whose expression and/or activity of at least one family member selected from the group consisting of GTPase activating protein 1, FGF binding protein, Mediator (MED), PVR, BET family is unchanged, the proportion of cells secreting cytokines in the cells of the present invention in which the expression of at least one family member selected from GTPase activating protein 1, FGF binding protein, Mediator (MED), PVR, or BET family is reduced and/or the activity is attenuated in at least one in vitro expansion stage can be increased by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved cytokine secretion capacity of the present invention may mean that the proportion of cells secreting cytokines in the cells of the present invention in which the expression and/or activity of at least one family member selected from GTPase activating protein 1, FGF binding protein, Mediator (MED), PVR, BET family is reduced and/or the activity is attenuated in at least one in vitro expansion stage can be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the cytokine secretion capacity of the cells of the present invention is determined by flow cytometry or CBA (Cytometric Bead Array).

For example, the improved in vitro tumor cell killing ability and/or improved in vivo tumor killing ability of the present invention may mean that the tumor cell killing rate of the cells of the present invention in which the expression and/or activity of at least one family member selected from GTPase activating protein 1, FGF binding protein, Mediator (MED), PVR, BET family is reduced and/or the activity is weakened in at least one in vitro expansion stage can be increased by at least about 1-50 times, for example, at least about 1 time, at least about 2 times, at least about 3 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved in vitro tumor cell killing ability and/or improved in vivo tumor killing ability of the present invention may mean that the tumor cell killing rate of the cells of the present invention in which the expression and/or activity of at least one family member selected from GTPase activating protein 1, FGF binding protein, Mediator (MED), PVR, BET family is reduced and/or the activity is weakened in at least one in vitro expansion stage can be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining. For example, tumor cell killing by a cell of the invention may refer to the ability of a cell to kill solid tumor cells.

For example, the cell subpopulation ratio improved by the present invention may include one or more selected from the following group: increased proportion of CD8⁺ cells, increased proportion of central memory cells and/or naive cells, decreased proportion of regulatory cells, increased proportion of activated cells, increased proportion of tumor-specific cells (with CD103⁺ CD39⁺ phenotype), increased proportion of stem-like cells, decreased proportion of exhausted cells, and decreased proportion of apoptotic cells.

For example, the increased proportion of CD8⁺ cells of the present invention may be an increase in the ratio of CD8 positive cells in the cells. The cell ratio can be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of activated cells increased by the present invention can be an increased ratio of CD28⁺, CD25⁺ and/or 41BB⁺ cells in the cells. For example, the proportion of activated cells in cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1 %, at least about 0.9%, at least about 0.8%, at least about 0.7%, at least about 0.6%, at least about 0.5%, at least about 0.4%, at least about 0.3%, at least about 0.2%, or at least about 0.1%, or can be increased by at least about 1-50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the reduced proportion of exhausted cells in the present invention may be an increased ratio of PD-1⁺, LAG-3⁺, TIM-3⁺, CD39⁺, CD38⁺ and/or CD101⁺ cells. For example, the proportion of exhausted cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or can be reduced by at least about 1-50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the proportion of regulatory cells decreased by the present invention can be a decrease in the ratio of CD4⁺ CD25⁺ Foxp3⁺ cells in the cells. For example, the proportion of regulatory cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of apoptotic cells reduced by the present invention can be a decrease in the ratio of AnnexinV⁺7-AAD⁺ cells and/or Annexin V⁺7-AAD⁻ cells in the cells. For example, the proportion of apoptotic cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the proportion of cells with stemness increased by the present invention can be an increase in the ratio of CD69 ⁻ CD39 ⁻ cells and/or TCF1⁺ cells in the cells. For example, the proportion of cells having stemness in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased central memory cell ratio of the present invention may be an increase in the ratio of CD45RA⁻ CCR7⁺ or CD45RO⁺ CD62L⁺ cells in the cells. For example, the proportion of central memory cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of naive T cells increased by the present invention can be an increase in the ratio of CD45RO⁻ CD62L⁺ cells in the cells. For example, the proportion of naive cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the culture method of the present invention may include a gene editing step for cells. For example, it includes: subjecting the cells to at least one stage of in vitro expansion, wherein, during at least one stage of in vitro expansion, a gene regulatory system may be introduced into the cells.

For example, the gene regulatory system can disrupt the target gene at the DNA level. For example, the gene regulatory system can disrupt the region or fragment of the target gene in the genome of the cell. For example, after using the gene regulatory system, the DNA region or fragment where the target gene is located in the cell is cleaved and the expression ability of the target gene is reduced or the activity of the target gene is inhibited. For example, the editing effect of the gene regulatory system on the target gene can be long-term and continuous. For example, in the cells of the present invention, the activity of at least one family member selected from the GTPase activating protein 1, FGF binding protein, Mediator (MED), PVR, and BET family is inhibited.

The genomic region described in the present invention is determined based on the human reference genome version hg38.

For example, the activity of RASA2 in the cells of the present invention is inhibited. For example, the preferred subregions of RASA2 in the cells shown in Table 1A of the present invention are knocked out and/or inhibited.

For example, the activity of FIBP in the cells of the present invention is inhibited. For example, the preferred subregions of FIBP in the cells shown in Table 1B of the present invention are knocked out and/or inhibited.

For example, the activity of MED12 in the cells of the present invention is inhibited. For example, the preferred subregions of MED12 in the cells shown in Table 1C of the present invention are knocked out and/or inhibited.

For example, the activity of TIGIT in the cells of the present invention is inhibited. For example, the preferred subregions of TIGIT in the cells shown in Table 1D of the present invention are knocked out and/or inhibited.

For example, the activity of BRD4 in the cells of the present invention is inhibited. For example, the preferred subregions of BRD4 in the cells shown in Table 1E of the present invention are knocked out and/or inhibited.

'For example, the gene regulation system may include a guide nucleic acid molecule and an enzyme protein. For example, the enzyme protein may have a nuclease activity, and the guide nucleic acid molecule may guide the enzyme protein to specifically cleave the region or fragment thereof where the target gene is located. For example, the guide nucleic acid molecule and the enzyme protein may exist in the form of a ribonucleoprotein complex (RNP) or exist independently of each other. For example, the enzyme protein may include a Cas protein. For example, a polynucleotide encoding a gRNA and a Cas protein may be introduced or independently introduced into a target cell.

For example, the present invention reduces the expression and/or weakens the activity of at least one target gene of a cell, and may include: introducing a ribonucleoprotein complex (RNP) comprising the guide nucleic acid molecule and the enzyme protein into the cell. For example, the enzyme protein may include a Cas protein, a Cas protein homolog, or a functionally active fragment thereof. For example, the guide nucleic acid molecule may include a guide RNA (gRNA). For example, a complex comprising a polynucleotide encoding a gRNA and a Cas protein may be introduced into the cell. For example, a complex comprising a gRNA and a Cas protein may be introduced into the cell.

For example, the gRNA can be used to bind to the sequence of the target gene. For example, the binding of the gRNA to the sequence of the target gene can be completely complementary, partially complementary, or hybridized to the sequence of the target gene under moderate stringency or stringent conditions. For example, the binding of the gRNA to the sequence of the target gene can enable the CRISPR system of the gRNA to specifically cleave the target gene.

For example, the editing target region of the present invention may be a region before the start codon. For example, the editing target region of the present invention may be a region with high transcription factor binding ability. For example, the editing target region of the present invention may be a region with a specific number of transcription factor binding numbers. For example, the editing target region of the present invention may be a continuous region with about 3 or more transcription factor binding numbers. For example, the genomic coordinates of the editing target region of the present invention may be selected from the preferred targeting subregions shown in Tables 1A to 1E.

For example, the guide nucleic acid molecule targeting RASA2 of the present invention can bind to a region or a fragment thereof selected from the group consisting of SEQ ID NOs: 1093-2184.

For example, the guide nucleic acid molecule targeting FIBP of the present invention can bind to a region or a fragment thereof selected from the group consisting of SEQ ID NOs: 2732-3278.

For example, the guide nucleic acid molecule targeting MED12 of the present invention can bind to a region or a fragment thereof selected from the group consisting of SEQ ID NOs: 4855-6430.

For example, the guide nucleic acid molecule targeting TIGIT of the present invention can bind to a region or a fragment thereof selected from the group consisting of SEQ ID NOs: 6988-7544.

For example, the guide nucleic acid molecule targeting BRD4 of the present invention can bind to a region or a fragment thereof selected from the group consisting of SEQ ID NOs: 10190-12834.

For example, when the gene editing system includes CRISPR/Cas9, the region targeted by the guide nucleic acid molecule of the present invention may have a protospacer adjacent motif (PAM) downstream, and the protospacer adjacent motif (PAM) may be AGG, TGG, GGG or CGG. For example, when the PAM region of the target gene is determined, a person skilled in the art can easily determine a target sequence consisting of about 15 to about 25 (e.g., about 15, about 16, about 17, about 18, about 19, about 20, about 21, about 22, about 23, about 24, about 25) nucleotides upstream of the 5' end of the PAM of the target gene, and a suitable gRNA can be designed for the target sequence. For example, the guide nucleic acid molecule of the present invention is capable of binding to a sequence consisting of about 15 to about 25 nucleotides upstream of the 5' end of the protospacer adjacent motif (PAM) selected from the group consisting of AGG, TGG, GGG and CGG.

For example, when the gene editing system includes CRISPR/Cas12, the region targeted by the guide nucleic acid molecule of the present invention may have a protospacer adjacent motif (PAM) upstream, and the protospacer adjacent motif (PAM) may be NTTN, TTYN, VTTV, TRTV, TTTV, TATV, TYCV, TNN, or NTN, wherein N is A, T, C or G, Y is T or C, V is A, C or G, and R is A or G. For example, when the PAM region of the target gene is determined, those skilled in the art can easily determine a target sequence consisting of about 15 to about 25 (e.g., about 15, about 16, about 17, about 18, about 19, about 20, about 21, about 22, about 23, about 24, about 25) nucleotides downstream of the 3' end of the PAM of the target gene, and can design a suitable gRNA for the target sequence. For example, the guide nucleic acid molecule can bind to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, or NTN, wherein N is A, T, C or G, Y is T or C, V is A, C or G, and R is A or G.

For example, when the gene editing system of the present invention comprises wild-type Cas12A (also referred to as Cpf1, such as AsCas12A, FnCas12A, LbCas12A, BbCas12A, CMaCas12A and OsCas12A), the region targeted by the guide nucleic acid molecule of the present invention may have a PAM sequence selected from the following upstream: NTTN, wherein N may be A, T, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as enAsCas12A (mutation sites E174R, S542R and K548R), the upstream of the region targeted by the guide nucleic acid molecule of the present invention may have a PAM sequence selected from the following: TTYN (TTTN/TTCN), VTTV (ATTV/CTTV/GTTV), or TRTV (TATV/TGTV), wherein N can be A, T, C or G, Y can be T or C, V can be A, C or G, and R can be A or G. For example, when the PAM region of the target gene is determined, those skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and can design a suitable gRNA for the target sequence.

For example, when the gene editing system of the present invention comprises a mutant Cas12A, such as opAsCas12A (mutation sites: E174R and S542R), the region targeted by the guide nucleic acid molecule of the present invention may have a PAM sequence selected from the following upstream: TTTV (TTTA, TTTC, or TTTG), wherein V may be A, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as AsCas12AUltra (mutation sites: M537R and F870L), the upstream of the region targeted by the guide nucleic acid molecule of the present invention may have a PAM sequence selected from the following: TTTV, TATV, or TYCV, wherein V may be A, C or G, and Y may be T or C. For example, when the PAM region of the target gene is determined, those skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises mutant Cas12A, such as hfCas12Max (mutation site: N243R/E336R/D892R) and Cas12Max (mutation site: N243R), the upstream of the region targeted by the guide nucleic acid molecule of the present invention may have a PAM sequence selected from the following: TNN, or NTN, wherein N may be A, T, C or G. For example, when the PAM region of the target gene is determined, those skilled in the art can easily determine the a target sequence consisting of about 17 to about 25 (e.g., about 17, about 18, about 19, about 20, about 21, about 22, about 23, about 24, or about 25) nucleotides downstream of the 3' end of the PAM, and a suitable gRNA can be designed for the target sequence.

For example, the guide nucleic acid molecule may comprise a sequence that can bind to a target sequence consisting of about 15 to about 25 nucleotides before the PAM region represented by AGG, TGG, GGG and/or CGG in the DNA encoding at least one family member member selected from the group consisting of GTPase activating protein 1, FGF binding protein, Mediator (MED), PVR, and BET families and/or functionally active fragments thereof. For example, the guide nucleic acid molecule may comprise a sequence that can bind to a target sequence consisting of about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20, about 15 to about 19, about 17 to about 19, or about 15 to about 17 nucleotides before the PAM region represented by AGG, TGG, GGG and/or CGG in the DNA encoding at least one family member member selected from the group consisting of GTPase activating protein 1, FGF binding protein, Mediator (MED), PVR, and BET families and/or functionally active fragments thereof. For example, the target sequence can be a region defined by the genomic coordinates shown in Tables 2A-2E, or a fragment thereof.

For example, the target sequence of the present invention can be the Ras-GAP structural functional domain of RASA2. For example, the target sequence of the present invention can be chr3:141529717-141529763, chr3:141529770-141529843, chr3:141553837-141553966, chr3:141570837-141570990, chr3:141571472-141571547, chr3:141572605-141572684, chr3:141572690-141572804, chr3:141573157-141573222, chr3:1 41574006-141574049, chr3:141576983-141577032, chr3:141577063-141577131, chr3:141580395-141580485, chr3:141586726- 141586864, chr3:141608480-141608679, chr3:141608687-141608714, chr3:141609482-141609524, chr3:141612412-141612507, chr3:141612768-141612863, chr3:141486577-141487341, chr3:141512136-141512298.

For example, the guide nucleic acid molecule can comprise a targeting domain of an sgRNA targeting RASA2 as shown in any one of SEQ ID NOs: 1-1092, 29247-29248, 29292-29314, a targeting domain of an sgRNA targeting FIBP as shown in any one of SEQ ID NOs: 2185-2731, 29249-29250, a targeting domain of an sgRNA targeting MED12 as shown in any one of SEQ ID NOs: 3279-4854, 29251-29252, a targeting domain of an sgRNA targeting TIGIT as shown in any one of SEQ ID NOs: 6431-6987, 29253-29254, or a targeting domain of an sgRNA targeting BRD4 as shown in any one of SEQ ID NOs: 7545-10189, 29255-29256.

For example, compared to cells in which the expression and/or activity of the target gene is not changed, the proportion of cells expressing the product of the target gene in the cells obtained by reducing the expression and/or weakening the activity of at least one target gene can be reduced and/or the expression level of the target gene in individual cells can be decreased.

For example, in the method of the present invention, the proportion of cells expressing the product of the target gene in the cells obtained by reducing the expression and/or attenuating the activity of at least one target gene in the cells is reduced by at least about 5% compared to cells in which the expression and/or activity of the target gene is not changed. For example, the proportion of cells expressing the product of the gene encoding at least one family member selected from the group consisting of GTPase activating protein 1, FGF binding protein, Mediator (MED), PVR, BET family, and/or functionally active fragments thereof is reduced by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the product of the gene encoding at least one family member selected from the group consisting of GTPase activating protein 1, FGF binding protein, Mediator (MED), PVR, and BET family and/or its functionally active fragments may be from a detectable proportion of cells to 1%. For example, the proportion of cells expressing the product of the gene encoding at least one family member selected from the group consisting of GTPase activating protein 1, FGF binding protein, Mediator (MED), PVR, and BET family and/or its functionally active fragments can be reduced to at least about 100-1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the product of the gene encoding at least one family member selected from GTPase activating protein 1, FGF binding protein, Mediator (MED), PVR, and BET family and/or its functionally active fragments can be detected by flow cytometry.

For example, in the method of the present invention, the expression of at least one target gene in the cells is reduced and/or the activity is weakened, and the proportion of cells expressing the product of the gene encoding at least one family member selected from the group consisting of GTPase activating protein 1, FGF binding protein, Mediator (MED), PVR, BET family and/or its functionally active fragments can be up to about 95%. For example, the proportion of cells expressing the product of the gene encoding at least one family member selected from the group consisting of GTPase activating protein 1, FGF binding protein, Mediator (MED), PVR, BET family and/or its functionally active fragments can be at most about 95-5%, such as at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing the product of the gene encoding at least one family member selected from GTPase activating protein 1, FGF binding protein, Mediator (MED), PVR, and BET family and/or their functionally active fragments can be detected by flow cytometry.

For example, in the method of the present invention, compared to the cells in which the expression and/or activity of the target gene is not changed, in the cells in which the expression of at least one target gene of the cell is reduced and/or the activity is weakened, the expression of the target gene in individual cells can be reduced by at least about 5%. For example, the expression of the target gene in a single cell can be reduced by at least about 100-5%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression of the target gene in a single cell can be from a detectable expression to 1%. For example, the expression level of the target gene in a single cell can be reduced to at least about 100-1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%.

For example, in the method of the present invention, in the cells in which the expression of at least one target gene is reduced and/or the activity is weakened, the expression amount of the target gene in a single cell can be at most about 95% of the cell whose expression and/or activity is not changed. For example, the expression amount of the gene encoding at least one family member selected from the group consisting of GTPase activating protein 1, FGF binding protein, Mediator (MED), PVR, and BET family and/or its functionally active fragment (e.g., a gene encoding RASA2, FIBP, MED12, TIGIT, and BRD4) in a single cell can be at most about 95-5% of that of the cells in which the expression and/or activity of the gene encoding at least one family member selected from the group consisting of GTPase activating protein 1, FGF binding protein, Mediator (MED), PVR, and BET family is unaltered, such as at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%.

For example, the method of the present invention comprises: subjecting the cells to at least one stage of in vitro expansion, wherein, in at least one stage of the in vitro expansion, the expression and/or activity of at least one family member selected from the group consisting of GTPase activating protein 1, FGF binding protein, Mediator (MED), PVR, and BET family in the cells is reduced.

For example, the TILs derived from tumor tissue, tumor-associated lymph nodes with or without tumor metastasis, tumor metastatic lesions, fragments of paracancerous tissue, pleural effusion and/or peritoneal effusion and not expanded in vitro are subjected to a first stage of in vitro expansion and a second stage of in vitro expansion, and in the second stage of in vitro expansion, the expression and/or activity of at least one family member selected from GTPase activating protein 1, FGF binding protein, Mediator (MED), PVR, and BET families of the TILs expanded in vitro in the first stage are reduced.

For example, the first stage in vitro expansion is performed for at least about 7 days. For example, the second stage in vitro expansion is performed for at least about 7 days.

For example, in a single stage of in vitro expansion of the present invention, the cell can be contacted with the one or more cell activators and the expression and/or activity of at least one family member selected from the GTPase activating protein 1, FGF binding protein, Mediator (MED), PVR, BET family and/or its functionally active fragments in the cell can be reduced. For example, the cell activator can include an agonist of one or more targets selected from the following group: CD3, CD28, HVEM, CD40L, OX40 and 4-1BB. For example, in a single stage of in vitro expansion, the expression and/or activity of at least one family member selected from the GTPase activating protein 1, FGF binding protein, Mediator (MED), PVR, BET family of the cell of the present invention is reduced and/or the activity is weakened and the cell is contacted with one or more cell activators of the present invention. For example, in the first stage of in vitro expansion of the present invention, the expression and/or activity of at least one family member selected from the GTPase activating protein 1, FGF binding protein, Mediator (MED), PVR, BET family of the TIL of the present invention can be reduced and/or the activity is weakened and the TIL is contacted with one or more cell activators of the present invention. For example, in the second stage of in vitro expansion of the present invention, the expression and/or activity of at least one family member selected from the GTPase activating protein 1, FGF binding protein, Mediator (MED), PVR, BET family of the TIL of the present invention can be reduced and/or the activity is weakened and the TIL is contacted with one or more cell activators of the present invention. For example, in the third stage of in vitro expansion of the present invention, the expression and/or activity of at least one family member selected from GTPase activating protein 1, FGF binding protein, Mediator (MED), PVR, BET family of the TIL of the present invention can be reduced and/or the activity is weakened and the TIL is contacted with one or more cell activators of the present invention.

For example, in a single stage of in vitro expansion, the cells of the present invention substantially simultaneously reduce the expression and/or weaken the activity of at least one family member selected from GTPase activating protein 1, FGF binding protein, Mediator (MED), PVR, and BET families, and contact with one or more cell activators of the present invention. For example, in a single stage of in vitro expansion, the cells of the present invention first reduce the expression and/or weaken the activity of at least one family member selected from GTPase activating protein 1, FGF binding protein, Mediator (MED), PVR, and BET families, for example, 2-48 hours in advance, for example, 2 hours in advance, 4 hours in advance, 8 hours in advance, 12 hours in advance, 24 hours in advance, or 48 hours in advance, and then contact with one or more cell activators of the present invention. For example, in a single stage of in vitro expansion, the cells of the present invention are first contacted with one or more cell activators of the present invention, for example, 2-48 hours in advance, such as 2 hours in advance, 4 hours in advance, 8 hours in advance, 12 hours in advance, 24 hours in advance, or 48 hours in advance, etc., and then the expression and/or activity of at least one family member selected from the group consisting of GTPase activating protein 1, FGF binding protein, Mediator (MED), PVR, and BET family is reduced.

For example, in the first stage of in vitro expansion of the present invention, the TIL of the present invention substantially simultaneously reduces the expression and/or weakens the activity of at least one family member selected from GTPase activating protein 1, FGF binding protein, Mediator (MED), PVR, and BET family, and contacts with one or more cell activators of the present invention. For example, in the second stage of in vitro expansion of the present invention, the TIL of the present invention substantially simultaneously reduces the expression and/or weakens the activity of at least one family member selected from GTPase activating protein 1, FGF binding protein, Mediator (MED), PVR, and BET family, and contacts with one or more cell activators of the present invention. For example, in the third stage of in vitro expansion of the present invention, the TIL of the present invention substantially simultaneously reduces the expression and/or weakens the activity of at least one family member selected from the group consisting of GTPase activating protein 1, FGF binding protein, Mediator (MED), PVR, and BET family, and contacts with one or more cell activators of the present invention.

### TIL Cell Culture

For example, the second stage in vitro expansion of the present invention is carried out for at least about 7 days. For example, the second stage in vitro expansion of the present invention can be carried out for at least about 7 days, at least about 8 days, at least about 9 days, at least about 10 days, at least about 11 days, at least about 12 days, at least about 13 days, or at least about 14 days. For example, the second stage in vitro expansion of the present invention can be carried out for about 7 days to about 14 days, preferably about 9 days to about 14 days, for example, the second stage in vitro expansion of the present invention can be carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage in vitro expansion of the present invention can be considered as the REP (rapid expansion protocol) stage. For example, the first stage of in vitro expansion of the present invention can be considered as the preREP stage.

For example, the first stage in vitro expansion of the present invention is carried out for at least about 7 days. For example, the first stage in vitro expansion of the present invention can be carried out for at least about 7 days, at least about 8 days, at least about 9 days, at least about 10 days, at least about 11 days, at least about 12 days, at least about 13 days, or at least about 14 days. For example, the first stage in vitro expansion of the present invention can be carried out for about 7 days to about 14 days, preferably about 9 days to about 14 days, for example, the first stage in vitro expansion of the present invention can be carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days.

For example, the number of days for the second stage of in vitro expansion of the present invention can be calculated from the start time of the second stage of in vitro expansion. For example, at the moment when the second stage of in vitro expansion begins, it can be considered that the second stage in vitro expansion has been carried out for about 0 hours. For example, at about 24 hours after the start of the second stage in vitro expansion, it can be considered that the second stage in vitro expansion has been carried out for about 1 day. For example, the day when the second stage in vitro expansion starts can be considered that the second stage in vitro expansion has been carried out for about 0 days. For example, the number of days for the second stage in vitro expansion of the present invention can be calculated by the number of days for the second stage in vitro expansion. For example, the day after the start of the second stage in vitro expansion, it can be considered that the second stage in vitro expansion has been carried out for about 1 day.

For example, the cell activator of the present invention may include one or more selected from the following group: CD80, CD86, B7-H3, 4-1BBL, CD27, CD30, CD134, B7h, CD40, LIGHT, and their functionally active fragments. For example, the cell activator of the present invention may include an agonist of one or more targets selected from the following group: CD3, CD28, HVEM, CD40L, OX40 and 4-1BB. For example, the cell activator of the present invention may include an antibody selected from the following group: CD3, CD28, HVEM, CD40L, OX40 and 4-1BB and their antigen-binding fragments. For example, the cell activator of the present invention may include a CD3 agonist. For example, the cell activator of the present invention may include an anti-CD3 antibody and/or an antigen-binding fragment thereof, such as OKT3 of Miltenyi Biotech, and SP34 of BD. For example, the cell activator of the present invention may include a CD28 agonist. For example, the cell activator of the present invention may comprise an anti-CD28 antibody and/or an antigen-binding fragment thereof, such as 15E8 from Merck.

For example, the cell activator of the present invention may comprise an anti-CD3 antibody and/or an antigen-binding fragment thereof, for example, it may comprise the light chain VL and heavy chain VH of OKT3 of Miltenyi Biotech, and it may comprise the light chain VL and heavy chain VH of SP34 of BD. For example, the cell activator of the present invention may comprise a CD28 agonist. For example, the cell activator of the present invention may comprise an anti-CD28 antibody and/or an antigen-binding fragment thereof, for example, it may comprise the light chain VL and heavy chain VH of 15E8 of Merck. For example, the cell activator of the present invention may comprise an anti-CD3 antibody and/or an antigen-binding fragment thereof, for example, it may comprise the light chain LCDR1-3 and heavy chain HCDR1-3 of OKT3 of Miltenyi Biotech, and it may comprise the light chain LCDR1-3 and heavy chain HCDR1-3 of SP34 of BD, and the anti-CD3 antibody and/or an antigen-binding fragment thereof of the present invention may have CD3 binding ability. For example, the cell activator of the present invention may comprise a CD28 agonist. For example, the cell activator of the present invention may comprise an anti-CD28 antibody and/or an antigen-binding fragment thereof, for example, it may comprise the light chain LCDR1-3 and heavy chain HCDR1-3 of Merck's 15E8, and the anti-CD28 antibody and/or an antigen-binding fragment thereof of the present invention may have CD28 binding ability. In the present invention, the antibody of the present invention or its antigen-binding protein comprises at least one CDR in the heavy chain variable region VH of the antibody and/or at least one CDR in the light chain variable region VL of the antibody. The CDR of the present invention may be defined according to the IMGT nomenclature, the CDR of the present invention may be defined according to Chothia, or the CDR of the present invention may be defined according to Kabat.

For example, contacting the cells of the present invention with one or more cell activators of the present invention may include one or more methods selected from the following group: (1) adding the cell activator of the present invention to the cell culture medium of the present invention; (2) adding engineered cells expressing the cell activator of the present invention to the cell culture medium of the present invention; (3) adding a solid phase medium containing the cell activator of the present invention to the cell culture medium of the present invention. For example, contacting the cells of the present invention with one or more cell activators of the present invention may include adding a solid phase medium containing the cell activator of the present invention to the cell culture medium of the present invention. For example, contacting the cells of the present invention with one or more cell activators of the present invention may include adding a solid phase medium containing the CD28 antibody and the CD3 antibody of the present invention to the cell culture medium of the present invention.

For example, the initial concentration of the cell activator in the cell culture medium of the present invention can be at least about 30 ng/mL. For example, the initial concentration of the CD28 antibody of the present invention in the cell culture medium of the present invention can be at least about 30 ng/mL; for example, the initial concentration of the CD3 antibody of the present invention in the cell culture medium of the present invention can be at least about 30 ng/mL. For example, the selection of the initial concentration of the CD28 antibody of the present invention can be independent of the selection of the initial concentration of the CD3 antibody of the present invention; for example, the initial concentrations of the CD28 antibody of the present invention and the CD3 antibody of the present invention in the cell culture medium of the present invention can be arbitrarily combined. For example, the initial concentration of the CD28 antibody of the present invention in the cell culture medium of the present invention can be arbitrarily selected from about 30 ng/mL-about 300 ng/mL. For example, the initial concentration of the CD3 antibody of the present invention in the cell culture medium of the present invention can be arbitrarily selected from about 30 ng/mL-about 300 ng/mL. For example, the initial concentration of the CD28 antibody of the present invention in the cell culture medium of the present invention can be arbitrarily selected from about 30 ng/mL-about 300 ng/mL, and the CD3 antibody of the present invention in the cell culture medium of the present invention can be arbitrarily selected from about 30 ng/mL to about 300 ng/mL, and the selection of the initial concentration of the CD28 antibody of the present invention can be independent of the selection of the initial concentration of the CD3 antibody of the present invention. For example, the diameter of the solid phase medium of the present invention can be about 500 nanometers to about 10 microns. For example, the diameter of the solid phase medium of the present invention can be measured by transmission electron microscopy. For example, the diameter of the solid phase medium of the present invention can be about 1 nanometer to about 500 nanometers. For example, the diameter of the solid phase medium of the present invention can be about 100 nanometers to about 500 nanometers. For example, the diameter of the solid phase medium of the present invention can be about 200 nanometers to about 500 nanometers. For example, the diameter of the solid phase medium of the present invention can be measured by transmission electron microscopy.

For example, the solid phase medium of the present invention may comprise a polymer. For example, the solid phase medium of the present invention may comprise dextran.

For example, the solid phase medium of the present invention contains at least about 25 µ g of the cell activating agent of the present invention per mg.

For example, the solid phase medium containing the cell activator of the present invention is added to the cell culture medium of the present invention at a ratio of about 100:1 to about 1:2000, preferably about 1:100 to about 1:2000. For example, the solid phase medium containing the cell activator of the present invention is added to the cell culture medium of the present invention at a ratio of about 2:1 to about 1:2.

For example, when the diameter of the solid phase medium of the present invention is about 100 nanometers to about 500 nanometers, the solid phase medium containing the cell activator of the present invention can be added to the cell culture medium of the present invention at a ratio of about 2:1 to about 1:2 of the solid phase medium of the present invention to the cells of the present invention. For example, when the diameter of the solid phase medium of the present invention is about 100 nanometers to about 500 nanometers, the solid phase medium containing the cell activator of the present invention, such as a CD3 agonist and/or a CD28 agonist, can be added to the cell culture medium of the present invention at a ratio of about 2:1 to about 1:2, about 2:1 to about 1:1, or about 1:1 to about 1:2 of the solid phase medium of the present invention to the cells of the present invention.

For example, when the diameter of the solid phase medium of the present invention is about 100 nanometers to about 500 nanometers, the solid phase medium containing the cell activator of the present invention can be added to the cell culture medium of the present invention at a ratio of about 1:100 to about 1:2000 of the solid phase medium of the present invention to the cells of the present invention. For example, when the diameter of the solid phase medium of the present invention is about 100 nanometers to about 500 nanometers, a solid medium comprising a CD28 agonist and a CD3 agonist of the present invention can be added to the cell culture medium of the present invention at a ratio of about 1:100 to about 1:2000, about 1:200 to about 1:2000, about 1:300 to about 1:2000, about 1:400 to about 1:2000, about 1:500 to about 1:2000, about 1:600 to about 1:2000, about 1:700 to about 1:2000, about 1:800 to about 1:2000, about 1:900 to about 1:2000, about 1:1000 to about 1:2000, about 1:1100 to about 1:2000, about 1:1200 to about 1:2000, about 1:1300 to about 1:2000, about 1:1400 to about 1:2000, about 1:1500 to about 1:2000, about 1:1600 to about 1:2000, about 1:1700 to about

1:2000, or about 1:1800 to about 1:2000 of the solid medium of the present invention to the cells of the present invention.

For example, the method of the present invention may further comprise: contacting the cells of the present invention with one or more cell growth factors during at least one stage of the in vitro expansion of the present invention.

For example, in a single stage of in vitro expansion of the present invention, the cells of the present invention can be contacted with the cell activator of the present invention and with one or more cell growth factors of the present invention. For example, in the first stage of in vitro expansion of the present invention, the TIL of the present invention can be contacted with the cell activator of the present invention and with one or more cell growth factors of the present invention. For example, in the second stage of in vitro expansion of the present invention, the TIL of the present invention can be contacted with the cell activator of the present invention and with one or more cell growth factors of the present invention. For example, in the third stage of in vitro expansion of the present invention, the TIL of the present invention can be contacted with the cell activator of the present invention and with one or more cell growth factors of the present invention.

For example, in a single stage of in vitro expansion of the present invention, the cells of the present invention are contacted with the cell activator of the present invention and one or more cell growth factors of the present invention at substantially the same time. For example, in a single stage of in vitro expansion of the present invention, the cells of the present invention can be contacted with one or more cell growth factors of the present invention and one or more cell activators of the present invention at substantially the same time. For example, in a single stage of in vitro expansion of the present invention, the cells of the present invention can be contacted with one or more cell growth factors of the present invention first, for example, 2-48 hours in advance, for example, 2 hours in advance, 4 hours in advance, 8 hours in advance, 12 hours in advance, 24 hours in advance, or 48 hours in advance, and then contacted with one or more cell activators of the present invention. For example, in a single stage of in vitro expansion of the present invention, the cells of the present invention may be first contacted with one or more cell activators of the present invention, for example, 2-48 hours in advance, such as 2 hours in advance, 4 hours in advance, 8 hours in advance, 12 hours in advance, 24 hours in advance, or 48 hours in advance, and then contacted with one or more cell growth factors of the present invention.

For example, in the first stage of in vitro expansion of the present invention, the cells of the present invention can be contacted with the cell activator of the present invention and one or more cell growth factors of the present invention at substantially the same time. For example, in the second stage of in vitro expansion of the present invention, the TIL of the present invention can be contacted with the cell activator of the present invention and one or more cell growth factors of the present invention at substantially the same time. For example, in the third stage of in vitro expansion of the present invention, the TIL of the present invention can be contacted with the cell activator of the present invention and one or more cell growth factors of the present invention at substantially the same time.

For example, the cell growth factor of the present invention can be selected from one or more of the following group: IL-2, IL-7, IL-12, IL-15, IL-21, interferon-γ, and their functionally active fragments. For example, the cell growth factor of the present invention can contain IL-2 and/or its functionally active fragments. For example, the functionally active fragments of IL-2 contain fragments of IL-2 that can bind to the IL-2 receptor of the cell as known in the art. For example, the cell growth factor of the present invention can contain IL-2 and/or its functionally active fragments, IL-7 and/or its functionally active fragments, and IL-15 and/or its functionally active fragments.

For example, contacting the cells of the present invention with one or more cell growth factors of the present invention may comprise adding the cell growth factors of the present invention to the cell culture medium of the present invention. For example, the initial concentration of the cell growth factors of the present invention in the cell culture medium of the present invention may be at least about 300 IU/mL. For example, the initial concentration of IL-2 of the present invention in the cell culture medium of the present invention may be at least about 300-9000 IU/mL, such as at least about 300 IU/mL, at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

For example, the cells of the present invention can reduce the amount of cytokines when in contact with IL-2, IL-7 and IL-15, relative to when in contact with IL-2 alone. For example, the amount of IL-2 added can be reduced under the condition of adding IL-7 and IL-15. For example, the concentration of IL-7 can be about 1 to 1000ng/mL, preferably about 1-100ng/mL. For example, the concentration of IL-15 can be about 1 to 1000ng/mL, preferably about 1-100ng/mL. For example, for the amount of IL-2 added, it can be reduced to the commonly used range in the art for various immune cells, for example, reduced to 50-10% of the commonly used range in the art, such as 50%, 20% or 10%. For example, for the amount of IL-2 added to TCR-T, the commonly used range in the art can be 30-300IU/mL. For example, for the amount of IL-2 added to TIL, the commonly used range in the art can be 300-9000IU/mL (e.g. 1000-9000IU/mL).

For example, the method of the present invention may further comprise: in at least one stage of the in vitro expansion of the present invention, the cells of the present invention may be co-cultured with feeder cells.

For example, in a single stage of in vitro expansion of the present invention, the cells of the present invention can be contacted with one or more cell activators and/or one or more cell growth factors and co-cultured with the feeder cells of the present invention. For example, a single stage of in vitro expansion of the present invention can refer to in vitro expansion of the present invention at the same stage, for example, it can be in vitro expansion at the first stage of the present invention, in vitro expansion at the second stage of the present invention, or in vitro expansion at the third stage of the present invention, etc.

For example, in the first stage in vitro expansion of the present invention, the TIL of the present invention can be contacted with one or more cell activators and/or one or more cell growth factors and co-cultured with the feeder of the present invention. For example, in the second stage in vitro expansion of the present invention, the TIL of the present invention can be contacted with one or more cell activators and/or one or more cell growth factors of the present invention and co-cultured with the feeder cells of the present invention. For example, in the third stage in vitro expansion of the present invention, the TIL of the present invention can be contacted with one or more cell activators and/or one or more cell growth factors of the present invention and co-cultured with the feeder cells of the present invention.

For example, in a single stage of in vitro expansion of the present invention, the cells of the present invention can be contacted with one or more cell activators and/or one or more cell growth factors of the present invention for a certain period of time, and then co-cultured with the feeder cells of the present invention. For example, in the first stage of in vitro expansion of the present invention, the TIL of the present invention can be contacted with one or more cell activators and/or one or more cell growth factors of the present invention for a certain period of time, and then co-cultured with the feeder cells of the present invention. For example, in the second stage of in vitro expansion of the present invention, the TIL of the present invention can be contacted with one or more cell activators and/or one or more cell growth factors of the present invention for a certain period of time, and then co-cultured with the feeder cells of the present invention. For example, in the third stage of in vitro expansion of the present invention, the TIL of the present invention can be contacted with one or more cell activators and/or one or more cell growth factors of the present invention for a certain period of time, and then co-cultured with the feeder cells of the present invention.

For example, in a single stage of in vitro expansion of the present invention, the cells of the present invention can be contacted with one or more cell activators and/or one or more cell growth factors of the present invention for a certain period of time before being co-cultured with the feeder cells of the present invention. For example, the certain period of time of the present invention can be at least about 1 hour. For example, the certain period of time of the present invention can be at least about 1-72 hours, such as at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours or at least about 72 hours. For example, the certain period of time of the present invention can be from about 2 hours to about 72 hours. For example, the certain time of the present invention can be about 6 hours to about 7 hours, about 6 hours to about 8 hours, about 6 hours to about 9 hours, or about 6 hours to about 10 hours, from about 6 hours to about 11 hours, from about 6 hours to about 12 hours, from about 6 hours to about 13 hours, from about 6 hours to about 14 hours, from about 6 hours to about 15 hours, from about 6 hours to about 16 hours, from about 6 hours to about 17 hours, from about 6 hours to about 18 hours, from about 6 hours to about 19 hours, from about 6 hours to about 20 hours, from about 6 hours to about 21 hours, from about 6 hours to about 22 hours, from about 6 hours to about 23 hours, from about 6 hours to about 24 hours, from about 6 hours to about 36 hours, from about 6 hours to about 48 hours, from about 6 hours to about 60 hours, or from about 6 hours to about 72 hours. For example, the certain time of the present invention can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain time of the present invention can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours or about 72 hours.

For example, the feeder cells of the present invention may comprise antigen presenting cells. For example, the feeder cells of the present invention may comprise one or more selected from the following groups: peripheral mononuclear cells, dendritic cells, and artificial antigen presenting cells. For example, the feeder cells of the present invention may be peripheral mononuclear cells. For example, the feeder cells of the present invention may be irradiated feeder cells. For example, the feeder cells of the present invention may be isolated artificial antigen presenting cells (aAPCs), and the artificial antigen presenting cells of the present invention may comprise cells expressing HLA-A/B/C, CD64, CD80, ICOS-L and/or CD58, and may be modified to express more than one cell activator of the present invention. For example, the feeder cells of the present invention may be irradiated, for example, may be irradiated with gamma rays, or may be irradiated with X rays.

For example, co-culturing the cells of the present invention with the feeder cells of the present invention may comprise contacting the surface of the feeder cells of the present invention with the surface of the cells of the present invention. For example, co-culturing the cells of the present invention with the feeder cells of the present invention comprises adding the feeder cells of the present invention to the cell culture medium of the present invention.

For example, the feeder cells of the present invention can be added to the cell culture medium of the present invention at a ratio of about 40:1 to about 400:1 of the feeder cells of the present invention to the cells of the present invention. For example, the feeder cells of the present invention can be added to the cell culture medium of the present invention at a ratio of about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, at about 70:1 to about 400:1, at about 80:1 to about 400:1, at about 90:1 to about 400:1, at about 100:1 to about 400:1, at about 200:1 to about 400:1, or at about 300:1 to about 400:1 of feeder cells of the invention to cells of the invention.

In another aspect, the present invention provides a method for culturing tumor infiltrating lymphocytes (TIL), which may comprise: (A) contacting a first TIL population derived from tumor tissue, tumor-associated lymph nodes with or without tumor metastasis, tumor metastatic lesions, fragments of peritumoral tissue, pleural effusion and/or peritoneal effusion and not expanded in vitro with one or more cell growth factors; wherein, a second TIL population is obtained through step (A); (B) reducing the expression and/or activity of at least one family member selected from the group consisting of GTPase activating protein 1, FGF binding protein, Mediator (MED), PVR, and BET family in the second TIL population; wherein, a third TIL population is obtained through step (B).

In another aspect, the present invention provides a method for culturing tumor infiltrating lymphocytes (TIL), comprising: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with one or more T cell growth factors, wherein a second TIL population is obtained through step (A); (B) reducing the expression and/or activity of at least one family member selected from the group consisting of GTPase activating protein 1, FGF binding protein, Mediator (MED), PVR, and BET family, and contacting the second TIL population with a T cell activator and/or a T cell growth factor, wherein a third TIL population is obtained through step (B); (C) the third TIL population is co-cultured with feeder cells, wherein a fourth TIL population is obtained through step (C).

In one embodiment, the first stage in vitro expansion of the present invention can be arbitrarily replaced with step (A) in the method of the above aspect. In one embodiment, the second stage in vitro expansion of the present invention can be arbitrarily replaced with step (B) in the method of the above aspect. In one embodiment, the TIL of the present invention that has been amplified in vitro in the first stage can be arbitrarily replaced with the second TIL group obtained by step (A) in the method of the above aspect. In one embodiment, the TIL of the present invention that has been amplified in vitro in the second stage can be arbitrarily replaced with the third TIL group obtained by step (B) in the method of the above aspect. In one embodiment, if necessary, the third stage in vitro expansion of the present invention can be arbitrarily replaced with any additional step (C) in the method of the above aspect. In one embodiment, if necessary, the TIL of the present invention that has been amplified in vitro in the third stage can be arbitrarily replaced with the fourth TIL group obtained by any additional step (C) in the method of the above aspect.

In another aspect, the present invention provides a method for culturing tumor infiltrating lymphocytes (TIL), which may include: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with multiple cell growth factors; wherein, a second TIL population is obtained through step (A); (B) contacting the second TIL population with multiple cell growth factors, with multiple cell activators, reducing the expression and/or weakening the activity of at least one family member selected from GTPase activating protein 1, FGF binding protein, Mediator (MED), PVR, and BET family, and co-culturing the TIL with feeder cells; wherein, a third TIL population is obtained through step (B).

In another aspect, the present invention provides a method for culturing tumor infiltrating lymphocytes (TILs), which may comprise: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with with a cell growth factor; wherein, a second TIL population is obtained through step (A); (B) the second TIL population can be contacted with a cell growth factor, with a cell activator, the expression and/or activity of at least one family member selected from the group consisting of GTPase activating protein 1, FGF binding protein, Mediator (MED), PVR, and BET family can be reduced and/or weakened, and the TIL can be co-cultured with feeder cells, and at least one family member selected from the group consisting of GTPase activating protein 1, FGF binding protein, Mediator (MED), PVR, and BET family can include RASA2, FIBP, MED12, TIGIT, and BRD4, respectively; wherein, a third TIL population is obtained through step (B).

In another aspect, the present invention provides a method for culturing tumor infiltrating lymphocytes (TIL). The method for obtaining TIL cells from a subject's tissue sample can be to obtain an in situ tumor sample or a metastatic tumor sample from a patient during surgery, the weight of which can be at least about 1g, or multiple pieces of tissue can be combined. Tumor tissue, tumor-associated lymph nodes with or without tumor metastasis, tumor metastatic lesions, fragments of paracancerous tissue, pleural effusion and/or peritoneal effusion are transported in a sample transport fluid, such as a commercially commonly used tumor tissue transport fluid, tumor tissue preservation fluid or tumor tissue transport fluid at about 2-8°C and processed within 48 hours. Tissue blocks can be mechanically broken into a size of about 1-27 cubic millimeters per block, transferred into a breathable culture bag or Grex, and cell serum-free culture medium and IL-2 at a concentration of 300-9000IU/mL (e.g., 1000-9000IU/mL, e.g., 6000IU/mL) are added and cultured for about 3-14 days. The cells in the culture medium are collected and transferred into a breathable culture bag, or a Grex, or a Xuri device. The serum-free culture medium of the cells can be supplemented with the CD28 antibody, CD3 antibody and CD28 antibody of the present invention, magnetic beads (e.g., Dynabeads) comprising CD3 antibody and CD28 antibody and/or nanomatrices (e.g., transACT) comprising CD3 antibody and CD28 antibody, IL-2 at a concentration of 300-9000 IU/mL (e.g., 1000-9000 IU/mL, e.g., 6000 IU/mL), and can reducing the expression and/or attenuating the activity of at least one family member selected from GTPase activating protein 1, FGF binding protein, Mediator (MED), PVR, BET family members (selected from GTPase activating protein 1 family members can include RASA2, FGF binding protein family members can include FIBP, Mediator (MED) family members can include MED12, PVR family members can include TIGIT, and BET family members can include BRD4, for example, by transducing with a ribonucleoprotein complex (RNP) containing the gRNA of the present invention and the Cas protein, or LNP containing the gRNA and the Cas protein, or LNP containing the nucleic acid encoding gRNA and Cas protein so that the cell ratio of the gene encoding at least one family member selected from GTPase activating protein 1, FGF binding protein, Mediator (MED), PVR, and BET family in the TIL is about 95% or less), after activating the TIL of the present invention for a certain period of time, irradiated PBMC is added (TIL and PBMC are in a ratio of about 1:40-about 1:400), and the culture is expanded for about 3-14 days. The cells in the culture medium can be collected using a cell processing system, washed, frozen, and detected. The CD3 ratio of the final product can be greater than 80%, the cell survival rate can be greater than 50%, and cells greater than 80% can be memory effector cells and effector cells. IFN-γ can be secreted after stimulation, and/or it can have the characteristic of an increased proportion of activated cells.

### IKZF1 Knockout

1. A method for culturing cells, the method comprising: reducing the expression and/or weakening the activity of IKAROS zinc finger protein family members and/or functionally active fragments thereof in the cells.
2. The method of embodiment 1, wherein the cells comprise immune cells.
3. The method according to embodiment 2, wherein the immune cells comprise phagocytes, lymphocytes, neutrophils, eosinophils and/or basophils.
4. A method according to any one of embodiments 2-3, wherein the immune cells comprise monocytes, macrophages and/or dendritic cells.
5. A method according to any one of embodiments 2-4, wherein the immune cells are derived from immune cells differentiated from stem cells.
6. A method according to embodiment 5, wherein the stem cells comprise induced pluripotent stem cells (iPSCs), embryonic stem cells, bone marrow stem cells, umbilical cord blood stem cells and/or peripheral blood stem cells.
7. The method according to any one of embodiments 2-6, wherein the immune cell comprises B cells, T cells, natural killer cells and/or natural killer-like T cells (NKT).
8. A method according to any one of embodiments 2-7, wherein the immune cells comprise αβ T cells and/or γδ T cells.
9. A method according to any one of embodiments 2-8, wherein the immune cells comprise tumor infiltrating lymphocytes (TILs).
10. A method according to embodiment 9, wherein the TIL is TIL derived from tumor tissue, tumor-associated lymph nodes with or without tumor metastasis, tumor metastatic lesions, fragments of peritumoral tissue, pleural effusion and/or peritoneal effusion and/or TIL derived from recovery after cryopreservation.
11. The method of embodiment 10, wherein the volume of the fragments is from about 1 cubic millimeter to about 27 cubic millimeters.
12. A method according to any one of embodiments 2-11, wherein the immune cell comprises an engineered immune receptor displayed on the cell surface.
13. A method according to embodiment 12, wherein the engineered immune receptor specifically binds to an antigen expressed on a target cell.
14. A method according to any one of embodiments 2-13, wherein the immune cell comprises a chimeric antigen receptor and/or a T cell receptor.
15. The method according to any one of embodiments 1-14, wherein reducing the expression and/or attenuating the activity of the IKAROS zinc finger protein family member in the cell comprises inhibiting the DNA binding function of the zinc finger structure.
16. The method of any one of embodiments 1-15, wherein compared to cells in which the expression and/or activity of the IKAROS zinc finger protein family member is not altered, cells obtained by reducing the expression and/or activity of the IKAROS zinc finger protein family members show improved cellular properties.
17. A method according to embodiment 16, wherein the improved cell characteristics include one or more selected from the following group: improved cell proliferation ability, increased proportion of live cells, improved proportion of cell subpopulations, increased cytokine secretion ability, improved in vitro tumor cell killing ability and improved in vivo tumor killing ability.
18. A method according to embodiment 17, wherein the improved proportion of cell subpopulations comprises one or more selected from the following group: an increased proportion of activated cells, a decreased proportion of regulatory cells, a decreased proportion of exhausted cells, an increased proportion of central memory cells and/or naive cells, a decreased proportion of apoptotic cells and an increased proportion of stem-like cells.
19. The method of any one of embodiments 1-18, wherein the IKAROS zinc finger protein family member comprises a zinc finger domain.
20. The method of any one of embodiments 1-19, wherein the IKAROS zinc finger protein family member comprises IKZF1.
21. The method according to any one of embodiments 1-20, wherein reducing the expression and/or attenuating the activity of the IKAROS zinc finger protein family member in the cell comprises introducing a gene regulatory system into the cell.
22. The method according to embodiment 21, wherein the gene regulatory system is capable of disrupting the IKAROS zinc finger protein family member at the DNA level; and optionally, selected from TNFAIP3, SOCS1, ZC3H12A, CBLB, FAS, ADNP, NFKBIA, PTPN6, TNIP1, LAG3, PD1, TIM3, BCL2L11, PTPN2, AFF3, AXL, NFE2L1, RARG, UBFD1, CRP, CYLD, GIF, KLF4, NDST1, NLRP1, SCGB1A1, ADCY7, ARIH2, CPT2, LNPEP, NOSIP, NPRL3, TANK, TRAF3, TSC1, ZBTB7B, ZC3H12D, RC3H2, RASA2, FIBP, MED12, TIGIT and BRD4, the expression and/or activity thereof is reduced.
23. A method according to any one of embodiments 21-22, wherein the gene regulatory system comprises a guiding nucleic acid molecule and an enzyme protein.
24. A method according to embodiment 23, wherein reducing the expression and/or weakening the activity of the IKAROS zinc finger protein family member comprises:
   introducing into the cell a complex comprising the guide nucleic acid molecule and the enzyme protein, or a complex comprising the guide nucleic acid molecule and a nucleic acid encoding the enzyme protein, an LNP comprising a gRNA and a Cas protein, or an LNP comprising a nucleic acid encoding a gRNA and a Cas protein.
25. A method according to any one of embodiments 23-24, wherein the enzyme protein comprises a Cas protein, a Cas protein homolog, or a functionally active fragment thereof, preferably selected from Cas 9 and Cas 12.
26. A method according to any one of embodiments 23-25, wherein the guide nucleic acid molecule comprises a guide RNA (gRNA).
27. The method of any one of embodiments 23-26, wherein the guide nucleic acid molecule is capable of binding to a sequence of the IKAROS zinc finger protein family member.
28. A method according to any one of embodiments 23-27, wherein the guide nucleic acid molecule binds to a sequence consisting of about 15 to about 25 nucleotides upstream of the 5' end of the protospacer adjacent motif (PAM) selected from the following group: AGG, TGG, CGG and GGG, or binds to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, and NTN, wherein N is A, T, C or G, Y is T or C, V is A, C or G, and R is A or G.
29. The method according to any one of embodiments 23-28, wherein the guide nucleic acid molecule binds to a region defined by the genomic coordinates selected from those shown in Table 1F or Table 2F, or a fragment thereof.
30. The method of any one of embodiments 23-29, wherein the guide nucleic acid molecule binds to at least one region or a fragment thereof selected from the group consisting of SEQ ID NOs: 16057-19278.
31. A method according to any one of embodiments 23-30, wherein the guide nucleic acid molecule includes a targeting domain, and the targeting domain comprises a sequence as shown in any one of SEQ ID NOs: 12835-16056, 29257-29258, 29279-29291.
32. The method according to any one of embodiments 1-31, wherein the proportion of cells expressing the product of the target gene in the cells obtained by reducing the expression and/or attenuating the activity of the IKAROS zinc finger protein family member is reduced and/or the expression level of the target gene in individual cells is decreased compared to cells in which the expression and/or activity of the IKAROS zinc finger protein family member is not altered.
33. The method according to any one of embodiments 1-32, wherein among the cells obtained by reducing the expression and/or attenuating the activity of the IKAROS zinc finger protein family member, the proportion of cells expressing the target gene is about 95% or less.
34. A cell obtained by the method of any one of embodiments 1-33.
35. A composition comprising the cell of embodiment 34.
36. A pharmaceutical composition comprising the cell of embodiment 34 and/or the composition of embodiment 35, and optionally a pharmaceutically acceptable carrier.
37. A method of influencing cell growth, comprising administering the cell of embodiment 34, the composition of embodiment 35 and/or the pharmaceutical composition of embodiment 36.
38. Use of the cell described in embodiment 34, the composition described in embodiment 35 and/or the pharmaceutical composition described in embodiment 36 in the preparation of a drug, wherein the drug is used to prevent and/or treat a disease and/or symptom.
39. The use according to embodiment 38, wherein the disease and/or symptom comprises a tumor.
40. The use according to any one of embodiments 38-39, wherein the disease and/or condition comprises a solid tumor.
41. The use according to any one of embodiments 38-40, wherein the disease and/or symptoms comprise one or more selected from the following group: melanoma, ovarian cancer, cervical cancer, lung cancer, bladder cancer, breast cancer, head and neck cancer, pancreatic cancer, liver cancer, gastric cancer, colorectal cancer and kidney cancer.

The present invention provides a method for reducing the expression and/or weakening the activity of an IKAROS zinc finger protein family member and/or a functionally active fragment thereof in the cell.

In one aspect, the present invention provides a method for culturing cells, so that the expression and/or activity of an IKAROS zinc finger protein family member and/or its functionally active fragment is reduced in the cell. For example, the IKAROS zinc finger protein family member may comprise a zinc finger domain. For example, the IKAROS zinc finger protein family member may comprise IKZF1.

For example, the target gene of the present invention may be a gene encoding a member of the IKAROS zinc finger protein family and/or a functionally active fragment thereof. For example, compared with a cell in which the expression and/or activity of the target gene is not changed, a cell obtained by reducing the expression and/or weakening the activity of at least one target gene of the cell may show improved cell characteristics. In one embodiment, a cell in which the expression and/or activity of the target gene is not changed may refer to a cell derived from the same donor and in which the expression and/or activity of at least one target gene of the cell has not been reduced and/or weakened.

In one embodiment, cells in which the expression and/or activity of the target gene is not altered may refer to cells derived from the same donor and in which the expression and/or activity of other genes other than the target gene of the cells have not been reduced (for example, knocking out the other gene has substantially no effect on the function of the cells).

For example, the cell comprises an immune cell. For example, the cell comprises an immune effector cell. For example, the cell comprises an immune effector T cell, an immune effector NK cell, an immune effector NKT cell. For example, the cell comprises a phagocyte, a lymphocyte, a neutrophil, an eosinophil and/or a basophil.

For example, the cells comprise monocytes, macrophages and/or dendritic cells.

For example, the cells of the present invention also include cells derived from stem cell differentiation. For example, the cells of the present invention also include cells derived from pluripotent stem cell differentiation. For example, obtaining the stem cells of the present invention can be produced by induction. For example, the above-mentioned stem cells of the present invention can include induced pluripotent stem cells (iPSC), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from reproductive tissue of a fetus. Exemplary pluripotent stem cells can also be generated from somatic cells by reprogramming them to a pluripotent state through the expression of certain transcription factors associated with pluripotency; these cells are referred to as "induced pluripotent stem cells" or "iPSCs."

For example, the cells include B cells, T cells, natural killer cells and/or natural killer-like T cells (NKT). For example, "unmodified cells" or "unmodified cells" may refer to cells or cell populations in which the genome is not modified and does not include a gene regulatory system or includes a control gene regulatory system (e.g., an empty vector control, a non-targeting gRNA, an interfering siRNA, etc.). For example, the cells include αβ T cells and/or γδ T cells. For example, the cells include tumor infiltrating lymphocytes (TILs). For example, the TILs are TILs derived from tumor tissue, tumor-associated lymph nodes with or without tumor metastasis, tumor metastasis lesions, fragments of paracancerous tissue, pleural effusions and/or peritoneal effusions and/or TILs recovered after cryopreservation.

For example, the TIL of the present invention can be TIL derived from tumor tissue, tumor-associated lymph nodes with or without tumor metastasis, tumor metastasis lesions, fragments of paracancerous tissue, pleural effusion and/or peritoneal effusion and/or TIL derived from recovery after cryopreservation. For example, the TIL of the present invention can be obtained by processing tumor tissue into tumor fragments. For example, the volume of the tumor fragments of the present invention is about 1-27 cubic millimeters. For example, the volume of a tumor fragment of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cell comprises an engineered immune receptor displayed on the cell surface. For example, the engineered immune receptor specifically binds to an antigen expressed on a target cell. For example, the cell comprises a chimeric antigen receptor and/or a T cell receptor.

In one aspect, the present invention provides a method for culturing tumor infiltrating lymphocytes (TIL), which may comprise: reducing the expression and/or activity of IKAROS zinc finger protein family members and/or functionally active fragments thereof in the TIL.

For example, TILs derived from tumor tissue, pleural effusion and/or peritoneal effusion and not expanded in vitro can be subjected to at least one stage of in vitro expansion, wherein, in at least one stage of the in vitro expansion, the expression and/or activity of IKAROS zinc finger protein family members and/or functionally active fragments thereof in the TILs can be reduced.

For example, the TILs of the present invention that are derived from tumor tissue, pleural effusion and/or peritoneal effusion and have not been expanded in vitro can be subjected to a first stage of in vitro expansion and a second stage of in vitro expansion, and in the second stage of in vitro expansion of the present invention, the expression and/or activity of IKAROS zinc finger protein family members and/or their functionally active fragments in the TILs can be reduced.

For example, the TILs of the present invention that are derived from tumor tissue, pleural effusion and/or peritoneal effusion and have not been expanded in vitro can be subjected to a first stage of in vitro expansion and a second stage of in vitro expansion, and in the first stage of in vitro expansion of the present invention, the expression and/or activity of IKAROS zinc finger protein family members and/or their functionally active fragments in the TILs can be reduced.

For example, the TIL of the present invention that is derived from tumor tissue, pleural effusion and/or peritoneal effusion and has not been expanded in vitro can be subjected to a first stage in vitro expansion and a second stage in vitro expansion, and in the first stage in vitro expansion of the present invention, the expression and/or activity of IKAROS zinc finger protein family members and/or their functionally active fragments in the TIL can be reduced, and in the second stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been expanded in vitro can be subjected to a first stage in vitro expansion, a second stage in vitro expansion and a third stage in vitro expansion, and in the first stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been expanded in vitro can be subjected to a first stage in vitro expansion, a second stage in vitro expansion and a third stage in vitro expansion, and in the second stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been expanded in vitro can be subjected to a first stage in vitro expansion, a second stage in vitro expansion and a third stage in vitro expansion, and in the third stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been amplified in vitro can be subjected to a first stage in vitro expansion, a second stage in vitro expansion and a third stage in vitro expansion, and in the first stage in vitro expansion of the present invention, the expression and/or activity of IKAROS zinc finger protein family members and/or their functionally active fragments in the TIL can be reduced, and in the second stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been expanded in vitro can be subjected to a first stage in vitro expansion, a second stage in vitro expansion and a third stage in vitro expansion, and in the first stage in vitro expansion of the present invention, the expression and/or activity of IKAROS zinc finger protein family members and/or their functionally active fragments in the TIL can be reduced, and in the third stage in vitro expansion of the present invention, the expression and/or activity of IKAROS zinc finger protein family members and/or their functionally active fragments in the TIL can be reduced.

For example, the TIL of the present invention that is derived from tumor tissue, pleural effusion and/or peritoneal effusion and has not been expanded in vitro can be subjected to a first stage in vitro expansion, a second stage in vitro expansion and a third stage in vitro expansion, and in the second stage in vitro expansion of the present invention, the expression and/or activity of IKAROS zinc finger protein family members and/or their functionally active fragments in the TIL can be reduced, and in the third stage in vitro expansion of the present invention, the expression and/or activity of IKAROS zinc finger protein family members and/or their functionally active fragments in the TIL can be reduced.

For example, the TILs derived from tumor tissue, pleural effusion and/or peritoneal effusion of the present invention and not expanded in vitro can be subjected to a first stage of in vitro expansion, a second stage of in vitro expansion and a third stage of in vitro expansion, and in the first stage in vitro expansion of the present invention, the expression and/or activity of IKAROS zinc finger protein family members and/or their functionally active fragments in the TIL can be reduced, and in the second stage in vitro expansion of the present invention, the expression and/or activity of IKAROS zinc finger protein family members and/or their functionally active fragments in the TIL can be reduced, and in the third stage in vitro expansion of the present invention, the expression and/or activity of IKAROS zinc finger protein family members and/or their functionally active fragments in the TIL can be reduced.

For example, cells obtained by reducing the expression and/or attenuating the activity of an IKAROS zinc finger protein family member exhibit improved cell properties compared to cells in which the expression and/or activity of the IKAROS zinc finger protein family member is not altered.

For example, the improved cell number of the present invention means that the cell number of the cells of the present invention in which the expression and/or activity of the IKAROS zinc finger protein family member is reduced and/or the activity is attenuated in at least one in vitro expansion stage can be increased by at least about 1 time to about 50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times, compared to cells in which the expression and/or activity of the IKAROS zinc finger protein family member is not altered.

For example, the increased proportion of live cells can be expressed as an increase in cell survival rate. For example, the increased proportion of live cells of the present invention can mean that the proportion of live cells of the cells of the present invention in which the expression and/or activity of the IKAROS zinc finger protein family member is reduced in at least one in vitro expansion stage can be increased by at least about 100% to about 0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the improved cytokine secretion capacity of the present invention may refer to an improved cytokine secretion capacity of the cell of a cytokine selected from the following group: IL-2, IL-6, CD107a, GZMB, TNF-α and IFN-γ. For example, the improved cytokine secretion capacity of the present invention may refer to an increase in the proportion of cells secreting cytokines in the cells of the present invention in which the expression and/or activity of the IKAROS zinc finger protein family member is reduced and/or the activity is attenuated in at least one in vitro expansion stage, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times, compared to cells in which the expression and/or activity of the IKAROS zinc finger protein family member is not changed. For example, the improved cytokine secretion capacity of the present invention may mean that the proportion of cells secreting cytokines in the cells of the present invention in which the expression and/or activity of the IKAROS zinc finger protein family member is reduced in at least one in vitro expansion stage can be increased by at least about 100% to about 0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the improved tumor cell killing ability of the present invention may mean that the tumor cell killing rate of the cells of the present invention in which the expression and/or activity of the IKAROS zinc finger protein family member is reduced in at least one in vitro expansion stage can be increased by at least about 1 to about 50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved tumor cell killing ability of the present invention can mean that the tumor cell killing rate of the cells of the present invention in which the expression and/or activity of the IKAROS zinc finger protein family member is reduced and/or the activity is attenuated in at least one in vitro expansion stage can be increased by at least about 100% to about 0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining. For example, the tumor cell killing of the cells of the present invention can refer to the ability of the cells to kill solid tumor cells.

For example, the improved cell subpopulation ratio of the present invention may include one or more selected from the following group: increased CD8⁺ cells, an increased proportion of central memory cells and/or naive cells, a decreased proportion of regulatory cells, an increased proportion of activated cells, an increased proportion of tumor-specific cells, and an increased proportion of stem-like cells.

For example, in the cells the proportion of CD8⁺ cells, central memory cells and/or naive cells, activated cells, tumor-specific cells and/or stem-like cells can be increased by at least about 100% to about 0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the reduced exhausted cell ratio of the present invention can be an increase in the ratio of PD-1⁺, LAG-3⁺, TIM-3⁺, and/or CD39⁺ cells in the cells. For example, the reduced proportion of regulatory cells in the present invention may be an increase in the proportion of CD4⁺ CD25⁺ Foxp3⁺ cells in the cells. For example, the reduced apoptotic cell ratio of the present invention can be a decrease in the proportion of CD95⁺ caspass3⁺ cells and/or CD95⁺ DR5⁺ cells in the cells.

For example, the proportion of exhausted cells, regulatory cells and/or apoptotic cells in a cell can be reduced by at least about 100% to about 0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be reduced by at least about 1 time to about 50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the culture method of the present invention may include a gene editing step for cells. For example, it includes: subjecting the cells to at least one stage of in vitro expansion, wherein, during at least one stage of in vitro expansion, a gene regulatory system may be introduced into the cells.

For example, the gene regulatory system can disrupt the target gene at the DNA level. For example, the gene regulatory system can disrupt the region or fragment of the target gene in the genome of the cell. For example, after using the gene regulatory system, the DNA region or fragment of the target gene in the cell is cleaved and the expression ability of the target gene is reduced, or the activity of the target gene is inhibited. For example, the editing effect of the gene regulatory system on the target gene can be long-term and continuous. The genomic region of the present invention is determined according to the human reference genome hg38 version.

For example, the gene regulation system may include a guide nucleic acid molecule and an enzyme protein. For example, the enzyme protein may have a nuclease activity, and the guide nucleic acid molecule may guide the enzyme protein to specifically cleave the region or fragment thereof where the target gene is located. For example, the guide nucleic acid molecule and the enzyme protein may exist in the form of a ribonucleoprotein complex (RNP) or exist independently of each other. For example, the enzyme protein may include a Cas protein. For example, a polynucleotide encoding a gRNA and a Cas protein may be introduced or independently introduced into a target cell.

For example, the present invention reduces the expression and/or weakens the activity of at least one target gene of a cell and may include: introducing a ribonucleoprotein complex (RNP) comprising the guide nucleic acid molecule and the enzyme protein into the cell. For example, the enzyme protein may include a Cas protein, a Cas protein homolog, or a functionally active fragment thereof. For example, the guide nucleic acid molecule may include a guide RNA (gRNA). For example, the guide nucleic acid molecule may include a guide RNA (gRNA). For example, a complex comprising a polynucleotide encoding a gRNA and a Cas protein may be introduced into the cell. For example, a complex comprising a gRNA and a Cas protein may be introduced into the cell.

For example, the gRNA can be used to bind to the sequence of the target gene. For example, the binding of the gRNA to the sequence of the target gene can be completely complementary, partially complementary, or hybridized to the sequence of the target gene under moderate stringency or stringent conditions. For example, the binding of the gRNA to the sequence of the target gene can enable the CRISPR system of the gRNA to specifically cleave the target gene.

For example, the editing target region of the present invention may be a region before the promoter. For example, the editing target region of the present invention may be a region with high binding affinity of transcription factors. For example, the editing target region of the present invention may be a region with a specific number of transcription factor bindings. For example, the editing target region of the present invention may be a continuous region with about 3 or more transcription factor bindings.

For example, when the gene editing system includes CRISPR/Cas9, the region targeted by the guide nucleic acid molecule of the present invention may have a protospacer adjacent motif (PAM) downstream, and the protospacer adjacent motif (PAM) may be AGG, TGG, GGG or CGG. For example, when the PAM region of the target gene is determined, a person skilled in the art can easily determine a target sequence consisting of about 15 to about 25 (e.g., about 15, about 16, about 17, about 18, about 19, about 20, about 21, about 22, about 23, about 24, about 25) nucleotides upstream of the 5' end of the PAM of the target gene, and a suitable gRNA can be designed for the target sequence. For example, the guide nucleic acid molecule can bind to a sequence consisting of about 15 to about 25 nucleotides upstream of the 5' end of the protospacer adjacent motif (PAM) selected from the following group: AGG, TGG, GGG and CGG.

For example, when the gene editing system includes CRISPR/Cas12, the region targeted by the guide nucleic acid molecule of the present invention may have a protospacer adjacent motif (PAM) upstream, and the protospacer adjacent motif (PAM) may be NTTN, TTYN, VTTV, TRTV, TTTV, TATV, TYCV, TNN, or NTN, wherein N is A, T, C or G, Y is T or C, V is A, C or G, and R is A or G. For example, when the PAM region of the target gene is determined, those skilled in the art can easily determine a target sequence consisting of about 15 to about 25 (e.g., about 15, about 16, about 17, about 18, about 19, about 20, about 21, about 22, about 23, about 24, about 25) nucleotides downstream of the 3' end of the PAM of the target gene, and can design a suitable gRNA for the target sequence. For example, the guide nucleic acid molecule can bind to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, or NTN, wherein N is A, T, C or G, Y is T or C, V is A, C or G, and R is A or G.

For example, when the gene editing system of the present invention comprises wild-type Cas12A (also referred to as Cpf1, such as AsCas12A, FnCas12A, LbCas12A, BbCas12A, CMaCas12A and OsCas12A), the upstream of the region targeted by the guide nucleic acid molecule of the present invention may have a PAM sequence selected from: NTTN, wherein N can be A, T, C or G. For example, when the PAM region of the target gene is determined, one skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and can design a suitable gRNA for the target sequence.

For example, when the gene editing system of the present invention comprises a mutant Cas12A, such as enAsCas12A (mutation sites E174R, S542R and K548R), the upstream of the region targeted by the guide nucleic acid molecule of the present invention may have a PAM sequence selected from the following: TTYN (TTTN/TTCN), VTTV (ATTV/CTTV/GTTV), or TRTV (TATV/TGTV), wherein N may be A, T, C or G, Y may be T or C, V may be A, C or G, and R may be A or G. For example, when the PAM region of the target gene is determined, those skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as opAsCas12A (mutation sites: E174R and S542R), the region targeted by the guide nucleic acid molecule of the present invention may have a PAM sequence selected from the following upstream: TTTV (TTTA, TTTC, or TTTG), wherein V may be A, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as AsCas12AUltra (mutation sites: M537R and F870L), the upstream of the region targeted by the guide nucleic acid molecule of the present invention may have a PAM sequence selected from the following: TTTV, TATV, or TYCV, wherein V may be A, C or G, and Y may be T or C. For example, when the PAM region of the target gene is determined, those skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises mutant Cas12A, such as hfCas12Max (mutation site: N243R/E336R/D892R) and Cas12Max (mutation site: N243R), the upstream of the region targeted by the guide nucleic acid molecule of the present invention may have a PAM sequence selected from the following: TNN, or NTN, wherein N may be A, T, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, the guide nucleic acid molecule can comprise a target sequence consisting of about 10 to about 30 nucleotides before the PAM region represented by AGG, TGG, GGG and/or CGG in the DNA encoding the IKAROS zinc finger protein family member and/or its functionally active fragment. For example, the guide nucleic acid molecule can comprise a sequence that can bind to a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides, before the PAM region represented by AGG, TGG, GGG and/or CGG in the DNA containing the gene encoding an IKAROS zinc finger protein family member and/or a functionally active fragment thereof. For example, the target sequence can be a region defined by the genomic coordinates shown in Table 1F or a fragment thereof.

For example, the target sequence of the present invention can be the C-Zinc_finger structural and functional domain, the Zinc_finger2 structural and functional domain or the Zinc_finger3 structural and functional domain of IKZF1. For example, the target sequence of the present invention can be chr7:50327705-50327877, chr7:50382595-50382830, chr7:50399941-50400296, chr7:50400527-50400604, chr7:50400848-50401107, chr7:50401112-50401195, chr7:50401215-50401542, chr7:50400609-50400790.

For example, the guide nucleic acid molecule can include a targeting domain that is complementary to a target sequence selected from the group consisting of SEQ ID NOs: 16057-19278.

For example, the guide nucleic acid molecule can include a targeting domain, which can comprise a sequence as shown in SEQ ID NOs: 12835-16056, 29257-29258, 29279-29291.

For example, the guide nucleic acid molecule can include a targeting domain, which can comprise a sequence as shown in SEQ ID NOs: 29257-29258, 29279-29291.

For example, compared with cells in which the expression and/or activity of at least one target gene of the cells is not changed, the proportion of cells expressing the product of the target gene in the cells obtained by reducing the expression and/or attenuating the activity of the target gene can be reduced and/or the expression level of the target gene in a single cell can be decreased.

For example, in the methods of the present invention, the proportion of cells expressing the product of the target gene is reduced by at least about 5% in the cells obtained by reducing the expression and/or attenuating the activity of at least one target gene in the cells, compared to cells in which the expression and/or activity of the target gene is not altered. For example, the proportion of cells expressing the product of the gene encoding the IKAROS zinc finger protein family member and/or its functionally active fragment is reduced by at least about 100% to about 5%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the product of the gene encoding the IKAROS zinc finger protein family member and/or its functionally active fragment can be from a detectable proportion of cells to 1%. For example, the proportion of cells expressing the product of the gene encoding the IKAROS zinc finger protein family member and/or its functionally active fragment can be reduced to at least about 100% to about 1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the product of the gene encoding the IKAROS zinc finger protein family member and/or its functionally active fragment can be detected by flow cytometry.

For example, the proportion of cells expressing the product of the gene encoding the IKAROS zinc finger protein family member and/or its functionally active fragment among the cells obtained by the method of the present invention with reduced expression and/or attenuated activity of at least one target gene of the cells can be up to about 95%. For example, the proportion of cells expressing the product of the gene encoding the IKAROS zinc finger protein family member and/or its functionally active fragment can be at most about 95% to about 5%, such as at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing the product of the gene encoding the IKAROS zinc finger protein family member and/or its functionally active fragment can be detected by flow cytometry.

For example, in the method of the present invention, compared with cells in which the expression and/or activity of at least one target gene of the cell is not changed, the expression level of the target gene in a single cell obtained by reducing the expression and/or weakening the activity of the target gene can be reduced by at least about 5%. For example, the expression level of the target gene in a single cell can be reduced by at least about 100% to about 5%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell can be from a detectable amount to 1%. For example, the expression level of the target gene in a single cell can be reduced to at least about 100% to about 1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%.

For example, in the method of the present invention, the expression level of the target gene in a single cell obtained by reducing the expression and/or weakening the activity of at least one target gene in the cell can be at most about 95% of the cell in which the expression and/or activity of the target gene is not changed. For example, the expression level of the gene encoding the IKAROS zinc finger protein family member and/or its functionally active fragment (e.g., the gene encoding IKZF1) in a single cell of the cell can be at most about 95% to about 5%, such as at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5% of that of the cells in which the expression and/or activity of the gene encoding the IKAROS zinc finger protein family member and/or its functionally active fragment is not altered.

For example, the method of the present invention comprises: subjecting the cell to at least one stage of in vitro expansion, wherein, during at least one stage of in vitro expansion, the expression and/or activity of an IKAROS zinc finger protein family member of the cell is reduced.

For example, TILs derived from tumor tissues, tumor-associated lymph nodes with or without tumor metastasis, tumor metastasis lesions, fragments of paracancerous tissue, pleural effusion and/or peritoneal effusion and not expanded in vitro undergo a first stage of in vitro expansion and a second stage of in vitro expansion, and in the second stage of in vitro expansion, the expression and/or activity of IKAROS zinc finger protein family members of the TILs expanded in vitro in the first stage are reduced.

For example, the first stage in vitro expansion is performed for at least about 7 days. For example, the second stage in vitro expansion is performed for at least about 7 days.

For example, in a single stage of in vitro expansion of the present invention, the cell can be contacted with the one or more cell activators and the expression and/or activity of the IKAROS zinc finger protein family members and/or their functionally active fragments in the cell can be reduced. For example, the cell activator can include an agonist of one or more targets selected from the following group: CD3, CD28, HVEM, CD40L, OX40 and 4-1BB. For example, in a single stage of in vitro expansion, the expression of the IKAROS zinc finger protein family members of the cells of the present invention is reduced and/or the activity is weakened and the cell is contacted with one or more cell activators of the present invention. For example, in the first stage of in vitro expansion of the present invention, the TIL of the present invention can be reduced in the expression and/or the activity of the IKAROS zinc finger protein family members of the present invention and contacted with one or more cell activators of the present invention. For example, in the second stage of in vitro expansion of the present invention, the TIL of the present invention can be reduced in the expression and/or the activity of the IKAROS zinc finger protein family members of the present invention and contacted with one or more cell activators of the present invention. For example, in the third stage in vitro expansion of the present invention, the TIL of the present invention can be reduced in expression and/or the activity of the IKAROS zinc finger protein family members of the present invention and contacted with one or more cell activators of the present invention.

For example, in a single stage of in vitro expansion of the present invention, the cells of the present invention can be made to reduce the expression and/or weaken the activity of the IKAROS zinc finger protein family members and contact one or more cell activators of the present invention at substantially the same time. For example, in a single stage of in vitro expansion of the present invention, the cells of the present invention can be made to reduce the expression and/or weaken the activity of the IKAROS zinc finger protein family members first, for example, 2 hours in advance, 4 hours in advance, 8 hours in advance, 12 hours in advance, 24 hours in advance, or 48 hours in advance, etc., and then contact with one or more cell activators of the present invention. For example, in a single stage of in vitro expansion of the present invention, the cells of the present invention can be made to contact with one or more cell activators of the present invention first, for example, 2 hours in advance, 4 hours in advance, 8 hours in advance, 12 hours in advance, 24 hours in advance, or 48 hours in advance, etc., and then to reduce the expression and/or weaken the activity of the IKAROS zinc finger protein family member.

For example, in the first stage of in vitro expansion of the present invention, the TIL of the present invention can be made to substantially simultaneously reduce the expression and/or weaken the activity of the IKAROS zinc finger protein family members and contact one or more cell activators of the present invention. For example, in the second stage of in vitro expansion of the present invention, the TIL of the present invention can be made to substantially simultaneously reduce the expression and/or weaken the activity of the IKAROS zinc finger protein family members and contact one or more cell activators of the present invention. For example, in the third stage of in vitro expansion of the present invention, the TIL of the present invention can be made to substantially simultaneously reduce the expression and/or weaken the activity of the IKAROS zinc finger protein family members and contact one or more cell activators of the present invention.

In another aspect, the present invention provides a method for culturing tumor infiltrating lymphocytes (TIL), which may comprise: (A) contacting a first TIL population derived from tumor tissue, pleural effusion and/or peritoneal effusion and not expanded in vitro with one or more cell growth factors; wherein, a second TIL population is obtained through step (A); (B) reducing the expression and/or weakening the activity of IKAROS zinc finger protein family members in the second TIL population; wherein, a third TIL population is obtained through step (B).

In one embodiment, the first stage in vitro expansion of the present invention can be arbitrarily replaced with step (A) in the method of the above aspect. In one embodiment, the second stage in vitro expansion of the present invention can be arbitrarily replaced with step (B) in the method of the above aspect. In one embodiment, the TILs of the present invention that have undergone the first stage of in vitro expansion can be arbitrarily replaced with the second TIL population obtained by step (A) in the method of the above aspect. In one embodiment, the TILs of the present invention that have undergone the second stage of in vitro expansion can be arbitrarily replaced with the third TIL population obtained by step (B) in the method of the above aspect. In one embodiment, if necessary, the third stage of in vitro expansion of the present invention can be arbitrarily replaced with any additional step (C) in the method of the above aspect. In one embodiment, if necessary, the TILs of the present invention that have undergone the third stage of in vitro expansion can be arbitrarily replaced with the fourth TIL group obtained in step (C) in the method of the above aspect.

In another aspect, the present invention provides a method for culturing tumor infiltrating lymphocytes (TIL), which may comprise: (A) a first TIL population derived from tumor tissue, pleural effusion and/or peritoneal effusion and not expanded in vitro may be contacted with a plurality of cell growth factors; wherein a second TIL population is obtained through step (A); (B) the second TIL population may be contacted with a plurality of cell growth factors, with a plurality of cell activators, the expression and/or activity of IKAROS zinc finger protein family members may be reduced, and the TIL may be co-cultured with feeder cells; wherein a third TIL population is obtained through step (B).

In another aspect, the present invention provides a method for culturing tumor infiltrating lymphocytes (TIL), which may comprise: (A) a first TIL population derived from tumor tissue, pleural effusion and/or peritoneal effusion and not expanded in vitro may be contacted with a cell growth factor; wherein, a second TIL population is obtained through step (A); (B) the second TIL population may be contacted with a cell growth factor, with a cell activator, the expression and/or activity of an IKAROS zinc finger protein family member may be reduced and the TIL may be co-cultured with a feeder cell, and the IKAROS zinc finger protein family member may include IKZF1; wherein, a third TIL population is obtained through step (B).

In another aspect, the present invention provides a method for culturing tumor infiltrating lymphocytes (TIL). The method for obtaining TIL cells from a subject's tissue sample can be that the patient obtains an in situ tumor sample or a metastatic tumor sample by surgery, and the weight can be at least about 1g, or multiple tissues can be combined. Tumor tissue, pleural effusion and/or peritoneal effusion are transported in a sample transport fluid, such as a commercially commonly used tumor tissue transport fluid, tumor tissue preservation fluid or tumor tissue transport fluid, at about 2-8 degrees and processed within 48 hours. The tissue blocks can be mechanically broken to a size of about 1-27 cubic millimeters per block, transferred into a breathable culture bag or Grex, and cell serum-free culture medium and IL-2 at a concentration of 300-9000IU/mL (for example, 1000-9000IU/mL, for example, 6000IU/mL) are added and cultured for about 3-14 days. The cells in the culture medium are collected and transferred into a permeable culture bag, or a Grex, or a Xuri device. The serum-free culture medium of the cells can be supplemented with the CD28 antibody, CD3 antibody and CD28 antibody of the present invention, magnetic beads (e.g., Dynabeads) containing CD3 antibody and CD28 antibody and/or nanomatrices (e.g., transACT) containing CD3 antibody and CD28 antibody, IL-2 at a concentration of 300-9000IU/mL (for example, 1000-90001U/mL, for example, 6000IU/mL), and reduces the expression and/or activity of members of the IKAROS zinc finger protein family (members of the IKAROS zinc finger protein family may include IKZF1, for example, the ratio of cells encoding genes of members of the IKAROS zinc finger protein family in the TIL may be about 95% or less by transduction with a ribonucleoprotein complex (RNP) containing the gRNA of the present invention and the Cas protein). After activating the TIL of the present invention for a certain period of time, add irradiated PBMC (TIL and PBMC at a ratio of about 1:40-about 1:400) and expand for about 3-14 days. Cells in the culture medium may be collected using a cell processing system, washed, frozen, and detected. The CD3 ratio of the final product may be greater than 80%, the cell survival rate may be greater than 50%, and cells greater than 80% may be memory effector cells and effector cells. After stimulation, IFN-γ can be secreted and/or the activated cell ratio can be increased.

### ADNP, NFKBIA, PTPN6, TNIP1 Knockout

1. A method for culturing cells, the method comprising: reducing the expression and/or attenuating the activity of at least one family member selected from the activity-dependent neuroprotective protein family, the NF-kappa-B inhibitory protein family, the protein tyrosine phosphatase family, and the A20 binding protein family and/or its functionally active fragments in the cells.
2. The method of embodiment 1, wherein the cells comprise immune cells.
3. The method according to embodiment 2, wherein the immune cells comprise phagocytes, lymphocytes, neutrophils, eosinophils and/or basophils.
4. A method according to any one of embodiments 2-3, wherein the immune cells comprise monocytes, macrophages and/or dendritic cells.
5. A method according to any one of embodiments 2-4, wherein the immune cells are derived from immune cells differentiated from stem cells.
6. The method according to embodiment 5, wherein the stem cells comprise induced pluripotent stem cells (iPSCs), embryonic stem cells, bone marrow stem cells, umbilical cord blood stem cells and/or peripheral blood stem cells.
7. A method according to any one of embodiments 2-6, wherein the immune cells comprise B cells, T cells, natural killer cells and/or natural killer-like T cells (NKT).
8. A method according to any one of embodiments 2-7, wherein the immune cells comprise αβ T cells and/or γδ T cells.
9. A method according to any one of embodiments 2-8, wherein the immune cells comprise tumor infiltrating lymphocytes (TILs).
10. A method according to embodiment 9, wherein the TIL is TIL derived from tumor tissue, tumor-associated lymph nodes with or without tumor metastasis, tumor metastatic lesions, fragments of peritumoral tissue, pleural effusion and/or peritoneal effusion and/or TIL derived from recovery after cryopreservation.
11. The method of embodiment 10, wherein the volume of the fragments is from about 1 cubic millimeter to about 27 cubic millimeters.
12. A method according to any one of embodiments 2-11, wherein the immune cell comprises an engineered immune receptor displayed on the cell surface.
13. A method according to embodiment 12, wherein the engineered immune receptor specifically binds to an antigen expressed on a target cell.
14. A method according to any one of embodiments 2-13, wherein the immune cell comprises a chimeric antigen receptor and/or a T cell receptor.
15. A method according to any one of embodiments 1-14, wherein reducing the expression and/or attenuating the activity in the cell of at least one family member selected from the activity-dependent neuroprotective protein family, the NF-kappa-B inhibitory protein family, the tyrosine phosphatase family and the A20 binding protein family and/or its functionally active fragments comprises an effect selected from the following group: inhibiting the DNA binding function of the zinc finger structure, inhibiting the function of binding to NF-kappa-B/REL, inhibiting the function of tyrosine phosphatase, and inhibiting the binding function to A20.
16. A method according to any one of embodiments 1 to 15, wherein the cells obtained by reducing the expression and/or attenuating the activity of at least one family member selected from the activity-dependent neuroprotective protein family, the NF-kappa-B inhibitory protein family, the protein tyrosine phosphatase family, the A20 binding protein family, and/or its functionally active fragments show improved cell properties compared to cells in which the expression of at least one family member selected from the activity-dependent neuroprotective protein family, the NF-kappa-B inhibitory protein family, the protein tyrosine phosphatase family, the A20 binding protein family, and/or its functionally active fragments is reduced and/or the activity is not changed.
17. A method according to embodiment 16, wherein the improved cell characteristics include one or more selected from the following group: improved cell proliferation ability, increased proportion of live cells, improved proportion of cell subpopulations, increased cytokine secretion ability, improved in vitro tumor cell killing ability and improved in vivo tumor killing ability.
18. A method according to embodiment 17, wherein the improved proportion of cell subpopulations comprises one or more selected from the following group: an increased proportion of activated cells, a decreased proportion of regulatory cells, a decreased proportion of exhausted cells, an increased proportion of central memory cells and/or naïve cells, a decreased proportion of apoptotic cells and an increased proportion of stem-like cells.
19. A method according to any one of embodiments 1-18, wherein the family members selected from the activity-dependent neuroprotective protein family, the NF-kappa-B inhibitory protein family, the protein tyrosine phosphatase family, and the A20 binding protein family respectively contain a zinc finger domain, an ankyrin (ANKYRIN) repeat domain, a SRC homolog (SH2) domain, and an A20 binding domain.
20. A method according to any one of embodiments 1-19, wherein the protein selected from the group consisting of the activity-dependent neuroprotective protein family, the NF-kappa-B inhibitory protein family, the protein tyrosine phosphoprotein family, the A20 binding protein family include ADNP, NFKBIA, PTPN6, and TNIP1, respectively.
21. A method according to any one of embodiments 1-20, wherein reducing the expression and/or attenuating the activity of a family member selected from the activity-dependent neuroprotective protein family, the NF-kappa-B inhibitory protein family, the protein tyrosine phosphatase family, and the A20 binding protein family in the cell comprises introducing a gene regulatory system into the cell.
22. A method according to embodiment 21, wherein the gene regulatory system disrupts the family members selected from the activity-dependent neuroprotective protein family, the NF-kappa-B inhibitory protein family, the protein tyrosine phosphatase family, and the A20 binding protein family at the DNA level; and optionally, selected from TNFAIP3, SOCS1, ZC3H12A, CBLB, FAS, IKZF1, LAG3, PD1, TIM3, BCL2L11, PTPN2, AFF3, AXL, NFE2L1, RARG, UBFD1, CRP, CYLD, GIF, KLF4, NDST1, NLRP1, SCGB1A1, ADCY7, ARIH2, CPT2, LNPEP, NOSIP, NPRL3, TANK, TRAF3, TSC1, ZBTB7B, ZC3H12D, RC3H2, RASA2, FIBP, MED12, TIGIT and BRD4, the expression and/or activity thereof in the cells are reduced.
23. A method according to any one of embodiments 21-22, wherein the gene regulatory system comprises a guiding nucleic acid molecule and an enzyme protein.
24. A method according to embodiment 23, wherein reducing the expression and/or weakening the activity of the family member selected from the activity-dependent neuroprotective protein family, the NF-kappa-B inhibitory protein family, the protein tyrosine phosphatase family, and the A20 binding protein family comprises: introducing into the cell a complex comprising the guide nucleic acid molecule and the enzyme protein, or a complex comprising the guide nucleic acid molecule and the nucleic acid encoding the enzyme protein, an LNP comprising a gRNA and a Cas protein, or an LNP comprising a nucleic acid encoding a gRNA and a Cas protein.
25. The method according to any one of embodiments 23-24, wherein the enzyme protein comprises a Cas protein, a Cas protein homolog, or a functionally active fragment thereof, preferably selected from Cas 9 and Cas 12.
26. A method according to any one of embodiments 23-25, wherein the guide nucleic acid molecule comprises a guide RNA (gRNA).
27. A method according to any one of embodiments 23-26, wherein the guide nucleic acid molecule binds to the sequence of at least one family member selected from the activity-dependent neuroprotective protein family, the NF-kappa-B inhibitory protein family, the protein tyrosine phosphatase family, and the A20 binding protein family.
28. A method according to any one of embodiments 23-27, wherein the guide nucleic acid molecule binds to a sequence consisting of about 15 to about 25 nucleotides upstream of the 5' end of the protospacer adjacent motif (PAM) selected from the following group: AGG, TGG, CGG and GGG, or binds to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, and NTN, wherein N is A, T, C or G, Y is T or C, V is A, C or G, and R is A or G.
29. A method according to any one of embodiments 23-28, wherein the guide nucleic acid molecule binds to a region or a fragment thereof defined by the genomic coordinates shown in Tables 1G-1J or Tables 2G-2J.
30. A method according to any one of embodiments 23-29, wherein the guide nucleic acid molecule binds to at least one region or a fragment thereof selected from the following group: SEQ ID NO: 20523-21766 (ADNP), SEQ ID NO: 22274-22780 (NFKBIA), SEQ ID NO: 23685-24588 (PTPN6), SEQ ID NO: 26918-29246 (TNIP1).
31. The method of any one of embodiments 23-30, wherein the guide nucleic acid molecule comprises a targeting domain comprising SEQ ID NOs: 19279-20522, 29271-29272 (ADNP), SEQ ID NOs: 21767-22273, 29273-29274 (NFKBIA), SEQ ID NOs: 22781-23684, 29275-29276 (PTPN6), SEQ ID NOs: 24589-26917, 29277-29278, 29315-29325 (TNIP1).
32. A method according to any one of embodiments 1-31, wherein the expression of family members selected from the activity-dependent neuroprotective protein family, NF-kappa-B inhibitory protein family, protein tyrosine phosphatase family, and A20 binding protein family is reduced and/or the activity is attenuated compared to cells in which the expression and/or activity of family members selected from the activity-dependent neuroprotective protein family, NF-kappa-B inhibitory protein family, protein tyrosine phosphatase family, and A20 binding protein family is not changed, so that the proportion of cells expressing the target gene in the obtained cells is reduced and/or the expression level of the target gene in individual cells is decreased.
33. A method according to any one of embodiments 1-32, wherein among the cells obtained by reducing the expression and/or weakening the activity of the family members selected from the activity-dependent neuroprotective protein family, NF-kappa-B inhibitory protein family, protein tyrosine phosphatase family, and A20 binding protein family, the proportion of cells expressing the target gene is less than about 95%.
34. A cell obtained by the method of any one of embodiments 1-33.
35. A pharmaceutical composition comprising the cell of embodiment 34, and optionally a pharmaceutically acceptable carrier.
36. A method of influencing cell growth, comprising administering the cell of embodiment 34 and/or the pharmaceutical composition of embodiment 35.
37. Use of the cells described in embodiment 34 and/or the pharmaceutical composition described in embodiment 35 in the preparation of a drug for preventing and/or treating a disease and/or symptom.
38. A drug for preventing and/or treating a disease and/or symptom, comprising administrating the cells described in Embodiment 34 and/or the pharmaceutical composition described in Embodiment 35 as active ingredients.
39. A method for preventing and/or treating a disease and/or a symptom, comprising administering the cell of embodiment 34 and/or the pharmaceutical composition of embodiment 35 to a subject in need.
40. The cell described in embodiment 34 and/or the pharmaceutical composition described in embodiment 35, which is used for preventing and/or treating diseases and/or symptoms.
41. The use according to embodiment 37, the medicament according to embodiment 38, the method according to embodiment 39, and/or the cell and/or the pharmaceutical composition for use according to embodiment 40, wherein the disease and/or symptom comprises a tumor.
42. The use according to embodiment 37, the medicament according to embodiment 38, the method according to embodiment 39, and/or the cell and/or the pharmaceutical composition for use according to embodiment 40, wherein the disease and/or condition comprises a solid tumor.
43. The use according to embodiment 37, the medicament according to embodiment 38, the method according to embodiment 39, and/or the cell and/or the pharmaceutical composition for use according to embodiment 40, wherein the disease and/or symptom comprises one or more selected from the following group: melanoma, ovarian cancer, cervical cancer, lung cancer, bladder cancer, breast cancer, head and neck cancer, pancreatic cancer, liver cancer, gastric cancer, colorectal cancer and kidney cancer.

The present invention provides a method for reducing the expression and/or attenuating the activity of at least one family member selected from activity-dependent neuroprotective protein family, the NF-kappa-B inhibitory protein family, the protein tyrosine phosphatase family, and the A20 binding protein family in the cells and/or a functionally active fragment thereof.

In one aspect, the present invention provides a method for culturing cells, wherein the expression of at least one family member selected from the activity-dependent neuroprotective protein family, the NF-kappa-B inhibitory protein family, the protein tyrosine phosphatase family, and the A20 binding protein family and/or its functionally active fragment is reduced and/or the activity is attenuated.

For example, the cell can further comprise reduced expression and/or reduced activity of a protein, optionally selected from the group consisting of TNFAIP3, SOCS1, ZC3H12A, CBLB, FAS, IKZF1, LAG3, PD1, TIM3, BCL2L11, PTPN2, AFF3, AXL, NFE2L1, RARG, UBFD1, CRP, CYLD, GIF, KLF4, NDST1, NLRP1, SCGB1A1, ADCY7, ARIH2, CPT2, LNPEP, NOSIP, NPRL3, TANK, TRAF3, TSC1, ZBTB7B, ZC3H12D, RC3H2, RASA2, FIBP, MED12, TIGIT, and BRD4.

For example, the activity-dependent neuroprotective protein family member may comprise a zinc finger domain. For example, the activity-dependent neuroprotective protein family member may comprise ADNP.

For example, the NF-kappa-B inhibitory protein family member may include an ankyrin repeat domain. For example, the NF-kappa-B inhibitory protein family member may include NFKBIA.

For example, the protein tyrosine phosphatase family member may comprise a Src homolog (SH2) domain. For example, the protein tyrosine phosphatase family member may comprise PTPN6.

For example, the A20 binding protein family member may comprise an A20 binding domain. For example, the A20 binding protein family member may comprise TNIP1.

For example, the target gene of the present invention may be a gene encoding at least one family member selected from the activity-dependent neuroprotective protein family, the NF-kappa-B inhibitory protein family, the protein tyrosine phosphatase family, and the A20 binding protein family, and/or a functionally active fragment thereof. For example, compared with cells in which the expression and/or activity of the target gene is not changed, cells obtained by reducing the expression and/or weakening the activity of at least one target gene of the cell may show improved cell characteristics. In one embodiment, cells in which the expression and/or activity of the target gene is not changed may refer to cells derived from the same donor and in which the expression and/or activity of at least one target gene of the cell has not been reduced and/or weakened. In one embodiment, cells in which the expression and/or activity of the target gene is not changed may refer to cells derived from the same donor and whose expression and/or activity of other genes other than the target gene of the cells have not been reduced (for example, knocking out the other gene has substantially no effect on cell function).

In one embodiment, the corresponding cells that have not reduced the expression and/or weakened the activity of at least one target gene of the cell may refer to cells isolated in the same manner from the same donor and have not reduced the expression and/or weakened the activity of at least one target gene of the cell. In one embodiment, the corresponding cells that have not reduced the expression and/or weakened the activity of at least one target gene of the cell may refer to cells from the same tumor source of the same donor and have not reduced the expression and/or weakened the activity of at least one target gene of the cell. In one embodiment, the corresponding cells that have not reduced the expression and/or weakened the activity of at least one target gene of the cell may refer to dividing cells from the same tumor source of the same donor into two groups, wherein one group of cells that have not reduced the expression and/or weakened the activity of at least one target gene of the cell may be the corresponding cells that have not reduced the expression and/or weakened the activity of at least one target gene of the cell. For example, the reduced expression and/or weakened activity of at least one target gene may mean that the target gene in a natural cell is in an expression state to a certain extent, and after the treatment of the present invention, the expression level of the target gene in the cell can be reduced, that is, the reduced expression level of the target gene can be such that the natural cell changes from expressing the target gene to basically not expressing the target gene or the amount of expression of the target gene is reduced.

For example, the cell comprises an immune cell. For example, the cell comprises an immune effector cell. For example, the cell comprises an immune effector T cell, an immune effector NK cell, an immune effector NKT cell. For example, the cell comprises a phagocyte, a lymphocyte, a neutrophil, an eosinophil and/or a basophil.

For example, the cells comprise monocytes, macrophages and/or dendritic cells.

For example, the cells of the present invention also include cells derived from differentiation of stem cells. For example, the cells of the present invention also include cells derived from differentiation of pluripotent stem cells. For example, obtaining the stem cells of present invention may be produced by induction. For example, the stem cells of the present invention may include induced pluripotent stem cells (iPSCs), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from reproductive tissue of a fetus. Exemplary pluripotent stem cells can also be generated from somatic cells by reprogramming them to a pluripotent state through the expression of certain transcription factors associated with pluripotency; these cells are referred to as "induced pluripotent stem cells" or "iPSCs."

For example, the cell comprises B cells, T cells, natural killer cells and/or natural killer-like T cells (NKT). For example, "unmodified cells" or "unmodified cells" may refer to cells or cell colonies in which the genome is not modified and does not comprise a gene regulatory system or comprises a control gene regulatory system (e.g., an empty vector control, a non-targeted gRNA, an interfering siRNA, etc.). For example, the cell comprises αβ T cells and/or γδ T cells. For example, the cell comprises tumor infiltrating lymphocytes (TIL). For example, the TIL is derived from tumor tissue, tumor-associated lymph nodes with or without tumor metastasis, tumor metastasis lesions, fragments of para cancerous tissue, pleural effusion and/or peritoneal effusion TIL and/or TIL recovered after cryopreservation.

For example, the TILs of the present invention can be TILs derived from tumor tissue, tumor-associated lymph nodes with or without tumor metastasis, tumor metastatic lesions, fragments of paracancerous tissue, pleural effusions and/or peritoneal effusions, and/or TILs derived from recovery after cryopreservation. For example, the TILs of the present invention can be obtained by processing tumor tissue into tumor fragments. For example, the volume of the tumor fragments of the present invention is about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cell comprises an engineered immune receptor displayed on the cell surface. For example, the engineered immune receptor specifically binds to an antigen expressed on a target cell. For example, the cell comprises a chimeric antigen receptor and/or a T cell receptor.

In one aspect, the present invention provides a method for culturing tumor infiltrating lymphocytes (TIL), which may include: reducing the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TIL.

For example, TILs derived from tumor tissue, tumor-associated lymph nodes with or without tumor metastasis, tumor metastatic lesions, fragments of paracancerous tissue, pleural effusion and/or peritoneal effusion and not expanded in vitro can be subjected to at least one stage of in vitro expansion, wherein, in at least one stage of the in vitro expansion, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced in the TILs.

For example, the TILs derived from tumor tissue, tumor-associated lymph nodes with or without tumor metastasis, tumor metastasis lesions, fragments of paracancerous tissue, pleural effusion and/or peritoneal effusion of the present invention and not expanded in vitro can be subjected to a first stage of in vitro expansion and a second stage of in vitro expansion, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced in the TILs. For example, the TILs derived from tumor tissue, tumor-associated lymph nodes with or without tumor metastasis, tumor metastasis lesions, fragments of paracancerous tissue, pleural effusion and/or peritoneal effusion of the present invention and not expanded in vitro can be subjected to a first stage of in vitro expansion and a second stage of in vitro expansion, and in the first stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family in the TIL and/or its functionally active fragments can be reduced.

For example, the TILs of the present invention that are derived from tumor tissue, tumor-associated lymph nodes with or without tumor metastasis, tumor metastatic lesions, fragments of paracancerous tissue, pleural effusion and/or peritoneal effusion and have not been expanded in vitro can be subjected to a first stage of in vitro expansion and a second stage of in vitro expansion, and in the first stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage in vitro expansion, a second stage in vitro expansion and a third stage in vitro expansion, and in the first stage in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced.

For example, the TILs derived from tumor tissue, tumor-associated lymph nodes with or without tumor metastasis, tumor metastatic lesions, fragments of paracancerous tissue, pleural effusion and/or peritoneal effusion of the present invention and not expanded in vitro can be subjected to a first stage of in vitro expansion, a second stage of in vitro expansion and a third stage of in vitro expansion, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the third stage in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the first stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced in the TILs.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the first stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family in the TILs and/or its functionally active fragments can be reduced, and in the third stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family in the TILs can be reduced.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the TILs can be reduced, and in the third stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced in the TILs.

For example, the TILs of the present invention derived from tumor tissue, tumor-associated lymph nodes with or without tumor metastasis, tumor metastatic lesions, fragments of paracancerous tissue, pleural effusion and/or peritoneal effusion and not expanded in vitro can be subjected to a first stage of in vitro expansion, a second stage of in vitro expansion and a third stage of in vitro expansion, and in the first stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced, and in the second stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family in the TIL and/or its functionally active fragments can be reduced, and in the third stage of in vitro expansion of the present invention, the expression and/or activity of at least one family member selected from the activity-dependent neuroprotective protein family, the NF-kappa-B inhibitory protein family, the protein tyrosine phosphatase family, and the A20 binding protein family and/or its functionally active fragments can be reduced.

For example, each stage of in vitro expansion can be divided by the change of TIL cell number, for example, when the number of TIL cells increases by at least about 1 time, it can be considered that TIL cells have entered the next stage of in vitro expansion. In some embodiments, when the number of TIL cells increases by at least about 1-1000 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, at least about 50 times, at least about 100 times, at least about 200 times, at least about 500 times, or at least about 1000 times, it can be considered that TIL cells have entered the next stage of in vitro expansion. For example, each stage of in vitro expansion can also be divided by the change of the conditions of TIL cell culture. For example, when cell activators and/or cell growth factors are added or supplemented to the cell culture medium, it can be considered that the TIL cells have entered the next stage of in vitro expansion. For example, when IL-2 is added or supplemented to the cell culture medium, it can be considered that the TIL cells have entered the next stage of in vitro expansion. For example, when one or more gene regulatory systems are added or supplemented to the cell culture medium, it can be considered that the TIL cells have entered the next stage of in vitro expansion. For example, when feeder cells are added or supplemented to the cell culture medium, it can be considered that the TIL cells have entered the next stage of in vitro expansion. For example, after the TIL cells are centrifuged and/or cell washed, it can be considered that the TIL cells have entered the next stage of in vitro expansion. For example, each stage can also be divided by the number of days of TIL cell culture. For example, after TIL cells are cultured in vitro for about 1-100 days, such as about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, the TIL cells can be considered to have entered the next stage of in vitro expansion.

For example, the reducing the expression and/or weakening the activity of the activity-dependent neuroprotective protein family member in the cell comprises inhibiting the DNA binding function of the zinc finger structure.

For example, the reducing the expression and/or weakening the activity of the NF-kappa-B inhibitory protein family member in the cell comprises inhibiting the function of binding to NF-kappa-B/REL.

For example, the reducing the expression and/or weakening the activity of a protein tyrosine phosphatase family member in the cell comprises inhibiting the function of tyrosine phosphatase.

For example, the reducing the expression and/or weakening the activity of the A20 binding protein family member in the cell comprises inhibiting the binding function to A20.

For example, compared to cells in which the expression and/or activity of at least one family member selected from the activity-dependent neuroprotective protein family, the NF-kappa-B inhibitory protein family, the protein tyrosine phosphatase family, and the A20 binding protein family is not changed, cells obtained by reducing the expression and/or attenuating the activity of at least one family member selected from the activity-dependent neuroprotective protein family, the NF-kappa-B inhibitory protein family, the protein tyrosine phosphatase family, and the A20 binding protein family show improved cell properties.

For example, the improved cell properties include one or more selected from the following group: improved cell proliferation ability (i.e., cell number), increased proportion of live cells, improved cell subpopulation proportions, enhanced cytokine secretion ability, enhanced in vitro tumor cell killing ability, and enhanced in vivo tumor killing ability.

For example, the improved cell subpopulation ratio comprises one or more selected from the following group: an increased ratio of activated cells, a decreased ratio of regulatory cells, a decreased ratio of exhausted cells, an increased ratio of central memory cells and/or naive cells, a decreased ratio of apoptotic cells, and an increased ratio of stem-like cells.

For example, the improved cell number of the present invention refers to that compared to cells whose expression and/or activity of at least one family member selected from the activity-dependent neuroprotective protein family, the NF-kappa-B inhibitory protein family, the protein tyrosine phosphatase family, and the A20 binding protein family is not changed, the number of cells of the cells of the present invention in which the expression and/or the activity of at least one family member selected from the activity-dependent neuroprotective protein family, the NF-kappa-B inhibitory protein family, the protein tyrosine phosphatase family, and the A20 binding protein family is reduced in at least one in vitro expansion stage can be increased by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the increased proportion of live cells can be expressed as an increase in cell survival rate. For example, the increased proportion of live cells in the present invention can mean that the proportion of live cells of the present invention in which the expression and/or activity of at least one family member selected from the activity-dependent neuroprotective protein family, the NF-kappa-B inhibitory protein family, the protein tyrosine phosphatase family, and the A20 binding protein family is reduced and/or the activity is weakened in at least one in vitro expansion stage can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the improved cytokine secretion capacity of the present invention may refer to the improved secretion capacity of the cell of a cytokine selected from the following group: IL-2, IL-6, CD107a, GZMB, TNF-α and IFN-γ. For example, the improved cytokine secretion capacity of the present invention may refer to that compared to cells whose expression and/or activity of at least one family member selected from the activity-dependent neuroprotective protein family, the NF-kappa-B inhibitory protein family, the protein tyrosine phosphatase family, and the A20 binding protein family is not changed, the proportion of cells secreting cytokines of the cells of the present invention in which the expression and/or the activity of at least one family member selected from the activity-dependent neuroprotective protein family, the NF-kappa-B inhibitory protein family, the protein tyrosine phosphatase family, and the A20 binding protein family is reduced in at least one in vitro expansion stage can be increased by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved cytokine secretion capacity of the present invention may mean that the proportion of cells secreting cytokines in the cells of the present invention in which the expression and/or activity of at least one family member selected from the activity-dependent neuroprotective protein family, the NF-kappa-B inhibitory protein family, the protein tyrosine phosphatase family, and the A20 binding protein family is reduced and/or the activity is attenuated in at least one in vitro expansion stage can be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the cytokine secretion capacity of the cells of the present invention is determined by flow cytometry or CBA (Cytometric Bead Array).

For example, the improved in vitro tumor cell killing ability and/or improved in vivo tumor killing ability of the present invention may refer to that compared to cells whose expression and/or activity of at least one family member selected from the activity-dependent neuroprotective protein family, the NF-kappa-B inhibitory protein family, the protein tyrosine phosphatase family, and the A20 binding protein family is not changed, the tumor cell killing rate of the cells of the present invention in which the expression and/or the activity of at least one family member selected from the activity-dependent neuroprotective protein family, the NF-kappa-B inhibitory protein family, the protein tyrosine phosphatase family, and the A20 binding protein family is reduced in at least one in vitro expansion stage can be increased by at least about 1-50 times, for example, at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved in vitro tumor cell killing ability and/or improved in vivo tumor killing ability of the present invention may mean that the tumor cell killing rate of the cells of the present invention in which the expression and/or activity of at least one family member selected from the activity-dependent neuroprotective protein family, the NF-kappa-B inhibitory protein family, the protein tyrosine phosphatase family, and the A20 binding protein family is reduced in at least one in vitro expansion stage can be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the killing rate of the cells of the present invention can be measured by the IncuCyte system or CFSE and DAPI staining. For example, tumor cell killing by a cell of the invention may refer to the ability of a cell to kill solid tumor cells.

For example, the cell subpopulation ratio improved by the present invention may include one or more selected from the following group: increased proportion of CD8⁺ cells, increased proportion of central memory cells and/or naive cells, decreased proportion of regulatory cells, increased proportion of activated cells, increased proportion of tumor-specific cells (with CD103⁺ CD39⁺ phenotype), increased proportion of stem-like cells, reduced proportion of exhausted cells, reduced proportion of apoptotic cells.

For example, an increase in to ratio of CD8⁺ cells may be an increase in the ratio of CD8 positive cells in the cells. The proportion of CD8⁺ cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of activated cells increased by the present invention can be an increase in the ratio of CD28⁺, CD25⁺ and/or 41BB⁺ cells in the cells. For example, the proportion of activated cells in cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1 %, at least about 0.9%, at least about 0.8%, at least about 0.7%, at least about 0.6%, at least about 0.5%, at least about 0.4%, at least about 0.3%, at least about 0.2%, or at least about 0.1%, or can be increased by at least about 1-50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the reduced proportion of exhausted cells in the present invention may be an increase in the ratio of PD-1⁺, LAG-3⁺, TIM-3⁺, CD39⁺, CD38⁺ and/or CD101⁺ cells in the cells. For example, the proportion of exhausted cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1 %, at least about 0.9%, at least about 0.8%, at least about 0.7%, at least about 0.6%, at least about 0.5%, at least about 0.4%, at least about 0.3%, at least about 0.2%, or at least about 0.1%, or can be reduced by at least about 1-50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the proportion of regulatory cells decreased by the present invention can be a decrease in the ratio of CD4⁺ CD25⁺ Foxp3⁺ cells in the cells. For example, the proportion of regulatory cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of apoptotic cells reduced by the present invention can be a decrease in the ratio of AnnexinV⁺7-AAD⁺ cells and/or Annexin V⁺7-AAD⁻ cells in the cells. For example, the proportion of apoptotic cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the proportion of cells with stemness increased by the present invention can be an increase in the ratio of CD69 ⁻ CD39 ⁻ cells and/or TCF1⁺ cells in the cells. For example, the proportion of cells having stemness in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased central memory cell ratio of the present invention may be an increase in the ratio of CD45RA ⁻ CCR7⁺ CD45RO⁺ CD62L⁺ cells in the cells. For example, the proportion of central memory cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the ratio of naive T cells increased by the present invention can be an increase in the ratio of CD45RO ⁻ CD62L⁺ cells in the cells. For example, the proportion of immature cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the culture method of the present invention may include a gene editing step for cells. For example, it includes: subjecting the cells to at least one stage of in vitro expansion, wherein, during at least one stage of in vitro expansion, a gene regulatory system may be introduced into the cells.

For example, the gene regulatory system can disrupt the target gene at the DNA level. For example, the gene regulatory system can disrupt the region or fragment of the target gene in the genome of the cell. For example, after using the gene regulatory system, the DNA region or fragment where the target gene is located in the cell is cleaved and the expression ability of the target gene is reduced or the activity of the target gene is inhibited. For example, the editing effect of the gene regulatory system on the target gene can be long-term and continuous. For example, in the cells of the present invention, the activity of at least one family member selected from the activity-dependent neuroprotective protein family, the NF-kappa-B inhibitory protein family, the protein tyrosine phosphatase family, and the A20 binding protein family is inhibited.

The genomic region described in the present invention is determined based on the human reference genome version hg38.

For example, the activity of ADNP in the cells of the present invention is inhibited. For example, the preferred subregions of ADNP shown in Table 1G of the present invention are knocked out and/or inhibited.

For example, the activity of NFKBIA is inhibited in the cells of the present invention. For example, the preferred subregions of NFKBIA in the cells shown in Table 1H of the present invention are knocked out and/or inhibited.

For example, the activity of PTPN6 in the cells of the present invention is inhibited. For example, the preferred subregions of PTPN6 in the cells shown in Table 1I of the present invention are knocked out and/or inhibited.

For example, the activity of TNIP1 in the cells of the present invention is inhibited. For example, the preferred subregions of TNIP1 in the cells shown in Table 1J of the present invention are knocked out and/or inhibited.

For example, the gene regulation system may include a guide nucleic acid molecule and an enzyme protein. For example, the enzyme protein may have a nuclease activity, and the guide nucleic acid molecule may guide the enzyme protein to specifically cleave the region or fragment thereof where the target gene is located. For example, the guide nucleic acid molecule and the enzyme protein may exist in the form of a ribonucleoprotein complex (RNP) or exist independently of each other. For example, the enzyme protein may include a Cas protein. For example, a polynucleotide encoding a gRNA and a Cas protein may be introduced or independently introduced into a target cell.

For example, the present invention reduces the expression and/or weakens the activity of at least one target gene of a cell, and may include: introducing a ribonucleoprotein complex (RNP) comprising the guide nucleic acid molecule and the enzyme protein into the cell. For example, the enzyme protein may include a Cas protein, a Cas protein homolog, or a functionally active fragment thereof. For example, the guide nucleic acid molecule may include a guide RNA (gRNA). For example, a complex comprising a polynucleotide encoding a gRNA and a Cas protein may be introduced into the cell. For example, a complex comprising a gRNA and a Cas protein may be introduced into the cell.

For example, the gRNA can be used to bind to the sequence of the target gene. For example, the binding of the gRNA to the sequence of the target gene can be completely complementary, partially complementary, or hybridize to the sequence of the target gene under moderate or stringent conditions. For example, the binding of the gRNA to the sequence of the target gene can make the CRISPR system of the gRNA to specifically cut the target gene.

For example, the editing target region of the present invention may be a region before the start codon. For example, the editing target region of the present invention may be a region with high transcription factor binding ability. For example, the editing target region of the present invention may be a region with a specific number of transcription factor binding numbers. For example, the editing target region of the present invention may be a continuous region with about 3 or more transcription factor binding numbers. For example, the genomic coordinates of the editing target region of the present invention may be selected from the preferred targeting subregions shown in Tables 1G to 1J.

For example, the ADNP-targeting guide nucleic acid molecule of the present invention can bind to a region or a fragment thereof selected from the group consisting of SEQ ID NOs: 20523-21766.

For example, the guide nucleic acid molecule targeting NFKBIA of the present invention can bind to a region or a fragment thereof selected from the group consisting of SEQ ID NOs: 22274-22780.

For example, the guide nucleic acid molecule targeting PTPN6 of the present invention can bind to a region or a fragment thereof selected from the group consisting of SEQ ID NOs: 23685-24588.

For example, the guide nucleic acid molecule targeting TNIP1 of the present invention can bind to a region or a fragment thereof selected from the group consisting of SEQ ID NOs: 26918-29246.

For example, when the gene editing system includes CRISPR/Cas9, the region targeted by the guide nucleic acid molecule of the present invention may have a protospacer adjacent motif (PAM) downstream, and the protospacer adjacent motif (PAM) may be AGG, TGG, GGG or CGG. For example, when the PAM region of the target gene is determined, a person skilled in the art can easily determine a target sequence consisting of about 15 to about 25 (e.g., about 15, about 16, about 17, about 18, about 19, about 20, about 21, about 22, about 23, about 24, about 25) nucleotides upstream of the 5' end of the PAM of the target gene, and a suitable gRNA can be designed for the target sequence. For example, the guide nucleic acid molecule can bind to a sequence consisting of about 15 to about 25 nucleotides upstream of the 5' end of the protospacer adjacent motif (PAM) selected from the following group: AGG, TGG, GGG and CGG.

For example, when the gene editing system includes CRISPR/Cas12, the region targeted by the guide nucleic acid molecule of the present invention may have a protospacer adjacent motif (PAM) upstream, and the protospacer adjacent motif (PAM) may be NTTN, TTYN, VTTV, TRTV, TTTV, TATV, TYCV, TNN, or NTN, wherein N is A, T, C or G, Y is T or C, V is A, C or G, and R is A or G. For example, when the PAM region of the target gene is determined, those skilled in the art can easily determine a target sequence consisting of about 15 to about 25 (e.g., about 15, about 16, about 17, about 18, about 19, about 20, about 21, about 22, about 23, about 24, about 25) nucleotides downstream of the 3' end of the PAM of the target gene, and can design a suitable gRNA for the target sequence. For example, the guide nucleic acid molecule can bind to a sequence consisting of about 15 to about 25 nucleotides downstream of the 3' end of the protospacer adjacent motif (PAM) selected from the following group: NTTN, TTYN, VTTV, TRTV, TTTV, TATV, TYCV, TNN, or NTN, wherein N is A, T, C or G, Y is T or C, V is A, C or G, and R is A or G.

For example, when the gene editing system of the present invention comprises wild-type Cas12A (also referred to as Cpf1, such as AsCas12A, FnCas12A, LbCas12A, BbCas12A, CMaCas12A and OsCas12A), the region targeted by the guide nucleic acid molecule of the present invention may have a PAM sequence selected from the following upstream: NTTN, wherein N may be A, T, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as enAsCas12A (mutation sites E174R, S542R and K548R), the upstream of the region targeted by the guide nucleic acid molecule of the present invention may have a PAM sequence selected from the following: TTYN (TTTN/TTCN), VTTV (ATTV/CTTV/GTTV), or TRTV (TATV/TGTV), wherein N may be A, T, C or G, Y may be T or C, V may be A, C or G, R can be A or G. For example, when the PAM region of the target gene is determined, one skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and can design a suitable gRNA for the target sequence.

For example, when the gene editing system of the present invention comprises a mutant Cas12A, such as opAsCas12A (mutation sites: E174R and S542R), the region targeted by the guide nucleic acid molecule of the present invention may have a PAM sequence selected from the following upstream: TTTV (TTTA, TTTC, or TTTG), wherein V may be A, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises a mutant Cas12A, such as AsCas12AUltra (mutation sites: M537R and F870L), the upstream of the region targeted by the guide nucleic acid molecule of the present invention may have a PAM sequence selected from the following: TTTV, TATV, or TYCV, wherein V may be A, C or G, and Y may be T or C. For example, when the PAM region of the target gene is determined, those skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and a suitable gRNA can be designed for the target sequence.

For example, when the gene editing system of the present invention comprises mutant Cas12A, such as hfCas12Max (mutation site: N243R/E336R/D892R) and Cas12Max (mutation site: N243R), the upstream of the region targeted by the guide nucleic acid molecule of the present invention may have a PAM sequence selected from the following: TNN, or NTN, wherein N may be A, T, C or G. For example, when the PAM region of the target gene is determined, a person skilled in the art can easily determine a target sequence consisting of about 17 to about 25 (e.g., about 17, about 18, about 19, about 20, about 21, about 22, about 23, about 24, or about 25) nucleotides downstream of the 3' end of the PAM of the target gene, and an appropriate gRNA can be designed for this target sequence.

For example, the guide nucleic acid molecule may comprise a target sequence consisting of about 15 to about 25 nucleotides before the PAM region represented by AGG, TGG, GGG and/or CGG in the DNA where the gene encoding at least one family member selected from the activity-dependent neuroprotective protein family, the NF-kappa-B inhibitory protein family, the protein tyrosine phosphatase family, and the A20 binding protein family and/or its functionally active fragment is located. For example, the guide nucleic acid molecule may comprise a sequence that can bind to a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20, about 15 to about 19, about 17 to about 19, or about 15 to about 17 nucleotides, before the PAM region represented by AGG, TGG, GGG and/or CGG in the DNA where the gene encoding at least one family member selected from the activity-dependent neuroprotective protein family, the NF-kappa-B inhibitory protein family, the protein tyrosine phosphatase family, and the A20 binding protein family and/or its functionally active fragment is located. For example, the target sequence can be a region defined by the genomic coordinates shown in Tables 2G-2J, or a fragment thereof.

For example, the target sequence of the present invention can be chr5:151029972-151030223, chr5:151030278-151030858, chr5:151035040-151035193, chr5:151035459-151035684, chr5:151039192-151039352, chr5:151042523-151043011, chr5:151045907-151046077, chr5:151049699-151049974, chr5:151056860-151057084, chr5:151060196-151060522.

For example, the guide nucleic acid molecule can include a targeting domain of an sgRNA targeting ADNP as shown in any one of SEQ ID NOs: 19279-20522, 29271-29272, a targeting domain of an sgRNA targeting NFKBIA as shown in any one of SEQ ID NOs: 21767-22273, 29273-29274, a targeting domain of an sgRNA targeting PTPN6 as shown in any one of SEQ ID NOs: 22781-23684, 29275-29276, or the targeting domain of an sgRNA targeting TNIP1 as shown in any one of SEQ ID NOs: 24589-26917, 29277-29278, 29315-29325.

For example, compared to cells in which the expression and/or activity of the target gene is not changed, the proportion of cells expressing the product of the target gene in the cells obtained by reducing the expression and/or weakening the activity of at least one target gene can be reduced and/or the expression level of the target gene in individual cells can be decreased.

For example, in the method of the present invention, the proportion of cells expressing the product of the target gene in the cells obtained by reducing the expression and/or attenuating the activity of at least one target gene in the cells is reduced by at least about 5% compared to cells in which the expression and/or activity of the target gene is not changed. For example, the proportion of cells expressing the product of the gene encoding at least one family member selected from the activity-dependent neuroprotective protein family, the NF-kappa-B inhibitory protein family, the protein tyrosine phosphatase family, the A20 binding protein family, and/or a functionally active fragment thereof is reduced by at least about 100-5%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the product of the gene encoding at least one family member selected from the activity-dependent neuroprotective protein family, the NF-kappa-B inhibitory protein family, the protein tyrosine phosphatase family, the A20 binding protein family, and/or its functionally active fragments can be reduced from a detectable cell proportion to 1%. For example, the proportion of cells expressing the product of the gene encoding at least one family member selected from the activity-dependent neuroprotective protein family, the NF-kappa-B inhibitory protein family, the protein tyrosine phosphatase family, the A20 binding protein family, and/or its functionally active fragments can be reduced to at least about 100-1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the product of the gene encoding at least one family member selected from the activity-dependent neuroprotective protein family, the NF-kappa-B inhibitory protein family, the protein tyrosine phosphatase family, the A20 binding protein family and/or its functionally active fragment can be detected by cell flow cytometry.

For example, in the method of the present invention, in the cells in which the expression of at least one target gene of the cells is reduced and/or the activity is attenuated, and the proportion of cells expressing the product of the gene encoding at least one family member selected from the activity-dependent neuroprotective protein family, the NF-kappa-B inhibitory protein family, the protein tyrosine phosphatase family, and the A20 binding protein family and/or its functionally active fragments can be up to about 95%. For example, the proportion of cells expressing the product of the gene encoding at least one family member selected from the group consisting of activity-dependent neuroprotective protein family, NF-kappa-B inhibitory protein family, protein tyrosine phosphatase family, A20 binding protein family, and/or functionally active fragments thereof can be at most about 95-5%, such as at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing the product of the gene encoding at least one family member selected from the activity-dependent neuroprotective protein family, the NF-kappa-B inhibitory protein family, the protein tyrosine phosphatase family, the A20 binding protein family and/or its functionally active fragments can be detected by flow cytometry.

For example, in the method of the present invention, compared with cells in which the expression and/or activity of at least one target gene of the cell is not changed, the expression level of the target gene in a single cell can be reduced by at least about 5%. For example, the expression level of the target gene in a single cell can be reduced by at least about 100-5%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell can range from a detectable expression level to 1%. For example, the expression level of the target gene in a single cell can be reduced to at least about 100-1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%.

For example, in the method of the present invention, in the cells in which the expression of at least one target gene of the cell is reduced and/or the activity is weakened, the expression amount of the target gene in a single cell can be at most about 95% of the cell whose expression and/or activity of the target gene is not changed. For example, the expression amount of the gene encoding at least one family member selected from the activity-dependent neuroprotective protein family, the NF-kappa-B inhibitory protein family, the protein tyrosine phosphatase family, and the A20 binding protein family and/or its functionally active fragment (e.g., a gene encoding ADNP, NFKBIA, PTPN6, TNIP1) in a single cell can be at most about 95-5%, for example, at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5% of that of the cells in which the expression and/or the activity of the gene encoding at least one family member selected from the activity-dependent neuroprotective protein family, the NF-kappa-B inhibitory protein family, the protein tyrosine phosphatase family, and the A20 binding protein family and/or its functionally active fragment is not altered.

For example, the method of the present invention comprises: subjecting the cells to at least one stage of in vitro expansion, wherein in at least one stage of the in vitro expansion, the expression and/or activity of at least one family member selected from the activity-dependent neuroprotective protein family, the NF-kappa-B inhibitory protein family, the protein tyrosine phosphatase family, and the A20 binding protein family of the cells is reduced.

For example, the TILs derived from tumor tissue, tumor-associated lymph nodes with or without tumor metastasis, tumor metastatic lesions, fragments of paracancerous tissue, pleural effusion and/or peritoneal effusion and not expanded in vitro are subjected to a first stage of in vitro expansion and a second stage of in vitro expansion, and in the second stage of in vitro expansion, the expression of at least one family member selected from the activity-dependent neuroprotective protein family, the NF-kappa-B inhibitory protein family, the protein tyrosine phosphatase family, and the A20 binding protein family of the TILs expanded in vitro in the first stage is reduced and/or the activity is attenuated.

For example, the first stage in vitro expansion is performed for at least about 7 days. For example, the second stage in vitro expansion is performed for at least about 7 days.

For example, in a single stage of in vitro expansion of the present invention, the cell can be contacted with the one or more cell activators and the expression and/or activity of at least one family member selected from the activity-dependent neuroprotective protein family, the NF-kappa-B inhibitory protein family, the protein tyrosine phosphatase family, and the A20 binding protein family and/or its functionally active fragments in the cell can be reduced. For example, the cell activator can include an agonist of one or more targets selected from the following group: CD3, CD28, HVEM, CD40L, OX40 and 4-1BB. For example, in a single stage of in vitro expansion, the expression and/or activity of at least one family member selected from the activity-dependent neuroprotective protein family, the NF-kappa-B inhibitory protein family, the protein tyrosine phosphatase family, and the A20 binding protein family of the cell of the present invention is reduced and/or the activity is weakened and the cell is contacted with one or more cell activators of the present invention. For example, in the first stage of in vitro expansion of the present invention, the expression and/or activity of at least one family member selected from activity-dependent neuroprotective protein family, the NF-kappa-B inhibitory protein family, the protein tyrosine phosphatase family, and the A20 binding protein family of the TIL of the present invention can be reduced and/or the activity is attenuated, and the TIL is contacted with one or more cell activators of the present invention. For example, in the second stage of in vitro expansion of the present invention, the expression of at least one family member selected from the activity-dependent neuroprotective protein family, the NF-kappa-B inhibitory protein family, the protein tyrosine phosphatase family, and the A20 binding protein family of the TIL of the present invention can be reduced and/or the activity is weakened, and the TIL is contacted with one or more cell activators of the present invention. For example, in the third stage of in vitro expansion of the present invention, the expression of at least one family member selected from the activity-dependent neuroprotective protein family, the NF-kappa-B inhibitory protein family, the protein tyrosine phosphatase family, and the A20 binding protein family of the TIL of the present invention can be reduced and/or the activity is weakened, and the TIL is contacted with one or more cell activators of the present invention.

For example, in a single stage of in vitro expansion, the cells of the present invention substantially simultaneously reduce the expression and/or weaken the activity of at least one family member selected from the activity-dependent neuroprotective protein family, the NF-kappa-B inhibitory protein family, the protein tyrosine phosphatase family, and the A20 binding protein family, and contact with one or more cell activators of the present invention. For example, in a single stage of in vitro expansion, the cells of the present invention first reduce the expression and/or weaken the activity of at least one family member selected from the activity-dependent neuroprotective protein family, the NF-kappa-B inhibitory protein family, the protein tyrosine phosphatase family, and the A20 binding protein family, for example, 2-48 hours in advance, for example, 2 hours in advance, 4 hours in advance, 8 hours in advance, 12 hours in advance, 24 hours in advance, or 48 hours in advance, and then contact with one or more cell activators of the present invention. For example, in a single stage of in vitro expansion, the cells of the present invention are first contacted with one or more cell activators of the present invention, for example, 2-48 hours in advance, such as 2 hours in advance, 4 hours in advance, 8 hours in advance, 12 hours in advance, 24 hours in advance, or 48 hours in advance, etc., and then the expression and/or activity of at least one family member selected from the activity-dependent neuroprotective protein family, the NF-kappa-B inhibitory protein family, the protein tyrosine phosphatase family, and the A20 binding protein family is reduced.

For example, in the first stage of in vitro expansion of the present invention, the TIL of the present invention substantially simultaneously reduces the expression and/or weakens the activity of at least one family member selected from the activity-dependent neuroprotective protein family, the NF-kappa-B inhibitory protein family, the protein tyrosine phosphatase family, and the A20 binding protein family, and contacts with one or more cell activators of the present invention. For example, in the second stage of in vitro expansion of the present invention, the TIL of the present invention substantially simultaneously reduces the expression and/or weakens the activity of at least one family member selected from the activity-dependent neuroprotective protein family, the NF-kappa-B inhibitory protein family, the protein tyrosine phosphatase family, and the A20 binding protein family, and contacts with one or more cell activators of the present invention. For example, in the third stage of in vitro expansion of the present invention, the TIL of the present invention substantially simultaneously reduces the expression of members of at least one family selected from the family of activity-dependent neuroprotective proteins, NF-kappa-B inhibitory proteins, protein tyrosine phosphatases, A20 binding proteins, and is contacted with one or more cell activators of the present invention.

### TIL Cell Culture

For example, the second stage in vitro expansion of the present invention is carried out for at least about 7 days. For example, the second stage in vitro expansion of the present invention can be carried out for at least about 7 days, at least about 8 days, at least about 9 days, at least about 10 days, at least about 11 days, at least about 12 days, at least about 13 days, or at least about 14 days. For example, the second stage in vitro expansion of the present invention can be carried out for about 7 days to about 14 days, preferably about 9 days to about 14 days, for example, the second stage in vitro expansion of the present invention can be carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage in vitro expansion of the present invention can be considered as the REP (rapid expansion protocol) stage. For example, the first stage of in vitro expansion of the present invention can be considered as the preREP stage.

For example, the first stage in vitro expansion of the present invention is carried out for at least about 7 days. For example, the first stage in vitro expansion of the present invention can be carried out for at least about 7 days, at least about 8 days, at least about 9 days, at least about 10 days, at least about 11 days, at least about 12 days, at least about 13 days, or at least about 14 days. For example, the first stage in vitro expansion of the present invention can be carried out for about 7 days to about 14 days, preferably about 9 days to about 14 days, for example, the second stage in vitro expansion of the present invention can be carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days.

For example, the number of days that the second stage in vitro expansion of the present invention is performed can be calculated from the start time of the second stage in vitro expansion. For example, when the second stage in vitro expansion starts, it can be considered that the second stage in vitro expansion has been performed for about 0 hours. For example, about 24 hours after the start of the second stage in vitro expansion, it can be considered that the second stage in vitro expansion has been performed for about 1 day. For example, the day when the second stage in vitro expansion starts can be considered that the second stage in vitro expansion has been performed for about 0 days. For example, the number of days that the second stage in vitro expansion of the present invention is performed can be calculated by the number of days that the second stage in vitro expansion is performed. For example, the day after the start of the second stage in vitro expansion, it can be considered that the second stage in vitro expansion has been performed for about 1 day.

For example, the cell activator of the present invention may include one or more selected from the following group: CD80, CD86, B7-H3, 4-1BBL, CD27, CD30, CD134, B7h, CD40, LIGHT, and their functionally active fragments. For example, the cell activator of the present invention may include an agonist of one or more targets selected from the following group: CD3, CD28, HVEM, CD40L, OX40 and 4-1BB. For example, the cell activator of the present invention may include an antibody selected from the following group: CD3, CD28, HVEM, CD40L, OX40 and 4-1BB and their antigen-binding fragments. For example, the cell activator of the present invention may include a CD3 agonist. For example, the cell activator of the present invention may include an anti-CD3 antibody and/or an antigen-binding fragment thereof, such as OKT3 of Miltenyi Biotech, and SP34 of BD. For example, the cell activator of the present invention may include a CD28 agonist. For example, the cell activator of the present invention may comprise an anti-CD28 antibody and/or an antigen-binding fragment thereof, such as 15E8 from Merck.

For example, the cell activator of the present invention may comprise an anti-CD3 antibody and/or an antigen-binding fragment thereof, for example, it may comprise the light chain VL and heavy chain VH of Miltenyi Biotech's OKT3, or it may comprise the light chain VL and heavy chain VH of BD's SP34. For example, the cell activator of the present invention may comprise a CD28 agonist. For example, the cell activator of the present invention may comprise an anti-CD28 antibody and/or an antigen-binding fragment thereof, for example, it may comprise the light chain VL and heavy chain VH of Merck's 15E8. For example, the cell activator of the present invention may comprise an anti-CD3 antibody and/or an antigen-binding fragment thereof, for example, it may comprise the light chain LCDR1-3 and heavy chain HCDR1-3 of Miltenyi Biotech's OKT3, may include light chain LCDR1-3 and heavy chain HCDR1-3 of SP34 of BD, and the anti-CD3 antibody and/or its antigen-binding fragment of the present invention may have CD3 binding ability. For example, the cell activator of the present invention may include a CD28 agonist. For example, the cell activator of the present invention may include an anti-CD28 antibody and/or an antigen-binding fragment thereof, such as light chain LCDR1-3 and heavy chain HCDR1-3 of 15E8 of Merck, and the anti-CD28 antibody and/or its antigen-binding fragment of the present invention may have CD28 binding ability. In the present invention, the antibody of the present invention or its antigen-binding protein comprises at least one CDR in the heavy chain variable region VH of the antibody and/or at least one CDR in the light chain variable region VL of the antibody. The CDR of the present invention may be defined according to the IMGT nomenclature, the CDR of the present invention may be defined according to Chothia, or the CDR of the present invention may be defined according to Kabat.

For example, contacting the cells of the present invention with one or more cell activators of the present invention may include one or more methods selected from the following group: (1) adding the cell activator of the present invention to the cell culture medium of the present invention; (2) adding engineered cells expressing the cell activator of the present invention to the cell culture medium of the present invention; (3) adding a solid phase medium containing the cell activator of the present invention to the cell culture medium of the present invention. For example, contacting the cells of the present invention with one or more cell activators of the present invention may include adding a solid phase medium containing the cell activator of the present invention to the cell culture medium of the present invention. For example, contacting the cells of the present invention with one or more cell activators of the present invention may include adding a solid phase medium containing the CD28 antibody and the CD3 antibody of the present invention to the cell culture medium of the present invention.

For example, the initial concentration of the cell activator in the cell culture medium of the present invention can be at least about 30 ng/mL. For example, the initial concentration of the CD28 antibody of the present invention in the cell culture medium of the present invention can be at least about 30 ng/mL; for example, the initial concentration of the CD3 antibody of the present invention in the cell culture medium of the present invention can be at least about 30 ng/mL. For example, the selection of the initial concentration of the CD28 antibody of the present invention can be independent of the selection of the initial concentration of the CD3 antibody of the present invention; for example, the initial concentrations of the CD28 antibody of the present invention and the CD3 antibody of the present invention in the cell culture medium of the present invention can be arbitrarily combined. For example, the initial concentration of the CD28 antibody of the present invention in the cell culture medium of the present invention can be arbitrarily selected from about 30 ng/mL to about 300 ng/mL. For example, the initial concentration of the CD3 antibody of the present invention in the cell culture medium of the present invention can be arbitrarily selected from about 30 ng/mL to about 300 ng/mL. For example, the initial concentration of the CD28 antibody of the present invention in the cell culture medium of the present invention can be arbitrarily selected from about 30 ng/mL to about 300 ng/mL, and the initial concentration of the CD3 antibody of the present invention in the cell culture medium of the present invention can be arbitrarily selected from about 30 ng/mL to about 300 ng/mL, and the selection of the initial concentration of the CD28 antibody of the present invention can be independent of the selection of the initial concentration of the CD3 antibody of the present invention. For example, the diameter of the solid phase medium of the present invention can be about 500 nanometers to about 10 microns. For example, the diameter of the solid phase medium of the present invention can be measured by transmission electron microscopy. For example, the diameter of the solid phase medium of the present invention can be about 1 nanometer to about 500 nanometers. For example, the diameter of the solid phase medium of the present invention can be about 100 nanometers to about 500 nanometers. For example, the diameter of the solid phase medium of the present invention can be about 200 nanometers to about 500 nanometers. For example, the diameter of the solid phase medium of the present invention can be measured by transmission electron microscopy.

For example, the solid phase medium of the present invention may comprise a polymer. For example, the solid phase medium of the present invention may comprise dextran.

For example, the solid phase medium of the present invention contains at least about 25 µ g of the cell activating agent of the present invention per mg.

For example, the solid phase medium containing the cell activator of the present invention is added to the cell culture medium of the present invention at a ratio of about 100:1 to about 1:2000, preferably about 1:100 to about 1:2000 of the solid phase medium of the present invention to the cells of the present invention. For example, the solid phase medium containing the cell activator of the present invention is added to the cell culture medium of the present invention at a ratio of about 2:1 to about 1:2 of the solid phase medium of the present invention to the cells of the present invention.

For example, when the diameter of the solid phase medium of the present invention is about 100 nanometers to about 500 nanometers, the solid phase medium containing the cell activator of the present invention can be added to the cell culture medium of the present invention at a ratio of about 2:1 to about 1:2 of the solid phase medium of the present invention to the cells of the present invention. For example, when the diameter of the solid phase medium of the present invention is about 100 nanometers to about 500 nanometers, the solid phase medium containing the cell activator of the present invention, such as a CD3 agonist and/or a CD28 agonist, can be added to the cell culture medium of the present invention at a ratio of about 2:1 to about 1:2, about 2:1 to about 1:1, or about 1:1 to about 1:2 of the solid phase medium of the present invention to the cells of the present invention.

For example, when the diameter of the solid phase medium of the present invention is about 100 nanometers to about 500 nanometers, the solid phase medium containing the cell activator of the present invention can be added to the cell culture medium of the present invention at a ratio of about 1:100 to about 1:2000 of the solid phase medium of the present invention to the cells of the present invention. For example, when the diameter of the solid phase medium of the present invention is about 100 nanometers to about 500 nanometers, at a ratio of about 1:100 to about 1:2000, about 1:200 to about 1:2000, about 1:300 to about 1:2000, about 1:400 to about 1:2000, about 1:500 to about 1:2000, about 1:600 to about 1:2000, about 1:700 to about 1:2000, about 1:800 to about 1:2000, 1:900 to about 1:2000, about 1:1000 to about 1:2000, about 1:1200 to about 1:2000, about 1:1400 to about 1:2000, about 1:1600 to about 1:2000, or about 1:1800 to about 1:2000 of the solid medium of the present invention to the cells of the present invention, for example, a solid medium comprising a CD28 agonist and a CD3 agonist of the present invention can be added to the cell culture medium of the present invention.

For example, the method of the present invention may further comprise: contacting the cells of the present invention with one or more cell growth factors during at least one stage of the in vitro expansion of the present invention.

For example, in a single stage of in vitro expansion of the present invention, the cells of the present invention can be contacted with the cell activator of the present invention and with one or more cell growth factors of the present invention. For example, in the first stage of in vitro expansion of the present invention, the TIL of the present invention can be contacted with the cell activator of the present invention and with one or more cell growth factors of the present invention. For example, in the second stage of in vitro expansion of the present invention, the TIL of the present invention can be contacted with the cell activator of the present invention and with one or more cell growth factors of the present invention. For example, in the third stage of in vitro expansion of the present invention, the TIL of the present invention can be contacted with the cell activator of the present invention and with one or more cell growth factors of the present invention.

For example, in a single stage of in vitro expansion of the present invention, the cells of the present invention are contacted with the cell activator of the present invention and one or more cell growth factors of the present invention at substantially the same time. For example, in a single stage of in vitro expansion of the present invention, the cells of the present invention are contacted with the one or more cell growth factors of the present invention and one or more cell growth factors of the present invention at substantially the same time. For example, in a single stage of in vitro expansion of the present invention, the cells of the present invention may be first contacted with one or more cell growth factors of the present invention, for example, 2-48 hours in advance, for example, 2 hours in advance, 4 hours in advance, 8 hours in advance, 12 hours in advance, 24 hours in advance, or 48 hours in advance, etc., and then contacted with one or more cell activators of the present invention. For example, in a single stage of in vitro expansion of the present invention, the cells of the present invention may be first contacted with one or more cell activators of the present invention, for example, 2-48 hours in advance, for example, 2 hours in advance, 4 hours in advance, 8 hours in advance, 12 hours in advance, 24 hours in advance, or 48 hours in advance, etc., and then contacted with one or more cell growth factors of the present invention.

For example, in the first stage of in vitro expansion of the present invention, the cells of the present invention can be contacted with the cell activator of the present invention and one or more cell growth factors of the present invention at substantially the same time. For example, in the second stage of in vitro expansion of the present invention, the TIL of the present invention can be contacted with the cell activator of the present invention and one or more cell growth factors of the present invention at substantially the same time. For example, in the third stage of in vitro expansion of the present invention, the TIL of the present invention can be contacted with the cell activator of the present invention and one or more cell growth factors of the present invention at substantially the same time.

For example, the cell growth factor of the present invention can be selected from one or more of the following group: IL-2, IL-7, IL-12, IL-15, IL-21, interferon-γ, and their functionally active fragments. For example, the cell growth factor of the present invention can contain IL-2 and/or its functionally active fragments. For example, the functionally active fragments of IL-2 can contain fragments of IL-2 that can bind to the IL-2 receptor of the cell known in the art. For example, the cell growth factor of the present invention can contain IL-2 and/or its functionally active fragments, IL-7 and/or its functionally active fragments, and IL-15 and/or its functionally active fragments.

For example, contacting the cells of the present invention with one or more cell growth factors of the present invention can include adding the cell growth factors of the present invention to the cell culture medium of the present invention. For example, the initial concentration of the cell growth factors of the present invention in the cell culture medium of the present invention can be at least about 300 IU/mL. For example, the initial concentration of the IL-2 of the present invention in the cell culture medium of the present invention can be at least about 300-9000 IU/mL, such as at least about 300 IU/mL, at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

For example, the cells of the present invention can reduce the amount of cytokines when in contact with IL-2, IL-7 and IL-15, relative to when in contact with IL-2 alone. For example, the amount of IL-2 added can be reduced under the condition of adding IL-7 and IL-15. For example, the concentration of IL-7 can be about 1 to 1000ng/mL, preferably about 1-100ng/mL. For example, the concentration of IL-15 can be about 1 to 1000ng/mL, preferably about 1-100ng/mL. For example, for the amount of IL-2 added, it can be reduced to the commonly used range in the art for various immune cells, for example, reduced to 50-10% of the commonly used range in the art, such as 50%, 20% or 10%. For example, for the amount of IL-2 added to TCR-T, the commonly used range in the art can be 30-300IU/mL. For example, for the amount of IL-2 added to TIL, the commonly used range in the art can be 300-9000IU/mL (e.g., 1000-9000IU/mL).

For example, the method of the present invention may further comprise: in at least one stage of the in vitro expansion of the present invention, the cells of the present invention may be co-cultured with feeder cells.

For example, in a single stage of in vitro expansion of the present invention, the cells of the present invention may be contacted with one or more cell activators and/or one or more cell growth factors and co-cultured with the feeder cells of the present invention. For example, a single stage of in vitro expansion of the present invention may refer to in vitro expansion of the present invention at the same stage, for example, in vitro expansion at the first stage of the present invention, in vitro expansion at the second stage of the present invention, or in vitro expansion at the third stage of the present invention, etc..

For example, in the first stage of in vitro expansion of the present invention, the TIL of the present invention can be contacted with one or more cell activators and/or one or more cell growth factors and co-cultured with the feeder cells of the present invention. For example, in the second stage in vitro expansion of the present invention, the TIL of the present invention can be contacted with one or more cell activators and/or one or more cell growth factors of the present invention and co-cultured with the feeder cells of the present invention. For example, in the third stage in vitro expansion of the present invention, the TIL of the present invention can be contacted with one or more cell activators and/or one or more cell growth factors of the present invention and co-cultured with the feeder cells of the present invention.

For example, in a single stage of in vitro expansion of the present invention, the cells of the present invention can be contacted with one or more cell activators and/or one or more cell growth factors of the present invention for a certain period of time, and then co-cultured with the feeder cells of the present invention. For example, in the first stage of in vitro expansion of the present invention, the TIL of the present invention can be contacted with one or more cell activators and/or one or more cell growth factors of the present invention for a certain period of time, and then co-cultured with the feeder cells of the present invention. For example, in the second stage of in vitro expansion of the present invention, the TIL of the present invention can be contacted with one or more cell activators and/or one or more cell growth factors of the present invention for a certain period of time, and then co-cultured with the feeder cells of the present invention. For example, in the third stage of in vitro expansion of the present invention, the TIL of the present invention can be contacted with one or more cell activators and/or one or more cell growth factors of the present invention for a certain period of time, and then co-cultured with the feeder cells of the present invention.

For example, in a single stage of in vitro expansion of the present invention, the cells of the present invention can be contacted with one or more cell activators and/or one or more cell growth factors of the present invention for a certain period of time before being co-cultured with the feeder cells of the present invention. For example, the certain period of time of the present invention can be at least about 1 hour. For example, the certain period of time of the present invention can be at least about 1-72 hours, such as at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours or at least about 72 hours. For example, a certain period of time of the present invention can be about 2 hours to about 72 hours. For example, the certain time of the present invention can be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. For example, the certain time of the present invention can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain time of the present invention can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours or about 72 hours.

For example, the feeder cells of the present invention may include antigen presenting cells. For example, the feeder cells of the present invention may include one or more selected from the following group: peripheral mononuclear cells, dendritic cells, and artificial antigen presenting cells. For example, the feeder cells of the present invention may be peripheral mononuclear cells. For example, the feeder cells of the present invention may be irradiated feeder cells. For example, the feeder cells of the present invention may be isolated artificial antigen presenting cells (aAPCs), and the artificial antigen presenting cells of the present invention may include cells expressing HLA-A/B/C, CD64, CD80, ICOS-L and/or CD58, and may be modified to express one or more cell activators of the invention. For example, the feeder cells of the invention may be irradiated, for example, may be irradiated with gamma rays, or may be irradiated with X rays.

For example, co-culturing the cells of the present invention with the feeder cells of the present invention may comprise contacting the surface of the feeder cells of the present invention with the surface of the cells of the present invention. For example, co-culturing the cells of the present invention with the feeder cells of the present invention comprises adding the feeder cells of the present invention to the cell culture medium of the present invention.

For example, the feeder cells of the present invention can be added to the cell culture medium of the present invention at a ratio of about 40:1 to about 400:1 of the feeder cells of the present invention to the cells of the present invention. For example, the feeder cells of the present invention can be added to the cell culture medium of the present invention at a ratio of about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, at about 70:1 to about 400:1, at about 80:1 to about 400:1, at about 90:1 to about 400:1, at about 100:1 to about 400:1, at about 200:1 to about 400:1, or at about 300:1 to about 400:1 of feeder cells of the invention to cells of the invention.

In another aspect, the present invention provides a method for culturing tumor infiltrating lymphocytes (TIL), which may comprise: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with one or more cell growth factors; wherein, a second TIL population is obtained through step (A); (B) reducing the expression and/or activity of at least one family member selected from the activity-dependent neuroprotective protein family, the NF-kappa-B inhibitory protein family, the protein tyrosine phosphatase family, and the A20 binding protein family in the second TIL population; wherein, a third TIL population is obtained through step (B).

In another aspect, the present invention provides a method for culturing tumor infiltrating lymphocytes (TILs), comprising: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with one or more T cell growth factors, wherein a second TIL population is obtained through step (A); (B) the expression and/or activity of at least one family member selected from the group consisting of activity-dependent neuroprotective protein family, NF-kappa-B inhibitory protein family, protein tyrosine phosphatase family, and A20 binding protein family in the second TIL population is reduced and/or the activity is weakened, and the second TIL population is contacted with a T cell activator and/or a T cell growth factor, wherein a third TIL population is obtained through step (B); and (C) the third TIL population is co-cultured with feeder cells, wherein a fourth TIL population is obtained through step (C).

In one embodiment, the first stage of in vitro expansion of the present invention can be arbitrarily replaced with step (A) in the method of the above aspect. In one embodiment, the second stage of in vitro expansion of the present invention can be arbitrarily replaced with step (B) in the method of the above aspect. In one embodiment, the TIL of the present invention that has been expanded in vitro in the first stage can be arbitrarily replaced with the second TIL group obtained by step (A) in the method of the above aspect. In one embodiment, the TIL of the present invention that has been expanded in vitro in the second stage can be arbitrarily replaced with the third TIL group obtained by step (B) in the method of the above aspect. In one embodiment, if necessary, the third stage in vitro expansion of the present invention can be arbitrarily replaced with any additional step (C) in the method of the above aspect. In one embodiment, if necessary, the TIL of the present invention that has been expanded in vitro in the third stage can be arbitrarily replaced with the fourth TIL group obtained by any additional step (C) in the method of the above aspect.

In another aspect, the present invention provides a method for culturing tumor infiltrating lymphocytes (TIL), which may comprise: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with a plurality of cell growth factors; wherein a second TIL population is obtained through step (A); (B) contacting the second TIL population with a plurality of cell growth factors, with a plurality of cell activators, reducing the expression and/or weakening the activity of at least one family member selected from the activity-dependent neuroprotective protein family, the NF-kappa-B inhibitory protein family, the protein tyrosine phosphatase family, and the A20 binding protein family, and co-culturing the TIL with feeder cells; wherein a third TIL population is obtained through step (B).

In another aspect, the present invention provides a method for culturing tumor infiltrating lymphocytes (TIL), which may include: (A) contacting a first TIL population derived from tumor tissue, tumor-associated lymph nodes with or without tumor metastasis, tumor metastatic lesions, fragments of paracancerous tissue, pleural effusion and/or peritoneal effusion and not expanded in vitro with a cell growth factor; wherein, a second TIL population is obtained through step (A); (B) contacting the second TIL population with a cell growth factor, with a cell activator, reducing the expression and/or weakening the activity of at least one family member selected from the activity-dependent neuroprotective protein family, the NF-kappa-B inhibitory protein family, the protein tyrosine phosphatase family, and the A20 binding protein family, and co-culturing the TIL with feeder cells, wherein at least one family member selected from the activity-dependent neuroprotective protein family, the NF-kappa-B inhibitory protein family, the protein tyrosine phosphatase family, and the A20 binding protein family may include ADNP, NFKBIA, PTPN6, and TNIP1, respectively; wherein, a third TIL population is obtained through step (B).

In another aspect, the present invention provides a method for culturing tumor infiltrating lymphocytes (TIL). The method for obtaining TIL cells from a subject's tissue sample can be to obtain an in situ tumor sample or a metastatic tumor sample from a patient during surgery, the weight of which can be at least about 1g, or multiple pieces of tissue can be combined. Tumor tissue, tumor-associated lymph nodes with or without tumor metastasis, tumor metastatic lesions, fragments of paracancerous tissue, pleural effusion and/or peritoneal effusion are transported in a sample transport fluid, such as a commercially commonly used tumor tissue transport fluid, tumor tissue preservation fluid or tumor tissue transport fluid at about 2-8°C and processed within 48 hours. Tissue blocks can be mechanically broken into a size of about 1-27 cubic millimeters per block, transferred into a breathable culture bag or Grex, and cell serum-free culture medium and IL-2 at a concentration of 300-9000IU/mL (e.g., 1000-9000IU/mL, e.g., 6000IU/mL) are added and cultured for about 3-14 days. The cells in the culture medium are collected and transferred into a permeable culture bag, or a Grex, or a Xuri device. The serum-free culture medium of the cells can be supplemented with the CD28 antibody, CD3 antibody and CD28 antibody of the present invention, magnetic beads (e.g., Dynabeads) comprising CD3 antibody and CD28 antibody and/or nanomatrix (e.g., transACT) comprising CD3 antibody and CD28 antibody, IL-2 at a concentration of 300-9000 IU/mL (e.g., 1000-9000 IU/mL, e.g., 6000 IU/mL), and reduces the expression and/or the activity of at least one family member selected from inhibitory protein family, the protein tyrosine phosphatase family, and the A20 binding protein family (members of the activity-dependent neuroprotective protein family may include ADNP, members of the NF-kappa-B inhibitory protein family may include NFKBIA, members of the protein tyrosine phosphatase family may include PTPN6, and members of the A20 binding protein family may include TNIP1, for example, by transduction using a ribonucleoprotein complex (RNP) formed by the gRNA of the present invention and the Cas protein, or LNPs comprising gRNA and Cas proteins, or LNPs comprising nucleic acids encoding gRNA and Cas proteins, so that the proportion of cells in the TILs expressing the gene encoding at least one family member selected from the activity-dependent neuroprotective protein family, the NF-kappa-B inhibitory protein family, the protein tyrosine phosphatase family, and the A20 binding protein family is about 95% or less), after activating the TIL of the present invention for a certain period of time, add irradiated PBMCs (TILs and PBMCs at a ratio of about 1:40 to about 1:400), and expand for about 3-14 days. Cells in the culture medium can be collected using a cell processing system, washed, frozen, and tested. The final product CD3 ratio can be greater than 80%, the cell survival rate can be greater than 50%, and cells greater than 80% can be memory effector cells and effector cells. After stimulation, IFN-γ can be secreted, and/or the activated cell ratio can be increased.

### Cells, Compositions, Treatment plans

In one aspect, the present invention provides a cell, and the cell of the present invention can be cultured according to the culture method of the present invention. In one embodiment, the cell provided by the present invention can include one or one batch of cells cultured by the culture method of the present invention. In one embodiment, the cell provided by the present invention can include multiple or multiple batches of cells cultured by the culture method of the present invention and combined in any proportion.

In some embodiments, the cells expanded using the method of the present invention can be administered to a patient as a pharmaceutical composition. In some embodiments, the pharmaceutical composition can be a suspension of cells in a sterile buffer. Cells expanded using the PBMCs of the present invention can be administered by any suitable route known in the art. In some embodiments, cells can be administered with a single intra-arterial or intravenous infusion, and the infusion can last for about 30 to 60 minutes. Other suitable routes of administration can include intraperitoneal, intrathecal, and intralymphatic administration.

In some embodiments, any suitable dose of cells may be administered. In some embodiments, for example, when the tumor is a melanoma, about 1×10⁹ to about 13.7×10¹⁰, preferably about 2.3×10⁹ to about 13.7×10¹⁰ cells can be administered. In some embodiments, about 1×10⁹ to about 12×10¹⁰ cells can be administered. In some embodiments, about 1.2×10¹⁰ to about 4.3×10¹⁰ cells can be administered. In some embodiments, about 3×10¹⁰ to about 12×10¹⁰ cells can be administered. In some embodiments, about 4×10¹⁰ to about 10×10¹⁰ cells can be administered. In some embodiments, about 5×10¹⁰ to about 8×10¹⁰ cells can be administered. In some embodiments, about 6×10¹⁰ to about 8×10¹⁰ cells can be administered. In some embodiments, about 7×10¹⁰ to about 8×10¹⁰ cells can be administered. In some embodiments, the therapeutically effective dose may be about 1×10⁹ to about 13.7×10¹⁰, preferably about 2.3×10⁹ to about 13.7×10¹⁰. In some embodiments, the therapeutically effective dose may be about 1×10⁹ to about 12×10¹⁰ cells. In some embodiments, the therapeutically effective dose may be about 1.2×10¹⁰ to about 4.3×10¹⁰ cells. In some embodiments, the therapeutically effective dose may be about 3×10¹⁰ to about 12×10¹⁰ cells. In some embodiments, the therapeutically effective dose may be about 4×10¹⁰ to about 10×10¹⁰ cells. In some embodiments, the therapeutically effective dose may be about 5×10¹⁰ to about 8×10¹⁰ cells. In some embodiments, the therapeutically effective dose may be about 6×10¹⁰ to about 8×10¹⁰ cells. In some embodiments, the therapeutically effective dose may be about 7×10¹⁰ to about 8×10¹⁰ cells.

In some embodiments, the number of cells provided in the composition of the present invention can be about 1×10⁶ -9×10¹³, for example about 1×10⁶, about 2×10⁶, about 3×10⁶, about 4×10⁶, about 5×10⁶, about 6×10⁶, about 7×10⁶, about 8×10⁶, about 9×10⁶, about 1×10⁷, about 2×10⁷, about 3×10⁷, about 4×10⁷, about 5×10⁷, about 6×10⁷, about 7×10⁷, about 8×10⁷, about 9×10⁷, about 1×10⁸, about 2×10⁸, about 3×10⁸, about 4×10⁸, about 5×10⁸, about 6×10⁸, about 7×10⁸, about 8×10⁸, about 9×10⁸, about 1×10⁹, about 2×10⁹, about 3×10⁹, about 4×10⁹, about 5×10⁹, about 6×10⁹, about 7×10⁹, about 8×10⁹, about 9×10⁹, about 1×10¹⁰, about 2×10¹⁰, about 3×10¹⁰, about 4×10¹⁰, about 5×10¹⁰, about 6×10¹⁰, about 7×10¹⁰, about 8×10¹⁰, about 9×10¹⁰, about 1×10¹¹, about 2×10¹¹, about 3×10¹¹, about 4×10¹¹, about 5×10¹¹, about 6×10¹¹, about 7×10¹¹, about 8×10¹¹, about 9×10¹¹, about 1×10¹², about 2×10¹², about 3×10¹², about 4×10¹², about 5×10¹², about 6×10¹², about 7×10¹², about 8×10¹², about 9×10¹², about 1×10¹³, about 2×10¹³, about 3×10¹³, about 4×10¹³, about 5×10¹³, about 6×10¹³, about 7×10¹³, about 8×10¹³, or about 9×10¹³. In some embodiments, the number of cells provided in the compositions of the present invention may range from about 1×10⁶ to 5×10⁶, about 5×10⁶ to 1×10⁷, about 1×10⁷ to 5×10⁷, about 5×10⁷ to 1×10⁸, about 1×10⁸ to 5×10⁸, about 5×10⁸ to 1×10⁹, about 1×10⁹ to 5×10⁹, about 5×10⁹ to 1×10¹⁰, about 1×10¹⁰ to 5×10¹⁰, about 5×10¹⁰ to 1×10¹¹, about 5×10¹¹ to 1×10¹², about 1×10¹² to 5×10¹², about 5×10¹² to 1×10¹³, about 1×10¹³ to 5×10¹³, or about 5×10¹³ to 9×10¹³.

In some embodiments, the concentration of cells provided in the compositions of the invention can be less than about 100-0.0001% w/w, w/v or v/v of the composition, such as about 100%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.9%, about 0.8%, about 0.7%, about 0.6%, about 0.5%, about 0.4%, about 0.3%, about 0.2 %, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001% w/w, w/v, or v/v.

In some embodiments, the cells provided in the compositions of the invention may be present at a concentration greater than about 90-0.0001% w/w, w/v, or v/v of the composition, such as about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17. %, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50%, about 4.25%, about 4%, about 3.75%, about 3.50%, about 3.25%, about 3%, about 2.75%, about 2.50%, about 2.25%, about 2%, about 1.75%, about 1.50%, about 1.25%, about 1%, about 0.9%, about 0.8%, about 0.7%, About 0.6%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.01%..005%, about 0.004%, about 0.003%, about 0.002%, about 0.001%, about 0.0009%, about 0.0008%, about 0.0007%, about 0.0006%, about 0.0005%, about 0.0004%, about 0.0003%, about or 0.0002%, or about 0.0001% w/w, w/v or v/v.

In some embodiments, the cells provided in the compositions of the invention may be present at a concentration ranging from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, from about 0.09% to about 22%, from about 0.1% to about 21%, from about 0.2% to about 20%, from about 0.3% to about 19%, from about 0.4% to about 18%, from about 0.5% to about 17%, from about 0.6% to about 16%, from about 0.7% to about 15%, from about 0.8% to about 14%, from about 0.9% to about 12%, or from about 1% to about 10% w/w, w/v or v/v.

In some embodiments, the cells provided in the compositions of the invention may be present in a concentration ranging from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, or about 0.1% to about 0.9% w/w, w/v, or v/v of the composition.

In some embodiments, the amount of cells provided in the compositions of the present invention may be equal to or less than about 10-0.0001 g, for example, about 10 g, about 9.5 g, about 9.0 g, about 8.5 g, about 8.0 g, about 7.5 g, about 7.0 g, about 6.5 g, about 6.0 g, about 5.5 g, about 5.0 g, about 4.5 g, about 4.0 g, about 3.5 g, about 3.0 g, about 2.5 g, about 2.0 g, about 1.5 g, about 1.0 g, about 0.95 g, about 0.9 g, about 0.85 g, about 0.8 g, about 0.75 g, about 0.7 g, about 0.65 g, about 0.6 g, about 0.55 g, about 0.5 g, about 0.45 g, about 0.4 g, about 0.35 g, about 0.3 g, about 0.25 g, about 0.2 g, about 0.15g, about 0.1g, about 0.09g, about 0.08g, about 0.07g, about 0.06g, about 0.05g, about 0.04g, about 0.03g, about 0.02g, about 0.01g, about 0.009g, about 0.008g, about 0.007g, about 0.006g, about 0.005g, about 0.004g, about 0.003g, about 0.002g, about 0.001g, about 0.0009g, about 0.0008g, about 0.0007g, about 0.0006g, about 0.0005g, about 0.0004g, about 0.0003g, about 0.0002g, or about 0.0001g.

In some embodiments, the amount of cells provided in the composition of the present invention can be greater than about 0.0001-10 g, such as about 0.0001 g, about 0.0002 g, about 0.0003 g, about 0.0004 g, about 0.0005 g, about 0.0006 g, about 0.0007 g, about 0.0008 g, about 0.0009 g, about 0.001 g, about 0.0015 g, about 0.002 g, about 0.0025 g, about 0.003g, about 0.0035g, about 0.004g, about 0.0045g, about 0.005g, about 0.0055g, about 0.006g, about 0.0065g, about 0.007g, about 0.0075g, about 0.008g, about 0.0085g, about 0.009g, about 0.0095g, about 0.01g, about 0.015g, about 0.02g, about 0.025g, about 0.03g, about 0.035g, about 0.04g, about 0.045g, about 0.05g, about 0.055g, about 0.06g, about 0.065g, about 0.07g, about 0.075g, about 0.08g, about 0.085g, about 0.09g, about 0.095g, about 0.1g, about 0.15g, about 0.2g, about 0.25g, about 0.3g, about 0.35g, about 0.4g, about 0.45g, about 0.5g, About 0.55g, about 0.6g, about 0.65g, about 0.7g, about 0.75g, about 0.8g, about 0.85g, about 0.9g, about 0.95g, about 1g, about 1.5g, about 2g, about 2.5g, about 3g, about 3.5g, about 4g, about 4.5g, about 5g, about 5.5g, about 6g, about 6.5g, about 7g, about 7.5g, about 8g, about 8.5g, about 9g, about 9.5g, or about 10g.

In some embodiments, the cells can be administered in a single dose. Such administration can be by injection, for example, intravenous injection. In some embodiments, the cells can be administered in multiple doses. The dose can be once, twice, three times, four times, five times, six times, or more than six times a year. The dose can be once a month, once every two weeks, once a week, or once every 2 days. In some embodiments, administration of the cells may be continuous.

In one aspect, the present invention provides a pharmaceutical composition, which in some embodiments may comprise the cell of the present invention and a pharmaceutically acceptable carrier.

In one aspect, the present invention provides a kit, which may include a cell activator, a cell growth factor and/or a feeder cell of the cell culture method of the present invention and an instruction manual recording the steps of the cell culture method of the present invention. In one aspect, the present invention provides a kit, which may include a cell of the present invention and/or a pharmaceutical composition of the present invention.

In one aspect, the present invention provides a method of affecting the growth of cells, such as tumor cells, which may include administering cells of the present invention and/or pharmaceutical compositions of the present invention to a subject. In some embodiments, affecting tumor growth may include reducing the volume of the tumor to about 99-0.1% of the volume before administration, such as about 99%, about 95%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.9%, about 0.8%, about 0.7%, about 0.6%, about 0.5%, about 0.4%, about 0.3%, about 0.2% or about 0.1%.

In one aspect, the present invention provides the use of the cell of the present invention and/or the pharmaceutical composition of the present invention in the preparation of a drug, and the drug of the present invention can be used to prevent and/or treat a disease and/or symptom. For example, the disease and/or symptom of the present invention can include a tumor. In some embodiments, the tumor of the present invention is selected from a solid tumor. In some embodiments, the tumor of the present invention can be selected from one or more of the following group: melanoma, ovarian cancer, cervical cancer, lung cancer, bladder cancer, breast cancer, head and neck cancer, pancreatic cancer, liver cancer, gastric cancer, colorectal cancer and kidney cancer.

In one aspect, the present invention provides a method for preventing and/or treating a disease and/or symptom, which may include administering the cells of the present invention and/or the pharmaceutical composition of the present invention to a subject. For example, the disease and/or symptom of the present invention may include a tumor. In some embodiments, the tumor of the present invention is selected from solid tumors. In some embodiments, the tumor of the present invention can be selected from one or more of the following group: melanoma, ovarian cancer, cervical cancer, lung cancer, bladder cancer, breast cancer, head and neck cancer, pancreatic cancer, liver cancer, gastric cancer, colorectal cancer and kidney cancer.

In one aspect, the present invention provides a TIL of the present invention and/or a pharmaceutical composition of the present invention, which can be used to prevent and/or treat a disease and/or symptom. For example, the disease and/or symptom of the present invention can include a tumor. In some embodiments, the tumor of the present invention is selected from a solid tumor. In some embodiments, the tumor of the present invention can be selected from one or more of the following group: melanoma, ovarian cancer, cervical cancer, lung cancer, bladder cancer, breast cancer, head and neck cancer, pancreatic cancer, liver cancer, gastric cancer, colorectal cancer and kidney cancer.

Without intending to be bound by any theory, the following examples are merely intended to illustrate the methods and uses of the present invention and are not intended to limit the scope of the present invention.

### EXAMPLES

### Example 1

### (I) TIL Cell Culture

### 1.1 Tumor Tissue Reception and Processing

### 1.1.1 Tissue Reception

Receive tumor tissue and blood samples from donors, check and record sample information, and print corresponding sample labels.

### 1.1.2 Tissue Processing and Culture

Use 75% alcohol to disinfect the sample tube and blood collection tube, and transfer them to the biosafety cabinet. Separate the PBMC cells in the blood sample and freeze them according to the above-mentioned PBMC manual separation and freezing operation procedures. Take a culture bottle or culture bag with a breathable surface, such as a culture bag (Origen), and add 300mL of thawed complete culture medium. The complete culture medium can be arbitrarily selected from X-vivo15 culture medium or other commercial T cell culture medium, such as Stem Cell, Lonza, Thermo, Miltenyi and other brands of T cell culture medium, and essential amino acids and antibiotics can be added, and IL-2 at a concentration of 300-9000IU/mL (e.g. 1000-9000IU/mL, such as 6000IU/mL) was added. Take several 10 cm culture dishes, add an appropriate amount of culture medium, use sterile ophthalmic forceps to remove the tumor tissue from the sample tube into a 10 cm culture dish, wash the tissue and replace the culture dish. Use ophthalmic scissors and ophthalmic forceps to perform preliminary shearing, remove adipose tissue and necrotic tissue, and continue to cut each tissue block into about 27 cubic millimeters in size. Take a non-suspended tumor tissue block, use a 20mL syringe to remove the internal piston, connect it to the culture bag, and use a pipette to transfer about 1g of tissue block into the culture bag through the syringe. Place the culture bag in a carbon dioxide incubator for culture. Clean the scissors and forceps, and after preliminary disinfection with 75% alcohol, sterilize them after ultrasonic cleaning to obtain the first TIL group.

### 1.2 Step (A) In Vitro Expansion and Harvesting

### 1.2.1 Step (A) In Vitro Expansion

Depending on the cell growth status, the medium was replenished or half-replaced every 3-7 days to ensure cell nutrition. Complete culture medium can be selected from X-vivo 15 medium or other commercial T cell culture medium, such as Stem Cell, Lonza, Thermo, Miltenyi and other brands of T cell culture medium, and essential amino acids and antibiotics can be added, and IL-2 (double heron and/or tetracycline) at a concentration of 300-9000 IU/mL (e.g. 1000-9000 IU/mL, such as 6000 IU/mL) can be added. 3-14 days of step (A), for example, samples can be taken and counted on the 13th or 14th day. If the number of cells was between 5×10⁵ to 5×10⁸ During this time, the harvesting step of step (A) was entered.

### 1.2.2 Gains from Step (A)

Collect the cells after in vitro expansion in step (A), centrifuge, discard the culture medium, wash the cells once with PBS or saline, obtain the TILs (second TIL population) expanded in vitro in step (A), and take samples to count and retain about 5×10⁵ to 2×10⁸ cells to enter the subsequent in vitro expansion step; about 5×10⁵ cells can be taken for quality control examination; the remaining cells can be added to the cryopreservation solution and cryopreserved as cryopreserved preREP TIL in vitro cells.

### 1.3 Step (B) TIL Activation

Continue to culture the TILs (second TIL population) expanded in vitro in step (A), or recover the frozen preREP TIL cells in vitro and perform TIL activation in step (B).

Complete culture medium can be selected from X-vivo 15 medium or other commercial T cell culture medium, such as Stem Cell, Lonza, Thermo, Miltenyi Biotech, etc. Essential amino acids and antibiotics can be added to adjust the cell density to 5×10⁵ to 2×10⁶ cells/mL, in a suspension 24-well culture plate, 1 mL/well, add IL-2 at a concentration of 300-9000 IU/mL (e.g., 1000-9000 IU/mL, e.g., 6000 IU/mL). T cell activators can be added to the culture medium of each TIL cell population at the same time, such as CD3 agonists and/or CD28 agonists, for example, about 30 ng/mL of CD3 antibody (Miltenyi Biotech, OKT3), about 30 ng/mL of CD28 antibody (Merck, 15E8), magnetic beads (diameter about 1 to 10 µm Dynabeads, Thermo Fisher) at a ratio of about 1:2-2:1 of magnetic beads to TIL, and/or transACT (diameter about 100 to 500 nm, Miltenyi) to TIL at a ratio of about 1:100-1:2000. Cultivate for about 0-4 days to obtain a third TIL population.

### 1.4 Step (C) TIL Cell Gene Editing

The sgRNA targeting each target selected from the present invention was synthesized, thawed and added with nuclease-free water to a concentration of about 100 µM. About 2 µL of gRNA (50 µM) was incubated at 95°C for 2 minutes for annealing and then added to P3 buffer, and 0.3-1 µL of Cas9 (such as Kaixia, Ke Rui or Acro, 10 mg/mL) was added and incubated at 25°C for 10 minutes to form a ribonucleoprotein complex (RNP). In P3 buffer (Lonza), the above RNP was mixed with about 1×10⁶ cells of the third TIL group and the cells were electroporated by a Lonza electroporator. For example, the electroporation procedure can be human T cell stim (EO115). The electroporated cells were cultured for about 0-4 days after gene editing to obtain a fourth TIL population.

### 1.5 Step (D) TIL Cell Culture after Gene Editing

Feeder cells (irradiated healthy donor PBMC T cells) were added to the fourth TIL cell group for culture. The contact time between TIL and feeder cells needs to be a certain time Tₙ after the contact between TIL and IL-2 and T cell activator in step (B). Later (for example, Tₙ may be from 0 hours to 12 days, for example, 24 hours or 48 hours). First, feeder cells mixed from 1 to 5 donors were recovered; the activated TIL cells and feeder cells were mixed according to an ratio of 1:200 of TIL cells: feeder cells, transferred to a G-Rex100 culture flask or breathable bag, complete culture medium were supplemented, samples were taken and counted every 1-3 days, and medium was replenished or half-replaced according to the cell status until the total number of cells was greater than 1×10⁹ or the in vitro expansion culture of step (D) was carried out for about 7 days to about 14 days, and the in vitro expansion culture of step (D) was terminated.

### 1.6 Harvesting of Tumor-Infiltrating Lymphocytes

Take the cells expanded in step (D), centrifuge and discard the culture supernatant, and wash three times with PBS or saline or compound electrolyte solution to obtain TILs expanded in step (D) (fifth TIL population). Samples were counted during the third wash. According to the counting results, the supernatant was discarded after the last centrifugation, and 3×10⁶ cells were sent for quality control testing; all the remaining cells were added to the freezing solution and the cell density was adjusted to 1-3×10⁸ cells/mL for cryopreservation.

### (II) TCR-T cell Culture

T cell activation: T cells frozen in liquid nitrogen were revived and cultured, and resuspended to 5E5/ml by centrifugation in T cell culture medium RPMI 1640 (Gibco) + 10% FBS (Bovogen), and T cell TransAct (Miltenyi) was added at a ratio of 1:100. Recombinant human IL-2 was added at a concentration of 30 IU/ml and cultured for about 72 hours.

TCR transduction: One day before transduction, use recombinant human fibrin fragments (Retronectin, Takara) with a final concentration of 15µg/mL to coat 24-well suspension culture plates, 250µL per well of the 24-well plate. Protect from light and store at 4°C overnight for use. Take out the coated 24-well plate, discard the coating solution, add 500µL of 2% BSA blocking solution and block at room temperature for 30 minutes. Discard the blocking solution, wash the plate twice with 500µL/well of 2.5% HEPES washing solution, and discard the washing solution. The experimental group was transduced with a retrovirus carrying a specific TCR nucleic acid fragment of the NY-ESO-1 antigen peptide. Add 0.1-1mL of retrovirus solution to each well, centrifuge at 32°C, 2000g, and centrifuge for 2 hours. Discard the supernatant of the 24-well plate, add recovered and activated T cells to each well of the 24-well plate, with a volume of 500-1000µL, and a cell concentration of approximately 5×10⁵ cells/mL. 30-32°C, 1000g, centrifuge for 10 minutes. After centrifugation, place the culture plate in an incubator at 37°C, 5% CO₂ and culture to obtain transduced cells. Culture for about 0-4 days after transduction to obtain TCR-T cell populations. Count the density and viability of transduced cells every 1-3 days, and add T cell culture medium according to the counting results, add recombinant human IL-2 at a concentration of 30-100 IU/ml, and adjust the initial culture cell density to 0.5-2×10⁶ cells/ml and continue culturing.

TCR-T gene editing: Synthesize the sgRNA targeting each target selected from the present invention, thaw and add nuclease-free water to a concentration of about 100 µM. Add about 2 µL of gRNA (50 µM) to P3 buffer after incubation at 95°C for 2 minutes to anneal, and add 0.3-1 µL of Cas9 (e.g., Kaixia, Kerui, Acro, 10 mg/mL) and incubate at 25°C for 10 minutes to form a ribonucleoprotein complex (RNP). In P3 buffer (Lonza), the above RNP was mixed with about 1×10⁶ TCR-T cells for electroporation. For example, the electroporation procedure can be human T cell stim (EO115). Recombinant human IL-2 with a concentration of 100 to 300 IU/ml was added to the electroporated T cells, and the culture was continued to obtain the target gene-edited TCR-T cells of the present invention.

### (III) Knockout Efficiency Detection

Reagents and materials used to detect the knockout efficiency of cells: DNA extraction solution (QuickExtract DNA extraction solution, Lucigen, QE09050), RNase/DNase free water (Tiangen), EDTA (Shenggong, 0.5M), Recombinant DNase I (RNase-free, TAKARA).

Extraction of genomic DNA: About 2-7 days after T cell knockout (refer to the gene editing method of step 1.4 of Example 1 (I) or the TCR-T gene editing method of Example 1 (II)), about 1×10⁵ to about 2×10⁵ cells were taken out and washed once with PBS, and then the gene-edited cells were resuspended in 44 µL PBS, and 6 µL of the prepared nuclease mixture (containing 1 µL DNase I and 5 µL 10×DNase I Buffer) was added, and incubated at 37°C for 5 minutes. 2.5 µL of 0.5M EDTA was added to the sample and incubated at 80°C for 10 minutes. After centrifugation and discarding the supernatant, 50 µL of DNA extraction solution was added to the cell pellet, and after a brief centrifugation, the following program was run: 75°C-10 minutes; 95°C-5 minutes; 4°C-maintain. A spectrophotometer (NanoDrop^{™}) can be used to detect the concentration of DNA samples.

Sequencing: PCR primers can be designed in the region about 100 to about 200 nucleotides upstream and downstream of the PAM site. Design the PCR reaction system as follows:

| Reagent | Volume |
|---|---|
| 2×PCR Premix Buffer | 25 µL |
| DNA Template | About 100-500 ng |
| Forward Primer (10 µM) | 1 µL |
| Reverse Primer (10 µM) | 1 µL |
| ddH₂O | Up to 50 µL |
| DMSO | 1.5 µL |

And amplify according to the following PCR program:

| Temperature | Time | |
|---|---|---|
| 95°C | 4 min | |
| 95°C | 15 s | Repeat these 3 steps for 35 cycles |
| 58°C | 30 s | |
| 72°C | 30 s | |
| 72°C | 7 s | |
| 4°C | Hold | |

The PCR products were analyzed by Sanger sequencing.

### Analysis of Crispr Cas9 Knockout Efficiency

The Crispr Cas9 knockout efficiency was analyzed based on the Sager sequencing data using the Tracking of Indels by DEcomposition (Tide) method. For specific methods, see (Brinkman et al, Nucl. Acids Res. (2014) or shinyapps.datacurators.nl/tide/). The knockout efficiency was analyzed by inputting the corresponding sgRNA sequence of the present invention, the control sequence before knockout, and the test sequence after Crispr Cas9 knockout, with the P-value threshold set to 0.001.

### (IV) Examination of Expansion Status

### Experimental Preparation

The proliferation of TIL cells was detected on days 7-10 after gene editing in Example 1 (i) (withdrawal of IL-2), and each group of TIL cells was harvested; or TCR-transduced T cells obtained in Example 1 (ii) were used.

The cells were washed once with PBS and resuspended in T cell culture medium (without IL-2). After counting, the cell density was adjusted to 1e5 to 2e6/mL and added to a flat-bottom 96-well plate at 100 µL/well. For the no-stimulation culture group, no cell activator was added to the culture medium. The stimulation group was stimulated by adding CD3 antibody (OKT3) at 30 ng/ml; the TransACT stimulation group was added with transACT (diameter about 100 to 500 nm, Miltenyi) to make the concentration of transACT working solution 1:1000 (v/v); the Medium group was added with only the same volume of cell culture medium. The fluorescence of T cells was analyzed by CTG kit (CellTiter-Glo Luminescent Cell Viability Assay, Promega) when they were plated, and the fluorescence of T cells was analyzed by CTG kit after 3 days. The expansion efficiency of T cells was characterized by the fluorescence on the third day/fluorescence at the time of plating.

### (V) Examination of Cell Killing Ability

Starting from the sixth day after gene editing, tumor target cells were plated in a 96-well flat-bottom plate, and the next day, each group of TIL cells were co-cultured with target cells at different effector-target ratios (T cells: target cells, E:T). Alternatively, T cells transduced with TCR obtained in Example 1 (II) were used.

100 µL of target cells and T cells were added, and three replicate wells were set for each group. A control group containing only target cells was also set. The target cells can be selected from Hey-T30 ovarian cancer cells and A375 melanoma cells.

According to the instructions of the apoptosis detection reagent (Incucyte Caspase-3/7 Green Dye for Apoptosis, Sartorius), the apoptosis detection reagent was added at 0.2 µL/well, and the culture medium was added to dilute Caspase 3/7 Green Dye at 25 µL/well. The activity of Caspase 3/7 was recorded using an Incucyte recorder (Sartorius) to analyze the killing ability of TIL cells on target cells, and the recording was performed once every 3 hours, with a total recording time of about 5 days.

### (VI) Examination of Cell Killing Ability

The expression of T cell exhaustion, stemness and other related molecules of the TIL cells obtained on the 8th day after gene editing was detected by flow cytometry; or the TCR-transduced T cells obtained in Example 1 (ii) were detected.

V-bottom 96-well plate, manufacturer Corning, catalog number 3894; flow cytometry tube, manufacturer Corning, catalog number 352052; flow cytometry antibodies were purchased from BD or Biolegend.

Cell surface molecule detection: 1×10⁵ to 5×10⁵ cell samples per group were added to a flow tube or a V-bottom 96-well plate. Centrifuge at 600g for 3 minutes and discard the supernatant. Wash once with PBS, 1mL/tube for flow tubes and 200µL/well for 96-well plates, and discard the supernatant. Add the prepared antibody working solution for cell surface staining, the antibody (BD or Biolegend) concentration was 1:100 to 1:200, containing active detection dye 1:10000. 100µL/tube for flow tubes and 50µL/well for 96-well plates, incubate at 2-8°C in the dark for 30 minutes. After surface staining, wash the cells once with PBS (200µ L/time for 96-well plates and 1mL/time for flow tubes), centrifuge at room temperature for 3 minutes at 600g, and discard the supernatant after centrifugation. Resuspend the cells with 100-500µL PBS and perform flow cytometry detection.

Intracellular molecule detection: Prepare antibody mixed working solution for cell surface staining CD3/CD4/CD8, antibody concentration was 1:100, cell viability detection dye concentration (1:10000), 96-well plate 50µL/well, flow tube 100µL/tube staining, incubate at 2-8°C in the dark for 30 minutes. Wash cells once with PBS (96-well plate 200µL/time, flow tube 1mL/time), centrifuge at room temperature 600g for 3 minutes, and discard supernatant after centrifugation. Add 100µL of fixation/permeabilization buffer (BD, Fixation/Permeabilization) to each well, incubate at 2-8°C in the dark for 20-40 minutes, and wash twice with ×Perm/Wash Buffer after fixation and permeabilization (96-well plate 200µL/time, flow tube 1mL/time), centrifuge at 600g for 3 minutes, and discard supernatant after centrifugation. Use 1×Perm/Wash Buffer to prepare intracellular molecule antibodies (such as TCF1), resuspend TIL cells (50µ L/well for 96-well plate, 100µL/tube for flow tube staining), incubate at 2-8°C in the dark for 30 minutes; after intracellular molecule staining, wash 1-2 times with 1 ×Perm/Wash Buffer (200µ L/time for 96-well plate, 1mL/time for flow tube), centrifuge at 600g for 3 minutes, and discard the supernatant after centrifugation. Resuspend cells with 100-500µL PBS and perform flow cytometry detection.

### (VII) Flow Cytometry Detection of Cytokine Expression

The TIL cell population obtained on the 7th or 8th day after gene editing in each experimental group was tested for cytokine expression by flow cytometry; or the TCR-transduced T cells obtained in Example 1 (ii) were tested.

Prepare the culture medium required for intracellular factor expression detection: Take TIL cell culture medium and add the following according to the volume ratio: Golgistop 0.7:1000, Golgiplug 1:1000, CD107a antibody 1:500, that is 2µL/mL. No interleukin was added.

### Detection Steps

After centrifugation, T cells from each test group were resuspended in the culture medium required for the above-mentioned intracellular factor expression detection, and the cell density was adjusted to 1×10⁶ cells/mL, and the cells were added to a 96-well plate, 200 µL/well. CD3 antibody (OKT3) 30 ng/ml was added to the CD3 antibody stimulation group; transACT (diameter about 100 to 500 nm, Miltenyi) was added to the TransACT stimulation group, and the concentration of transACT working solution was 1: 1000 (v/v); for the Medium group, only the same volume of cell culture medium was added. Incubate in a 37°C incubator overnight.

Sources of main reagents and materials for cytokine flow cytometry assay: V-bottom 96-well plate, manufacturer Corning, catalog number 3894; flow cytometry tube, manufacturer Corning, catalog number 352052; flow cytometry antibodies were purchased from BD or Biolegend.

After incubation, wash once with 200 µL/well PBS, centrifuge at 600g for 3 minutes, and discard the supernatant. Prepare antibody mixed working solution for cell surface staining CD3/CD4/CD8, antibody concentration was 1:100, cell viability detection dye concentration (1:10000), 50 µL/well for 96-well plate, 100 µL/tube for flow tube staining, incubate at 2-8°C in the dark for 30 minutes. Wash cells once with PBS (200 µL/time for 96-well plate, 1 mL/time for flow tube), centrifuge at room temperature for 3 minutes at 600g, and discard the supernatant after centrifugation. Add 100 µL of fixation/permeabilization buffer (BD, Fixation/Permeabilization) to each well, incubate at 2-8°C in the dark for 20-40 minutes, and wash twice with 1 ×Perm/Wash Buffer after fixation and permeabilization (200 µL/time for 96-well plate, 1 mL/time for flow tube), centrifuge at 600g for 3 minutes, and discard the supernatant after centrifugation. Use 1 ×Perm/Wash Buffer to prepare cytokine detection antibodies (such as GZMB, TNF-α, IFN-γ), and resuspend TIL cells (50µL/well for 96-well plate, 100µL/tube for flow tube staining), incubate at 2-8°C in the dark for 30 minutes; after cytokine staining, wash 1-2 times with 1 ×Perm/Wash Buffer (200µL/time for 96-well plate, 1mL/time for flow tube), centrifuge at 600g for 3 minutes, and discard the supernatant after centrifugation. Resuspend cells with 100-500µL PBS and perform flow cytometry detection.

### (VIII) Examination of Cell Apoptosis

The TIL population obtained on the 7th or 8th day after gene editing in each test group in Example 1 was tested for apoptosis; or the TCR-transduced T cells obtained in Example 1 (ii) were tested.

The TILs in the gene knockout group or control group (NT, no treatment) were used to detect the level of T cell apoptosis using a cell apoptosis detection kit (BD 559763 Annexin V PE Apoptosis kit).

### (IX) PDO Model Cultivation

Tumor tissue was obtained from surgery, transported and stored at 2-8°C after isolation, mechanically dissociated within 24 hours, and the tissue was cut into pieces of about 0.5 mm³. After measuring the size, digest the tissue with a dedicated tissue digestion solution (bioGenous) at 37°C and then terminate the reaction with 10% FBS. Filter the tissue suspension through a 100-µm filter, wash it, and mix it thoroughly with Matrigel on ice. Then, pipette the mixture onto the bottom of a cell culture plate. Place the plate in a 37°C incubator. After Matrigel has fully solidified, carefully add complete medium for culturing. When the organoids have grown to sufficient size and density, passage them or cryopreserve them to obtain a PDO (Patient-Derived Organoid) model.

Scrape the Matrigel and organoid mixture with the tip of a pipette tip and transfer it to a centrifuge tube containing basal medium. Pipette to separate the organoids from the Matrigel. After centrifugation, add the tissue digestion solution for passage, mix well, and place it in a 37° C incubator for digestion. Dilute the digestion solution with basal medium, centrifuge, and wash. Take a small amount of the cell suspension, add 8 times the volume of digestion solution to digest it into single cells. Terminate the reaction with FBS and then count. According to the counting results, pipette the required volume of organoids, centrifuge, and resuspend them with T cell medium; count the cells to be tested (such as TIL cells or TCR-T cells), and resuspend the cells to be tested at different effector-to-target ratios to the required density; add 100 µl of PDO target cells and the cells to be tested to a flat-bottom 96-well plate, with three replicates for each group, for PDO co-culture detection. Set several different effector-to-target ratios. At the same time, set a group containing only PDO target cells, and set a control group (NT) for co-culturing unedited cells to be tested only in PDO target cells.

### Detection of Killing Ability in the PDO Model

Prepare a PDO model according to the examples of the present invention and co-culture it with TIL cells or TCR-T cells. After adding the Caspase3/7 substrate for apoptosis signal labeling, place the experimental plate in an Incucyte for observation and recording.

### (X) Detection of the Ability of Cells to Kill in Multiple Rounds (or "Continuous Killing")

Use IncuCyte to monitor the change in the fluorescence intensity of the target cell A375-GFP in multiple rounds of killing. Uniformly seed A375-GFP cells in a multi-well plate and culture them at 37°C for 4 hours. Then, take unedited cells or cells with the target of the present invention knocked out, add them to the corresponding wells, and start recording the GFP fluorescence signal with IncuCyte. Each group has 3 replicates. After 24 hours of killing, take the co-culture supernatant (20 µL/well) for CBA detection; after 3 days of killing, uniformly seed a new multi-well plate with A375-GFP. After culturing at 37°C for 4 hours, transfer the mixture of unedited cells or cells with the target of the present invention knocked out and A375-GFP cells from the previous round of killing to a new 96-well plate, and use IncuCyte to record the change curve of the GFP fluorescence signal in the new round of killing.

### (XI) CBA Method to Detect Cytokine Release

The gene-edited cells were co-cultured with autologous tumor cells, and the co-culture supernatant was collected. The CBA kit (BD) was used to detect the cytokine release of cells that were not edited or that in which the target of the present invention was knocked out.

### (XII) In Vivo Efficacy Testing of Gene-Edited Cells

Tumor tissue samples were obtained during surgery and transferred to the laboratory under sterile conditions. Necrotic tumor tissue was removed in a sterile clean bench and the tumor tissue was cut into 1-2 mm³. The cut tumor tissue was dripped with a little Matrigel and inoculated subcutaneously into NOG mice (Vitamin Liva, strain code 408) in an SPF animal room. Each mouse was inoculated with 4-5 pieces of tissue. This batch of tumor tissues was F0 generation. When the F0 generation tumor tissue grew to 800 mm³ under the mouse skin, surgically remove the F0 tumor tissue and divide the tumor tissue into 1-2mm³. Part of the tissue was frozen for future use, and part was subcutaneously inoculated into the second batch of NOG mice, and continued to be propagated in the mice. This batch of tumor tissues was the F1 generation. This process was repeated until the tumor tissues were propagated to the F3 generation in the mice. The size of the tumor tissues was measured regularly until the tumor volume grew to about 100 mm³. Afterwards, the mice were randomly divided into groups. The mice in the NT group were intravenously injected with cells that were not gene-edited, and the mice in the experimental group were intravenously injected with cells edited by the gene of the present invention.

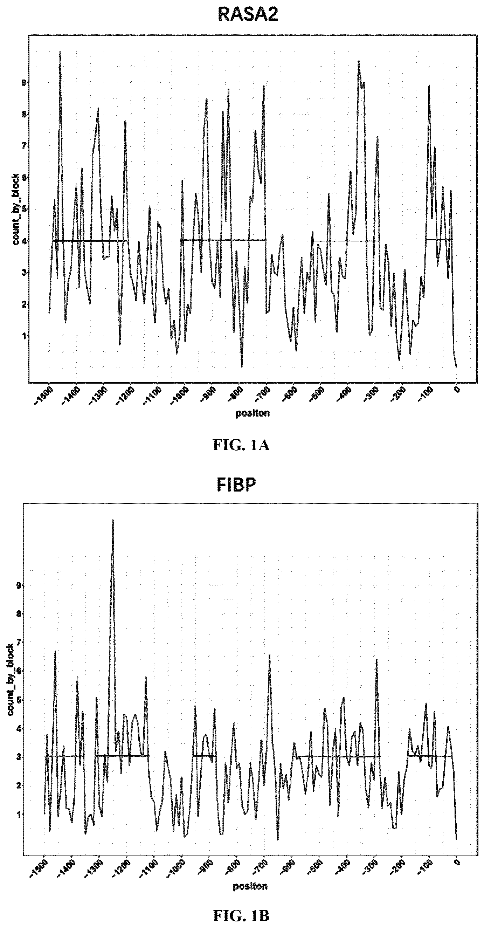

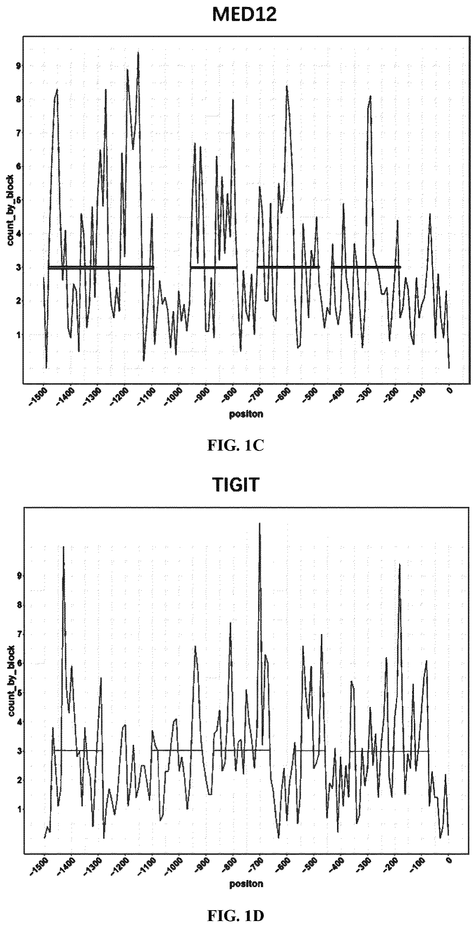

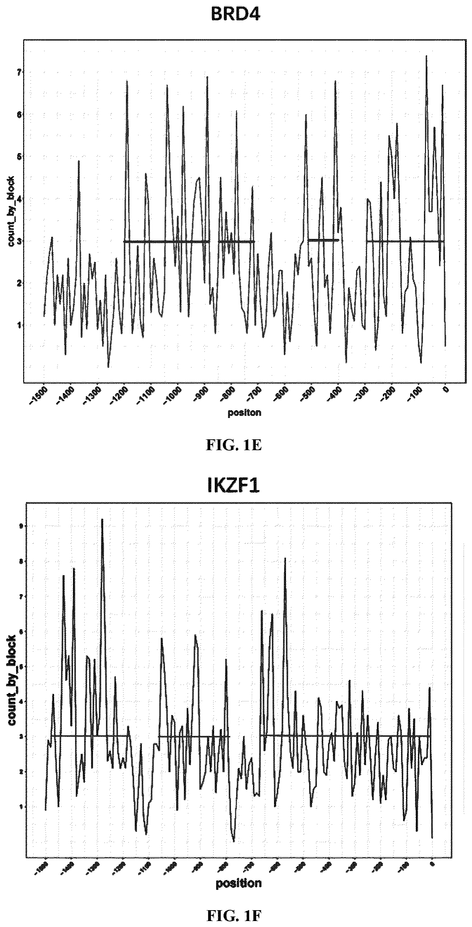

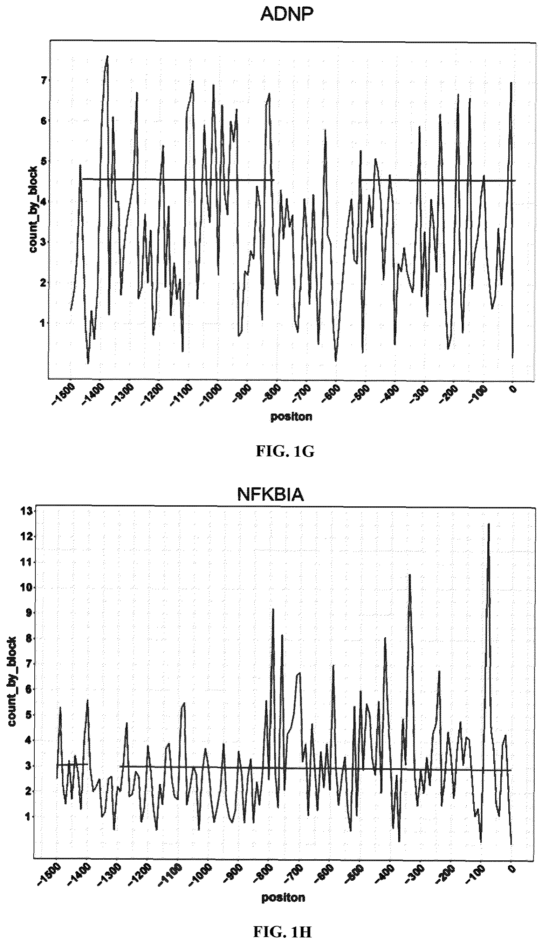

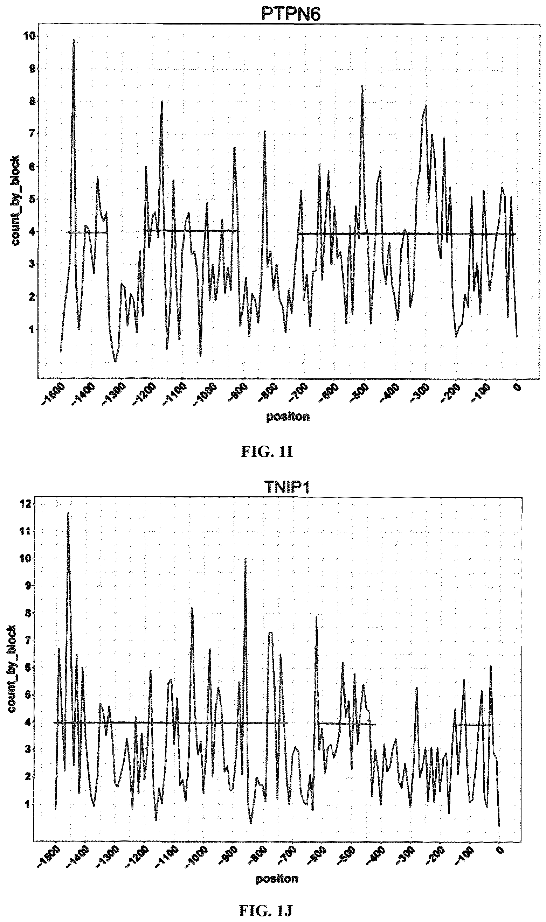

The sequence of the sgRNA (single guide RNA, or guide for short) targeting each target was synthesized according to the sequence provided by the present invention. Among them, the region targeting each target can be selected from: the exon region of the target gene, the intron region about 100bp or about 20bp away from the exon of the target gene, and the region about 1500bp before the start codon of the target gene. When the region targeting each target was selected from the region about 1500bp before the start codon of the target gene, FIGs. 1A-1D shows a continuous region with about 3 or more transcription factor binding numbers before the start codon provided by the present invention (such as the region with a vertical coordinate of more than 3 and a black line segment). The present invention also provides sgRNAs that can enhance cell function after knocking out the exon region targeting each target and the intron region about 100bp or about 20bp away from the exon of the target gene. Preferably, the sgRNA numbering information in the embodiment is as follows:

| sgRNA Index | sgRNA Sequence | SEQ ID NO: |
|---|---|---|
| RA2 | TTAGCATCAAGGCATGCCAT | 29247 |
| RA4 | AGGATCGACTTGTGGAACAA | 29248 |
| FP1 | CCGCTTTCCAGTGACCGACG | 29249 |
| FP2 | TTGCTGAATATTGTCCACCA | 29250 |
| ME2 | CAGTGAGTAGTGCCAAACCA | 29251 |
| ME4 | ACATCGACTGCTGGACAATG | 29252 |
| TI1 | CCCATCCTTCAAGGATCGAG | 29253 |
| TI4 | GCAGATGACCACCAGCGTCG | 29254 |
| BD3 | CCAGACCCCTGTCATGACAG | 29255 |
| BD4 | CACCAAACTCCTGAGCATCA | 29256 |
| IKZF1-1 | GAAAATGAATGGCTCCCACA | 29257 |
| IKZF1-2 | GATGGCTTGGTCCATCACGT | 29258 |
| TP2 | CTTGTGGCGCTGAAAACGAA | 29259 |
| TP-N20 | TCAACTGGTGTCGAGAAGTC | 29260 |
| ZC-13 | GTCGTGATGGTGTGAACACC | 29261 |
| ZC-25 | CACCACCCCGCGGGACTAGA | 29262 |
| SC3 | GGGCCCCCAGTAGAATCCGC | 29263 |
| SC-4 | GACGCCTGCGGATTCTACT | 29264 |
| CB1 | TTCCGCAAAATAGAGCCCCA | 29265 |
| RA2 | TTAGCATCAAGGCATGCCAT | 29266 |
| FP1 | CCGCTTTCCAGTGACCGACG | 29267 |
| FP2 | TTGCTGAATATTGTCCACCA | 29268 |
| ME2 | CAGTGAGTAGTGCCAAACCA | 29269 |
| ME4 | ACATCGACTGCTGGACAATG | 29270 |
| ADNP-2 | TCAGATTGTATGTAGTTACC | 29271 |
| ADNP-4 | TTGCACATCACTTACGAGAG | 29272 |
| NFKBIA-3 | GATCCGCCTCGAGCCGCAGG | 29273 |
| NFKBIA-4 | GGTTGGTGATCACAGCCAAG | 29274 |
| PTPN6-1 | CCAGCCGTACTATGCCACGA | 29275 |
| PTPN6-2 | CCAGGGTGGACGCTACACAG | 29276 |
| TNIP1-1 | ATAAAACTTACACTGGATGG | 29277 |
| TNIP1-3 | GATGAGCAATGGCAACAAAG | 29278 |
| IK1-1 | GCTTTGCTGTCCTCCCGAGG | 29279 |
| IK1-2 | GCACATCAAGCTGCATTCCG | 29280 |
| IK1-3 | CGGAATGCAGCTTGATGTGC | 29281 |
| IK1-4 | GGCCACCTGAGGACGCACTC | 29282 |
| IK1-5 | ACCTGAGGACGCACTCCGGT | 29283 |
| IK1-6 | GGACACCGAGAGCAACAACG | 29284 |
| IK1-7 | TGTGCGACGAGAACTCGTAC | 29285 |
| IK1-8 | TTCTCGTCGCACATAACGCG | 29286 |
| IK1-9 | TCTCGTCGCACATAACGCGA | 29287 |
| IK1-10 | CTCGTCGCACATAACGCGAG | 29288 |
| IK1-11 | GGTGAGGGAATCTCAGCCAG | 29289 |
| IK1-12 | CGAGCTACATGCATCTAGGG | 29290 |
| IK1-13 | GAAGACAGTCTCCTCCTACG | 29291 |
| RA2-1 | GAGTGCGACTGCAGAGCCCG | 29292 |
| RA2-2 | AAACAATCTCTCATTTTGTG | 29293 |
| RA2-3 | GAAGACTTGTGTAATCACAG | 29294 |
| RA2-4 | GCCTGTTGACTCCAATTCAG | 29295 |
| RA2-5 | TTAGCATCAAGGCATGCCAT | 29296 |
| RA2-6 | CATCCAAAACTGATGACCTG | 29297 |
| RA2-7 | TGCAGCAGCAGTCGTACAAG | 29298 |
| RA2-8 | GTCTAATTCAGCCACAGCAG | 29299 |
| RA2-9 | GGATGAGATGATGAAAATAG | 29300 |
| RA2-10 | GAGATGATGAAAATAGTGGG | 29301 |
| RA2-11 | AGATGATGAAAATAGTGGGA | 29302 |
| RA2-12 | ATTAAAACCTATTCTTGATG | 29303 |
| RA2-13 | CGATCCTATTAAATTGAAAG | 29304 |
| RA2-14 | AGATATCACACATTACAGTG | 29305 |
| RA2-15 | CTGCAGAATACTGAACATGA | 29306 |
| RA2-16 | GGTCGCAAATGAAAAGTATG | 29307 |
| RA2-17 | TACAAACTTTGGGAAGCTGG | 29308 |
| RA2-18 | AAAGCATAAACACAACTACC | 29309 |
| RA2-19 | ATTTCCGTTCAGATACACAG | 29310 |
| RA2-20 | AAAGAAACTTACGCAGTACA | 29311 |
| RA2-21 | TCTGCAGCTTAAGTAAACTG | 29312 |
| RA2-22 | GGAGGCAGATGACATTGAAG | 29313 |
| RA2-23 | TGTAATGACTGATGGACCAA | 29314 |
| TN1-1 | GCCAGAAGCCCAGATTAGGG | 29315 |
| TN1-2 | GGCCTGAAACCACTTCCGGG | 29316 |
| TN1-3 | ACAAATGAAAGCCTTCTGGG | 29317 |
| TN 1-4 | CTGAGCTGTTTACAGGACTG | 29318 |
| TN1-5 | GCTAAAAGCATTCAAAGATG | 29319 |
| TN1-6 | TGATCGTGAGCGCATGAATG | 29320 |
| TN1-7 | GTATTCCCTGCAGACCGACA | 29321 |
| TN1-8 | GAAGCAGGTGACTGACCTGG | 29322 |
| TN1-9 | GACGGCAGGTAAGGTCCCAG | 29323 |
| TN1-10 | GATGAGCAATGGCAACAAAG | 29324 |
| TN1-11 | CTCGGCACACACACTCAGCG | 29325 |

### Example 2

According to the experimental method in the example, the knockout effect of sgRNA targeting RASA2, FIBP, MED12, TIGIT, and BRD4 was detected.

### (A) Knockout Efficiency

| sgRNA Index | Knockout Efficiency (%) | sgRNA Index | Knockout Efficiencv (%) | sgRNA Index | Knockout Efficiency (%) |
|---|---|---|---|---|---|
| RA2 | A | RA2-2 | A | RA2-13 | D |
| RA4 | N/A | RA2-3 | A | RA2-14 | N/A |
| FP1 | A | RA2-4 | A | RA2-15 | A |
| FP2 | A | RA2-5 | A | RA2-16 | A |
| ME2 | A | RA2-6 | A | RA2-17 | A |
| ME4 | A | RA2-7 | B | RA2-18 | A |
| TI1 | A | RA2-8 | A | RA2-19 | A |
| TI4 | A | RA2-9 | A | RA2-20 | A |
| BD3 | A | RA2-10 | A | RA2-21 | A |
| BD4 | A | RA2-11 | A | RA2-22 | A |
| RA2-1 | A | RA2-12 | A | RA2-23 | A |

Among them, A means the knockout efficiency was greater than or equal to 70%, B means the knockout efficiency was greater than or equal to 50% and less than 70%, C means the knockout efficiency was greater than or equal to 10% and less than 50%, D means the knockout efficiency was less than 10%, and N/A means not detected.

Among them, donor 045 was a healthy donor and donor 026 was a healthy donor, who were used to isolate PBMC cells and then transduce TCR as TCR-T cells.

### (B) Proliferation Ability

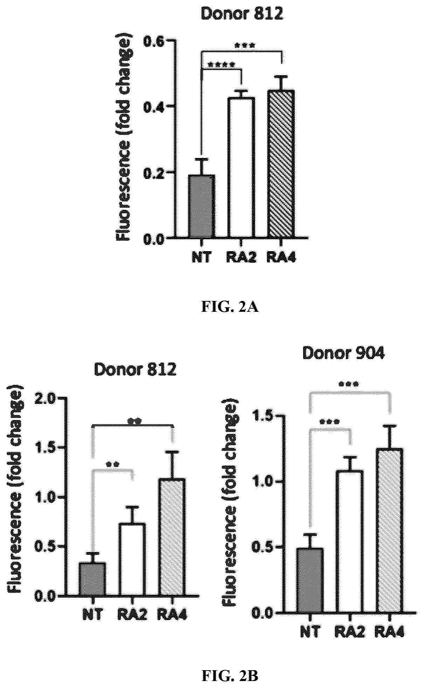

FIG. 2A shows that RASA2 gene-edited TIL cells in the no-stimulation group can have significant expansion capacity.

FIG. 2B shows that RASA2 gene-edited TIL cells in the CD3 antibody stimulation group can have significant expansion capacity.

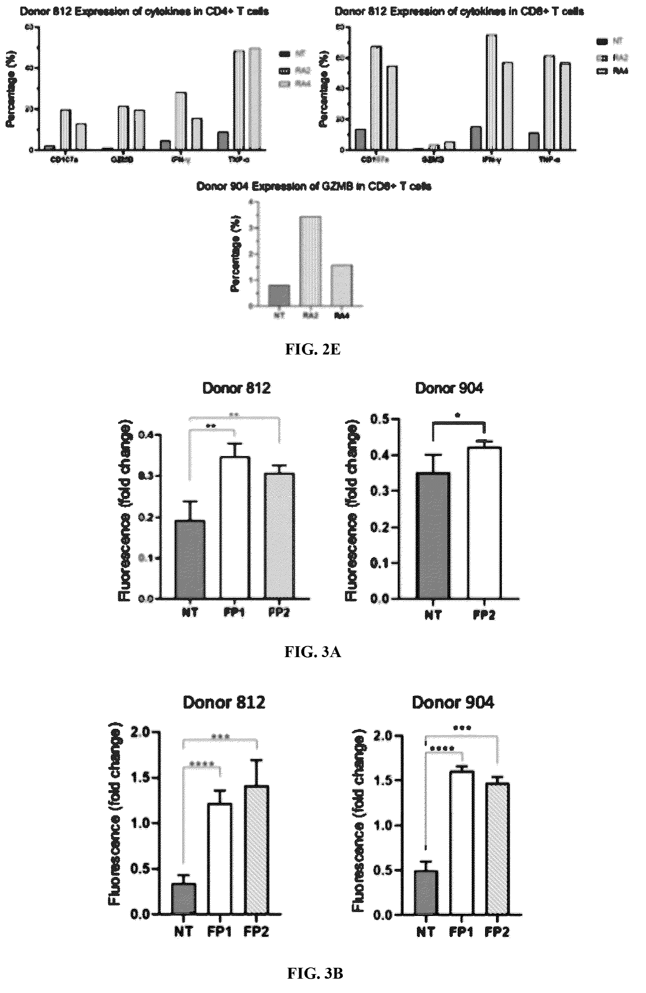

FIG. 3A shows that FIBP gene-edited TIL cells in the no-stimulation group can have significant expansion capacity.

FIG. 3B shows that FIBP gene-edited TIL cells in the CD3 antibody stimulation group can have significant expansion ability.

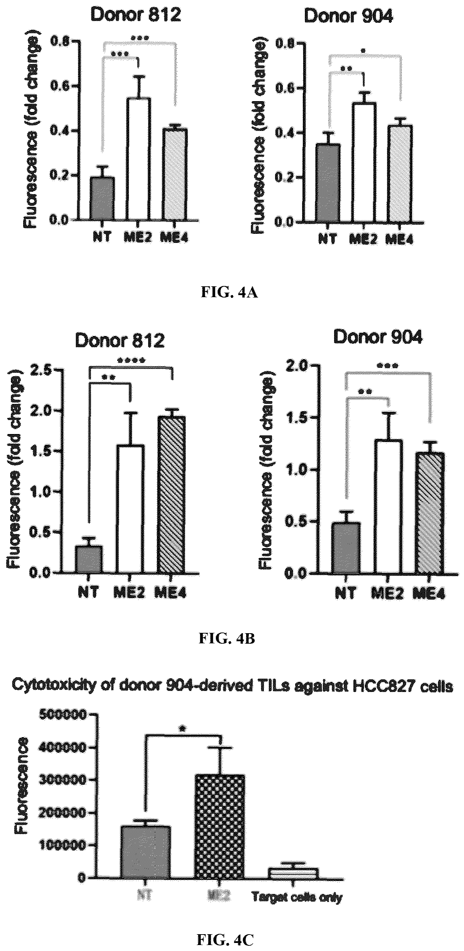

FIG. 4A shows that MED12 gene-edited TIL cells in the no-stimulation group can have significant expansion capacity.

FIG. 4B shows that the MED12 gene-edited TIL cells in the CD3 antibody stimulation group can have significant expansion ability.

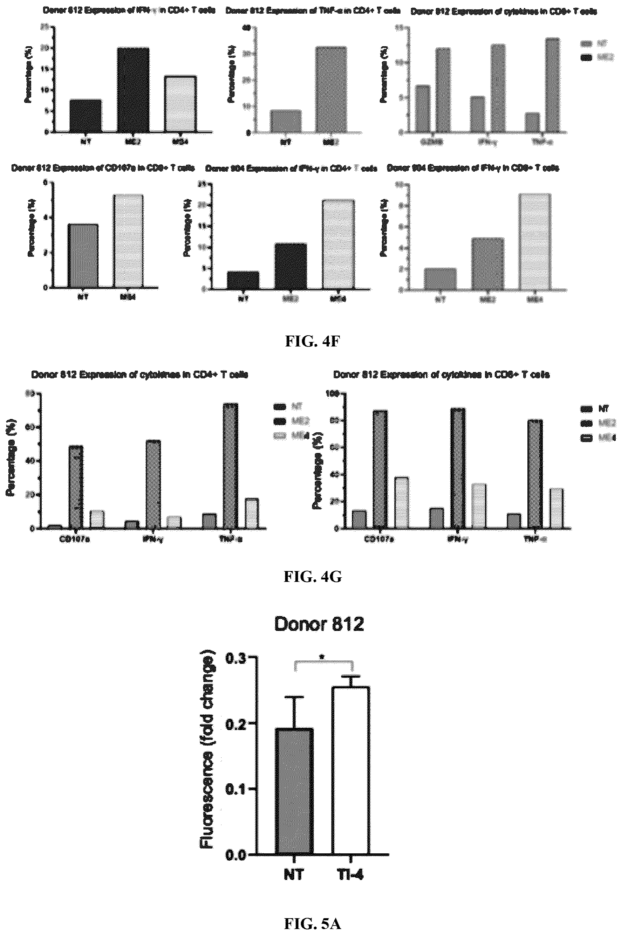

FIG. 5A shows that TIGIT gene-edited TIL cells in the no-stimulation group can have significant expansion capacity.

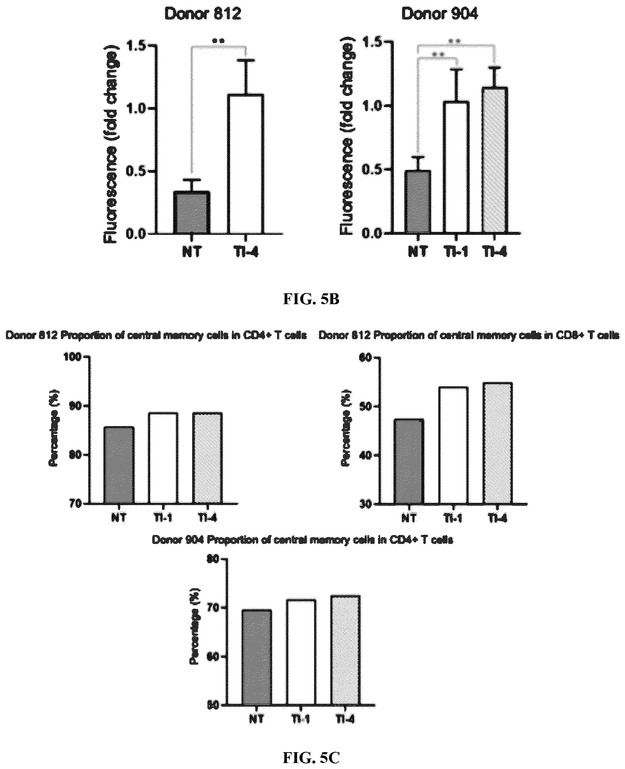

FIG. 5B shows that TIGIT gene-edited TIL cells in the CD3 antibody stimulation group can have significant expansion ability.

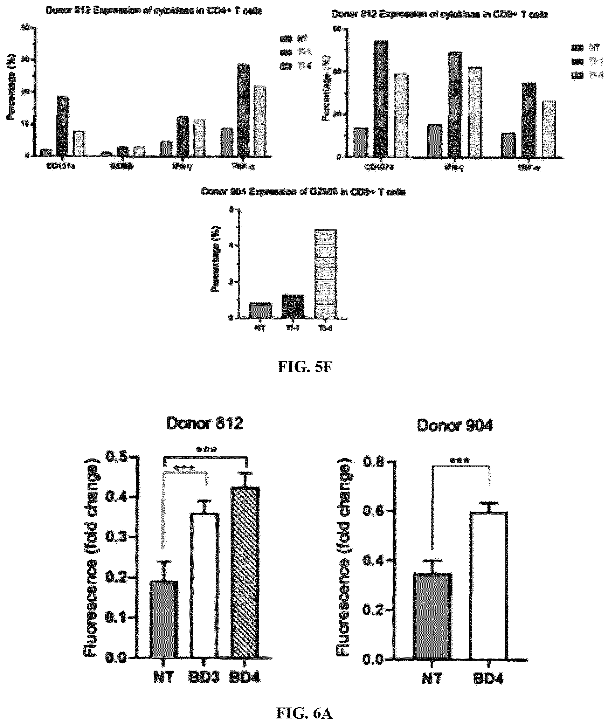

FIG. 6A shows that BRD4 gene-edited TIL cells in the no-stimulation group can have significant expansion capacity.

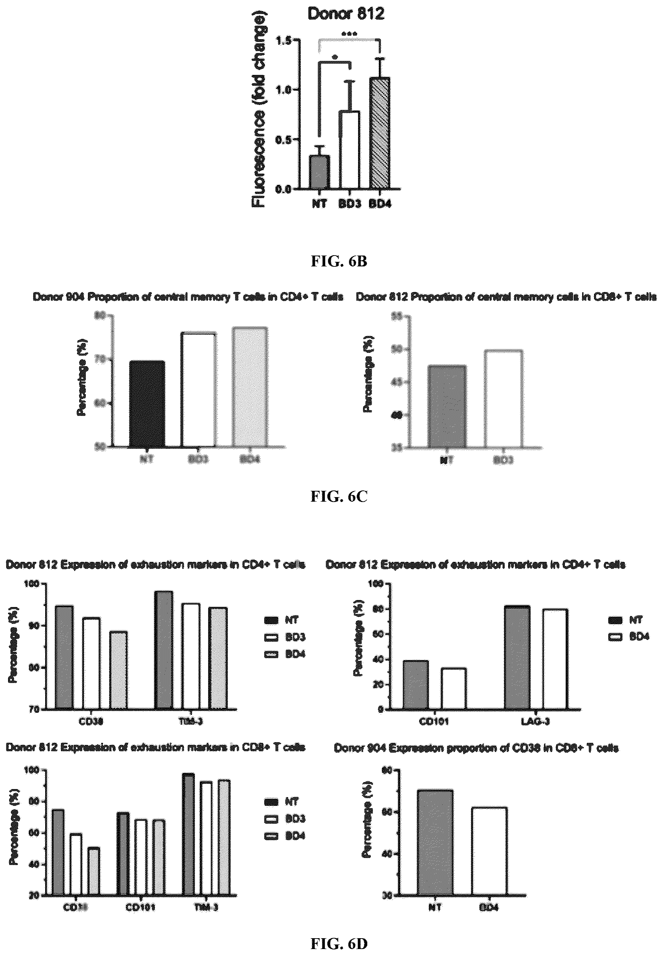

FIG. 6B shows that BRD4 gene-edited TIL cells in the CD3 antibody stimulation group can have significant expansion ability.

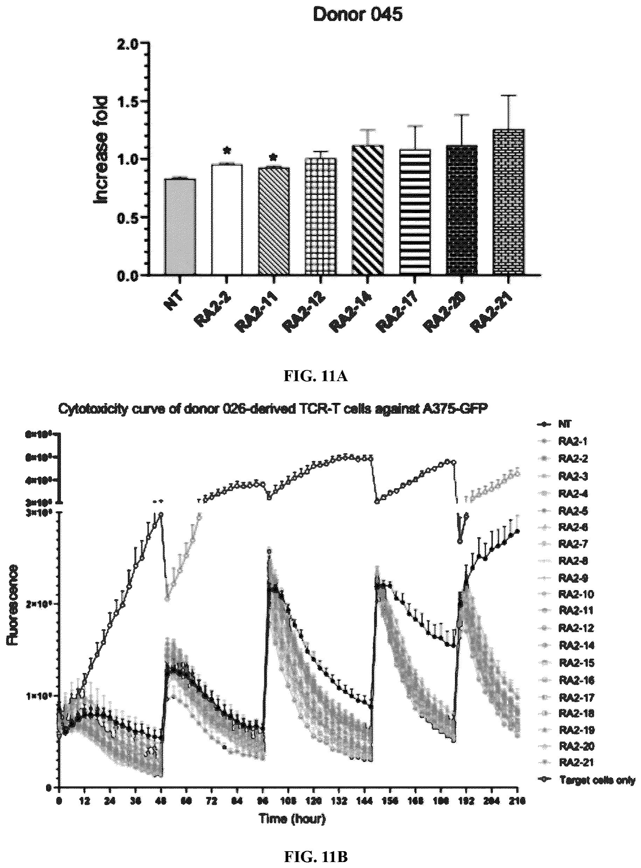

FIG. 11A shows that RASA2 gene-edited TCR T cells in the TransACT stimulation group can have significant expansion capacity.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved expansion capacity compared to the unedited control group (NT).

### (C) Killing Ability

FIG. 4C shows the target cell killing ability of MED12 gene-edited TIL cells.

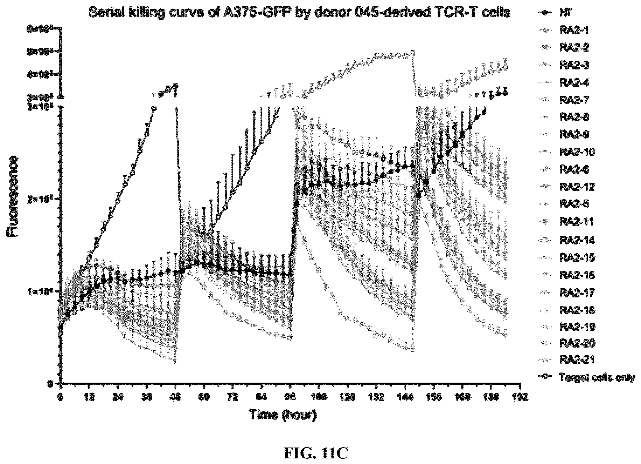

FIG. 11B and FIG. 11C show the target cell killing ability of RASA2 gene-edited TCR T cells.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved target cell killing ability and/or continuous killing ability compared with the control group (NT).

### (D) Flow cytometry

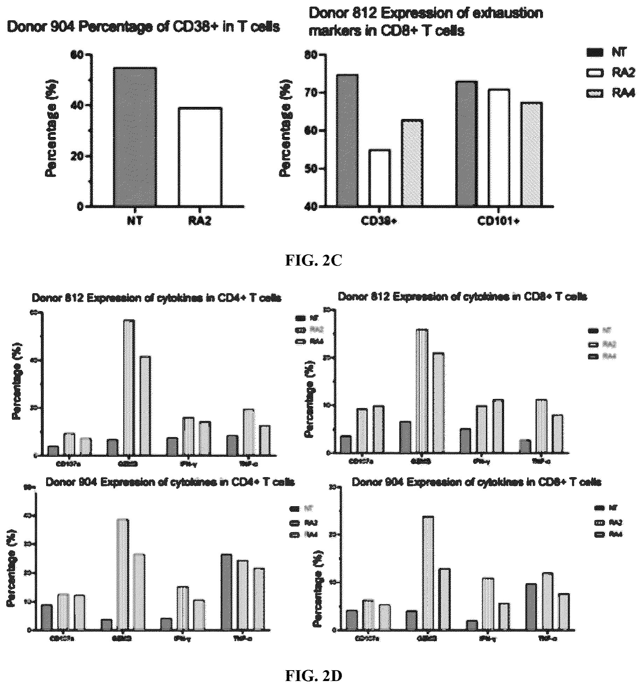

FIG. 2C shows that TIL cells after RASA2 gene editing have a lower proportion of exhausted T cells.

FIG. 2D shows that the RASA2 gene-edited TIL cells in the unstimulated group have a higher cytokine expression ratio.

FIG. 2E shows that the RASA2 gene-edited TIL cells in the stimulation group have a higher cytokine expression ratio.

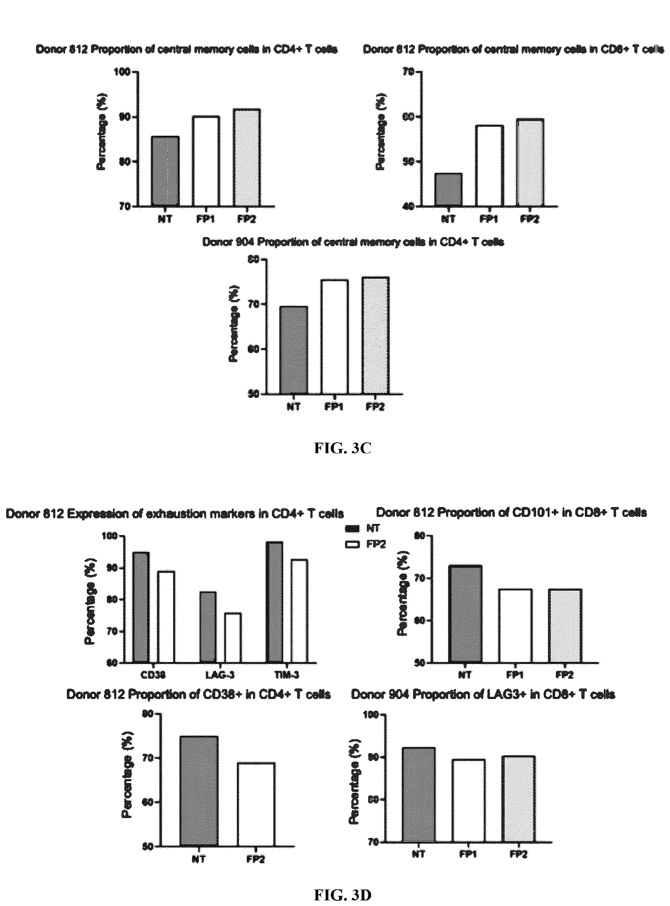

FIG. 3C shows that TIL cells after FIBP gene editing have a higher proportion of central memory T cells.

FIG. 3D shows that TIL cells after FIBP gene editing have a lower proportion of exhausted T cells.

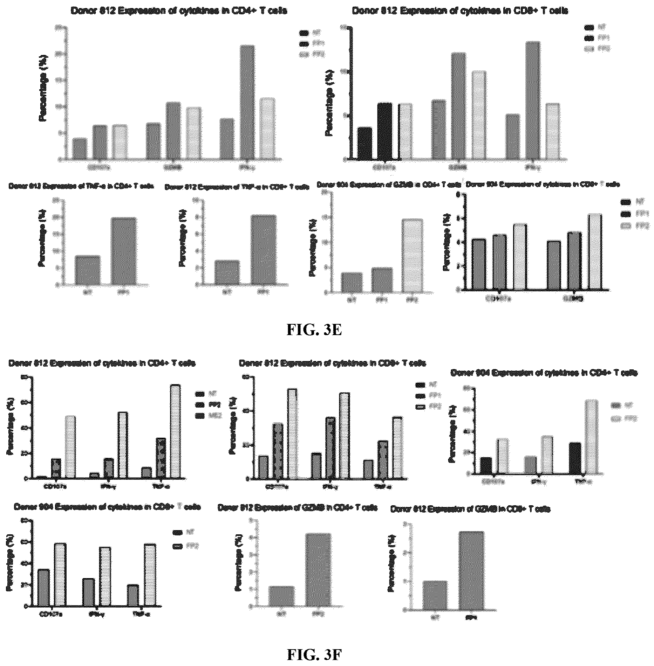

FIG. 3E shows that the FIBP gene-edited TIL cells in the unstimulated group have a higher cytokine expression ratio.

FIG. 3F shows that the FIBP gene-edited TIL cells in the stimulation group have a higher cytokine expression ratio.

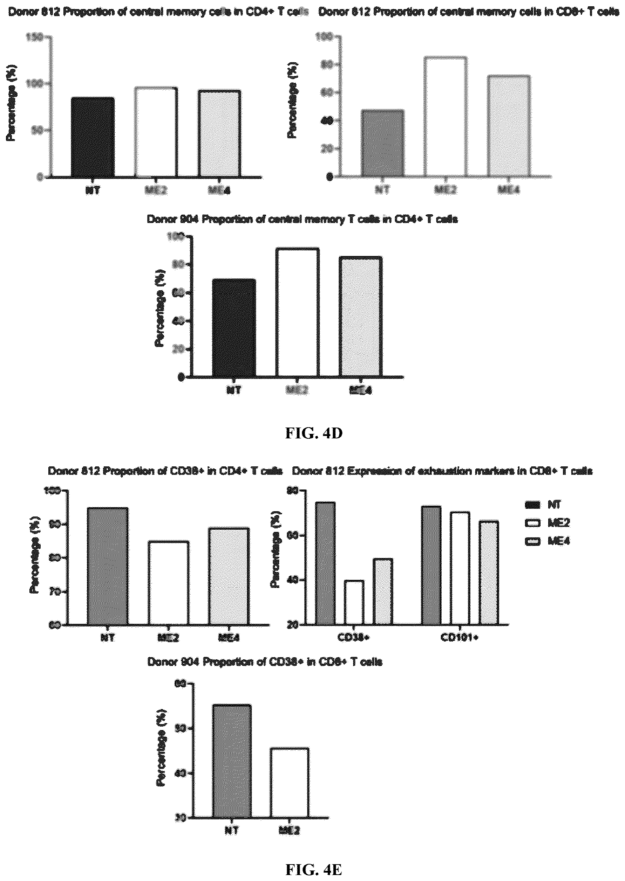

FIG. 4D shows that TIL cells after MED12 gene editing have a higher proportion of central memory T cells.

FIG. 4E shows that TIL cells after MED12 gene editing have a lower proportion of exhausted T cells.

FIG. 4F shows that the MED12 gene-edited TIL cells in the unstimulated group have a higher cytokine expression ratio.

FIG. 4G shows that the MED12 gene-edited TIL cells in the stimulation group have a higher cytokine expression ratio.

FIG. 5C shows that TIL cells after TIGIT gene editing have a higher proportion of central memory T cells.

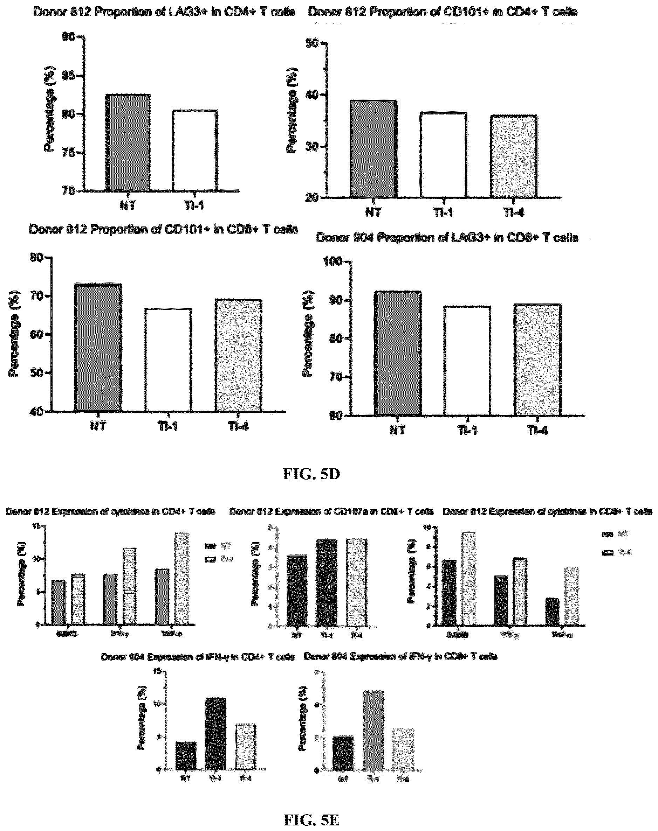

FIG. 5D shows that TIL cells after TIGIT gene editing have a lower proportion of exhausted T cells.

FIG. 5E shows that the TIL cells after TIGIT gene editing in the unstimulated group have a higher cytokine expression ratio.

FIG. 5F shows that the TIGIT gene-edited TIL cells in the stimulation group have a higher cytokine expression ratio.

FIG. 6C shows that TIL cells after BRD4 gene editing have a higher proportion of central memory T cells.

FIG. 6D shows that TIL cells after BRD4 gene editing have a lower proportion of exhausted T cells.

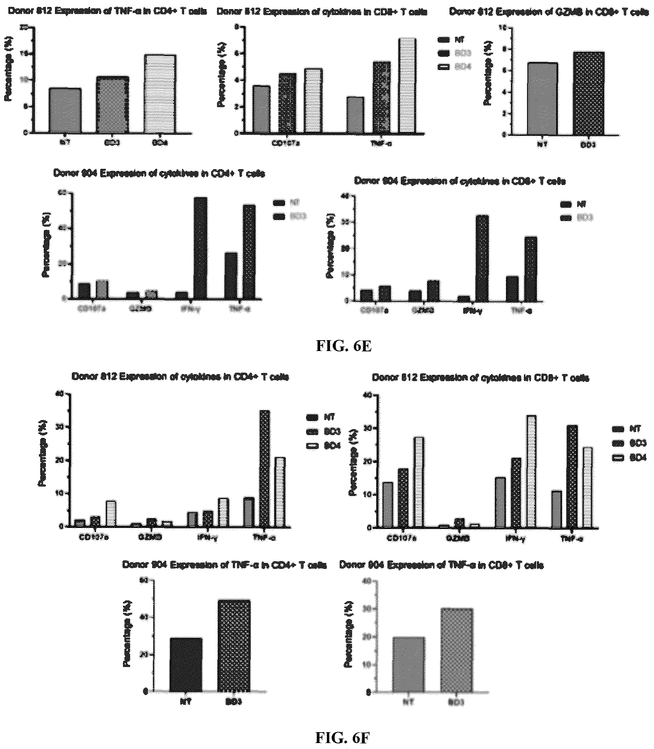

FIG. 6E shows that the BRD4 gene-edited TIL cells in the unstimulated group have a higher cytokine expression ratio.

FIG. 6F shows that the BRD4 gene-edited TIL cells in the stimulation group have a higher cytokine expression ratio.

The results show that compared to the control group (NT), the target gene-edited T cells of the present invention can have significantly improved cytokine expression capacity.

### (E) Cytokine Secretion Capacity

The CBA kit was used to detect the cytokine release of cells that were not edited or cells in which the target of the present invention was knocked out.

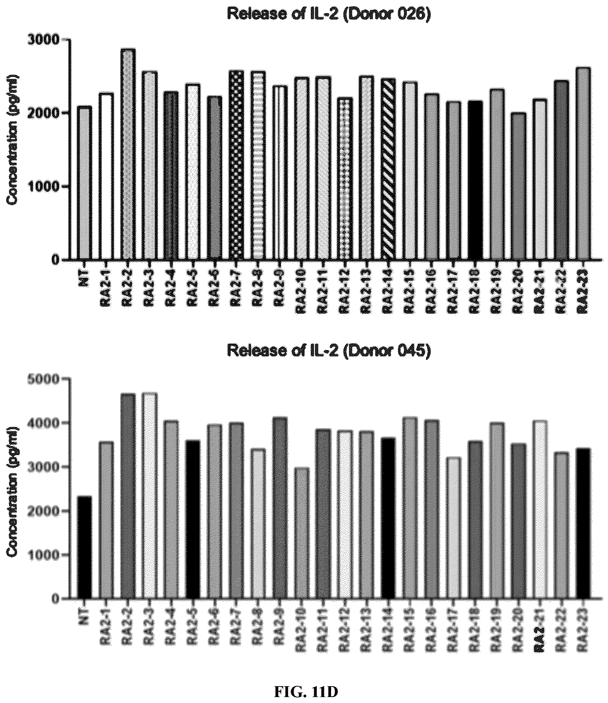

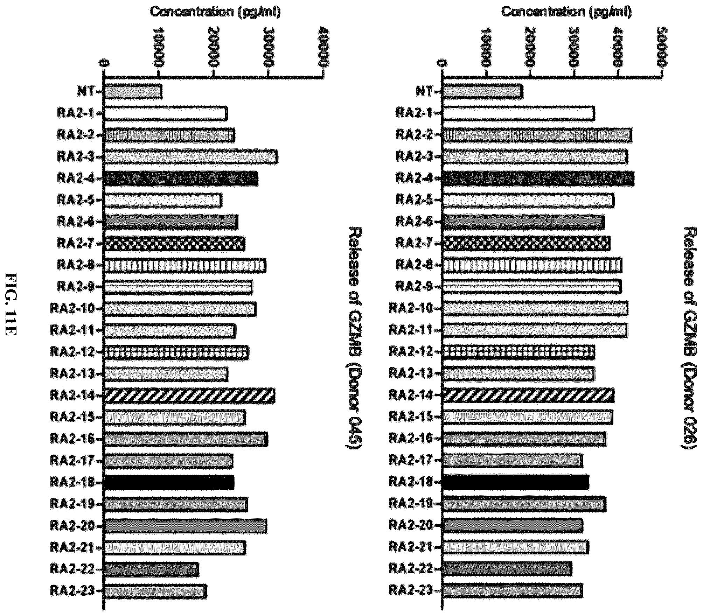

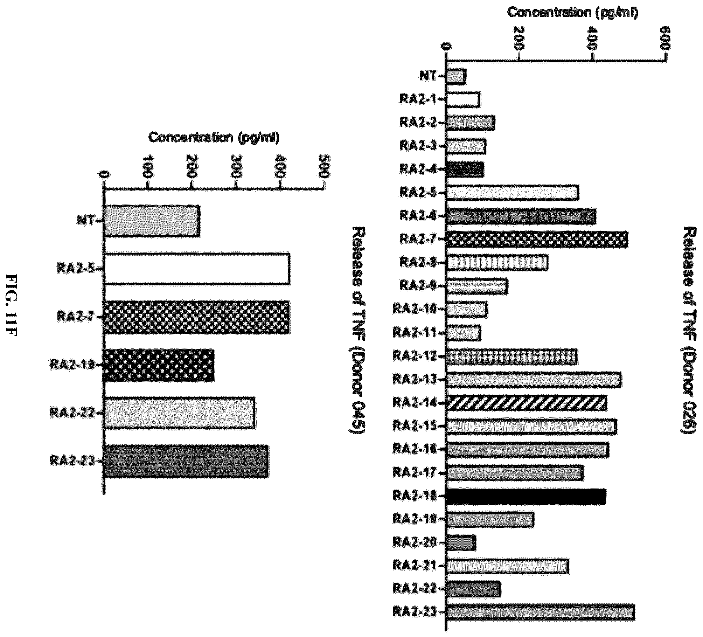

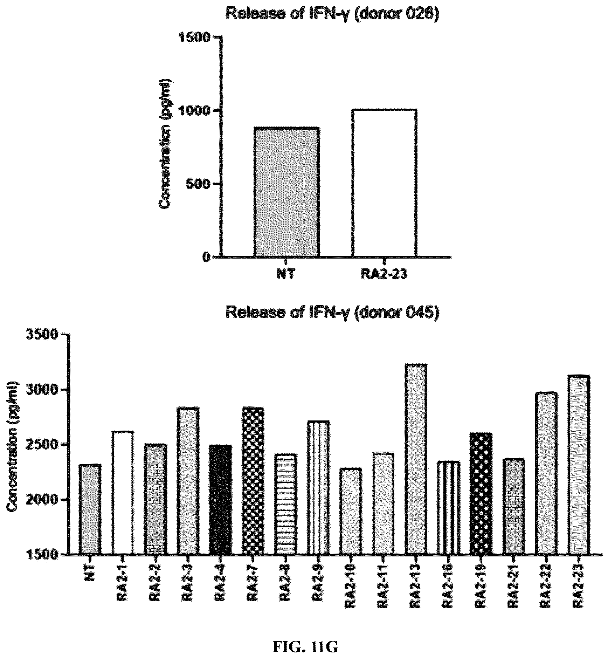

FIGs 11D, 11E, 11F, and 11G show the cytokine release of TCR T cells without editing or knockout of RASA2 target detected by CBA kit.

The results show that compared with the control group (NT), the target gene-edited T cells of the present invention can have significantly improved cytokine release capacity.

### Example 3

According to the experimental method in the example, the knockout effect of the sgRNA targeting IKZF1 was detected.

| sgRNA Index | Knockout Efficiency (%) | sgRNA Index | Knockout Efficiency (%) | sgRNA Index | Knockout Efficiency (%) |
|---|---|---|---|---|---|
| IKZF1-1 | A | IK1-4 | A | IK1-9 | A |
| IKZF1-2 | A | IK1-5 | B | IK1-10 | A |
| IK1-1 | A | IK1-6 | A | IK1-11 | A |
| IK1-2 | A | IK1-7 | B | IK1-12 | A |
| IK1-3 | B | IK1-8 | C | IK1-13 | B |

Among them, A represents a knockout efficiency greater than or equal to 70%, B represents a knockout efficiency greater than or equal to 50% and less than 70%, and C represents a knockout efficiency greater than or equal to 10% and less than 50%.

Among them, donor 005 was a healthy donor, donor 031 was a healthy donor, who were used to isolate PBMC cells and then transduce TCR as TCR-T cells. Among them, donor 107 was a patient with lung adenocarcinoma, donor 812 was a patient with malignant melanoma, donor 504 was a patient with ovarian cancer, donor 904 was a patient with lung cancer, and donor 906 was a patient with endometrial cancer, who were used to isolate and provide TIL.

### (B) Proliferation Ability

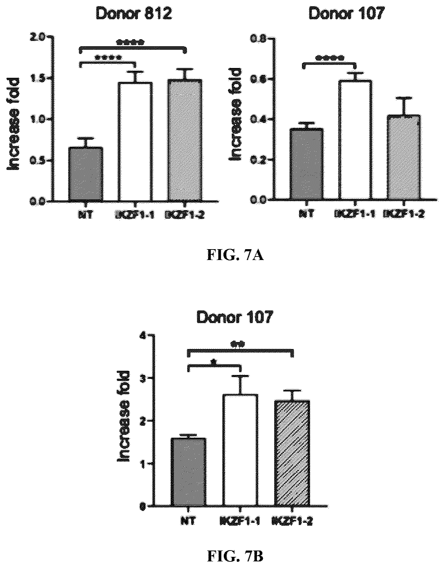

FIG. 7A shows the expansion fold change of IKZF1 gene-edited TILs in the non-stimulated Medium group.

FIG. 7B shows the expansion fold change of IKZF1 gene-edited TILs in the transACT stimulation group.

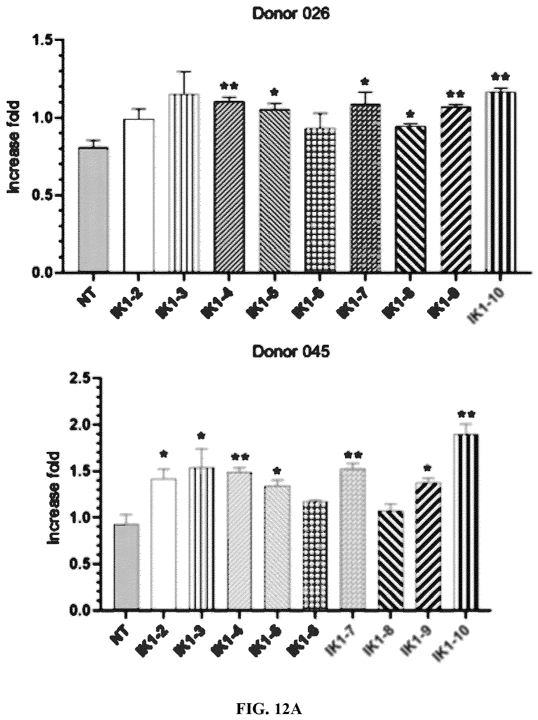

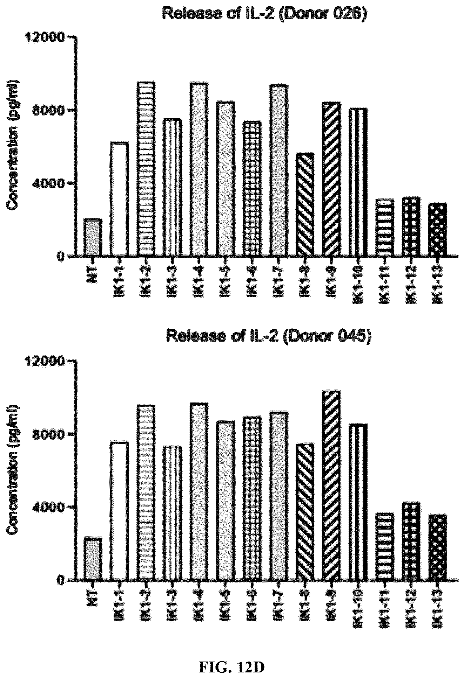

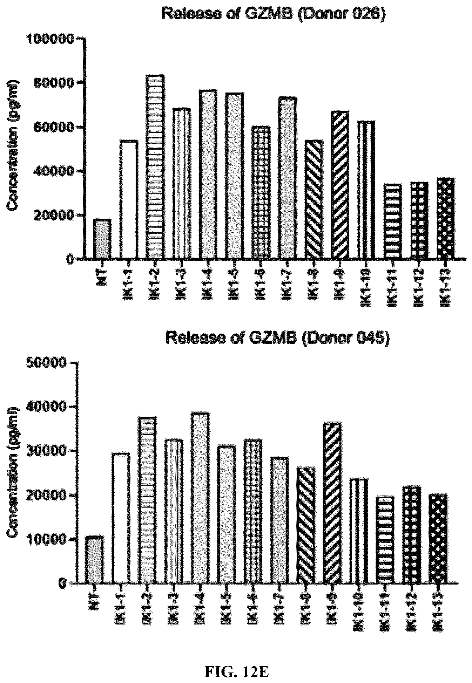

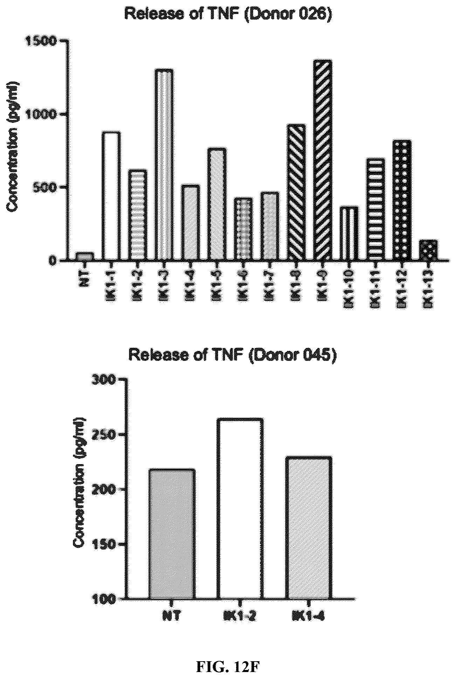

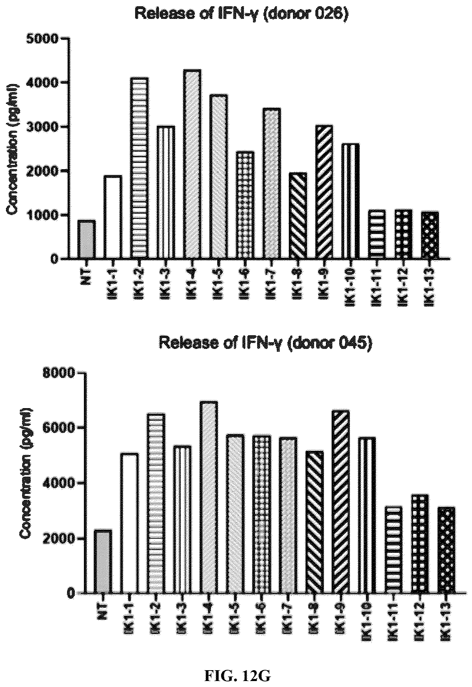

FIG. 12A shows that IKZF1 gene-edited TCR T cells in the TransACT stimulation group can have significant expansion capacity.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved expansion capacity compared to the unedited control group (NT).

### (C) Killing Ability

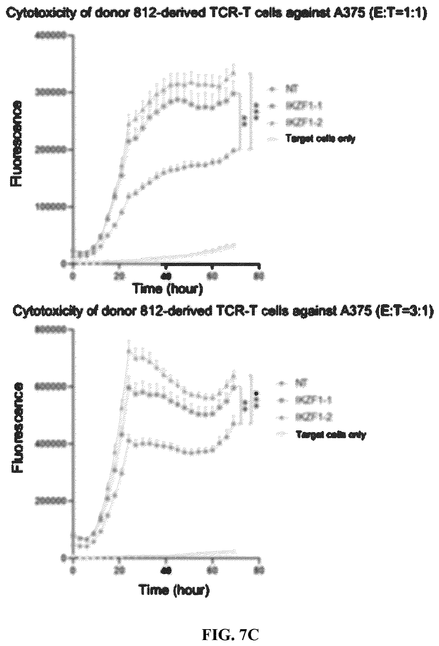

FIG. 7C shows the target cell killing ability of the IKZF1 gene-edited TIL cells of the first donor, with a detection time of 69 hours.

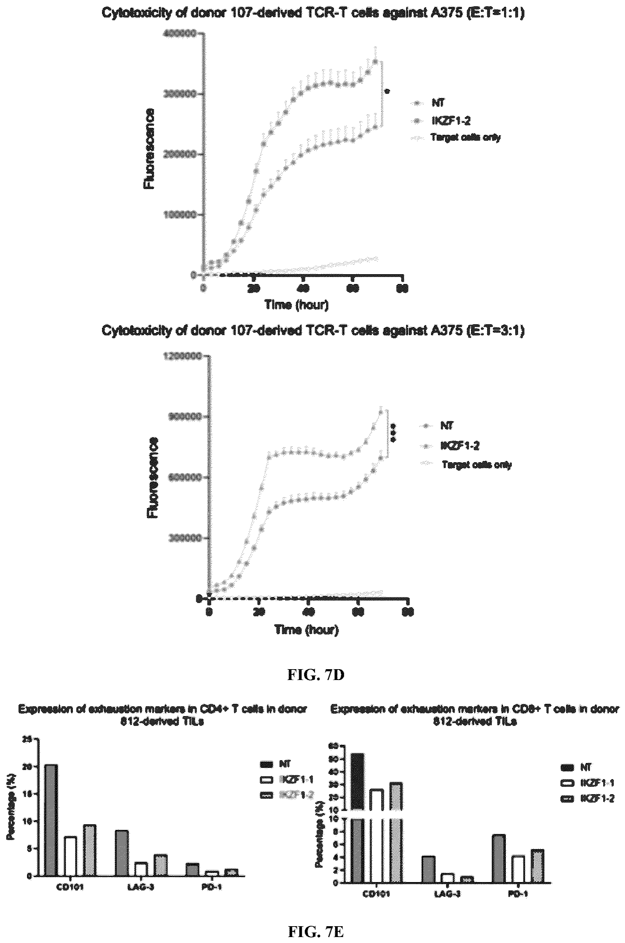

FIG. 7D shows the target cell killing ability of the IKZF1 gene-edited TIL cells from the second donor, with a detection time of 69 hours.

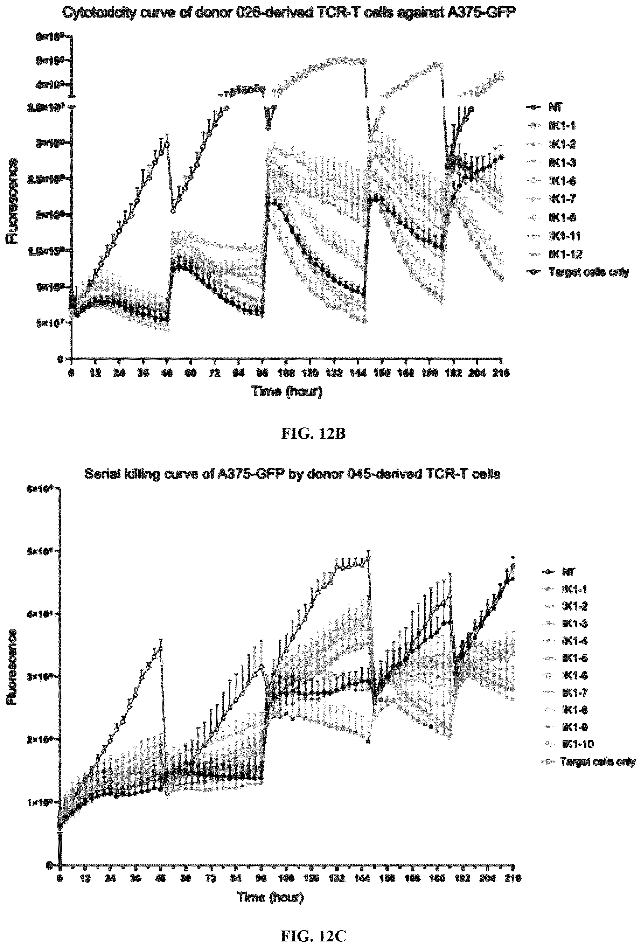

FIG. 12B and FIG. 12C show the target cell killing ability of IKZF1 gene-edited TCR T cells.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved target cell killing ability and/or continuous killing ability compared with the control group (NT).

### (D) Flow Cytometry

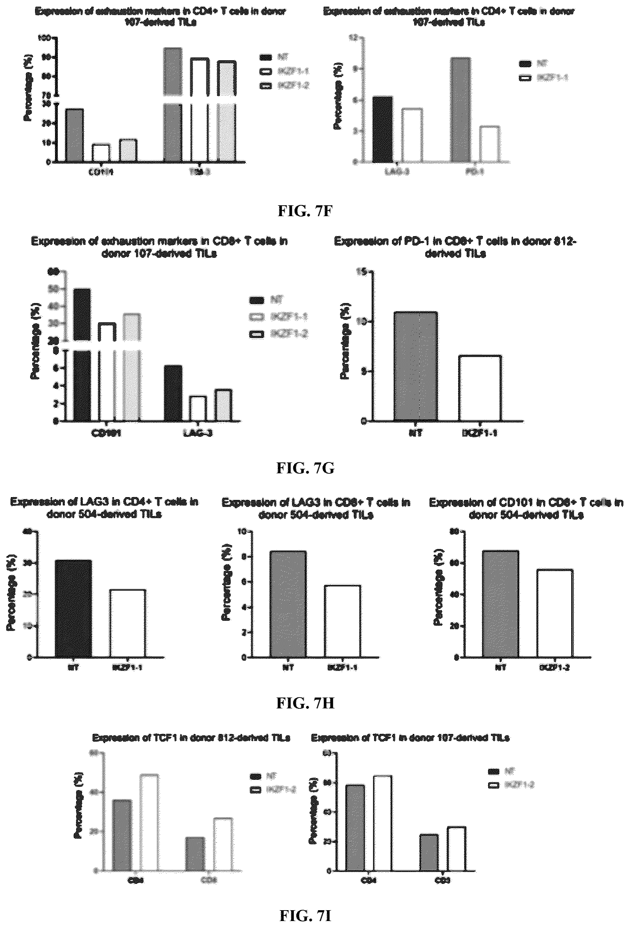

FIG. 7E, FIG. 7F, FIG. 7G, and FIG. 7H show the proportion of exhausted T cells in TIL cells after IKZF1 gene editing.

FIG7I shows the proportion of stem T cells in TIL cells after IKZF1 gene editing.

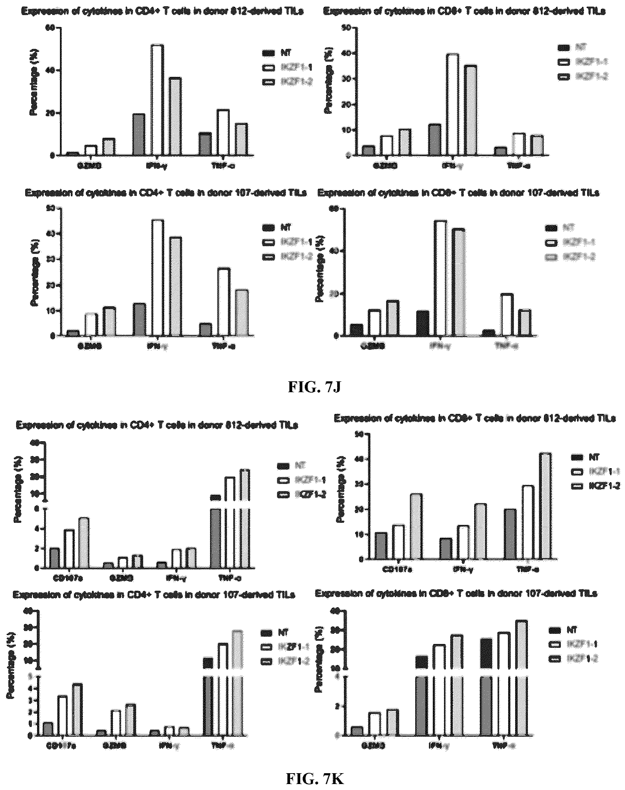

FIG. 7J shows the cytokine expression ratio in TIL cells after IKZF1 gene editing in the no stimulation group.

FIG. 7K shows the cytokine expression ratios of TIL cells after IKZF1 gene editing in the stimulation group.

The results show that compared with the control group (NT), the target gene-edited TCR T cells of the present invention can have significantly improved cell subpopulation ratios.

### (E) Multiple Rounds of Killing (or "Continuous Killing") Capability

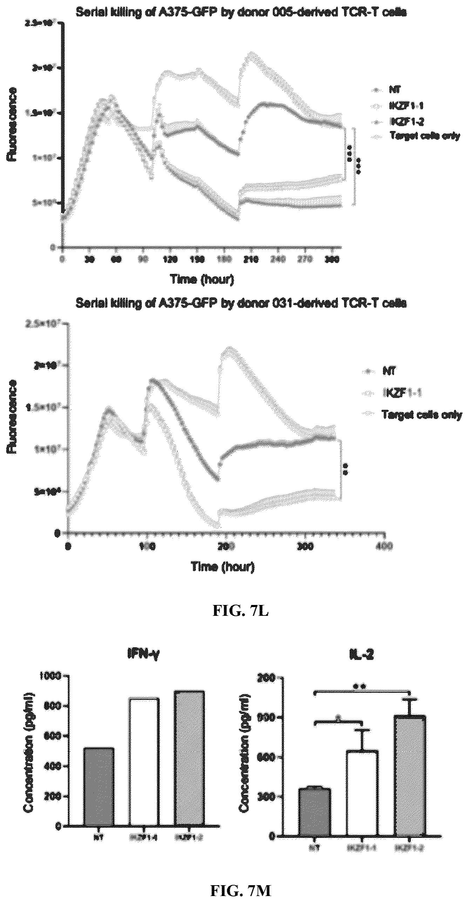

FIG. 7L shows the results of multiple rounds of killing by TCR T cells without editing or knocking out the IKZF1 target.

The results show that after multiple rounds of killing, the cells with the target of the present invention knocked out have a stronger ability to kill tumor cells than the unedited cells. The significant difference was relative to the unedited NT group, * indicates P<0.05, ** indicates P<0.01, and *** indicates P<0.001.

### (F) Cytokine Release Ability

The CBA kit was used to detect the cytokine release of cells that were not edited or cells in which the target of the present invention was knocked out.

FIG. 7M shows the cytokine release of TCR T cells without editing or knockout of IKZF1 target detected by CBA kit.

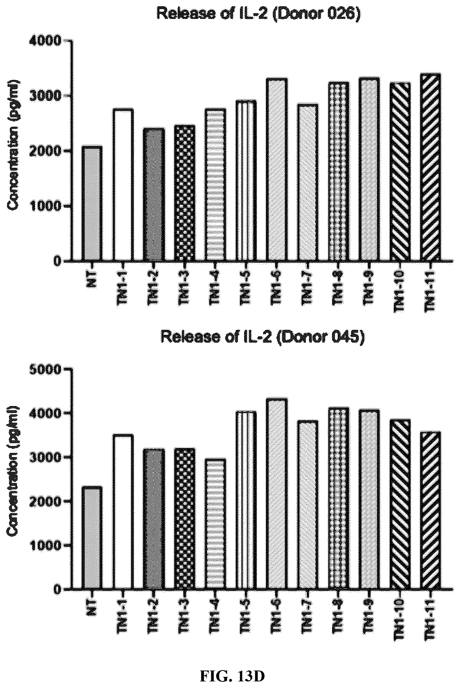

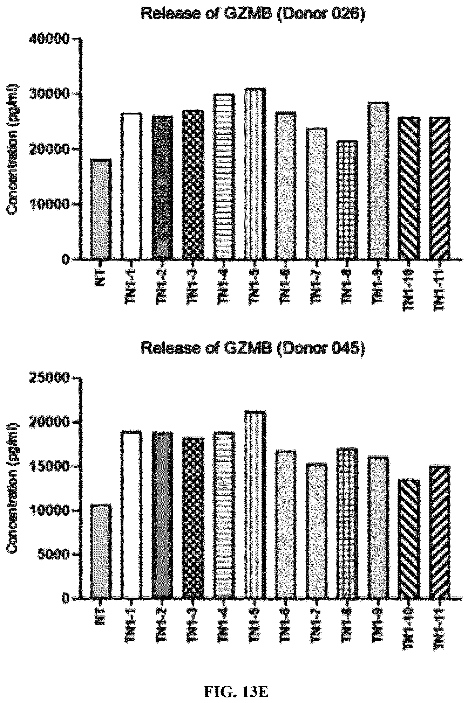

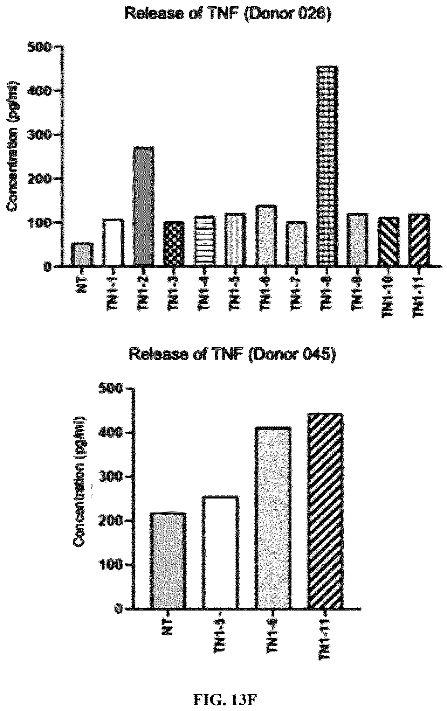

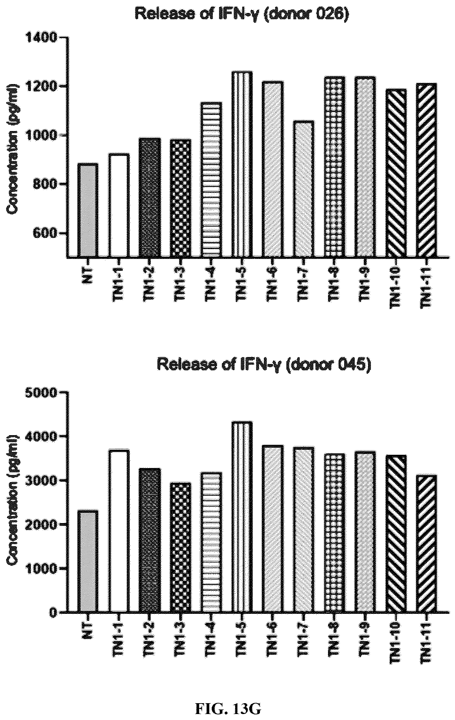

FIGs 13D, 13E, 13F, and 13G show the cytokine release of TCR T cells without editing or knockout of TNIP1 target detected by CBA kit.

The results show that compared to the control group (NT), the target gene-edited T cells of the present invention can have significantly improved cytokine release capacity.

### Example 4

According to the test method in the example, the knockout effect of a combination of two or more sgRNAs targeting IKZF1 and optionally other targets was detected.

When the target gene was edited in TIL cells, IKZF1 and TNFAIP3 of the present invention were simultaneously knocked out. The knockout efficiency of each target was detected by the cell knockout efficiency detection method of the above example. Among them, donor 309 was a patient with cervical cancer, donor 812 was a patient with malignant melanoma, and donor 504 was a patient with endometrial cancer.

| Combination | sgRNA Index | Knockout Efficiency (%) |
|---|---|---|
| IK-1&TP2 | TP2 | A |
| | IKZF1-1 | A |
| IK-2&TP2 | TP2 | A |
| | IKZF1-2 | A |

Wherein, A indicates that the knockout efficiency was greater than or equal to 70%.

When editing the target gene of TIL cells, the IKZF1 of the present invention was used together with multiple targets, for example, TNFAIP3 (TP), ZC3H12A (ZC), SOCS1 (SC), CBLB (CB), RASA2 (RA), FIBP (FP), and MED12 (ME) for simultaneous knockout. The knockout efficiency of each target was detected using the cell knockout efficiency detection method of the above example.

| Group | First Target gRNA | Knockout Efficiency of First Target gRNA | Second Target gRNA | Knockout Efficiency of Second Target gRNA |
|---|---|---|---|---|
| TP2 | IKZF1-2 | A | TP2 | A |
| TP-N20 | IKZF1-2 | A | TP-N20 | A |
| ZC-13 | IKZF1-2 | A | ZC-13 | A |
| ZC-25 | IKZF1-2 | A | ZC-25 | A |
| SC3 | IKZF1-2 | A | SC3 | N/A |
| SC-4 | IKZF1-2 | A | SC-4 | N/A |
| CB1 | IKZF1-2 | A | CB1 | A |
| RA2 | IKZF1-2 | B | RA2 | B |
| FP1 | IKZF1-2 | A | FP1 | A |
| FP2 | IKZF1-2 | A | FP2 | N/A |
| ME2 | IKZF1-2 | A | ME2 | A |
| ME4 | IKZF1-2 | B | ME4 | B |

Among them, A means that the knockout efficiency was greater than or equal to 70%, B means that the knockout efficiency was greater than or equal to 50% and less than 70%, and N/A means not detected.

### (B) Expansion

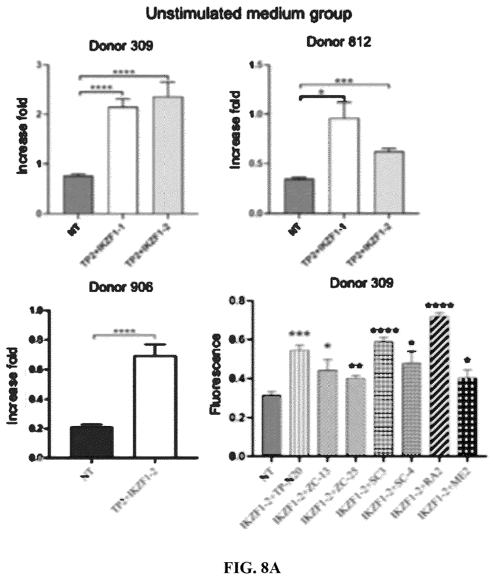

FIG 8A shows the expansion fold change of TILs after combinatorial gene editing in the non-stimulation Medium group.

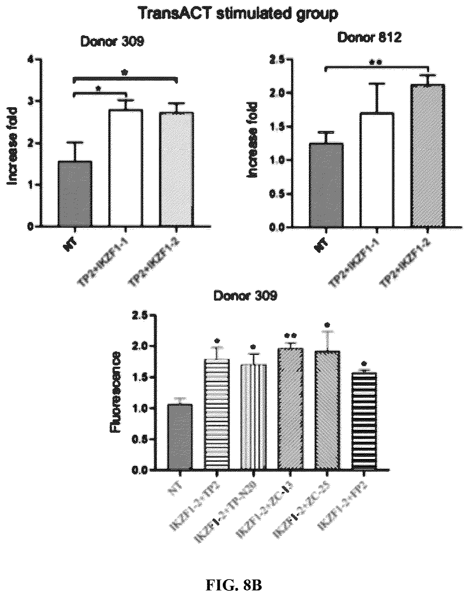

FIG 8B shows the expansion fold change of combinatorial gene-edited TILs in the TransACT stimulation group.

The significant difference is relative to the unedited NT group, * indicates P < 0.05, ** indicates P < 0.01, *** indicates P < 0.001, and **** indicates P < 0.0001. The results show that the target combination gene-edited TIL cells of the present invention can have significantly improved expansion capacity compared with the unedited control group (NT).

### (C) Killing Ability

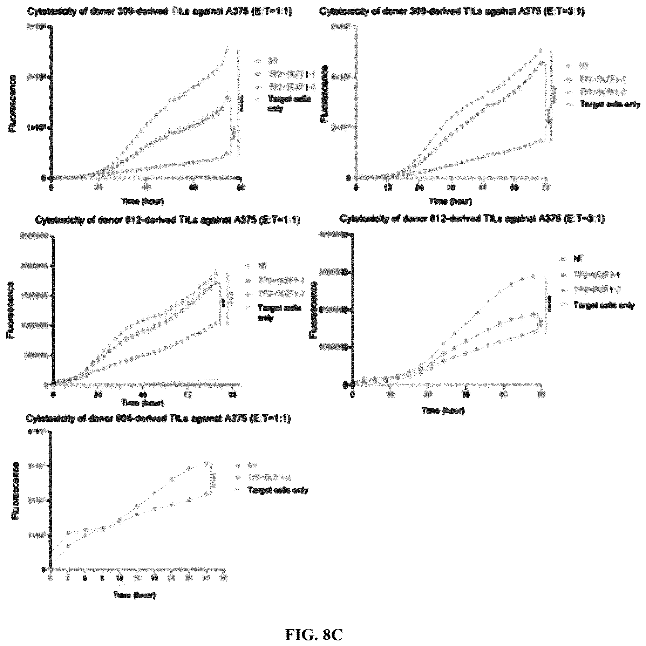

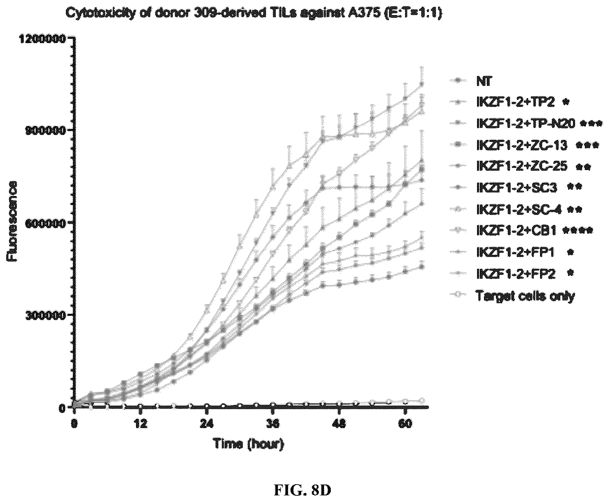

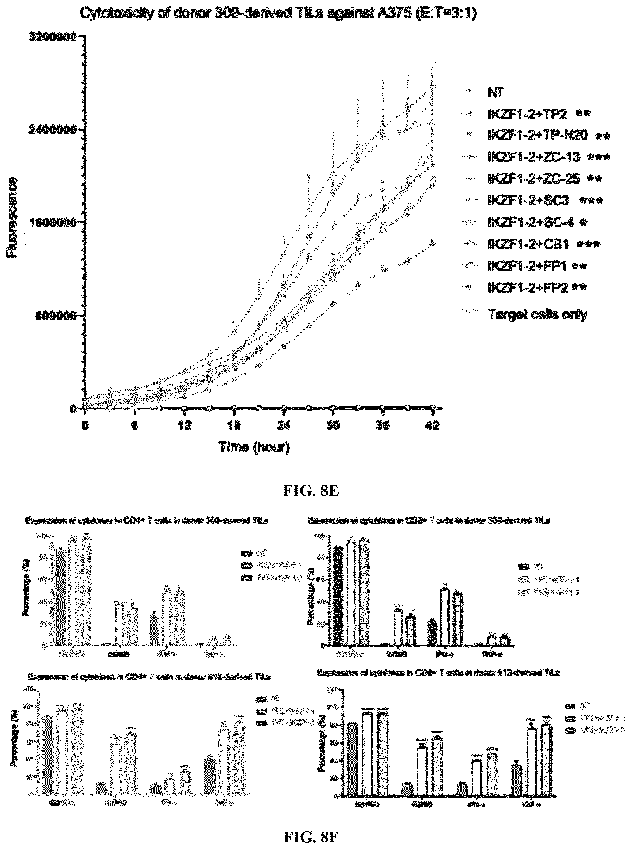

FIGs. 8C, 8D, and 8E show the target cell killing ability of combinatorial gene-edited TILs.

The significant difference is relative to the unedited NT group, * indicates P < 0.05, ** indicates P < 0.01, *** indicates P < 0.001, and **** indicates P < 0.0001. The results show that compared to the control group (NT), the target combination gene-edited TIL cells of the present invention can have significantly improved target cell killing ability.

### (D) Cytokine Expression and Release

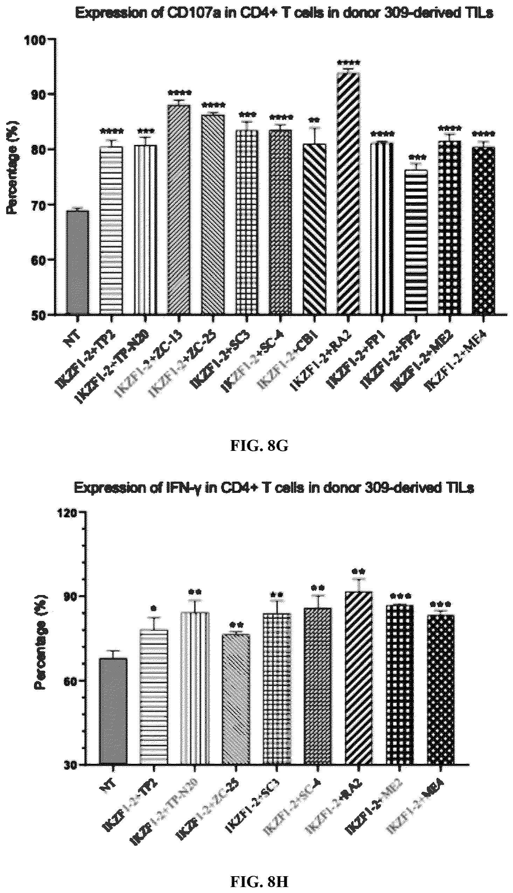

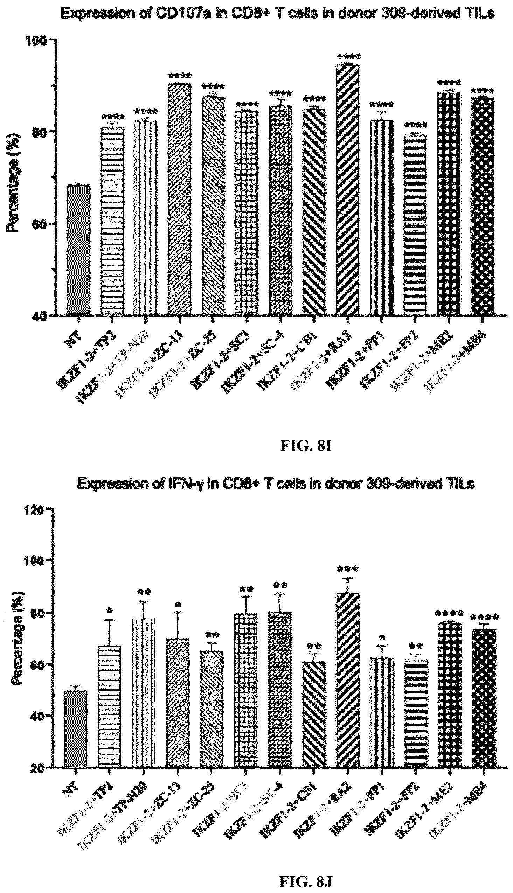

FIGs. 8F, 8G, 8H, 8I, and 8J show the cytokine expression ratios of the combination gene-edited TILs in the no stimulation group.

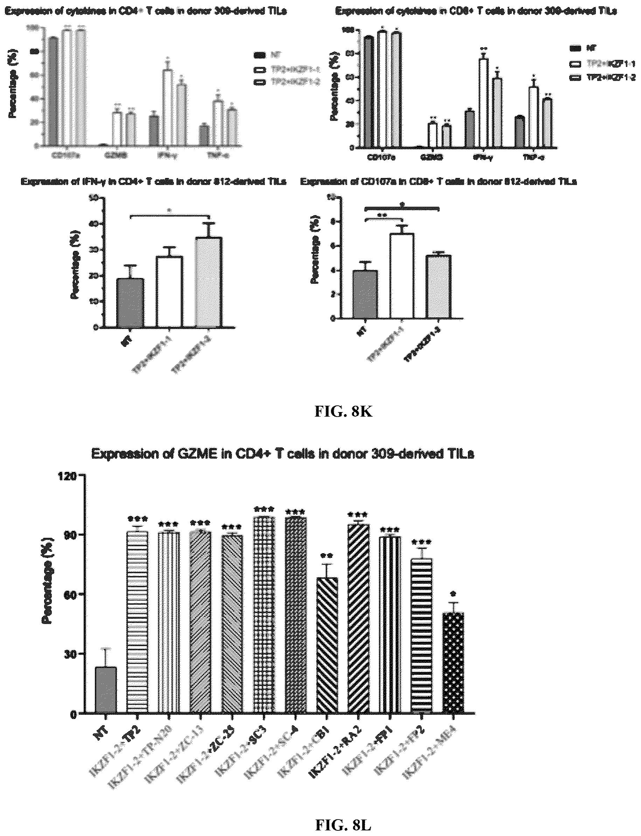

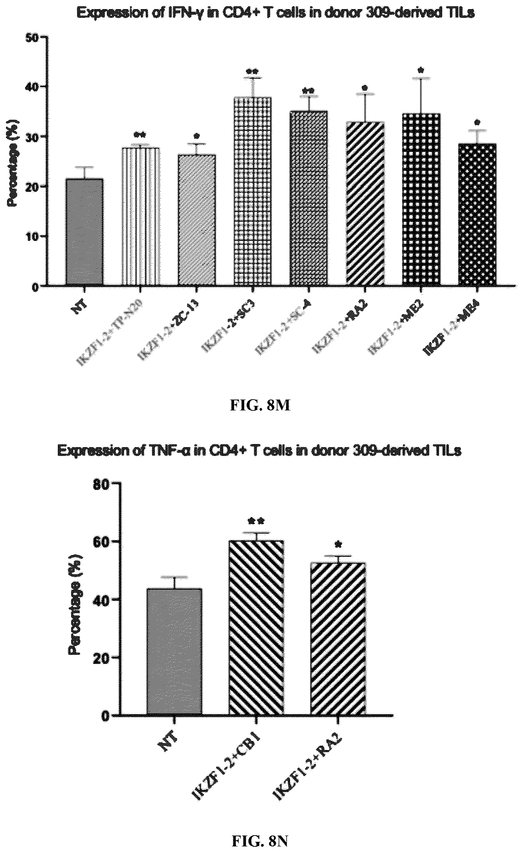

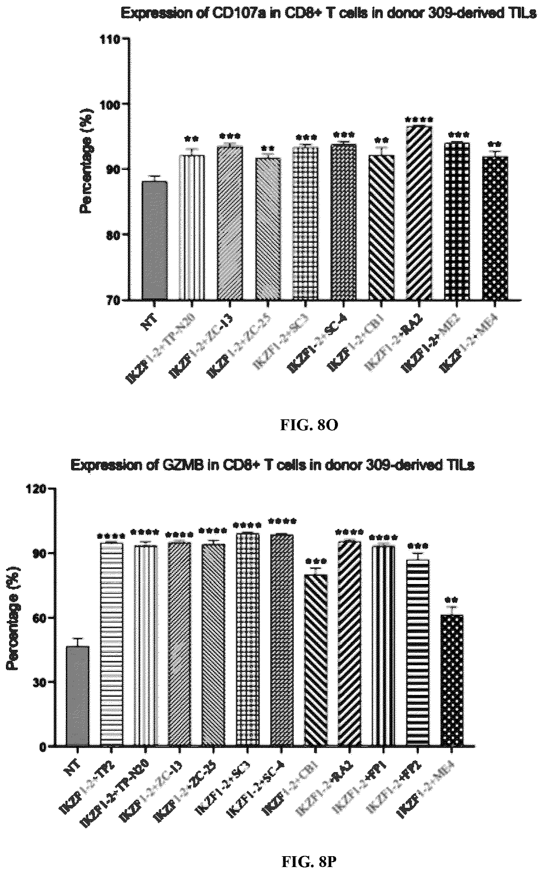

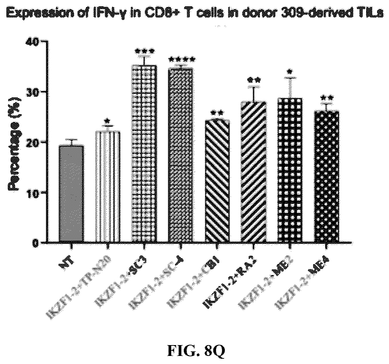

FIGs. 8K, 8L, 8M, 8N, 8O, 8P, and 8Q show the cytokine expression ratios of the combination gene-edited TILs of the stimulation group.

The results show that the TIL cells gene edited with the combination target of the present invention have higher functional cytokine expression capacity.

### Example 5

According to the experimental method in the example, the knockout effect of sgRNA targeting ADNP, NFKBIA, PTPN6, and TNIP1 was detected.

### (A) Knockout Efficiency

| sgRNA Index | Knockout Efficiency (%) | sgRNA Index | Knockout Efficiency (%) | sgRNA Index | Knockout Efficiency (%) |
|---|---|---|---|---|---|
| ADNP-2 | A | TNIP1-3 | A | TN1-6 | A |
| ADNP-4 | A | TN1-1 | A | TN1-7 | A |
| NFKBIA-3 | A | TN1-2 | B | TN1-8 | A |
| NFKBIA-4 | A | TN1-3 | A | TN1-9 | A |
| PTPN6-1 | A | TN1-4 | B | TN1-10 | A |
| PTPN6-2 | A | TN1-5 | B | TN1-11 | B |
| TNIP1-1 | A | | | | |

Among them, A means that the knockout efficiency was greater than or equal to 70%, and B means that the knockout efficiency was greater than or equal to 50% and less than 70%.

Among them, donor 045 was a healthy donor, donor 026 was a healthy donor, who were used to separate PBMC cells and then transduce TCR as TCR-T cells. Among them, donor 107 was a patient with lung adenocarcinoma, donor 812 was a patient with malignant melanoma, and donor 504 was a patient with ovarian cancer.

### (B) Proliferation Ability

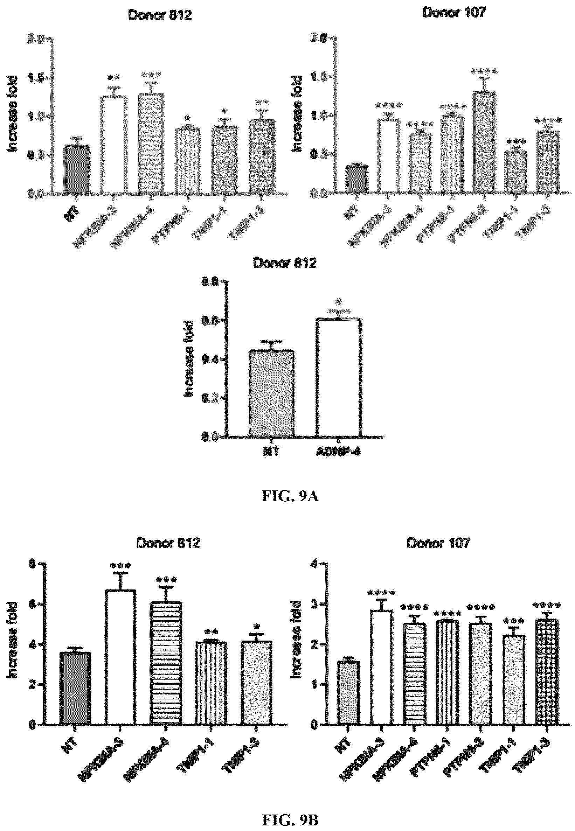

FIG9A shows the expansion fold change of TILs edited with the target gene of the present invention in the no stimulation group.

FIG9B shows the expansion fold change of TILs edited with the target gene of the present invention in the TransACT stimulation group.

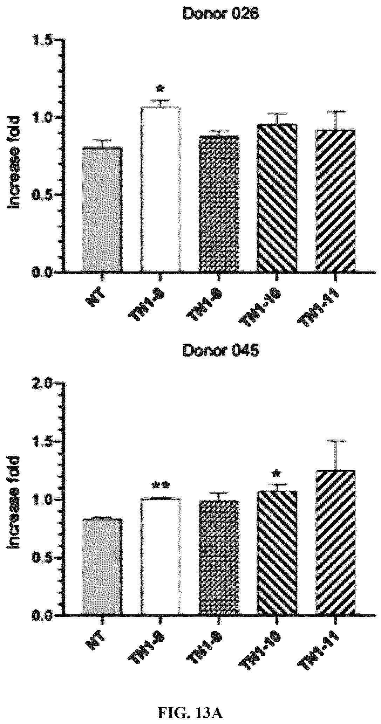

FIG. 13A shows that TNIP1 gene-edited TCR T cells in the TransACT stimulation group can have significant expansion capacity.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved expansion capacity compared to the unedited control group (NT).

### (C) Killing Ability

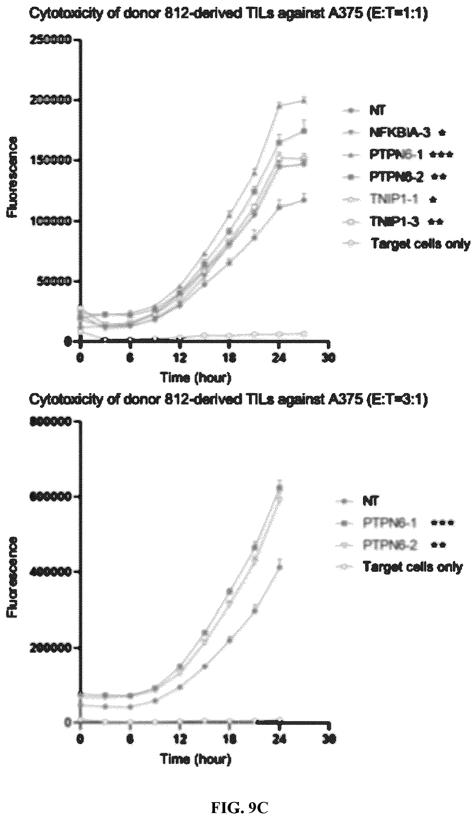

FIG9C shows the target cell killing ability of the TIL cells with the target gene of the present invention edited from donor 812.

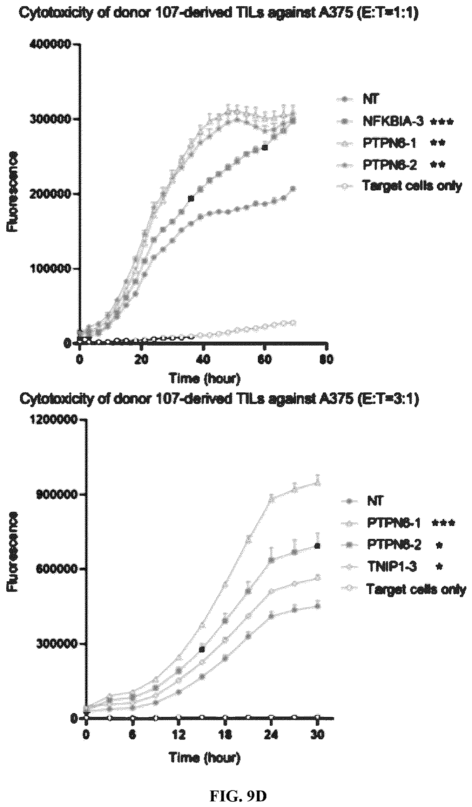

FIG9D shows the target cell killing ability of the TIL cells from donor 107 and with the target gene of the present invention edited.

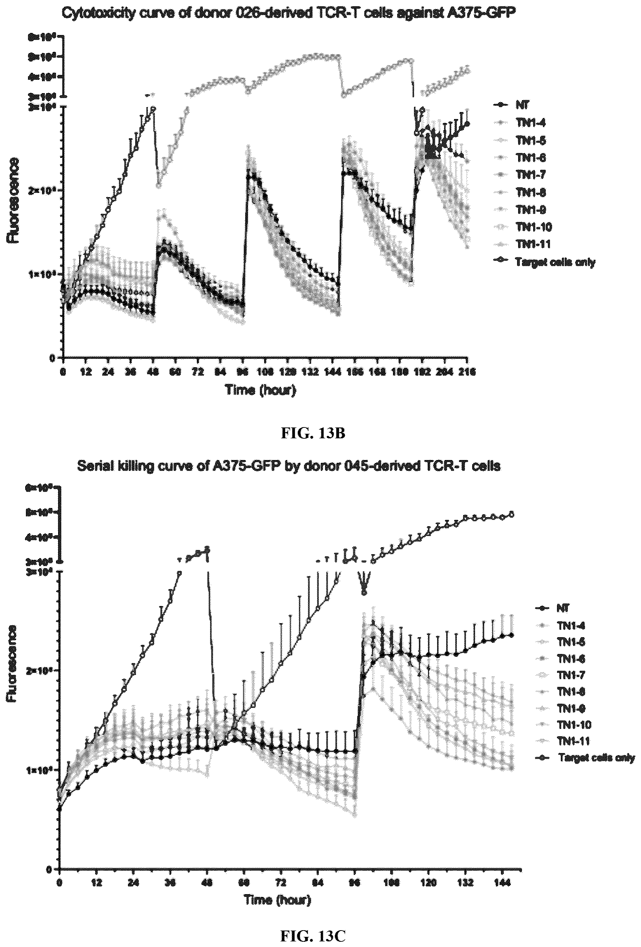

FIG. 13B and FIG. 13C show the target cell killing ability of TNIP1 gene-edited TCR T cells.

The significant difference is relative to the unedited NT group, * indicates P < 0.05, ** indicates P < 0.01, *** indicates P < 0.001, and **** indicates P < 0.0001. The results show that the target gene-edited T cells of the present invention can have significantly improved target cell killing ability and/or continuous killing ability compared with the control group (NT).

### (D) Flow Cytometry

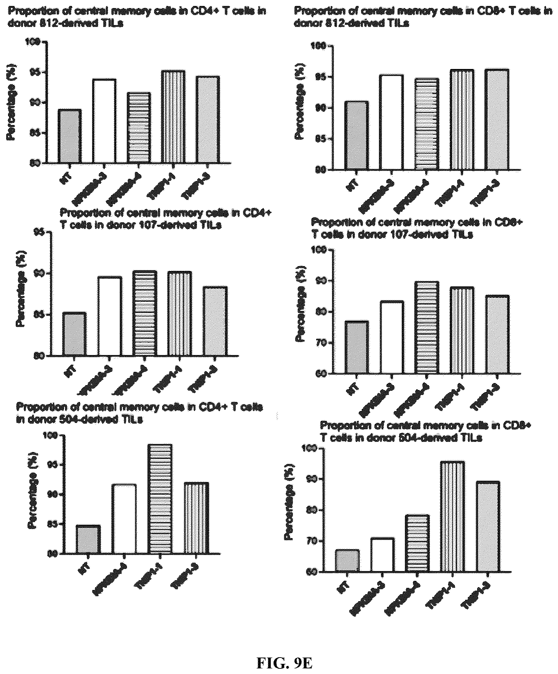

FIG 9E shows the proportion of central memory T cells in TIL cells after target gene editing of the present invention.

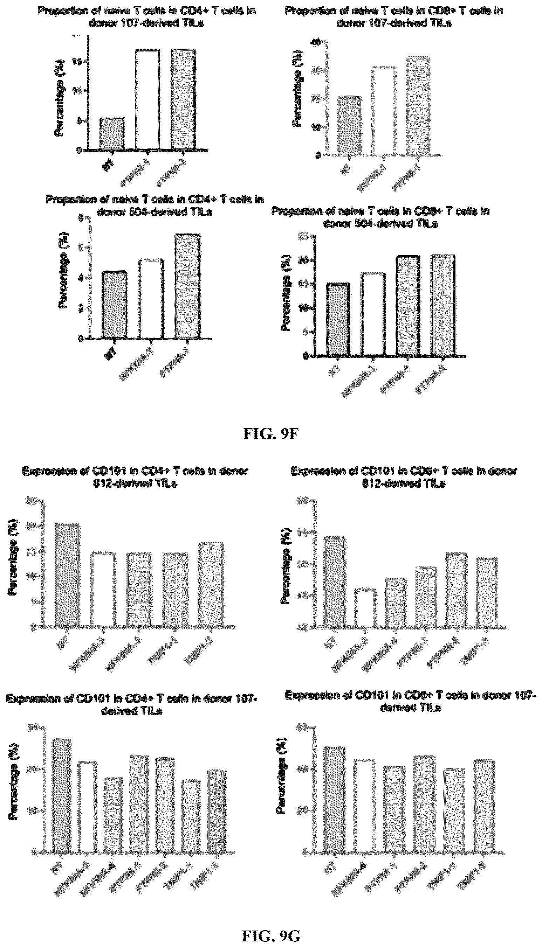

FIG 9F shows the proportion of naive T cells in TIL cells after target gene editing of the present invention.

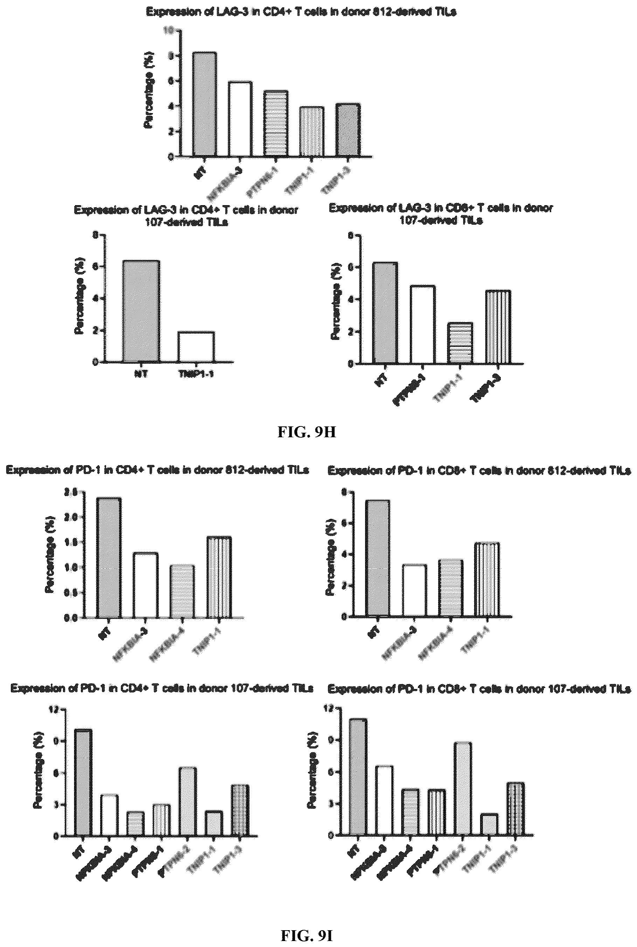

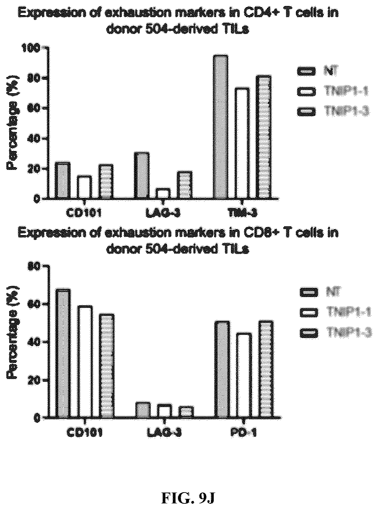

FIGs. 9G, 9H, 9I and 9J show the proportion of exhausted T cells in TIL cells after target gene editing of the present invention.

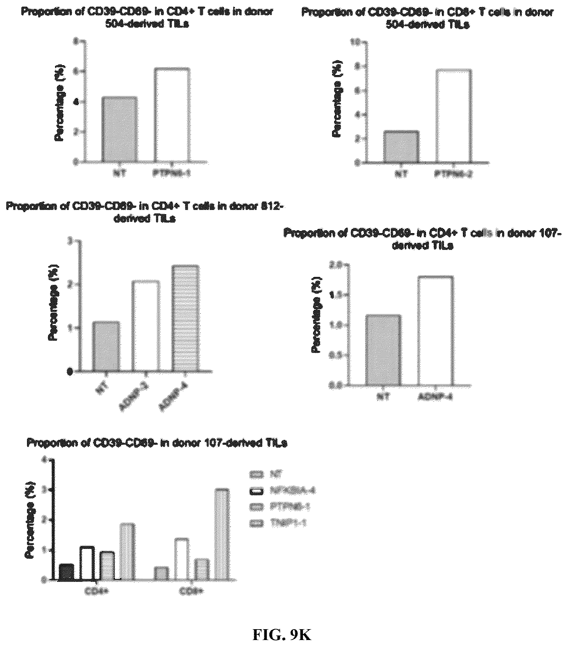

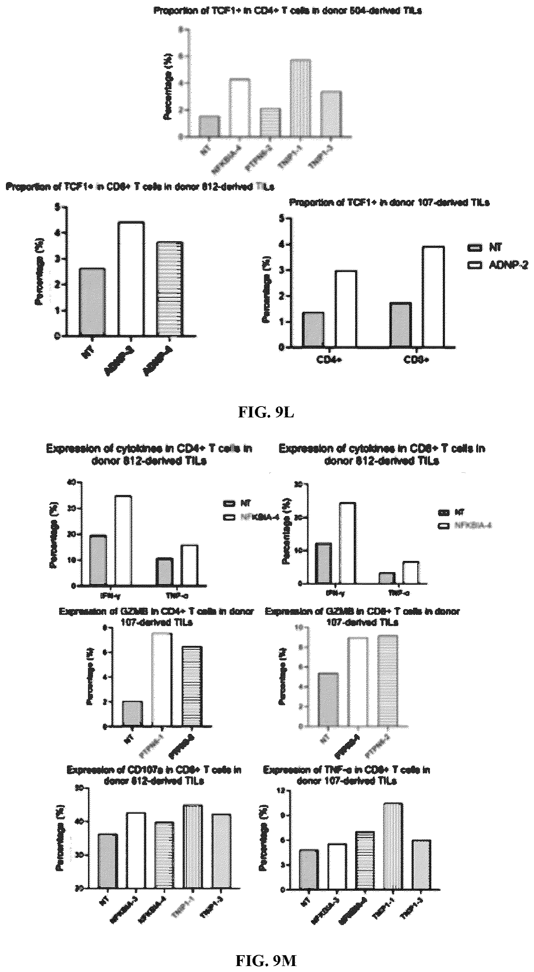

FIG. 9K and FIG. 9L show the proportion of stem T cells in TIL cells after target gene editing of the present invention.

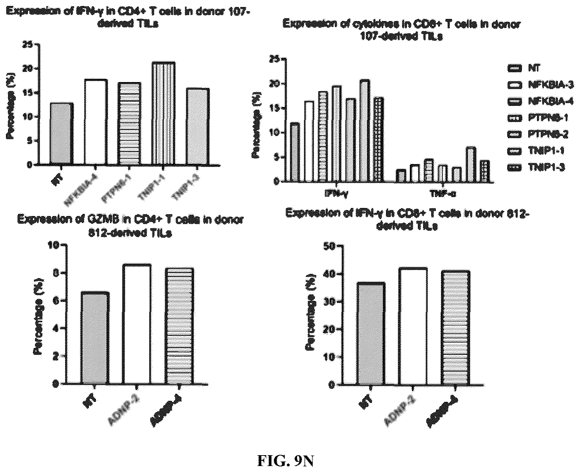

FIG. 9M and FIG. 9N show the cytokine expression ratios in TIL cells after target gene editing of the present invention in the non-stimulation group.

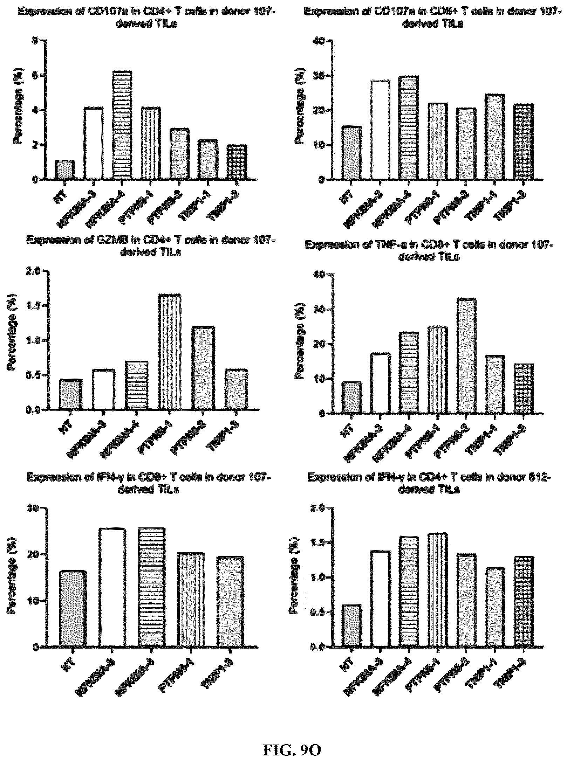

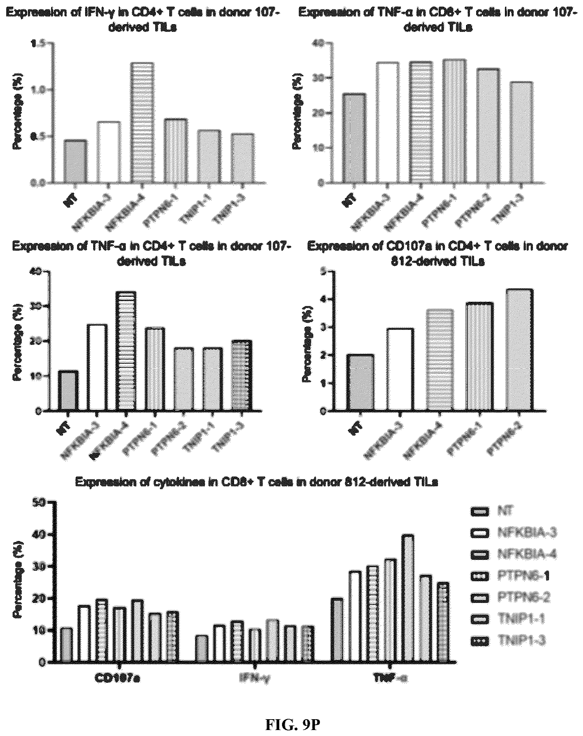

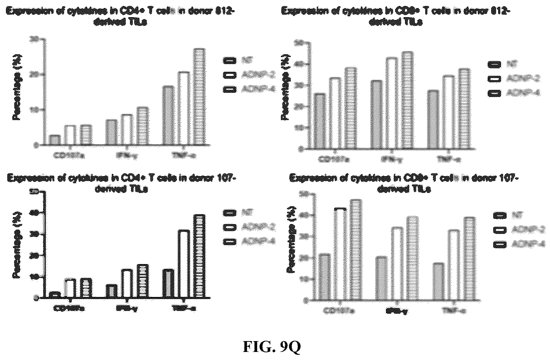

FIGs. 9O, 9P and 9Q show the cytokine expression ratios in TIL cells in the stimulation group after target gene editing of the present invention

The results show that compared to the control group (NT), the target gene-edited T cells of the present invention can have significantly improved cytokine expression capacity.

### (E) Multiple Rounds of Killing (or "Continuous Killing") Capability

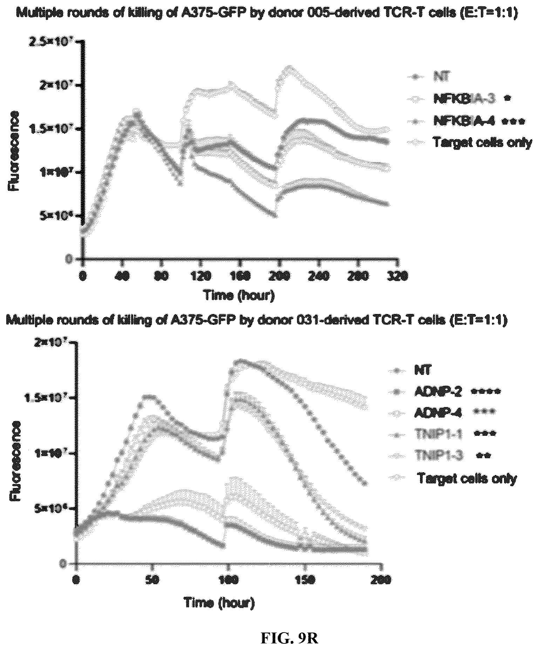

FIG. 9R shows the results of multiple rounds of killing by TCR T cells that were not edited or cells in which the target of the present invention was knocked out.

The results showed that after multiple rounds of killing, the cells with the target of the present invention knocked out had a stronger ability to kill tumor cells than the unedited cells. The significant difference was relative to the unedited NT group, * indicates P<0.05, ** indicates P<0.01, and *** indicates P<0.001.

### (F) Cytokine Release Ability

The CBA kit was used to detect the cytokine release of cells that were not edited or cells in which the target of the present invention was knocked out.

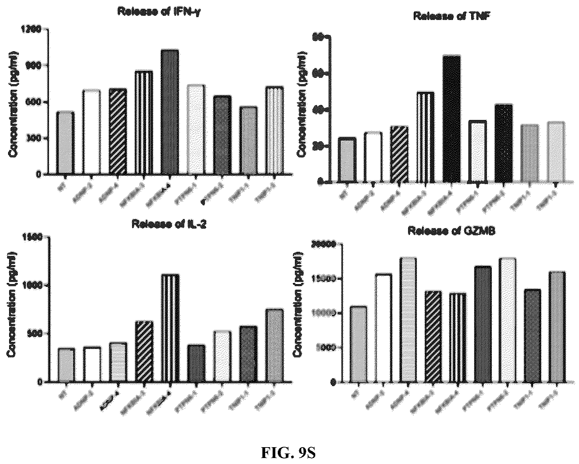

FIG. 9S shows the cytokine release as tested using a CBA kit of TCR T cells that were not edited or cells in which the target of the present invention was knocked out.

FIGs. 13D, 13E, 13F, and 13G show the cytokine release of TCR T cells without editing or cells with knockout of TNIP1 target detected by CBA kit.

The results show that compared to the control group (NT), the target gene-edited T cells of the present invention can have significantly improved cytokine release capacity.

### Example 6

According to the test method in the example, the knockout effect of a combination of sgRNAs targeting two or more selected from ADNP, NFKBIA, PTPN6, TNIP1 and optionally other targets was detected.

When the target gene was edited in TIL cells, IKZF1 and TNFAIP3 of the present invention were simultaneously knocked out. The knockout efficiency of each target was detected by the cell knockout efficiency detection method of the above example. Among them, donor 309 was a patient with cervical cancer, donor 812 was a patient with malignant melanoma, and donor 504 was a patient with endometrial cancer.

| Combination | sgRNA Index | Knockout Efficiency |
|---|---|---|
| TP2+ADNP-2 | TP2 | A |
| | ADNP-2 | A |
| TP2+ADNP-4 | TP2 | A |
| | ADNP-4 | A |
| TP2+NFKBIA-3 | TP2 | A |
| | NFKBIA-3 | A |
| TP2+NFKBIA-4 | TP2 | A |
| | NFKBIA-4 | A |
| TP2+PTPN6-1 | TP2 | A |
| | PTPN6-1 | A |
| TP2+PTPN6-2 | TP2 | A |
| | PTPN6-2 | A |
| TP2+TNIP1-1 | TP2 | A |
| | TNIP1-1 | A |
| TP2+TNIP 1-3 | TP2 | A |
| | TNIP1-3 | B |
| IKZF1-2+ TNIP1-1 | IKZF1-2 | A |
| | TNIP1-1 | A |

Among them, A means that the knockout efficiency was greater than or equal to 70%, and B means that the knockout efficiency was greater than or equal to 50% and less than 70%.

### (B) Expansion

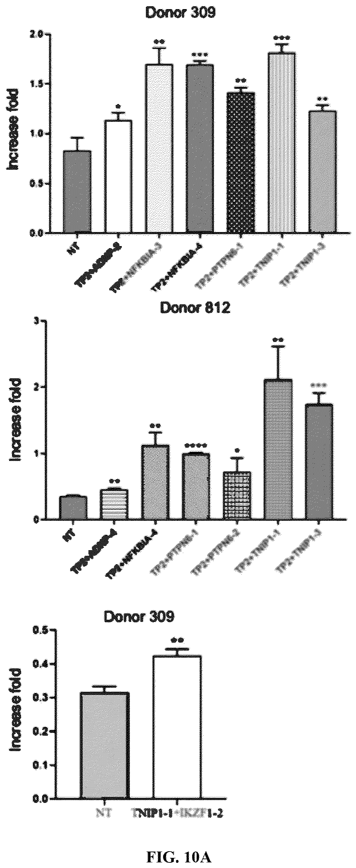

FIG. 10A shows the expansion fold change of TILs gene edited with the combination target of the present invention in the no stimulation group.

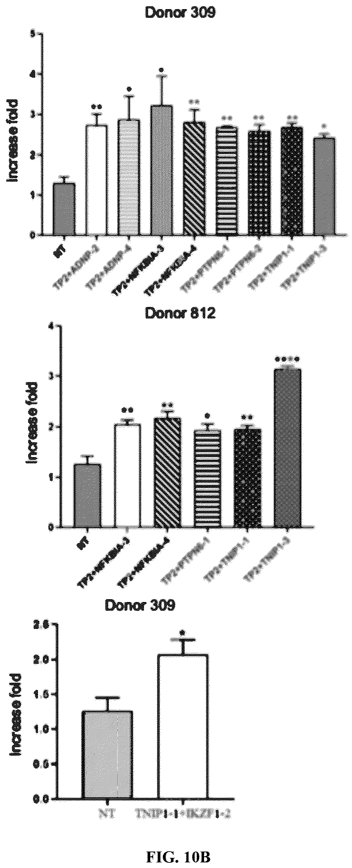

FIG. 10B shows the expansion fold change of TILs gene edited with the combination target of the present invention in the TransACT stimulation group.

The significant difference is relative to the unedited NT group, * indicates P < 0.05, ** indicates P < 0.01, *** indicates P < 0.001, and **** indicates P < 0.0001. The results show that compared to the gene editing control group (NT), the TIL cells gene edited with the combination target of the present invention can have significantly improved expansion capacity.

### (C) Killing Ability

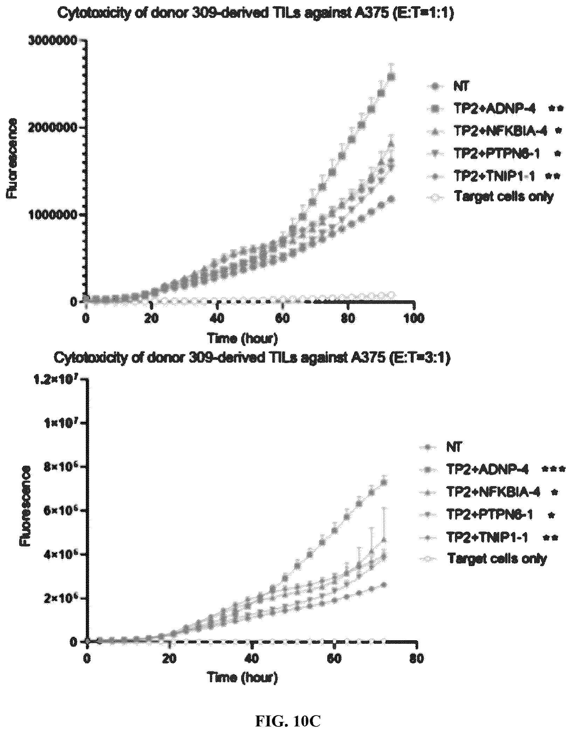

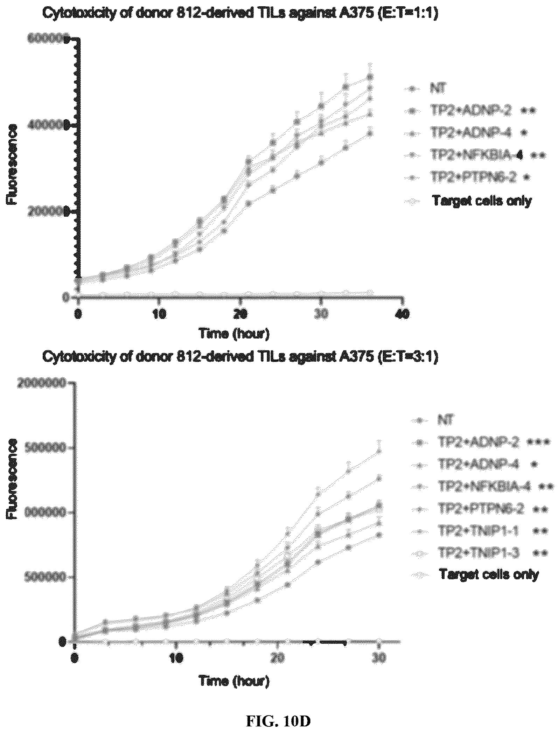

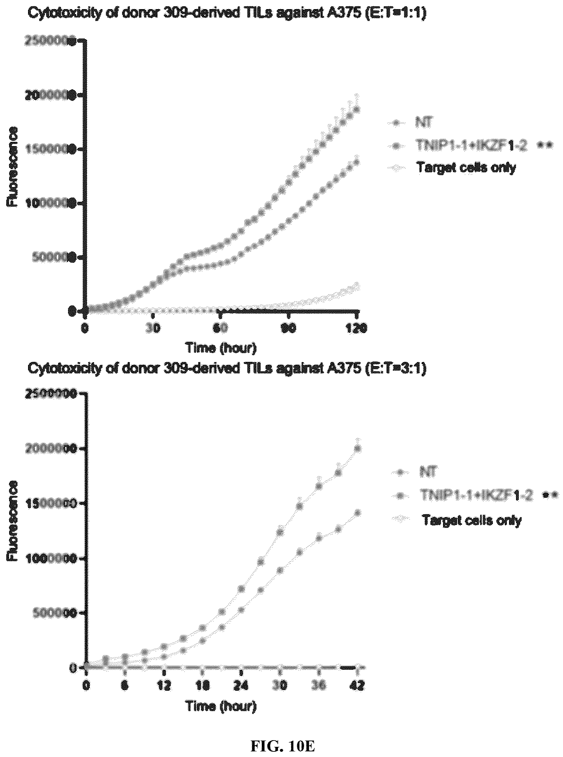

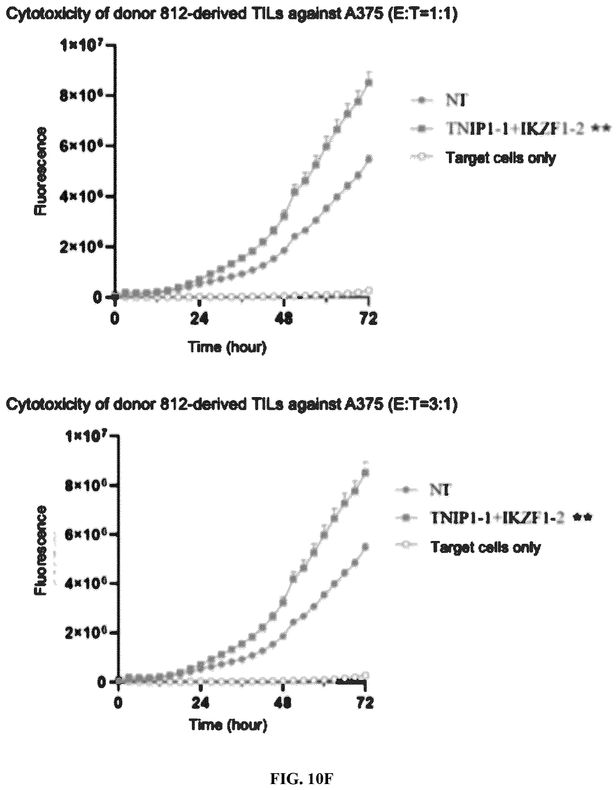

FIGs. 10C, 10D, 10E and 10F show the target cell killing ability of TILs gene edited with the combination target of the present invention.

The significant difference is relative to the unedited NT group, * indicates P < 0.05, ** indicates P < 0.01, *** indicates P < 0.001, and **** indicates P < 0.0001. The results show that compared to the control group (NT), the target combination gene-edited TIL cells of the present invention can have significantly improved target cell killing ability.

### (D) Cytokine Expression and Release

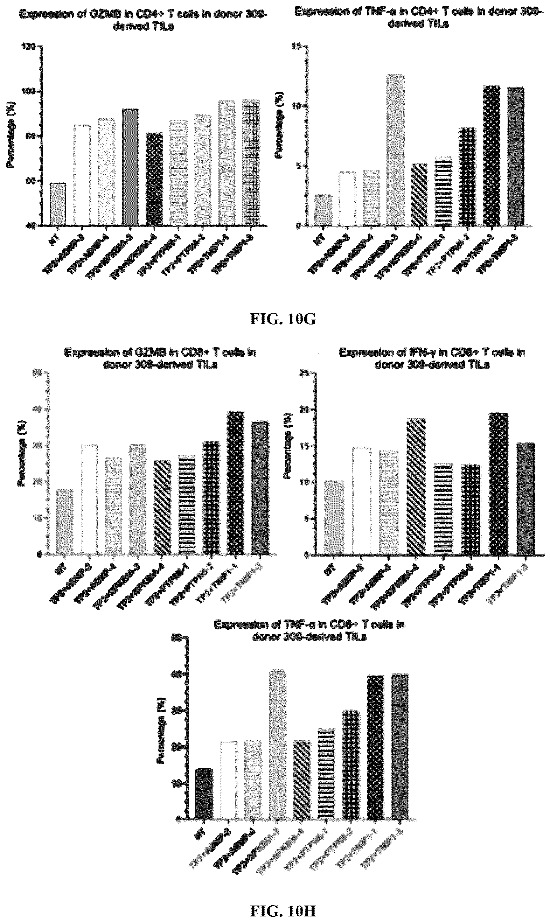

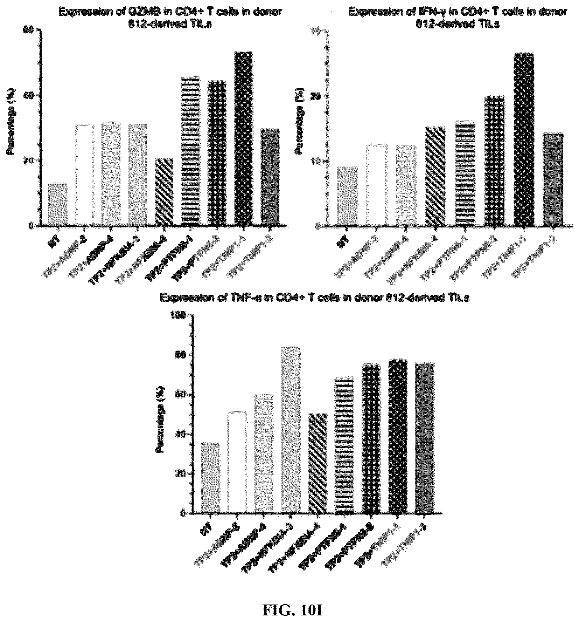

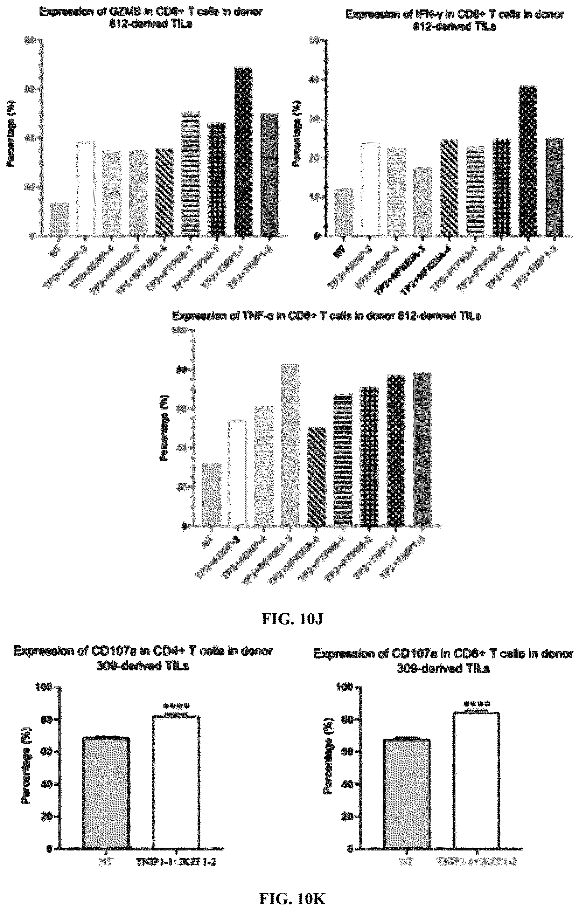

FIGs. 10G, 10H, 10I, 10J and 10K show the cytokine expression ratios of TILs gene edited with the combination target of the present invention in the no stimulation group.

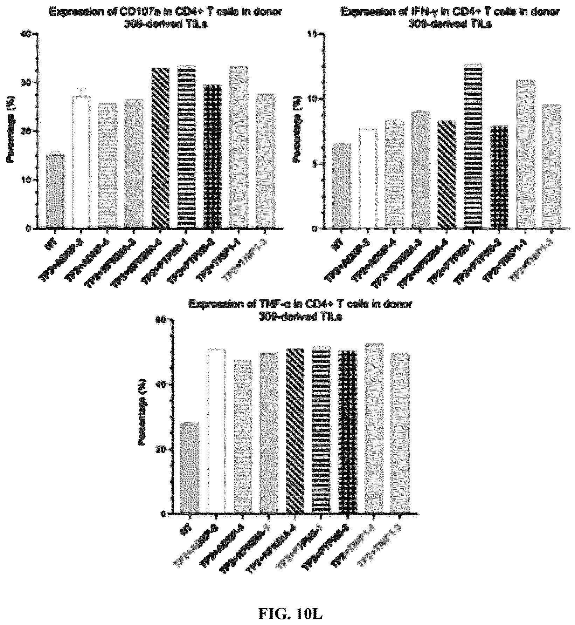

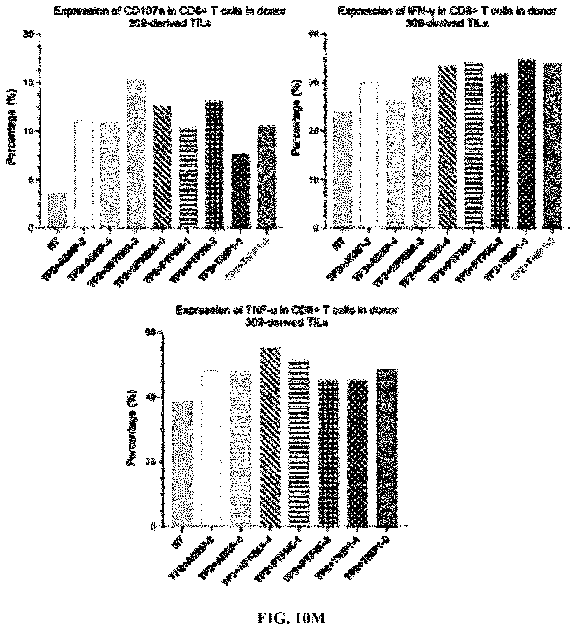

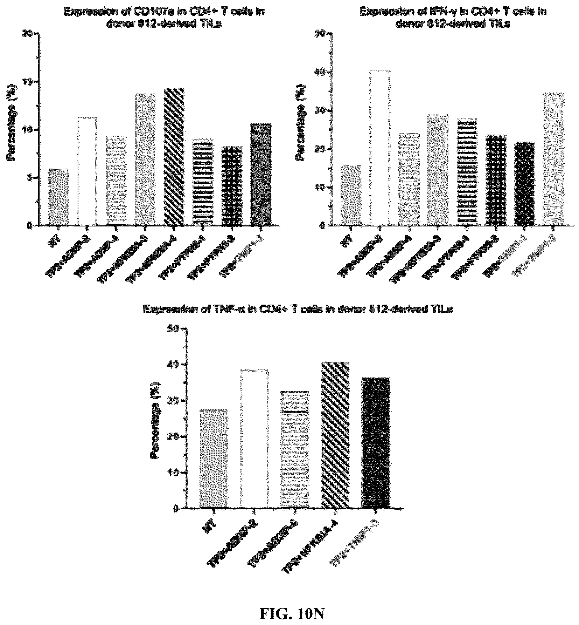

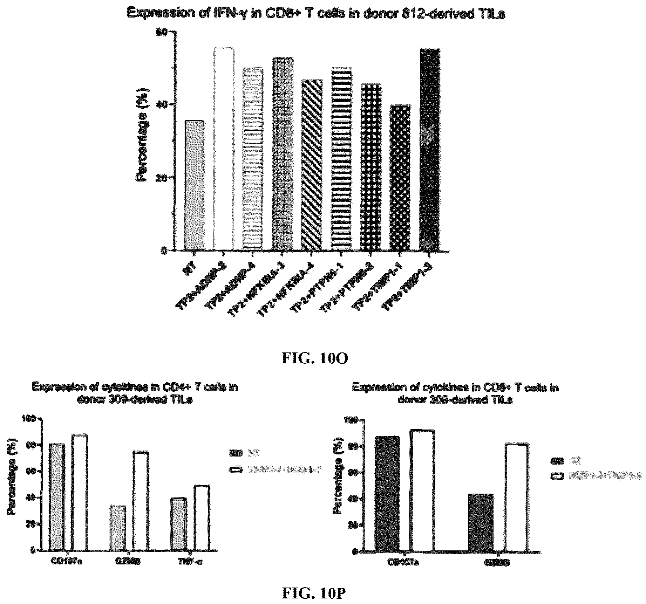

FIGs. 10L, 10M, 10N, 10O and 10P show the cytokine expression ratios of TILs gene edited with the combination target of the present invention in the stimulation group.

The results show that the TIL cells gene edited with the combination target of the present invention have higher functional cytokine expression capacity.

### (E) Cytokine Release Capacity

The CBA kit was used to detect the cytokine release of cells that were not edited or cells in which the target of the present invention was knocked out.

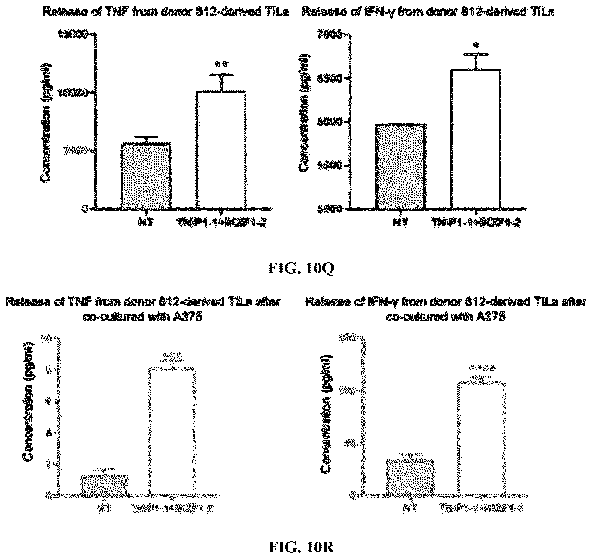

FIG. 10 Q shows the cytokine secretion of TIL cells in the transACT stimulation group.

FIG10R shows the secretion of cytokines by T cells after co-culture of TIL cells with target cells A375 for 24 hours.

The results show that compared to the control group (NT), the target gene-edited T cells of the present invention can have significantly improved cytokine release capacity.

### Example 7 In Vivo Efficacy Testing of Gene-Edited Cells

Immunodeficient mice (NOG mice, Vital River, strain code 408) were subcutaneously inoculated with A375 cells at a number of approximately 1×10⁶ cell/mouse. 7 days after inoculation, when the tumor volume reaches 50-80mm³, depending on the size of the tumor, the mice were randomly divided into groups according to the regimen of 1-3 × 10⁷ cells/mouse and 0.6E5*Bid*3 IL-2 injection volume (IU).

The day when each group of TCR-T cells with sgRNA number provided by the present invention for gene editing was injected into the tail vein was day 0. At the same time, IL-2 was injected intraperitoneally once every 12 hours for 6 consecutive times. The tumor volume was measured twice a week, and the tumor growth inhibition rate (TGI) was calculated on about day 28. The results showed that the mice in the experimental group produced better tumor control effects and/or functional cytokine release in vivo compared to the NT group, proving that the tumor inhibition effect of the cells knocked out by sgRNA provided by the present invention was significantly stronger than that of the NT group.

The foregoing detailed description is provided by way of explanation and examples, and is not intended to limit the scope of the appended claims. Various changes to the embodiments of the present invention are obvious to those of ordinary skill in the art and are intended to be within the scope of the appended claims and their equivalents.

**Table 1A. Genomic Coordinates of Preferred Human RASA2 Target Subregions**

| Region index | Coordinate range of subregion |
|---|---|
| RASA2 R_1 | chr3:141485587-141485807 |
| RASA2 R_2 | chr3:141485907-141486327 |
| RASA2 R_3 | chr3:141486507-141486747 |
| RASA2 R_4 | chr3:141486907-141487007 |
| RASA2 R_5 | chr3:141529717-141529763 |
| RASA2 R_6 | chr3:141529770-141529843 |
| RASA2 R_7 | chr3:141553837-141553966 |
| RASA2 R_8 | chr3:141570837-141570990 |
| RASA2 R_9 | chr3:141571472-141571547 |
| RASA2 R_10 | chr3:141572605-141572684 |
| RASA2 R_11 | chr3:141572690-141572804 |
| RASA2 R_12 | chr3:141573157-141573222 |
| RASA2 R_13 | chr3:141574006-141574049 |
| RASA2 R_14 | chr3:141576983-141577032 |
| RASA2 R_15 | chr3:141577063-141577131 |
| RASA2 R_16 | chr3:141580395-141580485 |
| RASA2 R_17 | chr3:141586726-141586864 |
| RASA2 R_18 | chr3:141608480-141608679 |
| RASA2 R_19 | chr3:141608687-141608714 |
| RASA2 R_20 | chr3:141609482-141609524 |
| RASA2 R_21 | chr3:141612412-141612507 |
| RASA2 R_22 | chr3:141612768-141612863 |
| RASA2 R_23 | chr3:141486577-141487341 |
| RASA2 R_24 | chr3:141512136-141512298 |

**Table 1B. Genomic Coordinates of Preferred Human FIBP Target Subregions**

| Region index | Coordinate range of subregion |
|---|---|
| FIBP R_1 | chr11:65889831-65889961 |
| FIBP R_2 | chr11:65889591-65889791 |
| FIBP R_3 | chr11:65889341-65889441 |
| FIBP R_4 | chr11:65888741-65889051 |
| FIBP R_5 | chr11:65888491-65888641 |

**Table 1C. Genomic Coordinates of Preferred Human MED12 Target Subregions**

| | |
|---|---|
| Region index | Coordinate range of subregion |
| MED12 R_1 | chrX:71117116-71117496 |
| MED12 R_2 | chrX:71117636-71117816 |
| MED12 R_3 | chrX:71117886-71118126 |
| MED12 R_4 | chrX:71118156-71118406 |

**Table 1D. Genomic Coordinates of Preferred Human TIGIT Target Subregions**

| Region index | Coordinate range of subregion |
|---|---|
| TIGIT R_1 | chr3:114292558-114292738 |
| TIGIT R_2 | chr3:114292918-114293108 |
| TIGIT R_3 | chr3:114293168-114293358 |
| TIGIT R_4 | chr3:114293658-114293948 |

**Table 1E. Genomic Coordinates of Preferred Human BRD4 Target Subregions**

| Region index | Coordinate range of subregion |
|---|---|
| BRD4 R_1 | chr19:15333439-15333739 |
| BRD4 R_2 | chr19:15333269-15333389 |
| BRD4 R_3 | chr19:15332939-15333059 |
| BRD4 R_4 | chr19:15332539-15332839 |

**Table 1F. Genomic Coordinates of Preferred Human IKZF1 Target Subregions**

| Region index | Coordinate range of subregion |
|---|---|
| IKZF1 R_1 | chr7: 50301965-50302615 |
| IKZF1 R_2 | chr7: 50302755-50303005 |
| IKZF1 R_3 | chr7: 50303135-50303425 |
| IKZF1 R_4 | chr7:50327705-50327877 |
| IKZF1 R_5 | chr7:50382595-50382830 |
| IKZF1 R_6 | chr7:50399941-50400296 |
| IKZF1 R_7 | chr7:50400527-50400604 |
| IKZF1 R_8 | chr7:50400848-50401107 |
| IKZF1 R_9 | chr7:50401112-50401195 |
| IKZF1 R_10 | chr7:50401215-50401542 |
| IKZF1 R_11 | chr7:50400609-50400790 |

**Table 1G. Genomic Coordinates of Preferred Human ADNP Target Subregions**

| Region index | Coordinate range of subregion |
|---|---|
| ADNP R_1 | chr20: 50932267-50932907 |
| ADNP R_2 | chr20: 50931447-50931957 |

**Table 1H. Genomic Coordinates of Preferred Human NFKBIA Target Subregions**

| Region index | Coordinate range of subregion |
|---|---|
| NFKBIA R_1 | chr14: 35406149-35406239 |
| NFKBIA R_2 | chr14: 35404959-35405949 |

**Table 1I. Genomic Coordinates of Preferred Human PTPN6 Target Subregions**

| Region index | Coordinate range of subregion |
|---|---|
| PTPN6 R_1 | chr12: 6945117-6945227 |
| PTPN6 R_2 | chr12: 6945357-6945657 |
| PTPN6 R_3 | chr12: 6945857-6946557 |

**Table 1J. Genomic Coordinates of Preferred Human TNIP1 Target Subregions**

| Region index | Coordinate range of subregion |
|---|---|
| TNIP1 R_1 | chr5: 151088415-151089175 |
| TNIP1 R_2 | chr5: 151088125-151088305 |
| TNIP1 R_3 | chr5: 151087715-151087835 |
| TNIP1 R_4 | chr5:151029972-151030223 |
| TNIP1 R_5 | chr5:151030278-151030858 |
| TNIP1 R_6 | chr5:151035040-151035193 |
| TNIP1 R_7 | chr5:151035459-151035684 |
| TNIP1 R_8 | chr5:151039192-151039352 |
| TNIP1 R_9 | chr5:151042523-151043011 |
| TNIP1 R_10 | chr5:151045907-151046077 |
| TNIP1 R_11 | chr5:151049699-151049974 |
| TNIP1 R_12 | chr5:151056860-151057084 |
| TNIP1 R_13 | chr5:151060196-151060522 |

**Table 2A. Genomic Coordinates of Human RASA2 in the Current Invention**

| Index | Chromosomal coordinate range | Index | Chromosomal coordinate range |
|---|---|---|---|
| RASA2 1 | chr3: 141485526-141485553 | RASA2 547 | chr3: 141505097-141505124 |
| RASA2 2 | chr3: 141485527-141485554 | RASA2 548 | chr3: 141505098-141505125 |
| RASA2 3 | chr3: 141485543-141485570 | RASA2 549 | chr3: 141505112-141505139 |
| RASA2 4 | chr3: 141485548-141485575 | RASA2 550 | chr3: 141505153-141505180 |
| RASA2 5 | chr3: 141485552-141485579 | RASA2 551 | chr3: 141505166-141505193 |
| RASA2 6 | chr3: 141485577-141485604 | RASA2 552 | chr3: 141505204-141505231 |
| RASA2 7 | chr3: 141485583-141485610 | RASA2 553 | chr3: 141505205-141505232 |
| RASA2 8 | chr3: 141485602-141485629 | RASA2 554 | chr3: 141505208-141505235 |
| RASA29 | chr3: 141485603-141485630 | RASA2 555 | chr3: 141505209-141505236 |
| RASA2 10 | chr3: 141485609-141485636 | RASA2 556 | chr3: 141505243-141505270 |
| RASA2 11 | chr3: 141485610-141485637 | RASA2 557 | chr3: 141505266-141505293 |
| RASA2 12 | chr3: 141485634-141485661 | RASA2 558 | chr3: 141505267-141505294 |
| RASA2 13 | chr3: 141485662-141485689 | RASA2 559 | chr3: 141505268-141505295 |
| RASA2 14 | chr3: 141485670-141485697 | RASA2 560 | chr3: 141505273-141505300 |
| RASA2 15 | chr3: 141485675-141485702 | RASA2 561 | chr3: 141505286-141505313 |
| RASA2 16 | chr3: 141485676-141485703 | RASA2 562 | chr3: 141505346-141505373 |
| RASA2 17 | chr3: 141485683-141485710 | RASA2 563 | chr3: 141505364-141505391 |
| RASA2 18 | chr3: 141485697-141485724 | RASA2 564 | chr3: 141505365-141505392 |
| RASA2 19 | chr3: 141485737-141485764 | RASA2 565 | chr3: 141505388-141505415 |
| RASA2 20 | chr3: 141485743-141485770 | RASA2 566 | chr3: 141505396-141505423 |
| RASA2 21 | chr3: 141485777-141485804 | RASA2 567 | chr3: 141505434-141505461 |
| RASA2 22 | chr3: 141485778-141485805 | RASA2 568 | chr3: 141505441-141505468 |
| RASA2 23 | chr3: 141485779-141485806 | RASA2 569 | chr3: 141505442-141505469 |
| RASA2 24 | chr3: 141485781-141485808 | RASA2 570 | chr3: 141505447-141505474 |
| RASA2 25 | chr3: 141485804-141485831 | RASA2 571 | chr3: 141505451-141505478 |
| RASA2 26 | chr3: 141485813-141485840 | RASA2 572 | chr3: 141505452-141505479 |
| RASA2 27 | chr3: 141485819-141485846 | RASA2 573 | chr3: 141505492-141505519 |
| RASA2 28 | chr3: 141485825-141485852 | RASA2 574 | chr3: 141505516-141505543 |
| RASA2 29 | chr3: 141485830-141485857 | RASA2 575 | chr3: 141505552-141505579 |
| RASA2 30 | chr3: 141485853-141485880 | RASA2 576 | chr3: 141505553-141505580 |
| RASA2 31 | chr3: 141485858-141485885 | RASA2 577 | chr3: 141505554-141505581 |
| RASA2 32 | chr3: 141485859-141485886 | RASA2 578 | chr3: 141505555-141505582 |
| RASA2 33 | chr3: 141485860-141485887 | RASA2 579 | chr3: 141505575-141505602 |
| RASA2 34 | chr3: 141485875-141485902 | RASA2 580 | chr3: 141505607-141505634 |
| RASA2 35 | chr3: 141485880-141485907 | RASA2 581 | chr3: 141505610-141505637 |
| RASA2 36 | chr3: 141485883-141485910 | RASA2 582 | chr3: 141505611-141505638 |
| RASA2 37 | chr3: 141485892-141485919 | RASA2 583 | chr3: 141505615-141505642 |
| RASA2 38 | chr3: 141485896-141485923 | RASA2 584 | chr3: 141505618-141505645 |
| RASA2 39 | chr3: 141485906-141485933 | RASA2 585 | chr3: 141505619-141505646 |
| RASA2 40 | chr3: 141485937-141485964 | RASA2 586 | chr3: 141505624-141505651 |
| RASA2 41 | chr3: 141485938-141485965 | RASA2 587 | chr3: 141505638-141505665 |
| RASA2 42 | chr3: 141485939-141485966 | RASA2 588 | chr3: 141505655-141505682 |
| RASA2 43 | chr3: 141485943-141485970 | RASA2 589 | chr3: 141505665-141505692 |
| RASA2 44 | chr3: 141485957-141485984 | RASA2 590 | chr3: 141505666-141505693 |
| RASA2 45 | chr3: 141485958-141485985 | RASA2 591 | chr3: 141505672-141505699 |
| RASA2 46 | chr3: 141485964-141485991 | RASA2 592 | chr3: 141505681-141505708 |
| RASA2 47 | chr3: 141485971-141485998 | RASA2 593 | chr3: 141505708-141505735 |
| RASA2 48 | chr3: 141485975-141486002 | RASA2 594 | chr3: 141505709-141505736 |
| RASA2 49 | chr3: 141485985-141486012 | RASA2 595 | chr3: 141505713-141505740 |
| RASA2 50 | chr3: 141485986-141486013 | RASA2 596 | chr3: 141505780-141505807 |
| RASA2 51 | chr3: 141485989-141486016 | RASA2 597 | chr3: 141505801-141505828 |
| RASA2 52 | chr3: 141486012-141486039 | RASA2 598 | chr3: 141505853-141505880 |
| RASA2 53 | chr3: 141486019-141486046 | RASA2 599 | chr3: 141505891-141505918 |
| RASA2 54 | chr3: 141486020-141486047 | RASA2 600 | chr3: 141505892-141505919 |
| RASA2 55 | chr3: 141486047-141486074 | RASA2 601 | chr3: 141505899-141505926 |
| RASA2 56 | chr3: 141486063-141486090 | RASA2 602 | chr3: 141505919-141505946 |
| RASA2 57 | chr3: 141486064-141486091 | RASA2 603 | chr3: 141505931-141505958 |
| RASA2 58 | chr3: 141486086-141486113 | RASA2 604 | chr3: 141505942-141505969 |
| RASA2 59 | chr3: 141486098-141486125 | RASA2 605 | chr3: 141505943-141505970 |
| RASA2 60 | chr3: 141486108-141486135 | RASA2 606 | chr3: 141505956-141505983 |
| RASA2 61 | chr3: 141486109-141486136 | RASA2 607 | chr3: 141505999-141506026 |
| RASA2 62 | chr3: 141486124-141486151 | RASA2 608 | chr3: 141506002-141506029 |
| RASA2 63 | chr3: 141486125-141486152 | RASA2 609 | chr3: 141506054-141506081 |
| RASA2 64 | chr3: 141486129-141486156 | RASA2 610 | chr3: 141506057-141506084 |
| RASA2 65 | chr3: 141486130-141486157 | RASA2 611 | chr3: 141506058-141506085 |
| RASA2 66 | chr3: 141486133-141486160 | RASA2 612 | chr3: 141506109-141506136 |
| RASA2 67 | chr3: 141486134-141486161 | RASA2 613 | chr3: 141506141-141506168 |
| RASA2 68 | chr3: 141486151-141486178 | RASA2 614 | chr3: 141506193-141506220 |
| RASA2 69 | chr3: 141486162-141486189 | RASA2 615 | chr3: 141506202-141506229 |
| RASA2 70 | chr3: 141486177-141486204 | RASA2 616 | chr3: 141506205-141506232 |
| RASA2 71 | chr3: 141486185-141486212 | RASA2 617 | chr3: 141506244-141506271 |
| RASA2 72 | chr3: 141486189-141486216 | RASA2 618 | chr3: 141506247-141506274 |
| RASA2 73 | chr3: 141486190-141486217 | RASA2 619 | chr3: 141506292-141506319 |
| RASA2 74 | chr3: 141486200-141486227 | RASA2 620 | chr3: 141506293-141506320 |
| RASA2 75 | chr3: 141486212-141486239 | RASA2 621 | chr3: 141506316-141506343 |
| RASA2 76 | chr3: 141486252-141486279 | RASA2 622 | chr3: 141506334-141506361 |
| RASA2 77 | chr3: 141486253-141486280 | RASA2 623 | chr3: 141506344-141506371 |
| RASA2 78 | chr3: 141486273-141486300 | RASA2 624 | chr3: 141506375-141506402 |
| RASA2 79 | chr3: 141486274-141486301 | RASA2 625 | chr3: 141506376-141506403 |
| RASA2 80 | chr3: 141486295-141486322 | RASA2 626 | chr3: 141506426-141506453 |
| RASA2 81 | chr3: 141486312-141486339 | RASA2 627 | chr3: 141509541-141509568 |
| RASA2 82 | chr3: 141486315-141486342 | RASA2 628 | chr3: 141509548-141509575 |
| RASA2 83 | chr3: 141486318-141486345 | RASA2 629 | chr3: 141509563-141509590 |
| RASA2 84 | chr3: 141486321-141486348 | RASA2 630 | chr3: 141509570-141509597 |
| RASA2 85 | chr3: 141486323-141486350 | RASA2 631 | chr3: 141509582-141509609 |
| RASA2 86 | chr3: 141486324-141486351 | RASA2 632 | chr3: 141509600-141509627 |
| RASA2 87 | chr3: 141486325-141486352 | RASA2 633 | chr3: 141509601-141509628 |
| RASA2 88 | chr3: 141486326-141486353 | RASA2 634 | chr3: 141509619-141509646 |
| RASA2 89 | chr3: 141486327-141486354 | RASA2 635 | chr3: 141509640-141509667 |
| RASA2 90 | chr3: 141486328-141486355 | RASA2 636 | chr3: 141509680-141509707 |
| RASA2 91 | chr3: 141486329-141486356 | RASA2 637 | chr3: 141509683-141509710 |
| RASA2 92 | chr3: 141486359-141486386 | RASA2 638 | chr3: 141509684-141509711 |
| RASA2 93 | chr3: 141486360-141486387 | RASA2 639 | chr3: 141509692-141509719 |
| RASA2 94 | chr3: 141486373-141486400 | RASA2 640 | chr3: 141509693-141509720 |
| RASA2 95 | chr3: 141486380-141486407 | RASA2 641 | chr3: 141509698-141509725 |
| RASA2 96 | chr3: 141486402-141486429 | RASA2 642 | chr3: 141509699-141509726 |
| RASA2 97 | chr3: 141486403-141486430 | RASA2 643 | chr3: 141509700-141509727 |
| RASA2 98 | chr3: 141486410-141486437 | RASA2 644 | chr3: 141509703-141509730 |
| RASA2 99 | chr3: 141486427-141486454 | RASA2 645 | chr3: 141509715-141509742 |
| RASA2 100 | chr3: 141486433-141486460 | RASA2 646 | chr3: 141509722-141509749 |
| RASA2 101 | chr3: 141486434-141486461 | RASA2 647 | chr3: 141509733-141509760 |
| RASA2 102 | chr3: 141486435-141486462 | RASA2 648 | chr3: 141512136-141512163 |
| RASA2 103 | chr3: 141486436-141486463 | RASA2 649 | chr3: 141512156-141512183 |
| RASA2 104 | chr3: 141486439-141486466 | RASA2 650 | chr3: 141512166-141512193 |
| RASA2 105 | chr3: 141486443-141486470 | RASA2 651 | chr3: 141512183-141512210 |
| RASA2 106 | chr3: 141486461-141486488 | RASA2 652 | chr3: 141512195-141512222 |
| RASA2 107 | chr3: 141486472-141486499 | RASA2 653 | chr3: 141512196-141512223 |
| RASA2 108 | chr3: 141486477-141486504 | RASA2 654 | chr3: 141512197-141512224 |
| RASA2 109 | chr3: 141486484-141486511 | RASA2 655 | chr3: 141512207-141512234 |
| RASA2 110 | chr3: 141486501-141486528 | RASA2 656 | chr3: 141512213-141512240 |
| RASA2 111 | chr3: 141486502-141486529 | RASA2 657 | chr3: 141512223-141512250 |
| RASA2 112 | chr3: 141486505-141486532 | RASA2 658 | chr3: 141512236-141512263 |
| RASA2 113 | chr3: 141486506-141486533 | RASA2 659 | chr3: 141512240-141512267 |
| RASA2 114 | chr3: 141486512-141486539 | RASA2 660 | chr3: 141512254-141512281 |
| RASA2 115 | chr3: 141486520-141486547 | RASA2 661 | chr3: 141512256-141512283 |
| RASA2 116 | chr3: 141486523-141486550 | RASA2 662 | chr3: 141512257-141512284 |
| RASA2 117 | chr3: 141486524-141486551 | RASA2 663 | chr3: 141512258-141512285 |
| RASA2 118 | chr3: 141486525-141486552 | RASA2 664 | chr3: 141512271-141512298 |
| RASA2 119 | chr3: 141486534-141486561 | RASA2 665 | chr3: 141516314-141516341 |
| RASA2 120 | chr3: 141486544-141486571 | RASA2 666 | chr3: 141516328-141516355 |
| RASA2 121 | chr3: 141486577-141486604 | RASA2 667 | chr3: 141516329-141516356 |
| RASA2 122 | chr3: 141486595-141486622 | RASA2 668 | chr3: 141516362-141516389 |
| RASA2 123 | chr3: 141486598-141486625 | RASA2 669 | chr3: 141516373-141516400 |
| RASA2 124 | chr3: 141486624-141486651 | RASA2 670 | chr3: 141516405-141516432 |
| RASA2 125 | chr3: 141486627-141486654 | RASA2 671 | chr3: 141516424-141516451 |
| RASA2 126 | chr3: 141486628-141486655 | RASA2 672 | chr3: 141529681-141529708 |
| RASA2 127 | chr3: 141486631-141486658 | RASA2 673 | chr3: 141529717-141529744 |
| RASA2 128 | chr3: 141486635-141486662 | RASA2 674 | chr3: 141529723-141529750 |
| RASA2 129 | chr3: 141486637-141486664 | RASA2 675 | chr3: 141529736-141529763 |
| RASA2 130 | chr3: 141486638-141486665 | RASA2 676 | chr3: 141529770-141529797 |
| RASA2 131 | chr3: 141486642-141486669 | RASA2 677 | chr3: 141529776-141529803 |
| RASA2 132 | chr3: 141486653-141486680 | RASA2 678 | chr3: 141529786-141529813 |
| RASA2 133 | chr3: 141486654-141486681 | RASA2 679 | chr3: 141529788-141529815 |
| RASA2 134 | chr3: 141486657-141486684 | RASA2 680 | chr3: 141540506-141540533 |
| RASA2 135 | chr3: 141486658-141486685 | RASA2 681 | chr3: 141540507-141540534 |
| RASA2 136 | chr3: 141486659-141486686 | RASA2 682 | chr3: 141540544-141540571 |
| RASA2 137 | chr3: 141486671-141486698 | RASA2 683 | chr3: 141540548-141540575 |
| RASA2 138 | chr3: 141486672-141486699 | RASA2 684 | chr3: 141540555-141540582 |
| RASA2 139 | chr3: 141486683-141486710 | RASA2 685 | chr3: 141540592-141540619 |
| RASA2 140 | chr3: 141486687-141486714 | RASA2 686 | chr3: 141553837-141553864 |
| RASA2 141 | chr3: 141486694-141486721 | RASA2 687 | chr3: 141553847-141553874 |
| RASA2 142 | chr3: 141486696-141486723 | RASA2 688 | chr3: 141553848-141553875 |
| RASA2 143 | chr3: 141486697-141486724 | RASA2 689 | chr3: 141553851-141553878 |
| RASA2 144 | chr3: 141486706-141486733 | RASA2 690 | chr3: 141553865-141553892 |
| RASA2 145 | chr3: 141486707-141486734 | RASA2 691 | chr3: 141553869-141553896 |
| RASA2 146 | chr3: 141486708-141486735 | RASA2 692 | chr3: 141553879-141553906 |
| RASA2 147 | chr3: 141486709-141486736 | RASA2 693 | chr3: 141553893-141553920 |
| RASA2 148 | chr3: 141486723-141486750 | RASA2 694 | chr3: 141553903-141553930 |
| RASA2 149 | chr3: 141486724-141486751 | RASA2 695 | chr3: 141553904-141553931 |
| RASA2 150 | chr3: 141486727-141486754 | RASA2 696 | chr3: 141553905-141553932 |
| RASA2 151 | chr3: 141486734-141486761 | RASA2 697 | chr3: 141553906-141553933 |
| RASA2 152 | chr3: 141486735-141486762 | RASA2 698 | chr3: 141553914-141553941 |
| RASA2 153 | chr3: 141486739-141486766 | RASA2 699 | chr3: 141553932-141553959 |
| RASA2 154 | chr3: 141486740-141486767 | RASA2 700 | chr3: 141553933-141553960 |
| RASA2 155 | chr3: 141486742-141486769 | RASA2 701 | chr3: 141553939-141553966 |
| RASA2 156 | chr3: 141486743-141486770 | RASA2 702 | chr3: 141555813-141555840 |
| RASA2 157 | chr3: 141486746-141486773 | RASA2 703 | chr3: 141555826-141555853 |
| RASA2 158 | chr3: 141486747-141486774 | RASA2 704 | chr3: 141555846-141555873 |
| RASA2 159 | chr3: 141486751-141486778 | RASA2 705 | chr3: 141555883-141555910 |
| RASA2 160 | chr3: 141486752-141486779 | RASA2 706 | chr3: 141555886-141555913 |
| RASA2 161 | chr3: 141486753-141486780 | RASA2 707 | chr3: 141558859-141558886 |
| RASA2 162 | chr3: 141486764-141486791 | RASA2 708 | chr3: 141558890-141558917 |
| RASA2 163 | chr3: 141486767-141486794 | RASA2 709 | chr3: 141558896-141558923 |
| RASA2 164 | chr3: 141486768-141486795 | RASA2 710 | chr3: 141558901-141558928 |
| RASA2 165 | chr3: 141486769-141486796 | RASA2 711 | chr3: 141558908-141558935 |
| RASA2 166 | chr3: 141486775-141486802 | RASA2 712 | chr3: 141558910-141558937 |
| RASA2 167 | chr3: 141486778-141486805 | RASA2 713 | chr3: 141558913-141558940 |
| RASA2 168 | chr3: 141486787-141486814 | RASA2 714 | chr3: 141558917-141558944 |
| RASA2 169 | chr3: 141486798-141486825 | RASA2 715 | chr3: 141558918-141558945 |
| RASA2 170 | chr3: 141486799-141486826 | RASA2 716 | chr3: 141558924-141558951 |
| RASA2 171 | chr3: 141486806-141486833 | RASA2 717 | chr3: 141558936-141558963 |
| RASA2 172 | chr3: 141486816-141486843 | RASA2 718 | chr3: 141558948-141558975 |
| RASA2 173 | chr3: 141486828-141486855 | RASA2 719 | chr3: 141558949-141558976 |
| RASA2 174 | chr3: 141486829-141486856 | RASA2 720 | chr3: 141558950-141558977 |
| RASA2 175 | chr3: 141486832-141486859 | RASA2 721 | chr3: 141559867-141559894 |
| RASA2 176 | chr3: 141486833-141486860 | RASA2 722 | chr3: 141559876-141559903 |
| RASA2 177 | chr3: 141486836-141486863 | RASA2 723 | chr3: 141559879-141559906 |
| RASA2 178 | chr3: 141486839-141486866 | RASA2 724 | chr3: 141559887-141559914 |
| RASA2 179 | chr3: 141486845-141486872 | RASA2 725 | chr3: 141559914-141559941 |
| RASA2 180 | chr3: 141486846-141486873 | RASA2 726 | chr3: 141559918-141559945 |
| RASA2 181 | chr3: 141486847-141486874 | RASA2 727 | chr3: 141559924-141559951 |
| RASA2 182 | chr3: 141486848-141486875 | RASA2 728 | chr3: 141559925-141559952 |
| RASA2 183 | chr3: 141486852-141486879 | RASA2 729 | chr3: 141559936-141559963 |
| RASA2 184 | chr3: 141486856-141486883 | RASA2 730 | chr3: 141559955-141559982 |
| RASA2 185 | chr3: 141486861-141486888 | RASA2 731 | chr3: 141559969-141559996 |
| RASA2 186 | chr3: 141486862-141486889 | RASA2 732 | chr3: 141559974-141560001 |
| RASA2 187 | chr3: 141486863-141486890 | RASA2 733 | chr3: 141559975-141560002 |
| RASA2 188 | chr3: 141486864-141486891 | RASA2 734 | chr3: 141559980-141560007 |
| RASA2 189 | chr3: 141486867-141486894 | RASA2 735 | chr3: 141559992-141560019 |
| RASA2 190 | chr3: 141486873-141486900 | RASA2 736 | chr3: 141570885-141570912 |
| RASA2 191 | chr3: 141486876-141486903 | RASA2 737 | chr3: 141570911-141570938 |
| RASA2 192 | chr3: 141486879-141486906 | RASA2 738 | chr3: 141570925-141570952 |
| RASA2 193 | chr3: 141486880-141486907 | RASA2 739 | chr3: 141570940-141570967 |
| RASA2 194 | chr3: 141486883-141486910 | RASA2 740 | chr3: 141570941-141570968 |
| RASA2 195 | chr3: 141486891-141486918 | RASA2 741 | chr3: 141570942-141570969 |
| RASA2 196 | chr3: 141486892-141486919 | RASA2 742 | chr3: 141570950-141570977 |
| RASA2 197 | chr3: 141486893-141486920 | RASA2 743 | chr3: 141570963-141570990 |
| RASA2 198 | chr3: 141486894-141486921 | RASA2 744 | chr3: 141571001-141571028 |
| RASA2 199 | chr3: 141486895-141486922 | RASA2 745 | chr3: 141571012-141571039 |
| RASA2 200 | chr3: 141486896-141486923 | RASA2 746 | chr3: 141571030-141571057 |
| RASA2 201 | chr3: 141486897-141486924 | RASA2 747 | chr3: 141571057-141571084 |
| RASA2 202 | chr3: 141486900-141486927 | RASA2 748 | chr3: 141571061-141571088 |
| RASA2 203 | chr3: 141486901-141486928 | RASA2 749 | chr3: 141571391-141571418 |
| RASA2 204 | chr3: 141486902-141486929 | RASA2 750 | chr3: 141571406-141571433 |
| RASA2 205 | chr3: 141486903-141486930 | RASA2 751 | chr3: 141571416-141571443 |
| RASA2 206 | chr3: 141486906-141486933 | RASA2 752 | chr3: 141571472-141571499 |
| RASA2 207 | chr3: 141486907-141486934 | RASA2 753 | chr3: 141571473-141571500 |
| RASA2 208 | chr3: 141486908-141486935 | RASA2 754 | chr3: 141571474-141571501 |
| RASA2 209 | chr3: 141486914-141486941 | RASA2 755 | chr3: 141571495-141571522 |
| RASA2 210 | chr3: 141486915-141486942 | RASA2 756 | chr3: 141571502-141571529 |
| RASA2 211 | chr3: 141486917-141486944 | RASA2 757 | chr3: 141571511-141571538 |
| RASA2 212 | chr3: 141486918-141486945 | RASA2 758 | chr3: 141571518-141571545 |
| RASA2 213 | chr3: 141486921-141486948 | RASA2 759 | chr3: 141571520-141571547 |
| RASA2 214 | chr3: 141486922-141486949 | RASA2 760 | chr3: 141572605-141572632 |
| RASA2 215 | chr3: 141486923-141486950 | RASA2 761 | chr3: 141572616-141572643 |
| RASA2 216 | chr3: 141486926-141486953 | RASA2 762 | chr3: 141572631-141572658 |
| RASA2 217 | chr3: 141486927-141486954 | RASA2 763 | chr3: 141572638-141572665 |
| RASA2 218 | chr3: 141486928-141486955 | RASA2 764 | chr3: 141572639-141572666 |
| RASA2 219 | chr3: 141486931-141486958 | RASA2 765 | chr3: 141572647-141572674 |
| RASA2 220 | chr3: 141486932-141486959 | RASA2 766 | chr3: 141572648-141572675 |
| RASA2 221 | chr3: 141486933-141486960 | RASA2 767 | chr3: 141572652-141572679 |
| RASA2 222 | chr3: 141486936-141486963 | RASA2 768 | chr3: 141572653-141572680 |
| RASA2 223 | chr3: 141486941-141486968 | RASA2 769 | chr3: 141572656-141572683 |
| RASA2 224 | chr3: 141486942-141486969 | RASA2 770 | chr3: 141572657-141572684 |
| RASA2 225 | chr3: 141486948-141486975 | RASA2 771 | chr3: 141572690-141572717 |
| RASA2 226 | chr3: 141486953-141486980 | RASA2 772 | chr3: 141572697-141572724 |
| RASA2 227 | chr3: 141486954-141486981 | RASA2 773 | chr3: 141572709-141572736 |
| RASA2 228 | chr3: 141486955-141486982 | RASA2 774 | chr3: 141573157-141573184 |
| RASA2 229 | chr3: 141486956-141486983 | RASA2 775 | chr3: 141573166-141573193 |
| RASA2 230 | chr3: 141486959-141486986 | RASA2 776 | chr3: 141573171-141573198 |
| RASA2 231 | chr3: 141486960-141486987 | RASA2 777 | chr3: 141573172-141573199 |
| RASA2 232 | chr3: 141486961-141486988 | RASA2 778 | chr3: 141573180-141573207 |
| RASA2 233 | chr3: 141486966-141486993 | RASA2 779 | chr3: 141573195-141573222 |
| RASA2 234 | chr3: 141486970-141486997 | RASA2 780 | chr3: 141573917-141573944 |
| RASA2 235 | chr3: 141486973-141487000 | RASA2 781 | chr3: 141573926-141573953 |
| RASA2 236 | chr3: 141486976-141487003 | RASA2 782 | chr3: 141573937-141573964 |
| RASA2 237 | chr3: 141486977-141487004 | RASA2 783 | chr3: 141574006-141574033 |
| RASA2 238 | chr3: 141486982-141487009 | RASA2 784 | chr3: 141574007-141574034 |
| RASA2 239 | chr3: 141486983-141487010 | RASA2 785 | chr3: 141574008-141574035 |
| RASA2 240 | chr3: 141486989-141487016 | RASA2 786 | chr3: 141574015-141574042 |
| RASA2 241 | chr3: 141486999-141487026 | RASA2 787 | chr3: 141574022-141574049 |
| RASA2 242 | chr3: 141487000-141487027 | RASA2 788 | chr3: 141574064-141574091 |
| RASA2 243 | chr3: 141487005-141487032 | RASA2 789 | chr3: 141576983-141577010 |
| RASA2 244 | chr3: 141487011-141487038 | RASA2 790 | chr3: 141576993-141577020 |
| RASA2 245 | chr3: 141487015-141487042 | RASA2 791 | chr3: 141577004-141577031 |
| RASA2 246 | chr3: 141487017-141487044 | RASA2 792 | chr3: 141577005-141577032 |
| RASA2 247 | chr3: 141487024-141487051 | RASA2 793 | chr3: 141577063-141577090 |
| RASA2 248 | chr3: 141487027-141487054 | RASA2 794 | chr3: 141577074-141577101 |
| RASA2 249 | chr3: 141487030-141487057 | RASA2 795 | chr3: 141577095-141577122 |
| RASA2 250 | chr3: 141487031-141487058 | RASA2 796 | chr3: 141577104-141577131 |
| RASA2 251 | chr3: 141487032-141487059 | RASA2 797 | chr3: 141578622-141578649 |
| RASA2 252 | chr3: 141487033-141487060 | RASA2 798 | chr3: 141578623-141578650 |
| RASA2 253 | chr3: 141487038-141487065 | RASA2 799 | chr3: 141578628-141578655 |
| RASA2 254 | chr3: 141487039-141487066 | RASA2 800 | chr3: 141578635-141578662 |
| RASA2 255 | chr3: 141487043-141487070 | RASA2 801 | chr3: 141578638-141578665 |
| RASA2 256 | chr3: 141487044-141487071 | RASA2 802 | chr3: 141578655-141578682 |
| RASA2 257 | chr3: 141487048-141487075 | RASA2 803 | chr3: 141578670-141578697 |
| RASA2 258 | chr3: 141487049-141487076 | RASA2 804 | chr3: 141578684-141578711 |
| RASA2 259 | chr3: 141487052-141487079 | RASA2 805 | chr3: 141578698-141578725 |
| RASA2 260 | chr3: 141487060-141487087 | RASA2 806 | chr3: 141578740-141578767 |
| RASA2 261 | chr3: 141487063-141487090 | RASA2 807 | chr3: 141580341-141580368 |
| RASA2 262 | chr3: 141487066-141487093 | RASA2 808 | chr3: 141580395-141580422 |
| RASA2 263 | chr3: 141487069-141487096 | RASA2 809 | chr3: 141580396-141580423 |
| RASA2 264 | chr3: 141487072-141487099 | RASA2 810 | chr3: 141580403-141580430 |
| RASA2 265 | chr3: 141487082-141487109 | RASA2 811 | chr3: 141580404-141580431 |
| RASA2 266 | chr3: 141487084-141487111 | RASA2 812 | chr3: 141580405-141580432 |
| RASA2 267 | chr3: 141487087-141487114 | RASA2 813 | chr3: 141580406-141580433 |
| RASA2 268 | chr3: 141487099-141487126 | RASA2 814 | chr3: 141580425-141580452 |
| RASA2 269 | chr3: 141487108-141487135 | RASA2 815 | chr3: 141580441-141580468 |
| RASA2 270 | chr3: 141487121-141487148 | RASA2 816 | chr3: 141581078-141581105 |
| RASA2 271 | chr3: 141487129-141487156 | RASA2 817 | chr3: 141581079-141581106 |
| RASA2 272 | chr3: 141487132-141487159 | RASA2 818 | chr3: 141581128-141581155 |
| RASA2 273 | chr3: 141487136-141487163 | RASA2 819 | chr3: 141581151-141581178 |
| RASA2 274 | chr3: 141487137-141487164 | RASA2 820 | chr3: 141581159-141581186 |
| RASA2 275 | chr3: 141487138-141487165 | RASA2 821 | chr3: 141581170-141581197 |
| RASA2 276 | chr3: 141487144-141487171 | RASA2 822 | chr3: 141586004-141586031 |
| RASA2 277 | chr3: 141487153-141487180 | RASA2 823 | chr3: 141586008-141586035 |
| RASA2 278 | chr3: 141487154-141487181 | RASA2 824 | chr3: 141586009-141586036 |
| RASA2 279 | chr3: 141487156-141487183 | RASA2 825 | chr3: 141586015-141586042 |
| RASA2 280 | chr3: 141487160-141487187 | RASA2 826 | chr3: 141586016-141586043 |
| RASA2 281 | chr3: 141487167-141487194 | RASA2 827 | chr3: 141586017-141586044 |
| RASA2 282 | chr3: 141487179-141487206 | RASA2 828 | chr3: 141586041-141586068 |
| RASA2 283 | chr3: 141487180-141487207 | RASA2 829 | chr3: 141586062-141586089 |
| RASA2 284 | chr3: 141487181-141487208 | RASA2 830 | chr3: 141586071-141586098 |
| RASA2 285 | chr3: 141487197-141487224 | RASA2 831 | chr3: 141586072-141586099 |
| RASA2 286 | chr3: 141487198-141487225 | RASA2 832 | chr3: 141586079-141586106 |
| RASA2 287 | chr3: 141487199-141487226 | RASA2 833 | chr3: 141586087-141586114 |
| **RASA2** 288 | chr3: 141487200-141487227 | RASA2 834 | chr3: 141586642-141586669 |
| RASA2 289 | chr3: 141487204-141487231 | RASA2 835 | chr3: 141586652-141586679 |
| RASA2 290 | chr3: 141487205-141487232 | RASA2 836 | chr3: 141586657-141586684 |
| RASA2 291 | chr3: 141487211-141487238 | RASA2 837 | chr3: 141586682-141586709 |
| RASA2 292 | chr3: 141487212-141487239 | RASA2 838 | chr3: 141586726-141586753 |
| RASA2 293 | chr3: 141487213-141487240 | RASA2 839 | chr3: 141586735-141586762 |
| RASA2 294 | chr3: 141502131-141502158 | RASA2 840 | chr3: 141586739-141586766 |
| RASA2 295 | chr3: 141502152-141502179 | RASA2 841 | chr3: 141586749-141586776 |
| RASA2 296 | chr3: 141502153-141502180 | RASA2 842 | chr3: 141586751-141586778 |
| RASA2 297 | chr3: 141502160-141502187 | RASA2 843 | chr3: 141586752-141586779 |
| RASA2 298 | chr3: 141502209-141502236 | RASA2 844 | chr3: 141586753-141586780 |
| RASA2 299 | chr3: 141502219-141502246 | RASA2 845 | chr3: 141590112-141590139 |
| RASA2 300 | chr3: 141502224-141502251 | RASA2 846 | chr3: 141590113-141590140 |
| RASA2 301 | chr3: 141502234-141502261 | RASA2 847 | chr3: 141607651-141607678 |
| RASA2 302 | chr3: 141502256-141502283 | RASA2 848 | chr3: 141607698-141607725 |
| RASA2 303 | chr3: 141502283-141502310 | RASA2 849 | chr3: 141607700-141607727 |
| RASA2 304 | chr3: 141502286-141502313 | RASA2 850 | chr3: 141607701-141607728 |
| RASA2 305 | chr3: 141502287-141502314 | RASA2 851 | chr3: 141607707-141607734 |
| RASA2 306 | chr3: 141502293-141502320 | RASA2 852 | chr3: 141608480-141608507 |
| RASA2 307 | chr3: 141502294-141502321 | RASA2 853 | chr3: 141608483-141608510 |
| RASA2 308 | chr3: 141502303-141502330 | RASA2 854 | chr3: 141608494-141608521 |
| RASA2 309 | chr3: 141502340-141502367 | RASA2 855 | chr3: 141608504-141608531 |
| RASA2 310 | chr3: 141502353-141502380 | RASA2 856 | chr3: 141608516-141608543 |
| RASA2 311 | chr3: 141502374-141502401 | RASA2 857 | chr3: 141608527-141608554 |
| RASA2 312 | chr3: 141502375-141502402 | RASA2 858 | chr3: 141608533-141608560 |
| RASA2 313 | chr3: 141502383-141502410 | RASA2 859 | chr3: 141608551-141608578 |
| RASA2 314 | chr3: 141502385-141502412 | RASA2 860 | chr3: 141608552-141608579 |
| RASA2 315 | chr3: 141502394-141502421 | RASA2 861 | chr3: 141608575-141608602 |
| RASA2 316 | chr3: 141502402-141502429 | RASA2 862 | chr3: 141608584-141608611 |
| RASA2 317 | chr3: 141502444-141502471 | RASA2 863 | chr3: 141608610-141608637 |
| RASA2 318 | chr3: 141502459-141502486 | RASA2 864 | chr3: 141608617-141608644 |
| RASA2 319 | chr3: 141502460-141502487 | RASA2 865 | chr3: 141608618-141608645 |
| RASA2 320 | chr3: 141502469-141502496 | RASA2 866 | chr3: 141608619-141608646 |
| RASA2 321 | chr3: 141502470-141502497 | RASA2 867 | chr3: 141608630-141608657 |
| RASA2 322 | chr3: 141502471-141502498 | RASA2 868 | chr3: 141608637-141608664 |
| RASA2 323 | chr3: 141502472-141502499 | RASA2 869 | chr3: 141608652-141608679 |
| RASA2 324 | chr3: 141502500-141502527 | RASA2 870 | chr3: 141608687-141608714 |
| RASA2 325 | chr3: 141502516-141502543 | RASA2 871 | chr3: 141609395-141609422 |
| RASA2 326 | chr3: 141502519-141502546 | RASA2 872 | chr3: 141609414-141609441 |
| RASA2 327 | chr3: 141502542-141502569 | RASA2 873 | chr3: 141609426-141609453 |
| RASA2 328 | chr3: 141502545-141502572 | RASA2 874 | chr3: 141609427-141609454 |
| RASA2 329 | chr3: 141502546-141502573 | RASA2 875 | chr3: 141609438-141609465 |
| RASA2 330 | chr3: 141502549-141502576 | RASA2 876 | chr3: 141609482-141609509 |
| RASA2 331 | chr3: 141502554-141502581 | RASA2 877 | chr3: 141609483-141609510 |
| RASA2 332 | chr3: 141502570-141502597 | RASA2 878 | chr3: 141609491-141609518 |
| RASA2 333 | chr3: 141502574-141502601 | RASA2 879 | chr3: 141609492-141609519 |
| RASA2 334 | chr3: 141502577-141502604 | RASA2 880 | chr3: 141609493-141609520 |
| RASA2 335 | chr3: 141502590-141502617 | RASA2 881 | chr3: 141609497-141609524 |
| RASA2 336 | chr3: 141502593-141502620 | RASA2 882 | chr3: 141609852-141609879 |
| RASA2 337 | chr3: 141502598-141502625 | RASA2 883 | chr3: 141609859-141609886 |
| RASA2 338 | chr3: 141502599-141502626 | RASA2 884 | chr3: 141609863-141609890 |
| RASA2 339 | chr3: 141502600-141502627 | RASA2 885 | chr3: 141609880-141609907 |
| RASA2 340 | chr3: 141502696-141502723 | RASA2 886 | chr3: 141609909-141609936 |
| RASA2 341 | chr3: 141502716-141502743 | RASA2 887 | chr3: 141609921-141609948 |
| RASA2 342 | chr3: 141502749-141502776 | RASA2 888 | chr3: 141609924-141609951 |
| RASA2 343 | chr3: 141502754-141502781 | RASA2 889 | chr3: 141609964-141609991 |
| RASA2 344 | chr3: 141502755-141502782 | RASA2 890 | chr3: 141609981-141610008 |
| RASA2 345 | chr3: 141502756-141502783 | RASA2 891 | chr3: 141610004-141610031 |
| RASA2 346 | chr3: 141502764-141502791 | RASA2 892 | chr3: 141610014-141610041 |
| RASA2 347 | chr3: 141502765-141502792 | RASA2 893 | chr3: 141610033-141610060 |
| RASA2 348 | chr3: 141502771-141502798 | RASA2 894 | chr3: 141610034-141610061 |
| RASA2 349 | chr3: 141502787-141502814 | RASA2 895 | chr3: 141610042-141610069 |
| RASA2 350 | chr3: 141502791-141502818 | RASA2 896 | chr3: 141612256-141612283 |
| RASA2 351 | chr3: 141502792-141502819 | RASA2 897 | chr3: 141612257-141612284 |
| RASA2 352 | chr3: 141502793-141502820 | RASA2 898 | chr3: 141612273-141612300 |
| RASA2 353 | chr3: 141502794-141502821 | RASA2 899 | chr3: 141612274-141612301 |
| RASA2 354 | chr3: 141502807-141502834 | RASA2 900 | chr3: 141612290-141612317 |
| RASA2 355 | chr3: 141502808-141502835 | RASA2 901 | chr3: 141612301-141612328 |
| RASA2 356 | chr3: 141502819-141502846 | RASA2 902 | chr3: 141612315-141612342 |
| RASA2 357 | chr3: 141502827-141502854 | RASA2 903 | chr3: 141612334-141612361 |
| RASA2 358 | chr3: 141502828-141502855 | RASA2 904 | chr3: 141612367-141612394 |
| RASA2 359 | chr3: 141502837-141502864 | RASA2 905 | chr3: 141612412-141612439 |
| RASA2 360 | chr3: 141502838-141502865 | RASA2 906 | chr3: 141612430-141612457 |
| RASA2 361 | chr3: 141502840-141502867 | RASA2 907 | chr3: 141612433-141612460 |
| RASA2 362 | chr3: 141502849-141502876 | RASA2 908 | chr3: 141612448-141612475 |
| RASA2 363 | chr3: 141502853-141502880 | RASA2 909 | chr3: 141612449-141612476 |
| RASA2 364 | chr3: 141502854-141502881 | RASA2 910 | chr3: 141612457-141612484 |
| RASA2 365 | chr3: 141502861-141502888 | RASA2 911 | chr3: 141612480-141612507 |
| RASA2 366 | chr3: 141502871-141502898 | RASA2 912 | chr3: 141612513-141612540 |
| RASA2 367 | chr3: 141502884-141502911 | RASA2 913 | chr3: 141612514-141612541 |
| RASA2 368 | chr3: 141502885-141502912 | RASA2 914 | chr3: 141612522-141612549 |
| RASA2 369 | chr3: 141502886-141502913 | RASA2 915 | chr3: 141612523-141612550 |
| RASA2 370 | chr3: 141502890-141502917 | RASA2 916 | chr3: 141612531-141612558 |
| RASA2 371 | chr3: 141502947-141502974 | RASA2 917 | chr3: 141612540-141612567 |
| RASA2 372 | chr3: 141502948-141502975 | RASA2 918 | chr3: 141612546-141612573 |
| RASA2 373 | chr3: 141502954-141502981 | RASA2 919 | chr3: 141612580-141612607 |
| RASA2 374 | chr3: 141502995-141503022 | RASA2 920 | chr3: 141612581-141612608 |
| RASA2 375 | chr3: 141503003-141503030 | RASA2 921 | chr3: 141612584-141612611 |
| RASA2 376 | chr3: 141503008-141503035 | RASA2 922 | chr3: 141612615-141612642 |
| RASA2 377 | chr3: 141503022-141503049 | RASA2 923 | chr3: 141612618-141612645 |
| RASA2 378 | chr3: 141503023-141503050 | RASA2 924 | chr3: 141612631-141612658 |
| RASA2 379 | chr3: 141503046-141503073 | RASA2 925 | chr3: 141612634-141612661 |
| RASA2 380 | chr3: 141503047-141503074 | RASA2 926 | chr3: 141612676-141612703 |
| RASA2 381 | chr3: 141503083-141503110 | RASA2 927 | chr3: 141612685-141612712 |
| RASA2 382 | chr3: 141503099-141503126 | RASA2 928 | chr3: 141612699-141612726 |
| RASA2 383 | chr3: 141503166-141503193 | RASA2 929 | chr3: 141612725-141612752 |
| RASA2 384 | chr3: 141503172-141503199 | RASA2 930 | chr3: 141612768-141612795 |
| RASA2 385 | chr3: 141503173-141503200 | RASA2 931 | chr3: 141612769-141612796 |
| RASA2 386 | chr3: 141503183-141503210 | RASA2 932 | chr3: 141612772-141612799 |
| RASA2 387 | chr3: 141503186-141503213 | RASA2 933 | chr3: 141612773-141612800 |
| RASA2 388 | chr3: 141503187-141503214 | RASA2 934 | chr3: 141612788-141612815 |
| RASA2 389 | chr3: 141503188-141503215 | RASA2 935 | chr3: 141612806-141612833 |
| RASA2 390 | chr3: 141503197-141503224 | RASA2 936 | chr3: 141612809-141612836 |
| RASA2 391 | chr3: 141503217-141503244 | RASA2 937 | chr3: 141612817-141612844 |
| RASA2 392 | chr3: 141503236-141503263 | RASA2 938 | chr3: 141612836-141612863 |
| RASA2 393 | chr3: 141503244-141503271 | RASA2 939 | chr3: 141612865-141612892 |
| RASA2 394 | chr3: 141503245-141503272 | RASA2 940 | chr3: 141612871-141612898 |
| RASA2 395 | chr3: 141503255-141503282 | RASA2 941 | chr3: 141612898-141612925 |
| RASA2 396 | chr3: 141503330-141503357 | RASA2 942 | chr3: 141612905-141612932 |
| RASA2 397 | chr3: 141503334-141503361 | RASA2 943 | chr3: 141612910-141612937 |
| RASA2 398 | chr3: 141503347-141503374 | RASA2 944 | chr3: 141612917-141612944 |
| RASA2 399 | chr3: 141503359-141503386 | RASA2 945 | chr3: 141612918-141612945 |
| RASA2 400 | chr3: 141503363-141503390 | RASA2 946 | chr3: 141612954-141612981 |
| RASA2 401 | chr3: 141503367-141503394 | RASA2 947 | chr3: 141612955-141612982 |
| RASA2 402 | chr3: 141503396-141503423 | RASA2 948 | chr3: 141612966-141612993 |
| RASA2 403 | chr3: 141503425-141503452 | RASA2 949 | chr3: 141613011-141613038 |
| RASA2 404 | chr3: 141503461-141503488 | RASA2 950 | chr3: 141613018-141613045 |
| RASA2 405 | chr3: 141503469-141503496 | RASA2 951 | chr3: 141613041-141613068 |
| RASA2 406 | chr3: 141503561-141503588 | RASA2 952 | chr3: 141613060-141613087 |
| RASA2 407 | chr3: 141503575-141503602 | RASA2 953 | chr3: 141613088-141613115 |
| RASA2 408 | chr3: 141503620-141503647 | RASA2 954 | chr3: 141613089-141613116 |
| RASA2 409 | chr3: 141503621-141503648 | RASA2 955 | chr3: 141613097-141613124 |
| RASA2 410 | chr3: 141503624-141503651 | RASA2 956 | chr3: 141613100-141613127 |
| RASA2 411 | chr3: 141503634-141503661 | RASA2 957 | chr3: 141613103-141613130 |
| RASA2 412 | chr3: 141503644-141503671 | RASA2 958 | chr3: 141613104-141613131 |
| RASA2 413 | chr3: 141503651-141503678 | RASA2 959 | chr3: 141613146-141613173 |
| RASA2 414 | chr3: 141503652-141503679 | RASA2 960 | chr3: 141613147-141613174 |
| RASA2 415 | chr3: 141503672-141503699 | RASA2 961 | chr3: 141613149-141613176 |
| RASA2 416 | chr3: 141503709-141503736 | RASA2 962 | chr3: 141613150-141613177 |
| RASA2 417 | chr3: 141503714-141503741 | RASA2 963 | chr3: 141613158-141613185 |
| RASA2 418 | chr3: 141503727-141503754 | RASA2 964 | chr3: 141613175-141613202 |
| RASA2 419 | chr3: 141503762-141503789 | RASA2 965 | chr3: 141613178-141613205 |
| RASA2 420 | chr3: 141503773-141503800 | RASA2 966 | chr3: 141613180-141613207 |
| RASA2 421 | chr3: 141503801-141503828 | RASA2 967 | chr3: 141613189-141613216 |
| RASA2 422 | chr3: 141503857-141503884 | RASA2 968 | chr3: 141613190-141613217 |
| RASA2 423 | chr3: 141503871-141503898 | RASA2 969 | chr3: 141613193-141613220 |
| RASA2 424 | chr3: 141503893-141503920 | RASA2 970 | chr3: 141613210-141613237 |
| RASA2 425 | chr3: 141503926-141503953 | RASA2 971 | chr3: 141613218-141613245 |
| RASA2 426 | chr3: 141503955-141503982 | RASA2 972 | chr3: 141613228-141613255 |
| RASA2 427 | chr3: 141503967-141503994 | RASA2 973 | chr3: 141613261-141613288 |
| RASA2 428 | chr3: 141503973-141504000 | RASA2 974 | chr3: 141613262-141613289 |
| RASA2 429 | chr3: 141504003-141504030 | RASA2 975 | chr3: 141613283-141613310 |
| RASA2 430 | chr3: 141504014-141504041 | RASA2 976 | chr3: 141613346-141613373 |
| RASA2 431 | chr3: 141504015-141504042 | RASA2 977 | chr3: 141613372-141613399 |
| RASA2 432 | chr3: 141504020-141504047 | RASA2 978 | chr3: 141613376-141613403 |
| RASA2 433 | chr3: 141504059-141504086 | RASA2 979 | chr3: 141613379-141613406 |
| RASA2 434 | chr3: 141504063-141504090 | RASA2 980 | chr3: 141613380-141613407 |
| RASA2 435 | chr3: 141504076-141504103 | RASA2 981 | chr3: 141613384-141613411 |
| RASA2 436 | chr3: 141504105-141504132 | RASA2 982 | chr3: 141613392-141613419 |
| RASA2 437 | chr3: 141504106-141504133 | RASA2 983 | chr3: 141613434-141613461 |
| RASA2 438 | chr3: 141504107-141504134 | RASA2 984 | chr3: 141613435-141613462 |
| RASA2 439 | chr3: 141504111-141504138 | RASA2 985 | chr3: 141613454-141613481 |
| RASA2 440 | chr3: 141504135-141504162 | RASA2 986 | chr3: 141613462-141613489 |
| RASA2 441 | chr3: 141504144-141504171 | RASA2 987 | chr3: 141613463-141613490 |
| RASA2 442 | chr3: 141504179-141504206 | RASA2 988 | chr3: 141613484-141613511 |
| RASA2 443 | chr3: 141504180-141504207 | RASA2 989 | chr3: 141613485-141613512 |
| RASA2 444 | chr3: 141504182-141504209 | RASA2 990 | chr3: 141613495-141613522 |
| RASA2 445 | chr3: 141504200-141504227 | RASA2 991 | chr3: 141613498-141613525 |
| RASA2 446 | chr3: 141504201-141504228 | RASA2 992 | chr3: 141613519-141613546 |
| RASA2 447 | chr3: 141504240-141504267 | RASA2 993 | chr3: 141613541-141613568 |
| RASA2 448 | chr3: 141504241-141504268 | RASA2 994 | chr3: 141613565-141613592 |
| RASA2 449 | chr3: 141504294-141504321 | RASA2 995 | chr3: 141613580-141613607 |
| RASA2 450 | chr3: 141504309-141504336 | RASA2 996 | chr3: 141613602-141613629 |
| RASA2 451 | chr3: 141504323-141504350 | RASA2 997 | chr3: 141613697-141613724 |
| RASA2 452 | chr3: 141504324-141504351 | RASA2 998 | chr3: 141613700-141613727 |
| RASA2 453 | chr3: 141504328-141504355 | RASA2 999 | chr3: 141613701-141613728 |
| RASA2 454 | chr3: 141504331-141504358 | RASA2 1000 | chr3: 141613709-141613736 |
| RASA2 455 | chr3: 141504338-141504365 | RASA2 1001 | chr3: 141613731-141613758 |
| RASA2 456 | chr3: 141504378-141504405 | RASA2 1002 | chr3: 141613754-141613781 |
| RASA2 457 | chr3: 141504392-141504419 | RASA2 1003 | chr3: 141613800-141613827 |
| RASA2 458 | chr3: 141504407-141504434 | RASA2 1004 | chr3: 141613854-141613881 |
| RASA2 459 | chr3: 141504408-141504435 | RASA2 1005 | chr3: 141613858-141613885 |
| RASA2 460 | chr3: 141504409-141504436 | RASA2 1006 | chr3: 141613894-141613921 |
| RASA2 461 | chr3: 141504451-141504478 | RASA2 1007 | chr3: 141613895-141613922 |
| RASA2 462 | chr3: 141504500-141504527 | RASA2 1008 | chr3: 141613927-141613954 |
| RASA2 463 | chr3: 141504507-141504534 | RASA2 1009 | chr3: 141613954-141613981 |
| RASA2 464 | chr3: 141504508-141504535 | RASA2 1010 | chr3: 141613993-141614020 |
| RASA2 465 | chr3: 141504520-141504547 | RASA2 1011 | chr3: 141614039-141614066 |
| RASA2 466 | chr3: 141504530-141504557 | RASA2 1012 | chr3: 141614065-141614092 |
| RASA2 467 | chr3: 141504548-141504575 | RASA2 1013 | chr3: 141614089-141614116 |
| RASA2 468 | chr3: 141504549-141504576 | RASA2 1014 | chr3: 141614096-141614123 |
| RASA2 469 | chr3: 141504557-141504584 | RASA2 1015 | chr3: 141614119-141614146 |
| RASA2 470 | chr3: 141504558-141504585 | RASA2 1016 | chr3: 141614126-141614153 |
| RASA2 471 | chr3: 141504567-141504594 | RASA2 1017 | chr3: 141614178-141614205 |
| RASA2 472 | chr3: 141504572-141504599 | RASA2 1018 | chr3: 141614184-141614211 |
| RASA2 473 | chr3: 141504573-141504600 | RASA2 1019 | chr3: 141614185-141614212 |
| RASA2 474 | chr3: 141504587-141504614 | RASA2 1020 | chr3: 141614186-141614213 |
| RASA2 475 | chr3: 141504588-141504615 | RASA2 1021 | chr3: 141614198-141614225 |
| RASA2 476 | chr3: 141504589-141504616 | RASA2 1022 | chr3: 141614199-141614226 |
| RASA2 477 | chr3: 141504592-141504619 | RASA2 1023 | chr3: 141614206-141614233 |
| RASA2 478 | chr3: 141504593-141504620 | RASA2 1024 | chr3: 141614228-141614255 |
| RASA2 479 | chr3: 141504594-141504621 | RASA2 1025 | chr3: 141614238-141614265 |
| RASA2 480 | chr3: 141504599-141504626 | RASA2 1026 | chr3: 141614241-141614268 |
| RASA2 481 | chr3: 141504635-141504662 | RASA2 1027 | chr3: 141614278-141614305 |
| RASA2 482 | chr3: 141504638-141504665 | RASA2 1028 | chr3: 141614301-141614328 |
| RASA2 483 | chr3: 141504650-141504677 | RASA2 1029 | chr3: 141614316-141614343 |
| RASA2 484 | chr3: 141504660-141504687 | RASA2 1030 | chr3: 141614332-141614359 |
| RASA2 485 | chr3: 141504665-141504692 | RASA2 1031 | chr3: 141614335-141614362 |
| RASA2 486 | chr3: 141504666-141504693 | RASA2 1032 | chr3: 141614349-141614376 |
| RASA2 487 | chr3: 141504667-141504694 | RASA2 1033 | chr3: 141614396-141614423 |
| RASA2 488 | chr3: 141504699-141504726 | RASA2 1034 | chr3: 141614411-141614438 |
| RASA2 489 | chr3: 141504718-141504745 | RASA2 1035 | chr3: 141614414-141614441 |
| RASA2 490 | chr3: 141504727-141504754 | RASA2 1036 | chr3: 141614425-141614452 |
| RASA2 491 | chr3: 141504732-141504759 | RASA2 1037 | chr3: 141614426-141614453 |
| RASA2 492 | chr3: 141504753-141504780 | RASA2 1038 | chr3: 141614428-141614455 |
| RASA2 493 | chr3: 141504764-141504791 | RASA2 1039 | chr3: 141614429-141614456 |
| RASA2 494 | chr3: 141504765-141504792 | RASA2 1040 | chr3: 141614436-141614463 |
| RASA2 495 | chr3: 141504770-141504797 | RASA2 1041 | chr3: 141614451-141614478 |
| RASA2 496 | chr3: 141504779-141504806 | RASA2 1042 | chr3: 141614457-141614484 |
| RASA2 497 | chr3: 141504789-141504816 | RASA2 1043 | chr3: 141614458-141614485 |
| RASA2 498 | chr3: 141504796-141504823 | RASA2 1044 | chr3: 141614465-141614492 |
| RASA2 499 | chr3: 141504799-141504826 | RASA2 1045 | chr3: 141614502-141614529 |
| RASA2 500 | chr3: 141504800-141504827 | RASA2 1046 | chr3: 141614520-141614547 |
| RASA2 501 | chr3: 141504805-141504832 | RASA2 1047 | chr3: 141614526-141614553 |
| RASA2 502 | chr3: 141504806-141504833 | RASA2 1048 | chr3: 141614556-141614583 |
| RASA2 503 | chr3: 141504813-141504840 | RASA2 1049 | chr3: 141614560-141614587 |
| RASA2 504 | chr3: 141504829-141504856 | RASA2 1050 | chr3: 141614561-141614588 |
| RASA2 505 | chr3: 141504832-141504859 | RASA2 1051 | chr3: 141614569-141614596 |
| RASA2 506 | chr3: 141504833-141504860 | RASA2 1052 | chr3: 141614570-141614597 |
| RASA2 507 | chr3: 141504838-141504865 | RASA2 1053 | chr3: 141614579-141614606 |
| RASA2 508 | chr3: 141504839-141504866 | RASA2 1054 | chr3: 141614580-141614607 |
| RASA2 509 | chr3: 141504840-141504867 | RASA2 1055 | chr3: 141614581-141614608 |
| RASA2 510 | chr3: 141504863-141504890 | RASA2 1056 | chr3: 141614601-141614628 |
| RASA2 511 | chr3: 141504871-141504898 | RASA2 1057 | chr3: 141614607-141614634 |
| RASA2 512 | chr3: 141504872-141504899 | RASA2 1058 | chr3: 141614616-141614643 |
| RASA2 513 | chr3: 141504875-141504902 | RASA2 1059 | chr3: 141614625-141614652 |
| RASA2 514 | chr3: 141504880-141504907 | RASA2 1060 | chr3: 141614644-141614671 |
| RASA2 515 | chr3: 141504885-141504912 | RASA2 1061 | chr3: 141614647-141614674 |
| RASA2 516 | chr3: 141504888-141504915 | RASA2 1062 | chr3: 141614653-141614680 |
| RASA2 517 | chr3: 141504889-141504916 | RASA2 1063 | chr3: 141614722-141614749 |
| RASA2 518 | chr3: 141504896-141504923 | RASA2 1064 | chr3: 141614723-141614750 |
| RASA2 519 | chr3: 141504905-141504932 | RASA2 1065 | chr3: 141614749-141614776 |
| RASA2 520 | chr3: 141504917-141504944 | RASA2 1066 | chr3: 141614771-141614798 |
| RASA2 521 | chr3: 141504941-141504968 | RASA2 1067 | chr3: 141614772-141614799 |
| RASA2 522 | chr3: 141504946-141504973 | RASA2 1068 | chr3: 141614810-141614837 |
| RASA2 523 | chr3: 141504952-141504979 | RASA2 1069 | chr3: 141614811-141614838 |
| RASA2 524 | chr3: 141504956-141504983 | RASA2 1070 | chr3: 141614817-141614844 |
| RASA2 525 | chr3: 141504957-141504984 | RASA2 1071 | chr3: 141614818-141614845 |
| RASA2 526 | chr3: 141504958-141504985 | RASA2 1072 | chr3: 141614835-141614862 |
| RASA2 527 | chr3: 141504973-141505000 | RASA2 1073 | chr3: 141614860-141614887 |
| RASA2 528 | chr3: 141504980-141505007 | RASA2 1074 | chr3: 141614875-141614902 |
| RASA2 529 | chr3: 141504981-141505008 | RASA2 1075 | chr3: 141614917-141614944 |
| RASA2 530 | chr3: 141504993-141505020 | RASA2 1076 | chr3: 141614924-141614951 |
| RASA2 531 | chr3: 141505001-141505028 | RASA2 1077 | chr3: 141614940-141614967 |
| RASA2 532 | chr3: 141505029-141505056 | RASA2 1078 | chr3: 141614972-141614999 |
| RASA2 533 | chr3: 141505033-141505060 | RASA2 1079 | chr3: 141614980-141615007 |
| RASA2 534 | chr3: 141505034-141505061 | RASA2 1080 | chr3: 141615005-141615032 |
| RASA2 535 | chr3: 141505041-141505068 | RASA2 1081 | chr3: 141615011-141615038 |
| RASA2 536 | chr3: 141505042-141505069 | RASA2 1082 | chr3: 141615030-141615057 |
| RASA2 537 | chr3: 141505059-141505086 | RASA2 1083 | chr3: 141615070-141615097 |
| RASA2 538 | chr3: 141505060-141505087 | RASA2 1084 | chr3: 141615088-141615115 |
| RASA2 539 | chr3: 141505062-141505089 | RASA2 1085 | chr3: 141615104-141615131 |
| RASA2 540 | chr3: 141505065-141505092 | RASA2 1086 | chr3: 141615114-141615141 |
| RASA2 541 | chr3: 141505068-141505095 | RASA2 1087 | chr3: 141615132-141615159 |
| RASA2 542 | chr3: 141505069-141505096 | RASA2 1088 | chr3: 141615171-141615198 |
| RASA2 543 | chr3: 141505079-141505106 | RASA2 1089 | chr3: 141615172-141615199 |
| RASA2 544 | chr3: 141505080-141505107 | RASA2 1090 | chr3: 141615222-141615249 |
| RASA2 545 | chr3: 141505088-141505115 | RASA2 1091 | chr3: 141615285-141615312 |
| RASA2 546 | chr3: 141505092-141505119 | RASA2 1092 | chr3: 141615339-141615366 |

**Tabel 2B. Genomic Coordinates of Human FIBP in the Current Invention**

| Index | Chromosomal coordinate range | Index | Chromosomal coordinate range |
|---|---|---|---|
| FIBP 1 | chr11: 65883736-65883763 | FIBP 275 | chr11: 65888424-65888451 |
| FIBP 2 | chr11: 65883739-65883766 | FIBP 276 | chr11: 65888426-65888453 |
| FIBP 3 | chr11: 65883743-65883770 | FIBP 277 | chr11: 65888427-65888454 |
| FIBP 4 | chr11: 65883744-65883771 | FIBP 278 | chr11: 65888433-65888460 |
| FIBP 5 | chr11: 65883785-65883812 | FIBP 279 | chr11: 65888440-65888467 |
| FIBP 6 | chr11: 65883789-65883816 | FIBP 280 | chr11: 65888441-65888468 |
| FIBP 7 | chr11: 65883790-65883817 | FIBP 281 | chr11: 65888442-65888469 |
| FIBP 8 | chr11: 65883791-65883818 | FIBP 282 | chr11: 65888443-65888470 |
| FIBP 9 | chr11: 65883810-65883837 | FIBP 283 | chr11: 65888446-65888473 |
| FIBP 10 | chr11: 65883811-65883838 | FIBP 284 | chr11: 65888461-65888488 |
| FIBP 11 | chr11: 65883819-65883846 | FIBP 285 | chr11: 65888471-65888498 |
| FIBP 12 | chr11: 65883820-65883847 | FIBP 286 | chr11: 65888472-65888499 |
| FIBP 13 | chr11: 65883828-65883855 | FIBP 287 | chr11: 65888478-65888505 |
| FIBP 14 | chr11: 65883832-65883859 | FIBP 288 | chr11: 65888481-65888508 |
| FIBP 15 | chr11: 65883836-65883863 | FIBP 289 | chr11: 65888489-65888516 |
| FIBP 16 | chr11: 65883848-65883875 | FIBP 290 | chr11: 65888494-65888521 |
| FIBP 17 | chr11: 65883851-65883878 | FIBP 291 | chr11: 65888495-65888522 |
| FIBP 18 | chr11: 65883879-65883906 | FIBP 292 | chr11: 65888500-65888527 |
| FIBP 19 | chr11: 65883886-65883913 | FIBP 293 | chr11: 65888501-65888528 |
| FIBP 20 | chr11: 65883888-65883915 | FIBP 294 | chr11: 65888504-65888531 |
| FIBP 21 | chr11: 65883889-65883916 | FIBP 295 | chr11: 65888512-65888539 |
| FIBP 22 | chr11: 65883890-65883917 | FIBP 296 | chr11: 65888524-65888551 |
| FIBP 23 | chr11: 65883891-65883918 | FIBP 297 | chr11: 65888525-65888552 |
| FIBP 24 | chr11: 65883892-65883919 | FIBP 298 | chr11: 65888530-65888557 |
| FIBP 25 | chr11: 65883901-65883928 | FIBP 299 | chr11: 65888531-65888558 |
| FIBP 26 | chr11: 65883902-65883929 | FIBP 300 | chr11: 65888539-65888566 |
| FIBP 27 | chr11: 65883903-65883930 | FIBP 301 | chr11: 65888540-65888567 |
| FIBP 28 | chr11: 65883915-65883942 | FIBP 302 | chr11: 65888541-65888568 |
| FIBP 29 | chr11: 65883922-65883949 | FIBP 303 | chr11: 65888542-65888569 |
| FIBP 30 | chr11: 65883926-65883953 | FIBP 304 | chr11: 65888544-65888571 |
| FIBP 31 | chr11: 65883927-65883954 | FIBP 305 | chr11: 65888545-65888572 |
| FIBP 32 | chr11: 65883930-65883957 | FIBP 306 | chr11: 65888546-65888573 |
| FIBP 33 | chr11: 65883938-65883965 | FIBP 307 | chr11: 65888548-65888575 |
| FIBP 34 | chr11: 65883939-65883966 | FIBP 308 | chr11: 65888550-65888577 |
| FIBP 35 | chr11: 65883942-65883969 | FIBP 309 | chr11: 65888551-65888578 |
| FIBP 36 | chr11: 65883962-65883989 | FIBP 310 | chr11: 65888552-65888579 |
| FIBP 37 | chr11: 65883968-65883995 | FIBP 311 | chr11: 65888555-65888582 |
| FIBP 38 | chr11: 65883971-65883998 | FIBP 312 | chr11: 65888556-65888583 |
| FIBP 39 | chr11: 65883972-65883999 | FIBP 313 | chr11: 65888559-65888586 |
| FIBP 40 | chr11: 65883980-65884007 | FIBP 314 | chr11: 65888561-65888588 |
| FIBP 41 | chr11: 65883983-65884010 | FIBP 315 | chr11: 65888566-65888593 |
| FIBP 42 | chr11: 65883984-65884011 | FIBP 316 | chr11: 65888567-65888594 |
| FIBP 43 | chr11: 65883998-65884025 | FIBP 317 | chr11: 65888575-65888602 |
| FIBP 44 | chr11: 65884002-65884029 | FIBP 318 | chr11: 65888576-65888603 |
| FIBP 45 | chr11: 65884007-65884034 | FIBP 319 | chr11: 65888577-65888604 |
| FIBP 46 | chr11: 65884008-65884035 | FIBP 320 | chr11: 65888578-65888605 |
| FIBP 47 | chr11: 65884013-65884040 | FIBP 321 | chr11: 65888581-65888608 |
| FIBP 48 | chr11: 65884014-65884041 | FIBP 322 | chr11: 65888590-65888617 |
| FIBP 49 | chr11: 65884015-65884042 | FIBP 323 | chr11: 65888593-65888620 |
| FIBP 50 | chr11: 65884027-65884054 | FIBP 324 | chr11: 65888609-65888636 |
| FIBP 51 | chr11: 65884028-65884055 | FIBP 325 | chr11: 65888614-65888641 |
| FIBP 52 | chr11: 65884036-65884063 | FIBP 326 | chr11: 65888615-65888642 |
| FIBP 53 | chr11: 65884367-65884394 | FIBP 327 | chr11: 65888618-65888645 |
| FIBP 54 | chr11: 65884379-65884406 | FIBP 328 | chr11: 65888619-65888646 |
| FIBP 55 | chr11: 65884380-65884407 | FIBP 329 | chr11: 65888620-65888647 |
| FIBP 56 | chr11: 65884385-65884412 | FIBP 330 | chr11: 65888621-65888648 |
| FIBP 57 | chr11: 65884391-65884418 | FIBP 331 | chr11: 65888625-65888652 |
| FIBP 58 | chr11: 65884398-65884425 | FIBP 332 | chr11: 65888629-65888656 |
| FIBP 59 | chr11: 65884409-65884436 | FIBP 333 | chr11: 65888630-65888657 |
| FIBP 60 | chr11: 65884432-65884459 | FIBP 334 | chr11: 65888631-65888658 |
| FIBP 61 | chr11: 65884439-65884466 | FIBP 335 | chr11: 65888634-65888661 |
| FIBP 62 | chr11: 65884442-65884469 | FIBP 336 | chr11: 65888635-65888662 |
| FIBP 63 | chr11: 65884443-65884470 | FIBP 337 | chr11: 65888638-65888665 |
| FIBP 64 | chr11: 65884448-65884475 | FIBP 338 | chr11: 65888652-65888679 |
| FIBP 65 | chr11: 65884452-65884479 | FIBP 339 | chr11: 65888653-65888680 |
| FIBP 66 | chr11: 65884453-65884480 | FIBP 340 | chr11: 65888656-65888683 |
| FIBP 67 | chr11: 65884460-65884487 | FIBP 341 | chr11: 65888660-65888687 |
| FIBP 68 | chr11: 65884468-65884495 | FIBP 342 | chr11: 65888667-65888694 |
| FIBP 69 | chr11: 65884469-65884496 | FIBP 343 | chr11: 65888674-65888701 |
| FIBP 70 | chr11: 65884483-65884510 | FIBP 344 | chr11: 65888675-65888702 |
| FIBP 71 | chr11: 65884555-65884582 | FIBP 345 | chr11: 65888677-65888704 |
| FIBP 72 | chr11: 65884567-65884594 | FIBP 346 | chr11: 65888678-65888705 |
| FIBP 73 | chr11: 65884569-65884596 | FIBP 347 | chr11: 65888679-65888706 |
| FIBP 74 | chr11: 65884575-65884602 | FIBP 348 | chr11: 65888680-65888707 |
| FIBP 75 | chr11: 65884584-65884611 | FIBP 349 | chr11: 65888684-65888711 |
| FIBP 76 | chr11: 65884588-65884615 | FIBP 350 | chr11: 65888702-65888729 |
| FIBP 77 | chr11: 65884589-65884616 | FIBP 351 | chr11: 65888710-65888737 |
| FIBP 78 | chr11: 65884598-65884625 | FIBP 352 | chr11: 65888713-65888740 |
| FIBP 79 | chr11: 65884601-65884628 | FIBP 353 | chr11: 65888724-65888751 |
| FIBP 80 | chr11: 65884621-65884648 | FIBP 354 | chr11: 65888731-65888758 |
| FIBP 81 | chr11: 65884622-65884649 | FIBP 355 | chr11: 65888732-65888759 |
| FIBP 82 | chr11: 65884627-65884654 | FIBP 356 | chr11: 65888733-65888760 |
| FIBP 83 | chr11: 65884629-65884656 | FIBP 357 | chr11: 65888734-65888761 |
| FIBP 84 | chr11: 65884635-65884662 | FIBP 358 | chr11: 65888744-65888771 |
| FIBP 85 | chr11: 65884636-65884663 | FIBP 359 | chr11: 65888745-65888772 |
| FIBP 86 | chr11: 65884637-65884664 | FIBP 360 | chr11: 65888746-65888773 |
| FIBP 87 | chr11: 65884638-65884665 | FIBP 361 | chr11: 65888754-65888781 |
| FIBP 88 | chr11: 65884641-65884668 | FIBP 362 | chr11: 65888757-65888784 |
| FIBP 89 | chr11: 65884642-65884669 | FIBP 363 | chr11: 65888764-65888791 |
| FIBP 90 | chr11: 65884643-65884670 | FIBP 364 | chr11: 65888765-65888792 |
| FIBP 91 | chr11: 65884644-65884671 | FIBP 365 | chr11: 65888769-65888796 |
| FIBP 92 | chr11: 65884645-65884672 | FIBP 366 | chr11: 65888770-65888797 |
| FIBP 93 | chr11: 65884650-65884677 | FIBP 367 | chr11: 65888773-65888800 |
| FIBP 94 | chr11: 65884918-65884945 | FIBP 368 | chr11: 65888782-65888809 |
| FIBP 95 | chr11: 65884927-65884954 | FIBP 369 | chr11: 65888783-65888810 |
| FIBP 96 | chr11: 65884934-65884961 | FIBP 370 | chr11: 65888794-65888821 |
| FIBP 97 | chr11: 65884949-65884976 | FIBP 371 | chr11: 65888797-65888824 |
| FIBP 98 | chr11: 65884953-65884980 | FIBP 372 | chr11: 65888799-65888826 |
| FIBP 99 | chr11: 65884966-65884993 | FIBP 373 | chr11: 65888800-65888827 |
| FIBP 100 | chr11: 65884967-65884994 | FIBP 374 | chr11: 65888801-65888828 |
| FIBP 101 | chr11: 65884971-65884998 | FIBP 375 | chr11: 65888808-65888835 |
| FIBP 102 | chr11: 65884975-65885002 | FIBP 376 | chr11: 65888810-65888837 |
| FIBP 103 | chr11: 65884976-65885003 | FIBP 377 | chr11: 65888811-65888838 |
| FIBP 104 | chr11: 65884977-65885004 | FIBP 378 | chr11: 65888815-65888842 |
| FIBP 105 | chr11: 65884986-65885013 | FIBP 379 | chr11: 65888838-65888865 |
| FIBP 106 | chr11: 65884987-65885014 | FIBP 380 | chr11: 65888839-65888866 |
| FIBP 107 | chr11: 65884994-65885021 | FIBP 381 | chr11: 65888840-65888867 |
| FIBP 108 | chr11: 65885051-65885078 | FIBP 382 | chr11: 65888845-65888872 |
| FIBP 109 | chr11: 65885054-65885081 | FIBP 383 | chr11: 65888854-65888881 |
| FIBP 110 | chr11: 65885055-65885082 | FIBP 384 | chr11: 65888859-65888886 |
| FIBP 111 | chr11: 65885056-65885083 | FIBP 385 | chr11: 65888860-65888887 |
| FIBP 112 | chr11: 65885062-65885089 | FIBP 386 | chr11: 65888870-65888897 |
| FIBP 113 | chr11: 65885063-65885090 | FIBP 387 | chr11: 65888873-65888900 |
| FIBP 114 | chr11: 65885064-65885091 | FIBP 388 | chr11: 65888874-65888901 |
| FIBP 115 | chr11: 65885065-65885092 | FIBP 389 | chr11: 65888877-65888904 |
| FIBP 116 | chr11: 65885071-65885098 | FIBP 390 | chr11: 65888881-65888908 |
| FIBP 117 | chr11: 65885077-65885104 | FIBP 391 | chr11: 65888910-65888937 |
| FIBP 118 | chr11: 65885091-65885118 | FIBP 392 | chr11: 65888911-65888938 |
| FIBP 119 | chr11: 65885100-65885127 | FIBP 393 | chr11: 65888914-65888941 |
| FIBP 120 | chr11: 65885109-65885136 | FIBP 394 | chr11: 65888916-65888943 |
| FIBP 121 | chr11: 65885113-65885140 | FIBP 395 | chr11: 65888917-65888944 |
| FIBP 122 | chr11: 65885118-65885145 | FIBP 396 | chr11: 65888918-65888945 |
| FIBP 123 | chr11: 65885127-65885154 | FIBP 397 | chr11: 65888928-65888955 |
| FIBP 124 | chr11: 65885136-65885163 | FIBP 398 | chr11: 65888933-65888960 |
| FIBP 125 | chr11: 65885148-65885175 | FIBP 399 | chr11: 65888936-65888963 |
| FIBP 126 | chr11: 65885160-65885187 | FIBP 400 | chr11: 65888948-65888975 |
| FIBP 127 | chr11: 65885166-65885193 | FIBP 401 | chr11: 65888954-65888981 |
| FIBP 128 | chr11: 65885167-65885194 | FIBP 402 | chr11: 65888955-65888982 |
| FIBP 129 | chr11: 65885175-65885202 | FIBP 403 | chr11: 65888958-65888985 |
| FIBP 130 | chr11: 65885176-65885203 | FIBP 404 | chr11: 65888971-65888998 |
| FIBP 131 | chr11: 65885177-65885204 | FIBP 405 | chr11: 65888972-65888999 |
| FIBP 132 | chr11: 65885178-65885205 | FIBP 406 | chr11: 65888975-65889002 |
| FIBP 133 | chr11: 65885179-65885206 | FIBP 407 | chr11: 65888976-65889003 |
| FIBP 134 | chr11: 65885182-65885209 | FIBP 408 | chr11: 65888977-65889004 |
| FIBP 135 | chr11: 65885183-65885210 | FIBP 409 | chr11: 65888978-65889005 |
| FIBP 136 | chr11: 65885184-65885211 | FIBP 410 | chr11: 65888982-65889009 |
| FIBP 137 | chr11: 65885185-65885212 | FIBP 411 | chr11: 65888983-65889010 |
| FIBP 138 | chr11: 65885482-65885509 | FIBP 412 | chr11: 65888984-65889011 |
| FIBP 139 | chr11: 65885485-65885512 | FIBP 413 | chr11: 65889004-65889031 |
| FIBP 140 | chr11: 65885486-65885513 | FIBP 414 | chr11: 65889012-65889039 |
| FIBP 141 | chr11: 65885493-65885520 | FIBP 415 | chr11: 65889013-65889040 |
| FIBP 142 | chr11: 65885494-65885521 | FIBP 416 | chr11: 65889014-65889041 |
| FIBP 143 | chr11: 65885495-65885522 | FIBP 417 | chr11: 65889017-65889044 |
| FIBP 144 | chr11: 65885496-65885523 | FIBP 418 | chr11: 65889018-65889045 |
| FIBP 145 | chr11: 65885501-65885528 | FIBP 419 | chr11: 65889021-65889048 |
| FIBP 146 | chr11: 65885508-65885535 | FIBP 420 | chr11: 65889022-65889049 |
| FIBP 147 | chr11: 65885516-65885543 | FIBP 421 | chr11: 65889023-65889050 |
| FIBP 148 | chr11: 65885517-65885544 | FIBP 422 | chr11: 65889027-65889054 |
| FIBP 149 | chr11: 65885524-65885551 | FIBP 423 | chr11: 65889034-65889061 |
| FIBP 150 | chr11: 65885528-65885555 | FIBP 424 | chr11: 65889036-65889063 |
| FIBP 151 | chr11: 65885529-65885556 | FIBP 425 | chr11: 65889042-65889069 |
| FIBP 152 | chr11: 65885538-65885565 | FIBP 426 | chr11: 65889051-65889078 |
| FIBP 153 | chr11: 65885546-65885573 | FIBP 427 | chr11: 65889054-65889081 |
| FIBP 154 | chr11: 65885550-65885577 | FIBP 428 | chr11: 65889062-65889089 |
| FIBP 155 | chr11: 65885586-65885613 | FIBP 429 | chr11: 65889072-65889099 |
| FIBP 156 | chr11: 65885603-65885630 | FIBP 430 | chr11: 65889082-65889109 |
| FIBP 157 | chr11: 65885615-65885642 | FIBP 431 | chr11: 65889083-65889110 |
| FIBP 158 | chr11: 65885616-65885643 | FIBP 432 | chr11: 65889090-65889117 |
| FIBP 159 | chr11: 65885625-65885652 | FIBP 433 | chr11: 65889091-65889118 |
| FIBP 160 | chr11: 65885652-65885679 | FIBP 434 | chr11: 65889092-65889119 |
| FIBP 161 | chr11: 65885655-65885682 | FIBP 435 | chr11: 65889121-65889148 |
| FIBP 162 | chr11: 65885656-65885683 | FIBP 436 | chr11: 65889127-65889154 |
| FIBP 163 | chr11: 65885662-65885689 | FIBP 437 | chr11: 65889132-65889159 |
| FIBP 164 | chr11: 65885663-65885690 | FIBP 438 | chr11: 65889133-65889160 |
| FIBP 165 | chr11: 65886298-65886325 | FIBP 439 | chr11: 65889134-65889161 |
| FIBP 166 | chr11: 65886306-65886333 | FIBP 440 | chr11: 65889148-65889175 |
| FIBP 167 | chr11: 65886315-65886342 | FIBP 441 | chr11: 65889155-65889182 |
| FIBP 168 | chr11: 65886316-65886343 | FIBP 442 | chr11: 65889156-65889183 |
| FIBP 169 | chr11: 65886318-65886345 | FIBP 443 | chr11: 65889172-65889199 |
| FIBP 170 | chr11: 65886321-65886348 | FIBP 444 | chr11: 65889173-65889200 |
| FIBP 171 | chr11: 65886326-65886353 | FIBP 445 | chr11: 65889180-65889207 |
| FIBP 172 | chr11: 65886330-65886357 | FIBP 446 | chr11: 65889181-65889208 |
| FIBP 173 | chr11: 65886345-65886372 | FIBP 447 | chr11: 65889184-65889211 |
| FIBP 174 | chr11: 65886346-65886373 | FIBP 448 | chr11: 65889203-65889230 |
| FIBP 175 | chr11: 65886365-65886392 | FIBP 449 | chr11: 65889216-65889243 |
| FIBP 176 | chr11: 65886368-65886395 | FIBP 450 | chr11: 65889219-65889246 |
| FIBP 177 | chr11: 65886376-65886403 | FIBP 451 | chr11: 65889223-65889250 |
| FIBP 178 | chr11: 65886377-65886404 | FIBP 452 | chr11: 65889230-65889257 |
| FIBP 179 | chr11: 65886378-65886405 | FIBP 453 | chr11: 65889231-65889258 |
| FIBP 180 | chr11: 65886386-65886413 | FIBP 454 | chr11: 65889232-65889259 |
| FIBP 181 | chr11: 65886392-65886419 | FIBP 455 | chr11: 65889255-65889282 |
| FIBP 182 | chr11: 65886395-65886422 | FIBP 456 | chr11: 65889258-65889285 |
| FIBP 183 | chr11: 65886402-65886429 | FIBP 457 | chr11: 65889259-65889286 |
| FIBP 184 | chr11: 65886403-65886430 | FIBP 458 | chr11: 65889284-65889311 |
| FIBP 185 | chr11: 65886404-65886431 | FIBP 459 | chr11: 65889289-65889316 |
| FIBP 186 | chr11: 65886405-65886432 | FIBP 460 | chr11: 65889292-65889319 |
| FIBP 187 | chr11: 65886411-65886438 | FIBP 461 | chr11: 65889293-65889320 |
| FIBP 188 | chr11: 65886412-65886439 | FIBP 462 | chr11: 65889294-65889321 |
| FIBP 189 | chr11: 65887579-65887606 | FIBP 463 | chr11: 65889295-65889322 |
| FIBP 190 | chr11: 65887582-65887609 | FIBP 464 | chr11: 65889307-65889334 |
| FIBP 191 | chr11: 65887594-65887621 | FIBP 465 | chr11: 65889308-65889335 |
| FIBP 192 | chr11: 65887595-65887622 | FIBP 466 | chr11: 65889309-65889336 |
| FIBP 193 | chr11: 65887599-65887626 | FIBP 467 | chr11: 65889313-65889340 |
| FIBP 194 | chr11: 65887606-65887633 | FIBP 468 | chr11: 65889333-65889360 |
| FIBP 195 | chr11: 65887610-65887637 | FIBP 469 | chr11: 65889354-65889381 |
| FIBP 196 | chr11: 65887627-65887654 | FIBP 470 | chr11: 65889365-65889392 |
| FIBP 197 | chr11: 65887628-65887655 | FIBP 471 | chr11: 65889366-65889393 |
| FIBP 198 | chr11: 65887637-65887664 | FIBP 472 | chr11: 65889367-65889394 |
| FIBP 199 | chr11: 65887646-65887673 | FIBP 473 | chr11: 65889368-65889395 |
| FIBP 200 | chr11: 65887650-65887677 | FIBP 474 | chr11: 65889371-65889398 |
| FIBP 201 | chr11: 65887667-65887694 | FIBP 475 | chr11: 65889379-65889406 |
| FIBP 202 | chr11: 65887679-65887706 | FIBP 476 | chr11: 65889380-65889407 |
| FIBP 203 | chr11: 65887685-65887712 | FIBP 477 | chr11: 65889381-65889408 |
| FIBP 204 | chr11: 65887686-65887713 | FIBP 478 | chr11: 65889384-65889411 |
| FIBP 205 | chr11: 65887691-65887718 | FIBP 479 | chr11: 65889385-65889412 |
| FIBP 206 | chr11: 65887692-65887719 | FIBP 480 | chr11: 65889386-65889413 |
| FIBP 207 | chr11: 65887695-65887722 | FIBP 481 | chr11: 65889395-65889422 |
| FIBP 208 | chr11: 65887696-65887723 | FIBP 482 | chr11: 65889399-65889426 |
| FIBP 209 | chr11: 65887706-65887733 | FIBP 483 | chr11: 65889400-65889427 |
| FIBP 210 | chr11: 65887707-65887734 | FIBP 484 | chr11: 65889413-65889440 |
| FIBP 211 | chr11: 65887726-65887753 | FIBP 485 | chr11: 65889414-65889441 |
| FIBP 212 | chr11: 65887911-65887938 | FIBP 486 | chr11: 65889443-65889470 |
| FIBP 213 | chr11: 65887914-65887941 | FIBP 487 | chr11: 65889447-65889474 |
| FIBP 214 | chr11: 65887918-65887945 | FIBP 488 | chr11: 65889457-65889484 |
| FIBP 215 | chr11: 65887919-65887946 | FIBP 489 | chr11: 65889465-65889492 |
| FIBP 216 | chr11: 65887930-65887957 | FIBP 490 | chr11: 65889466-65889493 |
| FIBP 217 | chr11: 65887931-65887958 | FIBP 491 | chr11: 65889468-65889495 |
| FIBP 218 | chr11: 65887933-65887960 | FIBP 492 | chr11: 65889492-65889519 |
| FIBP 219 | chr11: 65887934-65887961 | FIBP 493 | chr11: 65889493-65889520 |
| FIBP 220 | chr11: 65887935-65887962 | FIBP 494 | chr11: 65889506-65889533 |
| FIBP 221 | chr11: 65887938-65887965 | FIBP 495 | chr11: 65889509-65889536 |
| FIBP 222 | chr11: 65887945-65887972 | FIBP 496 | chr11: 65889512-65889539 |
| FIBP 223 | chr11: 65887950-65887977 | FIBP 497 | chr11: 65889515-65889542 |
| FIBP 224 | chr11: 65887954-65887981 | FIBP 498 | chr11: 65889516-65889543 |
| FIBP 225 | chr11: 65887957-65887984 | FIBP 499 | chr11: 65889537-65889564 |
| FIBP 226 | chr11: 65887970-65887997 | FIBP 500 | chr11: 65889540-65889567 |
| FIBP 227 | chr11: 65887971-65887998 | FIBP 501 | chr11: 65889543-65889570 |
| FIBP 228 | chr11: 65887974-65888001 | FIBP 502 | chr11: 65889546-65889573 |
| FIBP 229 | chr11: 65888002-65888029 | FIBP 503 | chr11: 65889547-65889574 |
| FIBP 230 | chr11: 65888006-65888033 | FIBP 504 | chr11: 65889559-65889586 |
| FIBP 231 | chr11: 65888011-65888038 | FIBP 505 | chr11: 65889570-65889597 |
| FIBP 232 | chr11: 65888014-65888041 | FIBP 506 | chr11: 65889573-65889600 |
| FIBP 233 | chr11: 65888017-65888044 | FIBP 507 | chr11: 65889574-65889601 |
| FIBP 234 | chr11: 65888024-65888051 | FIBP 508 | chr11: 65889577-65889604 |
| FIBP 235 | chr11: 65888046-65888073 | FIBP 509 | chr11: 65889582-65889609 |
| FIBP 236 | chr11: 65888051-65888078 | FIBP 510 | chr11: 65889583-65889610 |
| FIBP 237 | chr11: 65888067-65888094 | FIBP 511 | chr11: 65889587-65889614 |
| FIBP 238 | chr11: 65888068-65888095 | FIBP 512 | chr11: 65889588-65889615 |
| FIBP 239 | chr11: 65888073-65888100 | FIBP 513 | chr11: 65889589-65889616 |
| FIBP 240 | chr11: 65888081-65888108 | FIBP 514 | chr11: 65889603-65889630 |
| FIBP 241 | chr11: 65888089-65888116 | FIBP 515 | chr11: 65889614-65889641 |
| FIBP 242 | chr11: 65888090-65888117 | FIBP 516 | chr11: 65889622-65889649 |
| FIBP 243 | chr11: 65888091-65888118 | FIBP 517 | chr11: 65889631-65889658 |
| FIBP 244 | chr11: 65888099-65888126 | FIBP 518 | chr11: 65889661-65889688 |
| FIBP 245 | chr11: 65888100-65888127 | FIBP 519 | chr11: 65889662-65889689 |
| FIBP 246 | chr11: 65888102-65888129 | FIBP 520 | chr11: 65889675-65889702 |
| FIBP 247 | chr11: 65888109-65888136 | FIBP 521 | chr11: 65889686-65889713 |
| FIBP 248 | chr11: 65888110-65888137 | FIBP 522 | chr11: 65889693-65889720 |
| FIBP 249 | chr11: 65888116-65888143 | FIBP 523 | chr11: 65889705-65889732 |
| FIBP 250 | chr11: 65888118-65888145 | FIBP 524 | chr11: 65889709-65889736 |
| FIBP 251 | chr11: 65888121-65888148 | FIBP 525 | chr11: 65889714-65889741 |
| FIBP 252 | chr11: 65888130-65888157 | FIBP 526 | chr11: 65889715-65889742 |
| FIBP 253 | chr11: 65888313-65888340 | FIBP 527 | chr11: 65889728-65889755 |
| FIBP 254 | chr11: 65888314-65888341 | FIBP 528 | chr11: 65889732-65889759 |
| FIBP 255 | chr11: 65888315-65888342 | FIBP 529 | chr11: 65889733-65889760 |
| FIBP 256 | chr11: 65888322-65888349 | FIBP 530 | chr11: 65889767-65889794 |
| FIBP 257 | chr11: 65888323-65888350 | FIBP 531 | chr11: 65889771-65889798 |
| FIBP 258 | chr11: 65888324-65888351 | FIBP 532 | chr11: 65889780-65889807 |
| FIBP 259 | chr11: 65888329-65888356 | FIBP 533 | chr11: 65889830-65889857 |
| FIBP 260 | chr11: 65888333-65888360 | FIBP 534 | chr11: 65889836-65889863 |
| FIBP 261 | chr11: 65888334-65888361 | FIBP 535 | chr11: 65889839-65889866 |
| FIBP 262 | chr11: 65888342-65888369 | FIBP 536 | chr11: 65889845-65889872 |
| FIBP 263 | chr11: 65888350-65888377 | FIBP 537 | chr11: 65889846-65889873 |
| FIBP 264 | chr11: 65888353-65888380 | FIBP 538 | chr11: 65889849-65889876 |
| FIBP 265 | chr11: 65888354-65888381 | FIBP 539 | chr11: 65889876-65889903 |
| FIBP 266 | chr11: 65888367-65888394 | FIBP 540 | chr11: 65889900-65889927 |
| FIBP 267 | chr11: 65888387-65888414 | FIBP 541 | chr11: 65889905-65889932 |
| FIBP 268 | chr11: 65888389-65888416 | FIBP 542 | chr11: 65889906-65889933 |
| FIBP 269 | chr11: 65888390-65888417 | FIBP 543 | chr11: 65889913-65889940 |
| FIBP 270 | chr11: 65888391-65888418 | FIBP 544 | chr11: 65889932-65889959 |
| FIBP 271 | chr11: 65888393-65888420 | FIBP 545 | chr11: 65889934-65889961 |
| FIBP 272 | chr11: 65888403-65888430 | FIBP 546 | chr11: 65889946-65889973 |
| FIBP 273 | chr11: 65888404-65888431 | FIBP 547 | chr11: 65889952-65889979 |
| FIBP 274 | chr11: 65888405-65888432 | | |

**Tabel 2C. Genomic Coordinates of Human MED12 in the Current Invention**

| Index | Chromosomal coordinate range | Index | Chromosomal coordinate range |
|---|---|---|---|
| MED12 1 | chrX: 71117114-71117141 | MED12 789 | chrX: 71127425-71127452 |
| MED12 2 | chrX: 71117163-71117190 | MED12 790 | chrX: 71127428-71127455 |
| MED12 3 | chrX: 71117181-71117208 | MED12 791 | chrX: 71127429-71127456 |
| MED12 4 | chrX: 71117187-71117214 | MED12 792 | chrX: 71127430-71127457 |
| MED12 5 | chrX: 71117190-71117217 | MED12 793 | chrX: 71127435-71127462 |
| MED12 6 | chrX: 71117193-71117220 | MED12 794 | chrX: 71127441-71127468 |
| MED12 7 | chrX: 71117196-71117223 | MED12 795 | chrX: 71127450-71127477 |
| MED12 8 | chrX: 71117220-71117247 | MED12 796 | chrX: 71127453-71127480 |
| MED12 9 | chrX: 71117224-71117251 | MED12 797 | chrX: 71127457-71127484 |
| MED12 10 | chrX: 71117227-71117254 | MED12 798 | chrX: 71127462-71127489 |
| MED12 11 | chrX: 71117236-71117263 | MED12 799 | chrX: 71127463-71127490 |
| MED12 12 | chrX: 71117237-71117264 | MED12 800 | chrX: 71127464-71127491 |
| MED12 13 | chrX: 71117252-71117279 | MED12 801 | chrX: 71127873-71127900 |
| MED12 14 | chrX: 71117257-71117284 | MED12 802 | chrX: 71127878-71127905 |
| MED12 15 | chrX: 71117261-71117288 | MED12 803 | chrX: 71127881-71127908 |
| MED12 16 | chrX: 71117271-71117298 | MED12 804 | chrX: 71127882-71127909 |
| MED12 17 | chrX: 71117280-71117307 | MED12 805 | chrX: 71127885-71127912 |
| MED12 18 | chrX: 71117282-71117309 | MED12 806 | chrX: 71127886-71127913 |
| MED12 19 | chrX: 71117298-71117325 | MED12 807 | chrX: 71127909-71127936 |
| MED12 20 | chrX: 71117303-71117330 | MED12 808 | chrX: 71127918-71127945 |
| MED12 21 | chrX: 71117314-71117341 | MED12 809 | chrX: 71127919-71127946 |
| MED12 22 | chrX: 71117315-71117342 | MED12 810 | chrX: 71127935-71127962 |
| MED12 23 | chrX: 71117316-71117343 | MED12 811 | chrX: 71127936-71127963 |
| MED12 24 | chrX: 71117320-71117347 | MED12 812 | chrX: 71127939-71127966 |
| MED12 25 | chrX: 71117322-71117349 | MED12 813 | chrX: 71127966-71127993 |
| MED12 26 | chrX: 71117326-71117353 | MED12 814 | chrX: 71127995-71128022 |
| MED12 27 | chrX: 71117329-71117356 | MED12 815 | chrX: 71127996-71128023 |
| MED12 28 | chrX: 71117330-71117357 | MED12 816 | chrX: 71127997-71128024 |
| MED12 29 | chrX: 71117357-71117384 | MED12 817 | chrX: 71127998-71128025 |
| MED12 30 | chrX: 71117360-71117387 | MED12 818 | chrX: 71128003-71128030 |
| MED12 31 | chrX: 71117365-71117392 | MED12 819 | chrX: 71128009-71128036 |
| MED12 32 | chrX: 71117366-71117393 | MED12 820 | chrX: 71128015-71128042 |
| MED12 33 | chrX: 71117367-71117394 | MED12 821 | chrX: 71128049-71128076 |
| MED12 34 | chrX: 71117370-71117397 | MED12 822 | chrX: 71128050-71128077 |
| MED12 35 | chrX: 71117390-71117417 | MED12 823 | chrX: 71128058-71128085 |
| MED12 36 | chrX: 71117402-71117429 | MED12 824 | chrX: 71128074-71128101 |
| MED12 37 | chrX: 71117450-71117477 | MED12 825 | chrX: 71128075-71128102 |
| MED12 38 | chrX: 71117469-71117496 | MED12 826 | chrX: 71128076-71128103 |
| MED12 39 | chrX: 71117479-71117506 | MED12 827 | chrX: 71128081-71128108 |
| MED12 40 | chrX: 71117483-71117510 | MED12 828 | chrX: 71128082-71128109 |
| MED12 41 | chrX: 71117491-71117518 | MED12 829 | chrX: 71128094-71128121 |
| MED12 42 | chrX: 71117507-71117534 | MED12 830 | chrX: 71128099-71128126 |
| MED12 43 | chrX: 71117510-71117537 | MED12 831 | chrX: 71128100-71128127 |
| MED12 44 | chrX: 71117517-71117544 | MED12 832 | chrX: 71128101-71128128 |
| MED12 45 | chrX: 71117518-71117545 | MED12 833 | chrX: 71128106-71128133 |
| MED12 46 | chrX: 71117521-71117548 | MED12 834 | chrX: 71128113-71128140 |
| MED12 47 | chrX: 71117525-71117552 | MED12 835 | chrX: 71128114-71128141 |
| MED12 48 | chrX: 71117527-71117554 | MED12 836 | chrX: 71128115-71128142 |
| MED12 49 | chrX: 71117530-71117557 | MED12 837 | chrX: 71128119-71128146 |
| MED12 50 | chrX: 71117531-71117558 | MED12 838 | chrX: 71128120-71128147 |
| MED12 51 | chrX: 71117533-71117560 | MED12 839 | chrX: 71128269-71128296 |
| MED12 52 | chrX: 71117534-71117561 | MED12 840 | chrX: 71128270-71128297 |
| MED12 53 | chrX: 71117542-71117569 | MED12 841 | chrX: 71128290-71128317 |
| MED12 54 | chrX: 71117553-71117580 | MED12 842 | chrX: 71128291-71128318 |
| MED12 55 | chrX: 71117573-71117600 | MED12 843 | chrX: 71128307-71128334 |
| MED12 56 | chrX: 71117597-71117624 | MED12 844 | chrX: 71128314-71128341 |
| MED12 57 | chrX: 71117600-71117627 | MED12 845 | chrX: 71128331-71128358 |
| MED12 58 | chrX: 71117608-71117635 | MED12 846 | chrX: 71128335-71128362 |
| MED12 59 | chrX: 71117609-71117636 | MED12 847 | chrX: 71128340-71128367 |
| MED12 60 | chrX: 71117610-71117637 | MED12 848 | chrX: 71128351-71128378 |
| MED12 61 | chrX: 71117618-71117645 | MED12 849 | chrX: 71128376-71128403 |
| MED12 62 | chrX: 71117622-71117649 | MED12 850 | chrX: 71128379-71128406 |
| MED12 63 | chrX: 71117642-71117669 | MED12 851 | chrX: 71128385-71128412 |
| MED12 64 | chrX: 71117646-71117673 | MED12 852 | chrX: 71128391-71128418 |
| MED12 65 | chrX: 71117652-71117679 | MED12 853 | chrX: 71128424-71128451 |
| MED12 66 | chrX: 71117659-71117686 | MED12 854 | chrX: 71128425-71128452 |
| MED12 67 | chrX: 71117665-71117692 | MED12 855 | chrX: 71128426-71128453 |
| MED12 68 | chrX: 71117710-71117737 | MED12 856 | chrX: 71128429-71128456 |
| MED12 69 | chrX: 71117716-71117743 | MED12 857 | chrX: 71128430-71128457 |
| MED12 70 | chrX: 71117770-71117797 | MED12 858 | chrX: 71128431-71128458 |
| MED12 71 | chrX: 71117777-71117804 | MED12 859 | chrX: 71128571-71128598 |
| MED12 72 | chrX: 71117780-71117807 | MED12 860 | chrX: 71128591-71128618 |
| MED12 73 | chrX: 71117784-71117811 | MED12 861 | chrX: 71128594-71128621 |
| MED12 74 | chrX: 71117795-71117822 | MED12 862 | chrX: 71128601-71128628 |
| MED12 75 | chrX: 71117797-71117824 | MED12 863 | chrX: 71128606-71128633 |
| MED12 76 | chrX: 71117808-71117835 | MED12 864 | chrX: 71128630-71128657 |
| MED12 77 | chrX: 71117809-71117836 | MED12 865 | chrX: 71128641-71128668 |
| MED12 78 | chrX: 71117813-71117840 | MED12 866 | chrX: 71128645-71128672 |
| MED12 79 | chrX: 71117846-71117873 | MED12 867 | chrX: 71128646-71128673 |
| MED12 80 | chrX: 71117868-71117895 | MED12 868 | chrX: 71128669-71128696 |
| MED12 81 | chrX: 71117869-71117896 | MED12 869 | chrX: 71128692-71128719 |
| MED12 82 | chrX: 71117876-71117903 | MED12 870 | chrX: 71128695-71128722 |
| MED12 83 | chrX: 71117881-71117908 | MED12 871 | chrX: 71128699-71128726 |
| MED12 84 | chrX: 71117890-71117917 | MED12 872 | chrX: 71128700-71128727 |
| MED12 85 | chrX: 71117899-71117926 | MED12 873 | chrX: 71128708-71128735 |
| MED12 86 | chrX: 71117900-71117927 | MED12 874 | chrX: 71129088-71129115 |
| MED12 87 | chrX: 71117901-71117928 | MED12 875 | chrX: 71129101-71129128 |
| MED12 88 | chrX: 71117904-71117931 | MED12 876 | chrX: 71129106-71129133 |
| MED12 89 | chrX: 71117909-71117936 | MED12 877 | chrX: 71129114-71129141 |
| MED12 90 | chrX: 71117919-71117946 | MED12 878 | chrX: 71129115-71129142 |
| MED12 91 | chrX: 71117920-71117947 | MED12 879 | chrX: 71129116-71129143 |
| MED12 92 | chrX: 71117921-71117948 | MED12 880 | chrX: 71129121-71129148 |
| MED12 93 | chrX: 71117922-71117949 | MED12 881 | chrX: 71129137-71129164 |
| MED12 94 | chrX: 71117923-71117950 | MED12 882 | chrX: 71129144-71129171 |
| MED12 95 | chrX: 71117995-71118022 | MED12 883 | chrX: 71129145-71129172 |
| MED12 96 | chrX: 71117996-71118023 | MED12 884 | chrX: 71129153-71129180 |
| MED12 97 | chrX: 71118003-71118030 | MED12 885 | chrX: 71129157-71129184 |
| MED12 98 | chrX: 71118031-71118058 | MED12 886 | chrX: 71129163-71129190 |
| MED12 99 | chrX: 71118032-71118059 | MED12 887 | chrX: 71129166-71129193 |
| MED12 100 | chrX: 71118050-71118077 | MED12 888 | chrX: 71129172-71129199 |
| MED12 101 | chrX: 71118060-71118087 | MED12 889 | chrX: 71129185-71129212 |
| MED12 102 | chrX: 71118069-71118096 | MED12 890 | chrX: 71129186-71129213 |
| MED12 103 | chrX: 71118070-71118097 | MED12 891 | chrX: 71129197-71129224 |
| MED12 104 | chrX: 71118090-71118117 | MED12 892 | chrX: 71129202-71129229 |
| MED12 105 | chrX: 71118091-71118118 | MED12 893 | chrX: 71129291-71129318 |
| MED12 106 | chrX: 71118092-71118119 | MED12 894 | chrX: 71129292-71129319 |
| MED12 107 | chrX: 71118108-71118135 | MED12 895 | chrX: 71129293-71129320 |
| MED12 108 | chrX: 71118120-71118147 | MED12 896 | chrX: 71129304-71129331 |
| MED12 109 | chrX: 71118121-71118148 | MED12 897 | chrX: 71129306-71129333 |
| MED12 110 | chrX: 71118140-71118167 | MED12 898 | chrX: 71129310-71129337 |
| MED12 111 | chrX: 71118141-71118168 | MED12 899 | chrX: 71129311-71129338 |
| MED12 112 | chrX: 71118142-71118169 | MED12 900 | chrX: 71129312-71129339 |
| MED12 113 | chrX: 71118151-71118178 | MED12 901 | chrX: 71129321-71129348 |
| MED12 114 | chrX: 71118156-71118183 | MED12 902 | chrX: 71129335-71129362 |
| MED12 115 | chrX: 71118160-71118187 | MED12 903 | chrX: 71129336-71129363 |
| MED12 116 | chrX: 71118202-71118229 | MED12 904 | chrX: 71129340-71129367 |
| MED12 117 | chrX: 71118205-71118232 | MED12 905 | chrX: 71129343-71129370 |
| MED12 118 | chrX: 71118219-71118246 | MED12 906 | chrX: 71129350-71129377 |
| MED12 119 | chrX: 71118220-71118247 | MED12 907 | chrX: 71129353-71129380 |
| MED12 120 | chrX: 71118221-71118248 | MED12 908 | chrX: 71129356-71129383 |
| MED12 121 | chrX: 71118234-71118261 | MED12 909 | chrX: 71129360-71129387 |
| MED12 122 | chrX: 71118243-71118270 | MED12 910 | chrX: 71129364-71129391 |
| MED12 123 | chrX: 71118258-71118285 | MED12 911 | chrX: 71129367-71129394 |
| MED12 124 | chrX: 71118266-71118293 | MED12 912 | chrX: 71129370-71129397 |
| MED12 125 | chrX: 71118298-71118325 | MED12 913 | chrX: 71129371-71129398 |
| MED12 126 | chrX: 71118303-71118330 | MED12 914 | chrX: 71129377-71129404 |
| MED12 127 | chrX: 71118307-71118334 | MED12 915 | chrX: 71129387-71129414 |
| MED12 128 | chrX: 71118323-71118350 | MED12 916 | chrX: 71129394-71129421 |
| MED12 129 | chrX: 71118329-71118356 | MED12 917 | chrX: 71129403-71129430 |
| MED12 130 | chrX: 71118330-71118357 | MED12 918 | chrX: 71129408-71129435 |
| MED12 131 | chrX: 71118335-71118362 | MED12 919 | chrX: 71129409-71129436 |
| MED12 132 | chrX: 71118341-71118368 | MED12 920 | chrX: 71129410-71129437 |
| MED12 133 | chrX: 71118360-71118387 | MED12 921 | chrX: 71129411-71129438 |
| MED12 134 | chrX: 71118365-71118392 | MED12 922 | chrX: 71129415-71129442 |
| MED12 135 | chrX: 71118367-71118394 | MED12 923 | chrX: 71129419-71129446 |
| MED12 136 | chrX: 71118381-71118408 | MED12 924 | chrX: 71129420-71129447 |
| MED12 137 | chrX: 71118384-71118411 | MED12 925 | chrX: 71129425-71129452 |
| MED12 138 | chrX: 71118393-71118420 | MED12 926 | chrX: 71129653-71129680 |
| MED12 139 | chrX: 71118396-71118423 | MED12 927 | chrX: 71129654-71129681 |
| MED12 140 | chrX: 71118403-71118430 | MED12 928 | chrX: 71129655-71129682 |
| MED12 141 | chrX: 71118404-71118431 | MED12 929 | chrX: 71129661-71129688 |
| MED12 142 | chrX: 71118412-71118439 | MED12 930 | chrX: 71129662-71129689 |
| MED12 143 | chrX: 71118416-71118443 | MED12 931 | chrX: 71129663-71129690 |
| MED12 144 | chrX: 71118417-71118444 | MED12 932 | chrX: 71129666-71129693 |
| MED12 145 | chrX: 71118418-71118445 | MED12 933 | chrX: 71129667-71129694 |
| MED12 146 | chrX: 71118433-71118460 | MED12 934 | chrX: 71129671-71129698 |
| MED12 147 | chrX: 71118436-71118463 | MED12 935 | chrX: 71129674-71129701 |
| MED12 148 | chrX: 71118476-71118503 | MED12 936 | chrX: 71129677-71129704 |
| MED12 149 | chrX: 71118477-71118504 | MED12 937 | chrX: 71129692-71129719 |
| MED12 150 | chrX: 71118478-71118505 | MED12 938 | chrX: 71129695-71129722 |
| MED12 151 | chrX: 71118480-71118507 | MED12 939 | chrX: 71129715-71129742 |
| MED12 152 | chrX: 71118483-71118510 | MED12 940 | chrX: 71129718-71129745 |
| MED12 153 | chrX: 71118486-71118513 | MED12 941 | chrX: 71129721-71129748 |
| MED12 154 | chrX: 71118489-71118516 | MED12 942 | chrX: 71129722-71129749 |
| MED12 155 | chrX: 71118499-71118526 | MED12 943 | chrX: 71129725-71129752 |
| MED12 156 | chrX: 71118500-71118527 | MED12 944 | chrX: 71129728-71129755 |
| MED12 157 | chrX: 71118501-71118528 | MED12 945 | chrX: 71129731-71129758 |
| MED12 158 | chrX: 71118506-71118533 | MED12 946 | chrX: 71129736-71129763 |
| MED12 159 | chrX: 71118515-71118542 | MED12 947 | chrX: 71129737-71129764 |
| MED12 160 | chrX: 71118518-71118545 | MED12 948 | chrX: 71129740-71129767 |
| MED12 161 | chrX: 71118519-71118546 | MED12 949 | chrX: 71129744-71129771 |
| MED12 162 | chrX: 71118520-71118547 | MED12 950 | chrX: 71129747-71129774 |
| MED12 163 | chrX: 71118521-71118548 | MED12 951 | chrX: 71129751-71129778 |
| MED12 164 | chrX: 71118524-71118551 | MED12 952 | chrX: 71129752-71129779 |
| MED12 165 | chrX: 71118527-71118554 | MED12 953 | chrX: 71129755-71129782 |
| MED12 166 | chrX: 71118529-71118556 | MED12 954 | chrX: 71129772-71129799 |
| MED12 167 | chrX: 71118534-71118561 | MED12 955 | chrX: 71129783-71129810 |
| MED12 168 | chrX: 71118535-71118562 | MED12 956 | chrX: 71129787-71129814 |
| MED12 169 | chrX: 71118536-71118563 | MED12 957 | chrX: 71129806-71129833 |
| MED12 170 | chrX: 71118539-71118566 | MED12 958 | chrX: 71129824-71129851 |
| MED12 171 | chrX: 71118540-71118567 | MED12 959 | chrX: 71129829-71129856 |
| MED12 172 | chrX: 71118542-71118569 | MED12 960 | chrX: 71129849-71129876 |
| MED12 173 | chrX: 71118547-71118574 | MED12 961 | chrX: 71130008-71130035 |
| MED12 174 | chrX: 71118557-71118584 | MED12 962 | chrX: 71130013-71130040 |
| MED12 175 | chrX: 71118561-71118588 | MED12 963 | chrX: 71130014-71130041 |
| MED12 176 | chrX: 71118567-71118594 | MED12 964 | chrX: 71130018-71130045 |
| MED12 177 | chrX: 71118570-71118597 | MED12 965 | chrX: 71130029-71130056 |
| MED12 178 | chrX: 71118571-71118598 | MED12 966 | chrX: 71130047-71130074 |
| MED12 179 | chrX: 71118577-71118604 | MED12 967 | chrX: 71130055-71130082 |
| MED12 180 | chrX: 71118579-71118606 | MED12 968 | chrX: 71130085-71130112 |
| MED12 181 | chrX: 71118583-71118610 | MED12 969 | chrX: 71130089-71130116 |
| MED12 182 | chrX: 71118588-71118615 | MED12 970 | chrX: 71130094-71130121 |
| MED12 183 | chrX: 71118589-71118616 | MED12 971 | chrX: 71130095-71130122 |
| MED12 184 | chrX: 71118601-71118628 | MED12 972 | chrX: 71130111-71130138 |
| MED12 185 | chrX: 71118620-71118647 | MED12 973 | chrX: 71130112-71130139 |
| MED12 186 | chrX: 71118629-71118656 | MED12 974 | chrX: 71130124-71130151 |
| MED12 187 | chrX: 71118630-71118657 | MED12 975 | chrX: 71130134-71130161 |
| MED12 188 | chrX: 71118631-71118658 | MED12 976 | chrX: 71130143-71130170 |
| MED12 189 | chrX: 71118632-71118659 | MED12 977 | chrX: 71130146-71130173 |
| MED12 190 | chrX: 71118648-71118675 | MED12 978 | chrX: 71130147-71130174 |
| MED12 191 | chrX: 71118651-71118678 | MED12 979 | chrX: 71130148-71130175 |
| MED12 192 | chrX: 71118654-71118681 | MED12 980 | chrX: 71130149-71130176 |
| MED12 193 | chrX: 71118655-71118682 | MED12 981 | chrX: 71130163-71130190 |
| MED12 194 | chrX: 71118663-71118690 | MED12 982 | chrX: 71130181-71130208 |
| MED12 195 | chrX: 71118666-71118693 | MED12 983 | chrX: 71130182-71130209 |
| MED12 196 | chrX: 71118669-71118696 | MED12 984 | chrX: 71130183-71130210 |
| MED12 197 | chrX: 71118670-71118697 | MED12 985 | chrX: 71130184-71130211 |
| MED12 198 | chrX: 71118671-71118698 | MED12 986 | chrX: 71130188-71130215 |
| MED12 199 | chrX: 71118672-71118699 | MED12 987 | chrX: 71130192-71130219 |
| MED12 200 | chrX: 71118673-71118700 | MED12 988 | chrX: 71130198-71130225 |
| MED12 201 | chrX: 71118684-71118711 | MED12 989 | chrX: 71130204-71130231 |
| MED12 202 | chrX: 71118685-71118712 | MED12 990 | chrX: 71130205-71130232 |
| MED12 203 | chrX: 71118690-71118717 | MED12 991 | chrX: 71130206-71130233 |
| MED12 204 | chrX: 71118691-71118718 | MED12 992 | chrX: 71130207-71130234 |
| MED12 205 | chrX: 71118692-71118719 | MED12 993 | chrX: 71130211-71130238 |
| MED12 206 | chrX: 71118693-71118720 | MED12 994 | chrX: 71130213-71130240 |
| MED12 207 | chrX: 71118697-71118724 | MED12 995 | chrX: 71131525-71131552 |
| MED12 208 | chrX: 71118698-71118725 | MED12 996 | chrX: 71131529-71131556 |
| MED12 209 | chrX: 71118700-71118727 | MED12 997 | chrX: 71131537-71131564 |
| MED12 210 | chrX: 71118703-71118730 | MED12 998 | chrX: 71131538-71131565 |
| MED12 211 | chrX: 71118704-71118731 | MED12 999 | chrX: 71131558-71131585 |
| MED12 212 | chrX: 71118705-71118732 | MED12 1000 | chrX: 71131559-71131586 |
| MED12 213 | chrX: 71118706-71118733 | MED12 1001 | chrX: 71131566-71131593 |
| MED12 214 | chrX: 71118709-71118736 | MED12 1002 | chrX: 71131573-71131600 |
| MED12 215 | chrX: 71118714-71118741 | MED12 1003 | chrX: 71131581-71131608 |
| MED12 216 | chrX: 71118717-71118744 | MED12 1004 | chrX: 71131609-71131636 |
| MED12 217 | chrX: 71118718-71118745 | MED12 1005 | chrX: 71131611-71131638 |
| MED12 218 | chrX: 71118719-71118746 | MED12 1006 | chrX: 71131612-71131639 |
| MED12 219 | chrX: 71118720-71118747 | MED12 1007 | chrX: 71131613-71131640 |
| MED12 220 | chrX: 71118722-71118749 | MED12 1008 | chrX: 71132046-71132073 |
| MED12 221 | chrX: 71118723-71118750 | MED12 1009 | chrX: 71132056-71132083 |
| MED12 222 | chrX: 71118730-71118757 | MED12 1010 | chrX: 71132064-71132091 |
| MED12 223 | chrX: 71118731-71118758 | MED12 1011 | chrX: 71132067-71132094 |
| MED12 224 | chrX: 71118733-71118760 | MED12 1012 | chrX: 71132068-71132095 |
| MED12 225 | chrX: 71118734-71118761 | MED12 1013 | chrX: 71132069-71132096 |
| MED12 226 | chrX: 71118741-71118768 | MED12 1014 | chrX: 71132079-71132106 |
| MED12 227 | chrX: 71118742-71118769 | MED12 1015 | chrX: 71132083-71132110 |
| MED12 228 | chrX: 71118762-71118789 | MED12 1016 | chrX: 71132084-71132111 |
| MED12 229 | chrX: 71118763-71118790 | MED12 1017 | chrX: 71132091-71132118 |
| MED12 230 | chrX: 71118775-71118802 | MED12 1018 | chrX: 71132101-71132128 |
| MED12 231 | chrX: 71118781-71118808 | MED12 1019 | chrX: 71132107-71132134 |
| MED12 232 | chrX: 71118785-71118812 | MED12 1020 | chrX: 71132116-71132143 |
| MED12 233 | chrX: 71118786-71118813 | MED12 1021 | chrX: 71132117-71132144 |
| MED12 234 | chrX: 71118787-71118814 | MED12 1022 | chrX: 71132123-71132150 |
| MED12 235 | chrX: 71118791-71118818 | MED12 1023 | chrX: 71132128-71132155 |
| MED12 236 | chrX: 71118792-71118819 | MED12 1024 | chrX: 71132175-71132202 |
| MED12 237 | chrX: 71118793-71118820 | MED12 1025 | chrX: 71132176-71132203 |
| MED12 238 | chrX: 71118803-71118830 | MED12 1026 | chrX: 71132180-71132207 |
| MED12 239 | chrX: 71118809-71118836 | MED12 1027 | chrX: 71132184-71132211 |
| MED12 240 | chrX: 71118815-71118842 | MED12 1028 | chrX: 71132191-71132218 |
| MED12 241 | chrX: 71118818-71118845 | MED12 1029 | chrX: 71132196-71132223 |
| MED12 242 | chrX: 71118819-71118846 | MED12 1030 | chrX: 71132200-71132227 |
| MED12 243 | chrX: 71118824-71118851 | MED12 1031 | chrX: 71132351-71132378 |
| MED12 244 | chrX: 71118827-71118854 | MED12 1032 | chrX: 71132352-71132379 |
| MED12 245 | chrX: 71118829-71118856 | MED12 1033 | chrX: 71132359-71132386 |
| MED12 246 | chrX: 71118830-71118857 | MED12 1034 | chrX: 71132367-71132394 |
| MED12 247 | chrX: 71118838-71118865 | MED12 1035 | chrX: 71132373-71132400 |
| MED12 248 | chrX: 71118839-71118866 | MED12 1036 | chrX: 71132374-71132401 |
| MED12 249 | chrX: 71118840-71118867 | MED12 1037 | chrX: 71132378-71132405 |
| MED12 250 | chrX: 71118843-71118870 | MED12 1038 | chrX: 71132381-71132408 |
| MED12 251 | chrX: 71118844-71118871 | MED12 1039 | chrX: 71132409-71132436 |
| MED12 252 | chrX: 71118845-71118872 | MED12 1040 | chrX: 71132410-71132437 |
| MED12 253 | chrX: 71118851-71118878 | MED12 1041 | chrX: 71132411-71132438 |
| MED12 254 | chrX: 71119346-71119373 | MED12 1042 | chrX: 71132412-71132439 |
| MED12 255 | chrX: 71119356-71119383 | MED12 1043 | chrX: 71132413-71132440 |
| MED12 256 | chrX: 71119358-71119385 | MED12 1044 | chrX: 71132417-71132444 |
| MED12 257 | chrX: 71119363-71119390 | MED12 1045 | chrX: 71132418-71132445 |
| MED12 258 | chrX: 71119367-71119394 | MED12 1046 | chrX: 71132429-71132456 |
| MED12 259 | chrX: 71119377-71119404 | MED12 1047 | chrX: 71132430-71132457 |
| MED12 260 | chrX: 71119386-71119413 | MED12 1048 | chrX: 71132432-71132459 |
| MED12 261 | chrX: 71119404-71119431 | MED12 1049 | chrX: 71132433-71132460 |
| MED12 262 | chrX: 71119405-71119432 | MED12 1050 | chrX: 71132439-71132466 |
| MED12 263 | chrX: 71119406-71119433 | MED12 1051 | chrX: 71132440-71132467 |
| MED12 264 | chrX: 71119414-71119441 | MED12 1052 | chrX: 71132441-71132468 |
| MED12 265 | chrX: 71119416-71119443 | MED12 1053 | chrX: 71132450-71132477 |
| MED12 266 | chrX: 71119418-71119445 | MED12 1054 | chrX: 71132456-71132483 |
| MED12 267 | chrX: 71119449-71119476 | MED12 1055 | chrX: 71132457-71132484 |
| MED12 268 | chrX: 71119451-71119478 | MED12 1056 | chrX: 71132468-71132495 |
| MED12 269 | chrX: 71119469-71119496 | MED12 1057 | chrX: 71132469-71132496 |
| MED12 270 | chrX: 71119473-71119500 | MED12 1058 | chrX: 71132491-71132518 |
| MED12 271 | chrX: 71119476-71119503 | MED12 1059 | chrX: 71132499-71132526 |
| MED12 272 | chrX: 71119659-71119686 | MED12 1060 | chrX: 71132512-71132539 |
| MED12 273 | chrX: 71119660-71119687 | MED12 1061 | chrX: 71132518-71132545 |
| MED12 274 | chrX: 71119679-71119706 | MED12 1062 | chrX: 71132519-71132546 |
| MED12 275 | chrX: 71119680-71119707 | MED12 1063 | chrX: 71132520-71132547 |
| MED12 276 | chrX: 71119693-71119720 | MED12 1064 | chrX: 71132526-71132553 |
| MED12 277 | chrX: 71119723-71119750 | MED12 1065 | chrX: 71132527-71132554 |
| MED12 278 | chrX: 71119730-71119757 | MED12 1066 | chrX: 71132532-71132559 |
| MED12 279 | chrX: 71119735-71119762 | MED12 1067 | chrX: 71132850-71132877 |
| MED12 280 | chrX: 71119736-71119763 | MED12 1068 | chrX: 71132851-71132878 |
| MED12 281 | chrX: 71119740-71119767 | MED12 1069 | chrX: 71132855-71132882 |
| MED12 282 | chrX: 71119752-71119779 | MED12 1070 | chrX: 71132860-71132887 |
| MED12 283 | chrX: 71119761-71119788 | MED12 1071 | chrX: 71132867-71132894 |
| MED12 284 | chrX: 71119762-71119789 | MED12 1072 | chrX: 71132868-71132895 |
| MED12 285 | chrX: 71119763-71119790 | MED12 1073 | chrX: 71132874-71132901 |
| MED12 286 | chrX: 71119767-71119794 | MED12 1074 | chrX: 71132875-71132902 |
| MED12 287 | chrX: 71119772-71119799 | MED12 1075 | chrX: 71132879-71132906 |
| MED12 288 | chrX: 71119802-71119829 | MED12 1076 | chrX: 71132894-71132921 |
| MED12 289 | chrX: 71119807-71119834 | MED12 1077 | chrX: 71132895-71132922 |
| MED12 290 | chrX: 71119808-71119835 | MED12 1078 | chrX: 71132921-71132948 |
| MED12 291 | chrX: 71119815-71119842 | MED12 1079 | chrX: 71132926-71132953 |
| MED12 292 | chrX: 71119816-71119843 | MED12 1080 | chrX: 71132930-71132957 |
| MED12 293 | chrX: 71119819-71119846 | MED12 1081 | chrX: 71132931-71132958 |
| MED12 294 | chrX: 71119849-71119876 | MED12 1082 | chrX: 71132934-71132961 |
| MED12 295 | chrX: 71119851-71119878 | MED12 1083 | chrX: 71132935-71132962 |
| MED12 296 | chrX: 71119854-71119881 | MED12 1084 | chrX: 71132943-71132970 |
| MED12 297 | chrX: 71119856-71119883 | MED12 1085 | chrX: 71132944-71132971 |
| MED12 298 | chrX: 71119870-71119897 | MED12 1086 | chrX: 71132950-71132977 |
| MED12 299 | chrX: 71119877-71119904 | MED12 1087 | chrX: 71132953-71132980 |
| MED12 300 | chrX: 71119987-71120014 | MED12 1088 | chrX: 71132956-71132983 |
| MED12 301 | chrX: 71119988-71120015 | MED12 1089 | chrX: 71133110-71133137 |
| MED12 302 | chrX: 71120006-71120033 | MED12 1090 | chrX: 71133146-71133173 |
| MED12 303 | chrX: 71120008-71120035 | MED12 1091 | chrX: 71133162-71133189 |
| MED12 304 | chrX: 71120010-71120037 | MED12 1092 | chrX: 71133168-71133195 |
| MED12 305 | chrX: 71120014-71120041 | MED12 1093 | chrX: 71133176-71133203 |
| MED12 306 | chrX: 71120016-71120043 | MED12 1094 | chrX: 71133186-71133213 |
| MED12 307 | chrX: 71120017-71120044 | MED12 1095 | chrX: 71133194-71133221 |
| MED12 308 | chrX: 71120018-71120045 | MED12 1096 | chrX: 71133202-71133229 |
| MED12 309 | chrX: 71120021-71120048 | MED12 1097 | chrX: 71133203-71133230 |
| MED12 310 | chrX: 71120022-71120049 | MED12 1098 | chrX: 71133204-71133231 |
| MED12 311 | chrX: 71120055-71120082 | MED12 1099 | chrX: 71133209-71133236 |
| MED12 312 | chrX: 71120056-71120083 | MED12 1100 | chrX: 71134330-71134357 |
| MED12 313 | chrX: 71120057-71120084 | MED12 1101 | chrX: 71134333-71134360 |
| MED12 314 | chrX: 71120064-71120091 | MED12 1102 | chrX: 71134334-71134361 |
| MED12 315 | chrX: 71120071-71120098 | MED12 1103 | chrX: 71134335-71134362 |
| MED12 316 | chrX: 71120087-71120114 | MED12 1104 | chrX: 71134336-71134363 |
| MED12 317 | chrX: 71120105-71120132 | MED12 1105 | chrX: 71134337-71134364 |
| MED12 318 | chrX: 71120111-71120138 | MED12 1106 | chrX: 71134348-71134375 |
| MED12 319 | chrX: 71120113-71120140 | MED12 1107 | chrX: 71134349-71134376 |
| MED12 320 | chrX: 71120135-71120162 | MED12 1108 | chrX: 71134351-71134378 |
| MED12 321 | chrX: 71120143-71120170 | MED12 1109 | chrX: 71134378-71134405 |
| MED12 322 | chrX: 71120144-71120171 | MED12 1110 | chrX: 71134383-71134410 |
| MED12 323 | chrX: 71120160-71120187 | MED12 1111 | chrX: 71134384-71134411 |
| MED12 324 | chrX: 71120161-71120188 | MED12 1112 | chrX: 71134391-71134418 |
| MED12 325 | chrX: 71120165-71120192 | MED12 1113 | chrX: 71134392-71134419 |
| MED12 326 | chrX: 71120948-71120975 | MED12 1114 | chrX: 71134399-71134426 |
| MED12 327 | chrX: 71120949-71120976 | MED12 1115 | chrX: 71134400-71134427 |
| MED12 328 | chrX: 71120956-71120983 | MED12 1116 | chrX: 71134412-71134439 |
| MED12 329 | chrX: 71120957-71120984 | MED12 1117 | chrX: 71134415-71134442 |
| MED12 330 | chrX: 71120964-71120991 | MED12 1118 | chrX: 71134419-71134446 |
| MED12 331 | chrX: 71120967-71120994 | MED12 1119 | chrX: 71134422-71134449 |
| MED12 332 | chrX: 71120975-71121002 | MED12 1120 | chrX: 71134460-71134487 |
| MED12 333 | chrX: 71120976-71121003 | MED12 1121 | chrX: 71134703-71134730 |
| MED12 334 | chrX: 71120989-71121016 | MED12 1122 | chrX: 71134708-71134735 |
| MED12 335 | chrX: 71120994-71121021 | MED12 1123 | chrX: 71134733-71134760 |
| MED12 336 | chrX: 71121008-71121035 | MED12 1124 | chrX: 71134734-71134761 |
| MED12 337 | chrX: 71121013-71121040 | MED12 1125 | chrX: 71134741-71134768 |
| MED12 338 | chrX: 71121014-71121041 | MED12 1126 | chrX: 71134769-71134796 |
| MED12 339 | chrX: 71121022-71121049 | MED12 1127 | chrX: 71134777-71134804 |
| MED12 340 | chrX: 71121028-71121055 | MED12 1128 | chrX: 71134780-71134807 |
| MED12 341 | chrX: 71121029-71121056 | MED12 1129 | chrX: 71134807-71134834 |
| MED12 342 | chrX: 71121030-71121057 | MED12 1130 | chrX: 71134822-71134849 |
| MED12 343 | chrX: 71121031-71121058 | MED12 1131 | chrX: 71134830-71134857 |
| MED12 344 | chrX: 71121032-71121059 | MED12 1132 | chrX: 71134832-71134859 |
| MED12 345 | chrX: 71121036-71121063 | MED12 1133 | chrX: 71134838-71134865 |
| MED12 346 | chrX: 71121037-71121064 | MED12 1134 | chrX: 71134839-71134866 |
| MED12 347 | chrX: 71121042-71121069 | MED12 1135 | chrX: 71134840-71134867 |
| MED12 348 | chrX: 71121049-71121076 | MED12 1136 | chrX: 71134841-71134868 |
| MED12 349 | chrX: 71121050-71121077 | MED12 1137 | chrX: 71134846-71134873 |
| MED12 350 | chrX: 71121067-71121094 | MED12 1138 | chrX: 71135068-71135095 |
| MED12 351 | chrX: 71121072-71121099 | MED12 1139 | chrX: 71135071-71135098 |
| MED12 352 | chrX: 71121075-71121102 | MED12 1140 | chrX: 71135072-71135099 |
| MED12 353 | chrX: 71121078-71121105 | MED12 1141 | chrX: 71135074-71135101 |
| MED12 354 | chrX: 71121079-71121106 | MED12 1142 | chrX: 71135075-71135102 |
| MED12 355 | chrX: 71121083-71121110 | MED12 1143 | chrX: 71135076-71135103 |
| MED12 356 | chrX: 71121084-71121111 | MED12 1144 | chrX: 71135077-71135104 |
| MED12 357 | chrX: 71121085-71121112 | MED12 1145 | chrX: 71135079-71135106 |
| MED12 358 | chrX: 71121086-71121113 | MED12 1146 | chrX: 71135085-71135112 |
| MED12 359 | chrX: 71121089-71121116 | MED12 1147 | chrX: 71135098-71135125 |
| MED12 360 | chrX: 71121090-71121117 | MED12 1148 | chrX: 71135104-71135131 |
| MED12 361 | chrX: 71121107-71121134 | MED12 1149 | chrX: 71135109-71135136 |
| MED12 362 | chrX: 71121108-71121135 | MED12 1150 | chrX: 71135136-71135163 |
| MED12 363 | chrX: 71121124-71121151 | MED12 1151 | chrX: 71135146-71135173 |
| MED12 364 | chrX: 71121126-71121153 | MED12 1152 | chrX: 71135152-71135179 |
| MED12 365 | chrX: 71121136-71121163 | MED12 1153 | chrX: 71135153-71135180 |
| MED12 366 | chrX: 71121137-71121164 | MED12 1154 | chrX: 71135162-71135189 |
| MED12 367 | chrX: 71121138-71121165 | MED12 1155 | chrX: 71135163-71135190 |
| MED12 368 | chrX: 71121150-71121177 | MED12 1156 | chrX: 71135164-71135191 |
| MED12 369 | chrX: 71121151-71121178 | MED12 1157 | chrX: 71135165-71135192 |
| MED12 370 | chrX: 71121152-71121179 | MED12 1158 | chrX: 71135185-71135212 |
| MED12 371 | chrX: 71121300-71121327 | MED12 1159 | chrX: 71135186-71135213 |
| MED12 372 | chrX: 71121301-71121328 | MED12 1160 | chrX: 71135195-71135222 |
| MED12 373 | chrX: 71121304-71121331 | MED12 1161 | chrX: 71135196-71135223 |
| MED12 374 | chrX: 71121307-71121334 | MED12 1162 | chrX: 71135201-71135228 |
| MED12 375 | chrX: 71121308-71121335 | MED12 1163 | chrX: 71135207-71135234 |
| MED12 376 | chrX: 71121311-71121338 | MED12 1164 | chrX: 71135224-71135251 |
| MED12 377 | chrX: 71121312-71121339 | MED12 1165 | chrX: 71135227-71135254 |
| MED12 378 | chrX: 71121313-71121340 | MED12 1166 | chrX: 71135237-71135264 |
| MED12 379 | chrX: 71121320-71121347 | MED12 1167 | chrX: 71136254-71136281 |
| MED12 380 | chrX: 71121321-71121348 | MED12 1168 | chrX: 71136255-71136282 |
| MED12 381 | chrX: 71121335-71121362 | MED12 1169 | chrX: 71136263-71136290 |
| MED12 382 | chrX: 71121336-71121363 | MED12 1170 | chrX: 71136292-71136319 |
| MED12 383 | chrX: 71121353-71121380 | MED12 1171 | chrX: 71136293-71136320 |
| MED12 384 | chrX: 71121358-71121385 | MED12 1172 | chrX: 71136294-71136321 |
| MED12 385 | chrX: 71121367-71121394 | MED12 1173 | chrX: 71136297-71136324 |
| MED12 386 | chrX: 71121372-71121399 | MED12 1174 | chrX: 71136305-71136332 |
| MED12 387 | chrX: 71121386-71121413 | MED12 1175 | chrX: 71136306-71136333 |
| MED12 388 | chrX: 71121389-71121416 | MED12 1176 | chrX: 71136307-71136334 |
| MED12 389 | chrX: 71121390-71121417 | MED12 1177 | chrX: 71136314-71136341 |
| MED12 390 | chrX: 71121416-71121443 | MED12 1178 | chrX: 71136315-71136342 |
| MED12 391 | chrX: 71121421-71121448 | MED12 1179 | chrX: 71136334-71136361 |
| MED12 392 | chrX: 71121423-71121450 | MED12 1180 | chrX: 71136335-71136362 |
| MED12 393 | chrX: 71121429-71121456 | MED12 1181 | chrX: 71136336-71136363 |
| MED12 394 | chrX: 71121434-71121461 | MED12 1182 | chrX: 71136340-71136367 |
| MED12 395 | chrX: 71121435-71121462 | MED12 1183 | chrX: 71136341-71136368 |
| MED12 396 | chrX: 71121542-71121569 | MED12 1184 | chrX: 71136345-71136372 |
| MED12 397 | chrX: 71121543-71121570 | MED12 1185 | chrX: 71136350-71136377 |
| MED12 398 | chrX: 71121544-71121571 | MED12 1186 | chrX: 71136359-71136386 |
| MED12 399 | chrX: 71121576-71121603 | MED12 1187 | chrX: 71136364-71136391 |
| MED12 400 | chrX: 71121591-71121618 | MED12 1188 | chrX: 71136371-71136398 |
| MED12 401 | chrX: 71121596-71121623 | MED12 1189 | chrX: 71136378-71136405 |
| MED12 402 | chrX: 71121597-71121624 | MED12 1190 | chrX: 71136379-71136406 |
| MED12 403 | chrX: 71121600-71121627 | MED12 1191 | chrX: 71136383-71136410 |
| MED12 404 | chrX: 71121604-71121631 | MED12 1192 | chrX: 71136388-71136415 |
| MED12 405 | chrX: 71121608-71121635 | MED12 1193 | chrX: 71136394-71136421 |
| MED12 406 | chrX: 71121616-71121643 | MED12 1194 | chrX: 71136421-71136448 |
| MED12 407 | chrX: 71121620-71121647 | MED12 1195 | chrX: 71136427-71136454 |
| MED12 408 | chrX: 71121632-71121659 | MED12 1196 | chrX: 71136433-71136460 |
| MED12 409 | chrX: 71121633-71121660 | MED12 1197 | chrX: 71136442-71136469 |
| MED12 410 | chrX: 71121700-71121727 | MED12 1198 | chrX: 71136446-71136473 |
| MED12 411 | chrX: 71121701-71121728 | MED12 1199 | chrX: 71136449-71136476 |
| MED12 412 | chrX: 71121704-71121731 | MED12 1200 | chrX: 71136450-71136477 |
| MED12 413 | chrX: 71121707-71121734 | MED12 1201 | chrX: 71136451-71136478 |
| MED12 414 | chrX: 71121708-71121735 | MED12 1202 | chrX: 71136455-71136482 |
| MED12 415 | chrX: 71121709-71121736 | MED12 1203 | chrX: 71136456-71136483 |
| MED12 416 | chrX: 71121715-71121742 | MED12 1204 | chrX: 71136457-71136484 |
| MED12 417 | chrX: 71121721-71121748 | MED12 1205 | chrX: 71136462-71136489 |
| MED12 418 | chrX: 71121722-71121749 | MED12 1206 | chrX: 71136469-71136496 |
| MED12 419 | chrX: 71121723-71121750 | MED12 1207 | chrX: 71136476-71136503 |
| MED12 420 | chrX: 71121724-71121751 | MED12 1208 | chrX: 71136482-71136509 |
| MED12 421 | chrX: 71121739-71121766 | MED12 1209 | chrX: 71136488-71136515 |
| MED12 422 | chrX: 71121740-71121767 | MED12 1210 | chrX: 71136489-71136516 |
| MED12 423 | chrX: 71121748-71121775 | MED12 1211 | chrX: 71136490-71136517 |
| MED12 424 | chrX: 71121749-71121776 | MED12 1212 | chrX: 71136491-71136518 |
| MED12 425 | chrX: 71121756-71121783 | MED12 1213 | chrX: 71136506-71136533 |
| MED12 426 | chrX: 71121759-71121786 | MED12 1214 | chrX: 71136509-71136536 |
| MED12 427 | chrX: 71121763-71121790 | MED12 1215 | chrX: 71136515-71136542 |
| MED12 428 | chrX: 71121770-71121797 | MED12 1216 | chrX: 71136519-71136546 |
| MED12 429 | chrX: 71121771-71121798 | MED12 1217 | chrX: 71136520-71136547 |
| MED12 430 | chrX: 71121772-71121799 | MED12 1218 | chrX: 71136530-71136557 |
| MED12 431 | chrX: 71121780-71121807 | MED12 1219 | chrX: 71136545-71136572 |
| MED12 432 | chrX: 71121784-71121811 | MED12 1220 | chrX: 71136546-71136573 |
| MED12 433 | chrX: 71121785-71121812 | MED12 1221 | chrX: 71136547-71136574 |
| MED12 434 | chrX: 71121786-71121813 | MED12 1222 | chrX: 71136548-71136575 |
| MED12 435 | chrX: 71121790-71121817 | MED12 1223 | chrX: 71136551-71136578 |
| MED12 436 | chrX: 71121794-71121821 | MED12 1224 | chrX: 71136552-71136579 |
| MED12 437 | chrX: 71121798-71121825 | MED12 1225 | chrX: 71136553-71136580 |
| MED12 438 | chrX: 71121801-71121828 | MED12 1226 | chrX: 71136554-71136581 |
| MED12 439 | chrX: 71121804-71121831 | MED12 1227 | chrX: 71136555-71136582 |
| MED12 440 | chrX: 71121805-71121832 | MED12 1228 | chrX: 71136569-71136596 |
| MED12 441 | chrX: 71121810-71121837 | MED12 1229 | chrX: 71136581-71136608 |
| MED12 442 | chrX: 71121812-71121839 | MED12 1230 | chrX: 71136584-71136611 |
| MED12 443 | chrX: 71122201-71122228 | MED12 1231 | chrX: 71136585-71136612 |
| MED12 444 | chrX: 71122203-71122230 | MED12 1232 | chrX: 71136593-71136620 |
| MED12 445 | chrX: 71122205-71122232 | MED12 1233 | chrX: 71136594-71136621 |
| MED12 446 | chrX: 71122208-71122235 | MED12 1234 | chrX: 71136612-71136639 |
| MED12 447 | chrX: 71122218-71122245 | MED12 1235 | chrX: 71136615-71136642 |
| MED12 448 | chrX: 71122225-71122252 | MED12 1236 | chrX: 71136629-71136656 |
| MED12 449 | chrX: 71122243-71122270 | MED12 1237 | chrX: 71136634-71136661 |
| MED12 450 | chrX: 71122267-71122294 | MED12 1238 | chrX: 71136638-71136665 |
| MED12 451 | chrX: 71122275-71122302 | MED12 1239 | chrX: 71136645-71136672 |
| MED12 452 | chrX: 71122283-71122310 | MED12 1240 | chrX: 71136852-71136879 |
| MED12 453 | chrX: 71122290-71122317 | MED12 1241 | chrX: 71136854-71136881 |
| MED12 454 | chrX: 71122296-71122323 | MED12 1242 | chrX: 71136855-71136882 |
| MED12 455 | chrX: 71122297-71122324 | MED12 1243 | chrX: 71136859-71136886 |
| MED12 456 | chrX: 71122298-71122325 | MED12 1244 | chrX: 71136861-71136888 |
| MED12 457 | chrX: 71122299-71122326 | MED12 1245 | chrX: 71136864-71136891 |
| MED12 458 | chrX: 71122303-71122330 | MED12 1246 | chrX: 71136865-71136892 |
| MED12 459 | chrX: 71122307-71122334 | MED12 1247 | chrX: 71136867-71136894 |
| MED12 460 | chrX: 71122311-71122338 | MED12 1248 | chrX: 71136870-71136897 |
| MED12 461 | chrX: 71122312-71122339 | MED12 1249 | chrX: 71136871-71136898 |
| MED12 462 | chrX: 71122317-71122344 | MED12 1250 | chrX: 71136875-71136902 |
| MED12 463 | chrX: 71122320-71122347 | MED12 1251 | chrX: 71136880-71136907 |
| MED12 464 | chrX: 71122333-71122360 | MED12 1252 | chrX: 71136889-71136916 |
| MED12 465 | chrX: 71122341-71122368 | MED12 1253 | chrX: 71136893-71136920 |
| MED12 466 | chrX: 71122481-71122508 | MED12 1254 | chrX: 71136894-71136921 |
| MED12 467 | chrX: 71122487-71122514 | MED12 1255 | chrX: 71136906-71136933 |
| MED12 468 | chrX: 71122491-71122518 | MED12 1256 | chrX: 71136908-71136935 |
| MED12 469 | chrX: 71122498-71122525 | MED12 1257 | chrX: 71136909-71136936 |
| MED12 470 | chrX: 71122499-71122526 | MED12 1258 | chrX: 71136927-71136954 |
| MED12 471 | chrX: 71122510-71122537 | MED12 1259 | chrX: 71136930-71136957 |
| MED12 472 | chrX: 71122520-71122547 | MED12 1260 | chrX: 71136931-71136958 |
| MED12 473 | chrX: 71122525-71122552 | MED12 1261 | chrX: 71136935-71136962 |
| MED12 474 | chrX: 71122526-71122553 | MED12 1262 | chrX: 71136938-71136965 |
| MED12 475 | chrX: 71122527-71122554 | MED12 1263 | chrX: 71136944-71136971 |
| MED12 476 | chrX: 71122532-71122559 | MED12 1264 | chrX: 71136947-71136974 |
| MED12 477 | chrX: 71122549-71122576 | MED12 1265 | chrX: 71136948-71136975 |
| MED12 478 | chrX: 71122568-71122595 | MED12 1266 | chrX: 71136953-71136980 |
| MED12 479 | chrX: 71122581-71122608 | MED12 1267 | chrX: 71136954-71136981 |
| MED12 480 | chrX: 71122591-71122618 | MED12 1268 | chrX: 71136956-71136983 |
| MED12 481 | chrX: 71122606-71122633 | MED12 1269 | chrX: 71136961-71136988 |
| MED12 482 | chrX: 71122711-71122738 | MED12 1270 | chrX: 71136970-71136997 |
| MED12 483 | chrX: 71122713-71122740 | MED12 1271 | chrX: 71136971-71136998 |
| MED12 484 | chrX: 71122714-71122741 | MED12 1272 | chrX: 71136991-71137018 |
| MED12 485 | chrX: 71122715-71122742 | MED12 1273 | chrX: 71136998-71137025 |
| MED12 486 | chrX: 71122721-71122748 | MED12 1274 | chrX: 71137003-71137030 |
| MED12 487 | chrX: 71122723-71122750 | MED12 1275 | chrX: 71137006-71137033 |
| MED12 488 | chrX: 71122727-71122754 | MED12 1276 | chrX: 71137007-71137034 |
| MED12 489 | chrX: 71122728-71122755 | MED12 1277 | chrX: 71137013-71137040 |
| MED12 490 | chrX: 71122729-71122756 | MED12 1278 | chrX: 71137017-71137044 |
| MED12 491 | chrX: 71122732-71122759 | MED12 1279 | chrX: 71137021-71137048 |
| MED12 492 | chrX: 71122743-71122770 | MED12 1280 | chrX: 71137023-71137050 |
| MED12 493 | chrX: 71122744-71122771 | MED12 1281 | chrX: 71137029-71137056 |
| MED12 494 | chrX: 71122752-71122779 | MED12 1282 | chrX: 71137160-71137187 |
| MED12 495 | chrX: 71122784-71122811 | MED12 1283 | chrX: 71137163-71137190 |
| MED12 496 | chrX: 71122819-71122846 | MED12 1284 | chrX: 71137170-71137197 |
| MED12 497 | chrX: 71122824-71122851 | MED12 1285 | chrX: 71137171-71137198 |
| MED12 498 | chrX: 71122825-71122852 | MED12 1286 | chrX: 71137174-71137201 |
| MED12 499 | chrX: 71122826-71122853 | MED12 1287 | chrX: 71137178-71137205 |
| MED12 500 | chrX: 71122835-71122862 | MED12 1288 | chrX: 71137191-71137218 |
| MED12 501 | chrX: 71122836-71122863 | MED12 1289 | chrX: 71137192-71137219 |
| MED12 502 | chrX: 71122840-71122867 | MED12 1290 | chrX: 71137203-71137230 |
| MED12 503 | chrX: 71122841-71122868 | MED12 1291 | chrX: 71137204-71137231 |
| MED12 504 | chrX: 71122848-71122875 | MED12 1292 | chrX: 71137209-71137236 |
| MED12 505 | chrX: 71122854-71122881 | MED12 1293 | chrX: 71137213-71137240 |
| MED12 506 | chrX: 71122860-71122887 | MED12 1294 | chrX: 71137225-71137252 |
| MED12 507 | chrX: 71122861-71122888 | MED12 1295 | chrX: 71137235-71137262 |
| MED12 508 | chrX: 71122862-71122889 | MED12 1296 | chrX: 71137240-71137267 |
| MED12 509 | chrX: 71122871-71122898 | MED12 1297 | chrX: 71137241-71137268 |
| MED12 510 | chrX: 71123069-71123096 | MED12 1298 | chrX: 71137244-71137271 |
| MED12 511 | chrX: 71123072-71123099 | MED12 1299 | chrX: 71137245-71137272 |
| MED12 512 | chrX: 71123081-71123108 | MED12 1300 | chrX: 71137249-71137276 |
| MED12 513 | chrX: 71123091-71123118 | MED12 1301 | chrX: 71137257-71137284 |
| MED12 514 | chrX: 71123092-71123119 | MED12 1302 | chrX: 71137258-71137285 |
| MED12 515 | chrX: 71123099-71123126 | MED12 1303 | chrX: 71137259-71137286 |
| MED12 516 | chrX: 71123112-71123139 | MED12 1304 | chrX: 71137267-71137294 |
| MED12 517 | chrX: 71123113-71123140 | MED12 1305 | chrX: 71137268-71137295 |
| MED12 518 | chrX: 71123135-71123162 | MED12 1306 | chrX: 71137270-71137297 |
| MED12 519 | chrX: 71123139-71123166 | MED12 1307 | chrX: 71137271-71137298 |
| MED12 520 | chrX: 71123152-71123179 | MED12 1308 | chrX: 71137277-71137304 |
| MED12 521 | chrX: 71123155-71123182 | MED12 1309 | chrX: 71137303-71137330 |
| MED12 522 | chrX: 71123170-71123197 | MED12 1310 | chrX: 71137304-71137331 |
| MED12 523 | chrX: 71123182-71123209 | MED12 1311 | chrX: 71137305-71137332 |
| MED12 524 | chrX: 71123185-71123212 | MED12 1312 | chrX: 71137307-71137334 |
| MED12 525 | chrX: 71123186-71123213 | MED12 1313 | chrX: 71137319-71137346 |
| MED12 526 | chrX: 71123193-71123220 | MED12 1314 | chrX: 71137321-71137348 |
| MED12 527 | chrX: 71123194-71123221 | MED12 1315 | chrX: 71137329-71137356 |
| MED12 528 | chrX: 71123200-71123227 | MED12 1316 | chrX: 71137331-71137358 |
| MED12 529 | chrX: 71123207-71123234 | MED12 1317 | chrX: 71137342-71137369 |
| MED12 530 | chrX: 71123208-71123235 | MED12 1318 | chrX: 71137351-71137378 |
| MED12 531 | chrX: 71123574-71123601 | MED12 1319 | chrX: 71137352-71137379 |
| MED12 532 | chrX: 71123600-71123627 | MED12 1320 | chrX: 71137353-71137380 |
| MED12 533 | chrX: 71123601-71123628 | MED12 1321 | chrX: 71137357-71137384 |
| MED12 534 | chrX: 71123613-71123640 | MED12 1322 | chrX: 71137365-71137392 |
| MED12 535 | chrX: 71123616-71123643 | MED12 1323 | chrX: 71137371-71137398 |
| MED12 536 | chrX: 71123631-71123658 | MED12 1324 | chrX: 71137373-71137400 |
| MED12 537 | chrX: 71123637-71123664 | MED12 1325 | chrX: 71137374-71137401 |
| MED12 538 | chrX: 71123646-71123673 | MED12 1326 | chrX: 71137375-71137402 |
| MED12 539 | chrX: 71123647-71123674 | MED12 1327 | chrX: 71137380-71137407 |
| MED12 540 | chrX: 71123651-71123678 | MED12 1328 | chrX: 71137382-71137409 |
| MED12 541 | chrX: 71123657-71123684 | MED12 1329 | chrX: 71137383-71137410 |
| MED12 542 | chrX: 71123658-71123685 | MED12 1330 | chrX: 71137386-71137413 |
| MED12 543 | chrX: 71123666-71123693 | MED12 1331 | chrX: 71137387-71137414 |
| MED12 544 | chrX: 71123667-71123694 | MED12 1332 | chrX: 71137540-71137567 |
| MED12 545 | chrX: 71123672-71123699 | MED12 1333 | chrX: 71137541-71137568 |
| MED12 546 | chrX: 71123674-71123701 | MED12 1334 | chrX: 71137549-71137576 |
| MED12 547 | chrX: 71123681-71123708 | MED12 1335 | chrX: 71137550-71137577 |
| MED12 548 | chrX: 71123683-71123710 | MED12 1336 | chrX: 71137551-71137578 |
| MED12 549 | chrX: 71123686-71123713 | MED12 1337 | chrX: 71137590-71137617 |
| MED12 550 | chrX: 71123699-71123726 | MED12 1338 | chrX: 71137592-71137619 |
| MED12 551 | chrX: 71123711-71123738 | MED12 1339 | chrX: 71137603-71137630 |
| MED12 552 | chrX: 71123712-71123739 | MED12 1340 | chrX: 71137604-71137631 |
| MED12 553 | chrX: 71124134-71124161 | MED12 1341 | chrX: 71137609-71137636 |
| MED12 554 | chrX: 71124140-71124167 | MED12 1342 | chrX: 71137613-71137640 |
| MED12 555 | chrX: 71124147-71124174 | MED12 1343 | chrX: 71137620-71137647 |
| MED12 556 | chrX: 71124148-71124175 | MED12 1344 | chrX: 71137621-71137648 |
| MED12 557 | chrX: 71124152-71124179 | MED12 1345 | chrX: 71137631-71137658 |
| MED12 558 | chrX: 71124155-71124182 | MED12 1346 | chrX: 71137632-71137659 |
| MED12 559 | chrX: 71124157-71124184 | MED12 1347 | chrX: 71137635-71137662 |
| MED12 560 | chrX: 71124158-71124185 | MED12 1348 | chrX: 71137636-71137663 |
| MED12 561 | chrX: 71124161-71124188 | MED12 1349 | chrX: 71137637-71137664 |
| MED12 562 | chrX: 71124163-71124190 | MED12 1350 | chrX: 71137638-71137665 |
| MED12 563 | chrX: 71124164-71124191 | MED12 1351 | chrX: 71137643-71137670 |
| MED12 564 | chrX: 71124243-71124270 | MED12 1352 | chrX: 71137644-71137671 |
| MED12 565 | chrX: 71124244-71124271 | MED12 1353 | chrX: 71137667-71137694 |
| MED12 566 | chrX: 71124252-71124279 | MED12 1354 | chrX: 71137695-71137722 |
| MED12 567 | chrX: 71124253-71124280 | MED12 1355 | chrX: 71137696-71137723 |
| MED12 568 | chrX: 71124254-71124281 | MED12 1356 | chrX: 71137699-71137726 |
| MED12 569 | chrX: 71124267-71124294 | MED12 1357 | chrX: 71137700-71137727 |
| MED12 570 | chrX: 71124273-71124300 | MED12 1358 | chrX: 71137703-71137730 |
| MED12 571 | chrX: 71124274-71124301 | MED12 1359 | chrX: 71137704-71137731 |
| MED12 572 | chrX: 71124276-71124303 | MED12 1360 | chrX: 71137711-71137738 |
| MED12 573 | chrX: 71124283-71124310 | MED12 1361 | chrX: 71137718-71137745 |
| MED12 574 | chrX: 71124288-71124315 | MED12 1362 | chrX: 71137719-71137746 |
| MED12 575 | chrX: 71124297-71124324 | MED12 1363 | chrX: 71137722-71137749 |
| MED12 576 | chrX: 71124298-71124325 | MED12 1364 | chrX: 71137726-71137753 |
| MED12 577 | chrX: 71124299-71124326 | MED12 1365 | chrX: 71137727-71137754 |
| MED12 578 | chrX: 71124305-71124332 | MED12 1366 | chrX: 71137735-71137762 |
| MED12 579 | chrX: 71124306-71124333 | MED12 1367 | chrX: 71137745-71137772 |
| MED12 580 | chrX: 71124307-71124334 | MED12 1368 | chrX: 71137754-71137781 |
| MED12 581 | chrX: 71124311-71124338 | MED12 1369 | chrX: 71137762-71137789 |
| MED12 582 | chrX: 71124314-71124341 | MED12 1370 | chrX: 71137773-71137800 |
| MED12 583 | chrX: 71124315-71124342 | MED12 1371 | chrX: 71137774-71137801 |
| MED12 584 | chrX: 71124320-71124347 | MED12 1372 | chrX: 71137784-71137811 |
| MED12 585 | chrX: 71124321-71124348 | MED12 1373 | chrX: 71137792-71137819 |
| MED12 586 | chrX: 71124329-71124356 | MED12 1374 | chrX: 71137793-71137820 |
| MED12 587 | chrX: 71124332-71124359 | MED12 1375 | chrX: 71137794-71137821 |
| MED12 588 | chrX: 71124336-71124363 | MED12 1376 | chrX: 71137795-71137822 |
| MED12 589 | chrX: 71124339-71124366 | MED12 1377 | chrX: 71137796-71137823 |
| MED12 590 | chrX: 71124343-71124370 | MED12 1378 | chrX: 71137805-71137832 |
| MED12 591 | chrX: 71124344-71124371 | MED12 1379 | chrX: 71137809-71137836 |
| MED12 592 | chrX: 71124359-71124386 | MED12 1380 | chrX: 71137813-71137840 |
| MED12 593 | chrX: 71124369-71124396 | MED12 1381 | chrX: 71137818-71137845 |
| MED12 594 | chrX: 71124370-71124397 | MED12 1382 | chrX: 71137826-71137853 |
| MED12 595 | chrX: 71124737-71124764 | MED12 1383 | chrX: 71137827-71137854 |
| MED12 596 | chrX: 71124744-71124771 | MED12 1384 | chrX: 71137830-71137857 |
| MED12 597 | chrX: 71124745-71124772 | MED12 1385 | chrX: 71137831-71137858 |
| MED12 598 | chrX: 71124757-71124784 | MED12 1386 | chrX: 71137838-71137865 |
| MED12 599 | chrX: 71124758-71124785 | MED12 1387 | chrX: 71137843-71137870 |
| MED12 600 | chrX: 71124761-71124788 | MED12 1388 | chrX: 71137854-71137881 |
| MED12 601 | chrX: 71124767-71124794 | MED12 1389 | chrX: 71137858-71137885 |
| MED12 602 | chrX: 71124794-71124821 | MED12 1390 | chrX: 71137862-71137889 |
| MED12 603 | chrX: 71124796-71124823 | MED12 1391 | chrX: 71137863-71137890 |
| MED12 604 | chrX: 71124797-71124824 | MED12 1392 | chrX: 71137866-71137893 |
| MED12 605 | chrX: 71124800-71124827 | MED12 1393 | chrX: 71137869-71137896 |
| MED12 606 | chrX: 71124804-71124831 | MED12 1394 | chrX: 71137879-71137906 |
| MED12 607 | chrX: 71124833-71124860 | MED12 1395 | chrX: 71137882-71137909 |
| MED12 608 | chrX: 71124839-71124866 | MED12 1396 | chrX: 71137883-71137910 |
| MED12 609 | chrX: 71124964-71124991 | MED12 1397 | chrX: 71137892-71137919 |
| MED12 610 | chrX: 71124976-71125003 | MED12 1398 | chrX: 71137898-71137925 |
| MED12 611 | chrX: 71124977-71125004 | MED12 1399 | chrX: 71137899-71137926 |
| MED12 612 | chrX: 71124978-71125005 | MED12 1400 | chrX: 71137907-71137934 |
| MED12 613 | chrX: 71124982-71125009 | MED12 1401 | chrX: 71137908-71137935 |
| MED12 614 | chrX: 71124983-71125010 | MED12 1402 | chrX: 71137909-71137936 |
| MED12 615 | chrX: 71124984-71125011 | MED12 1403 | chrX: 71137910-71137937 |
| MED12 616 | chrX: 71124985-71125012 | MED12 1404 | chrX: 71137913-71137940 |
| MED12 617 | chrX: 71124994-71125021 | MED12 1405 | chrX: 71137917-71137944 |
| MED12 618 | chrX: 71124995-71125022 | MED12 1406 | chrX: 71137931-71137958 |
| MED12 619 | chrX: 71125015-71125042 | MED12 1407 | chrX: 71137932-71137959 |
| MED12 620 | chrX: 71125018-71125045 | MED12 1408 | chrX: 71137934-71137961 |
| MED12 621 | chrX: 71125019-71125046 | MED12 1409 | chrX: 71137941-71137968 |
| MED12 622 | chrX: 71125020-71125047 | MED12 1410 | chrX: 71140608-71140635 |
| MED12 623 | chrX: 71125021-71125048 | MED12 1411 | chrX: 71140611-71140638 |
| MED12 624 | chrX: 71125030-71125057 | MED12 1412 | chrX: 71140618-71140645 |
| MED12 625 | chrX: 71125039-71125066 | MED12 1413 | chrX: 71140619-71140646 |
| MED12 626 | chrX: 71125052-71125079 | MED12 1414 | chrX: 71140644-71140671 |
| MED12 627 | chrX: 71125053-71125080 | MED12 1415 | chrX: 71140663-71140690 |
| MED12 628 | chrX: 71125054-71125081 | MED12 1416 | chrX: 71140664-71140691 |
| MED12 629 | chrX: 71125055-71125082 | MED12 1417 | chrX: 71140672-71140699 |
| MED12 630 | chrX: 71125056-71125083 | MED12 1418 | chrX: 71140673-71140700 |
| MED12 631 | chrX: 71125057-71125084 | MED12 1419 | chrX: 71140676-71140703 |
| MED12 632 | chrX: 71125060-71125087 | MED12 1420 | chrX: 71140681-71140708 |
| MED12 633 | chrX: 71125061-71125088 | MED12 1421 | chrX: 71140684-71140711 |
| MED12 634 | chrX: 71125062-71125089 | MED12 1422 | chrX: 71140685-71140712 |
| MED12 635 | chrX: 71125063-71125090 | MED12 1423 | chrX: 71140694-71140721 |
| MED12 636 | chrX: 71125082-71125109 | MED12 1424 | chrX: 71140695-71140722 |
| MED12 637 | chrX: 71125083-71125110 | MED12 1425 | chrX: 71140704-71140731 |
| MED12 638 | chrX: 71125101-71125128 | MED12 1426 | chrX: 71140716-71140743 |
| MED12 639 | chrX: 71125105-71125132 | MED12 1427 | chrX: 71140727-71140754 |
| MED12 640 | chrX: 71125120-71125147 | MED12 1428 | chrX: 71140731-71140758 |
| MED12 641 | chrX: 71125124-71125151 | MED12 1429 | chrX: 71140735-71140762 |
| MED12 642 | chrX: 71125127-71125154 | MED12 1430 | chrX: 71140803-71140830 |
| MED12 643 | chrX: 71125128-71125155 | MED12 1431 | chrX: 71140821-71140848 |
| MED12 644 | chrX: 71125135-71125162 | MED12 1432 | chrX: 71140827-71140854 |
| MED12 645 | chrX: 71125136-71125163 | MED12 1433 | chrX: 71140830-71140857 |
| MED12 646 | chrX: 71125140-71125167 | MED12 1434 | chrX: 71140831-71140858 |
| MED12 647 | chrX: 71125141-71125168 | MED12 1435 | chrX: 71140836-71140863 |
| MED12 648 | chrX: 71125142-71125169 | MED12 1436 | chrX: 71140842-71140869 |
| MED12 649 | chrX: 71125143-71125170 | MED12 1437 | chrX: 71140843-71140870 |
| MED12 650 | chrX: 71125324-71125351 | MED12 1438 | chrX: 71140851-71140878 |
| MED12 651 | chrX: 71125327-71125354 | MED12 1439 | chrX: 71140854-71140881 |
| MED12 652 | chrX: 71125356-71125383 | MED12 1440 | chrX: 71140855-71140882 |
| MED12 653 | chrX: 71125360-71125387 | MED12 1441 | chrX: 71140861-71140888 |
| MED12 654 | chrX: 71125365-71125392 | MED12 1442 | chrX: 71140862-71140889 |
| MED12 655 | chrX: 71125373-71125400 | MED12 1443 | chrX: 71140870-71140897 |
| MED12 656 | chrX: 71125374-71125401 | MED12 1444 | chrX: 71140871-71140898 |
| MED12 657 | chrX: 71125375-71125402 | MED12 1445 | chrX: 71140874-71140901 |
| MED12 658 | chrX: 71125377-71125404 | MED12 1446 | chrX: 71140882-71140909 |
| MED12 659 | chrX: 71125378-71125405 | MED12 1447 | chrX: 71140884-71140911 |
| MED12 660 | chrX: 71125379-71125406 | MED12 1448 | chrX: 71140892-71140919 |
| MED12 661 | chrX: 71125424-71125451 | MED12 1449 | chrX: 71140893-71140920 |
| MED12 662 | chrX: 71125425-71125452 | MED12 1450 | chrX: 71140921-71140948 |
| MED12 663 | chrX: 71125426-71125453 | MED12 1451 | chrX: 71140922-71140949 |
| MED12 664 | chrX: 71125428-71125455 | MED12 1452 | chrX: 71140933-71140960 |
| MED12 665 | chrX: 71125433-71125460 | MED12 1453 | chrX: 71140949-71140976 |
| MED12 666 | chrX: 71125438-71125465 | MED12 1454 | chrX: 71140953-71140980 |
| MED12 667 | chrX: 71125448-71125475 | MED12 1455 | chrX: 71140956-71140983 |
| MED12 668 | chrX: 71125454-71125481 | MED12 1456 | chrX: 71140963-71140990 |
| MED12 669 | chrX: 71125455-71125482 | MED12 1457 | chrX: 71140964-71140991 |
| MED12 670 | chrX: 71125469-71125496 | MED12 1458 | chrX: 71140969-71140996 |
| MED12 671 | chrX: 71125472-71125499 | MED12 1459 | chrX: 71140981-71141008 |
| MED12 672 | chrX: 71125473-71125500 | MED12 1460 | chrX: 71141004-71141031 |
| MED12 673 | chrX: 71125480-71125507 | MED12 1461 | chrX: 71141005-71141032 |
| MED12 674 | chrX: 71125638-71125665 | MED12 1462 | chrX: 71141006-71141033 |
| MED12 675 | chrX: 71125639-71125666 | MED12 1463 | chrX: 71141009-71141036 |
| MED12 676 | chrX: 71125640-71125667 | MED12 1464 | chrX: 71141010-71141037 |
| MED12 677 | chrX: 71125641-71125668 | MED12 1465 | chrX: 71141013-71141040 |
| MED12 678 | chrX: 71125642-71125669 | MED12 1466 | chrX: 71141014-71141041 |
| MED12 679 | chrX: 71125649-71125676 | MED12 1467 | chrX: 71141015-71141042 |
| MED12 680 | chrX: 71125650-71125677 | MED12 1468 | chrX: 71141016-71141043 |
| MED12 681 | chrX: 71125651-71125678 | MED12 1469 | chrX: 71141017-71141044 |
| MED12 682 | chrX: 71125652-71125679 | MED12 1470 | chrX: 71141027-71141054 |
| MED12 683 | chrX: 71125653-71125680 | MED12 1471 | chrX: 71141041-71141068 |
| MED12 684 | chrX: 71125654-71125681 | MED12 1472 | chrX: 71141042-71141069 |
| MED12 685 | chrX: 71125664-71125691 | MED12 1473 | chrX: 71141056-71141083 |
| MED12 686 | chrX: 71125669-71125696 | MED12 1474 | chrX: 71141072-71141099 |
| MED12 687 | chrX: 71125674-71125701 | MED12 1475 | chrX: 71141073-71141100 |
| MED12 688 | chrX: 71125683-71125710 | MED12 1476 | chrX: 71141084-71141111 |
| MED12 689 | chrX: 71125687-71125714 | MED12 1477 | chrX: 71141085-71141112 |
| MED12 690 | chrX: 71125692-71125719 | MED12 1478 | chrX: 71141089-71141116 |
| MED12 691 | chrX: 71125700-71125727 | MED12 1479 | chrX: 71141124-71141151 |
| MED12 692 | chrX: 71126009-71126036 | MED12 1480 | chrX: 71141125-71141152 |
| MED12 693 | chrX: 71126012-71126039 | MED12 1481 | chrX: 71141126-71141153 |
| MED12 694 | chrX: 71126013-71126040 | MED12 1482 | chrX: 71141133-71141160 |
| MED12 695 | chrX: 71126014-71126041 | MED12 1483 | chrX: 71141134-71141161 |
| MED12 696 | chrX: 71126015-71126042 | MED12 1484 | chrX: 71141137-71141164 |
| MED12 697 | chrX: 71126016-71126043 | MED12 1485 | chrX: 71141138-71141165 |
| MED12 698 | chrX: 71126017-71126044 | MED12 1486 | chrX: 71141145-71141172 |
| MED12 699 | chrX: 71126021-71126048 | MED12 1487 | chrX: 71141151-71141178 |
| MED12 700 | chrX: 71126022-71126049 | MED12 1488 | chrX: 71141152-71141179 |
| MED12 701 | chrX: 71126028-71126055 | MED12 1489 | chrX: 71141153-71141180 |
| MED12 702 | chrX: 71126029-71126056 | MED12 1490 | chrX: 71141157-71141184 |
| MED12 703 | chrX: 71126030-71126057 | MED12 1491 | chrX: 71141158-71141185 |
| MED12 704 | chrX: 71126031-71126058 | MED12 1492 | chrX: 71141164-71141191 |
| MED12 705 | chrX: 71126043-71126070 | MED12 1493 | chrX: 71141165-71141192 |
| MED12 706 | chrX: 71126067-71126094 | MED12 1494 | chrX: 71141188-71141215 |
| MED12 707 | chrX: 71126071-71126098 | MED12 1495 | chrX: 71141191-71141218 |
| MED12 708 | chrX: 71126078-71126105 | MED12 1496 | chrX: 71141192-71141219 |
| MED12 709 | chrX: 71126082-71126109 | MED12 1497 | chrX: 71141200-71141227 |
| MED12 710 | chrX: 71126083-71126110 | MED12 1498 | chrX: 71141305-71141332 |
| MED12 711 | chrX: 71126084-71126111 | MED12 1499 | chrX: 71141308-71141335 |
| MED12 712 | chrX: 71126112-71126139 | MED12 1500 | chrX: 71141310-71141337 |
| MED12 713 | chrX: 71126116-71126143 | MED12 1501 | chrX: 71141338-71141365 |
| MED12 714 | chrX: 71126118-71126145 | MED12 1502 | chrX: 71141341-71141368 |
| MED12 715 | chrX: 71126122-71126149 | MED12 1503 | chrX: 71141342-71141369 |
| MED12 716 | chrX: 71126128-71126155 | MED12 1504 | chrX: 71141343-71141370 |
| MED12 717 | chrX: 71126133-71126160 | MED12 1505 | chrX: 71141344-71141371 |
| MED12 718 | chrX: 71126134-71126161 | MED12 1506 | chrX: 71141347-71141374 |
| MED12 719 | chrX: 71126139-71126166 | MED12 1507 | chrX: 71141348-71141375 |
| MED12 720 | chrX: 71126314-71126341 | MED12 1508 | chrX: 71141349-71141376 |
| MED12 721 | chrX: 71126322-71126349 | MED12 1509 | chrX: 71141353-71141380 |
| MED12 722 | chrX: 71126324-71126351 | MED12 1510 | chrX: 71141354-71141381 |
| MED12 723 | chrX: 71126325-71126352 | MED12 1511 | chrX: 71141355-71141382 |
| MED12 724 | chrX: 71126331-71126358 | MED12 1512 | chrX: 71141360-71141387 |
| MED12 725 | chrX: 71126332-71126359 | MED12 1513 | chrX: 71141361-71141388 |
| MED12 726 | chrX: 71126345-71126372 | MED12 1514 | chrX: 71141365-71141392 |
| MED12 727 | chrX: 71126346-71126373 | MED12 1515 | chrX: 71141366-71141393 |
| MED12 728 | chrX: 71126357-71126384 | MED12 1516 | chrX: 71141367-71141394 |
| MED12 729 | chrX: 71126378-71126405 | MED12 1517 | chrX: 71141368-71141395 |
| MED12 730 | chrX: 71126379-71126406 | MED12 1518 | chrX: 71141862-71141889 |
| MED12 731 | chrX: 71126380-71126407 | MED12 1519 | chrX: 71141868-71141895 |
| MED12 732 | chrX: 71126394-71126421 | MED12 1520 | chrX: 71141869-71141896 |
| MED12 733 | chrX: 71126401-71126428 | MED12 1521 | chrX: 71141870-71141897 |
| MED12 734 | chrX: 71126413-71126440 | MED12 1522 | chrX: 71141875-71141902 |
| MED12 735 | chrX: 71126433-71126460 | MED12 1523 | chrX: 71141878-71141905 |
| MED12 736 | chrX: 71126435-71126462 | MED12 1524 | chrX: 71141879-71141906 |
| MED12 737 | chrX: 71126458-71126485 | MED12 1525 | chrX: 71141885-71141912 |
| MED12 738 | chrX: 71126461-71126488 | MED12 1526 | chrX: 71141891-71141918 |
| MED12 739 | chrX: 71126462-71126489 | MED12 1527 | chrX: 71141908-71141935 |
| MED12 740 | chrX: 71126463-71126490 | MED12 1528 | chrX: 71141909-71141936 |
| MED12 741 | chrX: 71126470-71126497 | MED12 1529 | chrX: 71141910-71141937 |
| MED12 742 | chrX: 71126471-71126498 | MED12 1530 | chrX: 71141915-71141942 |
| MED12 743 | chrX: 71126472-71126499 | MED12 1531 | chrX: 71141939-71141966 |
| MED12 744 | chrX: 71126477-71126504 | MED12 1532 | chrX: 71141961-71141988 |
| MED12 745 | chrX: 71126480-71126507 | MED12 1533 | chrX: 71142149-71142176 |
| MED12 746 | chrX: 71126481-71126508 | MED12 1534 | chrX: 71142150-71142177 |
| MED12 747 | chrX: 71126484-71126511 | MED12 1535 | chrX: 71142178-71142205 |
| MED12 748 | chrX: 71126950-71126977 | MED12 1536 | chrX: 71142179-71142206 |
| MED12 749 | chrX: 71126951-71126978 | MED12 1537 | chrX: 71142181-71142208 |
| MED12 750 | chrX: 71126952-71126979 | MED12 1538 | chrX: 71142183-71142210 |
| MED12 751 | chrX: 71126953-71126980 | MED12 1539 | chrX: 71142189-71142216 |
| MED12 752 | chrX: 71126969-71126996 | MED12 1540 | chrX: 71142190-71142217 |
| MED12 753 | chrX: 71126993-71127020 | MED12 1541 | chrX: 71142196-71142223 |
| MED12 754 | chrX: 71126999-71127026 | MED12 1542 | chrX: 71142200-71142227 |
| MED12 755 | chrX: 71127002-71127029 | MED12 1543 | chrX: 71142203-71142230 |
| MED12 756 | chrX: 71127003-71127030 | MED12 1544 | chrX: 71142220-71142247 |
| MED12 757 | chrX: 71127015-71127042 | MED12 1545 | chrX: 71142223-71142250 |
| MED12 758 | chrX: 71127038-71127065 | MED12 1546 | chrX: 71142226-71142253 |
| MED12 759 | chrX: 71127046-71127073 | MED12 1547 | chrX: 71142254-71142281 |
| MED12 760 | chrX: 71127049-71127076 | MED12 1548 | chrX: 71142255-71142282 |
| MED12 761 | chrX: 71127052-71127079 | MED12 1549 | chrX: 71142256-71142283 |
| MED12 762 | chrX: 71127070-71127097 | MED12 1550 | chrX: 71142259-71142286 |
| MED12 763 | chrX: 71127080-71127107 | MED12 1551 | chrX: 71142260-71142287 |
| MED12 764 | chrX: 71127087-71127114 | MED12 1552 | chrX: 71142264-71142291 |
| MED12 765 | chrX: 71127089-71127116 | MED12 1553 | chrX: 71142265-71142292 |
| MED12 766 | chrX: 71127091-71127118 | MED12 1554 | chrX: 71142275-71142302 |
| MED12 767 | chrX: 71127095-71127122 | MED12 1555 | chrX: 71142276-71142303 |
| MED12 768 | chrX: 71127097-71127124 | MED12 1556 | chrX: 71142281-71142308 |
| MED12 769 | chrX: 71127103-71127130 | MED12 1557 | chrX: 71142282-71142309 |
| MED12 770 | chrX: 71127104-71127131 | MED12 1558 | chrX: 71142285-71142312 |
| MED12 771 | chrX: 71127105-71127132 | MED12 1559 | chrX: 71142286-71142313 |
| MED12 772 | chrX: 71127106-71127133 | MED12 1560 | chrX: 71142287-71142314 |
| MED12 773 | chrX: 71127109-71127136 | MED12 1561 | chrX: 71142299-71142326 |
| MED12 774 | chrX: 71127121-71127148 | MED12 1562 | chrX: 71142300-71142327 |
| MED12 775 | chrX: 71127309-71127336 | MED12 1563 | chrX: 71142315-71142342 |
| MED12 776 | chrX: 71127310-71127337 | MED12 1564 | chrX: 71142316-71142343 |
| MED12 777 | chrX: 71127313-71127340 | MED12 1565 | chrX: 71142319-71142346 |
| MED12 778 | chrX: 71127314-71127341 | MED12 1566 | chrX: 71142320-71142347 |
| MED12 779 | chrX: 71127317-71127344 | MED12 1567 | chrX: 71142321-71142348 |
| MED12 780 | chrX: 71127318-71127345 | MED12 1568 | chrX: 71142330-71142357 |
| MED12 781 | chrX: 71127332-71127359 | MED12 1569 | chrX: 71142342-71142369 |
| MED12 782 | chrX: 71127333-71127360 | MED12 1570 | chrX: 71142356-71142383 |
| MED12 783 | chrX: 71127342-71127369 | MED12 1571 | chrX: 71142357-71142384 |
| MED12 784 | chrX: 71127350-71127377 | MED12 1572 | chrX: 71142409-71142436 |
| MED12 785 | chrX: 71127366-71127393 | MED12 1573 | chrX: 71142410-71142437 |
| MED12 786 | chrX: 71127371-71127398 | MED12 1574 | chrX: 71142411-71142438 |
| MED12 787 | chrX: 71127380-71127407 | MED12 1575 | chrX: 71142425-71142452 |
| MED12 788 | chrX: 71127399-71127426 | MED12 1576 | chrX: 71142426-71142453 |

**Tabel 2D. Genomic Coordinates of Human TIGIT in the Current Invention**

| Index | Chromosomal coordinate range | Index | Chromosomal coordinate range |
|---|---|---|---|
| TIGIT 1 | chr3: 114292538-114292565 | TIGIT 280 | chr3: 114296328-114296355 |
| TIGIT 2 | chr3: 114292548-114292575 | TIGIT 281 | chr3: 114296336-114296363 |
| TIGIT 3 | chr3: 114292549-114292576 | **TIGIT** 282 | chr3: 114296337-114296364 |
| TIGIT 4 | chr3: 114292553-114292580 | TIGIT 283 | chr3: 114296344-114296371 |
| TIGIT 5 | chr3: 114292561-114292588 | TIGIT 284 | chr3: 114296345-114296372 |
| TIGIT 6 | chr3: 114292566-114292593 | TIGIT 285 | chr3: 114296360-114296387 |
| TIGIT 7 | chr3: 114292567-114292594 | TIGIT 286 | chr3: 114296363-114296390 |
| TIGIT 8 | chr3: 114292571-114292598 | TIGIT 287 | chr3: 114296373-114296400 |
| TIGIT 9 | chr3: 114292576-114292603 | **TIGIT** 288 | chr3: 114296377-114296404 |
| TIGIT 10 | chr3: 114292577-114292604 | TIGIT 289 | chr3: 114296383-114296410 |
| TIGIT 11 | chr3: 114292583-114292610 | TIGIT 290 | chr3: 114296384-114296411 |
| TIGIT 12 | chr3: 114292584-114292611 | TIGIT 291 | chr3: 114296410-114296437 |
| TIGIT 13 | chr3: 114292585-114292612 | TIGIT 292 | chr3: 114296432-114296459 |
| TIGIT 14 | chr3: 114292589-114292616 | TIGIT 293 | chr3: 114296444-114296471 |
| TIGIT 15 | chr3: 114292602-114292629 | TIGIT 294 | chr3: 114296445-114296472 |
| TIGIT 16 | chr3: 114292611-114292638 | TIGIT 295 | chr3: 114296457-114296484 |
| TIGIT 17 | chr3: 114292612-114292639 | TIGIT 296 | chr3: 114296459-114296486 |
| TIGIT 18 | chr3: 114292615-114292642 | TIGIT 297 | chr3: 114296480-114296507 |
| TIGIT 19 | chr3: 114292624-114292651 | TIGIT 298 | chr3: 114296496-114296523 |
| TIGIT 20 | chr3: 114292627-114292654 | TIGIT 299 | chr3: 114296497-114296524 |
| TIGIT 21 | chr3: 114292632-114292659 | TIGIT 300 | chr3: 114296524-114296551 |
| TIGIT 22 | chr3: 114292636-114292663 | TIGIT 301 | chr3: 114296527-114296554 |
| TIGIT 23 | chr3: 114292637-114292664 | TIGIT 302 | chr3: 114296561-114296588 |
| TIGIT 24 | chr3: 114292651-114292678 | TIGIT 303 | chr3: 114296567-114296594 |
| TIGIT 25 | chr3: 114292665-114292692 | TIGIT 304 | chr3: 114296588-114296615 |
| TIGIT 26 | chr3: 114292670-114292697 | TIGIT 305 | chr3: 114296597-114296624 |
| TIGIT 27 | chr3: 114292675-114292702 | TIGIT 306 | chr3: 114296598-114296625 |
| TIGIT 28 | chr3: 114292700-114292727 | TIGIT 307 | chr3: 114296606-114296633 |
| TIGIT 29 | chr3: 114292705-114292732 | TIGIT 308 | chr3: 114296613-114296640 |
| TIGIT 30 | chr3: 114292714-114292741 | TIGIT 309 | chr3: 114296629-114296656 |
| TIGIT 31 | chr3: 114292715-114292742 | TIGIT 310 | chr3: 114299572-114299599 |
| TIGIT 32 | chr3: 114292719-114292746 | TIGIT 311 | chr3: 114299683-114299710 |
| TIGIT 33 | chr3: 114292733-114292760 | TIGIT 312 | chr3: 114299689-114299716 |
| TIGIT 34 | chr3: 114292738-114292765 | TIGIT 313 | chr3: 114307928-114307955 |
| TIGIT 35 | chr3: 114292754-114292781 | TIGIT 314 | chr3: 114307930-114307957 |
| TIGIT 36 | chr3: 114292755-114292782 | TIGIT 315 | chr3: 114307931-114307958 |
| TIGIT 37 | chr3: 114292760-114292787 | TIGIT 316 | chr3: 114307936-114307963 |
| TIGIT 38 | chr3: 114292763-114292790 | TIGIT 317 | chr3: 114307962-114307989 |
| TIGIT 39 | chr3: 114292766-114292793 | TIGIT 318 | chr3: 114307966-114307993 |
| TIGIT 40 | chr3: 114292767-114292794 | TIGIT 319 | chr3: 114307967-114307994 |
| TIGIT 41 | chr3: 114292768-114292795 | TIGIT 320 | chr3: 114307968-114307995 |
| TIGIT 42 | chr3: 114292775-114292802 | TIGIT 321 | chr3: 114307976-114308003 |
| TIGIT 43 | chr3: 114292776-114292803 | TIGIT 322 | chr3: 114307977-114308004 |
| TIGIT 44 | chr3: 114292777-114292804 | TIGIT 323 | chr3: 114307982-114308009 |
| TIGIT 45 | chr3: 114292797-114292824 | TIGIT 324 | chr3: 114307983-114308010 |
| TIGIT 46 | chr3: 114292798-114292825 | TIGIT 325 | chr3: 114307984-114308011 |
| TIGIT 47 | chr3: 114292799-114292826 | TIGIT 326 | chr3: 114307985-114308012 |
| TIGIT 48 | chr3: 114292804-114292831 | TIGIT 327 | chr3: 114307995-114308022 |
| TIGIT 49 | chr3: 114292819-114292846 | TIGIT 328 | chr3: 114307996-114308023 |
| TIGIT 50 | chr3: 114292820-114292847 | TIGIT 329 | chr3: 114307999-114308026 |
| TIGIT 51 | chr3: 114292821-114292848 | TIGIT 330 | chr3: 114308004-114308031 |
| TIGIT 52 | chr3: 114292831-114292858 | TIGIT 331 | chr3: 114308014-114308041 |
| TIGIT 53 | chr3: 114292837-114292864 | TIGIT 332 | chr3: 114308015-114308042 |
| TIGIT 54 | chr3: 114292838-114292865 | TIGIT 333 | chr3: 114308016-114308043 |
| TIGIT 55 | chr3: 114292855-114292882 | TIGIT 334 | chr3: 114308021-114308048 |
| TIGIT 56 | chr3: 114292856-114292883 | TIGIT 335 | chr3: 114308055-114308082 |
| TIGIT 57 | chr3: 114292859-114292886 | TIGIT 336 | chr3: 114308072-114308099 |
| TIGIT 58 | chr3: 114292862-114292889 | TIGIT 337 | chr3: 114308073-114308100 |
| TIGIT 59 | chr3: 114292865-114292892 | TIGIT 338 | chr3: 114308080-114308107 |
| TIGIT 60 | chr3: 114292866-114292893 | TIGIT 339 | chr3: 114308095-114308122 |
| TIGIT 61 | chr3: 114292870-114292897 | TIGIT 340 | chr3: 114308100-114308127 |
| TIGIT 62 | chr3: 114292873-114292900 | TIGIT 341 | chr3: 114308114-114308141 |
| TIGIT 63 | chr3: 114292877-114292904 | TIGIT 342 | chr3: 114308124-114308151 |
| TIGIT 64 | chr3: 114292881-114292908 | TIGIT 343 | chr3: 114308135-114308162 |
| TIGIT 65 | chr3: 114292897-114292924 | TIGIT 344 | chr3: 114308206-114308233 |
| TIGIT 66 | chr3: 114292900-114292927 | TIGIT 345 | chr3: 114308272-114308299 |
| TIGIT 67 | chr3: 114292904-114292931 | TIGIT 346 | chr3: 114308274-114308301 |
| TIGIT 68 | chr3: 114292908-114292935 | TIGIT 347 | chr3: 114308282-114308309 |
| TIGIT 69 | chr3: 114292913-114292940 | TIGIT 348 | chr3: 114308294-114308321 |
| TIGIT 70 | chr3: 114292916-114292943 | TIGIT 349 | chr3: 114308300-114308327 |
| TIGIT 71 | chr3: 114292917-114292944 | TIGIT 350 | chr3: 114308303-114308330 |
| TIGIT 72 | chr3: 114292918-114292945 | TIGIT 351 | chr3: 114308307-114308334 |
| TIGIT 73 | chr3: 114292919-114292946 | TIGIT 352 | chr3: 114308313-114308340 |
| TIGIT 74 | chr3: 114292922-114292949 | TIGIT 353 | chr3: 114308314-114308341 |
| TIGIT 75 | chr3: 114292923-114292950 | TIGIT 354 | chr3: 114308315-114308342 |
| TIGIT 76 | chr3: 114292924-114292951 | TIGIT 355 | chr3: 114308326-114308353 |
| TIGIT 77 | chr3: 114292931-114292958 | TIGIT 356 | chr3: 114308335-114308362 |
| TIGIT 78 | chr3: 114292947-114292974 | TIGIT 357 | chr3: 114308368-114308395 |
| TIGIT 79 | chr3: 114292948-114292975 | TIGIT 358 | chr3: 114308380-114308407 |
| TIGIT 80 | chr3: 114292954-114292981 | TIGIT 359 | chr3: 114308391-114308418 |
| TIGIT 81 | chr3: 114292968-114292995 | TIGIT 360 | chr3: 114308422-114308449 |
| TIGIT 82 | chr3: 114292981-114293008 | TIGIT 361 | chr3: 114308428-114308455 |
| TIGIT 83 | chr3: 114292994-114293021 | TIGIT 362 | chr3: 114308440-114308467 |
| TIGIT 84 | chr3: 114293014-114293041 | TIGIT 363 | chr3: 114308441-114308468 |
| TIGIT 85 | chr3: 114293015-114293042 | TIGIT 364 | chr3: 114308445-114308472 |
| TIGIT 86 | chr3: 114293016-114293043 | TIGIT 365 | chr3: 114308449-114308476 |
| TIGIT 87 | chr3: 114293018-114293045 | TIGIT 366 | chr3: 114308456-114308483 |
| **TIGIT** 88 | chr3: 114293022-114293049 | TIGIT 367 | chr3: 114308457-114308484 |
| TIGIT 89 | chr3: 114293031-114293058 | TIGIT 368 | chr3: 114308469-114308496 |
| TIGIT 90 | chr3: 114293037-114293064 | TIGIT 369 | chr3: 114308473-114308500 |
| TIGIT 91 | chr3: 114293067-114293094 | TIGIT 370 | chr3: 114308514-114308541 |
| TIGIT 92 | chr3: 114293068-114293095 | TIGIT 371 | chr3: 114308518-114308545 |
| TIGIT 93 | chr3: 114293069-114293096 | TIGIT 372 | chr3: 114308541-114308568 |
| TIGIT 94 | chr3: 114293070-114293097 | TIGIT 373 | chr3: 114308545-114308572 |
| TIGIT 95 | chr3: 114293075-114293102 | TIGIT 374 | chr3: 114308562-114308589 |
| TIGIT 96 | chr3: 114293089-114293116 | TIGIT 375 | chr3: 114308577-114308604 |
| TIGIT 97 | chr3: 114293092-114293119 | TIGIT 376 | chr3: 114308578-114308605 |
| TIGIT 98 | chr3: 114293093-114293120 | TIGIT 377 | chr3: 114308579-114308606 |
| TIGIT 99 | chr3: 114293097-114293124 | TIGIT 378 | chr3: 114308585-114308612 |
| TIGIT 100 | chr3: 114293098-114293125 | TIGIT 379 | chr3: 114308596-114308623 |
| TIGIT 101 | chr3: 114293099-114293126 | TIGIT 380 | chr3: 114308604-114308631 |
| TIGIT 102 | chr3: 114293134-114293161 | TIGIT 381 | chr3: 114308635-114308662 |
| TIGIT 103 | chr3: 114293142-114293169 | TIGIT 382 | chr3: 114308636-114308663 |
| TIGIT 104 | chr3: 114293146-114293173 | TIGIT 383 | chr3: 114308637-114308664 |
| TIGIT 105 | chr3: 114293149-114293176 | TIGIT 384 | chr3: 114308638-114308665 |
| TIGIT 106 | chr3: 114293157-114293184 | TIGIT 385 | chr3: 114308639-114308666 |
| TIGIT 107 | chr3: 114293165-114293192 | TIGIT 386 | chr3: 114308659-114308686 |
| TIGIT 108 | chr3: 114293166-114293193 | TIGIT 387 | chr3: 114308672-114308699 |
| TIGIT 109 | chr3: 114293185-114293212 | TIGIT 388 | chr3: 114308680-114308707 |
| TIGIT 110 | chr3: 114293186-114293213 | TIGIT 389 | chr3: 114308693-114308720 |
| TIGIT 111 | chr3: 114293187-114293214 | TIGIT 390 | chr3: 114308701-114308728 |
| TIGIT 112 | chr3: 114293188-114293215 | TIGIT 391 | chr3: 114308718-114308745 |
| TIGIT 113 | chr3: 114293189-114293216 | TIGIT 392 | chr3: 114308719-114308746 |
| TIGIT 114 | chr3: 114293190-114293217 | TIGIT 393 | chr3: 114308720-114308747 |
| TIGIT 115 | chr3: 114293193-114293220 | TIGIT 394 | chr3: 114308721-114308748 |
| TIGIT 116 | chr3: 114293194-114293221 | TIGIT 395 | chr3: 114308725-114308752 |
| TIGIT 117 | chr3: 114293197-114293224 | TIGIT 396 | chr3: 114308732-114308759 |
| TIGIT 118 | chr3: 114293205-114293232 | TIGIT 397 | chr3: 114308738-114308765 |
| TIGIT 119 | chr3: 114293209-114293236 | TIGIT 398 | chr3: 114308757-114308784 |
| TIGIT 120 | chr3: 114293210-114293237 | TIGIT 399 | chr3: 114308762-114308789 |
| TIGIT 121 | chr3: 114293221-114293248 | TIGIT 400 | chr3: 114308763-114308790 |
| TIGIT 122 | chr3: 114293227-114293254 | TIGIT 401 | chr3: 114308773-114308800 |
| TIGIT 123 | chr3: 114293233-114293260 | TIGIT 402 | chr3: 114308774-114308801 |
| TIGIT 124 | chr3: 114293234-114293261 | TIGIT 403 | chr3: 114308775-114308802 |
| TIGIT 125 | chr3: 114293238-114293265 | TIGIT 404 | chr3: 114308784-114308811 |
| TIGIT 126 | chr3: 114293239-114293266 | TIGIT 405 | chr3: 114308785-114308812 |
| TIGIT 127 | chr3: 114293248-114293275 | TIGIT 406 | chr3: 114308793-114308820 |
| TIGIT 128 | chr3: 114293250-114293277 | TIGIT 407 | chr3: 114308794-114308821 |
| TIGIT 129 | chr3: 114293271-114293298 | TIGIT 408 | chr3: 114308795-114308822 |
| TIGIT 130 | chr3: 114293274-114293301 | TIGIT 409 | chr3: 114308797-114308824 |
| TIGIT 131 | chr3: 114293275-114293302 | TIGIT 410 | chr3: 114308803-114308830 |
| TIGIT 132 | chr3: 114293286-114293313 | TIGIT 411 | chr3: 114308816-114308843 |
| TIGIT 133 | chr3: 114293287-114293314 | TIGIT 412 | chr3: 114308817-114308844 |
| TIGIT 134 | chr3: 114293288-114293315 | TIGIT 413 | chr3: 114308827-114308854 |
| TIGIT 135 | chr3: 114293289-114293316 | TIGIT 414 | chr3: 114308837-114308864 |
| TIGIT 136 | chr3: 114293291-114293318 | TIGIT 415 | chr3: 114308860-114308887 |
| TIGIT 137 | chr3: 114293292-114293319 | TIGIT 416 | chr3: 114308866-114308893 |
| TIGIT 138 | chr3: 114293297-114293324 | TIGIT 417 | chr3: 114308874-114308901 |
| TIGIT 139 | chr3: 114293309-114293336 | TIGIT 418 | chr3: 114308875-114308902 |
| TIGIT 140 | chr3: 114293310-114293337 | TIGIT 419 | chr3: 114308876-114308903 |
| TIGIT 141 | chr3: 114293321-114293348 | TIGIT 420 | chr3: 114308888-114308915 |
| TIGIT 142 | chr3: 114293322-114293349 | TIGIT 421 | chr3: 114308889-114308916 |
| TIGIT 143 | chr3: 114293323-114293350 | TIGIT 422 | chr3: 114308896-114308923 |
| TIGIT 144 | chr3: 114293336-114293363 | TIGIT 423 | chr3: 114308905-114308932 |
| TIGIT 145 | chr3: 114293337-114293364 | TIGIT 424 | chr3: 114308906-114308933 |
| TIGIT 146 | chr3: 114293375-114293402 | TIGIT 425 | chr3: 114308910-114308937 |
| TIGIT 147 | chr3: 114293385-114293412 | TIGIT 426 | chr3: 114308950-114308977 |
| TIGIT 148 | chr3: 114293393-114293420 | TIGIT 427 | chr3: 114308966-114308993 |
| TIGIT 149 | chr3: 114293402-114293429 | TIGIT 428 | chr3: 114308972-114308999 |
| TIGIT 150 | chr3: 114293416-114293443 | TIGIT 429 | chr3: 114308983-114309010 |
| TIGIT 151 | chr3: 114293439-114293466 | TIGIT 430 | chr3: 114308994-114309021 |
| TIGIT 152 | chr3: 114293464-114293491 | TIGIT 431 | chr3: 114308996-114309023 |
| TIGIT 153 | chr3: 114293465-114293492 | TIGIT 432 | chr3: 114308997-114309024 |
| TIGIT 154 | chr3: 114293483-114293510 | TIGIT 433 | chr3: 114308998-114309025 |
| TIGIT 155 | chr3: 114293510-114293537 | TIGIT 434 | chr3: 114309001-114309028 |
| TIGIT 156 | chr3: 114293516-114293543 | TIGIT 435 | chr3: 114309007-114309034 |
| TIGIT 157 | chr3: 114293523-114293550 | TIGIT 436 | chr3: 114309008-114309035 |
| TIGIT 158 | chr3: 114293524-114293551 | TIGIT 437 | chr3: 114309011-114309038 |
| TIGIT 159 | chr3: 114293528-114293555 | TIGIT 438 | chr3: 114309029-114309056 |
| TIGIT 160 | chr3: 114293539-114293566 | TIGIT 439 | chr3: 114309030-114309057 |
| TIGIT 161 | chr3: 114293540-114293567 | TIGIT 440 | chr3: 114309031-114309058 |
| TIGIT 162 | chr3: 114293541-114293568 | TIGIT 441 | chr3: 114309047-114309074 |
| TIGIT 163 | chr3: 114293542-114293569 | TIGIT 442 | chr3: 114309048-114309075 |
| TIGIT 164 | chr3: 114293564-114293591 | TIGIT 443 | chr3: 114309050-114309077 |
| TIGIT 165 | chr3: 114293568-114293595 | TIGIT 444 | chr3: 114309051-114309078 |
| TIGIT 166 | chr3: 114293590-114293617 | TIGIT 445 | chr3: 114309060-114309087 |
| TIGIT 167 | chr3: 114293591-114293618 | TIGIT 446 | chr3: 114309079-114309106 |
| TIGIT 168 | chr3: 114293596-114293623 | TIGIT 447 | chr3: 114309089-114309116 |
| TIGIT 169 | chr3: 114293605-114293632 | TIGIT 448 | chr3: 114309116-114309143 |
| TIGIT 170 | chr3: 114293606-114293633 | TIGIT 449 | chr3: 114309129-114309156 |
| TIGIT 171 | chr3: 114293623-114293650 | TIGIT 450 | chr3: 114309136-114309163 |
| TIGIT 172 | chr3: 114293626-114293653 | TIGIT 451 | chr3: 114309137-114309164 |
| TIGIT 173 | chr3: 114293636-114293663 | TIGIT 452 | chr3: 114309143-114309170 |
| TIGIT 174 | chr3: 114293657-114293684 | TIGIT 453 | chr3: 114309144-114309171 |
| TIGIT 175 | chr3: 114293671-114293698 | TIGIT 454 | chr3: 114309156-114309183 |
| TIGIT 176 | chr3: 114293680-114293707 | TIGIT 455 | chr3: 114309157-114309184 |
| TIGIT 177 | chr3: 114293684-114293711 | TIGIT 456 | chr3: 114309158-114309185 |
| TIGIT 178 | chr3: 114293685-114293712 | TIGIT 457 | chr3: 114309165-114309192 |
| TIGIT 179 | chr3: 114293721-114293748 | TIGIT 458 | chr3: 114309166-114309193 |
| TIGIT 180 | chr3: 114293726-114293753 | TIGIT 459 | chr3: 114309167-114309194 |
| TIGIT 181 | chr3: 114293727-114293754 | TIGIT 460 | chr3: 114309168-114309195 |
| TIGIT 182 | chr3: 114293805-114293832 | TIGIT 461 | chr3: 114309169-114309196 |
| TIGIT 183 | chr3: 114293820-114293847 | TIGIT 462 | chr3: 114309173-114309200 |
| TIGIT 184 | chr3: 114293827-114293854 | TIGIT 463 | chr3: 114309174-114309201 |
| TIGIT 185 | chr3: 114293837-114293864 | TIGIT 464 | chr3: 114309178-114309205 |
| TIGIT 186 | chr3: 114293849-114293876 | TIGIT 465 | chr3: 114309185-114309212 |
| TIGIT 187 | chr3: 114293856-114293883 | TIGIT 466 | chr3: 114309186-114309213 |
| TIGIT 188 | chr3: 114293865-114293892 | TIGIT 467 | chr3: 114309229-114309256 |
| TIGIT 189 | chr3: 114293881-114293908 | TIGIT 468 | chr3: 114309251-114309278 |
| TIGIT 190 | chr3: 114293882-114293909 | TIGIT 469 | chr3: 114309252-114309279 |
| TIGIT 191 | chr3: 114293903-114293930 | TIGIT 470 | chr3: 114309257-114309284 |
| TIGIT 192 | chr3: 114293919-114293946 | TIGIT 471 | chr3: 114309258-114309285 |
| TIGIT 193 | chr3: 114293937-114293964 | TIGIT 472 | chr3: 114309259-114309286 |
| TIGIT 194 | chr3: 114293947-114293974 | TIGIT 473 | chr3: 114309263-114309290 |
| TIGIT 195 | chr3: 114293952-114293979 | TIGIT 474 | chr3: 114309264-114309291 |
| TIGIT 196 | chr3: 114293958-114293985 | TIGIT 475 | chr3: 114309265-114309292 |
| TIGIT 197 | chr3: 114293976-114294003 | TIGIT 476 | chr3: 114309266-114309293 |
| TIGIT 198 | chr3: 114293985-114294012 | TIGIT 477 | chr3: 114309301-114309328 |
| TIGIT 199 | chr3: 114293989-114294016 | TIGIT 478 | chr3: 114309302-114309329 |
| TIGIT 200 | chr3: 114294043-114294070 | TIGIT 479 | chr3: 114309306-114309333 |
| TIGIT 201 | chr3: 114294046-114294073 | TIGIT 480 | chr3: 114309347-114309374 |
| TIGIT 202 | chr3: 114294047-114294074 | TIGIT 481 | chr3: 114309352-114309379 |
| TIGIT 203 | chr3: 114294061-114294088 | TIGIT 482 | chr3: 114309356-114309383 |
| TIGIT 204 | chr3: 114294062-114294089 | TIGIT 483 | chr3: 114309357-114309384 |
| TIGIT 205 | chr3: 114294068-114294095 | TIGIT 484 | chr3: 114309358-114309385 |
| TIGIT 206 | chr3: 114294069-114294096 | TIGIT 485 | chr3: 114309359-114309386 |
| TIGIT 207 | chr3: 114294070-114294097 | TIGIT 486 | chr3: 114309361-114309388 |
| TIGIT 208 | chr3: 114294076-114294103 | TIGIT 487 | chr3: 114309365-114309392 |
| TIGIT 209 | chr3: 114294079-114294106 | TIGIT 488 | chr3: 114309374-114309401 |
| TIGIT 210 | chr3: 114294080-114294107 | TIGIT 489 | chr3: 114309394-114309421 |
| TIGIT 211 | chr3: 114294090-114294117 | TIGIT 490 | chr3: 114309395-114309422 |
| TIGIT 212 | chr3: 114294091-114294118 | TIGIT 491 | chr3: 114309401-114309428 |
| TIGIT 213 | chr3: 114294096-114294123 | TIGIT 492 | chr3: 114309423-114309450 |
| TIGIT 214 | chr3: 114294101-114294128 | TIGIT 493 | chr3: 114309426-114309453 |
| TIGIT 215 | chr3: 114294110-114294137 | TIGIT 494 | chr3: 114309443-114309470 |
| TIGIT 216 | chr3: 114294111-114294138 | TIGIT 495 | chr3: 114309485-114309512 |
| TIGIT 217 | chr3: 114294112-114294139 | TIGIT 496 | chr3: 114309486-114309513 |
| TIGIT 218 | chr3: 114294117-114294144 | TIGIT 497 | chr3: 114309487-114309514 |
| TIGIT 219 | chr3: 114295518-114295545 | TIGIT 498 | chr3: 114309489-114309516 |
| TIGIT 220 | chr3: 114295530-114295557 | TIGIT 499 | chr3: 114309496-114309523 |
| TIGIT 221 | chr3: 114295533-114295560 | TIGIT 500 | chr3: 114309533-114309560 |
| TIGIT 222 | chr3: 114295539-114295566 | TIGIT 501 | chr3: 114309555-114309582 |
| TIGIT 223 | chr3: 114295540-114295567 | TIGIT 502 | chr3: 114309573-114309600 |
| TIGIT 224 | chr3: 114295547-114295574 | TIGIT 503 | chr3: 114309574-114309601 |
| TIGIT 225 | chr3: 114295548-114295575 | TIGIT 504 | chr3: 114309583-114309610 |
| TIGIT 226 | chr3: 114295549-114295576 | TIGIT 505 | chr3: 114309593-114309620 |
| TIGIT 227 | chr3: 114295672-114295699 | TIGIT 506 | chr3: 114309624-114309651 |
| TIGIT 228 | chr3: 114295676-114295703 | TIGIT 507 | chr3: 114309631-114309658 |
| TIGIT 229 | chr3: 114295677-114295704 | TIGIT 508 | chr3: 114309639-114309666 |
| TIGIT 230 | chr3: 114295678-114295705 | TIGIT 509 | chr3: 114309645-114309672 |
| TIGIT 231 | chr3: 114295681-114295708 | TIGIT 510 | chr3: 114309667-114309694 |
| TIGIT 232 | chr3: 114295683-114295710 | TIGIT 511 | chr3: 114309711-114309738 |
| TIGIT 233 | chr3: 114295848-114295875 | TIGIT 512 | chr3: 114309736-114309763 |
| TIGIT 234 | chr3: 114295852-114295879 | TIGIT 513 | chr3: 114309765-114309792 |
| TIGIT 235 | chr3: 114295860-114295887 | TIGIT 514 | chr3: 114309766-114309793 |
| TIGIT 236 | chr3: 114295861-114295888 | TIGIT 515 | chr3: 114309784-114309811 |
| TIGIT 237 | chr3: 114295870-114295897 | TIGIT 516 | chr3: 114309787-114309814 |
| TIGIT 238 | chr3: 114295873-114295900 | TIGIT 517 | chr3: 114309790-114309817 |
| TIGIT 239 | chr3: 114295880-114295907 | TIGIT 518 | chr3: 114309801-114309828 |
| TIGIT 240 | chr3: 114295906-114295933 | TIGIT 519 | chr3: 114309807-114309834 |
| TIGIT 241 | chr3: 114295911-114295938 | TIGIT 520 | chr3: 114309811-114309838 |
| TIGIT 242 | chr3: 114295912-114295939 | TIGIT 521 | chr3: 114309823-114309850 |
| TIGIT 243 | chr3: 114295927-114295954 | TIGIT 522 | chr3: 114309824-114309851 |
| TIGIT 244 | chr3: 114295928-114295955 | TIGIT 523 | chr3: 114309845-114309872 |
| TIGIT 245 | chr3: 114295934-114295961 | TIGIT 524 | chr3: 114309852-114309879 |
| TIGIT 246 | chr3: 114295941-114295968 | TIGIT 525 | chr3: 114309853-114309880 |
| TIGIT 247 | chr3: 114295942-114295969 | TIGIT 526 | chr3: 114309875-114309902 |
| TIGIT 248 | chr3: 114295964-114295991 | TIGIT 527 | chr3: 114309876-114309903 |
| TIGIT 249 | chr3: 114295985-114296012 | TIGIT 528 | chr3: 114309880-114309907 |
| TIGIT 250 | chr3: 114295991-114296018 | TIGIT 529 | chr3: 114309897-114309924 |
| TIGIT 251 | chr3: 114296013-114296040 | TIGIT 530 | chr3: 114309898-114309925 |
| TIGIT 252 | chr3: 114296019-114296046 | TIGIT 531 | chr3: 114309918-114309945 |
| TIGIT 253 | chr3: 114296024-114296051 | TIGIT 532 | chr3: 114309919-114309946 |
| TIGIT 254 | chr3: 114296025-114296052 | TIGIT 533 | chr3: 114309924-114309951 |
| TIGIT 255 | chr3: 114296030-114296057 | TIGIT 534 | chr3: 114309926-114309953 |
| TIGIT 256 | chr3: 114296042-114296069 | TIGIT 535 | chr3: 114309936-114309963 |
| TIGIT 257 | chr3: 114296043-114296070 | TIGIT 536 | chr3: 114309937-114309964 |
| TIGIT 258 | chr3: 114296046-114296073 | TIGIT 537 | chr3: 114310015-114310042 |
| TIGIT 259 | chr3: 114296047-114296074 | TIGIT 538 | chr3: 114310022-114310049 |
| TIGIT 260 | chr3: 114296048-114296075 | TIGIT 539 | chr3: 114310025-114310052 |
| TIGIT 261 | chr3: 114296066-114296093 | TIGIT 540 | chr3: 114310030-114310057 |
| TIGIT 262 | chr3: 114296078-114296105 | TIGIT 541 | chr3: 114310035-114310062 |
| TIGIT 263 | chr3: 114296079-114296106 | TIGIT 542 | chr3: 114310053-114310080 |
| TIGIT 264 | chr3: 114296089-114296116 | TIGIT 543 | chr3: 114310071-114310098 |
| TIGIT 265 | chr3: 114296110-114296137 | TIGIT 544 | chr3: 114310074-114310101 |
| TIGIT 266 | chr3: 114296123-114296150 | TIGIT 545 | chr3: 114310082-114310109 |
| TIGIT 267 | chr3: 114296140-114296167 | TIGIT 546 | chr3: 114310089-114310116 |
| TIGIT 268 | chr3: 114296153-114296180 | TIGIT 547 | chr3: 114310091-114310118 |
| TIGIT 269 | chr3: 114296157-114296184 | TIGIT 548 | chr3: 114310092-114310119 |
| TIGIT 270 | chr3: 114296162-114296189 | TIGIT 549 | chr3: 114310093-114310120 |
| TIGIT 271 | chr3: 114296172-114296199 | TIGIT 550 | chr3: 114310099-114310126 |
| TIGIT 272 | chr3: 114296184-114296211 | TIGIT 551 | chr3: 114310106-114310133 |
| TIGIT 273 | chr3: 114296200-114296227 | TIGIT 552 | chr3: 114310126-114310153 |
| TIGIT 274 | chr3: 114296201-114296228 | TIGIT 553 | chr3: 114310155-114310182 |
| TIGIT 275 | chr3: 114296241-114296268 | TIGIT 554 | chr3: 114310198-114310225 |
| TIGIT 276 | chr3: 114296242-114296269 | TIGIT 555 | chr3: 114310230-114310257 |
| TIGIT 277 | chr3: 114296243-114296270 | TIGIT 556 | chr3: 114310239-114310266 |
| TIGIT 278 | chr3: 114296281-114296308 | TIGIT 557 | chr3: 114310240-114310267 |
| TIGIT 279 | chr3: 114296295-114296322 | | |

**Tabel 2E. Genomic Coordinates of Human BRD4 in the Current Invention**

| Index | Chromosomal coordinate range | Index | Chromosomal coordinate range |
|---|---|---|---|
| BRD4 1 | chr19: 15235582-15235609 | BRD4 1324 | chr19: 15248999-15249026 |
| BRD4 2 | chr19: 15235614-15235641 | BRD4 1325 | chr19: 15249003-15249030 |
| BRD4 3 | chr19: 15235615-15235642 | BRD4 1326 | chr19: 15249004-15249031 |
| BRD4 4 | chr19: 15235647-15235674 | BRD4 1327 | chr19: 15249005-15249032 |
| BRD4 5 | chr19: 15235675-15235702 | BRD4 1328 | chr19: 15249012-15249039 |
| BRD4 6 | chr19: 15235676-15235703 | BRD4 1329 | chr19: 15249013-15249040 |
| BRD4 7 | chr19: 15235677-15235704 | BRD4 1330 | chr19: 15249031-15249058 |
| BRD4 8 | chr19: 15235678-15235705 | BRD4 1331 | chr19: 15249035-15249062 |
| BRD4 9 | chr19: 15235679-15235706 | BRD4 1332 | chr19: 15249036-15249063 |
| BRD4 10 | chr19: 15235684-15235711 | BRD4 1333 | chr19: 15249037-15249064 |
| BRD4 11 | chr19: 15235702-15235729 | BRD4 1334 | chr19: 15249040-15249067 |
| BRD4 12 | chr19: 15235708-15235735 | BRD4 1335 | chr19: 15249044-15249071 |
| BRD4 13 | chr19: 15235717-15235744 | BRD4 1336 | chr19: 15249049-15249076 |
| BRD4 14 | chr19: 15235730-15235757 | BRD4 1337 | chr19: 15249053-15249080 |
| BRD4 15 | chr19: 15235731-15235758 | BRD4 1338 | chr19: 15249059-15249086 |
| BRD4 16 | chr19: 15235735-15235762 | BRD4 1339 | chr19: 15249068-15249095 |
| BRD4 17 | chr19: 15235736-15235763 | BRD4 1340 | chr19: 15249071-15249098 |
| BRD4 18 | chr19: 15235740-15235767 | BRD4 1341 | chr19: 15249072-15249099 |
| BRD4 19 | chr19: 15235741-15235768 | BRD4 1342 | chr19: 15249078-15249105 |
| BRD4 20 | chr19: 15235747-15235774 | BRD4 1343 | chr19: 15249084-15249111 |
| BRD4 21 | chr19: 15235751-15235778 | BRD4 1344 | chr19: 15249088-15249115 |
| **BRD4** 22 | chr19: 15235789-15235816 | BRD4 1345 | chr19: 15249089-15249116 |
| BRD4 23 | chr19: 15235794-15235821 | BRD4 1346 | chr19: 15249091-15249118 |
| BRD4 24 | chr19: 15235795-15235822 | BRD4 1347 | chr19: 15249111-15249138 |
| BRD4 25 | chr19: 15235808-15235835 | BRD4 1348 | chr19: 15249112-15249139 |
| BRD4 26 | chr19: 15235809-15235836 | BRD4 1349 | chr19: 15249113-15249140 |
| BRD4 27 | chr19: 15235811-15235838 | BRD4 1350 | chr19: 15249114-15249141 |
| **BRD4** 28 | chr19: 15235812-15235839 | BRD4 1351 | chr19: 15249119-15249146 |
| BRD4 29 | chr19: 15235817-15235844 | BRD4 1352 | chr19: 15249125-15249152 |
| BRD4 30 | chr19: 15235823-15235850 | BRD4 1353 | chr19: 15249126-15249153 |
| BRD4 31 | chr19: 15235827-15235854 | BRD4 1354 | chr19: 15249130-15249157 |
| BRD4 32 | chr19: 15235828-15235855 | BRD4 1355 | chr19: 15249133-15249160 |
| BRD4 33 | chr19: 15235829-15235856 | BRD4 1356 | chr19: 15249134-15249161 |
| BRD4 34 | chr19: 15235850-15235877 | BRD4 1357 | chr19: 15249135-15249162 |
| BRD4 35 | chr19: 15235851-15235878 | BRD4 1358 | chr19: 15249147-15249174 |
| BRD4 36 | chr19: 15235885-15235912 | BRD4 1359 | chr19: 15249148-15249175 |
| BRD4 37 | chr19: 15235892-15235919 | BRD4 1360 | chr19: 15249152-15249179 |
| BRD4 38 | chr19: 15235896-15235923 | BRD4 1361 | chr19: 15249166-15249193 |
| BRD4 39 | chr19: 15235897-15235924 | BRD4 1362 | chr19: 15249171-15249198 |
| BRD4 40 | chr19: 15235898-15235925 | BRD4 1363 | chr19: 15249181-15249208 |
| BRD4 41 | chrl9: 15235914-15235941 | BRD4 1364 | chr19: 15249183-15249210 |
| BRD4 42 | chr19: 15235915-15235942 | BRD4 1365 | chr19: 15249188-15249215 |
| BRD4 43 | chr19: 15235919-15235946 | BRD4 1366 | chr19: 15249189-15249216 |
| BRD4 44 | chr19: 15235934-15235961 | BRD4 1367 | chr19: 15249202-15249229 |
| BRD4 45 | chr19: 15235935-15235962 | BRD4 1368 | chr19: 15249203-15249230 |
| BRD4 46 | chr19: 15235938-15235965 | BRD4 1369 | chr19: 15249207-15249234 |
| BRD4 47 | chr19: 15235939-15235966 | BRD4 1370 | chr19: 15249214-15249241 |
| BRD4 48 | chr19: 15235948-15235975 | BRD4 1371 | chr19: 15249251-15249278 |
| BRD4 49 | chr19: 15235982-15236009 | BRD4 1372 | chr19: 15249257-15249284 |
| BRD4 50 | chr19: 15235983-15236010 | BRD4 1373 | chr19: 15249261-15249288 |
| BRD4 51 | chr19: 15235984-15236011 | BRD4 1374 | chr19: 15249267-15249294 |
| BRD4 52 | chr19: 15235989-15236016 | BRD4 1375 | chr19: 15249273-15249300 |
| BRD4 53 | chr19: 15235992-15236019 | BRD4 1376 | chr19: 15249274-15249301 |
| BRD4 54 | chr19: 15235997-15236024 | BRD4 1377 | chr19: 15249275-15249302 |
| BRD4 55 | chr19: 15236020-15236047 | BRD4 1378 | chr19: 15249290-15249317 |
| BRD4 56 | chr19: 15236021-15236048 | BRD4 1379 | chr19: 15252973-15253000 |
| BRD4 57 | chr19: 15236026-15236053 | BRD4 1380 | chr19: 15252974-15253001 |
| BRD4 58 | chr19: 15236039-15236066 | BRD4 1381 | chr19: 15253009-15253036 |
| BRD4 59 | chr19: 15236041-15236068 | BRD4 1382 | chr19: 15253010-15253037 |
| BRD4 60 | chr19: 15236042-15236069 | BRD4 1383 | chr19: 15253014-15253041 |
| BRD4 61 | chr19: 15236043-15236070 | BRD4 1384 | chr19: 15253015-15253042 |
| BRD4 62 | chr19: 15236044-15236071 | BRD4 1385 | chr19: 15253020-15253047 |
| BRD4 63 | chr19: 15236045-15236072 | BRD4 1386 | chr19: 15253027-15253054 |
| BRD4 64 | chr19: 15236053-15236080 | BRD4 1387 | chr19: 15253028-15253055 |
| BRD4 65 | chr19: 15236057-15236084 | BRD4 1388 | chr19: 15253031-15253058 |
| BRD4 66 | chr19: 15236074-15236101 | BRD4 1389 | chr19: 15253032-15253059 |
| BRD4 67 | chr19: 15236081-15236108 | BRD4 1390 | chr19: 15253040-15253067 |
| BRD4 68 | chr19: 15236082-15236109 | BRD4 1391 | chr19: 15253048-15253075 |
| BRD4 69 | chr19: 15236085-15236112 | BRD4 1392 | chr19: 15253049-15253076 |
| BRD4 70 | chr19: 15236088-15236115 | BRD4 1393 | chr19: 15253061-15253088 |
| BRD4 71 | chr19: 15236090-15236117 | BRD4 1394 | chr19: 15253074-15253101 |
| BRD4 72 | chr19: 15236094-15236121 | BRD4 1395 | chr19: 15253077-15253104 |
| BRD4 73 | chr19: 15236099-15236126 | BRD4 1396 | chr19: 15253092-15253119 |
| BRD4 74 | chr19: 15236100-15236127 | BRD4 1397 | chr19: 15253093-15253120 |
| BRD4 75 | chrl9: 15236111-15236138 | BRD4 1398 | chr19: 15253104-15253131 |
| BRD4 76 | chrl9: 15236121-15236148 | BRD4 1399 | chr19: 15253105-15253132 |
| BRD4 77 | chr19: 15236123-15236150 | BRD4 1400 | chr19: 15253106-15253133 |
| BRD4 78 | chr19: 15236132-15236159 | BRD4 1401 | chr19: 15253107-15253134 |
| BRD4 79 | chr19: 15236133-15236160 | BRD4 1402 | chr19: 15253108-15253135 |
| BRD4 80 | chrl9: 15236134-15236161 | BRD4 1403 | chr19: 15253111-15253138 |
| BRD4 81 | chr19: 15236140-15236167 | BRD4 1404 | chr19: 15253112-15253139 |
| **BRD4** 82 | chrl9: 15236141-15236168 | BRD4 1405 | chr19: 15253113-15253140 |
| BRD4 83 | chrl9: 15236222-15236249 | BRD4 1406 | chr19: 15253120-15253147 |
| BRD4 84 | chr19: 15236223-15236250 | BRD4 1407 | chr19: 15253121-15253148 |
| BRD4 85 | chr19: 15236224-15236251 | BRD4 1408 | chr19: 15253123-15253150 |
| BRD4 86 | chrl9: 15236234-15236261 | BRD4 1409 | chr19: 15253128-15253155 |
| BRD4 87 | chr19: 15236235-15236262 | BRD4 1410 | chr19: 15253129-15253156 |
| **BRD4** 88 | chrl9: 15236254-15236281 | BRD4 1411 | chr19: 15253136-15253163 |
| BRD4 89 | chr19: 15236258-15236285 | BRD4 1412 | chr19: 15253162-15253189 |
| BRD4 90 | chr19: 15236259-15236286 | BRD4 1413 | chr19: 15253166-15253193 |
| BRD4 91 | chr19: 15236260-15236287 | BRD4 1414 | chr19: 15253178-15253205 |
| BRD4 92 | chr19: 15236268-15236295 | BRD4 1415 | chr19: 15253182-15253209 |
| BRD4 93 | chr19: 15236269-15236296 | BRD4 1416 | chr19: 15253186-15253213 |
| BRD4 94 | chr19: 15236274-15236301 | BRD4 1417 | chr19: 15253191-15253218 |
| BRD4 95 | chr19: 15236279-15236306 | BRD4 1418 | chr19: 15253197-15253224 |
| BRD4 96 | chr19: 15236288-15236315 | BRD4 1419 | chr19: 15253198-15253225 |
| BRD4 97 | chr19: 15236306-15236333 | BRD4 1420 | chr19: 15253199-15253226 |
| BRD4 98 | chr19: 15236307-15236334 | BRD4 1421 | chr19: 15253206-15253233 |
| BRD4 99 | chr19: 15236316-15236343 | BRD4 1422 | chr19: 15253215-15253242 |
| BRD4 100 | chr19: 15236321-15236348 | BRD4 1423 | chr19: 15253216-15253243 |
| BRD4 101 | chr19: 15236324-15236351 | BRD4 1424 | chr19: 15253217-15253244 |
| BRD4 102 | chr19: 15236327-15236354 | BRD4 1425 | chr19: 15253221-15253248 |
| BRD4 103 | chr19: 15236342-15236369 | BRD4 1426 | chr19: 15253228-15253255 |
| BRD4 104 | chr19: 15236343-15236370 | BRD4 1427 | chr19: 15253229-15253256 |
| BRD4 105 | chr19: 15236344-15236371 | BRD4 1428 | chr19: 15253237-15253264 |
| BRD4 106 | chr19: 15236345-15236372 | BRD4 1429 | chr19: 15253238-15253265 |
| BRD4 107 | chr19: 15236346-15236373 | BRD4 1430 | chr19: 15253246-15253273 |
| BRD4 108 | chr19: 15236351-15236378 | BRD4 1431 | chr19: 15253252-15253279 |
| BRD4 109 | chr19: 15236363-15236390 | BRD4 1432 | chr19: 15253257-15253284 |
| BRD4 110 | chr19: 15236364-15236391 | BRD4 1433 | chr19: 15253268-15253295 |
| BRD4 111 | chr19: 15236365-15236392 | BRD4 1434 | chr19: 15253276-15253303 |
| BRD4 112 | chr19: 15236366-15236393 | BRD4 1435 | chr19: 15253277-15253304 |
| BRD4 113 | chr19: 15236381-15236408 | BRD4 1436 | chr19: 15253280-15253307 |
| BRD4 114 | chr19: 15236394-15236421 | BRD4 1437 | chr19: 15253281-15253308 |
| BRD4 115 | chr19: 15236418-15236445 | BRD4 1438 | chr19: 15253286-15253313 |
| BRD4 116 | chr19: 15236421-15236448 | BRD4 1439 | chr19: 15253291-15253318 |
| BRD4 117 | chr19: 15236426-15236453 | BRD4 1440 | chr19: 15253292-15253319 |
| BRD4 118 | chrl9: 15236427-15236454 | BRD4 1441 | chr19: 15253293-15253320 |
| BRD4 119 | chr19: 15236428-15236455 | BRD4 1442 | chr19: 15253299-15253326 |
| BRD4 120 | chr19: 15236434-15236461 | BRD4 1443 | chr19: 15253303-15253330 |
| BRD4 121 | chr19: 15236438-15236465 | BRD4 1444 | chr19: 15253307-15253334 |
| BRD4 122 | chr19: 15236443-15236470 | BRD4 1445 | chr19: 15253317-15253344 |
| BRD4 123 | chr19: 15236446-15236473 | BRD4 1446 | chr19: 15253318-15253345 |
| BRD4 124 | chr19: 15236447-15236474 | BRD4 1447 | chr19: 15253319-15253346 |
| BRD4 125 | chr19: 15236455-15236482 | BRD4 1448 | chr19: 15253322-15253349 |
| BRD4 126 | chr19: 15236469-15236496 | BRD4 1449 | chr19: 15253325-15253352 |
| BRD4 127 | chr19: 15236476-15236503 | BRD4 1450 | chr19: 15253354-15253381 |
| BRD4 128 | chr19: 15236477-15236504 | BRD4 1451 | chr19: 15253361-15253388 |
| BRD4 129 | chr19: 15236478-15236505 | BRD4 1452 | chr19: 15253362-15253389 |
| BRD4 130 | chr19: 15236479-15236506 | BRD4 1453 | chr19: 15253363-15253390 |
| BRD4 131 | chr19: 15236480-15236507 | BRD4 1454 | chr19: 15253368-15253395 |
| BRD4 132 | chr19: 15236483-15236510 | BRD4 1455 | chr19: 15253373-15253400 |
| BRD4 133 | chrl9: 15236484-15236511 | BRD4 1456 | chr19: 15253374-15253401 |
| BRD4 134 | chr19: 15236485-15236512 | BRD4 1457 | chr19: 15253377-15253404 |
| BRD4 135 | chr19: 15236489-15236516 | BRD4 1458 | chr19: 15253384-15253411 |
| BRD4 136 | chr19: 15236490-15236517 | BRD4 1459 | chr19: 15253385-15253412 |
| BRD4 137 | chr19: 15236495-15236522 | BRD4 1460 | chr19: 15253392-15253419 |
| BRD4 138 | chr19: 15236496-15236523 | BRD4 1461 | chr19: 15253397-15253424 |
| BRD4 139 | chr19: 15236497-15236524 | BRD4 1462 | chr19: 15253401-15253428 |
| BRD4 140 | chr19: 15236498-15236525 | BRD4 1463 | chr19: 15253404-15253431 |
| BRD4 141 | chr19: 15236499-15236526 | BRD4 1464 | chr19: 15253407-15253434 |
| BRD4 142 | chr19: 15236504-15236531 | BRD4 1465 | chr19: 15253410-15253437 |
| BRD4 143 | chr19: 15236508-15236535 | BRD4 1466 | chr19: 15253411-15253438 |
| BRD4 144 | chrl9: 15236514-15236541 | BRD4 1467 | chr19: 15253420-15253447 |
| BRD4 145 | chr19: 15236515-15236542 | BRD4 1468 | chr19: 15253421-15253448 |
| BRD4 146 | chr19: 15236518-15236545 | BRD4 1469 | chr19: 15253422-15253449 |
| BRD4 147 | chr19: 15236521-15236548 | BRD4 1470 | chr19: 15253430-15253457 |
| BRD4 148 | chr19: 15236522-15236549 | BRD4 1471 | chr19: 15253431-15253458 |
| BRD4 149 | chr19: 15236523-15236550 | BRD4 1472 | chr19: 15253451-15253478 |
| BRD4 150 | chr19: 15236538-15236565 | BRD4 1473 | chr19: 15253452-15253479 |
| BRD4 151 | chr19: 15236545-15236572 | BRD4 1474 | chr19: 15253453-15253480 |
| BRD4 152 | chr19: 15236550-15236577 | BRD4 1475 | chr19: 15253460-15253487 |
| BRD4 153 | chr19: 15236551-15236578 | BRD4 1476 | chr19: 15253466-15253493 |
| BRD4 154 | chr19: 15236573-15236600 | BRD4 1477 | chr19: 15253467-15253494 |
| BRD4 155 | chr19: 15236584-15236611 | BRD4 1478 | chr19: 15253472-15253499 |
| BRD4 156 | chr19: 15236585-15236612 | BRD4 1479 | chr19: 15253473-15253500 |
| BRD4 157 | chr19: 15236594-15236621 | BRD4 1480 | chr19: 15253488-15253515 |
| BRD4 158 | chr19: 15236595-15236622 | BRD4 1481 | chr19: 15253491-15253518 |
| BRD4 159 | chr19: 15236598-15236625 | BRD4 1482 | chr19: 15253492-15253519 |
| BRD4 160 | chr19: 15236599-15236626 | BRD4 1483 | chr19: 15253507-15253534 |
| BRD4 161 | chr19: 15236601-15236628 | BRD4 1484 | chr19: 15253516-15253543 |
| BRD4 162 | chr19: 15236605-15236632 | BRD4 1485 | chr19: 15253523-15253550 |
| BRD4 163 | chr19: 15236606-15236633 | BRD4 1486 | chr19: 15253524-15253551 |
| BRD4 164 | chr19: 15236607-15236634 | BRD4 1487 | chr19: 15253530-15253557 |
| BRD4 165 | chrl9: 15236614-15236641 | BRD4 1488 | chr19: 15253534-15253561 |
| BRD4 166 | chr19: 15236629-15236656 | BRD4 1489 | chr19: 15253565-15253592 |
| BRD4 167 | chr19: 15236630-15236657 | BRD4 1490 | chr19: 15253566-15253593 |
| BRD4 168 | chr19: 15236631-15236658 | BRD4 1491 | chr19: 15253567-15253594 |
| BRD4 169 | chr19: 15236632-15236659 | BRD4 1492 | chr19: 15253570-15253597 |
| BRD4 170 | chr19: 15236635-15236662 | BRD4 1493 | chr19: 15253571-15253598 |
| BRD4 171 | chr19: 15236654-15236681 | BRD4 1494 | chr19: 15253572-15253599 |
| BRD4 172 | chr19: 15236666-15236693 | BRD4 1495 | chr19: 15253576-15253603 |
| BRD4 173 | chr19: 15236667-15236694 | BRD4 1496 | chr19: 15253582-15253609 |
| BRD4 174 | chr19: 15236675-15236702 | BRD4 1497 | chr19: 15253586-15253613 |
| BRD4 175 | chr19: 15236676-15236703 | BRD4 1498 | chr19: 15253587-15253614 |
| BRD4 176 | chr19: 15236681-15236708 | BRD4 1499 | chr19: 15253588-15253615 |
| BRD4 177 | chr19: 15236684-15236711 | BRD4 1500 | chr19: 15253594-15253621 |
| BRD4 178 | chr19: 15236685-15236712 | BRD4 1501 | chr19: 15253600-15253627 |
| BRD4 179 | chr19: 15236688-15236715 | BRD4 1502 | chr19: 15253604-15253631 |
| BRD4 180 | chr19: 15236689-15236716 | BRD4 1503 | chr19: 15253605-15253632 |
| BRD4 181 | chr19: 15236693-15236720 | BRD4 1504 | chr19: 15253616-15253643 |
| BRD4 182 | chr19: 15236694-15236721 | BRD4 1505 | chr19: 15253617-15253644 |
| BRD4 183 | chr19: 15236695-15236722 | BRD4 1506 | chr19: 15253620-15253647 |
| BRD4 184 | chr19: 15236700-15236727 | BRD4 1507 | chr19: 15253628-15253655 |
| BRD4 185 | chr19: 15236710-15236737 | BRD4 1508 | chr19: 15253632-15253659 |
| BRD4 186 | chr19: 15236730-15236757 | BRD4 1509 | chr19: 15253633-15253660 |
| BRD4 187 | chr19: 15236732-15236759 | BRD4 1510 | chr19: 15253660-15253687 |
| BRD4 188 | chr19: 15236733-15236760 | BRD4 1511 | chr19: 15253676-15253703 |
| BRD4 189 | chr19: 15236738-15236765 | BRD4 1512 | chr19: 15253682-15253709 |
| BRD4 190 | chr19: 15236739-15236766 | BRD4 1513 | chr19: 15253685-15253712 |
| BRD4 191 | chr19: 15236740-15236767 | BRD4 1514 | chr19: 15253689-15253716 |
| BRD4 192 | chr19: 15236746-15236773 | BRD4 1515 | chr19: 15253692-15253719 |
| BRD4 193 | chr19: 15236747-15236774 | BRD4 1516 | chr19: 15253706-15253733 |
| BRD4 194 | chr19: 15236748-15236775 | BRD4 1517 | chr19: 15253707-15253734 |
| BRD4 195 | chr19: 15236749-15236776 | BRD4 1518 | chr19: 15253715-15253742 |
| BRD4 196 | chr19: 15236757-15236784 | BRD4 1519 | chr19: 15253716-15253743 |
| BRD4 197 | chr19: 15236758-15236785 | BRD4 1520 | chr19: 15253717-15253744 |
| BRD4 198 | chr19: 15236769-15236796 | BRD4 1521 | chr19: 15253721-15253748 |
| BRD4 199 | chr19: 15236778-15236805 | BRD4 1522 | chr19: 15253727-15253754 |
| BRD4 200 | chr19: 15236779-15236806 | BRD4 1523 | chr19: 15253728-15253755 |
| BRD4 201 | chr19: 15236780-15236807 | BRD4 1524 | chr19: 15253729-15253756 |
| BRD4 202 | chr19: 15236786-15236813 | BRD4 1525 | chr19: 15253747-15253774 |
| BRD4 203 | chr19: 15236791-15236818 | BRD4 1526 | chr19: 15253749-15253776 |
| BRD4 204 | chr19: 15236798-15236825 | BRD4 1527 | chr19: 15253750-15253777 |
| BRD4 205 | chr19: 15236799-15236826 | BRD4 1528 | chr19: 15253760-15253787 |
| BRD4 206 | chr19: 15236832-15236859 | BRD4 1529 | chr19: 15253761-15253788 |
| BRD4 207 | chr19: 15236863-15236890 | BRD4 1530 | chr19: 15253766-15253793 |
| BRD4 208 | chr19: 15236879-15236906 | BRD4 1531 | chr19: 15253768-15253795 |
| BRD4 209 | chr19: 15236894-15236921 | BRD4 1532 | chr19: 15253777-15253804 |
| BRD4 210 | chr19: 15236906-15236933 | BRD4 1533 | chr19: 15254133-15254160 |
| BRD4 211 | chr19: 15236910-15236937 | BRD4 1534 | chr19: 15254146-15254173 |
| BRD4 212 | chr19: 15236918-15236945 | BRD4 1535 | chr19: 15254151-15254178 |
| BRD4 213 | chr19: 15236923-15236950 | BRD4 1536 | chr19: 15254157-15254184 |
| BRD4 214 | chr19: 15236932-15236959 | BRD4 1537 | chr19: 15254166-15254193 |
| BRD4 215 | chr19: 15236934-15236961 | BRD4 1538 | chr19: 15254184-15254211 |
| BRD4 216 | chr19: 15236942-15236969 | BRD4 1539 | chr19: 15254200-15254227 |
| BRD4 217 | chr19: 15236943-15236970 | BRD4 1540 | chr19: 15254205-15254232 |
| BRD4 218 | chr19: 15236948-15236975 | BRD4 1541 | chr19: 15254208-15254235 |
| BRD4 219 | chr19: 15236952-15236979 | BRD4 1542 | chr19: 15254214-15254241 |
| BRD4 220 | chr19: 15236971-15236998 | BRD4 1543 | chr19: 15254220-15254247 |
| BRD4 221 | chr19: 15236972-15236999 | BRD4 1544 | chr19: 15254221-15254248 |
| **BRD4** 222 | chr19: 15236973-15237000 | BRD4 1545 | chr19: 15254238-15254265 |
| BRD4 223 | chr19: 15236989-15237016 | BRD4 1546 | chr19: 15254253-15254280 |
| BRD4 224 | chr19: 15236994-15237021 | BRD4 1547 | chr19: 15254254-15254281 |
| BRD4 225 | chr19: 15237003-15237030 | BRD4 1548 | chr19: 15254260-15254287 |
| BRD4 226 | chr19: 15237016-15237043 | BRD4 1549 | chr19: 15255270-15255297 |
| BRD4 227 | chr19: 15237017-15237044 | BRD4 1550 | chr19: 15255271-15255298 |
| **BRD4** 228 | chr19: 15237021-15237048 | BRD4 1551 | chr19: 15255272-15255299 |
| BRD4 229 | chr19: 15237026-15237053 | BRD4 1552 | chr19: 15255276-15255303 |
| BRD4 230 | chr19: 15237034-15237061 | BRD4 1553 | chr19: 15255277-15255304 |
| BRD4 231 | chr19: 15237038-15237065 | BRD4 1554 | chr19: 15255278-15255305 |
| BRD4 232 | chr19: 15237039-15237066 | BRD4 1555 | chr19: 15255286-15255313 |
| BRD4 233 | chr19: 15237040-15237067 | BRD4 1556 | chr19: 15255287-15255314 |
| BRD4 234 | chr19: 15237044-15237071 | BRD4 1557 | chr19: 15255296-15255323 |
| BRD4 235 | chr19: 15237045-15237072 | BRD4 1558 | chr19: 15255299-15255326 |
| BRD4 236 | chr19: 15237052-15237079 | BRD4 1559 | chr19: 15255302-15255329 |
| BRD4 237 | chr19: 15237054-15237081 | BRD4 1560 | chr19: 15255307-15255334 |
| BRD4 238 | chr19: 15237056-15237083 | BRD4 1561 | chr19: 15255316-15255343 |
| BRD4 239 | chr19: 15237057-15237084 | BRD4 1562 | chr19: 15255332-15255359 |
| BRD4 240 | chr19: 15237058-15237085 | BRD4 1563 | chr19: 15255336-15255363 |
| BRD4 241 | chr19: 15237075-15237102 | BRD4 1564 | chr19: 15255342-15255369 |
| BRD4 242 | chr19: 15237079-15237106 | BRD4 1565 | chr19: 15255343-15255370 |
| BRD4 243 | chr19: 15237089-15237116 | BRD4 1566 | chr19: 15255344-15255371 |
| BRD4 244 | chr19: 15237090-15237117 | BRD4 1567 | chr19: 15255371-15255398 |
| BRD4 245 | chrl9: 15237094-15237121 | BRD4 1568 | chr19: 15255372-15255399 |
| BRD4 246 | chr19: 15237095-15237122 | BRD4 1569 | chr19: 15255373-15255400 |
| BRD4 247 | chr19: 15237109-15237136 | BRD4 1570 | chr19: 15255377-15255404 |
| BRD4 248 | chr19: 15237110-15237137 | BRD4 1571 | chr19: 15255388-15255415 |
| BRD4 249 | chr19: 15237111-15237138 | BRD4 1572 | chr19: 15255389-15255416 |
| BRD4 250 | chr19: 15237112-15237139 | BRD4 1573 | chr19: 15255392-15255419 |
| BRD4 251 | chr19: 15237114-15237141 | BRD4 1574 | chr19: 15255393-15255420 |
| BRD4 252 | chr19: 15237118-15237145 | BRD4 1575 | chr19: 15255406-15255433 |
| BRD4 253 | chr19: 15237119-15237146 | BRD4 1576 | chr19: 15255423-15255450 |
| BRD4 254 | chr19: 15237125-15237152 | BRD4 1577 | chr19: 15255424-15255451 |
| BRD4 255 | chr19: 15237126-15237153 | BRD4 1578 | chr19: 15255440-15255467 |
| BRD4 256 | chr19: 15237129-15237156 | BRD4 1579 | chr19: 15255441-15255468 |
| BRD4 257 | chr19: 15237135-15237162 | BRD4 1580 | chr19: 15255442-15255469 |
| BRD4 258 | chr19: 15237136-15237163 | BRD4 1581 | chr19: 15255444-15255471 |
| BRD4 259 | chr19: 15237142-15237169 | BRD4 1582 | chr19: 15255452-15255479 |
| BRD4 260 | chr19: 15237143-15237170 | BRD4 1583 | chr19: 15255453-15255480 |
| BRD4 261 | chrl9: 15237147-15237174 | BRD4 1584 | chr19: 15255464-15255491 |
| BRD4 262 | chr19: 15237182-15237209 | BRD4 1585 | chr19: 15255483-15255510 |
| BRD4 263 | chr19: 15237188-15237215 | BRD4 1586 | chr19: 15255485-15255512 |
| BRD4 264 | chrl9: 15237197-15237224 | BRD4 1587 | chr19: 15255492-15255519 |
| BRD4 265 | chrl9: 15237216-15237243 | BRD4 1588 | chr19: 15255496-15255523 |
| BRD4 266 | chrl9: 15237217-15237244 | BRD4 1589 | chr19: 15255504-15255531 |
| BRD4 267 | chr19: 15237220-15237247 | BRD4 1590 | chr19: 15255527-15255554 |
| BRD4 268 | chr19: 15237221-15237248 | BRD4 1591 | chr19: 15255528-15255555 |
| BRD4 269 | chr19: 15237222-15237249 | BRD4 1592 | chr19: 15255531-15255558 |
| BRD4 270 | chr19: 15237223-15237250 | BRD4 1593 | chr19: 15255532-15255559 |
| BRD4 271 | chrl9: 15237224-15237251 | BRD4 1594 | chr19: 15255533-15255560 |
| BRD4 272 | chr19: 15237225-15237252 | BRD4 1595 | chr19: 15255534-15255561 |
| BRD4 273 | chr19: 15237226-15237253 | BRD4 1596 | chr19: 15255537-15255564 |
| BRD4 274 | chrl9: 15237227-15237254 | BRD4 1597 | chr19: 15255540-15255567 |
| BRD4 275 | chr19: 15237228-15237255 | BRD4 1598 | chr19: 15255548-15255575 |
| BRD4 276 | chr19: 15237229-15237256 | BRD4 1599 | chr19: 15255549-15255576 |
| BRD4 277 | chr19: 15237230-15237257 | BRD4 1600 | chr19: 15255555-15255582 |
| BRD4 278 | chr19: 15237231-15237258 | BRD4 1601 | chr19: 15255574-15255601 |
| BRD4 279 | chr19: 15237232-15237259 | BRD4 1602 | chr19: 15255575-15255602 |
| BRD4 280 | chr19: 15237233-15237260 | BRD4 1603 | chr19: 15255585-15255612 |
| BRD4 281 | chr19: 15237235-15237262 | BRD4 1604 | chr19: 15256037-15256064 |
| **BRD4** 282 | chr19: 15237238-15237265 | BRD4 1605 | chr19: 15256038-15256065 |
| BRD4 283 | chr19: 15237239-15237266 | BRD4 1606 | chr19: 15256063-15256090 |
| BRD4 284 | chr19: 15237240-15237267 | BRD4 1607 | chr19: 15256080-15256107 |
| BRD4 285 | chr19: 15237266-15237293 | BRD4 1608 | chr19: 15256083-15256110 |
| BRD4 286 | chr19: 15237317-15237344 | BRD4 1609 | chr19: 15256086-15256113 |
| BRD4 287 | chr19: 15237321-15237348 | BRD4 1610 | chr19: 15256094-15256121 |
| **BRD4** 288 | chr19: 15237351-15237378 | BRD4 1611 | chr19: 15256116-15256143 |
| BRD4 289 | chr19: 15237389-15237416 | BRD4 1612 | chr19: 15256143-15256170 |
| BRD4 290 | chr19: 15237392-15237419 | BRD4 1613 | chr19: 15256149-15256176 |
| BRD4 291 | chr19: 15237408-15237435 | BRD4 1614 | chr19: 15256156-15256183 |
| BRD4 292 | chr19: 15237425-15237452 | BRD4 1615 | chr19: 15256182-15256209 |
| BRD4 293 | chr19: 15237450-15237477 | BRD4 1616 | chr19: 15256195-15256222 |
| BRD4 294 | chrl9: 15237454-15237481 | BRD4 1617 | chr19: 15256196-15256223 |
| BRD4 295 | chr19: 15237476-15237503 | BRD4 1618 | chr19: 15256197-15256224 |
| BRD4 296 | chr19: 15237479-15237506 | BRD4 1619 | chr19: 15256207-15256234 |
| BRD4 297 | chr19: 15237492-15237519 | BRD4 1620 | chr19: 15256208-15256235 |
| BRD4 298 | chr19: 15237493-15237520 | BRD4 1621 | chr19: 15256229-15256256 |
| BRD4 299 | chr19: 15237498-15237525 | BRD4 1622 | chr19: 15256252-15256279 |
| BRD4 300 | chr19: 15237510-15237537 | BRD4 1623 | chr19: 15256946-15256973 |
| BRD4 301 | chr19: 15237518-15237545 | BRD4 1624 | chr19: 15256948-15256975 |
| BRD4 302 | chr19: 15237537-15237564 | BRD4 1625 | chr19: 15256951-15256978 |
| BRD4 303 | chr19: 15237543-15237570 | BRD4 1626 | chr19: 15256957-15256984 |
| BRD4 304 | chr19: 15237546-15237573 | BRD4 1627 | chr19: 15256958-15256985 |
| BRD4 305 | chr19: 15237558-15237585 | BRD4 1628 | chr19: 15256963-15256990 |
| BRD4 306 | chr19: 15237632-15237659 | BRD4 1629 | chr19: 15256964-15256991 |
| BRD4 307 | chr19: 15237635-15237662 | BRD4 1630 | chr19: 15256965-15256992 |
| BRD4 308 | chr19: 15237636-15237663 | BRD4 1631 | chr19: 15256969-15256996 |
| BRD4 309 | chr19: 15237640-15237667 | BRD4 1632 | chr19: 15256981-15257008 |
| BRD4 310 | chr19: 15237652-15237679 | BRD4 1633 | chr19: 15256985-15257012 |
| BRD4 311 | chr19: 15237656-15237683 | BRD4 1634 | chr19: 15256999-15257026 |
| BRD4 312 | chr19: 15237659-15237686 | BRD4 1635 | chr19: 15257002-15257029 |
| BRD4 313 | chr19: 15237672-15237699 | BRD4 1636 | chr19: 15257010-15257037 |
| BRD4 314 | chr19: 15237678-15237705 | BRD4 1637 | chr19: 15257032-15257059 |
| BRD4 315 | chr19: 15237679-15237706 | BRD4 1638 | chr19: 15257034-15257061 |
| BRD4 316 | chr19: 15237687-15237714 | BRD4 1639 | chr19: 15257040-15257067 |
| BRD4 317 | chr19: 15237692-15237719 | BRD4 1640 | chr19: 15257041-15257068 |
| BRD4 318 | chr19: 15237694-15237721 | BRD4 1641 | chr19: 15257044-15257071 |
| BRD4 319 | chr19: 15237695-15237722 | BRD4 1642 | chr19: 15257045-15257072 |
| BRD4 320 | chr19: 15237696-15237723 | BRD4 1643 | chr19: 15257046-15257073 |
| BRD4 321 | chr19: 15237700-15237727 | BRD4 1644 | chr19: 15257058-15257085 |
| BRD4 322 | chr19: 15237710-15237737 | BRD4 1645 | chr19: 15257061-15257088 |
| BRD4 323 | chr19: 15237711-15237738 | BRD4 1646 | chr19: 15257062-15257089 |
| BRD4 324 | chr19: 15237719-15237746 | BRD4 1647 | chr19: 15257065-15257092 |
| BRD4 325 | chr19: 15237727-15237754 | BRD4 1648 | chr19: 15257066-15257093 |
| BRD4 326 | chr19: 15237734-15237761 | BRD4 1649 | chr19: 15257067-15257094 |
| BRD4 327 | chr19: 15237737-15237764 | BRD4 1650 | chr19: 15257068-15257095 |
| BRD4 328 | chr19: 15237741-15237768 | BRD4 1651 | chr19: 15257074-15257101 |
| BRD4 329 | chr19: 15237756-15237783 | BRD4 1652 | chr19: 15257077-15257104 |
| BRD4 330 | chr19: 15237759-15237786 | BRD4 1653 | chr19: 15257078-15257105 |
| BRD4 331 | chr19: 15237772-15237799 | BRD4 1654 | chr19: 15257079-15257106 |
| BRD4 332 | chr19: 15237773-15237800 | BRD4 1655 | chr19: 15257080-15257107 |
| BRD4 333 | chr19: 15237778-15237805 | BRD4 1656 | chr19: 15257082-15257109 |
| BRD4 334 | chr19: 15237792-15237819 | BRD4 1657 | chr19: 15257088-15257115 |
| BRD4 335 | chr19: 15237799-15237826 | BRD4 1658 | chr19: 15257098-15257125 |
| BRD4 336 | chr19: 15237800-15237827 | BRD4 1659 | chr19: 15257101-15257128 |
| BRD4 337 | chr19: 15237801-15237828 | BRD4 1660 | chr19: 15257107-15257134 |
| BRD4 338 | chr19: 15237802-15237829 | BRD4 1661 | chr19: 15257116-15257143 |
| BRD4 339 | chr19: 15237805-15237832 | BRD4 1662 | chr19: 15257119-15257146 |
| BRD4 340 | chr19: 15237806-15237833 | BRD4 1663 | chr19: 15257124-15257151 |
| BRD4 341 | chr19: 15237810-15237837 | BRD4 1664 | chr19: 15257128-15257155 |
| BRD4 342 | chr19: 15237811-15237838 | BRD4 1665 | chr19: 15257140-15257167 |
| BRD4 343 | chr19: 15237812-15237839 | BRD4 1666 | chr19: 15257150-15257177 |
| BRD4 344 | chr19: 15237813-15237840 | BRD4 1667 | chr19: 15257155-15257182 |
| BRD4 345 | chr19: 15237814-15237841 | BRD4 1668 | chr19: 15257156-15257183 |
| BRD4 346 | chr19: 15237825-15237852 | BRD4 1669 | chr19: 15257173-15257200 |
| BRD4 347 | chr19: 15237845-15237872 | BRD4 1670 | chr19: 15263396-15263423 |
| BRD4 348 | chr19: 15237846-15237873 | BRD4 1671 | chr19: 15263404-15263431 |
| BRD4 349 | chr19: 15237847-15237874 | BRD4 1672 | chr19: 15263405-15263432 |
| BRD4 350 | chr19: 15237848-15237875 | BRD4 1673 | chr19: 15263406-15263433 |
| BRD4 351 | chr19: 15237851-15237878 | BRD4 1674 | chr19: 15263408-15263435 |
| BRD4 352 | chr19: 15237852-15237879 | BRD4 1675 | chr19: 15263409-15263436 |
| BRD4 353 | chr19: 15237856-15237883 | BRD4 1676 | chr19: 15263412-15263439 |
| BRD4 354 | chr19: 15237864-15237891 | BRD4 1677 | chr19: 15263414-15263441 |
| BRD4 355 | chr19: 15237880-15237907 | BRD4 1678 | chr19: 15263419-15263446 |
| BRD4 356 | chr19: 15237882-15237909 | BRD4 1679 | chr19: 15263426-15263453 |
| BRD4 357 | chr19: 15237884-15237911 | BRD4 1680 | chr19: 15263431-15263458 |
| BRD4 358 | chr19: 15237889-15237916 | BRD4 1681 | chr19: 15263434-15263461 |
| BRD4 359 | chr19: 15237890-15237917 | BRD4 1682 | chr19: 15263435-15263462 |
| BRD4 360 | chr19: 15237892-15237919 | BRD4 1683 | chr19: 15263440-15263467 |
| BRD4 361 | chr19: 15237893-15237920 | BRD4 1684 | chr19: 15263443-15263470 |
| BRD4 362 | chr19: 15237896-15237923 | BRD4 1685 | chr19: 15263446-15263473 |
| BRD4 363 | chr19: 15237900-15237927 | BRD4 1686 | chr19: 15263454-15263481 |
| BRD4 364 | chr19: 15237901-15237928 | BRD4 1687 | chr19: 15263468-15263495 |
| BRD4 365 | chr19: 15237906-15237933 | BRD4 1688 | chr19: 15263498-15263525 |
| BRD4 366 | chr19: 15237907-15237934 | BRD4 1689 | chr19: 15263505-15263532 |
| BRD4 367 | chr19: 15237910-15237937 | BRD4 1690 | chr19: 15263507-15263534 |
| BRD4 368 | chrl9: 15237914-15237941 | BRD4 1691 | chr19: 15263508-15263535 |
| BRD4 369 | chr19: 15237915-15237942 | BRD4 1692 | chr19: 15263512-15263539 |
| BRD4 370 | chr19: 15237916-15237943 | BRD4 1693 | chr19: 15263525-15263552 |
| BRD4 371 | chr19: 15237920-15237947 | BRD4 1694 | chr19: 15263536-15263563 |
| BRD4 372 | chr19: 15237921-15237948 | BRD4 1695 | chr19: 15263539-15263566 |
| BRD4 373 | chr19: 15237922-15237949 | BRD4 1696 | chr19: 15264377-15264404 |
| BRD4 374 | chr19: 15237923-15237950 | BRD4 1697 | chr19: 15264378-15264405 |
| BRD4 375 | chr19: 15237927-15237954 | BRD4 1698 | chr19: 15264382-15264409 |
| BRD4 376 | chr19: 15237928-15237955 | BRD4 1699 | chr19: 15264383-15264410 |
| BRD4 377 | chr19: 15237929-15237956 | BRD4 1700 | chr19: 15264395-15264422 |
| BRD4 378 | chr19: 15237941-15237968 | BRD4 1701 | chr19: 15264396-15264423 |
| BRD4 379 | chr19: 15237944-15237971 | BRD4 1702 | chr19: 15264399-15264426 |
| BRD4 380 | chr19: 15237945-15237972 | BRD4 1703 | chr19: 15264400-15264427 |
| BRD4 381 | chr19: 15237949-15237976 | BRD4 1704 | chr19: 15264401-15264428 |
| BRD4 382 | chr19: 15237964-15237991 | BRD4 1705 | chr19: 15264403-15264430 |
| BRD4 383 | chr19: 15237965-15237992 | BRD4 1706 | chr19: 15264421-15264448 |
| BRD4 384 | chr19: 15237973-15238000 | BRD4 1707 | chr19: 15264431-15264458 |
| BRD4 385 | chr19: 15237974-15238001 | BRD4 1708 | chr19: 15264454-15264481 |
| BRD4 386 | chr19: 15237975-15238002 | BRD4 1709 | chr19: 15264459-15264486 |
| BRD4 387 | chr19: 15237993-15238020 | BRD4 1710 | chr19: 15264460-15264487 |
| BRD4 388 | chr19: 15237996-15238023 | BRD4 1711 | chr19: 15264465-15264492 |
| BRD4 389 | chr19: 15238011-15238038 | BRD4 1712 | chr19: 15264466-15264493 |
| BRD4 390 | chr19: 15238034-15238061 | BRD4 1713 | chr19: 15264469-15264496 |
| BRD4 391 | chr19: 15238036-15238063 | BRD4 1714 | chr19: 15264471-15264498 |
| BRD4 392 | chr19: 15238037-15238064 | BRD4 1715 | chr19: 15264475-15264502 |
| BRD4 393 | chr19: 15238045-15238072 | BRD4 1716 | chr19: 15264477-15264504 |
| BRD4 394 | chr19: 15238046-15238073 | BRD4 1717 | chr19: 15264480-15264507 |
| BRD4 395 | chr19: 15238064-15238091 | BRD4 1718 | chr19: 15264492-15264519 |
| BRD4 396 | chr19: 15238065-15238092 | BRD4 1719 | chr19: 15264494-15264521 |
| BRD4 397 | chr19: 15238066-15238093 | BRD4 1720 | chr19: 15264509-15264536 |
| BRD4 398 | chr19: 15238073-15238100 | BRD4 1721 | chr19: 15264529-15264556 |
| BRD4 399 | chr19: 15238074-15238101 | BRD4 1722 | chr19: 15264540-15264567 |
| BRD4 400 | chr19: 15238079-15238106 | BRD4 1723 | chr19: 15264559-15264586 |
| BRD4 401 | chr19: 15238080-15238107 | BRD4 1724 | chr19: 15264562-15264589 |
| BRD4 402 | chr19: 15238081-15238108 | BRD4 1725 | chr19: 15264565-15264592 |
| BRD4 403 | chr19: 15238082-15238109 | BRD4 1726 | chr19: 15264566-15264593 |
| BRD4 404 | chr19: 15238089-15238116 | BRD4 1727 | chr19: 15264568-15264595 |
| BRD4 405 | chr19: 15238107-15238134 | BRD4 1728 | chr19: 15264582-15264609 |
| BRD4 406 | chr19: 15238109-15238136 | BRD4 1729 | chr19: 15264583-15264610 |
| BRD4 407 | chr19: 15238113-15238140 | BRD4 1730 | chr19: 15264598-15264625 |
| BRD4 408 | chr19: 15238121-15238148 | BRD4 1731 | chr19: 15264601-15264628 |
| BRD4 409 | chr19: 15238134-15238161 | BRD4 1732 | chr19: 15264610-15264637 |
| BRD4 410 | chr19: 15238135-15238162 | BRD4 1733 | chr19: 15264614-15264641 |
| BRD4 411 | chr19: 15238136-15238163 | BRD4 1734 | chr19: 15264616-15264643 |
| BRD4 412 | chr19: 15238139-15238166 | BRD4 1735 | chr19: 15264632-15264659 |
| BRD4 413 | chr19: 15238142-15238169 | BRD4 1736 | chr19: 15264638-15264665 |
| BRD4 414 | chr19: 15238143-15238170 | BRD4 1737 | chr19: 15264642-15264669 |
| BRD4 415 | chr19: 15238146-15238173 | BRD4 1738 | chr19: 15264645-15264672 |
| BRD4 416 | chr19: 15238149-15238176 | BRD4 1739 | chr19: 15264649-15264676 |
| BRD4 417 | chr19: 15238150-15238177 | BRD4 1740 | chr19: 15264650-15264677 |
| BRD4 418 | chr19: 15238153-15238180 | BRD4 1741 | chr19: 15264651-15264678 |
| BRD4 419 | chr19: 15238166-15238193 | BRD4 1742 | chr19: 15264652-15264679 |
| BRD4 420 | chrl9: 15238174-15238201 | BRD4 1743 | chr19: 15264653-15264680 |
| BRD4 421 | chr19: 15238182-15238209 | BRD4 1744 | chr19: 15264658-15264685 |
| BRD4 422 | chr19: 15238186-15238213 | BRD4 1745 | chr19: 15264659-15264686 |
| BRD4 423 | chr19: 15238194-15238221 | BRD4 1746 | chr19: 15264660-15264687 |
| BRD4 424 | chr19: 15238209-15238236 | BRD4 1747 | chr19: 15264661-15264688 |
| BRD4 425 | chr19: 15238219-15238246 | BRD4 1748 | chr19: 15264669-15264696 |
| BRD4 426 | chr19: 15238220-15238247 | BRD4 1749 | chr19: 15264670-15264697 |
| BRD4 427 | chr19: 15238228-15238255 | BRD4 1750 | chr19: 15264673-15264700 |
| BRD4 428 | chr19: 15238229-15238256 | BRD4 1751 | chr19: 15264682-15264709 |
| BRD4 429 | chr19: 15238233-15238260 | BRD4 1752 | chr19: 15264684-15264711 |
| BRD4 430 | chr19: 15238236-15238263 | BRD4 1753 | chr19: 15264685-15264712 |
| BRD4 431 | chr19: 15238237-15238264 | BRD4 1754 | chr19: 15264686-15264713 |
| BRD4 432 | chr19: 15238241-15238268 | BRD4 1755 | chr19: 15264693-15264720 |
| BRD4 433 | chr19: 15238242-15238269 | BRD4 1756 | chr19: 15264696-15264723 |
| BRD4 434 | chr19: 15238243-15238270 | BRD4 1757 | chr19: 15264699-15264726 |
| BRD4 435 | chr19: 15238250-15238277 | BRD4 1758 | chr19: 15264700-15264727 |
| BRD4 436 | chr19: 15238260-15238287 | BRD4 1759 | chr19: 15264701-15264728 |
| BRD4 437 | chr19: 15238265-15238292 | BRD4 1760 | chr19: 15264702-15264729 |
| BRD4 438 | chr19: 15238270-15238297 | BRD4 1761 | chr19: 15264705-15264732 |
| BRD4 439 | chr19: 15238271-15238298 | BRD4 1762 | chr19: 15264708-15264735 |
| BRD4 440 | chr19: 15238281-15238308 | BRD4 1763 | chr19: 15264746-15264773 |
| BRD4 441 | chr19: 15238282-15238309 | BRD4 1764 | chr19: 15264754-15264781 |
| BRD4 442 | chr19: 15238283-15238310 | BRD4 1765 | chr19: 15264756-15264783 |
| BRD4 443 | chr19: 15238288-15238315 | BRD4 1766 | chr19: 15264757-15264784 |
| BRD4 444 | chr19: 15238296-15238323 | BRD4 1767 | chr19: 15264765-15264792 |
| BRD4 445 | chr19: 15238297-15238324 | BRD4 1768 | chr19: 15265330-15265357 |
| BRD4 446 | chr19: 15238299-15238326 | BRD4 1769 | chr19: 15265331-15265358 |
| BRD4 447 | chr19: 15238300-15238327 | BRD4 1770 | chr19: 15265334-15265361 |
| BRD4 448 | chr19: 15238303-15238330 | BRD4 1771 | chr19: 15265335-15265362 |
| BRD4 449 | chr19: 15238306-15238333 | BRD4 1772 | chr19: 15265341-15265368 |
| BRD4 450 | chr19: 15238309-15238336 | BRD4 1773 | chr19: 15265342-15265369 |
| BRD4 451 | chr19: 15238312-15238339 | BRD4 1774 | chr19: 15265343-15265370 |
| BRD4 452 | chr19: 15238313-15238340 | BRD4 1775 | chr19: 15265346-15265373 |
| BRD4 453 | chr19: 15238314-15238341 | BRD4 1776 | chr19: 15265353-15265380 |
| BRD4 454 | chr19: 15238342-15238369 | BRD4 1777 | chr19: 15265358-15265385 |
| BRD4 455 | chr19: 15238344-15238371 | BRD4 1778 | chr19: 15265359-15265386 |
| BRD4 456 | chr19: 15238363-15238390 | BRD4 1779 | chr19: 15265362-15265389 |
| BRD4 457 | chr19: 15238366-15238393 | BRD4 1780 | chr19: 15265363-15265390 |
| BRD4 458 | chr19: 15238392-15238419 | BRD4 1781 | chr19: 15265366-15265393 |
| BRD4 459 | chr19: 15238408-15238435 | BRD4 1782 | chr19: 15265374-15265401 |
| BRD4 460 | chr19: 15238422-15238449 | BRD4 1783 | chr19: 15265376-15265403 |
| BRD4 461 | chr19: 15238425-15238452 | BRD4 1784 | chr19: 15265377-15265404 |
| BRD4 462 | chr19: 15238432-15238459 | BRD4 1785 | chr19: 15265381-15265408 |
| BRD4 463 | chr19: 15238436-15238463 | BRD4 1786 | chr19: 15265382-15265409 |
| BRD4 464 | chr19: 15238439-15238466 | BRD4 1787 | chr19: 15265383-15265410 |
| BRD4 465 | chr19: 15238440-15238467 | BRD4 1788 | chr19: 15265389-15265416 |
| BRD4 466 | chr19: 15238442-15238469 | BRD4 1789 | chr19: 15265394-15265421 |
| BRD4 467 | chr19: 15238717-15238744 | BRD4 1790 | chr19: 15265395-15265422 |
| BRD4 468 | chr19: 15238720-15238747 | BRD4 1791 | chr19: 15265398-15265425 |
| BRD4 469 | chr19: 15238721-15238748 | BRD4 1792 | chr19: 15265399-15265426 |
| BRD4 470 | chr19: 15238722-15238749 | BRD4 1793 | chr19: 15265400-15265427 |
| BRD4 471 | chr19: 15238725-15238752 | BRD4 1794 | chr19: 15265401-15265428 |
| BRD4 472 | chr19: 15238730-15238757 | BRD4 1795 | chr19: 15265407-15265434 |
| BRD4 473 | chr19: 15238733-15238760 | BRD4 1796 | chr19: 15265411-15265438 |
| BRD4 474 | chr19: 15238748-15238775 | BRD4 1797 | chr19: 15265412-15265439 |
| BRD4 475 | chr19: 15238749-15238776 | BRD4 1798 | chr19: 15265413-15265440 |
| BRD4 476 | chr19: 15238752-15238779 | BRD4 1799 | chr19: 15265414-15265441 |
| BRD4 477 | chr19: 15238753-15238780 | BRD4 1800 | chr19: 15265415-15265442 |
| BRD4 478 | chr19: 15238763-15238790 | BRD4 1801 | chr19: 15265416-15265443 |
| BRD4 479 | chr19: 15238769-15238796 | BRD4 1802 | chr19: 15265422-15265449 |
| BRD4 480 | chr19: 15238770-15238797 | BRD4 1803 | chr19: 15265425-15265452 |
| BRD4 481 | chr19: 15238776-15238803 | BRD4 1804 | chr19: 15265426-15265453 |
| BRD4 482 | chr19: 15238780-15238807 | BRD4 1805 | chr19: 15265427-15265454 |
| BRD4 483 | chr19: 15238781-15238808 | BRD4 1806 | chr19: 15265428-15265455 |
| BRD4 484 | chr19: 15238785-15238812 | BRD4 1807 | chr19: 15265440-15265467 |
| BRD4 485 | chr19: 15238786-15238813 | BRD4 1808 | chr19: 15265444-15265471 |
| BRD4 486 | chr19: 15238787-15238814 | BRD4 1809 | chr19: 15265445-15265472 |
| BRD4 487 | chr19: 15238788-15238815 | BRD4 1810 | chr19: 15265446-15265473 |
| BRD4 488 | chr19: 15238791-15238818 | BRD4 1811 | chr19: 15265449-15265476 |
| BRD4 489 | chr19: 15238792-15238819 | BRD4 1812 | chr19: 15265467-15265494 |
| BRD4 490 | chr19: 15238799-15238826 | BRD4 1813 | chr19: 15265468-15265495 |
| BRD4 491 | chr19: 15238800-15238827 | BRD4 1814 | chr19: 15265469-15265496 |
| BRD4 492 | chr19: 15238801-15238828 | BRD4 1815 | chr19: 15265474-15265501 |
| BRD4 493 | chr19: 15238808-15238835 | BRD4 1816 | chr19: 15265479-15265506 |
| BRD4 494 | chr19: 15238809-15238836 | BRD4 1817 | chr19: 15265483-15265510 |
| BRD4 495 | chr19: 15238810-15238837 | BRD4 1818 | chr19: 15265488-15265515 |
| BRD4 496 | chr19: 15238813-15238840 | BRD4 1819 | chr19: 15265489-15265516 |
| BRD4 497 | chr19: 15238816-15238843 | BRD4 1820 | chr19: 15265490-15265517 |
| BRD4 498 | chr19: 15238819-15238846 | BRD4 1821 | chr19: 15265496-15265523 |
| BRD4 499 | chr19: 15238829-15238856 | BRD4 1822 | chr19: 15265500-15265527 |
| BRD4 500 | chr19: 15238850-15238877 | BRD4 1823 | chr19: 15265501-15265528 |
| BRD4 501 | chr19: 15238853-15238880 | BRD4 1824 | chr19: 15265505-15265532 |
| BRD4 502 | chr19: 15238866-15238893 | BRD4 1825 | chr19: 15265506-15265533 |
| BRD4 503 | chr19: 15238889-15238916 | BRD4 1826 | chr19: 15265507-15265534 |
| BRD4 504 | chr19: 15238890-15238917 | BRD4 1827 | chr19: 15265512-15265539 |
| BRD4 505 | chr19: 15238911-15238938 | BRD4 1828 | chr19: 15265517-15265544 |
| BRD4 506 | chr19: 15238912-15238939 | BRD4 1829 | chr19: 15265527-15265554 |
| BRD4 507 | chr19: 15238913-15238940 | BRD4 1830 | chr19: 15265530-15265557 |
| BRD4 508 | chr19: 15238920-15238947 | BRD4 1831 | chr19: 15265533-15265560 |
| BRD4 509 | chr19: 15238921-15238948 | BRD4 1832 | chr19: 15265539-15265566 |
| BRD4 510 | chr19: 15238928-15238955 | BRD4 1833 | chr19: 15265545-15265572 |
| BRD4 511 | chr19: 15238931-15238958 | BRD4 1834 | chr19: 15265546-15265573 |
| BRD4 512 | chr19: 15238940-15238967 | BRD4 1835 | chr19: 15265547-15265574 |
| BRD4 513 | chr19: 15238941-15238968 | BRD4 1836 | chr19: 15265552-15265579 |
| BRD4 514 | chr19: 15238944-15238971 | BRD4 1837 | chr19: 15265553-15265580 |
| BRD4 515 | chr19: 15238949-15238976 | BRD4 1838 | chr19: 15265560-15265587 |
| BRD4 516 | chr19: 15238950-15238977 | BRD4 1839 | chr19: 15265563-15265590 |
| BRD4 517 | chr19: 15238955-15238982 | BRD4 1840 | chr19: 15265587-15265614 |
| BRD4 518 | chr19: 15238957-15238984 | BRD4 1841 | chr19: 15265595-15265622 |
| BRD4 519 | chr19: 15238958-15238985 | BRD4 1842 | chr19: 15265605-15265632 |
| BRD4 520 | chr19: 15238964-15238991 | BRD4 1843 | chr19: 15265617-15265644 |
| BRD4 521 | chr19: 15238965-15238992 | BRD4 1844 | chr19: 15265628-15265655 |
| BRD4 522 | chr19: 15238970-15238997 | BRD4 1845 | chr19: 15265637-15265664 |
| BRD4 523 | chr19: 15238980-15239007 | BRD4 1846 | chr19: 15265642-15265669 |
| BRD4 524 | chr19: 15239032-15239059 | BRD4 1847 | chr19: 15265643-15265670 |
| BRD4 525 | chr19: 15239037-15239064 | BRD4 1848 | chr19: 15267415-15267442 |
| BRD4 526 | chr19: 15239038-15239065 | BRD4 1849 | chr19: 15267426-15267453 |
| BRD4 527 | chr19: 15239039-15239066 | BRD4 1850 | chr19: 15267429-15267456 |
| BRD4 528 | chr19: 15239042-15239069 | BRD4 1851 | chr19: 15267430-15267457 |
| BRD4 529 | chr19: 15239043-15239070 | BRD4 1852 | chr19: 15267436-15267463 |
| BRD4 530 | chr19: 15239050-15239077 | BRD4 1853 | chr19: 15267442-15267469 |
| BRD4 531 | chr19: 15239051-15239078 | BRD4 1854 | chr19: 15267449-15267476 |
| BRD4 532 | chr19: 15239054-15239081 | BRD4 1855 | chr19: 15267454-15267481 |
| BRD4 533 | chr19: 15239055-15239082 | BRD4 1856 | chr19: 15267455-15267482 |
| BRD4 534 | chr19: 15239058-15239085 | BRD4 1857 | chr19: 15267515-15267542 |
| BRD4 535 | chr19: 15239069-15239096 | BRD4 1858 | chr19: 15267524-15267551 |
| BRD4 536 | chr19: 15239073-15239100 | BRD4 1859 | chr19: 15267527-15267554 |
| BRD4 537 | chr19: 15239079-15239106 | BRD4 1860 | chr19: 15267528-15267555 |
| BRD4 538 | chr19: 15239080-15239107 | BRD4 1861 | chr19: 15267534-15267561 |
| BRD4 539 | chrl9: 15239084-15239111 | BRD4 1862 | chr19: 15267543-15267570 |
| BRD4 540 | chr19: 15239090-15239117 | BRD4 1863 | chr19: 15267544-15267571 |
| BRD4 541 | chrl9: 15239094-15239121 | BRD4 1864 | chr19: 15267545-15267572 |
| BRD4 542 | chr19: 15239095-15239122 | BRD4 1865 | chr19: 15267547-15267574 |
| BRD4 543 | chr19: 15239108-15239135 | BRD4 1866 | chr19: 15268143-15268170 |
| BRD4 544 | chr19: 15239114-15239141 | BRD4 1867 | chr19: 15268144-15268171 |
| BRD4 545 | chr19: 15239123-15239150 | BRD4 1868 | chr19: 15268153-15268180 |
| BRD4 546 | chr19: 15239125-15239152 | BRD4 1869 | chr19: 15268157-15268184 |
| BRD4 547 | chr19: 15239130-15239157 | BRD4 1870 | chr19: 15268158-15268185 |
| BRD4 548 | chr19: 15239133-15239160 | BRD4 1871 | chr19: 15268171-15268198 |
| BRD4 549 | chr19: 15239135-15239162 | BRD4 1872 | chr19: 15268180-15268207 |
| BRD4 550 | chrl9: 15239144-15239171 | BRD4 1873 | chr19: 15268181-15268208 |
| BRD4 551 | chr19: 15239145-15239172 | BRD4 1874 | chr19: 15268189-15268216 |
| BRD4 552 | chr19: 15239155-15239182 | BRD4 1875 | chr19: 15268225-15268252 |
| BRD4 553 | chrl9: 15239164-15239191 | BRD4 1876 | chr19: 15268226-15268253 |
| BRD4 554 | chr19: 15239170-15239197 | BRD4 1877 | chr19: 15268227-15268254 |
| BRD4 555 | chr19: 15239173-15239200 | BRD4 1878 | chr19: 15268230-15268257 |
| BRD4 556 | chr19: 15239176-15239203 | BRD4 1879 | chr19: 15268887-15268914 |
| BRD4 557 | chr19: 15239177-15239204 | BRD4 1880 | chr19: 15268888-15268915 |
| BRD4 558 | chr19: 15239178-15239205 | BRD4 1881 | chr19: 15268891-15268918 |
| BRD4 559 | chr19: 15239179-15239206 | BRD4 1882 | chr19: 15268892-15268919 |
| BRD4 560 | chr19: 15239182-15239209 | BRD4 1883 | chr19: 15268893-15268920 |
| BRD4 561 | chr19: 15239186-15239213 | BRD4 1884 | chr19: 15268904-15268931 |
| BRD4 562 | chr19: 15239202-15239229 | BRD4 1885 | chr19: 15268923-15268950 |
| BRD4 563 | chr19: 15239206-15239233 | BRD4 1886 | chr19: 15268946-15268973 |
| BRD4 564 | chr19: 15239212-15239239 | BRD4 1887 | chr19: 15268958-15268985 |
| BRD4 565 | chr19: 15239234-15239261 | BRD4 1888 | chr19: 15268968-15268995 |
| BRD4 566 | chr19: 15239235-15239262 | BRD4 1889 | chr19: 15268985-15269012 |
| BRD4 567 | chr19: 15239242-15239269 | BRD4 1890 | chr19: 15268991-15269018 |
| BRD4 568 | chr19: 15239243-15239270 | BRD4 1891 | chr19: 15269005-15269032 |
| BRD4 569 | chr19: 15239253-15239280 | BRD4 1892 | chr19: 15269006-15269033 |
| BRD4 570 | chr19: 15239257-15239284 | BRD4 1893 | chr19: 15269012-15269039 |
| BRD4 571 | chr19: 15239258-15239285 | BRD4 1894 | chr19: 15269019-15269046 |
| BRD4 572 | chr19: 15239259-15239286 | BRD4 1895 | chr19: 15269042-15269069 |
| BRD4 573 | chr19: 15239262-15239289 | BRD4 1896 | chr19: 15272789-15272816 |
| BRD4 574 | chr19: 15239263-15239290 | BRD4 1897 | chr19: 15272790-15272817 |
| BRD4 575 | chrl9: 15239264-15239291 | BRD4 1898 | chr19: 15272791-15272818 |
| BRD4 576 | chr19: 15239371-15239398 | BRD4 1899 | chr19: 15272794-15272821 |
| BRD4 577 | chr19: 15239372-15239399 | BRD4 1900 | chr19: 15272796-15272823 |
| BRD4 578 | chr19: 15239373-15239400 | BRD4 1901 | chr19: 15272797-15272824 |
| BRD4 579 | chr19: 15239379-15239406 | BRD4 1902 | chr19: 15272801-15272828 |
| BRD4 580 | chr19: 15239382-15239409 | BRD4 1903 | chr19: 15272802-15272829 |
| BRD4 581 | chr19: 15239386-15239413 | BRD4 1904 | chr19: 15272807-15272834 |
| BRD4 582 | chr19: 15239387-15239414 | BRD4 1905 | chr19: 15272817-15272844 |
| BRD4 583 | chr19: 15239391-15239418 | BRD4 1906 | chr19: 15272824-15272851 |
| BRD4 584 | chr19: 15239418-15239445 | BRD4 1907 | chr19: 15272829-15272856 |
| BRD4 585 | chr19: 15239419-15239446 | BRD4 1908 | chr19: 15272838-15272865 |
| BRD4 586 | chr19: 15239420-15239447 | BRD4 1909 | chr19: 15272842-15272869 |
| BRD4 587 | chr19: 15239421-15239448 | BRD4 1910 | chr19: 15272857-15272884 |
| BRD4 588 | chr19: 15239427-15239454 | BRD4 1911 | chr19: 15272875-15272902 |
| BRD4 589 | chr19: 15239430-15239457 | BRD4 1912 | chr19: 15272878-15272905 |
| BRD4 590 | chr19: 15239433-15239460 | BRD4 1913 | chr19: 15272890-15272917 |
| BRD4 591 | chr19: 15239436-15239463 | BRD4 1914 | chr19: 15272892-15272919 |
| BRD4 592 | chr19: 15239448-15239475 | BRD4 1915 | chr19: 15272894-15272921 |
| BRD4 593 | chr19: 15239449-15239476 | BRD4 1916 | chr19: 15272906-15272933 |
| BRD4 594 | chr19: 15239450-15239477 | BRD4 1917 | chr19: 15272907-15272934 |
| BRD4 595 | chr19: 15239451-15239478 | BRD4 1918 | chr19: 15272916-15272943 |
| BRD4 596 | chr19: 15239455-15239482 | BRD4 1919 | chr19: 15272917-15272944 |
| BRD4 597 | chr19: 15239463-15239490 | BRD4 1920 | chr19: 15272921-15272948 |
| BRD4 598 | chr19: 15239479-15239506 | BRD4 1921 | chr19: 15272924-15272951 |
| BRD4 599 | chr19: 15239481-15239508 | BRD4 1922 | chr19: 15272926-15272953 |
| BRD4 600 | chr19: 15239491-15239518 | BRD4 1923 | chr19: 15272931-15272958 |
| BRD4 601 | chr19: 15239493-15239520 | BRD4 1924 | chr19: 15272932-15272959 |
| BRD4 602 | chrl9: 15239494-15239521 | BRD4 1925 | chr19: 15272933-15272960 |
| BRD4 603 | chr19: 15239495-15239522 | BRD4 1926 | chr19: 15272934-15272961 |
| BRD4 604 | chr19: 15239499-15239526 | BRD4 1927 | chr19: 15272937-15272964 |
| BRD4 605 | chr19: 15239502-15239529 | BRD4 1928 | chr19: 15272938-15272965 |
| BRD4 606 | chr19: 15239514-15239541 | BRD4 1929 | chr19: 15272939-15272966 |
| BRD4 607 | chr19: 15239517-15239544 | BRD4 1930 | chr19: 15272940-15272967 |
| BRD4 608 | chr19: 15239518-15239545 | BRD4 1931 | chr19: 15272941-15272968 |
| BRD4 609 | chr19: 15239521-15239548 | BRD4 1932 | chr19: 15272945-15272972 |
| BRD4 610 | chr19: 15239522-15239549 | BRD4 1933 | chr19: 15272951-15272978 |
| BRD4 611 | chr19: 15239636-15239663 | BRD4 1934 | chr19: 15272960-15272987 |
| BRD4 612 | chr19: 15239637-15239664 | BRD4 1935 | chr19: 15272964-15272991 |
| BRD4 613 | chr19: 15239638-15239665 | BRD4 1936 | chr19: 15272968-15272995 |
| BRD4 614 | chr19: 15239642-15239669 | BRD4 1937 | chr19: 15272969-15272996 |
| BRD4 615 | chr19: 15239645-15239672 | BRD4 1938 | chr19: 15272970-15272997 |
| BRD4 616 | chr19: 15239649-15239676 | BRD4 1939 | chr19: 15272974-15273001 |
| BRD4 617 | chr19: 15239650-15239677 | BRD4 1940 | chr19: 15272975-15273002 |
| BRD4 618 | chr19: 15239651-15239678 | BRD4 1941 | chr19: 15272980-15273007 |
| BRD4 619 | chr19: 15239652-15239679 | BRD4 1942 | chr19: 15272981-15273008 |
| BRD4 620 | chr19: 15239668-15239695 | BRD4 1943 | chr19: 15273001-15273028 |
| BRD4 621 | chr19: 15239669-15239696 | BRD4 1944 | chr19: 15273002-15273029 |
| BRD4 622 | chr19: 15239676-15239703 | BRD4 1945 | chr19: 15273003-15273030 |
| BRD4 623 | chr19: 15239677-15239704 | BRD4 1946 | chr19: 15273009-15273036 |
| BRD4 624 | chr19: 15239678-15239705 | BRD4 1947 | chr19: 15273030-15273057 |
| BRD4 625 | chr19: 15239679-15239706 | BRD4 1948 | chr19: 15273041-15273068 |
| BRD4 626 | chr19: 15239680-15239707 | BRD4 1949 | chr19: 15273045-15273072 |
| BRD4 627 | chr19: 15239686-15239713 | BRD4 1950 | chr19: 15273046-15273073 |
| BRD4 628 | chr19: 15239692-15239719 | BRD4 1951 | chr19: 15273047-15273074 |
| BRD4 629 | chr19: 15239697-15239724 | BRD4 1952 | chr19: 15273048-15273075 |
| BRD4 630 | chr19: 15239698-15239725 | BRD4 1953 | chr19: 15273054-15273081 |
| BRD4 631 | chr19: 15239699-15239726 | BRD4 1954 | chr19: 15273055-15273082 |
| BRD4 632 | chr19: 15239706-15239733 | BRD4 1955 | chr19: 15273076-15273103 |
| BRD4 633 | chr19: 15239707-15239734 | BRD4 1956 | chr19: 15273077-15273104 |
| BRD4 634 | chr19: 15239710-15239737 | BRD4 1957 | chr19: 15273083-15273110 |
| BRD4 635 | chrl9: 15239714-15239741 | BRD4 1958 | chr19: 15273090-15273117 |
| BRD4 636 | chr19: 15239717-15239744 | BRD4 1959 | chr19: 15273102-15273129 |
| BRD4 637 | chr19: 15239724-15239751 | BRD4 1960 | chr19: 15273109-15273136 |
| BRD4 638 | chr19: 15239725-15239752 | BRD4 1961 | chr19: 15273110-15273137 |
| BRD4 639 | chr19: 15239732-15239759 | BRD4 1962 | chr19: 15273115-15273142 |
| BRD4 640 | chr19: 15239759-15239786 | BRD4 1963 | chr19: 15273125-15273152 |
| BRD4 641 | chr19: 15239760-15239787 | BRD4 1964 | chr19: 15275633-15275660 |
| BRD4 642 | chr19: 15239761-15239788 | BRD4 1965 | chr19: 15275656-15275683 |
| BRD4 643 | chr19: 15239765-15239792 | BRD4 1966 | chr19: 15275664-15275691 |
| BRD4 644 | chr19: 15239766-15239793 | BRD4 1967 | chr19: 15275667-15275694 |
| BRD4 645 | chr19: 15239767-15239794 | BRD4 1968 | chr19: 15275673-15275700 |
| BRD4 646 | chr19: 15239770-15239797 | BRD4 1969 | chr19: 15275674-15275701 |
| BRD4 647 | chr19: 15239771-15239798 | BRD4 1970 | chr19: 15275675-15275702 |
| BRD4 648 | chr19: 15239776-15239803 | BRD4 1971 | chr19: 15275699-15275726 |
| BRD4 649 | chr19: 15239778-15239805 | BRD4 1972 | chr19: 15275703-15275730 |
| BRD4 650 | chr19: 15239779-15239806 | BRD4 1973 | chr19: 15275704-15275731 |
| BRD4 651 | chr19: 15239781-15239808 | BRD4 1974 | chr19: 15275707-15275734 |
| BRD4 652 | chr19: 15239798-15239825 | BRD4 1975 | chr19: 15275709-15275736 |
| BRD4 653 | chr19: 15239799-15239826 | BRD4 1976 | chr19: 15275712-15275739 |
| BRD4 654 | chr19: 15239800-15239827 | BRD4 1977 | chr19: 15275727-15275754 |
| BRD4 655 | chr19: 15239803-15239830 | BRD4 1978 | chr19: 15275739-15275766 |
| BRD4 656 | chr19: 15239809-15239836 | BRD4 1979 | chr19: 15275740-15275767 |
| BRD4 657 | chr19: 15239817-15239844 | BRD4 1980 | chr19: 15275743-15275770 |
| BRD4 658 | chr19: 15239821-15239848 | BRD4 1981 | chr19: 15275752-15275779 |
| BRD4 659 | chr19: 15239892-15239919 | BRD4 1982 | chr19: 15275753-15275780 |
| BRD4 660 | chr19: 15239893-15239920 | BRD4 1983 | chr19: 15275754-15275781 |
| BRD4 661 | chr19: 15239894-15239921 | BRD4 1984 | chr19: 15275755-15275782 |
| BRD4 662 | chr19: 15239897-15239924 | BRD4 1985 | chr19: 15275756-15275783 |
| BRD4 663 | chr19: 15239898-15239925 | BRD4 1986 | chr19: 15275757-15275784 |
| BRD4 664 | chr19: 15239902-15239929 | BRD4 1987 | chr19: 15275767-15275794 |
| BRD4 665 | chr19: 15239909-15239936 | BRD4 1988 | chr19: 15275771-15275798 |
| BRD4 666 | chr19: 15239910-15239937 | BRD4 1989 | chr19: 15275772-15275799 |
| BRD4 667 | chr19: 15239911-15239938 | BRD4 1990 | chr19: 15275778-15275805 |
| BRD4 668 | chr19: 15239912-15239939 | BRD4 1991 | chr19: 15275790-15275817 |
| BRD4 669 | chr19: 15239915-15239942 | BRD4 1992 | chr19: 15275802-15275829 |
| BRD4 670 | chr19: 15239922-15239949 | BRD4 1993 | chr19: 15275813-15275840 |
| BRD4 671 | chr19: 15239925-15239952 | BRD4 1994 | chr19: 15275832-15275859 |
| BRD4 672 | chr19: 15239926-15239953 | BRD4 1995 | chr19: 15275833-15275860 |
| BRD4 673 | chr19: 15239931-15239958 | BRD4 1996 | chr19: 15275834-15275861 |
| BRD4 674 | chr19: 15239937-15239964 | BRD4 1997 | chr19: 15275839-15275866 |
| BRD4 675 | chr19: 15239952-15239979 | BRD4 1998 | chr19: 15275840-15275867 |
| BRD4 676 | chr19: 15239953-15239980 | BRD4 1999 | chr19: 15275845-15275872 |
| BRD4 677 | chr19: 15239954-15239981 | BRD4 2000 | chr19: 15275848-15275875 |
| BRD4 678 | chr19: 15239955-15239982 | BRD4 2001 | chr19: 15275860-15275887 |
| BRD4 679 | chr19: 15239956-15239983 | BRD4 2002 | chr19: 15275861-15275888 |
| BRD4 680 | chr19: 15239972-15239999 | BRD4 2003 | chr19: 15275863-15275890 |
| BRD4 681 | chr19: 15239973-15240000 | BRD4 2004 | chr19: 15275864-15275891 |
| BRD4 682 | chr19: 15239974-15240001 | BRD4 2005 | chr19: 15275870-15275897 |
| BRD4 683 | chr19: 15239977-15240004 | BRD4 2006 | chr19: 15275874-15275901 |
| BRD4 684 | chr19: 15239978-15240005 | BRD4 2007 | chr19: 15275876-15275903 |
| BRD4 685 | chr19: 15239979-15240006 | BRD4 2008 | chr19: 15275892-15275919 |
| BRD4 686 | chr19: 15239980-15240007 | BRD4 2009 | chr19: 15275893-15275920 |
| BRD4 687 | chr19: 15239988-15240015 | BRD4 2010 | chr19: 15275894-15275921 |
| BRD4 688 | chr19: 15239991-15240018 | BRD4 2011 | chr19: 15275897-15275924 |
| BRD4 689 | chr19: 15240000-15240027 | BRD4 2012 | chr19: 15275898-15275925 |
| BRD4 690 | chr19: 15240006-15240033 | BRD4 2013 | chr19: 15275900-15275927 |
| BRD4 691 | chr19: 15240007-15240034 | BRD4 2014 | chr19: 15275909-15275936 |
| BRD4 692 | chr19: 15240013-15240040 | BRD4 2015 | chr19: 15275916-15275943 |
| BRD4 693 | chr19: 15240022-15240049 | BRD4 2016 | chr19: 15275941-15275968 |
| BRD4 694 | chr19: 15242875-15242902 | BRD4 2017 | chr19: 15275943-15275970 |
| BRD4 695 | chr19: 15242884-15242911 | BRD4 2018 | chr19: 15275944-15275971 |
| BRD4 696 | chr19: 15242885-15242912 | BRD4 2019 | chr19: 15275952-15275979 |
| BRD4 697 | chr19: 15242886-15242913 | BRD4 2020 | chr19: 15275962-15275989 |
| BRD4 698 | chr19: 15242892-15242919 | BRD4 2021 | chr19: 15275966-15275993 |
| BRD4 699 | chr19: 15242898-15242925 | BRD4 2022 | chr19: 15275988-15276015 |
| BRD4 700 | chr19: 15242899-15242926 | BRD4 2023 | chr19: 15276007-15276034 |
| BRD4 701 | chrl9: 15242904-15242931 | BRD4 2024 | chr19: 15276017-15276044 |
| BRD4 702 | chr19: 15242905-15242932 | BRD4 2025 | chr19: 15276018-15276045 |
| BRD4 703 | chr19: 15242906-15242933 | BRD4 2026 | chr19: 15276029-15276056 |
| BRD4 704 | chr19: 15242913-15242940 | BRD4 2027 | chr19: 15276035-15276062 |
| BRD4 705 | chr19: 15242915-15242942 | BRD4 2028 | chr19: 15276036-15276063 |
| BRD4 706 | chrl9: 15242916-15242943 | BRD4 2029 | chr19: 15276037-15276064 |
| BRD4 707 | chr19: 15242922-15242949 | BRD4 2030 | chr19: 15276039-15276066 |
| BRD4 708 | chr19: 15242928-15242955 | BRD4 2031 | chr19: 15276040-15276067 |
| BRD4 709 | chr19: 15242936-15242963 | BRD4 2032 | chr19: 15276052-15276079 |
| BRD4 710 | chr19: 15242941-15242968 | BRD4 2033 | chr19: 15276059-15276086 |
| BRD4 711 | chr19: 15242945-15242972 | BRD4 2034 | chr19: 15276060-15276087 |
| BRD4 712 | chr19: 15242951-15242978 | BRD4 2035 | chr19: 15276062-15276089 |
| BRD4 713 | chrl9: 15242954-15242981 | BRD4 2036 | chr19: 15276071-15276098 |
| BRD4 714 | chr19: 15242955-15242982 | BRD4 2037 | chr19: 15276077-15276104 |
| BRD4 715 | chr19: 15242956-15242983 | BRD4 2038 | chr19: 15276078-15276105 |
| BRD4 716 | chrl9: 15242957-15242984 | BRD4 2039 | chr19: 15276085-15276112 |
| BRD4 717 | chr19: 15242960-15242987 | BRD4 2040 | chr19: 15276086-15276113 |
| BRD4 718 | chrl9: 15242967-15242994 | BRD4 2041 | chr19: 15276088-15276115 |
| BRD4 719 | chr19: 15242972-15242999 | BRD4 2042 | chr19: 15276099-15276126 |
| BRD4 720 | chr19: 15242973-15243000 | BRD4 2043 | chr19: 15276105-15276132 |
| BRD4 721 | chrl9: 15242974-15243001 | BRD4 2044 | chr19: 15276115-15276142 |
| BRD4 722 | chr19: 15242975-15243002 | BRD4 2045 | chr19: 15276124-15276151 |
| BRD4 723 | chr19: 15242978-15243005 | BRD4 2046 | chr19: 15276133-15276160 |
| BRD4 724 | chr19: 15242982-15243009 | BRD4 2047 | chr19: 15276136-15276163 |
| BRD4 725 | chr19: 15242983-15243010 | BRD4 2048 | chr19: 15276137-15276164 |
| BRD4 726 | chrl9: 15242984-15243011 | BRD4 2049 | chr19: 15276143-15276170 |
| BRD4 727 | chr19: 15242985-15243012 | BRD4 2050 | chr19: 15276147-15276174 |
| BRD4 728 | chr19: 15242986-15243013 | BRD4 2051 | chr19: 15276148-15276175 |
| BRD4 729 | chrl9: 15242987-15243014 | BRD4 2052 | chr19: 15276149-15276176 |
| BRD4 730 | chrl9: 15242994-15243021 | BRD4 2053 | chr19: 15276150-15276177 |
| BRD4 731 | chr19: 15242995-15243022 | BRD4 2054 | chr19: 15276170-15276197 |
| BRD4 732 | chr19: 15243000-15243027 | BRD4 2055 | chr19: 15276171-15276198 |
| BRD4 733 | chr19: 15243001-15243028 | BRD4 2056 | chr19: 15276190-15276217 |
| BRD4 734 | chr19: 15243002-15243029 | BRD4 2057 | chr19: 15276191-15276218 |
| BRD4 735 | chr19: 15243005-15243032 | BRD4 2058 | chr19: 15276195-15276222 |
| BRD4 736 | chr19: 15243008-15243035 | BRD4 2059 | chr19: 15276223-15276250 |
| BRD4 737 | chr19: 15243009-15243036 | BRD4 2060 | chr19: 15276224-15276251 |
| BRD4 738 | chr19: 15243010-15243037 | BRD4 2061 | chr19: 15276225-15276252 |
| BRD4 739 | chr19: 15243011-15243038 | BRD4 2062 | chr19: 15276227-15276254 |
| BRD4 740 | chr19: 15243018-15243045 | BRD4 2063 | chr19: 15276231-15276258 |
| BRD4 741 | chr19: 15243022-15243049 | BRD4 2064 | chr19: 15276238-15276265 |
| BRD4 742 | chr19: 15243023-15243050 | BRD4 2065 | chr19: 15276279-15276306 |
| BRD4 743 | chrl9: 15243024-15243051 | BRD4 2066 | chr19: 15276282-15276309 |
| BRD4 744 | chr19: 15243025-15243052 | BRD4 2067 | chr19: 15276290-15276317 |
| BRD4 745 | chr19: 15243028-15243055 | BRD4 2068 | chr19: 15276291-15276318 |
| BRD4 746 | chr19: 15243040-15243067 | BRD4 2069 | chr19: 15276300-15276327 |
| BRD4 747 | chr19: 15243041-15243068 | BRD4 2070 | chr19: 15276310-15276337 |
| BRD4 748 | chr19: 15243055-15243082 | BRD4 2071 | chr19: 15276311-15276338 |
| BRD4 749 | chr19: 15243056-15243083 | BRD4 2072 | chr19: 15276313-15276340 |
| BRD4 750 | chr19: 15243062-15243089 | BRD4 2073 | chr19: 15276314-15276341 |
| BRD4 751 | chr19: 15243065-15243092 | BRD4 2074 | chr19: 15276332-15276359 |
| BRD4 752 | chr19: 15243066-15243093 | BRD4 2075 | chr19: 15276333-15276360 |
| BRD4 753 | chr19: 15243067-15243094 | BRD4 2076 | chr19: 15276335-15276362 |
| BRD4 754 | chr19: 15243068-15243095 | BRD4 2077 | chr19: 15276351-15276378 |
| BRD4 755 | chr19: 15243084-15243111 | BRD4 2078 | chr19: 15276352-15276379 |
| BRD4 756 | chr19: 15243085-15243112 | BRD4 2079 | chr19: 15276360-15276387 |
| BRD4 757 | chr19: 15243086-15243113 | BRD4 2080 | chr19: 15276363-15276390 |
| BRD4 758 | chr19: 15243089-15243116 | BRD4 2081 | chr19: 15276364-15276391 |
| BRD4 759 | chr19: 15243092-15243119 | BRD4 2082 | chr19: 15276365-15276392 |
| BRD4 760 | chrl9: 15243096-15243123 | BRD4 2083 | chr19: 15276366-15276393 |
| BRD4 761 | chrl9: 15243097-15243124 | BRD4 2084 | chr19: 15276367-15276394 |
| BRD4 762 | chr19: 15243098-15243125 | BRD4 2085 | chr19: 15276371-15276398 |
| BRD4 763 | chr19: 15243102-15243129 | BRD4 2086 | chr19: 15276372-15276399 |
| BRD4 764 | chr19: 15243103-15243130 | BRD4 2087 | chr19: 15276373-15276400 |
| BRD4 765 | chr19: 15243104-15243131 | BRD4 2088 | chr19: 15276375-15276402 |
| BRD4 766 | chrl9: 15243107-15243134 | BRD4 2089 | chr19: 15276381-15276408 |
| BRD4 767 | chr19: 15243110-15243137 | BRD4 2090 | chr19: 15276382-15276409 |
| BRD4 768 | chr19: 15243111-15243138 | BRD4 2091 | chr19: 15276383-15276410 |
| BRD4 769 | chr19: 15243112-15243139 | BRD4 2092 | chr19: 15276388-15276415 |
| BRD4 770 | chr19: 15243113-15243140 | BRD4 2093 | chr19: 15276389-15276416 |
| BRD4 771 | chr19: 15243116-15243143 | BRD4 2094 | chr19: 15276390-15276417 |
| BRD4 772 | chr19: 15243119-15243146 | BRD4 2095 | chr19: 15276420-15276447 |
| BRD4 773 | chr19: 15243122-15243149 | BRD4 2096 | chr19: 15276424-15276451 |
| BRD4 774 | chr19: 15243151-15243178 | BRD4 2097 | chr19: 15276425-15276452 |
| BRD4 775 | chr19: 15243152-15243179 | BRD4 2098 | chr19: 15276432-15276459 |
| BRD4 776 | chr19: 15243153-15243180 | BRD4 2099 | chr19: 15276439-15276466 |
| BRD4 777 | chr19: 15243156-15243183 | BRD4 2100 | chr19: 15276446-15276473 |
| BRD4 778 | chrl9: 15243157-15243184 | BRD4 2101 | chr19: 15276450-15276477 |
| BRD4 779 | chr19: 15243158-15243185 | BRD4 2102 | chr19: 15276454-15276481 |
| BRD4 780 | chr19: 15243161-15243188 | BRD4 2103 | chr19: 15276455-15276482 |
| BRD4 781 | chr19: 15243162-15243189 | BRD4 2104 | chr19: 15276462-15276489 |
| BRD4 782 | chr19: 15243163-15243190 | BRD4 2105 | chr19: 15276463-15276490 |
| BRD4 783 | chr19: 15243164-15243191 | BRD4 2106 | chr19: 15276493-15276520 |
| BRD4 784 | chrl9: 15243167-15243194 | BRD4 2107 | chr19: 15276507-15276534 |
| BRD4 785 | chr19: 15243171-15243198 | BRD4 2108 | chr19: 15276521-15276548 |
| BRD4 786 | chr19: 15243172-15243199 | BRD4 2109 | chr19: 15276522-15276549 |
| BRD4 787 | chr19: 15243173-15243200 | BRD4 2110 | chr19: 15276540-15276567 |
| BRD4 788 | chrl9: 15243174-15243201 | BRD4 2111 | chr19: 15276541-15276568 |
| BRD4 789 | chr19: 15243175-15243202 | BRD4 2112 | chr19: 15276549-15276576 |
| BRD4 790 | chr19: 15243176-15243203 | BRD4 2113 | chr19: 15276554-15276581 |
| BRD4 791 | chr19: 15243179-15243206 | BRD4 2114 | chr19: 15276563-15276590 |
| BRD4 792 | chr19: 15243180-15243207 | BRD4 2115 | chr19: 15276564-15276591 |
| BRD4 793 | chr19: 15243181-15243208 | BRD4 2116 | chr19: 15276568-15276595 |
| BRD4 794 | chr19: 15243182-15243209 | BRD4 2117 | chr19: 15276573-15276600 |
| BRD4 795 | chr19: 15243186-15243213 | BRD4 2118 | chr19: 15276574-15276601 |
| BRD4 796 | chrl9: 15243187-15243214 | BRD4 2119 | chr19: 15276575-15276602 |
| BRD4 797 | chr19: 15243202-15243229 | BRD4 2120 | chr19: 15276576-15276603 |
| BRD4 798 | chr19: 15243206-15243233 | BRD4 2121 | chr19: 15276584-15276611 |
| BRD4 799 | chr19: 15243207-15243234 | BRD4 2122 | chr19: 15276606-15276633 |
| BRD4 800 | chr19: 15243215-15243242 | BRD4 2123 | chr19: 15276609-15276636 |
| BRD4 801 | chr19: 15243221-15243248 | BRD4 2124 | chr19: 15276621-15276648 |
| BRD4 802 | chr19: 15243222-15243249 | BRD4 2125 | chr19: 15276622-15276649 |
| BRD4 803 | chr19: 15243223-15243250 | BRD4 2126 | chr19: 15276641-15276668 |
| BRD4 804 | chrl9: 15243224-15243251 | BRD4 2127 | chr19: 15276642-15276669 |
| BRD4 805 | chr19: 15243249-15243276 | BRD4 2128 | chr19: 15276646-15276673 |
| BRD4 806 | chrl9: 15243257-15243284 | BRD4 2129 | chr19: 15276647-15276674 |
| BRD4 807 | chr19: 15243268-15243295 | BRD4 2130 | chr19: 15276648-15276675 |
| BRD4 808 | chr19: 15243271-15243298 | BRD4 2131 | chr19: 15276651-15276678 |
| BRD4 809 | chr19: 15243276-15243303 | BRD4 2132 | chr19: 15276656-15276683 |
| BRD4 810 | chr19: 15243277-15243304 | BRD4 2133 | chr19: 15276657-15276684 |
| BRD4 811 | chr19: 15243278-15243305 | BRD4 2134 | chr19: 15276669-15276696 |
| BRD4 812 | chr19: 15243281-15243308 | BRD4 2135 | chr19: 15276674-15276701 |
| BRD4 813 | chr19: 15243282-15243309 | BRD4 2136 | chr19: 15276679-15276706 |
| BRD4 814 | chr19: 15243283-15243310 | BRD4 2137 | chr19: 15276704-15276731 |
| BRD4 815 | chrl9: 15243287-15243314 | BRD4 2138 | chr19: 15276713-15276740 |
| BRD4 816 | chr19: 15243290-15243317 | BRD4 2139 | chr19: 15276742-15276769 |
| BRD4 817 | chr19: 15243315-15243342 | BRD4 2140 | chr19: 15276744-15276771 |
| BRD4 818 | chr19: 15243316-15243343 | BRD4 2141 | chr19: 15276747-15276774 |
| BRD4 819 | chrl9: 15243317-15243344 | BRD4 2142 | chr19: 15276749-15276776 |
| BRD4 820 | chr19: 15243318-15243345 | BRD4 2143 | chr19: 15276771-15276798 |
| BRD4 821 | chr19: 15243319-15243346 | BRD4 2144 | chr19: 15276778-15276805 |
| **BRD4** 822 | chr19: 15243320-15243347 | BRD4 2145 | chr19: 15276780-15276807 |
| BRD4 823 | chrl9: 15243324-15243351 | BRD4 2146 | chr19: 15276787-15276814 |
| BRD4 824 | chr19: 15243325-15243352 | BRD4 2147 | chr19: 15276789-15276816 |
| BRD4 825 | chr19: 15243326-15243353 | BRD4 2148 | chr19: 15276792-15276819 |
| BRD4 826 | chr19: 15243329-15243356 | BRD4 2149 | chr19: 15276793-15276820 |
| BRD4 827 | chr19: 15243331-15243358 | BRD4 2150 | chr19: 15276809-15276836 |
| **BRD4** 828 | chr19: 15243332-15243359 | BRD4 2151 | chr19: 15276819-15276846 |
| BRD4 829 | chr19: 15243333-15243360 | BRD4 2152 | chr19: 15276820-15276847 |
| BRD4 830 | chrl9: 15243334-15243361 | BRD4 2153 | chr19: 15276821-15276848 |
| BRD4 831 | chr19: 15243335-15243362 | BRD4 2154 | chr19: 15276823-15276850 |
| BRD4 832 | chr19: 15243341-15243368 | BRD4 2155 | chr19: 15276824-15276851 |
| BRD4 833 | chr19: 15243342-15243369 | BRD4 2156 | chr19: 15276833-15276860 |
| BRD4 834 | chr19: 15243348-15243375 | BRD4 2157 | chr19: 15276836-15276863 |
| BRD4 835 | chr19: 15243349-15243376 | BRD4 2158 | chr19: 15276842-15276869 |
| BRD4 836 | chr19: 15243350-15243377 | BRD4 2159 | chr19: 15276843-15276870 |
| BRD4 837 | chr19: 15243353-15243380 | BRD4 2160 | chr19: 15276847-15276874 |
| BRD4 838 | chr19: 15243357-15243384 | BRD4 2161 | chr19: 15276848-15276875 |
| BRD4 839 | chr19: 15243358-15243385 | BRD4 2162 | chr19: 15276857-15276884 |
| BRD4 840 | chrl9: 15243364-15243391 | BRD4 2163 | chr19: 15276863-15276890 |
| BRD4 841 | chr19: 15243368-15243395 | BRD4 2164 | chr19: 15276867-15276894 |
| BRD4 842 | chr19: 15243376-15243403 | BRD4 2165 | chr19: 15276868-15276895 |
| BRD4 843 | chr19: 15243380-15243407 | BRD4 2166 | chr19: 15276869-15276896 |
| BRD4 844 | chr19: 15243381-15243408 | BRD4 2167 | chr19: 15276874-15276901 |
| BRD4 845 | chr19: 15243382-15243409 | BRD4 2168 | chr19: 15276875-15276902 |
| BRD4 846 | chr19: 15243383-15243410 | BRD4 2169 | chr19: 15276885-15276912 |
| BRD4 847 | chrl9: 15243387-15243414 | BRD4 2170 | chr19: 15276893-15276920 |
| BRD4 848 | chr19: 15243388-15243415 | BRD4 2171 | chr19: 15276894-15276921 |
| BRD4 849 | chr19: 15243391-15243418 | BRD4 2172 | chr19: 15276899-15276926 |
| BRD4 850 | chr19: 15243392-15243419 | BRD4 2173 | chr19: 15276902-15276929 |
| BRD4 851 | chrl9: 15243397-15243424 | BRD4 2174 | chr19: 15276914-15276941 |
| BRD4 852 | chr19: 15243398-15243425 | BRD4 2175 | chr19: 15276915-15276942 |
| BRD4 853 | chr19: 15243401-15243428 | BRD4 2176 | chr19: 15276917-15276944 |
| BRD4 854 | chr19: 15243405-15243432 | BRD4 2177 | chr19: 15276918-15276945 |
| BRD4 855 | chr19: 15243406-15243433 | BRD4 2178 | chr19: 15276938-15276965 |
| BRD4 856 | chr19: 15243411-15243438 | BRD4 2179 | chr19: 15276941-15276968 |
| BRD4 857 | chr19: 15243412-15243439 | BRD4 2180 | chr19: 15276942-15276969 |
| BRD4 858 | chr19: 15243417-15243444 | BRD4 2181 | chr19: 15276943-15276970 |
| BRD4 859 | chrl9: 15243424-15243451 | BRD4 2182 | chr19: 15276944-15276971 |
| BRD4 860 | chr19: 15243425-15243452 | BRD4 2183 | chr19: 15276959-15276986 |
| BRD4 861 | chrl9: 15243434-15243461 | BRD4 2184 | chr19: 15276966-15276993 |
| BRD4 862 | chr19: 15243441-15243468 | BRD4 2185 | chr19: 15276969-15276996 |
| BRD4 863 | chr19: 15243442-15243469 | BRD4 2186 | chr19: 15276970-15276997 |
| BRD4 864 | chr19: 15243443-15243470 | BRD4 2187 | chr19: 15276971-15276998 |
| BRD4 865 | chr19: 15243453-15243480 | BRD4 2188 | chr19: 15276972-15276999 |
| BRD4 866 | chrl9: 15243464-15243491 | BRD4 2189 | chr19: 15276977-15277004 |
| BRD4 867 | chr19: 15243482-15243509 | BRD4 2190 | chr19: 15276981-15277008 |
| BRD4 868 | chr19: 15243485-15243512 | BRD4 2191 | chr19: 15276987-15277014 |
| BRD4 869 | chr19: 15244204-15244231 | BRD4 2192 | chr19: 15276997-15277024 |
| BRD4 870 | chr19: 15244205-15244232 | BRD4 2193 | chr19: 15277006-15277033 |
| BRD4 871 | chr19: 15244207-15244234 | BRD4 2194 | chr19: 15277019-15277046 |
| BRD4 872 | chr19: 15244210-15244237 | BRD4 2195 | chr19: 15277029-15277056 |
| BRD4 873 | chr19: 15244220-15244247 | BRD4 2196 | chr19: 15277068-15277095 |
| BRD4 874 | chr19: 15244229-15244256 | BRD4 2197 | chr19: 15277095-15277122 |
| BRD4 875 | chr19: 15244230-15244257 | BRD4 2198 | chr19: 15277096-15277123 |
| BRD4 876 | chr19: 15244238-15244265 | BRD4 2199 | chr19: 15277101-15277128 |
| BRD4 877 | chr19: 15244239-15244266 | BRD4 2200 | chr19: 15277107-15277134 |
| BRD4 878 | chr19: 15244240-15244267 | BRD4 2201 | chr19: 15277116-15277143 |
| BRD4 879 | chr19: 15244243-15244270 | BRD4 2202 | chr19: 15277123-15277150 |
| BRD4 880 | chr19: 15244258-15244285 | BRD4 2203 | chr19: 15277137-15277164 |
| BRD4 881 | chr19: 15244261-15244288 | BRD4 2204 | chr19: 15277140-15277167 |
| **BRD4** 882 | chr19: 15244265-15244292 | BRD4 2205 | chr19: 15277142-15277169 |
| BRD4 883 | chr19: 15244266-15244293 | BRD4 2206 | chr19: 15277147-15277174 |
| BRD4 884 | chrl9: 15244267-15244294 | BRD4 2207 | chr19: 15277148-15277175 |
| BRD4 885 | chr19: 15244271-15244298 | BRD4 2208 | chr19: 15277154-15277181 |
| BRD4 886 | chr19: 15244276-15244303 | BRD4 2209 | chr19: 15277188-15277215 |
| BRD4 887 | chr19: 15244277-15244304 | BRD4 2210 | chr19: 15277191-15277218 |
| **BRD4** 888 | chr19: 15244278-15244305 | BRD4 2211 | chr19: 15277204-15277231 |
| BRD4 889 | chr19: 15244279-15244306 | BRD4 2212 | chr19: 15277209-15277236 |
| BRD4 890 | chr19: 15244288-15244315 | BRD4 2213 | chr19: 15277240-15277267 |
| BRD4 891 | chrl9: 15244294-15244321 | BRD4 2214 | chr19: 15277259-15277286 |
| BRD4 892 | chr19: 15244298-15244325 | BRD4 2215 | chr19: 15277269-15277296 |
| BRD4 893 | chrl9: 15244304-15244331 | BRD4 2216 | chr19: 15277270-15277297 |
| BRD4 894 | chr19: 15244308-15244335 | BRD4 2217 | chr19: 15277274-15277301 |
| BRD4 895 | chr19: 15244309-15244336 | BRD4 2218 | chr19: 15277283-15277310 |
| BRD4 896 | chr19: 15244313-15244340 | BRD4 2219 | chr19: 15277284-15277311 |
| BRD4 897 | chrl9: 15244314-15244341 | BRD4 2220 | chr19: 15277285-15277312 |
| BRD4 898 | chr19: 15244322-15244349 | BRD4 2221 | chr19: 15277322-15277349 |
| BRD4 899 | chr19: 15244329-15244356 | BRD4 2222 | chr19: 15277330-15277357 |
| BRD4 900 | chr19: 15244330-15244357 | BRD4 2223 | chr19: 15277331-15277358 |
| BRD4 901 | chr19: 15244331-15244358 | BRD4 2224 | chr19: 15277332-15277359 |
| BRD4 902 | chr19: 15244348-15244375 | BRD4 2225 | chr19: 15277341-15277368 |
| BRD4 903 | chr19: 15244349-15244376 | BRD4 2226 | chr19: 15277344-15277371 |
| BRD4 904 | chr19: 15244350-15244377 | BRD4 2227 | chr19: 15277345-15277372 |
| BRD4 905 | chr19: 15244355-15244382 | BRD4 2228 | chr19: 15277352-15277379 |
| BRD4 906 | chr19: 15244356-15244383 | BRD4 2229 | chr19: 15277354-15277381 |
| BRD4 907 | chr19: 15244357-15244384 | BRD4 2230 | chr19: 15277363-15277390 |
| BRD4 908 | chrl9: 15244364-15244391 | BRD4 2231 | chr19: 15277379-15277406 |
| BRD4 909 | chr19: 15244368-15244395 | BRD4 2232 | chr19: 15277425-15277452 |
| BRD4 910 | chr19: 15244369-15244396 | BRD4 2233 | chr19: 15277456-15277483 |
| BRD4 911 | chr19: 15244371-15244398 | BRD4 2234 | chr19: 15277479-15277506 |
| BRD4 912 | chr19: 15244376-15244403 | BRD4 2235 | chr19: 15280244-15280271 |
| BRD4 913 | chr19: 15244377-15244404 | BRD4 2236 | chr19: 15280248-15280275 |
| BRD4 914 | chr19: 15244380-15244407 | BRD4 2237 | chr19: 15280256-15280283 |
| BRD4 915 | chrl9: 15244387-15244414 | BRD4 2238 | chr19: 15280262-15280289 |
| BRD4 916 | chr19: 15244389-15244416 | BRD4 2239 | chr19: 15280263-15280290 |
| BRD4 917 | chr19: 15244390-15244417 | BRD4 2240 | chr19: 15280264-15280291 |
| BRD4 918 | chr19: 15244393-15244420 | BRD4 2241 | chr19: 15280270-15280297 |
| BRD4 919 | chrl9: 15244394-15244421 | BRD4 2242 | chr19: 15280281-15280308 |
| BRD4 920 | chr19: 15244395-15244422 | BRD4 2243 | chr19: 15280282-15280309 |
| BRD4 921 | chr19: 15244396-15244423 | BRD4 2244 | chr19: 15280288-15280315 |
| BRD4 922 | chr19: 15244399-15244426 | BRD4 2245 | chr19: 15280289-15280316 |
| BRD4 923 | chr19: 15244401-15244428 | BRD4 2246 | chr19: 15280296-15280323 |
| BRD4 924 | chr19: 15244414-15244441 | BRD4 2247 | chr19: 15280298-15280325 |
| BRD4 925 | chr19: 15244415-15244442 | BRD4 2248 | chr19: 15280299-15280326 |
| BRD4 926 | chr19: 15244416-15244443 | BRD4 2249 | chr19: 15280301-15280328 |
| BRD4 927 | chr19: 15244429-15244456 | BRD4 2250 | chr19: 15280307-15280334 |
| BRD4 928 | chrl9: 15244434-15244461 | BRD4 2251 | chr19: 15280329-15280356 |
| BRD4 929 | chr19: 15244437-15244464 | BRD4 2252 | chr19: 15280336-15280363 |
| BRD4 930 | chr19: 15244438-15244465 | BRD4 2253 | chr19: 15280338-15280365 |
| BRD4 931 | chr19: 15244441-15244468 | BRD4 2254 | chr19: 15280353-15280380 |
| BRD4 932 | chr19: 15244442-15244469 | BRD4 2255 | chr19: 15280354-15280381 |
| BRD4 933 | chr19: 15244443-15244470 | BRD4 2256 | chr19: 15280362-15280389 |
| BRD4 934 | chr19: 15244444-15244471 | BRD4 2257 | chr19: 15280363-15280390 |
| BRD4 935 | chrl9: 15244447-15244474 | BRD4 2258 | chr19: 15280364-15280391 |
| BRD4 936 | chr19: 15244450-15244477 | BRD4 2259 | chr19: 15280368-15280395 |
| BRD4 937 | chr19: 15244451-15244478 | BRD4 2260 | chr19: 15280373-15280400 |
| BRD4 938 | chr19: 15244452-15244479 | BRD4 2261 | chr19: 15280385-15280412 |
| BRD4 939 | chr19: 15244453-15244480 | BRD4 2262 | chr19: 15280386-15280413 |
| BRD4 940 | chr19: 15244456-15244483 | BRD4 2263 | chr19: 15280393-15280420 |
| BRD4 941 | chr19: 15244459-15244486 | BRD4 2264 | chr19: 15280401-15280428 |
| BRD4 942 | chr19: 15244477-15244504 | BRD4 2265 | chr19: 15280402-15280429 |
| BRD4 943 | chr19: 15244480-15244507 | BRD4 2266 | chr19: 15280443-15280470 |
| BRD4 944 | chr19: 15244483-15244510 | BRD4 2267 | chr19: 15331812-15331839 |
| BRD4 945 | chr19: 15244486-15244513 | BRD4 2268 | chr19: 15331813-15331840 |
| BRD4 946 | chr19: 15244489-15244516 | BRD4 2269 | chr19: 15331814-15331841 |
| BRD4 947 | chr19: 15244492-15244519 | BRD4 2270 | chr19: 15331815-15331842 |
| BRD4 948 | chr19: 15244493-15244520 | BRD4 2271 | chr19: 15331817-15331844 |
| BRD4 949 | chrl9: 15244494-15244521 | BRD4 2272 | chr19: 15331818-15331845 |
| BRD4 950 | chr19: 15244495-15244522 | BRD4 2273 | chr19: 15331820-15331847 |
| BRD4 951 | chr19: 15244502-15244529 | BRD4 2274 | chr19: 15331825-15331852 |
| BRD4 952 | chr19: 15244503-15244530 | BRD4 2275 | chr19: 15331828-15331855 |
| BRD4 953 | chrl9: 15244504-15244531 | BRD4 2276 | chr19: 15331835-15331862 |
| BRD4 954 | chr19: 15244507-15244534 | BRD4 2277 | chr19: 15331836-15331863 |
| BRD4 955 | chr19: 15244510-15244537 | BRD4 2278 | chr19: 15331837-15331864 |
| BRD4 956 | chr19: 15244511-15244538 | BRD4 2279 | chr19: 15331840-15331867 |
| BRD4 957 | chr19: 15244512-15244539 | BRD4 2280 | chr19: 15331841-15331868 |
| BRD4 958 | chr19: 15244513-15244540 | BRD4 2281 | chr19: 15331850-15331877 |
| BRD4 959 | chrl9: 15244516-15244543 | BRD4 2282 | chr19: 15331857-15331884 |
| BRD4 960 | chr19: 15244523-15244550 | BRD4 2283 | chr19: 15331858-15331885 |
| BRD4 961 | chrl9: 15244524-15244551 | BRD4 2284 | chr19: 15331859-15331886 |
| BRD4 962 | chr19: 15244525-15244552 | BRD4 2285 | chr19: 15331868-15331895 |
| BRD4 963 | chr19: 15244531-15244558 | BRD4 2286 | chr19: 15331874-15331901 |
| BRD4 964 | chr19: 15244537-15244564 | BRD4 2287 | chr19: 15331877-15331904 |
| BRD4 965 | chr19: 15244538-15244565 | BRD4 2288 | chr19: 15331880-15331907 |
| BRD4 966 | chr19: 15244539-15244566 | BRD4 2289 | chr19: 15331882-15331909 |
| BRD4 967 | chr19: 15244559-15244586 | BRD4 2290 | chr19: 15331883-15331910 |
| BRD4 968 | chr19: 15244561-15244588 | BRD4 2291 | chr19: 15331885-15331912 |
| BRD4 969 | chr19: 15244562-15244589 | BRD4 2292 | chr19: 15331886-15331913 |
| BRD4 970 | chr19: 15244567-15244594 | BRD4 2293 | chr19: 15331888-15331915 |
| BRD4 971 | chr19: 15244571-15244598 | BRD4 2294 | chr19: 15331889-15331916 |
| BRD4 972 | chr19: 15244693-15244720 | BRD4 2295 | chr19: 15331892-15331919 |
| BRD4 973 | chr19: 15244694-15244721 | BRD4 2296 | chr19: 15331901-15331928 |
| BRD4 974 | chr19: 15244695-15244722 | BRD4 2297 | chr19: 15331902-15331929 |
| BRD4 975 | chr19: 15244701-15244728 | BRD4 2298 | chr19: 15331910-15331937 |
| BRD4 976 | chr19: 15244702-15244729 | BRD4 2299 | chr19: 15332264-15332291 |
| BRD4 977 | chr19: 15244703-15244730 | BRD4 2300 | chr19: 15332267-15332294 |
| BRD4 978 | chrl9: 15244704-15244731 | BRD4 2301 | chr19: 15332268-15332295 |
| BRD4 979 | chr19: 15244709-15244736 | BRD4 2302 | chr19: 15332269-15332296 |
| BRD4 980 | chr19: 15244721-15244748 | BRD4 2303 | chr19: 15332275-15332302 |
| BRD4 981 | chr19: 15244722-15244749 | BRD4 2304 | chr19: 15332279-15332306 |
| BRD4 982 | chr19: 15244725-15244752 | BRD4 2305 | chr19: 15332284-15332311 |
| BRD4 983 | chr19: 15244726-15244753 | BRD4 2306 | chr19: 15332289-15332316 |
| BRD4 984 | chr19: 15244727-15244754 | BRD4 2307 | chr19: 15332295-15332322 |
| BRD4 985 | chrl9: 15244734-15244761 | BRD4 2308 | chr19: 15332303-15332330 |
| BRD4 986 | chr19: 15244735-15244762 | BRD4 2309 | chr19: 15332310-15332337 |
| BRD4 987 | chr19: 15244736-15244763 | BRD4 2310 | chr19: 15332313-15332340 |
| BRD4 988 | chr19: 15244744-15244771 | BRD4 2311 | chr19: 15332314-15332341 |
| BRD4 989 | chr19: 15244755-15244782 | BRD4 2312 | chr19: 15332315-15332342 |
| BRD4 990 | chrl9: 15244757-15244784 | BRD4 2313 | chr19: 15332316-15332343 |
| BRD4 991 | chr19: 15247022-15247049 | BRD4 2314 | chr19: 15332319-15332346 |
| BRD4 992 | chr19: 15247034-15247061 | BRD4 2315 | chr19: 15332323-15332350 |
| BRD4 993 | chr19: 15247050-15247077 | BRD4 2316 | chr19: 15332327-15332354 |
| BRD4 994 | chr19: 15247075-15247102 | BRD4 2317 | chr19: 15332330-15332357 |
| BRD4 995 | chr19: 15247091-15247118 | BRD4 2318 | chr19: 15332331-15332358 |
| BRD4 996 | chr19: 15247095-15247122 | BRD4 2319 | chr19: 15332337-15332364 |
| BRD4 997 | chr19: 15247158-15247185 | BRD4 2320 | chr19: 15332340-15332367 |
| BRD4 998 | chr19: 15247176-15247203 | BRD4 2321 | chr19: 15332343-15332370 |
| BRD4 999 | chr19: 15247179-15247206 | BRD4 2322 | chr19: 15332344-15332371 |
| BRD4 1000 | chr19: 15247180-15247207 | BRD4 2323 | chr19: 15332345-15332372 |
| BRD4 1001 | chr19: 15247183-15247210 | BRD4 2324 | chr19: 15332346-15332373 |
| BRD4 1002 | chr19: 15247184-15247211 | BRD4 2325 | chr19: 15332347-15332374 |
| BRD4 1003 | chr19: 15247185-15247212 | BRD4 2326 | chr19: 15332348-15332375 |
| BRD4 1004 | chr19: 15247186-15247213 | BRD4 2327 | chr19: 15332349-15332376 |
| BRD4 1005 | chr19: 15247187-15247214 | BRD4 2328 | chr19: 15332350-15332377 |
| BRD4 1006 | chr19: 15247189-15247216 | BRD4 2329 | chr19: 15332351-15332378 |
| BRD4 1007 | chr19: 15247191-15247218 | BRD4 2330 | chr19: 15332359-15332386 |
| BRD4 1008 | chr19: 15247192-15247219 | BRD4 2331 | chr19: 15332370-15332397 |
| BRD4 1009 | chr19: 15247193-15247220 | BRD4 2332 | chr19: 15332376-15332403 |
| BRD4 1010 | chr19: 15247198-15247225 | BRD4 2333 | chr19: 15332377-15332404 |
| BRD4 1011 | chr19: 15247203-15247230 | BRD4 2334 | chr19: 15332380-15332407 |
| BRD4 1012 | chr19: 15247204-15247231 | BRD4 2335 | chr19: 15332381-15332408 |
| BRD4 1013 | chr19: 15247210-15247237 | BRD4 2336 | chr19: 15332384-15332411 |
| BRD4 1014 | chr19: 15247215-15247242 | BRD4 2337 | chr19: 15332392-15332419 |
| BRD4 1015 | chr19: 15247237-15247264 | BRD4 2338 | chr19: 15332393-15332420 |
| BRD4 1016 | chr19: 15247246-15247273 | BRD4 2339 | chr19: 15332394-15332421 |
| BRD4 1017 | chr19: 15247247-15247274 | BRD4 2340 | chr19: 15332403-15332430 |
| BRD4 1018 | chr19: 15247248-15247275 | BRD4 2341 | chr19: 15332405-15332432 |
| BRD4 1019 | chr19: 15247259-15247286 | BRD4 2342 | chr19: 15332414-15332441 |
| BRD4 1020 | chr19: 15247265-15247292 | BRD4 2343 | chr19: 15332415-15332442 |
| BRD4 1021 | chr19: 15247268-15247295 | BRD4 2344 | chr19: 15332424-15332451 |
| BRD4 1022 | chr19: 15247271-15247298 | BRD4 2345 | chr19: 15332427-15332454 |
| BRD4 1023 | chr19: 15247272-15247299 | BRD4 2346 | chr19: 15332434-15332461 |
| BRD4 1024 | chr19: 15247275-15247302 | BRD4 2347 | chr19: 15332437-15332464 |
| BRD4 1025 | chr19: 15247276-15247303 | BRD4 2348 | chr19: 15332439-15332466 |
| BRD4 1026 | chr19: 15247277-15247304 | BRD4 2349 | chr19: 15332443-15332470 |
| BRD4 1027 | chr19: 15247280-15247307 | BRD4 2350 | chr19: 15332447-15332474 |
| BRD4 1028 | chr19: 15247285-15247312 | BRD4 2351 | chr19: 15332449-15332476 |
| BRD4 1029 | chr19: 15247288-15247315 | BRD4 2352 | chr19: 15332452-15332479 |
| BRD4 1030 | chr19: 15247289-15247316 | BRD4 2353 | chr19: 15332453-15332480 |
| BRD4 1031 | chr19: 15247290-15247317 | BRD4 2354 | chr19: 15332454-15332481 |
| BRD4 1032 | chr19: 15247294-15247321 | BRD4 2355 | chr19: 15332455-15332482 |
| BRD4 1033 | chr19: 15247295-15247322 | BRD4 2356 | chr19: 15332469-15332496 |
| BRD4 1034 | chr19: 15247309-15247336 | BRD4 2357 | chr19: 15332485-15332512 |
| BRD4 1035 | chr19: 15247312-15247339 | BRD4 2358 | chr19: 15332498-15332525 |
| BRD4 1036 | chr19: 15247313-15247340 | BRD4 2359 | chr19: 15332499-15332526 |
| BRD4 1037 | chr19: 15247314-15247341 | BRD4 2360 | chr19: 15332503-15332530 |
| BRD4 1038 | chr19: 15247327-15247354 | BRD4 2361 | chr19: 15332506-15332533 |
| BRD4 1039 | chr19: 15247333-15247360 | BRD4 2362 | chr19: 15332509-15332536 |
| BRD4 1040 | chr19: 15247334-15247361 | BRD4 2363 | chr19: 15332512-15332539 |
| BRD4 1041 | chr19: 15247343-15247370 | BRD4 2364 | chr19: 15332515-15332542 |
| BRD4 1042 | chr19: 15247349-15247376 | BRD4 2365 | chr19: 15332518-15332545 |
| BRD4 1043 | chr19: 15247359-15247386 | BRD4 2366 | chr19: 15332521-15332548 |
| BRD4 1044 | chr19: 15247360-15247387 | BRD4 2367 | chr19: 15332524-15332551 |
| BRD4 1045 | chr19: 15247361-15247388 | BRD4 2368 | chr19: 15332527-15332554 |
| BRD4 1046 | chr19: 15247368-15247395 | BRD4 2369 | chr19: 15332530-15332557 |
| BRD4 1047 | chr19: 15247374-15247401 | BRD4 2370 | chr19: 15332540-15332567 |
| BRD4 1048 | chr19: 15247382-15247409 | BRD4 2371 | chr19: 15332547-15332574 |
| BRD4 1049 | chr19: 15247391-15247418 | BRD4 2372 | chr19: 15332561-15332588 |
| BRD4 1050 | chr19: 15247436-15247463 | BRD4 2373 | chr19: 15332568-15332595 |
| BRD4 1051 | chr19: 15247440-15247467 | BRD4 2374 | chr19: 15332569-15332596 |
| BRD4 1052 | chr19: 15247441-15247468 | BRD4 2375 | chr19: 15332570-15332597 |
| BRD4 1053 | chr19: 15247447-15247474 | BRD4 2376 | chr19: 15332575-15332602 |
| BRD4 1054 | chr19: 15247448-15247475 | BRD4 2377 | chr19: 15332578-15332605 |
| BRD4 1055 | chr19: 15247456-15247483 | BRD4 2378 | chr19: 15332581-15332608 |
| BRD4 1056 | chr19: 15247461-15247488 | BRD4 2379 | chr19: 15332584-15332611 |
| BRD4 1057 | chr19: 15247462-15247489 | BRD4 2380 | chr19: 15332585-15332612 |
| BRD4 1058 | chr19: 15247467-15247494 | BRD4 2381 | chr19: 15332597-15332624 |
| BRD4 1059 | chr19: 15247468-15247495 | BRD4 2382 | chr19: 15332598-15332625 |
| BRD4 1060 | chr19: 15247469-15247496 | BRD4 2383 | chr19: 15332599-15332626 |
| BRD4 1061 | chr19: 15247478-15247505 | BRD4 2384 | chr19: 15332602-15332629 |
| BRD4 1062 | chr19: 15247486-15247513 | BRD4 2385 | chr19: 15332603-15332630 |
| BRD4 1063 | chr19: 15247491-15247518 | BRD4 2386 | chr19: 15332604-15332631 |
| BRD4 1064 | chr19: 15247492-15247519 | BRD4 2387 | chr19: 15332605-15332632 |
| BRD4 1065 | chr19: 15247493-15247520 | BRD4 2388 | chr19: 15332610-15332637 |
| BRD4 1066 | chr19: 15247496-15247523 | BRD4 2389 | chr19: 15332611-15332638 |
| BRD4 1067 | chr19: 15247504-15247531 | BRD4 2390 | chr19: 15332614-15332641 |
| BRD4 1068 | chr19: 15247511-15247538 | BRD4 2391 | chr19: 15332622-15332649 |
| BRD4 1069 | chr19: 15247515-15247542 | BRD4 2392 | chr19: 15332629-15332656 |
| BRD4 1070 | chr19: 15247516-15247543 | BRD4 2393 | chr19: 15332630-15332657 |
| BRD4 1071 | chr19: 15247517-15247544 | BRD4 2394 | chr19: 15332631-15332658 |
| BRD4 1072 | chr19: 15247545-15247572 | BRD4 2395 | chr19: 15332632-15332659 |
| BRD4 1073 | chr19: 15247546-15247573 | BRD4 2396 | chr19: 15332641-15332668 |
| BRD4 1074 | chr19: 15247557-15247584 | BRD4 2397 | chr19: 15332648-15332675 |
| BRD4 1075 | chr19: 15247558-15247585 | BRD4 2398 | chr19: 15332649-15332676 |
| BRD4 1076 | chr19: 15247559-15247586 | BRD4 2399 | chr19: 15332652-15332679 |
| BRD4 1077 | chr19: 15247560-15247587 | BRD4 2400 | chr19: 15332663-15332690 |
| BRD4 1078 | chr19: 15247571-15247598 | BRD4 2401 | chr19: 15332671-15332698 |
| BRD4 1079 | chr19: 15247582-15247609 | BRD4 2402 | chr19: 15332676-15332703 |
| BRD4 1080 | chr19: 15247590-15247617 | BRD4 2403 | chr19: 15332677-15332704 |
| BRD4 1081 | chr19: 15247599-15247626 | BRD4 2404 | chr19: 15332681-15332708 |
| BRD4 1082 | chr19: 15247600-15247627 | BRD4 2405 | chr19: 15332682-15332709 |
| BRD4 1083 | chr19: 15247601-15247628 | BRD4 2406 | chr19: 15332683-15332710 |
| BRD4 1084 | chr19: 15247621-15247648 | BRD4 2407 | chr19: 15332684-15332711 |
| BRD4 1085 | chr19: 15247630-15247657 | BRD4 2408 | chr19: 15332685-15332712 |
| BRD4 1086 | chr19: 15247648-15247675 | BRD4 2409 | chr19: 15332690-15332717 |
| BRD4 1087 | chr19: 15247649-15247676 | BRD4 2410 | chr19: 15332691-15332718 |
| BRD4 1088 | chr19: 15247661-15247688 | BRD4 2411 | chr19: 15332694-15332721 |
| BRD4 1089 | chr19: 15247662-15247689 | BRD4 2412 | chr19: 15332702-15332729 |
| BRD4 1090 | chr19: 15247663-15247690 | BRD4 2413 | chr19: 15332706-15332733 |
| BRD4 1091 | chr19: 15247669-15247696 | BRD4 2414 | chr19: 15332707-15332734 |
| BRD4 1092 | chr19: 15247670-15247697 | BRD4 2415 | chr19: 15332714-15332741 |
| BRD4 1093 | chr19: 15247672-15247699 | BRD4 2416 | chr19: 15332715-15332742 |
| BRD4 1094 | chr19: 15247673-15247700 | BRD4 2417 | chr19: 15332729-15332756 |
| BRD4 1095 | chr19: 15247674-15247701 | BRD4 2418 | chr19: 15332735-15332762 |
| BRD4 1096 | chr19: 15247678-15247705 | BRD4 2419 | chr19: 15332760-15332787 |
| BRD4 1097 | chr19: 15247682-15247709 | BRD4 2420 | chr19: 15332770-15332797 |
| BRD4 1098 | chr19: 15247703-15247730 | BRD4 2421 | chr19: 15332782-15332809 |
| BRD4 1099 | chr19: 15247707-15247734 | BRD4 2422 | chr19: 15332783-15332810 |
| BRD4 1100 | chr19: 15247710-15247737 | BRD4 2423 | chr19: 15332786-15332813 |
| BRD4 1101 | chr19: 15247711-15247738 | BRD4 2424 | chr19: 15332787-15332814 |
| BRD4 1102 | chr19: 15247714-15247741 | BRD4 2425 | chr19: 15332790-15332817 |
| BRD4 1103 | chr19: 15247720-15247747 | BRD4 2426 | chr19: 15332794-15332821 |
| BRD4 1104 | chr19: 15247722-15247749 | BRD4 2427 | chr19: 15332799-15332826 |
| BRD4 1105 | chr19: 15247733-15247760 | BRD4 2428 | chr19: 15332800-15332827 |
| BRD4 1106 | chr19: 15247739-15247766 | BRD4 2429 | chr19: 15332801-15332828 |
| BRD4 1107 | chr19: 15247746-15247773 | BRD4 2430 | chr19: 15332802-15332829 |
| BRD4 1108 | chr19: 15247747-15247774 | BRD4 2431 | chr19: 15332803-15332830 |
| BRD4 1109 | chr19: 15247767-15247794 | BRD4 2432 | chr19: 15332820-15332847 |
| BRD4 1110 | chr19: 15247769-15247796 | BRD4 2433 | chr19: 15332823-15332850 |
| BRD4 1111 | chr19: 15247787-15247814 | BRD4 2434 | chr19: 15332826-15332853 |
| BRD4 1112 | chr19: 15247794-15247821 | BRD4 2435 | chr19: 15332829-15332856 |
| BRD4 1113 | chr19: 15247807-15247834 | BRD4 2436 | chr19: 15332832-15332859 |
| BRD4 1114 | chr19: 15247824-15247851 | BRD4 2437 | chr19: 15332833-15332860 |
| BRD4 1115 | chr19: 15247825-15247852 | BRD4 2438 | chr19: 15332834-15332861 |
| BRD4 1116 | chr19: 15247826-15247853 | BRD4 2439 | chr19: 15332846-15332873 |
| BRD4 1117 | chr19: 15247846-15247873 | BRD4 2440 | chr19: 15332847-15332874 |
| BRD4 1118 | chr19: 15247847-15247874 | BRD4 2441 | chr19: 15332848-15332875 |
| BRD4 1119 | chr19: 15247853-15247880 | BRD4 2442 | chr19: 15332849-15332876 |
| BRD4 1120 | chr19: 15247854-15247881 | BRD4 2443 | chr19: 15332850-15332877 |
| BRD4 1121 | chr19: 15247856-15247883 | BRD4 2444 | chr19: 15332858-15332885 |
| BRD4 1122 | chr19: 15247857-15247884 | BRD4 2445 | chr19: 15332862-15332889 |
| BRD4 1123 | chr19: 15247863-15247890 | BRD4 2446 | chr19: 15332863-15332890 |
| BRD4 1124 | chr19: 15247868-15247895 | BRD4 2447 | chr19: 15332866-15332893 |
| BRD4 1125 | chr19: 15247873-15247900 | BRD4 2448 | chr19: 15332867-15332894 |
| BRD4 1126 | chr19: 15247874-15247901 | BRD4 2449 | chr19: 15332872-15332899 |
| BRD4 1127 | chr19: 15247875-15247902 | BRD4 2450 | chr19: 15332879-15332906 |
| BRD4 1128 | chr19: 15247876-15247903 | BRD4 2451 | chr19: 15332880-15332907 |
| BRD4 1129 | chr19: 15247877-15247904 | BRD4 2452 | chr19: 15332881-15332908 |
| BRD4 1130 | chr19: 15247885-15247912 | BRD4 2453 | chr19: 15332889-15332916 |
| BRD4 1131 | chr19: 15247886-15247913 | BRD4 2454 | chr19: 15332890-15332917 |
| BRD4 1132 | chr19: 15247896-15247923 | BRD4 2455 | chr19: 15332891-15332918 |
| BRD4 1133 | chr19: 15247902-15247929 | BRD4 2456 | chr19: 15332892-15332919 |
| BRD4 1134 | chr19: 15247903-15247930 | BRD4 2457 | chr19: 15332898-15332925 |
| BRD4 1135 | chr19: 15247904-15247931 | BRD4 2458 | chr19: 15332899-15332926 |
| BRD4 1136 | chr19: 15247906-15247933 | BRD4 2459 | chr19: 15332900-15332927 |
| BRD4 1137 | chr19: 15247907-15247934 | BRD4 2460 | chr19: 15332904-15332931 |
| BRD4 1138 | chr19: 15247908-15247935 | BRD4 2461 | chr19: 15332905-15332932 |
| BRD4 1139 | chr19: 15247915-15247942 | BRD4 2462 | chr19: 15332906-15332933 |
| BRD4 1140 | chr19: 15247929-15247956 | BRD4 2463 | chr19: 15332914-15332941 |
| BRD4 1141 | chr19: 15247937-15247964 | BRD4 2464 | chr19: 15332917-15332944 |
| BRD4 1142 | chr19: 15247950-15247977 | BRD4 2465 | chr19: 15332922-15332949 |
| BRD4 1143 | chr19: 15247951-15247978 | BRD4 2466 | chr19: 15332925-15332952 |
| BRD4 1144 | chr19: 15247958-15247985 | BRD4 2467 | chr19: 15332932-15332959 |
| BRD4 1145 | chr19: 15247962-15247989 | BRD4 2468 | chr19: 15332935-15332962 |
| BRD4 1146 | chr19: 15247965-15247992 | BRD4 2469 | chr19: 15332947-15332974 |
| BRD4 1147 | chr19: 15247966-15247993 | BRD4 2470 | chr19: 15332953-15332980 |
| BRD4 1148 | chr19: 15247967-15247994 | BRD4 2471 | chr19: 15332956-15332983 |
| BRD4 1149 | chr19: 15247972-15247999 | BRD4 2472 | chr19: 15332967-15332994 |
| BRD4 1150 | chr19: 15247980-15248007 | BRD4 2473 | chr19: 15332975-15333002 |
| BRD4 1151 | chr19: 15247981-15248008 | BRD4 2474 | chr19: 15332977-15333004 |
| BRD4 1152 | chr19: 15247982-15248009 | BRD4 2475 | chr19: 15332978-15333005 |
| BRD4 1153 | chr19: 15247983-15248010 | BRD4 2476 | chr19: 15332982-15333009 |
| BRD4 1154 | chr19: 15247994-15248021 | BRD4 2477 | chr19: 15332983-15333010 |
| BRD4 1155 | chr19: 15247997-15248024 | BRD4 2478 | chr19: 15332986-15333013 |
| BRD4 1156 | chr19: 15248003-15248030 | BRD4 2479 | chr19: 15332989-15333016 |
| BRD4 1157 | chr19: 15248004-15248031 | BRD4 2480 | chr19: 15332998-15333025 |
| BRD4 1158 | chr19: 15248015-15248042 | BRD4 2481 | chr19: 15332999-15333026 |
| BRD4 1159 | chr19: 15248016-15248043 | BRD4 2482 | chr19: 15333000-15333027 |
| BRD4 1160 | chr19: 15248024-15248051 | BRD4 2483 | chr19: 15333019-15333046 |
| BRD4 1161 | chr19: 15248028-15248055 | BRD4 2484 | chr19: 15333026-15333053 |
| BRD4 1162 | chr19: 15248029-15248056 | BRD4 2485 | chr19: 15333035-15333062 |
| BRD4 1163 | chr19: 15248035-15248062 | BRD4 2486 | chr19: 15333036-15333063 |
| BRD4 1164 | chr19: 15248036-15248063 | BRD4 2487 | chr19: 15333037-15333064 |
| BRD4 1165 | chr19: 15248041-15248068 | BRD4 2488 | chr19: 15333038-15333065 |
| BRD4 1166 | chr19: 15248056-15248083 | BRD4 2489 | chr19: 15333039-15333066 |
| BRD4 1167 | chr19: 15248066-15248093 | BRD4 2490 | chr19: 15333050-15333077 |
| BRD4 1168 | chr19: 15248069-15248096 | BRD4 2491 | chr19: 15333051-15333078 |
| BRD4 1169 | chr19: 15248070-15248097 | BRD4 2492 | chr19: 15333054-15333081 |
| BRD4 1170 | chr19: 15248109-15248136 | BRD4 2493 | chr19: 15333057-15333084 |
| BRD4 1171 | chr19: 15248110-15248137 | BRD4 2494 | chr19: 15333058-15333085 |
| BRD4 1172 | chr19: 15248111-15248138 | BRD4 2495 | chr19: 15333059-15333086 |
| BRD4 1173 | chr19: 15248112-15248139 | BRD4 2496 | chr19: 15333062-15333089 |
| BRD4 1174 | chr19: 15248127-15248154 | BRD4 2497 | chr19: 15333076-15333103 |
| BRD4 1175 | chr19: 15248131-15248158 | BRD4 2498 | chr19: 15333080-15333107 |
| BRD4 1176 | chr19: 15248149-15248176 | BRD4 2499 | chr19: 15333096-15333123 |
| BRD4 1177 | chr19: 15248167-15248194 | BRD4 2500 | chr19: 15333097-15333124 |
| BRD4 1178 | chr19: 15248168-15248195 | BRD4 2501 | chr19: 15333098-15333125 |
| BRD4 1179 | chr19: 15248172-15248199 | BRD4 2502 | chr19: 15333099-15333126 |
| BRD4 1180 | chr19: 15248185-15248212 | BRD4 2503 | chr19: 15333125-15333152 |
| BRD4 1181 | chr19: 15248212-15248239 | BRD4 2504 | chr19: 15333139-15333166 |
| BRD4 1182 | chr19: 15248213-15248240 | BRD4 2505 | chr19: 15333145-15333172 |
| BRD4 1183 | chr19: 15248216-15248243 | BRD4 2506 | chr19: 15333153-15333180 |
| BRD4 1184 | chr19: 15248217-15248244 | BRD4 2507 | chr19: 15333164-15333191 |
| BRD4 1185 | chr19: 15248222-15248249 | BRD4 2508 | chr19: 15333165-15333192 |
| BRD4 1186 | chr19: 15248223-15248250 | BRD4 2509 | chr19: 15333166-15333193 |
| BRD4 1187 | chr19: 15248231-15248258 | BRD4 2510 | chr19: 15333167-15333194 |
| BRD4 1188 | chr19: 15248257-15248284 | BRD4 2511 | chr19: 15333174-15333201 |
| BRD4 1189 | chr19: 15248258-15248285 | BRD4 2512 | chr19: 15333177-15333204 |
| BRD4 1190 | chr19: 15248259-15248286 | BRD4 2513 | chr19: 15333180-15333207 |
| BRD4 1191 | chr19: 15248268-15248295 | BRD4 2514 | chr19: 15333181-15333208 |
| BRD4 1192 | chr19: 15248272-15248299 | BRD4 2515 | chr19: 15333208-15333235 |
| BRD4 1193 | chr19: 15248273-15248300 | BRD4 2516 | chr19: 15333211-15333238 |
| BRD4 1194 | chr19: 15248274-15248301 | BRD4 2517 | chr19: 15333216-15333243 |
| BRD4 1195 | chr19: 15248276-15248303 | BRD4 2518 | chr19: 15333217-15333244 |
| BRD4 1196 | chr19: 15248277-15248304 | BRD4 2519 | chr19: 15333232-15333259 |
| BRD4 1197 | chr19: 15248278-15248305 | BRD4 2520 | chr19: 15333237-15333264 |
| BRD4 1198 | chr19: 15248279-15248306 | BRD4 2521 | chr19: 15333241-15333268 |
| BRD4 1199 | chr19: 15248280-15248307 | BRD4 2522 | chr19: 15333255-15333282 |
| BRD4 1200 | chr19: 15248288-15248315 | BRD4 2523 | chr19: 15333260-15333287 |
| BRD4 1201 | chr19: 15248289-15248316 | BRD4 2524 | chr19: 15333278-15333305 |
| BRD4 1202 | chr19: 15248290-15248317 | BRD4 2525 | chr19: 15333294-15333321 |
| BRD4 1203 | chr19: 15248322-15248349 | BRD4 2526 | chr19: 15333297-15333324 |
| BRD4 1204 | chr19: 15248331-15248358 | BRD4 2527 | chr19: 15333301-15333328 |
| BRD4 1205 | chr19: 15248332-15248359 | BRD4 2528 | chr19: 15333304-15333331 |
| BRD4 1206 | chr19: 15248333-15248360 | BRD4 2529 | chr19: 15333307-15333334 |
| BRD4 1207 | chr19: 15248339-15248366 | BRD4 2530 | chr19: 15333310-15333337 |
| BRD4 1208 | chr19: 15248345-15248372 | BRD4 2531 | chr19: 15333311-15333338 |
| BRD4 1209 | chr19: 15248346-15248373 | BRD4 2532 | chr19: 15333338-15333365 |
| BRD4 1210 | chr19: 15248353-15248380 | BRD4 2533 | chr19: 15333341-15333368 |
| BRD4 1211 | chr19: 15248365-15248392 | BRD4 2534 | chr19: 15333346-15333373 |
| BRD4 1212 | chr19: 15248366-15248393 | BRD4 2535 | chr19: 15333347-15333374 |
| BRD4 1213 | chr19: 15248383-15248410 | BRD4 2536 | chr19: 15333353-15333380 |
| BRD4 1214 | chr19: 15248384-15248411 | BRD4 2537 | chr19: 15333357-15333384 |
| BRD4 1215 | chr19: 15248394-15248421 | BRD4 2538 | chr19: 15333358-15333385 |
| BRD4 1216 | chr19: 15248395-15248422 | BRD4 2539 | chr19: 15333362-15333389 |
| BRD4 1217 | chr19: 15248397-15248424 | BRD4 2540 | chr19: 15333364-15333391 |
| BRD4 1218 | chr19: 15248398-15248425 | BRD4 2541 | chr19: 15333369-15333396 |
| BRD4 1219 | chr19: 15248401-15248428 | BRD4 2542 | chr19: 15333372-15333399 |
| BRD4 1220 | chr19: 15248402-15248429 | BRD4 2543 | chr19: 15333375-15333402 |
| BRD4 1221 | chr19: 15248405-15248432 | BRD4 2544 | chr19: 15333376-15333403 |
| BRD4 1222 | chr19: 15248406-15248433 | BRD4 2545 | chr19: 15333377-15333404 |
| BRD4 1223 | chr19: 15248407-15248434 | BRD4 2546 | chr19: 15333387-15333414 |
| BRD4 1224 | chr19: 15248411-15248438 | BRD4 2547 | chr19: 15333399-15333426 |
| BRD4 1225 | chr19: 15248412-15248439 | BRD4 2548 | chr19: 15333400-15333427 |
| BRD4 1226 | chr19: 15248413-15248440 | BRD4 2549 | chr19: 15333403-15333430 |
| BRD4 1227 | chr19: 15248415-15248442 | BRD4 2550 | chr19: 15333406-15333433 |
| BRD4 1228 | chr19: 15248416-15248443 | BRD4 2551 | chr19: 15333409-15333436 |
| BRD4 1229 | chr19: 15248417-15248444 | BRD4 2552 | chr19: 15333413-15333440 |
| BRD4 1230 | chr19: 15248421-15248448 | BRD4 2553 | chr19: 15333416-15333443 |
| BRD4 1231 | chr19: 15248422-15248449 | BRD4 2554 | chr19: 15333420-15333447 |
| BRD4 1232 | chr19: 15248423-15248450 | BRD4 2555 | chr19: 15333427-15333454 |
| BRD4 1233 | chr19: 15248424-15248451 | BRD4 2556 | chr19: 15333432-15333459 |
| BRD4 1234 | chr19: 15248425-15248452 | BRD4 2557 | chr19: 15333433-15333460 |
| BRD4 1235 | chr19: 15248433-15248460 | BRD4 2558 | chr19: 15333434-15333461 |
| BRD4 1236 | chr19: 15248440-15248467 | BRD4 2559 | chr19: 15333438-15333465 |
| BRD4 1237 | chr19: 15248461-15248488 | BRD4 2560 | chr19: 15333455-15333482 |
| BRD4 1238 | chr19: 15248462-15248489 | BRD4 2561 | chr19: 15333469-15333496 |
| BRD4 1239 | chr19: 15248466-15248493 | BRD4 2562 | chr19: 15333473-15333500 |
| BRD4 1240 | chr19: 15248468-15248495 | BRD4 2563 | chr19: 15333478-15333505 |
| BRD4 1241 | chr19: 15248469-15248496 | BRD4 2564 | chr19: 15333492-15333519 |
| BRD4 1242 | chr19: 15248475-15248502 | BRD4 2565 | chr19: 15333497-15333524 |
| BRD4 1243 | chr19: 15248481-15248508 | BRD4 2566 | chr19: 15333504-15333531 |
| BRD4 1244 | chr19: 15248485-15248512 | BRD4 2567 | chr19: 15333505-15333532 |
| BRD4 1245 | chr19: 15248486-15248513 | BRD4 2568 | chr19: 15333509-15333536 |
| BRD4 1246 | chr19: 15248493-15248520 | BRD4 2569 | chr19: 15333510-15333537 |
| BRD4 1247 | chr19: 15248495-15248522 | BRD4 2570 | chr19: 15333518-15333545 |
| BRD4 1248 | chr19: 15248496-15248523 | BRD4 2571 | chr19: 15333522-15333549 |
| BRD4 1249 | chr19: 15248509-15248536 | BRD4 2572 | chr19: 15333523-15333550 |
| BRD4 1250 | chr19: 15248516-15248543 | BRD4 2573 | chr19: 15333524-15333551 |
| BRD4 1251 | chr19: 15248525-15248552 | BRD4 2574 | chr19: 15333533-15333560 |
| BRD4 1252 | chr19: 15248528-15248555 | BRD4 2575 | chr19: 15333545-15333572 |
| BRD4 1253 | chr19: 15248530-15248557 | BRD4 2576 | chr19: 15333546-15333573 |
| BRD4 1254 | chr19: 15248531-15248558 | BRD4 2577 | chr19: 15333549-15333576 |
| BRD4 1255 | chr19: 15248541-15248568 | BRD4 2578 | chr19: 15333552-15333579 |
| BRD4 1256 | chr19: 15248542-15248569 | BRD4 2579 | chr19: 15333553-15333580 |
| BRD4 1257 | chr19: 15248543-15248570 | BRD4 2580 | chr19: 15333560-15333587 |
| BRD4 1258 | chr19: 15248556-15248583 | BRD4 2581 | chr19: 15333561-15333588 |
| BRD4 1259 | chr19: 15248557-15248584 | BRD4 2582 | chr19: 15333565-15333592 |
| BRD4 1260 | chr19: 15248573-15248600 | BRD4 2583 | chr19: 15333570-15333597 |
| BRD4 1261 | chr19: 15248576-15248603 | BRD4 2584 | chr19: 15333588-15333615 |
| BRD4 1262 | chr19: 15248577-15248604 | BRD4 2585 | chr19: 15333597-15333624 |
| BRD4 1263 | chr19: 15248580-15248607 | BRD4 2586 | chr19: 15333607-15333634 |
| BRD4 1264 | chr19: 15248583-15248610 | BRD4 2587 | chr19: 15333611-15333638 |
| BRD4 1265 | chr19: 15248584-15248611 | BRD4 2588 | chr19: 15333630-15333657 |
| BRD4 1266 | chr19: 15248592-15248619 | BRD4 2589 | chr19: 15333631-15333658 |
| BRD4 1267 | chr19: 15248601-15248628 | BRD4 2590 | chr19: 15333632-15333659 |
| BRD4 1268 | chr19: 15248604-15248631 | BRD4 2591 | chr19: 15333637-15333664 |
| BRD4 1269 | chr19: 15248619-15248646 | BRD4 2592 | chr19: 15333638-15333665 |
| BRD4 1270 | chr19: 15248633-15248660 | BRD4 2593 | chr19: 15333643-15333670 |
| BRD4 1271 | chr19: 15248636-15248663 | BRD4 2594 | chr19: 15333646-15333673 |
| BRD4 1272 | chr19: 15248639-15248666 | BRD4 2595 | chr19: 15333649-15333676 |
| BRD4 1273 | chr19: 15248651-15248678 | BRD4 2596 | chr19: 15333650-15333677 |
| BRD4 1274 | chr19: 15248652-15248679 | BRD4 2597 | chr19: 15333661-15333688 |
| BRD4 1275 | chr19: 15248670-15248697 | BRD4 2598 | chr19: 15333680-15333707 |
| BRD4 1276 | chr19: 15248675-15248702 | BRD4 2599 | chr19: 15333683-15333710 |
| BRD4 1277 | chr19: 15248676-15248703 | BRD4 2600 | chr19: 15333687-15333714 |
| BRD4 1278 | chr19: 15248682-15248709 | BRD4 2601 | chr19: 15333688-15333715 |
| BRD4 1279 | chr19: 15248700-15248727 | BRD4 2602 | chr19: 15333689-15333716 |
| BRD4 1280 | chr19: 15248701-15248728 | BRD4 2603 | chr19: 15333694-15333721 |
| BRD4 1281 | chr19: 15248709-15248736 | BRD4 2604 | chr19: 15333705-15333732 |
| BRD4 1282 | chr19: 15248719-15248746 | BRD4 2605 | chr19: 15333706-15333733 |
| BRD4 1283 | chr19: 15248723-15248750 | BRD4 2606 | chr19: 15333709-15333736 |
| BRD4 1284 | chr19: 15248737-15248764 | BRD4 2607 | chr19: 15333712-15333739 |
| BRD4 1285 | chr19: 15248742-15248769 | BRD4 2608 | chr19: 15333728-15333755 |
| BRD4 1286 | chr19: 15248753-15248780 | BRD4 2609 | chr19: 15333729-15333756 |
| BRD4 1287 | chr19: 15248754-15248781 | BRD4 2610 | chr19: 15333764-15333791 |
| BRD4 1288 | chr19: 15248755-15248782 | BRD4 2611 | chr19: 15333765-15333792 |
| BRD4 1289 | chr19: 15248759-15248786 | BRD4 2612 | chr19: 15333766-15333793 |
| BRD4 1290 | chr19: 15248795-15248822 | BRD4 2613 | chr19: 15333774-15333801 |
| BRD4 1291 | chr19: 15248796-15248823 | BRD4 2614 | chr19: 15333778-15333805 |
| BRD4 1292 | chr19: 15248813-15248840 | BRD4 2615 | chr19: 15333799-15333826 |
| BRD4 1293 | chr19: 15248818-15248845 | BRD4 2616 | chr19: 15333804-15333831 |
| BRD4 1294 | chr19: 15248819-15248846 | BRD4 2617 | chr19: 15333805-15333832 |
| BRD4 1295 | chr19: 15248822-15248849 | BRD4 2618 | chr19: 15333810-15333837 |
| BRD4 1296 | chr19: 15248823-15248850 | BRD4 2619 | chr19: 15333813-15333840 |
| BRD4 1297 | chr19: 15248834-15248861 | BRD4 2620 | chr19: 15333828-15333855 |
| BRD4 1298 | chr19: 15248835-15248862 | BRD4 2621 | chr19: 15333847-15333874 |
| BRD4 1299 | chr19: 15248857-15248884 | BRD4 2622 | chr19: 15333855-15333882 |
| BRD4 1300 | chr19: 15248858-15248885 | BRD4 2623 | chr19: 15333856-15333883 |
| BRD4 1301 | chr19: 15248862-15248889 | BRD4 2624 | chr19: 15333858-15333885 |
| BRD4 1302 | chr19: 15248863-15248890 | BRD4 2625 | chr19: 15333861-15333888 |
| BRD4 1303 | chr19: 15248864-15248891 | BRD4 2626 | chr19: 15333864-15333891 |
| BRD4 1304 | chr19: 15248871-15248898 | BRD4 2627 | chr19: 15333870-15333897 |
| BRD4 1305 | chr19: 15248872-15248899 | BRD4 2628 | chr19: 15333886-15333913 |
| BRD4 1306 | chr19: 15248886-15248913 | BRD4 2629 | chr19: 15333887-15333914 |
| BRD4 1307 | chr19: 15248887-15248914 | BRD4 2630 | chr19: 15333890-15333917 |
| BRD4 1308 | chr19: 15248888-15248915 | BRD4 2631 | chr19: 15333896-15333923 |
| BRD4 1309 | chr19: 15248893-15248920 | BRD4 2632 | chr19: 15333909-15333936 |
| BRD4 1310 | chr19: 15248933-15248960 | BRD4 2633 | chr19: 15333910-15333937 |
| BRD4 1311 | chr19: 15248934-15248961 | BRD4 2634 | chr19: 15333934-15333961 |
| BRD4 1312 | chr19: 15248935-15248962 | BRD4 2635 | chr19: 15333936-15333963 |
| BRD4 1313 | chr19: 15248940-15248967 | BRD4 2636 | chr19: 15333937-15333964 |
| BRD4 1314 | chr19: 15248954-15248981 | BRD4 2637 | chr19: 15333945-15333972 |
| BRD4 1315 | chr19: 15248973-15249000 | BRD4 2638 | chr19: 15333950-15333977 |
| BRD4 1316 | chr19: 15248974-15249001 | BRD4 2639 | chr19: 15333959-15333986 |
| BRD4 1317 | chr19: 15248977-15249004 | BRD4 2640 | chr19: 15333986-15334013 |
| BRD4 1318 | chr19: 15248987-15249014 | BRD4 2641 | chr19: 15333997-15334024 |
| BRD4 1319 | chr19: 15248990-15249017 | BRD4 2642 | chr19: 15334007-15334034 |
| BRD4 1320 | chr19: 15248991-15249018 | BRD4 2643 | chr19: 15334008-15334035 |
| BRD4 1321 | chr19: 15248994-15249021 | BRD4 2644 | chr19: 15334020-15334047 |
| BRD4 1322 | chr19: 15248995-15249022 | BRD4 2645 | chr19: 15334034-15334061 |
| BRD4 1323 | chr19: 15248998-15249025 | | |

**Tabel 2F. Genomic Coordinates of Human IKZF1 in the Current Invention**

| Index | Chromosomal coordinate range | Index | Chromosomal coordinate range |
|---|---|---|---|
| IKZF1 1 | chr7:50301937-50301964 | IKZF1 1612 | chr7:50360207-50360234 |
| IKZF1 2 | chr7:50301962-50301989 | IKZF1 1613 | chr7:50360208-50360235 |
| IKZF1 3 | chr7:50301979-50302006 | IKZF1 1614 | chr7:50360214-50360241 |
| IKZF1 4 | chr7:50301980-50302007 | IKZF1 1615 | chr7:50360215-50360242 |
| IKZF1 5 | chr7:50301984-50302011 | IKZF1 1616 | chr7:50360217-50360244 |
| IKZF1 6 | chr7:50301992-50302019 | IKZF1 1617 | chr7:50360218-50360245 |
| IKZF1 7 | chr7:50301999-50302026 | IKZF1 1618 | chr7:50360226-50360253 |
| IKZF1 8 | chr7:50302000-50302027 | IKZF1 1619 | chr7:50360230-50360257 |
| IKZF1 9 | chr7:50302007-50302034 | IKZF1 1620 | chr7:50360247-50360274 |
| IKZF1 10 | chr7:50302011-50302038 | IKZF1 1621 | chr7:50360248-50360275 |
| IKZF1 11 | chr7:50302012-50302039 | IKZF1 1622 | chr7:50360249-50360276 |
| IKZF1 12 | chr7:50302013-50302040 | IKZF1 1623 | chr7:50360257-50360284 |
| IKZF1 13 | chr7:50302020-50302047 | IKZF1 1624 | chr7:50360258-50360285 |
| IKZF1 14 | chr7:50302021-50302048 | IKZF1 1625 | chr7:50360260-50360287 |
| IKZF1 15 | chr7:50302040-50302067 | IKZF1 1626 | chr7:50360261-50360288 |
| IKZF1 16 | chr7:50302061-50302088 | IKZF1 1627 | chr7:50360263-50360290 |
| IKZF1 17 | chr7:50302068-50302095 | IKZF1 1628 | chr7:50360265-50360292 |
| IKZF1 18 | chr7:50302073-50302100 | IKZF1 1629 | chr7:50360269-50360296 |
| IKZF1 19 | chr7:50302076-50302103 | IKZF1 1630 | chr7:50360270-50360297 |
| IKZF1 20 | chr7:50302105-50302132 | IKZF1 1631 | chr7:50360288-50360315 |
| IKZF1 21 | chr7:50302117-50302144 | IKZF1 1632 | chr7:50360291-50360318 |
| IKZF1 22 | chr7:50302118-50302145 | IKZF1 1633 | chr7:50360309-50360336 |
| IKZF1 23 | chr7:50302120-50302147 | IKZF1 1634 | chr7:50360331-50360358 |
| IKZF1 24 | chr7:50302121-50302148 | IKZF1 1635 | chr7:50360334-50360361 |
| IKZF1 25 | chr7:50302124-50302151 | IKZF1 1636 | chr7:50360335-50360362 |
| IKZF1 26 | chr7:50302125-50302152 | IKZF1 1637 | chr7:50360338-50360365 |
| IKZF1 27 | chr7:50302135-50302162 | IKZF1 1638 | chr7:50360345-50360372 |
| IKZF1 28 | chr7:50302136-50302163 | IKZF1 1639 | chr7:50360357-50360384 |
| IKZF1 29 | chr7:50302137-50302164 | IKZF1 1640 | chr7:50360373-50360400 |
| IKZF1 30 | chr7:50302140-50302167 | IKZF1 1641 | chr7:50360394-50360421 |
| IKZF1 31 | chr7:50302150-50302177 | IKZF1 1642 | chr7:50360400-50360427 |
| IKZF1 32 | chr7:50302151-50302178 | IKZF1 1643 | chr7:50360401-50360428 |
| IKZF1 33 | chr7:50302152-50302179 | IKZF1 1644 | chr7:50360447-50360474 |
| IKZF1 34 | chr7:50302160-50302187 | IKZF1 1645 | chr7:50360453-50360480 |
| IKZF1 35 | chr7:50302161-50302188 | IKZF1 1646 | chr7:50360469-50360496 |
| IKZF1 36 | chr7:50302163-50302190 | IKZF1 1647 | chr7:50360485-50360512 |
| IKZF1 37 | chr7:50302177-50302204 | IKZF1 1648 | chr7:50360486-50360513 |
| IKZF1 38 | chr7:50302206-50302233 | IKZF1 1649 | chr7:50360487-50360514 |
| IKZF1 39 | chr7:50302211-50302238 | IKZF1 1650 | chr7:50360500-50360527 |
| IKZF1 40 | chr7:50302212-50302239 | IKZF1 1651 | chr7:50360506-50360533 |
| IKZF1 41 | chr7:50302225-50302252 | IKZF1 1652 | chr7:50360529-50360556 |
| IKZF1 42 | chr7:50302226-50302253 | IKZF1 1653 | chr7:50360544-50360571 |
| IKZF1 43 | chr7:50302243-50302270 | IKZF1 1654 | chr7:50360548-50360575 |
| IKZF1 44 | chr7:50302244-50302271 | IKZF1 1655 | chr7:50360555-50360582 |
| IKZF1 45 | chr7:50302247-50302274 | IKZF1 1656 | chr7:50360562-50360589 |
| IKZF1 46 | chr7:50302248-50302275 | IKZF1 1657 | chr7:50360563-50360590 |
| IKZF1 47 | chr7:50302249-50302276 | IKZF1 1658 | chr7:50360572-50360599 |
| IKZF1 48 | chr7:50302255-50302282 | IKZF1 1659 | chr7:50360590-50360617 |
| IKZF1 49 | chr7:50302258-50302285 | IKZF1 1660 | chr7:50360592-50360619 |
| IKZF1 50 | chr7:50302262-50302289 | IKZF1 1661 | chr7:50360593-50360620 |
| IKZF1 51 | chr7:50302274-50302301 | IKZF1 1662 | chr7:50360625-50360652 |
| IKZF1 52 | chr7:50302277-50302304 | IKZF1 1663 | chr7:50360660-50360687 |
| IKZF1 53 | chr7:50302282-50302309 | IKZF1 1664 | chr7:50360674-50360701 |
| IKZF1 54 | chr7:50302290-50302317 | IKZF1 1665 | chr7:50360694-50360721 |
| IKZF1 55 | chr7:50302302-50302329 | IKZF1 1666 | chr7:50360702-50360729 |
| IKZF1 56 | chr7:50302303-50302330 | IKZF1 1667 | chr7:50360703-50360730 |
| IKZF1 57 | chr7:50302308-50302335 | IKZF1 1668 | chr7:50360729-50360756 |
| IKZF1 58 | chr7:50302311-50302338 | IKZF1 1669 | chr7:50360736-50360763 |
| IKZF1 59 | chr7:50302316-50302343 | IKZF1 1670 | chr7:50360742-50360769 |
| IKZF1 60 | chr7:50302324-50302351 | IKZF1 1671 | chr7:50360743-50360770 |
| IKZF1 61 | chr7:50302329-50302356 | IKZF1 1672 | chr7:50360744-50360771 |
| IKZF1 62 | chr7:50302336-50302363 | IKZF1 1673 | chr7:50360753-50360780 |
| IKZF1 63 | chr7:50302350-50302377 | IKZF1 1674 | chr7:50360754-50360781 |
| IKZF1 64 | chr7:50302361-50302388 | IKZF1 1675 | chr7:50360758-50360785 |
| IKZF1 65 | chr7:50302375-50302402 | IKZF1 1676 | chr7:50360765-50360792 |
| IKZF1 66 | chr7:50302391-50302418 | IKZF1 1677 | chr7:50360766-50360793 |
| IKZF1 67 | chr7:50302392-50302419 | IKZF1 1678 | chr7:50360769-50360796 |
| IKZF1 68 | chr7:50302393-50302420 | IKZF1 1679 | chr7:50360770-50360797 |
| IKZF1 69 | chr7:50302399-50302426 | IKZF1 1680 | chr7:50360775-50360802 |
| IKZF1 70 | chr7:50302400-50302427 | IKZF1 1681 | chr7:50360780-50360807 |
| IKZF1 71 | chr7:50302401-50302428 | IKZF1 1682 | chr7:50360785-50360812 |
| IKZF1 72 | chr7:50302402-50302429 | IKZF1 1683 | chr7:50360795-50360822 |
| IKZF1 73 | chr7:50302403-50302430 | IKZF1 1684 | chr7:50360805-50360832 |
| IKZF1 74 | chr7:50302419-50302446 | IKZF1 1685 | chr7:50360815-50360842 |
| IKZF1 75 | chr7:50302422-50302449 | IKZF1 1686 | chr7:50360828-50360855 |
| IKZF1 76 | chr7:50302423-50302450 | IKZF1 1687 | chr7:50360829-50360856 |
| IKZF1 77 | chr7:50302431-50302458 | IKZF1 1688 | chr7:50360830-50360857 |
| IKZF1 78 | chr7:50302438-50302465 | IKZF1 1689 | chr7:50360831-50360858 |
| IKZF1 79 | chr7:50302440-50302467 | IKZF1 1690 | chr7:50360838-50360865 |
| IKZF1 80 | chr7:50302445-50302472 | IKZF1 1691 | chr7:50360839-50360866 |
| IKZF1 81 | chr7:50302452-50302479 | IKZF1 1692 | chr7:50360840-50360867 |
| IKZF1 82 | chr7:50302455-50302482 | IKZF1 1693 | chr7:50360842-50360869 |
| IKZF1 83 | chr7:50302460-50302487 | IKZF1 1694 | chr7:50360847-50360874 |
| IKZF1 84 | chr7:50302463-50302490 | IKZF1 1695 | chr7:50360849-50360876 |
| IKZF1 85 | chr7:50302487-50302514 | IKZF1 1696 | chr7:50360850-50360877 |
| IKZF1 86 | chr7:50302502-50302529 | IKZF1 1697 | chr7:50360859-50360886 |
| IKZF1 87 | chr7:50302509-50302536 | IKZF1 1698 | chr7:50360882-50360909 |
| IKZF1 88 | chr7:50302524-50302551 | IKZF1 1699 | chr7:50360893-50360920 |
| IKZF1 89 | chr7:50302535-50302562 | IKZF1 1700 | chr7:50360894-50360921 |
| IKZF1 90 | chr7:50302538-50302565 | IKZF1 1701 | chr7:50360895-50360922 |
| IKZF1 91 | chr7:50302541-50302568 | IKZF1 1702 | chr7:50360900-50360927 |
| IKZF1 92 | chr7:50302543-50302570 | IKZF1 1703 | chr7:50360901-50360928 |
| IKZF1 93 | chr7:50302545-50302572 | IKZF1 1704 | chr7:50360902-50360929 |
| IKZF1 94 | chr7:50302546-50302573 | IKZF1 1705 | chr7:50360905-50360932 |
| IKZF1 95 | chr7:50302552-50302579 | IKZF1 1706 | chr7:50360908-50360935 |
| IKZF1 96 | chr7:50302563-50302590 | IKZF1 1707 | chr7:50360909-50360936 |
| IKZF1 97 | chr7:50302590-50302617 | IKZF1 1708 | chr7:50360910-50360937 |
| IKZF1 98 | chr7:50302591-50302618 | IKZF1 1709 | chr7:50360913-50360940 |
| IKZF1 99 | chr7:50302592-50302619 | IKZF1 1710 | chr7:50360921-50360948 |
| IKZF1 100 | chr7:50302602-50302629 | IKZF1 1711 | chr7:50360922-50360949 |
| IKZF1 101 | chr7:50302606-50302633 | IKZF1 1712 | chr7:50360940-50360967 |
| IKZF1 102 | chr7:50302607-50302634 | IKZF1 1713 | chr7:50360941-50360968 |
| IKZF1 103 | chr7:50302608-50302635 | IKZF1 1714 | chr7:50360952-50360979 |
| IKZF1 104 | chr7:50302612-50302639 | IKZF1 1715 | chr7:50360961-50360988 |
| IKZF1 105 | chr7:50302630-50302657 | IKZF1 1716 | chr7:50360962-50360989 |
| IKZF1 106 | chr7:50302643-50302670 | IKZF1 1717 | chr7:50360966-50360993 |
| IKZF1 107 | chr7:50302650-50302677 | IKZF1 1718 | chr7:50360980-50361007 |
| IKZF1 108 | chr7:50302651-50302678 | IKZF1 1719 | chr7:50360981-50361008 |
| IKZF1 109 | chr7:50302662-50302689 | IKZF1 1720 | chr7:50360989-50361016 |
| IKZF1 110 | chr7:50302663-50302690 | IKZF1 1721 | chr7:50360996-50361023 |
| IKZF1 111 | chr7:50302684-50302711 | IKZF1 1722 | chr7:50361002-50361029 |
| IKZF1 112 | chr7:50302685-50302712 | IKZF1 1723 | chr7:50361003-50361030 |
| IKZF1 113 | chr7:50302694-50302721 | IKZF1 1724 | chr7:50361035-50361062 |
| IKZF1 114 | chr7:50302718-50302745 | IKZF1 1725 | chr7:50361064-50361091 |
| IKZF1 115 | chr7:50302719-50302746 | IKZF1 1726 | chr7:50361078-50361105 |
| IKZF1 116 | chr7:50302725-50302752 | IKZF1 1727 | chr7:50361079-50361106 |
| IKZF1 117 | chr7:50302726-50302753 | IKZF1 1728 | chr7:50361080-50361107 |
| IKZF1 118 | chr7:50302740-50302767 | IKZF1 1729 | chr7:50361084-50361111 |
| IKZF1 119 | chr7:50302749-50302776 | IKZF1 1730 | chr7:50361091-50361118 |
| IKZF1 120 | chr7:50302754-50302781 | IKZF1 1731 | chr7:50361092-50361119 |
| IKZF1 121 | chr7:50302760-50302787 | IKZF1 1732 | chr7:50361103-50361130 |
| IKZF1 122 | chr7:50302761-50302788 | IKZF1 1733 | chr7:50361107-50361134 |
| IKZF1 123 | chr7:50302765-50302792 | IKZF1 1734 | chr7:50361124-50361151 |
| IKZF1 124 | chr7:50302775-50302802 | IKZF1 1735 | chr7:50361127-50361154 |
| IKZF1 125 | chr7:50302782-50302809 | IKZF1 1736 | chr7:50361131-50361158 |
| IKZF1 126 | chr7:50302787-50302814 | IKZF1 1737 | chr7:50361132-50361159 |
| IKZF1 127 | chr7:50302790-50302817 | IKZF1 1738 | chr7:50361150-50361177 |
| IKZF1 128 | chr7:50302797-50302824 | IKZF1 1739 | chr7:50361163-50361190 |
| IKZF1 129 | chr7:50302798-50302825 | IKZF1 1740 | chr7:50361172-50361199 |
| IKZF1 130 | chr7:50302799-50302826 | IKZF1 1741 | chr7:50361173-50361200 |
| IKZF1 131 | chr7:50302803-50302830 | IKZF1 1742 | chr7:50361178-50361205 |
| IKZF1 132 | chr7:50302804-50302831 | IKZF1 1743 | chr7:50361179-50361206 |
| IKZF1 133 | chr7:50302805-50302832 | IKZF1 1744 | chr7:50361185-50361212 |
| IKZF1 134 | chr7:50302806-50302833 | IKZF1 1745 | chr7:50361201-50361228 |
| IKZF1 135 | chr7:50302818-50302845 | IKZF1 1746 | chr7:50361214-50361241 |
| IKZF1 136 | chr7:50302820-50302847 | IKZF1 1747 | chr7:50361215-50361242 |
| IKZF1 137 | chr7:50302821-50302848 | IKZF1 1748 | chr7:50361216-50361243 |
| IKZF1 138 | chr7:50302827-50302854 | IKZF1 1749 | chr7:50361254-50361281 |
| IKZF1 139 | chr7:50302829-50302856 | IKZF1 1750 | chr7:50361255-50361282 |
| IKZF1 140 | chr7:50302832-50302859 | IKZF1 1751 | chr7:50361256-50361283 |
| IKZF1 141 | chr7:50302838-50302865 | IKZF1 1752 | chr7:50361257-50361284 |
| IKZF1 142 | chr7:50302842-50302869 | IKZF1 1753 | chr7:50361262-50361289 |
| IKZF1 143 | chr7:50302843-50302870 | IKZF1 1754 | chr7:50361270-50361297 |
| IKZF1 144 | chr7:50302844-50302871 | IKZF1 1755 | chr7:50361300-50361327 |
| IKZF1 145 | chr7:50302858-50302885 | IKZF1 1756 | chr7:50361370-50361397 |
| IKZF1 146 | chr7:50302859-50302886 | IKZF1 1757 | chr7:50361375-50361402 |
| IKZF1 147 | chr7:50302860-50302887 | IKZF1 1758 | chr7:50361382-50361409 |
| IKZF1 148 | chr7:50302864-50302891 | IKZF1 1759 | chr7:50361383-50361410 |
| IKZF1 149 | chr7:50302877-50302904 | IKZF1 1760 | chr7:50361418-50361445 |
| IKZF1 150 | chr7:50302878-50302905 | IKZF1 1761 | chr7:50361419-50361446 |
| IKZF1 151 | chr7:50302886-50302913 | IKZF1 1762 | chr7:50361425-50361452 |
| IKZF1 152 | chr7:50302893-50302920 | IKZF1 1763 | chr7:50361437-50361464 |
| IKZF1 153 | chr7:50302903-50302930 | IKZF1 1764 | chr7:50361443-50361470 |
| IKZF1 154 | chr7:50302904-50302931 | IKZF1 1765 | chr7:50361452-50361479 |
| IKZF1 155 | chr7:50302907-50302934 | IKZF1 1766 | chr7:50361453-50361480 |
| IKZF1 156 | chr7:50302909-50302936 | IKZF1 1767 | chr7:50361454-50361481 |
| IKZF1 157 | chr7:50302910-50302937 | IKZF1 1768 | chr7:50361458-50361485 |
| IKZF1 158 | chr7:50302920-50302947 | IKZF1 1769 | chr7:50361465-50361492 |
| IKZF1 159 | chr7:50302923-50302950 | IKZF1 1770 | chr7:50361475-50361502 |
| IKZF1 160 | chr7:50302926-50302953 | IKZF1 1771 | chr7:50361480-50361507 |
| IKZF1 161 | chr7:50302927-50302954 | IKZF1 1772 | chr7:50361481-50361508 |
| IKZF1 162 | chr7:50302935-50302962 | IKZF1 1773 | chr7:50361482-50361509 |
| IKZF1 163 | chr7:50302941-50302968 | IKZF1 1774 | chr7:50361489-50361516 |
| IKZF1 164 | chr7:50302943-50302970 | IKZF1 1775 | chr7:50361490-50361517 |
| IKZF1 165 | chr7:50302946-50302973 | IKZF1 1776 | chr7:50361499-50361526 |
| IKZF1 166 | chr7:50302950-50302977 | IKZF1 1777 | chr7:50361508-50361535 |
| IKZF1 167 | chr7:50302955-50302982 | IKZF1 1778 | chr7:50361517-50361544 |
| IKZF1 168 | chr7:50302959-50302986 | IKZF1 1779 | chr7:50361524-50361551 |
| IKZF1 169 | chr7:50302963-50302990 | IKZF1 1780 | chr7:50361534-50361561 |
| IKZF1 170 | chr7:50302964-50302991 | IKZF1 1781 | chr7:50361538-50361565 |
| IKZF1 171 | chr7:50302976-50303003 | IKZF1 1782 | chr7:50361547-50361574 |
| IKZF1 172 | chr7:50302977-50303004 | IKZF1 1783 | chr7:50361568-50361595 |
| IKZF1 173 | chr7:50302978-50303005 | IKZF1 1784 | chr7:50361570-50361597 |
| IKZF1 174 | chr7:50302997-50303024 | IKZF1 1785 | chr7:50361575-50361602 |
| IKZF1 175 | chr7:50302998-50303025 | IKZF1 1786 | chr7:50361583-50361610 |
| IKZF1 176 | chr7:50303014-50303041 | IKZF1 1787 | chr7:50361593-50361620 |
| IKZF1 177 | chr7:50303018-50303045 | IKZF1 1788 | chr7:50361598-50361625 |
| IKZF1 178 | chr7:50303019-50303046 | IKZF1 1789 | chr7:50361610-50361637 |
| IKZF1 179 | chr7:50303024-50303051 | IKZF1 1790 | chr7:50361611-50361638 |
| IKZF1 180 | chr7:50303027-50303054 | IKZF1 1791 | chr7:50361614-50361641 |
| IKZF1 181 | chr7:50303032-50303059 | IKZF1 1792 | chr7:50361617-50361644 |
| IKZF1 182 | chr7:50303033-50303060 | IKZF1 1793 | chr7:50361620-50361647 |
| IKZF1 183 | chr7:50303040-50303067 | IKZF1 1794 | chr7:50361623-50361650 |
| IKZF1 184 | chr7:50303041-50303068 | IKZF1 1795 | chr7:50361624-50361651 |
| IKZF1 185 | chr7:50303055-50303082 | IKZF1 1796 | chr7:50361625-50361652 |
| IKZF1 186 | chr7:50303056-50303083 | IKZF1 1797 | chr7:50361626-50361653 |
| IKZF1 187 | chr7:50303057-50303084 | IKZF1 1798 | chr7:50361636-50361663 |
| IKZF1 188 | chr7:50303058-50303085 | IKZF1 1799 | chr7:50361642-50361669 |
| IKZF1 189 | chr7:50303059-50303086 | IKZF1 1800 | chr7:50361668-50361695 |
| IKZF1 190 | chr7:50303066-50303093 | IKZF1 1801 | chr7:50361678-50361705 |
| IKZF1 191 | chr7:50303067-50303094 | IKZF1 1802 | chr7:50361682-50361709 |
| IKZF1 192 | chr7:50303073-50303100 | IKZF1 1803 | chr7:50361687-50361714 |
| IKZF1 193 | chr7:50303076-50303103 | IKZF1 1804 | chr7:50361701-50361728 |
| IKZF1 194 | chr7:50303089-50303116 | IKZF1 1805 | chr7:50361702-50361729 |
| IKZF1 195 | chr7:50303090-50303117 | IKZF1 1806 | chr7:50361703-50361730 |
| IKZF1 196 | chr7:50303093-50303120 | IKZF1 1807 | chr7:50361707-50361734 |
| IKZF1 197 | chr7:50303108-50303135 | IKZF1 1808 | chr7:50361708-50361735 |
| IKZF1 198 | chr7:50303109-50303136 | IKZF1 1809 | chr7:50361712-50361739 |
| IKZF1 199 | chr7:50303112-50303139 | IKZF1 1810 | chr7:50361713-50361740 |
| IKZF1 200 | chr7:50303113-50303140 | IKZF1 1811 | chr7:50361716-50361743 |
| IKZF1 201 | chr7:50303117-50303144 | IKZF1 1812 | chr7:50361743-50361770 |
| IKZF1 202 | chr7:50303120-50303147 | IKZF1 1813 | chr7:50361744-50361771 |
| IKZF1 203 | chr7:50303123-50303150 | IKZF1 1814 | chr7:50361747-50361774 |
| IKZF1 204 | chr7:50303138-50303165 | IKZF1 1815 | chr7:50361750-50361777 |
| IKZF1 205 | chr7:50303139-50303166 | IKZF1 1816 | chr7:50361753-50361780 |
| IKZF1 206 | chr7:50303159-50303186 | IKZF1 1817 | chr7:50361757-50361784 |
| IKZF1 207 | chr7:50303162-50303189 | IKZF1 1818 | chr7:50361758-50361785 |
| IKZF1 208 | chr7:50303163-50303190 | IKZF1 1819 | chr7:50361759-50361786 |
| IKZF1 209 | chr7:50303168-50303195 | IKZF1 1820 | chr7:50361766-50361793 |
| IKZF1 210 | chr7:50303169-50303196 | IKZF1 1821 | chr7:50361767-50361794 |
| IKZF1 211 | chr7:50303170-50303197 | IKZF 1 1822 | chr7:50361775-50361802 |
| IKZF1 212 | chr7:50303171-50303198 | IKZF1 1823 | chr7:50361776-50361803 |
| IKZF1 213 | chr7:50303173-50303200 | IKZF1 1824 | chr7:50361779-50361806 |
| IKZF1 214 | chr7:50303174-50303201 | IKZF1 1825 | chr7:50361782-50361809 |
| IKZF1 215 | chr7:50303175-50303202 | IKZF1 1826 | chr7:50361785-50361812 |
| IKZF1 216 | chr7:50303182-50303209 | IKZF1 1827 | chr7:50361798-50361825 |
| IKZF1 217 | chr7:50303183-50303210 | IKZF1 1828 | chr7:50361799-50361826 |
| IKZF1 218 | chr7:50303193-50303220 | IKZF1 1829 | chr7:50361823-50361850 |
| IKZF1 219 | chr7:50303194-50303221 | IKZF1 1830 | chr7:50361825-50361852 |
| IKZF1 220 | chr7:50303228-50303255 | IKZF1 1831 | chr7:50361834-50361861 |
| IKZF1 221 | chr7:50303230-50303257 | IKZF1 1832 | chr7:50361844-50361871 |
| IKZF1 222 | chr7:50303239-50303266 | IKZF1 1833 | chr7:50361848-50361875 |
| IKZF1 223 | chr7:50303240-50303267 | IKZF1 1834 | chr7:50361868-50361895 |
| IKZF1 224 | chr7:50303243-50303270 | IKZF1 1835 | chr7:50361897-50361924 |
| IKZF1 225 | chr7:50303250-50303277 | IKZF1 1836 | chr7:50361898-50361925 |
| IKZF1 226 | chr7:50303258-50303285 | IKZF1 1837 | chr7:50361909-50361936 |
| IKZF1 227 | chr7:50303265-50303292 | IKZF1 1838 | chr7:50361922-50361949 |
| IKZF1 228 | chr7:50303266-50303293 | IKZF1 1839 | chr7:50361932-50361959 |
| IKZF1 229 | chr7:50303267-50303294 | IKZF1 1840 | chr7:50361955-50361982 |
| IKZF1 230 | chr7:50303273-50303300 | IKZF1 1841 | chr7:50361956-50361983 |
| IKZF1 231 | chr7:50303274-50303301 | IKZF1 1842 | chr7:50361957-50361984 |
| IKZF1 232 | chr7:50303278-50303305 | IKZF1 1843 | chr7:50361965-50361992 |
| IKZF1 233 | chr7:50303279-50303306 | IKZF1 1844 | chr7:50361966-50361993 |
| IKZF1 234 | chr7:50303285-50303312 | IKZF1 1845 | chr7:50361967-50361994 |
| IKZF1 235 | chr7:50303286-50303313 | IKZF1 1846 | chr7:50361976-50362003 |
| IKZF1 236 | chr7:50303287-50303314 | IKZF1 1847 | chr7:50362038-50362065 |
| IKZF1 237 | chr7:50303297-50303324 | IKZF1 1848 | chr7:50362043-50362070 |
| IKZF1 238 | chr7:50303298-50303325 | IKZF1 1849 | chr7:50362073-50362100 |
| IKZF1 239 | chr7:50303299-50303326 | IKZF1 1850 | chr7:50362074-50362101 |
| IKZF1 240 | chr7:50303304-50303331 | IKZF1 1851 | chr7:50362075-50362102 |
| IKZF1 241 | chr7:50303320-50303347 | IKZF1 1852 | chr7:50362086-50362113 |
| IKZF1 242 | chr7:50303330-50303357 | IKZF1 1853 | chr7:50362103-50362130 |
| IKZF1 243 | chr7:50303336-50303363 | IKZF1 1854 | chr7:50362119-50362146 |
| IKZF1 244 | chr7:50303337-50303364 | IKZF1 1855 | chr7:50362130-50362157 |
| IKZF1 245 | chr7:50303346-50303373 | IKZF1 1856 | chr7:50362134-50362161 |
| IKZF1 246 | chr7:50303347-50303374 | IKZF1 1857 | chr7:50362140-50362167 |
| IKZF1 247 | chr7:50303352-50303379 | IKZF1 1858 | chr7:50362150-50362177 |
| IKZF1 248 | chr7:50303369-50303396 | IKZF1 1859 | chr7:50362151-50362178 |
| IKZF1 249 | chr7:50303379-50303406 | IKZF1 1860 | chr7:50362167-50362194 |
| IKZF1 250 | chr7:50303380-50303407 | IKZF1 1861 | chr7:50362178-50362205 |
| IKZF1 251 | chr7:50303386-50303413 | IKZF1 1862 | chr7:50362206-50362233 |
| IKZF1 252 | chr7:50303387-50303414 | IKZF1 1863 | chr7:50362221-50362248 |
| IKZF1 253 | chr7:50303396-50303423 | IKZF1 1864 | chr7:50362222-50362249 |
| IKZF1 254 | chr7:50303430-50303457 | IKZF1 1865 | chr7:50362235-50362262 |
| IKZF1 255 | chr7:50303433-50303460 | IKZF1 1866 | chr7:50362245-50362272 |
| IKZF1 256 | chr7:50303436-50303463 | IKZF1 1867 | chr7:50362246-50362273 |
| IKZF1 257 | chr7:50303443-50303470 | IKZF1 1868 | chr7:50362252-50362279 |
| IKZF1 258 | chr7:50303446-50303473 | IKZF1 1869 | chr7:50362254-50362281 |
| IKZF1 259 | chr7:50303447-50303474 | IKZF1 1870 | chr7:50362255-50362282 |
| IKZF1 260 | chr7:50303450-50303477 | IKZF1 1871 | chr7:50362264-50362291 |
| IKZF1 261 | chr7:50303451-50303478 | IKZF1 1872 | chr7:50362265-50362292 |
| IKZF1 262 | chr7:50303459-50303486 | IKZF1 1873 | chr7:50362272-50362299 |
| IKZF1 263 | chr7:50303463-50303490 | IKZF1 1874 | chr7:50362274-50362301 |
| IKZF1 264 | chr7:50303472-50303499 | IKZF1 1875 | chr7:50362276-50362303 |
| IKZF1 265 | chr7:50303473-50303500 | IKZF1 1876 | chr7:50362305-50362332 |
| IKZF1 266 | chr7:50303478-50303505 | IKZF1 1877 | chr7:50362313-50362340 |
| IKZF1 267 | chr7:50303491-50303518 | IKZF1 1878 | chr7:50362314-50362341 |
| IKZF1 268 | chr7:50303493-50303520 | IKZF1 1879 | chr7:50362315-50362342 |
| IKZF1 269 | chr7:50303494-50303521 | IKZF1 1880 | chr7:50362318-50362345 |
| IKZF1 270 | chr7:50303495-50303522 | IKZF1 1881 | chr7:50362319-50362346 |
| IKZF1 271 | chr7:50303497-50303524 | IKZF1 1882 | chr7:50362320-50362347 |
| IKZF1 272 | chr7:50303498-50303525 | IKZF1 1883 | chr7:50362321-50362348 |
| IKZF1 273 | chr7:50303499-50303526 | IKZF1 1884 | chr7:50362327-50362354 |
| IKZF1 274 | chr7:50303508-50303535 | IKZF1 1885 | chr7:50362336-50362363 |
| IKZF1 275 | chr7:50303514-50303541 | IKZF1 1886 | chr7:50362337-50362364 |
| IKZF1 276 | chr7:50303525-50303552 | IKZF1 1887 | chr7:50362341-50362368 |
| IKZF1 277 | chr7:50303533-50303560 | IKZF1 1888 | chr7:50362357-50362384 |
| IKZF1 278 | chr7:50303534-50303561 | IKZF1 1889 | chr7:50362365-50362392 |
| IKZF1 279 | chr7:50304042-50304069 | IKZF1 1890 | chr7:50362374-50362401 |
| IKZF1 280 | chr7:50304043-50304070 | IKZF1 1891 | chr7:50362406-50362433 |
| IKZF1 281 | chr7:50304048-50304075 | IKZF1 1892 | chr7:50362408-50362435 |
| IKZF1 282 | chr7:50304053-50304080 | IKZF1 1893 | chr7:50362412-50362439 |
| IKZF1 283 | chr7:50304054-50304081 | IKZF1 1894 | chr7:50362424-50362451 |
| IKZF1 284 | chr7:50304061-50304088 | IKZF1 1895 | chr7:50362444-50362471 |
| IKZF1 285 | chr7:50304062-50304089 | IKZF1 1896 | chr7:50362445-50362472 |
| IKZF1 286 | chr7:50304071-50304098 | IKZF1 1897 | chr7:50362450-50362477 |
| IKZF1 287 | chr7:50304072-50304099 | IKZF1 1898 | chr7:50362451-50362478 |
| IKZF1 288 | chr7:50304075-50304102 | IKZF1 1899 | chr7:50362453-50362480 |
| IKZF1 289 | chr7:50304078-50304105 | IKZF1 1900 | chr7:50362463-50362490 |
| IKZF1 290 | chr7:50304088-50304115 | IKZF1 1901 | chr7:50362464-50362491 |
| IKZF1 291 | chr7:50304093-50304120 | IKZF1 1902 | chr7:50362467-50362494 |
| IKZF1 292 | chr7:50304096-50304123 | IKZF1 1903 | chr7:50362485-50362512 |
| IKZF1 293 | chr7:50304108-50304135 | IKZF1 1904 | chr7:50362486-50362513 |
| IKZF1 294 | chr7:50304115-50304142 | IKZF1 1905 | chr7:50362494-50362521 |
| IKZF1 295 | chr7:50304118-50304145 | IKZF1 1906 | chr7:50362506-50362533 |
| IKZF1 296 | chr7:50304121-50304148 | IKZF1 1907 | chr7:50362512-50362539 |
| IKZF1 297 | chr7:50304122-50304149 | IKZF1 1908 | chr7:50362538-50362565 |
| IKZF1 298 | chr7:50304125-50304152 | IKZF1 1909 | chr7:50362564-50362591 |
| IKZF1 299 | chr7:50304126-50304153 | IKZF1 1910 | chr7:50362565-50362592 |
| IKZF1 300 | chr7:50304127-50304154 | IKZF1 1911 | chr7:50362600-50362627 |
| IKZF1 301 | chr7:50304128-50304155 | IKZF1 1912 | chr7:50362620-50362647 |
| IKZF1 302 | chr7:50304131-50304158 | IKZF1 1913 | chr7:50362632-50362659 |
| IKZF1 303 | chr7:50304134-50304161 | IKZF1 1914 | chr7:50362633-50362660 |
| IKZF1 304 | chr7:50304137-50304164 | IKZF1 1915 | chr7:50362662-50362689 |
| IKZF1 305 | chr7:50304138-50304165 | IKZF1 1916 | chr7:50362672-50362699 |
| IKZF1 306 | chr7:50304139-50304166 | IKZF1 1917 | chr7:50362673-50362700 |
| IKZF1 307 | chr7:50304144-50304171 | IKZF1 1918 | chr7:50362688-50362715 |
| IKZF1 308 | chr7:50304146-50304173 | IKZF1 1919 | chr7:50362689-50362716 |
| IKZF1 309 | chr7:50304161-50304188 | IKZF1 1920 | chr7:50362693-50362720 |
| IKZF1 310 | chr7:50304167-50304194 | IKZF1 1921 | chr7:50362696-50362723 |
| IKZF1 311 | chr7:50304168-50304195 | IKZF1 1922 | chr7:50362730-50362757 |
| IKZF1 312 | chr7:50304170-50304197 | IKZF1 1923 | chr7:50362739-50362766 |
| IKZF1 313 | chr7:50304179-50304206 | IKZF1 1924 | chr7:50362751-50362778 |
| IKZF1 314 | chr7:50304184-50304211 | IKZF1 1925 | chr7:50362752-50362779 |
| IKZF1 315 | chr7:50304192-50304219 | IKZF1 1926 | chr7:50362755-50362782 |
| IKZF1 316 | chr7:50304198-50304225 | IKZF1 1927 | chr7:50362766-50362793 |
| IKZF1 317 | chr7:50304199-50304226 | IKZF1 1928 | chr7:50362772-50362799 |
| IKZF1 318 | chr7:50304200-50304227 | IKZF1 1929 | chr7:50362779-50362806 |
| IKZF1 319 | chr7:50304207-50304234 | IKZF1 1930 | chr7:50362786-50362813 |
| IKZF1 320 | chr7:50304208-50304235 | IKZF1 1931 | chr7:50362787-50362814 |
| IKZF1 321 | chr7:50304217-50304244 | IKZF1 1932 | chr7:50362798-50362825 |
| IKZF1 322 | chr7:50304218-50304245 | IKZF1 1933 | chr7:50362810-50362837 |
| IKZF1 323 | chr7:50304219-50304246 | IKZF1 1934 | chr7:50362811-50362838 |
| IKZF1 324 | chr7:50304220-50304247 | IKZF1 1935 | chr7:50362812-50362839 |
| IKZF1 325 | chr7:50304226-50304253 | IKZF1 1936 | chr7:50362827-50362854 |
| IKZF1 326 | chr7:50304232-50304259 | IKZF1 1937 | chr7:50362842-50362869 |
| IKZF1 327 | chr7:50304233-50304260 | IKZF1 1938 | chr7:50362856-50362883 |
| IKZF1 328 | chr7:50304234-50304261 | IKZF1 1939 | chr7:50362857-50362884 |
| IKZF1 329 | chr7:50304237-50304264 | IKZF1 1940 | chr7:50362858-50362885 |
| IKZF1 330 | chr7:50304238-50304265 | IKZF1 1941 | chr7:50362866-50362893 |
| IKZF1 331 | chr7:50304239-50304266 | IKZF1 1942 | chr7:50362869-50362896 |
| IKZF1 332 | chr7:50304241-50304268 | IKZF1 1943 | chr7:50362874-50362901 |
| IKZF1 333 | chr7:50304243-50304270 | IKZF1 1944 | chr7:50362883-50362910 |
| IKZF1 334 | chr7:50304244-50304271 | IKZF1 1945 | chr7:50362886-50362913 |
| IKZF1 335 | chr7:50304280-50304307 | IKZF1 1946 | chr7:50362887-50362914 |
| IKZF1 336 | chr7:50304281-50304308 | IKZF1 1947 | chr7:50362893-50362920 |
| IKZF1 337 | chr7:50304286-50304313 | IKZF1 1948 | chr7:50362899-50362926 |
| IKZF1 338 | chr7:50304292-50304319 | IKZF1 1949 | chr7:50362903-50362930 |
| IKZF1 339 | chr7:50304293-50304320 | IKZF1 1950 | chr7:50362904-50362931 |
| IKZF1 340 | chr7:50304303-50304330 | IKZF1 1951 | chr7:50362913-50362940 |
| IKZF1 341 | chr7:50304304-50304331 | IKZF1 1952 | chr7:50362917-50362944 |
| IKZF1 342 | chr7:50304305-50304332 | IKZF1 1953 | chr7:50362918-50362945 |
| IKZF1 343 | chr7:50304315-50304342 | IKZF1 1954 | chr7:50362924-50362951 |
| IKZF1 344 | chr7:50304316-50304343 | IKZF1 1955 | chr7:50362928-50362955 |
| IKZF1 345 | chr7:50304317-50304344 | IKZF1 1956 | chr7:50362929-50362956 |
| IKZF1 346 | chr7:50304318-50304345 | IKZF1 1957 | chr7:50362930-50362957 |
| IKZF1 347 | chr7:50304319-50304346 | IKZF1 1958 | chr7:50362935-50362962 |
| IKZF1 348 | chr7:50304322-50304349 | IKZF1 1959 | chr7:50362938-50362965 |
| IKZF1 349 | chr7:50304323-50304350 | IKZF1 1960 | chr7:50362947-50362974 |
| IKZF1 350 | chr7:50304325-50304352 | IKZF1 1961 | chr7:50362948-50362975 |
| IKZF1 351 | chr7:50304329-50304356 | IKZF1 1962 | chr7:50362959-50362986 |
| IKZF1 352 | chr7:50304333-50304360 | IKZF1 1963 | chr7:50362963-50362990 |
| IKZF1 353 | chr7:50304342-50304369 | IKZF1 1964 | chr7:50362966-50362993 |
| IKZF1 354 | chr7:50304343-50304370 | IKZF1 1965 | chr7:50362979-50363006 |
| IKZF1 355 | chr7:50304344-50304371 | IKZF1 1966 | chr7:50363010-50363037 |
| IKZF1 356 | chr7:50304353-50304380 | IKZF1 1967 | chr7:50363011-50363038 |
| IKZF1 357 | chr7:50304359-50304386 | IKZF1 1968 | chr7:50363015-50363042 |
| IKZF1 358 | chr7:50304360-50304387 | IKZF1 1969 | chr7:50363021-50363048 |
| IKZF1 359 | chr7:50304361-50304388 | IKZF1 1970 | chr7:50363022-50363049 |
| IKZF1 360 | chr7:50304365-50304392 | IKZF1 1971 | chr7:50363026-50363053 |
| IKZF1 361 | chr7:50304372-50304399 | IKZF1 1972 | chr7:50363027-50363054 |
| IKZF1 362 | chr7:50304374-50304401 | IKZF1 1973 | chr7:50363033-50363060 |
| IKZF1 363 | chr7:50304375-50304402 | IKZF1 1974 | chr7:50363039-50363066 |
| IKZF1 364 | chr7:50304377-50304404 | IKZF1 1975 | chr7:50363047-50363074 |
| IKZF1 365 | chr7:50304404-50304431 | IKZF1 1976 | chr7:50363048-50363075 |
| IKZF1 366 | chr7:50304407-50304434 | IKZF1 1977 | chr7:50363052-50363079 |
| IKZF1 367 | chr7:50304408-50304435 | IKZF1 1978 | chr7:50363061-50363088 |
| IKZF1 368 | chr7:50304409-50304436 | IKZF1 1979 | chr7:50363065-50363092 |
| IKZF1 369 | chr7:50304412-50304439 | IKZF1 1980 | chr7:50363066-50363093 |
| IKZF1 370 | chr7:50304416-50304443 | IKZF1 1981 | chr7:50363067-50363094 |
| IKZF1 371 | chr7:50304417-50304444 | IKZF1 1982 | chr7:50363068-50363095 |
| IKZF1 372 | chr7:50304420-50304447 | IKZF1 1983 | chr7:50363092-50363119 |
| IKZF1 373 | chr7:50304421-50304448 | IKZF1 1984 | chr7:50363093-50363120 |
| IKZF1 374 | chr7:50304422-50304449 | IKZF1 1985 | chr7:50363102-50363129 |
| IKZF1 375 | chr7:50304429-50304456 | IKZF1 1986 | chr7:50363104-50363131 |
| IKZF1 376 | chr7:50304445-50304472 | IKZF1 1987 | chr7:50363110-50363137 |
| IKZF1 377 | chr7:50304446-50304473 | IKZF1 1988 | chr7:50363111-50363138 |
| IKZF1 378 | chr7:50304449-50304476 | IKZF1 1989 | chr7:50363116-50363143 |
| IKZF1 379 | chr7:50304456-50304483 | IKZF 1 1990 | chr7:50363117-50363144 |
| IKZF1 380 | chr7:50304457-50304484 | IKZF1 1991 | chr7:50363118-50363145 |
| IKZF1 381 | chr7:50304459-50304486 | IKZF1 1992 | chr7:50363119-50363146 |
| IKZF1 382 | chr7:50304460-50304487 | IKZF1 1993 | chr7:50363128-50363155 |
| IKZF1 383 | chr7:50304461-50304488 | IKZF1 1994 | chr7:50363129-50363156 |
| IKZF1 384 | chr7:50304464-50304491 | IKZF1 1995 | chr7:50363135-50363162 |
| IKZF1 385 | chr7:50304469-50304496 | IKZF1 1996 | chr7:50363136-50363163 |
| IKZF1 386 | chr7:50304470-50304497 | IKZF1 1997 | chr7:50363141-50363168 |
| IKZF1 387 | chr7:50304475-50304502 | IKZF1 1998 | chr7:50363142-50363169 |
| IKZF1 388 | chr7:50304478-50304505 | IKZF1 1999 | chr7:50363157-50363184 |
| IKZF1 389 | chr7:50304479-50304506 | IKZF1 2000 | chr7:50363162-50363189 |
| IKZF1 390 | chr7:50304480-50304507 | IKZF1 2001 | chr7:50363165-50363192 |
| IKZF1 391 | chr7:50304481-50304508 | IKZF1 2002 | chr7:50363166-50363193 |
| IKZF1 392 | chr7:50304484-50304511 | IKZF1 2003 | chr7:50363174-50363201 |
| IKZF1 393 | chr7:50304488-50304515 | IKZF1 2004 | chr7:50363175-50363202 |
| IKZF1 394 | chr7:50304493-50304520 | IKZF1 2005 | chr7:50363180-50363207 |
| IKZF1 395 | chr7:50304496-50304523 | IKZF1 2006 | chr7:50363188-50363215 |
| IKZF1 396 | chr7:50304497-50304524 | IKZF1 2007 | chr7:50363200-50363227 |
| IKZF1 397 | chr7:50304498-50304525 | IKZF1 2008 | chr7:50363203-50363230 |
| IKZF1 398 | chr7:50304499-50304526 | IKZF1 2009 | chr7:50363218-50363245 |
| IKZF1 399 | chr7:50304504-50304531 | IKZF1 2010 | chr7:50363219-50363246 |
| IKZF1 400 | chr7:50304509-50304536 | IKZF1 2011 | chr7:50363222-50363249 |
| IKZF1 401 | chr7:50304510-50304537 | IKZF1 2012 | chr7:50363223-50363250 |
| IKZF1 402 | chr7:50304511-50304538 | IKZF1 2013 | chr7:50363225-50363252 |
| IKZF1 403 | chr7:50304512-50304539 | IKZF1 2014 | chr7:50363226-50363253 |
| IKZF1 404 | chr7:50304518-50304545 | IKZF1 2015 | chr7:50363233-50363260 |
| IKZF1 405 | chr7:50304519-50304546 | IKZF1 2016 | chr7:50363234-50363261 |
| IKZF1 406 | chr7:50304522-50304549 | IKZF1 2017 | chr7:50363235-50363262 |
| IKZF1 407 | chr7:50304527-50304554 | IKZF1 2018 | chr7:50363236-50363263 |
| IKZF1 408 | chr7:50304695-50304722 | IKZF1 2019 | chr7:50363241-50363268 |
| IKZF1 409 | chr7:50304701-50304728 | IKZF1 2020 | chr7:50363247-50363274 |
| IKZF1 410 | chr7:50304710-50304737 | IKZF1 2021 | chr7:50363248-50363275 |
| IKZF1 411 | chr7:50304721-50304748 | IKZF1 2022 | chr7:50363261-50363288 |
| IKZF1 412 | chr7:50304722-50304749 | IKZF1 2023 | chr7:50363268-50363295 |
| IKZF1 413 | chr7:50304723-50304750 | IKZF1 2024 | chr7:50363272-50363299 |
| IKZF1 414 | chr7:50304726-50304753 | IKZF1 2025 | chr7:50363273-50363300 |
| IKZF1 415 | chr7:50304735-50304762 | IKZF1 2026 | chr7:50363276-50363303 |
| IKZF1 416 | chr7:50304738-50304765 | IKZF1 2027 | chr7:50363280-50363307 |
| IKZF1 417 | chr7:50304739-50304766 | IKZF1 2028 | chr7:50363291-50363318 |
| IKZF1 418 | chr7:50304744-50304771 | IKZF1 2029 | chr7:50363292-50363319 |
| IKZF1 419 | chr7:50304745-50304772 | IKZF1 2030 | chr7:50363293-50363320 |
| IKZF1 420 | chr7:50304747-50304774 | IKZF1 2031 | chr7:50363302-50363329 |
| IKZF1 421 | chr7:50304748-50304775 | IKZF1 2032 | chr7:50363308-50363335 |
| IKZF1 422 | chr7:50304759-50304786 | IKZF1 2033 | chr7:50363321-50363348 |
| IKZF1 423 | chr7:50304767-50304794 | IKZF1 2034 | chr7:50363325-50363352 |
| IKZF1 424 | chr7:50304768-50304795 | IKZF1 2035 | chr7:50363328-50363355 |
| IKZF1 425 | chr7:50304769-50304796 | IKZF1 2036 | chr7:50363332-50363359 |
| IKZF1 426 | chr7:50304770-50304797 | IKZF1 2037 | chr7:50363337-50363364 |
| IKZF1 427 | chr7:50304774-50304801 | IKZF1 2038 | chr7:50363356-50363383 |
| IKZF1 428 | chr7:50304775-50304802 | IKZF1 2039 | chr7:50363370-50363397 |
| IKZF1 429 | chr7:50304777-50304804 | IKZF1 2040 | chr7:50363371-50363398 |
| IKZF1 430 | chr7:50304778-50304805 | IKZF1 2041 | chr7:50363373-50363400 |
| IKZF1 431 | chr7:50304779-50304806 | IKZF1 2042 | chr7:50363378-50363405 |
| IKZF1 432 | chr7:50304787-50304814 | IKZF1 2043 | chr7:50363379-50363406 |
| IKZF1 433 | chr7:50304792-50304819 | IKZF1 2044 | chr7:50363383-50363410 |
| IKZF1 434 | chr7:50304793-50304820 | IKZF1 2045 | chr7:50363393-50363420 |
| IKZF1 435 | chr7:50304797-50304824 | IKZF1 2046 | chr7:50363415-50363442 |
| IKZF1 436 | chr7:50304798-50304825 | IKZF1 2047 | chr7:50363427-50363454 |
| IKZF1 437 | chr7:50304801-50304828 | IKZF1 2048 | chr7:50363432-50363459 |
| IKZF1 438 | chr7:50304802-50304829 | IKZF1 2049 | chr7:50363435-50363462 |
| IKZF1 439 | chr7:50304811-50304838 | IKZF1 2050 | chr7:50363453-50363480 |
| IKZF1 440 | chr7:50304815-50304842 | IKZF1 2051 | chr7:50363471-50363498 |
| IKZF1 441 | chr7:50304816-50304843 | IKZF1 2052 | chr7:50363472-50363499 |
| IKZF1 442 | chr7:50304822-50304849 | IKZF1 2053 | chr7:50363473-50363500 |
| IKZF1 443 | chr7:50304823-50304850 | IKZF1 2054 | chr7:50363486-50363513 |
| IKZF1 444 | chr7:50304831-50304858 | IKZF1 2055 | chr7:50363487-50363514 |
| IKZF1 445 | chr7:50304834-50304861 | IKZF1 2056 | chr7:50363493-50363520 |
| IKZF1 446 | chr7:50304839-50304866 | IKZF1 2057 | chr7:50363494-50363521 |
| IKZF1 447 | chr7:50304853-50304880 | IKZF1 2058 | chr7:50363522-50363549 |
| IKZF1 448 | chr7:50304854-50304881 | IKZF1 2059 | chr7:50363525-50363552 |
| IKZF1 449 | chr7:50304855-50304882 | IKZF1 2060 | chr7:50363526-50363553 |
| IKZF 1 450 | chr7:50304858-50304885 | IKZF1 2061 | chr7:50363527-50363554 |
| IKZF1 451 | chr7:50304861-50304888 | IKZF1 2062 | chr7:50363534-50363561 |
| IKZF1 452 | chr7:50304869-50304896 | IKZF1 2063 | chr7:50363535-50363562 |
| IKZF1 453 | chr7:50304879-50304906 | IKZF1 2064 | chr7:50363536-50363563 |
| IKZF1 454 | chr7:50304880-50304907 | IKZF1 2065 | chr7:50363548-50363575 |
| IKZF1 455 | chr7:50304897-50304924 | IKZF1 2066 | chr7:50363549-50363576 |
| IKZF1 456 | chr7:50304898-50304925 | IKZF1 2067 | chr7:50363556-50363583 |
| IKZF1 457 | chr7:50304899-50304926 | IKZF1 2068 | chr7:50363557-50363584 |
| IKZF1 458 | chr7:50304919-50304946 | IKZF1 2069 | chr7:50363574-50363601 |
| IKZF1 459 | chr7:50308690-50308717 | IKZF1 2070 | chr7:50363584-50363611 |
| IKZF 1 460 | chr7:50308693-50308720 | IKZF1 2071 | chr7:50363598-50363625 |
| IKZF1 461 | chr7:50308697-50308724 | IKZF1 2072 | chr7:50363612-50363639 |
| IKZF1 462 | chr7:50308700-50308727 | IKZF1 2073 | chr7:50363616-50363643 |
| IKZF1 463 | chr7:50308703-50308730 | IKZF1 2074 | chr7:50363619-50363646 |
| IKZF1 464 | chr7:50308712-50308739 | IKZF1 2075 | chr7:50363637-50363664 |
| IKZF1 465 | chr7:50308713-50308740 | IKZF1 2076 | chr7:50363650-50363677 |
| IKZF1 466 | chr7:50308722-50308749 | IKZF1 2077 | chr7:50363663-50363690 |
| IKZF1 467 | chr7:50308725-50308752 | IKZF1 2078 | chr7:50363664-50363691 |
| IKZF1 468 | chr7:50308728-50308755 | IKZF1 2079 | chr7:50363669-50363696 |
| IKZF 1 469 | chr7:50308734-50308761 | IKZF1 2080 | chr7:50363670-50363697 |
| IKZF1 470 | chr7:50308742-50308769 | IKZF1 2081 | chr7:50363673-50363700 |
| IKZF1 471 | chr7:50308743-50308770 | IKZF1 2082 | chr7:50363682-50363709 |
| IKZF1 472 | chr7:50308756-50308783 | IKZF1 2083 | chr7:50363685-50363712 |
| IKZF1 473 | chr7:50308771-50308798 | IKZF1 2084 | chr7:50363688-50363715 |
| IKZF1 474 | chr7:50308772-50308799 | IKZF1 2085 | chr7:50363704-50363731 |
| IKZF1 475 | chr7:50308775-50308802 | IKZF1 2086 | chr7:50363713-50363740 |
| IKZF1 476 | chr7:50308778-50308805 | IKZF1 2087 | chr7:50363718-50363745 |
| IKZF1 477 | chr7:50308779-50308806 | IKZF1 2088 | chr7:50363722-50363749 |
| IKZF1 478 | chr7:50308782-50308809 | IKZF1 2089 | chr7:50363735-50363762 |
| IKZF1 479 | chr7:50308783-50308810 | IKZF1 2090 | chr7:50363747-50363774 |
| IKZF 1 480 | chr7:50308786-50308813 | IKZF1 2091 | chr7:50363775-50363802 |
| IKZF1 481 | chr7:50308790-50308817 | IKZF1 2092 | chr7:50363782-50363809 |
| IKZF1 482 | chr7:50308791-50308818 | IKZF1 2093 | chr7:50363791-50363818 |
| IKZF1 483 | chr7:50308792-50308819 | IKZF1 2094 | chr7:50363794-50363821 |
| IKZF 1 484 | chr7:50308793-50308820 | IKZF1 2095 | chr7:50363803-50363830 |
| IKZF1 485 | chr7:50308794-50308821 | IKZF1 2096 | chr7:50363832-50363859 |
| IKZF1 486 | chr7:50308797-50308824 | IKZF1 2097 | chr7:50363835-50363862 |
| IKZF1 487 | chr7:50308798-50308825 | IKZF1 2098 | chr7:50363836-50363863 |
| IKZF1 488 | chr7:50308811-50308838 | IKZF1 2099 | chr7:50363840-50363867 |
| IKZF1 489 | chr7:50308824-50308851 | IKZF1 2100 | chr7:50363843-50363870 |
| IKZF1 490 | chr7:50308825-50308852 | IKZF1 2101 | chr7:50363846-50363873 |
| IKZF1 491 | chr7:50308832-50308859 | IKZF1 2102 | chr7:50363849-50363876 |
| IKZF1 492 | chr7:50308833-50308860 | IKZF1 2103 | chr7:50363858-50363885 |
| IKZF1 493 | chr7:50308835-50308862 | IKZF1 2104 | chr7:50363863-50363890 |
| IKZF1 494 | chr7:50308840-50308867 | IKZF1 2105 | chr7:50363864-50363891 |
| IKZF1 495 | chr7:50308841-50308868 | IKZF1 2106 | chr7:50363865-50363892 |
| IKZF1 496 | chr7:50308855-50308882 | IKZF1 2107 | chr7:50363870-50363897 |
| IKZF1 497 | chr7:50308861-50308888 | IKZF1 2108 | chr7:50363888-50363915 |
| IKZF1 498 | chr7:50308866-50308893 | IKZF1 2109 | chr7:50363898-50363925 |
| IKZF1 499 | chr7:50308872-50308899 | IKZF1 2110 | chr7:50363901-50363928 |
| IKZF1 500 | chr7:50308876-50308903 | IKZF1 2111 | chr7:50363902-50363929 |
| IKZF1 501 | chr7:50308880-50308907 | IKZF1 2112 | chr7:50363906-50363933 |
| IKZF1 502 | chr7:50308883-50308910 | IKZF1 2113 | chr7:50363912-50363939 |
| IKZF1 503 | chr7:50308894-50308921 | IKZF1 2114 | chr7:50363934-50363961 |
| IKZF1 504 | chr7:50318392-50318419 | IKZF1 2115 | chr7:50363937-50363964 |
| IKZF 1 505 | chr7:50318403-50318430 | IKZF1 2116 | chr7:50363942-50363969 |
| IKZF1 506 | chr7:50318409-50318436 | IKZF1 2117 | chr7:50363949-50363976 |
| IKZF1 507 | chr7:50318412-50318439 | IKZF1 2118 | chr7:50363965-50363992 |
| IKZF1 508 | chr7:50318413-50318440 | IKZF1 2119 | chr7:50363973-50364000 |
| IKZF1 509 | chr7:50318414-50318441 | IKZF1 2120 | chr7:50364061-50364088 |
| IKZF1 510 | chr7:50318415-50318442 | IKZF1 2121 | chr7:50364087-50364114 |
| IKZF1 511 | chr7:50318420-50318447 | IKZF1 2122 | chr7:50364103-50364130 |
| IKZF1 512 | chr7:50318421-50318448 | IKZF1 2123 | chr7:50364104-50364131 |
| IKZF1 513 | chr7:50318437-50318464 | IKZF1 2124 | chr7:50364149-50364176 |
| IKZF1 514 | chr7:50318438-50318465 | IKZF1 2125 | chr7:50364152-50364179 |
| IKZF1 515 | chr7:50318449-50318476 | IKZF1 2126 | chr7:50364153-50364180 |
| IKZF1 516 | chr7:50318450-50318477 | IKZF1 2127 | chr7:50364179-50364206 |
| IKZF1 517 | chr7:50318475-50318502 | IKZF1 2128 | chr7:50364185-50364212 |
| IKZF1 518 | chr7:50318479-50318506 | IKZF1 2129 | chr7:50364190-50364217 |
| IKZF1 519 | chr7:50318481-50318508 | IKZF1 2130 | chr7:50364191-50364218 |
| IKZF1 520 | chr7:50318482-50318509 | IKZF1 2131 | chr7:50364192-50364219 |
| IKZF1 521 | chr7:50318490-50318517 | IKZF1 2132 | chr7:50364231-50364258 |
| IKZF1 522 | chr7:50318491-50318518 | IKZF1 2133 | chr7:50364238-50364265 |
| IKZF1 523 | chr7:50318498-50318525 | IKZF1 2134 | chr7:50364239-50364266 |
| IKZF1 524 | chr7:50318500-50318527 | IKZF1 2135 | chr7:50364273-50364300 |
| IKZF1 525 | chr7:50318503-50318530 | IKZF1 2136 | chr7:50364279-50364306 |
| IKZF1 526 | chr7:50318540-50318567 | IKZF1 2137 | chr7:50364280-50364307 |
| IKZF1 527 | chr7:50318541-50318568 | IKZF1 2138 | chr7:50364286-50364313 |
| IKZF1 528 | chr7:50318558-50318585 | IKZF1 2139 | chr7:50364301-50364328 |
| IKZF1 529 | chr7:50318559-50318586 | IKZF1 2140 | chr7:50364323-50364350 |
| IKZF1 530 | chr7:50318563-50318590 | IKZF1 2141 | chr7:50364342-50364369 |
| IKZF1 531 | chr7:50319029-50319056 | IKZF1 2142 | chr7:50364343-50364370 |
| IKZF1 532 | chr7:50319030-50319057 | IKZF1 2143 | chr7:50364344-50364371 |
| IKZF1 533 | chr7:50319031-50319058 | IKZF1 2144 | chr7:50364357-50364384 |
| IKZF1 534 | chr7:50319034-50319061 | IKZF1 2145 | chr7:50364386-50364413 |
| IKZF1 535 | chr7:50319038-50319065 | IKZF1 2146 | chr7:50364460-50364487 |
| IKZF1 536 | chr7:50319050-50319077 | IKZF1 2147 | chr7:50364466-50364493 |
| IKZF1 537 | chr7:50319051-50319078 | IKZF1 2148 | chr7:50364476-50364503 |
| IKZF1 538 | chr7:50319052-50319079 | IKZF1 2149 | chr7:50364483-50364510 |
| IKZF1 539 | chr7:50319060-50319087 | IKZF1 2150 | chr7:50364494-50364521 |
| IKZF 1 540 | chr7:50319075-50319102 | IKZF1 2151 | chr7:50364495-50364522 |
| IKZF1 541 | chr7:50319090-50319117 | IKZF1 2152 | chr7:50364496-50364523 |
| IKZF1 542 | chr7:50319091-50319118 | IKZF1 2153 | chr7:50364504-50364531 |
| IKZF1 543 | chr7:50319101-50319128 | IKZF1 2154 | chr7:50364559-50364586 |
| IKZF 1 544 | chr7:50327611-50327638 | IKZF1 2155 | chr7:50364560-50364587 |
| IKZF1 545 | chr7:50327612-50327639 | IKZF1 2156 | chr7:50364565-50364592 |
| IKZF 1 546 | chr7:50327616-50327643 | IKZF1 2157 | chr7:50364566-50364593 |
| IKZF1 547 | chr7:50327648-50327675 | IKZF1 2158 | chr7:50364578-50364605 |
| IKZF 1 548 | chr7:50327649-50327676 | IKZF1 2159 | chr7:50364582-50364609 |
| IKZF1 549 | chr7:50327650-50327677 | IKZF1 2160 | chr7:50364594-50364621 |
| IKZF1 550 | chr7:50327651-50327678 | IKZF1 2161 | chr7:50364595-50364622 |
| IKZF1 551 | chr7:50327652-50327679 | IKZF1 2162 | chr7:50364596-50364623 |
| IKZF1 552 | chr7:50327667-50327694 | IKZF1 2163 | chr7:50364640-50364667 |
| IKZF1 553 | chr7:50327676-50327703 | IKZF1 2164 | chr7:50364648-50364675 |
| IKZF1 554 | chr7:50327685-50327712 | IKZF1 2165 | chr7:50364653-50364680 |
| IKZF1 555 | chr7:50327686-50327713 | IKZF1 2166 | chr7:50364656-50364683 |
| IKZF1 556 | chr7:50327691-50327718 | IKZF1 2167 | chr7:50364687-50364714 |
| IKZF1 557 | chr7:50327694-50327721 | IKZF1 2168 | chr7:50364688-50364715 |
| IKZF1 558 | chr7:50327695-50327722 | IKZF1 2169 | chr7:50364700-50364727 |
| IKZF1 559 | chr7:50327696-50327723 | IKZF1 2170 | chr7:50364714-50364741 |
| IKZF1 560 | chr7:50327697-50327724 | IKZF1 2171 | chr7:50364715-50364742 |
| IKZF1 561 | chr7:50327698-50327725 | IKZF1 2172 | chr7:50364716-50364743 |
| IKZF1 562 | chr7:50327705-50327732 | IKZF1 2173 | chr7:50364724-50364751 |
| IKZF1 563 | chr7:50327710-50327737 | IKZF1 2174 | chr7:50364737-50364764 |
| IKZF1 564 | chr7:50327713-50327740 | IKZF1 2175 | chr7:50364740-50364767 |
| IKZF1 565 | chr7:50327716-50327743 | IKZF1 2176 | chr7:50364744-50364771 |
| IKZF1 566 | chr7:50327730-50327757 | IKZF1 2177 | chr7:50364754-50364781 |
| IKZF1 567 | chr7:50327731-50327758 | IKZF1 2178 | chr7:50364796-50364823 |
| IKZF1 568 | chr7:50327737-50327764 | IKZF1 2179 | chr7:50364802-50364829 |
| IKZF1 569 | chr7:50327738-50327765 | IKZF1 2180 | chr7:50364810-50364837 |
| IKZF1 570 | chr7:50327739-50327766 | IKZF1 2181 | chr7:50364816-50364843 |
| IKZF1 571 | chr7:50327749-50327776 | IKZF1 2182 | chr7:50364817-50364844 |
| IKZF1 572 | chr7:50352641-50352668 | IKZF1 2183 | chr7:50364819-50364846 |
| IKZF1 573 | chr7:50352660-50352687 | IKZF1 2184 | chr7:50364820-50364847 |
| IKZF1 574 | chr7:50352662-50352689 | IKZF1 2185 | chr7:50364824-50364851 |
| IKZF1 575 | chr7:50352681-50352708 | IKZF1 2186 | chr7:50364825-50364852 |
| IKZF1 576 | chr7:50352716-50352743 | IKZF1 2187 | chr7:50364836-50364863 |
| IKZF1 577 | chr7:50352747-50352774 | IKZF1 2188 | chr7:50364846-50364873 |
| IKZF1 578 | chr7:50352750-50352777 | IKZF1 2189 | chr7:50364847-50364874 |
| IKZF1 579 | chr7:50352790-50352817 | IKZF1 2190 | chr7:50364855-50364882 |
| IKZF1 580 | chr7:50352807-50352834 | IKZF1 2191 | chr7:50364856-50364883 |
| IKZF1 581 | chr7:50352830-50352857 | IKZF1 2192 | chr7:50364857-50364884 |
| IKZF1 582 | chr7:50352835-50352862 | IKZF1 2193 | chr7:50364858-50364885 |
| IKZF1 583 | chr7:50352836-50352863 | IKZF1 2194 | chr7:50364862-50364889 |
| IKZF1 584 | chr7:50352842-50352869 | IKZF1 2195 | chr7:50364863-50364890 |
| IKZF1 585 | chr7:50352870-50352897 | IKZF1 2196 | chr7:50364865-50364892 |
| IKZF1 586 | chr7:50352874-5035290 1 | IKZF1 2197 | chr7:50364871-50364898 |
| IKZF1 587 | chr7:50352890-50352917 | IKZF1 2198 | chr7:50364875-50364902 |
| IKZF1 588 | chr7:50352937-50352964 | IKZF1 2199 | chr7:50364876-50364903 |
| IKZF1 589 | chr7:50352980-50353007 | IKZF1 2200 | chr7:50364908-50364935 |
| IKZF1 590 | chr7:50352986-50353013 | IKZF1 2201 | chr7:50364912-50364939 |
| IKZF1 591 | chr7:50353003-50353030 | IKZF1 2202 | chr7:50364913-50364940 |
| IKZF1 592 | chr7:50353018-50353045 | IKZF1 2203 | chr7:50364917-50364944 |
| IKZF1 593 | chr7:50353023-50353050 | IKZF1 2204 | chr7:50364929-50364956 |
| IKZF1 594 | chr7:50353031-50353058 | IKZF1 2205 | chr7:50364930-50364957 |
| IKZF1 595 | chr7:50353032-50353059 | IKZF1 2206 | chr7:50364931-50364958 |
| IKZF1 596 | chr7:50353033-50353060 | IKZF1 2207 | chr7:50364962-50364989 |
| IKZF1 597 | chr7:50353041-50353068 | IKZF1 2208 | chr7:50364970-50364997 |
| IKZF1 598 | chr7:50353042-50353069 | IKZF1 2209 | chr7:50364971-50364998 |
| IKZF1 599 | chr7:50353081-50353108 | IKZF1 2210 | chr7:50364972-50364999 |
| IKZF1 600 | chr7:50353093-50353120 | IKZF1 2211 | chr7:50364977-50365004 |
| IKZF1 601 | chr7:50353094-50353121 | IKZF1 2212 | chr7:50365037-50365064 |
| IKZF 1 602 | chr7:50353095-50353122 | IKZF1 2213 | chr7:50365038-50365065 |
| IKZF1 603 | chr7:50353110-50353137 | IKZF1 2214 | chr7:50365059-50365086 |
| IKZF1 604 | chr7:50353114-50353141 | IKZF1 2215 | chr7:50365072-50365099 |
| IKZF1 605 | chr7:50353119-50353146 | IKZF1 2216 | chr7:50365078-50365105 |
| IKZF1 606 | chr7:50353126-50353153 | IKZF1 2217 | chr7:50365102-50365129 |
| IKZF1 607 | chr7:50353127-50353154 | IKZF1 2218 | chr7:50365104-50365131 |
| IKZF1 608 | chr7:50353139-50353166 | IKZF1 2219 | chr7:50365112-50365139 |
| IKZF1 609 | chr7:50353145-50353172 | IKZF 1 2220 | chr7:50365121-50365148 |
| IKZF1 610 | chr7:50353148-50353175 | IKZF 1 2221 | chr7:50365122-50365149 |
| IKZF1 611 | chr7:50353152-50353179 | **IKZF 1** 2222 | chr7:50365125-50365152 |
| IKZF1 612 | chr7:50353153-50353180 | IKZF 1 2223 | chr7:50365127-50365154 |
| IKZF1 613 | chr7:50353155-50353182 | IKZF 1 2224 | chr7:50365133-50365160 |
| IKZF1 614 | chr7:50353161-50353188 | IKZF 1 2225 | chr7:50365143-50365170 |
| IKZF1 615 | chr7:50353162-50353189 | IKZF1 2226 | chr7:50365144-50365171 |
| IKZF1 616 | chr7:50353163-50353190 | **IKZF 1** 2227 | chr7:50365151-50365178 |
| IKZF1 617 | chr7:50353164-50353191 | IKZF1 2228 | chr7:50365156-50365183 |
| IKZF1 618 | chr7:50353165-50353192 | IKZF1 2229 | chr7:50365157-50365184 |
| IKZF1 619 | chr7:50353193-50353220 | IKZF1 2230 | chr7:50365158-50365185 |
| IKZF1 620 | chr7:50353194-50353221 | **IKZF 1** 2231 | chr7:50365198-50365225 |
| IKZF1 621 | chr7:50353198-50353225 | **IKZF1** 2232 | chr7:50365223-50365250 |
| IKZF1 622 | chr7:50353199-50353226 | IKZF1 2233 | chr7:50365228-50365255 |
| IKZF1 623 | chr7:50353203-50353230 | IKZF1 2234 | chr7:50365260-50365287 |
| IKZF1 624 | chr7:50353206-50353233 | IKZF1 2235 | chr7:50365261-50365288 |
| IKZF1 625 | chr7:50353225-50353252 | IKZF1 2236 | chr7:50365262-50365289 |
| IKZF1 626 | chr7:50353226-50353253 | **IKZF1** 2237 | chr7:50365264-50365291 |
| IKZF1 627 | chr7:50353231-50353258 | **IKZF 1** 2238 | chr7:50365270-50365297 |
| IKZF1 628 | chr7:50353239-50353266 | IKZF1 2239 | chr7:50365277-50365304 |
| IKZF1 629 | chr7:50353248-50353275 | IKZF 1 2240 | chr7:50365278-50365305 |
| IKZF1 630 | chr7:50353288-50353315 | IKZF 1 2241 | chr7:503 653 08-503 653 3 5 |
| IKZF1 631 | chr7:50353293-50353320 | IKZF1 2242 | chr7:50365330-50365357 |
| IKZF1 632 | chr7:50353299-50353326 | IKZF1 2243 | chr7:50365378-50365405 |
| IKZF1 633 | chr7:50353310-50353337 | IKZF1 2244 | chr7:50365379-50365406 |
| IKZF1 634 | chr7:50353314-50353341 | IKZF1 2245 | chr7:50365394-50365421 |
| IKZF1 635 | chr7:50353325-50353352 | IKZF 1 2246 | chr7:50365414-50365441 |
| IKZF1 636 | chr7:50353344-50353371 | IKZF 1 2247 | chr7:50365420-50365447 |
| IKZF1 637 | chr7:50353353-50353380 | IKZF1 2248 | chr7:50365485-50365512 |
| IKZF1 638 | chr7:50353355-50353382 | IKZF1 2249 | chr7:50365491-50365518 |
| IKZF1 639 | chr7:50353357-50353384 | IKZF1 2250 | chr7:50365513-50365540 |
| IKZF1 640 | chr7:50353358-50353385 | IKZF1 2251 | chr7:50365514-50365541 |
| IKZF1 641 | chr7:50353359-50353386 | IKZF1 2252 | chr7:50365573-50365600 |
| IKZF1 642 | chr7:50353362-50353389 | IKZF1 2253 | chr7:50365581-50365608 |
| IKZF1 643 | chr7:50353363-50353390 | IKZF1 2254 | chr7:50365586-50365613 |
| IKZF1 644 | chr7:50353364-50353391 | IKZF1 2255 | chr7:50365596-50365623 |
| IKZF1 645 | chr7:50353367-50353394 | IKZF1 2256 | chr7:50365616-50365643 |
| IKZF1 646 | chr7:50353371-50353398 | IKZF1 2257 | chr7:50365621-50365648 |
| IKZF1 647 | chr7:50353374-50353401 | IKZF1 2258 | chr7:50365636-50365663 |
| IKZF1 648 | chr7:50353383-50353410 | IKZF1 2259 | chr7:50365657-50365684 |
| IKZF1 649 | chr7:50353387-50353414 | IKZF1 2260 | chr7:50365658-50365685 |
| IKZF 1 650 | chr7:50353398-50353425 | IKZF1 2261 | chr7:50365659-50365686 |
| IKZF1 651 | chr7:50353401-50353428 | IKZF1 2262 | chr7:50365684-50365711 |
| IKZF1 652 | chr7:50353407-50353434 | IKZF1 2263 | chr7:50365696-50365723 |
| IKZF1 653 | chr7:50353413-50353440 | IKZF1 2264 | chr7:50365704-50365731 |
| IKZF1 654 | chr7:50353415-50353442 | IKZF1 2265 | chr7:50365729-50365756 |
| IKZF1 655 | chr7:50353416-50353443 | IKZF1 2266 | chr7:50365738-50365765 |
| IKZF1 656 | chr7:50353417-50353444 | IKZF1 2267 | chr7:50365746-50365773 |
| IKZF1 657 | chr7:50353420-50353447 | IKZF1 2268 | chr7:50365754-50365781 |
| IKZF1 658 | chr7:50353422-50353449 | IKZF1 2269 | chr7:50365755-50365782 |
| IKZF1 659 | chr7:50353432-50353459 | IKZF1 2270 | chr7:50365761-50365788 |
| IKZF1 660 | chr7:50353433-50353460 | IKZF1 2271 | chr7:50365763-50365790 |
| IKZF1 661 | chr7:50353450-50353477 | IKZF1 2272 | chr7:50365764-50365791 |
| IKZF1 662 | chr7:50353451-50353478 | IKZF1 2273 | chr7:50365781-50365808 |
| IKZF1 663 | chr7:50353480-50353507 | IKZF1 2274 | chr7:50365782-50365809 |
| IKZF1 664 | chr7:50353481-50353508 | IKZF1 2275 | chr7:50365805-50365832 |
| IKZF1 665 | chr7:50353484-50353511 | IKZF1 2276 | chr7:50365837-50365864 |
| IKZF1 666 | chr7:50353487-50353514 | IKZF1 2277 | chr7:50365870-50365897 |
| IKZF1 667 | chr7:50353488-50353515 | IKZF1 2278 | chr7:50365871-50365898 |
| IKZF1 668 | chr7:50353495-50353522 | IKZF1 2279 | chr7:50365879-50365906 |
| IKZF1 669 | chr7:50353500-50353527 | IKZF1 2280 | chr7:50365880-50365907 |
| IKZF1 670 | chr7:50353503-50353530 | IKZF1 2281 | chr7:50365885-50365912 |
| IKZF1 671 | chr7:50353507-50353534 | IKZF1 2282 | chr7:50365901-50365928 |
| IKZF1 672 | chr7:50353508-50353535 | IKZF1 2283 | chr7:50365921-50365948 |
| IKZF1 673 | chr7:50353509-50353536 | IKZF1 2284 | chr7:50365922-50365949 |
| IKZF1 674 | chr7:50353518-50353545 | IKZF1 2285 | chr7:50365923-50365950 |
| IKZF1 675 | chr7:50353519-50353546 | IKZF1 2286 | chr7:50365941-50365968 |
| IKZF1 676 | chr7:50353522-50353549 | IKZF1 2287 | chr7:50365949-50365976 |
| IKZF1 677 | chr7:50353541-50353568 | IKZF1 2288 | chr7:50365956-50365983 |
| IKZF1 678 | chr7:50353543-50353570 | IKZF1 2289 | chr7:50365960-50365987 |
| IKZF1 679 | chr7:50353545-50353572 | IKZF1 2290 | chr7:50365966-50365993 |
| IKZF1 680 | chr7:50353548-50353575 | IKZF1 2291 | chr7:50365967-50365994 |
| IKZF1 681 | chr7:50353555-50353582 | IKZF1 2292 | chr7:50365969-50365996 |
| IKZF1 682 | chr7:50353557-50353584 | IKZF1 2293 | chr7:50365996-50366023 |
| IKZF1 683 | chr7:50353558-50353585 | IKZF1 2294 | chr7:50365997-50366024 |
| IKZF1 684 | chr7:50353561-50353588 | IKZF1 2295 | chr7:50366004-50366031 |
| IKZF1 685 | chr7:50353580-50353607 | IKZF1 2296 | chr7:50366018-50366045 |
| IKZF1 686 | chr7:50353585-50353612 | IKZF1 2297 | chr7:50366019-50366046 |
| IKZF1 687 | chr7:50353590-50353617 | IKZF1 2298 | chr7:50366033-50366060 |
| IKZF1 688 | chr7:50353593-50353620 | IKZF1 2299 | chr7:50366048-50366075 |
| IKZF1 689 | chr7:50353594-50353621 | IKZF1 2300 | chr7:50366059-50366086 |
| IKZF 1 690 | chr7:50353603-50353630 | IKZF1 2301 | chr7:50366060-50366087 |
| IKZF1 691 | chr7:50353607-50353634 | IKZF1 2302 | chr7:50366070-50366097 |
| IKZF1 692 | chr7:50353623-50353650 | IKZF1 2303 | chr7:50366071-50366098 |
| IKZF1 693 | chr7:50353624-50353651 | IKZF1 2304 | chr7:50366072-50366099 |
| IKZF1 694 | chr7:50353625-50353652 | IKZF1 2305 | chr7:50366083-50366110 |
| IKZF1 695 | chr7:50353626-50353653 | IKZF1 2306 | chr7:50366084-50366111 |
| IKZF1 696 | chr7:50353627-50353654 | IKZF1 2307 | chr7:50366087-50366114 |
| IKZF1 697 | chr7:50353628-50353655 | IKZF1 2308 | chr7:50366090-50366117 |
| IKZF1 698 | chr7:50353629-50353656 | IKZF1 2309 | chr7:50366091-50366118 |
| IKZF1 699 | chr7:50353645-50353672 | IKZF1 2310 | chr7:50366094-50366121 |
| IKZF1 700 | chr7:50353646-50353673 | IKZF1 2311 | chr7:50366095-50366122 |
| IKZF1 701 | chr7:50353651-50353678 | IKZF1 2312 | chr7:50366100-50366127 |
| IKZF1 702 | chr7:50353653-50353680 | IKZF1 2313 | chr7:50366101-50366128 |
| IKZF1 703 | chr7:50353663-50353690 | IKZF1 2314 | chr7:50366125-50366152 |
| IKZF1 704 | chr7:50353674-5035370 1 | IKZF1 2315 | chr7:50366143-50366170 |
| IKZF1 705 | chr7:50353684-50353711 | IKZF1 2316 | chr7:50366167-50366194 |
| IKZF1 706 | chr7:50353685-50353712 | IKZF1 2317 | chr7:50366196-50366223 |
| IKZF1 707 | chr7:50353686-50353713 | IKZF1 2318 | chr7:50366197-50366224 |
| IKZF1 708 | chr7:50353697-50353724 | IKZF1 2319 | chr7:50366198-50366225 |
| IKZF1 709 | chr7:50353776-50353803 | IKZF1 2320 | chr7:50366199-50366226 |
| IKZF1 710 | chr7:50353783-50353810 | IKZF1 2321 | chr7:50366215-50366242 |
| IKZF1 711 | chr7:50353784-50353811 | IKZF1 2322 | chr7:50366228-50366255 |
| IKZF1 712 | chr7:50353786-50353813 | IKZF1 2323 | chr7:50366229-50366256 |
| IKZF1 713 | chr7:50353791-50353818 | IKZF1 2324 | chr7:50366242-50366269 |
| IKZF1 714 | chr7:50353804-50353831 | IKZF1 2325 | chr7:50366287-50366314 |
| IKZF1 715 | chr7:50353805-50353832 | IKZF1 2326 | chr7:50366302-50366329 |
| IKZF1 716 | chr7: 50353 809-50353 836 | IKZF1 2327 | chr7:50366303-50366330 |
| IKZF1 717 | chr7:50353827-50353854 | IKZF1 2328 | chr7:50366324-50366351 |
| IKZF1 718 | chr7:50353833-50353860 | IKZF1 2329 | chr7:50366334-50366361 |
| IKZF1 719 | chr7:50353834-50353861 | IKZF1 2330 | chr7:50366365-50366392 |
| IKZF1 720 | chr7:50353840-50353867 | IKZF1 2331 | chr7:50366376-50366403 |
| IKZF1 721 | chr7:50353845-50353872 | IKZF1 2332 | chr7:50366377-50366404 |
| IKZF1 722 | chr7:50353846-50353873 | IKZF1 2333 | chr7:50366381-50366408 |
| IKZF1 723 | chr7:50353868-50353895 | IKZF1 2334 | chr7:50367988-50368015 |
| IKZF1 724 | chr7:50353869-50353896 | IKZF1 2335 | chr7:50367989-50368016 |
| IKZF1 725 | chr7:50353875-50353902 | IKZF1 2336 | chr7:50367990-50368017 |
| IKZF1 726 | chr7:50353876-50353903 | IKZF1 2337 | chr7:50367991-50368018 |
| IKZF1 727 | chr7:50353877-50353904 | IKZF1 2338 | chr7:50368004-50368031 |
| IKZF1 728 | chr7:50353878-50353905 | IKZF1 2339 | chr7:50368005-50368032 |
| IKZF1 729 | chr7:50353882-50353909 | IKZF1 2340 | chr7:50368037-50368064 |
| IKZF1 730 | chr7:50353883-50353910 | IKZF1 2341 | chr7:50368050-50368077 |
| IKZF1 731 | chr7:50353884-50353911 | IKZF1 2342 | chr7:50368051-50368078 |
| IKZF1 732 | chr7:50353885-50353912 | IKZF1 2343 | chr7:50368061-50368088 |
| IKZF1 733 | chr7:50353891-50353918 | IKZF1 2344 | chr7:50368077-50368104 |
| IKZF1 734 | chr7:50353901-50353928 | IKZF1 2345 | chr7:50368103-50368130 |
| IKZF1 735 | chr7:50353902-50353929 | IKZF1 2346 | chr7:50368112-50368139 |
| IKZF1 736 | chr7:50353909-50353936 | IKZF1 2347 | chr7:50368142-50368169 |
| IKZF1 737 | chr7:50353910-50353937 | IKZF1 2348 | chr7:50368152-50368179 |
| IKZF1 738 | chr7:50353911-50353938 | IKZF1 2349 | chr7:50368153-50368180 |
| IKZF1 739 | chr7:50353912-50353939 | IKZF1 2350 | chr7:50368154-50368181 |
| IKZF1 740 | chr7:50353913-50353940 | IKZF1 2351 | chr7:50368155-50368182 |
| IKZF1 741 | chr7:50353920-50353947 | IKZF1 2352 | chr7:50368186-50368213 |
| IKZF1 742 | chr7:50353925-50353952 | IKZF1 2353 | chr7:50368187-50368214 |
| IKZF1 743 | chr7:50353931-50353958 | IKZF1 2354 | chr7:50368195-50368222 |
| IKZF1 744 | chr7:50353932-50353959 | IKZF1 2355 | chr7:50368199-50368226 |
| IKZF1 745 | chr7:50353933-50353960 | IKZF1 2356 | chr7:50368216-50368243 |
| IKZF1 746 | chr7:50353941-50353968 | IKZF1 2357 | chr7:50368222-50368249 |
| IKZF1 747 | chr7:50353942-50353969 | IKZF1 2358 | chr7:50368223-50368250 |
| IKZF1 748 | chr7:50353943-50353970 | IKZF1 2359 | chr7:50368233-50368260 |
| IKZF1 749 | chr7:50353946-50353973 | IKZF1 2360 | chr7:50368241-50368268 |
| IKZF1 750 | chr7:50353948-50353975 | IKZF1 2361 | chr7:50368242-50368269 |
| IKZF1 751 | chr7:50353950-50353977 | IKZF1 2362 | chr7:50368249-50368276 |
| IKZF1 752 | chr7:50353971-50353998 | IKZF1 2363 | chr7:50368255-50368282 |
| IKZF1 753 | chr7:50353974-5035400 1 | IKZF1 2364 | chr7:50368264-50368291 |
| IKZF1 754 | chr7:50354002-50354029 | IKZF1 2365 | chr7:50368268-50368295 |
| IKZF1 755 | chr7:50354005-50354032 | IKZF1 2366 | chr7:50368269-50368296 |
| IKZF1 756 | chr7:50354006-50354033 | IKZF1 2367 | chr7:50368273-50368300 |
| IKZF1 757 | chr7:50354009-50354036 | IKZF1 2368 | chr7:50368293-50368320 |
| IKZF1 758 | chr7:50354010-50354037 | IKZF1 2369 | chr7:50368294-50368321 |
| IKZF1 759 | chr7:50354011-50354038 | IKZF1 2370 | chr7:50368319-50368346 |
| IKZF1 760 | chr7: 503540 12-50354039 | IKZF1 2371 | chr7:50368364-50368391 |
| IKZF1 761 | chr7:50354013-50354040 | IKZF1 2372 | chr7:50368372-50368399 |
| IKZF1 762 | chr7:50354025-50354052 | IKZF1 2373 | chr7:50368373-50368400 |
| IKZF1 763 | chr7:50354030-50354057 | IKZF1 2374 | chr7:50368401-50368428 |
| IKZF1 764 | chr7:50354047-50354074 | IKZF1 2375 | chr7:50368409-50368436 |
| IKZF1 765 | chr7:50354053-50354080 | IKZF1 2376 | chr7:50368416-50368443 |
| IKZF1 766 | chr7:50354063-50354090 | IKZF1 2377 | chr7:50368437-50368464 |
| IKZF1 767 | chr7:50354068-50354095 | IKZF1 2378 | chr7:50368438-50368465 |
| IKZF1 768 | chr7:50354084-50354111 | IKZF1 2379 | chr7:50368443-50368470 |
| IKZF1 769 | chr7:50354085-50354112 | IKZF1 2380 | chr7:50368449-50368476 |
| IKZF1 770 | chr7:50354086-50354113 | IKZF1 2381 | chr7:50368470-50368497 |
| IKZF1 771 | chr7:50354090-50354117 | IKZF1 2382 | chr7:50368476-50368503 |
| IKZF1 772 | chr7:50354099-50354126 | IKZF1 2383 | chr7:50368513-50368540 |
| IKZF1 773 | chr7:50354100-50354127 | IKZF1 2384 | chr7:50368521-50368548 |
| IKZF1 774 | chr7:50354109-50354136 | IKZF1 2385 | chr7:50368535-50368562 |
| IKZF1 775 | chr7:50354114-50354141 | IKZF1 2386 | chr7:50368541-50368568 |
| IKZF1 776 | chr7:50354119-50354146 | IKZF1 2387 | chr7:50368544-50368571 |
| IKZF1 777 | chr7:50354123-50354150 | IKZF1 2388 | chr7:50368549-50368576 |
| IKZF1 778 | chr7:50354131-50354158 | IKZF1 2389 | chr7:50368573-50368600 |
| IKZF1 779 | chr7:50354136-50354163 | IKZF1 2390 | chr7:50368579-50368606 |
| IKZF1 780 | chr7:50354137-50354164 | IKZF1 2391 | chr7:50368583-50368610 |
| IKZF1 781 | chr7:50354146-50354173 | IKZF1 2392 | chr7:50368592-50368619 |
| IKZF1 782 | chr7:50354168-50354195 | IKZF1 2393 | chr7:50368603-50368630 |
| IKZF1 783 | chr7:50354173-50354200 | IKZF1 2394 | chr7:50368604-50368631 |
| IKZF1 784 | chr7:50354176-50354203 | IKZF1 2395 | chr7:50368612-50368639 |
| IKZF1 785 | chr7:50354177-50354204 | IKZF1 2396 | chr7:50368636-50368663 |
| IKZF1 786 | chr7:50354180-50354207 | IKZF1 2397 | chr7:50368676-50368703 |
| IKZF1 787 | chr7:50354189-50354216 | IKZF1 2398 | chr7:50368677-50368704 |
| IKZF1 788 | chr7:50354190-50354217 | IKZF1 2399 | chr7:50368711-50368738 |
| IKZF1 789 | chr7:50354191-50354218 | IKZF1 2400 | chr7:50368776-50368803 |
| IKZF1 790 | chr7:50354200-50354227 | IKZF1 2401 | chr7:50368778-50368805 |
| IKZF1 791 | chr7:50354209-50354236 | IKZF1 2402 | chr7:50368795-50368822 |
| IKZF1 792 | chr7:50354210-50354237 | IKZF1 2403 | chr7:50368796-50368823 |
| IKZF1 793 | chr7:50354211-50354238 | IKZF1 2404 | chr7:50368797-50368824 |
| IKZF1 794 | chr7:50354219-50354246 | IKZF1 2405 | chr7:50368805-50368832 |
| IKZF1 795 | chr7:50354222-50354249 | IKZF1 2406 | chr7:50368820-50368847 |
| IKZF1 796 | chr7:50354232-50354259 | IKZF1 2407 | chr7:50368826-50368853 |
| IKZF1 797 | chr7:50354248-50354275 | IKZF1 2408 | chr7:50368863-50368890 |
| IKZF1 798 | chr7:50354264-50354291 | IKZF1 2409 | chr7:50368901-50368928 |
| IKZF1 799 | chr7:50354276-50354303 | IKZF1 2410 | chr7:50368908-50368935 |
| IKZF1 800 | chr7:50354277-50354304 | IKZF1 2411 | chr7:50368916-50368943 |
| IKZF1 801 | chr7:50354284-50354311 | IKZF1 2412 | chr7:50368934-50368961 |
| IKZF1 802 | chr7:50354285-50354312 | IKZF1 2413 | chr7:50368939-50368966 |
| IKZF1 803 | chr7:50354307-50354334 | IKZF1 2414 | chr7:50368943-50368970 |
| IKZF1 804 | chr7:50354308-50354335 | IKZF1 2415 | chr7:50368990-50369017 |
| IKZF1 805 | chr7:50354316-50354343 | IKZF1 2416 | chr7:50369026-50369053 |
| IKZF1 806 | chr7:50354317-50354344 | IKZF1 2417 | chr7:50369030-50369057 |
| IKZF1 807 | chr7:50354318-50354345 | IKZF1 2418 | chr7:50369068-50369095 |
| IKZF1 808 | chr7:50354320-50354347 | IKZF1 2419 | chr7:50369077-50369104 |
| IKZF1 809 | chr7:50354321-50354348 | IKZF1 2420 | chr7:50369101-50369128 |
| IKZF1 810 | chr7:50354324-50354351 | IKZF1 2421 | chr7:50369168-50369195 |
| IKZF1 811 | chr7:50354326-50354353 | IKZF1 2422 | chr7:50369174-50369201 |
| IKZF1 812 | chr7:50354329-50354356 | IKZF1 2423 | chr7:50369285-50369312 |
| IKZF1 813 | chr7:50354330-50354357 | IKZF1 2424 | chr7:50369286-50369313 |
| IKZF1 814 | chr7:50354331-50354358 | IKZF1 2425 | chr7:50369290-50369317 |
| IKZF1 815 | chr7:50354332-50354359 | IKZF1 2426 | chr7:50369324-50369351 |
| IKZF1 816 | chr7:50354333-50354360 | IKZF1 2427 | chr7:50369346-50369373 |
| IKZF1 817 | chr7:50354340-50354367 | IKZF1 2428 | chr7:50369347-50369374 |
| IKZF1 818 | chr7:50354349-50354376 | IKZF1 2429 | chr7:50369359-50369386 |
| IKZF1 819 | chr7:50354354-50354381 | IKZF1 2430 | chr7:50369418-50369445 |
| IKZF1 820 | chr7:50354368-50354395 | IKZF1 2431 | chr7:50369500-50369527 |
| IKZF1 821 | chr7:50354387-50354414 | IKZF1 2432 | chr7:50369508-50369535 |
| IKZF1 822 | chr7:50354395-50354422 | IKZF1 2433 | chr7:50369509-50369536 |
| IKZF1 823 | chr7:50354401-50354428 | IKZF1 2434 | chr7:50369516-50369543 |
| IKZF1 824 | chr7:50354402-50354429 | IKZF1 2435 | chr7:50369517-50369544 |
| IKZF1 825 | chr7:50354403-50354430 | IKZF1 2436 | chr7:50369518-50369545 |
| IKZF1 826 | chr7:50354408-50354435 | IKZF1 2437 | chr7:50369528-50369555 |
| IKZF1 827 | chr7:50354414-50354441 | IKZF1 2438 | chr7:50369536-50369563 |
| IKZF1 828 | chr7:50354417-50354444 | IKZF1 2439 | chr7:50369572-50369599 |
| IKZF1 829 | chr7:50354418-50354445 | IKZF1 2440 | chr7:50369576-50369603 |
| IKZF1 830 | chr7:50354419-50354446 | IKZF1 2441 | chr7:50369577-50369604 |
| IKZF1 831 | chr7:50354420-50354447 | IKZF1 2442 | chr7:50369578-50369605 |
| IKZF1 832 | chr7:50354429-50354456 | IKZF1 2443 | chr7:50369579-50369606 |
| IKZF1 833 | chr7:50354430-50354457 | IKZF1 2444 | chr7:50369580-50369607 |
| IKZF1 834 | chr7:50354431-50354458 | IKZF1 2445 | chr7:50369593-50369620 |
| IKZF1 835 | chr7:50354436-50354463 | IKZF1 2446 | chr7:50369600-50369627 |
| IKZF1 836 | chr7:50354437-50354464 | IKZF1 2447 | chr7:50369608-50369635 |
| IKZF1 837 | chr7:50354443-50354470 | IKZF1 2448 | chr7:50369617-50369644 |
| IKZF1 838 | chr7:50354444-50354471 | IKZF1 2449 | chr7:50369620-50369647 |
| IKZF1 839 | chr7:50354466-50354493 | IKZF1 2450 | chr7:50369644-50369671 |
| IKZF1 840 | chr7:50354474-50354501 | IKZF1 2451 | chr7:50369645-50369672 |
| IKZF1 841 | chr7:50354475-50354502 | IKZF1 2452 | chr7:50369646-50369673 |
| IKZF1 842 | chr7:50354476-50354503 | IKZF1 2453 | chr7:50369647-50369674 |
| IKZF1 843 | chr7:50354477-50354504 | IKZF1 2454 | chr7:50369653-50369680 |
| IKZF1 844 | chr7:50354486-50354513 | IKZF1 2455 | chr7:50369654-50369681 |
| IKZF1 845 | chr7:50354489-50354516 | IKZF1 2456 | chr7:50369662-50369689 |
| IKZF1 846 | chr7:50354493-50354520 | IKZF1 2457 | chr7:50369691-50369718 |
| IKZF1 847 | chr7:50354494-50354521 | IKZF1 2458 | chr7:50369692-50369719 |
| IKZF1 848 | chr7:50354497-50354524 | IKZF1 2459 | chr7:50369693-50369720 |
| IKZF1 849 | chr7:50354505-50354532 | IKZF1 2460 | chr7:50369700-50369727 |
| IKZF1 850 | chr7:50354510-50354537 | IKZF1 2461 | chr7:50369701-50369728 |
| IKZF1 851 | chr7:50354522-50354549 | IKZF1 2462 | chr7:50369702-50369729 |
| IKZF1 852 | chr7:50354523-50354550 | IKZF1 2463 | chr7:50369703-50369730 |
| IKZF1 853 | chr7:50354578-50354605 | IKZF1 2464 | chr7:50369713-50369740 |
| IKZF1 854 | chr7:50354627-50354654 | IKZF1 2465 | chr7:50369716-50369743 |
| IKZF1 855 | chr7:50354629-50354656 | IKZF1 2466 | chr7:50369719-50369746 |
| IKZF1 856 | chr7:50354635-50354662 | IKZF1 2467 | chr7:50369720-50369747 |
| IKZF1 857 | chr7:50354636-50354663 | IKZF1 2468 | chr7:50369731-50369758 |
| IKZF1 858 | chr7:50354643-50354670 | IKZF1 2469 | chr7:50369757-50369784 |
| IKZF1 859 | chr7:50354650-50354677 | IKZF1 2470 | chr7:50369801-50369828 |
| IKZF1 860 | chr7:50354668-50354695 | IKZF1 2471 | chr7:50369811-50369838 |
| IKZF1 861 | chr7:50354674-5035470 1 | IKZF1 2472 | chr7:50369815-50369842 |
| IKZF1 862 | chr7:50354675-50354702 | IKZF1 2473 | chr7:50369816-50369843 |
| IKZF1 863 | chr7:50354686-50354713 | IKZF1 2474 | chr7:50369828-50369855 |
| IKZF1 864 | chr7:50354702-50354729 | IKZF1 2475 | chr7:50369839-50369866 |
| IKZF1 865 | chr7:50354703-50354730 | IKZF1 2476 | chr7:50370026-50370053 |
| IKZF1 866 | chr7:50354722-50354749 | IKZF1 2477 | chr7:50370047-50370074 |
| IKZF1 867 | chr7:50354729-50354756 | IKZF1 2478 | chr7:50370076-50370103 |
| IKZF1 868 | chr7:50354733-50354760 | IKZF1 2479 | chr7:503 70106-50370133 |
| IKZF1 869 | chr7:50354737-50354764 | IKZF1 2480 | chr7:50370131-50370158 |
| IKZF1 870 | chr7:50354738-50354765 | IKZF1 2481 | chr7:50370145-50370172 |
| IKZF1 871 | chr7:50354743-50354770 | IKZF1 2482 | chr7:50370173-50370200 |
| IKZF1 872 | chr7:50354745-50354772 | IKZF1 2483 | chr7:50370175-50370202 |
| IKZF1 873 | chr7:50354757-50354784 | IKZF1 2484 | chr7:50370191-50370218 |
| IKZF1 874 | chr7:50354758-50354785 | IKZF1 2485 | chr7:50370237-50370264 |
| IKZF1 875 | chr7:50354762-50354789 | IKZF1 2486 | chr7:50370256-50370283 |
| IKZF1 876 | chr7:50354763-50354790 | IKZF1 2487 | chr7:50370261-50370288 |
| IKZF1 877 | chr7:50354764-50354791 | IKZF1 2488 | chr7:50370262-50370289 |
| IKZF1 878 | chr7:50354768-50354795 | IKZF1 2489 | chr7:50370273-50370300 |
| IKZF1 879 | chr7:50354775-50354802 | IKZF1 2490 | chr7:50370289-50370316 |
| IKZF1 880 | chr7:50354779-50354806 | IKZF1 2491 | chr7:50370294-50370321 |
| IKZF1 881 | chr7:50354780-50354807 | IKZF1 2492 | chr7:50370297-50370324 |
| IKZF1 882 | chr7:50354781-50354808 | IKZF1 2493 | chr7:50370298-50370325 |
| IKZF1 883 | chr7:50354821-50354848 | IKZF1 2494 | chr7:50370299-50370326 |
| IKZF1 884 | chr7:50354824-50354851 | IKZF1 2495 | chr7:50376511-50376538 |
| IKZF1 885 | chr7:50354825-50354852 | IKZF1 2496 | chr7:50376514-50376541 |
| IKZF1 886 | chr7:50354826-50354853 | IKZF1 2497 | chr7:50376533-50376560 |
| IKZF1 887 | chr7:50354829-50354856 | IKZF1 2498 | chr7:50376548-50376575 |
| IKZF1 888 | chr7:50354837-50354864 | IKZF1 2499 | chr7:50376549-50376576 |
| IKZF1 889 | chr7:50354838-50354865 | IKZF1 2500 | chr7:50376569-50376596 |
| IKZF1 890 | chr7:50354844-50354871 | IKZF1 2501 | chr7:50376570-50376597 |
| IKZF1 891 | chr7:50354845-50354872 | IKZF1 2502 | chr7:50376571-50376598 |
| IKZF1 892 | chr7:50354856-50354883 | IKZF1 2503 | chr7:50376581-50376608 |
| IKZF1 893 | chr7:50354875-50354902 | IKZF1 2504 | chr7:50376583-50376610 |
| IKZF1 894 | chr7:50354918-50354945 | IKZF1 2505 | chr7:50376586-50376613 |
| IKZF1 895 | chr7:50354919-50354946 | IKZF1 2506 | chr7:50376610-50376637 |
| IKZF1 896 | chr7:50354923-50354950 | IKZF1 2507 | chr7:50376611-50376638 |
| IKZF1 897 | chr7:50354942-50354969 | IKZF1 2508 | chr7:50376626-50376653 |
| IKZF1 898 | chr7:50354969-50354996 | IKZF1 2509 | chr7:50376632-50376659 |
| IKZF1 899 | chr7:50354975-50355002 | IKZF1 2510 | chr7:50376636-50376663 |
| IKZF1 900 | chr7:50354982-50355009 | IKZF1 2511 | chr7:50376637-50376664 |
| IKZF1 901 | chr7:50354987-50355014 | IKZF1 2512 | chr7:50376644-50376671 |
| IKZF1 902 | chr7:50354994-50355021 | IKZF1 2513 | chr7:50376652-50376679 |
| IKZF1 903 | chr7:50355002-50355029 | IKZF1 2514 | chr7:50376656-50376683 |
| IKZF1 904 | chr7:50355003-50355030 | IKZF1 2515 | chr7:50376657-50376684 |
| IKZF1 905 | chr7:50355007-50355034 | IKZF1 2516 | chr7:50376661-50376688 |
| IKZF1 906 | chr7: 503550 12-50355039 | IKZF1 2517 | chr7:50376662-50376689 |
| IKZF1 907 | chr7:50355015-50355042 | IKZF1 2518 | chr7:50376666-50376693 |
| IKZF1 908 | chr7:50355016-50355043 | IKZF1 2519 | chr7:50376668-50376695 |
| IKZF1 909 | chr7:50355017-50355044 | IKZF1 2520 | chr7:50376671-50376698 |
| IKZF1 910 | chr7:50355018-50355045 | IKZF1 2521 | chr7:50376689-50376716 |
| IKZF1 911 | chr7:50355023-50355050 | IKZF1 2522 | chr7:50376709-50376736 |
| IKZF1 912 | chr7:50355028-50355055 | IKZF1 2523 | chr7:50376713-50376740 |
| IKZF1 913 | chr7:50355029-50355056 | IKZF1 2524 | chr7:50376714-50376741 |
| IKZF1 914 | chr7:50355035-50355062 | IKZF1 2525 | chr7:50376728-50376755 |
| IKZF1 915 | chr7:50355036-50355063 | IKZF1 2526 | chr7:50376729-50376756 |
| IKZF1 916 | chr7:50355037-50355064 | IKZF1 2527 | chr7:50376745-50376772 |
| IKZF1 917 | chr7:50355038-50355065 | IKZF1 2528 | chr7:50376757-50376784 |
| IKZF1 918 | chr7:50355042-50355069 | IKZF1 2529 | chr7:50376758-50376785 |
| IKZF1 919 | chr7:50355045-50355072 | IKZF1 2530 | chr7:50376767-50376794 |
| IKZF1 920 | chr7:50355051-50355078 | IKZF1 2531 | chr7:50376772-50376799 |
| IKZF1 921 | chr7:50355078-50355105 | IKZF1 2532 | chr7:50376777-50376804 |
| IKZF1 922 | chr7:50355079-50355106 | IKZF1 2533 | chr7:50376786-50376813 |
| IKZF1 923 | chr7:50355083-50355110 | IKZF1 2534 | chr7:50382513-50382540 |
| IKZF1 924 | chr7:50355090-50355117 | IKZF1 2535 | chr7:50382516-50382543 |
| IKZF1 925 | chr7:50355095-50355122 | IKZF1 2536 | chr7:50382520-50382547 |
| IKZF1 926 | chr7:50355097-50355124 | IKZF1 2537 | chr7:50382524-50382551 |
| IKZF1 927 | chr7:50355098-50355125 | IKZF1 2538 | chr7:50382531-50382558 |
| IKZF1 928 | chr7:50355099-50355126 | IKZF1 2539 | chr7:50382535-50382562 |
| IKZF1 929 | chr7:50355106-50355133 | IKZF1 2540 | chr7:50382536-50382563 |
| IKZF1 930 | chr7:50355115-50355142 | IKZF1 2541 | chr7:50382543-50382570 |
| IKZF1 931 | chr7:50355127-50355154 | IKZF1 2542 | chr7:50382544-50382571 |
| IKZF1 932 | chr7:50355137-50355164 | IKZF1 2543 | chr7:50382545-50382572 |
| IKZF1 933 | chr7:50355140-50355167 | IKZF1 2544 | chr7:50382548-50382575 |
| IKZF1 934 | chr7:50355143-50355170 | IKZF1 2545 | chr7:50382549-50382576 |
| IKZF1 935 | chr7:50355165-50355192 | IKZF1 2546 | chr7:50382553-50382580 |
| IKZF1 936 | chr7:50355168-50355195 | IKZF1 2547 | chr7:50382563-50382590 |
| IKZF1 937 | chr7:50355180-50355207 | IKZF1 2548 | chr7:50382564-50382591 |
| IKZF1 938 | chr7:50355181-50355208 | IKZF1 2549 | chr7:50382573-50382600 |
| IKZF1 939 | chr7:50355182-50355209 | IKZF1 2550 | chr7:50382577-50382604 |
| IKZF1 940 | chr7:50355184-50355211 | IKZF1 2551 | chr7:50382582-50382609 |
| IKZF1 941 | chr7:50355185-50355212 | IKZF1 2552 | chr7:50382583-50382610 |
| IKZF1 942 | chr7:50355186-50355213 | IKZF1 2553 | chr7:50382595-50382622 |
| IKZF1 943 | chr7:50355187-50355214 | IKZF1 2554 | chr7:50382597-50382624 |
| IKZF1 944 | chr7:50355200-50355227 | IKZF1 2555 | chr7:50382598-50382625 |
| IKZF1 945 | chr7:50355217-50355244 | IKZF1 2556 | chr7:50382599-50382626 |
| IKZF1 946 | chr7:50355218-50355245 | IKZF1 2557 | chr7:50382601-50382628 |
| IKZF1 947 | chr7:50355227-50355254 | IKZF1 2558 | chr7:50382621-50382648 |
| IKZF1 948 | chr7:50355228-50355255 | IKZF1 2559 | chr7:50382632-50382659 |
| IKZF1 949 | chr7:50355253-50355280 | IKZF1 2560 | chr7:50382633-50382660 |
| IKZF1 950 | chr7:50355259-50355286 | IKZF1 2561 | chr7:50382643-50382670 |
| IKZF1 951 | chr7:50355260-50355287 | IKZF1 2562 | chr7:50382646-50382673 |
| IKZF1 952 | chr7:50355263-50355290 | IKZF1 2563 | chr7:50382647-50382674 |
| IKZF1 953 | chr7:50355264-50355291 | IKZF1 2564 | chr7:50382660-50382687 |
| IKZF1 954 | chr7:50355269-50355296 | IKZF1 2565 | chr7:50382664-50382691 |
| IKZF1 955 | chr7:50355282-50355309 | IKZF1 2566 | chr7:50382667-50382694 |
| IKZF1 956 | chr7:50355291-50355318 | IKZF1 2567 | chr7:50382670-50382697 |
| IKZF1 957 | chr7:50355302-50355329 | IKZF1 2568 | chr7:50382678-50382705 |
| IKZF1 958 | chr7:50355303-50355330 | IKZF1 2569 | chr7:50382679-50382706 |
| IKZF1 959 | chr7:50355306-50355333 | IKZF1 2570 | chr7:50382681-50382708 |
| IKZF1 960 | chr7:50355311-50355338 | IKZF1 2571 | chr7:50382685-50382712 |
| IKZF1 961 | chr7:50355329-50355356 | IKZF1 2572 | chr7:50382688-50382715 |
| IKZF1 962 | chr7:50355330-50355357 | IKZF1 2573 | chr7:50382691-50382718 |
| IKZF1 963 | chr7:50355331-50355358 | IKZF1 2574 | chr7:50382693-50382720 |
| IKZF1 964 | chr7:50355359-50355386 | IKZF1 2575 | chr7:50382704-50382731 |
| IKZF1 965 | chr7:50355363-50355390 | IKZF1 2576 | chr7:50382705-50382732 |
| IKZF1 966 | chr7:50355364-50355391 | IKZF1 2577 | chr7:50382706-50382733 |
| IKZF1 967 | chr7:50355368-50355395 | IKZF1 2578 | chr7:50387321-50387348 |
| IKZF1 968 | chr7:50355369-50355396 | IKZF1 2579 | chr7:50387339-50387366 |
| IKZF1 969 | chr7:50355370-50355397 | IKZF1 2580 | chr7:50387348-50387375 |
| IKZF1 970 | chr7:50355376-50355403 | IKZF1 2581 | chr7:50387353-50387380 |
| IKZF1 971 | chr7:50355394-50355421 | IKZF1 2582 | chr7:50387376-50387403 |
| IKZF1 972 | chr7:50355399-50355426 | IKZF1 2583 | chr7:50387380-50387407 |
| IKZF1 973 | chr7:50355400-50355427 | IKZF1 2584 | chr7:50387410-50387437 |
| IKZF1 974 | chr7:50355404-50355431 | IKZF1 2585 | chr7:50387419-50387446 |
| IKZF1 975 | chr7:50355406-50355433 | IKZF1 2586 | chr7:50387420-50387447 |
| IKZF1 976 | chr7:50355410-50355437 | IKZF1 2587 | chr7:50387424-50387451 |
| IKZF1 977 | chr7:50355411-50355438 | IKZF1 2588 | chr7:50387428-50387455 |
| IKZF1 978 | chr7:50355414-50355441 | IKZF1 2589 | chr7:50387429-50387456 |
| IKZF1 979 | chr7:50355422-50355449 | IKZF1 2590 | chr7:50387444-50387471 |
| IKZF1 980 | chr7:50355427-50355454 | IKZF1 2591 | chr7:50387448-50387475 |
| IKZF1 981 | chr7:50355430-50355457 | IKZF1 2592 | chr7:50387452-50387479 |
| IKZF1 982 | chr7:50355436-50355463 | IKZF1 2593 | chr7:50387461-50387488 |
| IKZF1 983 | chr7:50355441-50355468 | IKZF1 2594 | chr7:50387464-50387491 |
| IKZF1 984 | chr7:50355459-50355486 | IKZF1 2595 | chr7:50387466-50387493 |
| IKZF1 985 | chr7:50355460-50355487 | IKZF1 2596 | chr7:50387467-50387494 |
| IKZF1 986 | chr7:50355473-50355500 | IKZF1 2597 | chr7:50391708-50391735 |
| IKZF1 987 | chr7:50355474-50355501 | IKZF1 2598 | chr7:50391734-50391761 |
| IKZF1 988 | chr7:50355475-50355502 | IKZF1 2599 | chr7:50391756-50391783 |
| IKZF1 989 | chr7:50355476-50355503 | IKZF1 2600 | chr7:50391769-50391796 |
| IKZF1 990 | chr7:50355488-50355515 | IKZF1 2601 | chr7:50391779-50391806 |
| IKZF1 991 | chr7:50355493-50355520 | IKZF1 2602 | chr7:50391828-50391855 |
| IKZF1 992 | chr7:50355494-50355521 | IKZF1 2603 | chr7:50391837-50391864 |
| IKZF1 993 | chr7:50355495-50355522 | IKZF1 2604 | chr7:50391848-50391875 |
| IKZF1 994 | chr7:50355497-50355524 | IKZF1 2605 | chr7:50399891-50399918 |
| IKZF1 995 | chr7:50355507-50355534 | IKZF1 2606 | chr7:50399892-50399919 |
| IKZF1 996 | chr7:50355508-50355535 | IKZF1 2607 | chr7:50399893-50399920 |
| IKZF1 997 | chr7:50355510-50355537 | IKZF1 2608 | chr7:50399899-50399926 |
| IKZF1 998 | chr7:50355514-50355541 | IKZF1 2609 | chr7:50399900-50399927 |
| IKZF1 999 | chr7:50355515-50355542 | IKZF1 2610 | chr7:50399904-50399931 |
| IKZF 1 1000 | chr7:50355516-50355543 | IKZF1 2611 | chr7:50399912-50399939 |
| IKZF 1 1001 | chr7:50355544-50355571 | IKZF1 2612 | chr7:50399928-50399955 |
| IKZF 1 1002 | chr7:50355557-50355584 | IKZF1 2613 | chr7:50399933-50399960 |
| IKZF 1 1003 | chr7:50355595-50355622 | IKZF1 2614 | chr7:50399941-50399968 |
| IKZF 1 1004 | chr7:50355629-50355656 | IKZF1 2615 | chr7:50399942-50399969 |
| IKZF 1 1005 | chr7:50355668-50355695 | IKZF1 2616 | chr7:50399944-50399971 |
| IKZF 1 1006 | chr7:50355679-50355706 | IKZF1 2617 | chr7:50399957-50399984 |
| IKZF 1 1007 | chr7:50355680-50355707 | IKZF1 2618 | chr7:50399974-50400001 |
| IKZF 1 1008 | chr7:50355697-50355724 | IKZF1 2619 | chr7:50399990-50400017 |
| IKZF 1 1009 | chr7:50355715-50355742 | IKZF1 2620 | chr7:50399991-50400018 |
| IKZF 1 1010 | chr7:50355716-50355743 | IKZF1 2621 | chr7:50400003-50400030 |
| IKZF 1 1011 | chr7:50355717-50355744 | IKZF1 2622 | chr7:50400007-50400034 |
| IKZF 1 1012 | chr7:50355718-50355745 | IKZF1 2623 | chr7:50400008-50400035 |
| IKZF 1 1013 | chr7:50355737-50355764 | IKZF1 2624 | chr7:50400009-50400036 |
| IKZF 1 1014 | chr7:50355746-50355773 | IKZF1 2625 | chr7:50400010-50400037 |
| IKZF 1 1015 | chr7:50355749-50355776 | IKZF1 2626 | chr7:50400020-50400047 |
| IKZF 1 1016 | chr7:50355752-50355779 | IKZF1 2627 | chr7:50400030-50400057 |
| IKZF 1 1017 | chr7:50355753-50355780 | IKZF1 2628 | chr7:50400031-50400058 |
| IKZF 1 1018 | chr7:50355760-50355787 | IKZF1 2629 | chr7:50400038-50400065 |
| IKZF 1 1019 | chr7:50355787-50355814 | IKZF1 2630 | chr7:50400044-50400071 |
| IKZF 1 1020 | chr7:50355788-50355815 | IKZF1 2631 | chr7:50400045-50400072 |
| IKZF 1 1021 | chr7:50355791-50355818 | IKZF1 2632 | chr7:50400046-50400073 |
| IKZF 1 1022 | chr7:50355804-50355831 | IKZF1 2633 | chr7:50400047-50400074 |
| IKZF 1 1023 | chr7:50355808-50355835 | IKZF1 2634 | chr7:50400050-50400077 |
| IKZF 1 1024 | chr7:50355809-50355836 | IKZF1 2635 | chr7:50400051-50400078 |
| IKZF 1 1025 | chr7:50355815-50355842 | IKZF1 2636 | chr7:50400052-50400079 |
| IKZF 1 1026 | chr7:50355826-50355853 | IKZF1 2637 | chr7:50400058-50400085 |
| IKZF 1 1027 | chr7:50355827-50355854 | IKZF1 2638 | chr7:50400061-50400088 |
| IKZF 1 1028 | chr7:50355834-50355861 | IKZF1 2639 | chr7:50400067-50400094 |
| IKZF 1 1029 | chr7:50355837-50355864 | IKZF1 2640 | chr7:50400068-50400095 |
| IKZF 1 1030 | chr7:50355838-50355865 | IKZF1 2641 | chr7:50400069-50400096 |
| IKZF 1 1031 | chr7:50355841-50355868 | IKZF1 2642 | chr7:50400070-50400097 |
| IKZF 1 1032 | chr7:50355843-50355870 | IKZF1 2643 | chr7:50400080-50400107 |
| IKZF 1 1033 | chr7:50355849-50355876 | IKZF1 2644 | chr7:50400090-50400117 |
| IKZF 1 1034 | chr7:50355869-50355896 | IKZF1 2645 | chr7:50400091-50400118 |
| IKZF 1 1035 | chr7:50355872-50355899 | IKZF1 2646 | chr7:50400102-50400129 |
| IKZF 1 1036 | chr7:50355873-50355900 | IKZF1 2647 | chr7:50400103-50400130 |
| IKZF 1 1037 | chr7:50355881-50355908 | IKZF1 2648 | chr7:50400106-50400133 |
| IKZF 1 1038 | chr7:50355885-50355912 | IKZF1 2649 | chr7:50400109-50400136 |
| IKZF 1 1039 | chr7:50355886-50355913 | IKZF1 2650 | chr7:50400110-50400137 |
| IKZF 1 1040 | chr7:50355887-50355914 | IKZF1 2651 | chr7:50400111-50400138 |
| IKZF 1 1041 | chr7:50355890-50355917 | IKZF1 2652 | chr7:50400124-50400151 |
| IKZF 1 1042 | chr7:50355894-50355921 | IKZF1 2653 | chr7:50400133-50400160 |
| IKZF 1 1043 | chr7:50355895-50355922 | IKZF1 2654 | chr7:50400139-50400166 |
| IKZF 1 1044 | chr7:50355901-50355928 | IKZF1 2655 | chr7:50400140-50400167 |
| IKZF 1 1045 | chr7:50355930-50355957 | IKZF1 2656 | chr7:50400141-50400168 |
| IKZF 1 1046 | chr7:50355931-50355958 | IKZF1 2657 | chr7:50400142-50400169 |
| IKZF 1 1047 | chr7:50355932-50355959 | IKZF1 2658 | chr7:50400149-50400176 |
| IKZF 1 1048 | chr7:50355941-50355968 | IKZF1 2659 | chr7:50400151-50400178 |
| IKZF 1 1049 | chr7:50355948-50355975 | IKZF1 2660 | chr7:50400153-50400180 |
| IKZF 1 1050 | chr7:50355961-50355988 | IKZF1 2661 | chr7:50400161-50400188 |
| IKZF 1 1051 | chr7:50355982-50356009 | IKZF1 2662 | chr7:50400162-50400189 |
| IKZF 1 1052 | chr7:50355993-50356020 | IKZF1 2663 | chr7:50400173-50400200 |
| IKZF 1 1053 | chr7:50355994-50356021 | IKZF1 2664 | chr7:50400175-50400202 |
| IKZF 1 1054 | chr7:50355995-50356022 | IKZF1 2665 | chr7:50400183-50400210 |
| IKZF 1 *1055* | chr7:50356011-50356038 | IKZF1 2666 | chr7:50400184-50400211 |
| IKZF 1 1056 | chr7:50356034-50356061 | IKZF1 2667 | chr7:50400193-50400220 |
| IKZF 1 1057 | chr7:50356048-50356075 | IKZF1 2668 | chr7:50400197-50400224 |
| IKZF 1 1058 | chr7:50356049-50356076 | IKZF1 2669 | chr7:50400202-50400229 |
| IKZF 1 1059 | chr7:50356071-50356098 | IKZF1 2670 | chr7:50400203-50400230 |
| IKZF 1 1060 | chr7:50356076-50356103 | IKZF1 2671 | chr7:50400214-50400241 |
| IKZF 1 1061 | chr7:50356078-50356105 | IKZF1 2672 | chr7:50400215-50400242 |
| IKZF 1 1062 | chr7:50356079-50356106 | IKZF1 2673 | chr7:50400233-50400260 |
| IKZF 1 1063 | chr7:50356080-50356107 | IKZF1 2674 | chr7:50400234-50400261 |
| IKZF 1 1064 | chr7:50356081-50356108 | IKZF1 2675 | chr7:50400235-50400262 |
| IKZF 1 1065 | chr7:50356084-50356111 | IKZF1 2676 | chr7:50400249-50400276 |
| IKZF 1 1066 | chr7:50356110-50356137 | IKZF1 2677 | chr7:50400256-50400283 |
| IKZF 1 1067 | chr7:50356111-50356138 | IKZF1 2678 | chr7:50400257-50400284 |
| IKZF 1 1068 | chr7:50356125-50356152 | IKZF1 2679 | chr7:50400266-50400293 |
| IKZF 1 1069 | chr7:50356126-50356153 | IKZF1 2680 | chr7:50400269-50400296 |
| IKZF 1 1070 | chr7:50356127-50356154 | IKZF1 2681 | chr7:50400306-50400333 |
| IKZF 1 1071 | chr7:50356150-50356177 | IKZF1 2682 | chr7:50400311-50400338 |
| IKZF 1 1072 | chr7:50356158-50356185 | IKZF1 2683 | chr7:50400314-50400341 |
| IKZF1 1073 | chr7:50356174-50356201 | IKZF1 2684 | chr7:50400315-50400342 |
| IKZF 1 1074 | chr7:50356175-50356202 | IKZF1 2685 | chr7:50400323-50400350 |
| IKZF 1 1075 | chr7:50356179-50356206 | IKZF1 2686 | chr7:50400324-50400351 |
| IKZF 1 1076 | chr7:50356204-50356231 | IKZF1 2687 | chr7:50400325-50400352 |
| IKZF 1 1077 | chr7:50356215-50356242 | IKZF1 2688 | chr7:50400328-50400355 |
| IKZF 1 1078 | chr7:50356237-50356264 | IKZF1 2689 | chr7:50400331-50400358 |
| IKZF 1 1079 | chr7:50356255-50356282 | IKZF1 2690 | chr7:50400363-50400390 |
| IKZF 1 1080 | chr7:50356264-50356291 | IKZF1 2691 | chr7:50400368-50400395 |
| IKZF 1 1081 | chr7:50356265-50356292 | IKZF1 2692 | chr7:50400375-50400402 |
| IKZF 1 1082 | chr7:50356280-50356307 | IKZF1 2693 | chr7:50400379-50400406 |
| IKZF 1 1083 | chr7:50356281-50356308 | IKZF1 2694 | chr7:50400386-50400413 |
| IKZF 1 1084 | chr7:50356282-50356309 | IKZF1 2695 | chr7:50400389-50400416 |
| IKZF 1 1085 | chr7:50356290-50356317 | IKZF1 2696 | chr7:50400392-50400419 |
| IKZF 1 1086 | chr7:50356295-50356322 | IKZF1 2697 | chr7:50400394-50400421 |
| IKZF 1 1087 | chr7:50356300-50356327 | IKZF1 2698 | chr7:50400407-50400434 |
| IKZF 1 1088 | chr7:50356301-50356328 | IKZF1 2699 | chr7:50400408-50400435 |
| IKZF 1 1089 | chr7:50356310-50356337 | IKZF1 2700 | chr7:50400409-50400436 |
| IKZF 1 1090 | chr7:50356325-50356352 | IKZF1 2701 | chr7:50400414-50400441 |
| IKZF 1 1091 | chr7:50356332-50356359 | IKZF1 2702 | chr7:50400421-50400448 |
| IKZF 1 1092 | chr7:50356352-50356379 | IKZF1 2703 | chr7:50400428-50400455 |
| IKZF 1 1093 | chr7:50356361-50356388 | IKZF1 2704 | chr7:50400444-50400471 |
| IKZF 1 1094 | chr7:50356366-50356393 | IKZF1 2705 | chr7:50400445-50400472 |
| IKZF 1 1095 | chr7:50356384-50356411 | IKZF1 2706 | chr7:50400457-50400484 |
| IKZF 1 1096 | chr7:50356385-50356412 | IKZF1 2707 | chr7:50400468-50400495 |
| IKZF1 1097 | chr7:50356388-50356415 | IKZF1 2708 | chr7:50400480-50400507 |
| IKZF 1 1098 | chr7:50356420-50356447 | IKZF1 2709 | chr7:50400484-50400511 |
| IKZF 1 1099 | chr7:50356437-50356464 | IKZF1 2710 | chr7:50400485-50400512 |
| IKZF 1 1100 | chr7:50356438-50356465 | IKZF1 2711 | chr7:50400494-50400521 |
| IKZF 1 1101 | chr7:50356493-50356520 | IKZF1 2712 | chr7:50400500-50400527 |
| IKZF 1 1102 | chr7:50356508-50356535 | IKZF1 2713 | chr7:50400504-50400531 |
| IKZF 1 1103 | chr7:50356509-50356536 | IKZF1 2714 | chr7:50400516-50400543 |
| IKZF 1 1104 | chr7:50356510-50356537 | IKZF1 2715 | chr7:50400525-50400552 |
| IKZF 1 1105 | chr7:50356529-50356556 | IKZF1 2716 | chr7:50400527-50400554 |
| IKZF 1 1106 | chr7:50356553-50356580 | IKZF1 2717 | chr7:50400532-50400559 |
| IKZF 1 1107 | chr7:50356631-50356658 | IKZF1 2718 | chr7:50400541-50400568 |
| IKZF 1 1108 | chr7:50356645-50356672 | IKZF1 2719 | chr7:50400546-50400573 |
| IKZF 1 1109 | chr7:50356646-50356673 | IKZF1 2720 | chr7:50400558-50400585 |
| IKZF1 1110 | chr7:50356655-50356682 | IKZF1 2721 | chr7:50400564-50400591 |
| IKZF1 1111 | chr7:50356659-50356686 | IKZF1 2722 | chr7:50400570-50400597 |
| IKZF 1 1112 | chr7:50356660-50356687 | IKZF1 2723 | chr7:50400575-50400602 |
| IKZF 1 1113 | chr7:50356662-50356689 | IKZF1 2724 | chr7:50400576-50400603 |
| IKZF 1 1114 | chr7:50356664-50356691 | IKZF1 2725 | chr7:50400577-50400604 |
| IKZF 1 1115 | chr7:50356674-50356701 | IKZF1 2726 | chr7:50400609-50400636 |
| IKZF 1 1116 | chr7:50356677-50356704 | **IKZF1** 2727 | chr7:50400615-50400642 |
| IKZF 1 1117 | chr7:50356708-50356735 | IKZF1 2728 | chr7:50400630-50400657 |
| IKZF 1 1118 | chr7:50356716-50356743 | IKZF1 2729 | chr7:50400631-50400658 |
| IKZF 1 1119 | chr7:50356724-50356751 | IKZF1 2730 | chr7:50400634-50400661 |
| IKZF 1 1120 | chr7:50356733-50356760 | IKZF1 2731 | chr7:50400635-50400662 |
| IKZF 1 1121 | chr7:50356734-50356761 | IKZF1 2732 | chr7:50400640-50400667 |
| IKZF 1 1122 | chr7:50356750-50356777 | IKZF1 2733 | chr7:50400641-50400668 |
| IKZF 1 1123 | chr7:50356753-50356780 | IKZF1 2734 | chr7:50400642-50400669 |
| IKZF 1 1124 | chr7:50356756-50356783 | IKZF1 2735 | chr7:50400643-50400670 |
| IKZF 1 1125 | chr7:50356762-50356789 | IKZF1 2736 | chr7:50400646-50400673 |
| IKZF 1 1126 | chr7:50356776-50356803 | IKZF1 2737 | chr7:50400647-50400674 |
| IKZF 1 1127 | chr7:50356793-50356820 | IKZF1 2738 | chr7:50400648-50400675 |
| IKZF 1 1128 | chr7:50356794-50356821 | IKZF1 2739 | chr7:50400653-50400680 |
| IKZF 1 1129 | chr7:50356815-50356842 | IKZF1 2740 | chr7:50400654-50400681 |
| IKZF 1 1130 | chr7:50356816-50356843 | IKZF1 2741 | chr7:50400655-50400682 |
| IKZF 1 1131 | chr7:50356829-50356856 | IKZF1 2742 | chr7:50400660-50400687 |
| IKZF 1 1132 | chr7:50356837-50356864 | IKZF1 2743 | chr7:50400663-50400690 |
| IKZF 1 1133 | chr7:50356838-50356865 | IKZF1 2744 | chr7:50400664-50400691 |
| IKZF 1 1134 | chr7:50356841-50356868 | IKZF1 2745 | chr7:50400665-50400692 |
| IKZF 1 1135 | chr7:50356842-50356869 | IKZF1 2746 | chr7:50400686-50400713 |
| IKZF 1 1136 | chr7:50356844-50356871 | IKZF1 2747 | chr7:50400689-50400716 |
| IKZF1 1137 | chr7:50356845-50356872 | IKZF1 2748 | chr7:50400692-50400719 |
| IKZF1 1138 | chr7:50356846-50356873 | IKZF1 2749 | chr7:50400697-50400724 |
| IKZF1 1139 | chr7:50356849-50356876 | IKZF1 2750 | chr7:50400699-50400726 |
| IKZF1 1140 | chr7:50356852-50356879 | IKZF1 2751 | chr7:50400700-50400727 |
| IKZF1 1141 | chr7:50356855-50356882 | IKZF1 2752 | chr7:50400703-50400730 |
| IKZF1 1142 | chr7:50356860-50356887 | IKZF1 2753 | chr7:50400718-50400745 |
| IKZF1 1143 | chr7:50356861-50356888 | IKZF1 2754 | chr7:50400726-50400753 |
| IKZF1 1144 | chr7:50356864-50356891 | IKZF1 2755 | chr7:50400753-50400780 |
| IKZF1 1145 | chr7:50356865-50356892 | IKZF1 2756 | chr7:50400761-50400788 |
| IKZF1 1146 | chr7:50356869-50356896 | IKZF1 2757 | chr7:50400762-50400789 |
| IKZF1 1147 | chr7:50356870-50356897 | IKZF1 2758 | chr7:50400763-50400790 |
| IKZF1 1148 | chr7:50356873-50356900 | IKZF1 2759 | chr7:50400800-50400827 |
| IKZF1 1149 | chr7:50356883-50356910 | IKZF1 2760 | chr7:50400804-50400831 |
| IKZF1 1150 | chr7:50356884-50356911 | IKZF1 2761 | chr7:50400805-50400832 |
| IKZF1 1151 | chr7:50356885-50356912 | IKZF1 2762 | chr7:50400814-50400841 |
| IKZF1 1152 | chr7:50356888-50356915 | IKZF1 2763 | chr7:50400815-50400842 |
| IKZF1 1153 | chr7:50356895-50356922 | IKZF1 2764 | chr7:50400848-50400875 |
| IKZF1 1154 | chr7:50356908-50356935 | IKZF1 2765 | chr7:50400860-50400887 |
| IKZF1 1155 | chr7:50356916-50356943 | IKZF1 2766 | chr7:50400861-50400888 |
| IKZF1 1156 | chr7:50356923-50356950 | IKZF1 2767 | chr7:50400864-50400891 |
| IKZF1 1157 | chr7:50356924-50356951 | IKZF1 2768 | chr7:50400875-50400902 |
| IKZF1 1158 | chr7:50356925-50356952 | IKZF1 2769 | chr7:50400876-50400903 |
| IKZF1 1159 | chr7:50356932-50356959 | IKZF1 2770 | chr7:50400877-50400904 |
| IKZF1 1160 | chr7:50356933-50356960 | IKZF1 2771 | chr7:50400882-50400909 |
| IKZF1 1161 | chr7:50356940-50356967 | IKZF1 2772 | chr7:50400897-50400924 |
| IKZF1 1162 | chr7:50356941-50356968 | IKZF1 2773 | chr7:50400898-50400925 |
| IKZF1 1163 | chr7:50356945-50356972 | IKZF1 2774 | chr7:50400903-50400930 |
| IKZF1 1164 | chr7:50356948-50356975 | IKZF1 2775 | chr7:50400913-50400940 |
| IKZF1 1165 | chr7:50356979-50357006 | IKZF1 2776 | chr7:50400921-50400948 |
| IKZF1 1166 | chr7:50356986-50357013 | **IKZF1** 2777 | chr7:50400922-50400949 |
| IKZF1 1167 | chr7:50356996-50357023 | IKZF1 2778 | chr7:50400923-50400950 |
| IKZF1 1168 | chr7:50357010-50357037 | IKZF1 2779 | chr7:50400929-50400956 |
| IKZF1 1169 | chr7:50357011-50357038 | IKZF1 2780 | chr7:50400957-50400984 |
| IKZF1 1170 | chr7:50357016-50357043 | IKZF1 2781 | chr7:50400958-50400985 |
| IKZF1 1171 | chr7:50357017-50357044 | IKZF1 2782 | chr7:50400962-50400989 |
| IKZF1 1172 | chr7:50357018-50357045 | IKZF1 2783 | chr7:50400974-50401001 |
| IKZF1 1173 | chr7:50357020-50357047 | IKZF1 2784 | chr7:50400980-50401007 |
| IKZF1 1174 | chr7:50357021-50357048 | IKZF1 2785 | chr7:50400990-50401017 |
| IKZF1 1175 | chr7:50357026-50357053 | IKZF1 2786 | chr7:50400991-50401018 |
| IKZF1 1176 | chr7:50357027-50357054 | IKZF1 2787 | chr7:50400992-50401019 |
| IKZF1 1177 | chr7:50357028-50357055 | IKZF1 2788 | chr7:50401001-50401028 |
| IKZF1 1178 | chr7:503 5 7034-503 57061 | IKZF1 2789 | chr7:50401002-50401029 |
| IKZF1 1179 | chr7:50357049-50357076 | IKZF1 2790 | chr7:50401005-50401032 |
| IKZF1 1180 | chr7:503 5 7065-503 57092 | IKZF1 2791 | chr7:50401028-50401055 |
| IKZF1 1181 | chr7:50357074-50357101 | IKZF1 2792 | chr7:50401042-50401069 |
| IKZF1 1182 | chr7:50357076-50357103 | IKZF1 2793 | chr7:50401047-50401074 |
| IKZF1 1183 | chr7:50357077-50357104 | IKZF1 2794 | chr7:50401048-50401075 |
| IKZF1 1184 | chr7:50357079-50357106 | IKZF1 2795 | chr7:50401049-50401076 |
| IKZF 1 1185 | chr7:50357086-50357113 | IKZF1 2796 | chr7:50401054-50401081 |
| IKZF 1 1186 | chr7:50357087-50357114 | IKZF1 2797 | chr7:50401055-50401082 |
| IKZF1 1187 | chr7:50357088-50357115 | IKZF1 2798 | chr7:50401063-50401090 |
| IKZF1 1188 | chr7:50357092-50357119 | IKZF1 2799 | chr7:50401068-50401095 |
| IKZF1 1189 | chr7:50357093-50357120 | IKZF1 2800 | chr7:50401069-50401096 |
| IKZF1 1190 | chr7:50357096-50357123 | IKZF1 2801 | chr7:5040 1070-50401 097 |
| IKZF1 1191 | chr7:50357097-50357124 | IKZF1 2802 | chr7:50401075-50401102 |
| IKZF1 1192 | chr7:50357105-50357132 | IKZF1 2803 | chr7:50401080-50401107 |
| IKZF1 1193 | chr7:50357108-50357135 | IKZF1 2804 | chr7:50401112-50401139 |
| IKZF1 1194 | chr7:50357109-50357136 | IKZF1 2805 | chr7:50401113-50401140 |
| IKZF1 1195 | chr7:50357126-50357153 | IKZF1 2806 | chr7:50401114-50401141 |
| IKZF1 1196 | chr7:50357133-50357160 | IKZF1 2807 | chr7:50401115-50401142 |
| IKZF1 1197 | chr7:50357143-50357170 | IKZF1 2808 | chr7:50401118-50401145 |
| IKZF1 1198 | chr7:50357149-50357176 | IKZF1 2809 | chr7:50401119-50401146 |
| IKZF1 1199 | chr7:50357166-50357193 | IKZF1 2810 | chr7:50401126-50401153 |
| IKZF1 1200 | chr7:50357170-50357197 | IKZF1 2811 | chr7:50401133-50401160 |
| IKZF1 1201 | chr7:50357171-50357198 | IKZF1 2812 | chr7:50401142-50401169 |
| IKZF1 1202 | chr7:50357175-50357202 | IKZF1 2813 | chr7:50401149-50401176 |
| IKZF1 1203 | chr7:50357176-50357203 | IKZF1 2814 | chr7:50401155-50401182 |
| IKZF1 1204 | chr7:50357177-50357204 | IKZF1 2815 | chr7:50401156-50401183 |
| IKZF1 *1205* | chr7:50357182-50357209 | IKZF1 2816 | chr7:50401159-50401186 |
| IKZF1 1206 | chr7:50357185-50357212 | IKZF1 2817 | chr7:50401160-50401187 |
| IKZF1 1207 | chr7:50357188-50357215 | IKZF1 2818 | chr7:50401161-50401188 |
| IKZF1 1208 | chr7:50357189-50357216 | IKZF1 2819 | chr7:50401163-50401190 |
| IKZF1 1209 | chr7:50357196-50357223 | IKZF1 2820 | chr7:50401168-50401195 |
| IKZF1 1210 | chr7:50357199-50357226 | IKZF1 2821 | chr7:50401215-50401242 |
| IKZF1 1211 | chr7:50357200-50357227 | IKZF1 2822 | chr7:50401227-50401254 |
| IKZF 1 1212 | chr7:50357201-50357228 | IKZF1 2823 | chr7:50401231-50401258 |
| IKZF 1 1213 | chr7:50357202-50357229 | IKZF1 2824 | chr7:50401232-50401259 |
| IKZF 1 1214 | chr7:50357203-50357230 | IKZF1 2825 | chr7:50401233-50401260 |
| IKZF 1 1215 | chr7:50357206-50357233 | IKZF1 2826 | chr7:50401260-50401287 |
| IKZF 1 1216 | chr7:50357214-50357241 | IKZF1 2827 | chr7:50401261-50401288 |
| IKZF 1 1217 | chr7:50357215-50357242 | IKZF1 2828 | chr7:50401264-50401291 |
| IKZF 1 1218 | chr7:50357216-50357243 | IKZF1 2829 | chr7:50401267-50401294 |
| IKZF 1 1219 | chr7:50357217-50357244 | IKZF1 2830 | chr7:50401268-50401295 |
| IKZF 1 1220 | chr7:50357226-50357253 | IKZF1 2831 | chr7:50401277-50401304 |
| IKZF 1 1221 | chr7:50357227-50357254 | IKZF1 2832 | chr7:50401278-50401305 |
| IKZF 1 1222 | chr7:50357228-50357255 | IKZF1 2833 | chr7:50401282-50401309 |
| IKZF 1 1223 | chr7:50357230-50357257 | IKZF1 2834 | chr7:50401283-50401310 |
| IKZF 1 1224 | chr7:50357242-50357269 | IKZF1 2835 | chr7:50401291-50401318 |
| IKZF 1 1225 | chr7:50357249-50357276 | IKZF1 2836 | chr7:50401292-50401319 |
| IKZF 1 1226 | chr7:50357256-50357283 | IKZF1 2837 | chr7:50401301-50401328 |
| IKZF 1 1227 | chr7:50357263-50357290 | IKZF1 2838 | chr7:50401302-50401329 |
| IKZF 1 1228 | chr7:50357271-50357298 | IKZF1 2839 | chr7:50401311-50401338 |
| IKZF 1 1229 | chr7:50357276-50357303 | IKZF1 2840 | chr7:50401312-50401339 |
| IKZF 1 1230 | chr7:50357277-50357304 | IKZF1 2841 | chr7:50401319-50401346 |
| IKZF 1 1231 | chr7:50357286-50357313 | IKZF1 2842 | chr7:50401322-50401349 |
| IKZF 1 1232 | chr7:50357291-50357318 | IKZF1 2843 | chr7:50401325-50401352 |
| IKZF 1 1233 | chr7:50357292-50357319 | IKZF1 2844 | chr7:50401334-50401361 |
| IKZF 1 1234 | chr7:50357299-50357326 | IKZF1 2845 | chr7:50401350-50401377 |
| IKZF 1 1235 | chr7:50357314-50357341 | IKZF1 2846 | chr7:50401362-50401389 |
| IKZF 1 1236 | chr7:50357315-50357342 | IKZF1 2847 | chr7:50401366-50401393 |
| IKZF 1 1237 | chr7:50357316-50357343 | IKZF1 2848 | chr7:50401368-50401395 |
| IKZF 1 1238 | chr7:50357331-50357358 | IKZF1 2849 | chr7:50401369-50401396 |
| IKZF 1 1239 | chr7:50357335-50357362 | IKZF1 2850 | chr7:50401371-50401398 |
| IKZF 1 1240 | chr7:50357338-50357365 | IKZF1 2851 | chr7:50401372-50401399 |
| IKZF 1 1241 | chr7:50357341-50357368 | IKZF1 2852 | chr7:50401385-50401412 |
| IKZF 1 1242 | chr7:50357342-50357369 | IKZF1 2853 | chr7:50401386-50401413 |
| IKZF 1 1243 | chr7:50357343-50357370 | IKZF1 2854 | chr7:50401387-50401414 |
| IKZF 1 1244 | chr7:50357346-50357373 | IKZF1 2855 | chr7:50401396-50401423 |
| IKZF 1 1245 | chr7:50357349-50357376 | IKZF1 2856 | chr7:50401397-50401424 |
| IKZF 1 1246 | chr7:50357350-50357377 | IKZF1 2857 | chr7:50401398-50401425 |
| IKZF 1 1247 | chr7:50357351-50357378 | IKZF1 2858 | chr7:50401407-50401434 |
| IKZF 1 1248 | chr7:50357352-50357379 | IKZF1 2859 | chr7:50401408-50401435 |
| IKZF 1 1249 | chr7:50357353-50357380 | IKZF1 2860 | chr7:50401428-50401455 |
| IKZF 1 1250 | chr7:50357354-50357381 | IKZF1 2861 | chr7:50401429-50401456 |
| IKZF 1 1251 | chr7:50357357-50357384 | IKZF1 2862 | chr7:50401435-50401462 |
| IKZF 1 1252 | chr7:50357360-50357387 | IKZF1 2863 | chr7:50401436-50401463 |
| IKZF 1 1253 | chr7:50357366-50357393 | IKZF1 2864 | chr7:50401437-50401464 |
| IKZF 1 1254 | chr7:50357376-50357403 | IKZF1 2865 | chr7:50401438-50401465 |
| IKZF 1 *1255* | chr7:50357377-50357404 | IKZF1 2866 | chr7:50401450-50401477 |
| IKZF 1 1256 | chr7:50357387-50357414 | IKZF1 2867 | chr7:50401451-50401478 |
| IKZF 1 1257 | chr7:50357388-50357415 | IKZF1 2868 | chr7:50401456-50401483 |
| IKZF 1 1258 | chr7:50357389-50357416 | IKZF1 2869 | chr7:50401466-50401493 |
| IKZF 1 1259 | chr7:50357390-50357417 | IKZF1 2870 | chr7:50401467-50401494 |
| IKZF 1 1260 | chr7:50357391-50357418 | IKZF1 2871 | chr7:50401468-50401495 |
| IKZF 1 1261 | chr7:50357394-50357421 | IKZF1 2872 | chr7:50401469-50401496 |
| IKZF 1 1262 | chr7:50357397-50357424 | IKZF1 2873 | chr7:50401485-50401512 |
| IKZF 1 1263 | chr7:50357412-50357439 | IKZF1 2874 | chr7:50401496-50401523 |
| IKZF 1 1264 | chr7:50357413-50357440 | IKZF1 2875 | chr7:50401501-50401528 |
| IKZF 1 1265 | chr7:50357419-50357446 | IKZF1 2876 | chr7:50401502-50401529 |
| IKZF 1 1266 | chr7:50357442-50357469 | IKZF1 2877 | chr7:50401515-50401542 |
| IKZF 1 1267 | chr7:50357443-50357470 | IKZF1 2878 | chr7:50401550-50401577 |
| IKZF 1 1268 | chr7:50357463-50357490 | IKZF1 2879 | chr7:50401559-50401586 |
| IKZF 1 1269 | chr7:50357464-50357491 | IKZF1 2880 | chr7:50401565-50401592 |
| IKZF 1 1270 | chr7:50357465-50357492 | IKZF1 2881 | chr7:50401569-50401596 |
| IKZF 1 1271 | chr7:50357466-50357493 | IKZF1 2882 | chr7:50401583-50401610 |
| IKZF 1 1272 | chr7:50357467-50357494 | IKZF1 2883 | chr7:50401587-50401614 |
| IKZF 1 1273 | chr7:50357468-50357495 | IKZF1 2884 | chr7:50401588-50401615 |
| IKZF 1 1274 | chr7:50357471-50357498 | IKZF1 2885 | chr7:50401599-50401626 |
| IKZF 1 1275 | chr7:50357474-50357501 | IKZF1 2886 | chr7:50401609-50401636 |
| IKZF 1 1276 | chr7:50357475-50357502 | IKZF1 2887 | chr7:50401616-50401643 |
| IKZF 1 1277 | chr7:50357476-50357503 | IKZF1 2888 | chr7:50401624-50401651 |
| IKZF 1 1278 | chr7:50357479-50357506 | IKZF1 2889 | chr7:50401635-50401662 |
| IKZF 1 1279 | chr7:50357480-50357507 | IKZF1 2890 | chr7:50401640-50401667 |
| IKZF 1 1280 | chr7:50357483-50357510 | IKZF1 2891 | chr7:50401645-50401672 |
| IKZF 1 1281 | chr7:50357505-50357532 | IKZF1 2892 | chr7:50401646-50401673 |
| IKZF 1 1282 | chr7:50357510-50357537 | IKZF1 2893 | chr7:50401676-50401703 |
| IKZF 1 1283 | chr7:50357511-50357538 | IKZF1 2894 | chr7:50401677-50401704 |
| IKZF 1 1284 | chr7:50357525-50357552 | IKZF1 2895 | chr7:50401684-50401711 |
| IKZF 1 1285 | chr7:50357526-50357553 | IKZF1 2896 | chr7:50401716-50401743 |
| IKZF 1 1286 | chr7:50357527-50357554 | IKZF1 2897 | chr7:50401720-50401747 |
| IKZF 1 1287 | chr7:50357532-50357559 | IKZF1 2898 | chr7:50401721-50401748 |
| IKZF 1 1288 | chr7:50357533-50357560 | IKZF1 2899 | chr7:50401732-50401759 |
| IKZF 1 1289 | chr7:50357541-50357568 | IKZF1 2900 | chr7:50401750-50401777 |
| IKZF 1 1290 | chr7:50357549-50357576 | IKZF1 2901 | chr7:50401751-50401778 |
| IKZF 1 1291 | chr7:50357577-50357604 | IKZF1 2902 | chr7:50401808-50401835 |
| IKZF 1 1292 | chr7:50357580-50357607 | IKZF1 2903 | chr7:50401809-50401836 |
| IKZF 1 1293 | chr7:50357582-50357609 | IKZF1 2904 | chr7:50401841-50401868 |
| IKZF 1 1294 | chr7:50357593-50357620 | IKZF1 2905 | chr7:50401852-50401879 |
| IKZF 1 1295 | chr7:50357604-50357631 | IKZF1 2906 | chr7:50401863-50401890 |
| IKZF 1 1296 | chr7:50357605-50357632 | IKZF1 2907 | chr7:50401895-50401922 |
| IKZF 1 1297 | chr7:50357617-50357644 | IKZF1 2908 | chr7:50401899-50401926 |
| IKZF 1 1298 | chr7:50357626-50357653 | IKZF1 2909 | chr7:50401903-50401930 |
| IKZF 1 1299 | chr7:50357627-50357654 | IKZF1 2910 | chr7:50401908-50401935 |
| IKZF 1 1300 | chr7:50357629-50357656 | IKZF1 2911 | chr7:50401918-50401945 |
| IKZF 1 1301 | chr7:50357630-50357657 | IKZF1 2912 | chr7:50401925-50401952 |
| IKZF 1 1302 | chr7:50357631-50357658 | IKZF1 2913 | chr7:50401935-50401962 |
| IKZF 1 1303 | chr7:50357650-50357677 | IKZF1 2914 | chr7:50401936-50401963 |
| IKZF 1 1304 | chr7:50357651-50357678 | IKZF1 2915 | chr7:50401938-50401965 |
| IKZF 1 1305 | chr7:50357664-50357691 | IKZF1 2916 | chr7:50401945-50401972 |
| IKZF 1 1306 | chr7:50357670-50357697 | IKZF1 2917 | chr7:50401946-50401973 |
| IKZF 1 1307 | chr7:50357682-50357709 | IKZF1 2918 | chr7:50401964-50401991 |
| IKZF 1 1308 | chr7:50357683-50357710 | IKZF1 2919 | chr7:50401980-50402007 |
| IKZF 1 1309 | chr7:50357692-50357719 | IKZF1 2920 | chr7:50401985-50402012 |
| IKZF 1 1310 | chr7:50357693-50357720 | IKZF1 2921 | chr7:50401990-50402017 |
| IKZF 1 1311 | chr7:50357699-50357726 | IKZF1 2922 | chr7:50401997-50402024 |
| IKZF 1 1312 | chr7:50357702-50357729 | IKZF1 2923 | chr7:50402000-50402027 |
| IKZF 1 1313 | chr7:50357703-50357730 | IKZF1 2924 | chr7:50402001-50402028 |
| IKZF 1 1314 | chr7:50357704-50357731 | IKZF1 2925 | chr7:50402004-50402031 |
| IKZF 1 1315 | chr7:50357705-50357732 | IKZF1 2926 | chr7:50402007-50402034 |
| IKZF 1 1316 | chr7:50357733-50357760 | IKZF1 2927 | chr7:50402010-50402037 |
| IKZF 1 1317 | chr7:50357735-50357762 | IKZF1 2928 | chr7:50402011-50402038 |
| IKZF 1 1318 | chr7:50357749-50357776 | IKZF1 2929 | chr7:50402012-50402039 |
| IKZF 1 1319 | chr7:50357764-50357791 | IKZF1 2930 | chr7:50402016-50402043 |
| IKZF 1 1320 | chr7:50357772-50357799 | IKZF1 2931 | chr7:50402048-50402075 |
| IKZF 1 1321 | chr7:50357777-50357804 | IKZF1 2932 | chr7:50402050-50402077 |
| IKZF 1 1322 | chr7:50357778-50357805 | IKZF1 2933 | chr7:50402051-50402078 |
| IKZF 1 1323 | chr7:50357781-50357808 | IKZF1 2934 | chr7:50402052-50402079 |
| IKZF 1 1324 | chr7:50357782-50357809 | IKZF1 2935 | chr7:50402055-50402082 |
| IKZF 1 1325 | chr7:50357785-50357812 | IKZF1 2936 | chr7:50402056-50402083 |
| IKZF 1 1326 | chr7:50357803-50357830 | IKZF1 2937 | chr7:50402060-50402087 |
| IKZF 1 1327 | chr7:50357804-50357831 | IKZF1 2938 | chr7:50402066-50402093 |
| IKZF 1 1328 | chr7:50357810-50357837 | IKZF1 2939 | chr7:50402070-50402097 |
| IKZF 1 1329 | chr7:50357826-50357853 | IKZF1 2940 | chr7:50402077-50402104 |
| IKZF 1 1330 | chr7:50357842-50357869 | IKZF1 2941 | chr7:50402087-50402114 |
| IKZF 1 1331 | chr7:50357854-50357881 | IKZF1 2942 | chr7:50402088-50402115 |
| IKZF 1 1332 | chr7:50357857-50357884 | IKZF1 2943 | chr7:50402101-50402128 |
| IKZF 1 1333 | chr7:50357858-50357885 | IKZF1 2944 | chr7:50402102-50402129 |
| IKZF 1 1334 | chr7:50357865-50357892 | IKZF1 2945 | chr7:50402115-50402142 |
| IKZF 1 1335 | chr7:50357869-50357896 | IKZF1 2946 | chr7:50402116-50402143 |
| IKZF 1 1336 | chr7:50357873-50357900 | IKZF1 2947 | chr7:50402133-50402160 |
| IKZF 1 1337 | chr7:50357874-50357901 | IKZF1 2948 | chr7:50402136-50402163 |
| IKZF 1 1338 | chr7:50357881-50357908 | IKZF1 2949 | chr7:50402158-50402185 |
| IKZF 1 1339 | chr7:50357885-50357912 | IKZF1 2950 | chr7:50402177-50402204 |
| IKZF 1 1340 | chr7:50357891-50357918 | IKZF1 2951 | chr7:50402180-50402207 |
| IKZF 1 1341 | chr7:50357905-50357932 | IKZF1 2952 | chr7:50402200-50402227 |
| IKZF 1 1342 | chr7:50357914-50357941 | IKZF1 2953 | chr7:50402220-50402247 |
| IKZF 1 1343 | chr7:50357915-50357942 | IKZF1 2954 | chr7:50402231-50402258 |
| IKZF 1 1344 | chr7:50357916-50357943 | IKZF1 2955 | chr7:50402241-50402268 |
| IKZF 1 1345 | chr7:50357917-50357944 | IKZF1 2956 | chr7:50402242-50402269 |
| IKZF 1 1346 | chr7:50357935-50357962 | IKZF1 2957 | chr7:50402280-50402307 |
| IKZF 1 1347 | chr7:50357936-50357963 | IKZF1 2958 | chr7:50402281-50402308 |
| IKZF 1 1348 | chr7:50357947-50357974 | IKZF1 2959 | chr7:50402282-50402309 |
| IKZF 1 1349 | chr7:50357948-50357975 | IKZF1 2960 | chr7:50402285-50402312 |
| IKZF 1 1350 | chr7:50357969-50357996 | IKZF1 2961 | chr7:50402299-50402326 |
| IKZF 1 1351 | chr7:50357973-50358000 | IKZF1 2962 | chr7:50402311-50402338 |
| IKZF 1 1352 | chr7:50357997-50358024 | IKZF1 2963 | chr7:50402312-50402339 |
| IKZF 1 1353 | chr7:50358011-50358038 | IKZF1 2964 | chr7:50402332-50402359 |
| IKZF 1 1354 | chr7:50358014-50358041 | IKZF1 2965 | chr7:50402335-50402362 |
| IKZF 1 1355 | chr7:50358020-50358047 | IKZF1 2966 | chr7:50402336-50402363 |
| IKZF 1 1356 | chr7:50358021-50358048 | IKZF1 2967 | chr7:50402357-50402384 |
| IKZF 1 1357 | chr7:50358032-50358059 | IKZF1 2968 | chr7:50402370-50402397 |
| IKZF 1 1358 | chr7:50358033-50358060 | IKZF1 2969 | chr7:50402377-50402404 |
| IKZF 1 1359 | chr7:50358034-50358061 | IKZF1 2970 | chr7:50402380-50402407 |
| IKZF 1 1360 | chr7:50358040-50358067 | IKZF1 2971 | chr7:50402399-50402426 |
| IKZF 1 1361 | chr7:50358063-50358090 | IKZF1 2972 | chr7:50402400-50402427 |
| IKZF 1 1362 | chr7:50358093-50358120 | IKZF1 2973 | chr7:50402411-50402438 |
| IKZF 1 1363 | chr7:50358094-50358121 | IKZF1 2974 | chr7:50402412-50402439 |
| IKZF1 1364 | chr7:50358134-50358161 | IKZF1 2975 | chr7:50402422-50402449 |
| IKZF 1 1365 | chr7:50358135-50358162 | IKZF1 2976 | chr7:50402431-50402458 |
| IKZF 1 1366 | chr7:50358137-50358164 | IKZF1 2977 | chr7:50402432-50402459 |
| IKZF 1 1367 | chr7:50358144-50358171 | IKZF1 2978 | chr7:50402433-50402460 |
| IKZF 1 1368 | chr7:50358150-50358177 | IKZF1 2979 | chr7:50402445-50402472 |
| IKZF 1 1369 | chr7:50358160-50358187 | IKZF1 2980 | chr7:50402446-50402473 |
| IKZF 1 1370 | chr7:50358172-50358199 | IKZF1 2981 | chr7:50402455-50402482 |
| IKZF 1 1371 | chr7:50358173-50358200 | IKZF1 2982 | chr7:50402456-50402483 |
| IKZF 1 1372 | chr7:50358182-50358209 | IKZF1 2983 | chr7:50402465-50402492 |
| IKZF 1 1373 | chr7:50358183-50358210 | IKZF1 2984 | chr7:50402471-50402498 |
| IKZF 1 1374 | chr7:50358186-50358213 | IKZF1 2985 | chr7:50402472-50402499 |
| IKZF 1 1375 | chr7:50358216-50358243 | IKZF1 2986 | chr7:50402473-50402500 |
| IKZF 1 1376 | chr7:50358217-50358244 | IKZF1 2987 | chr7:50402474-50402501 |
| IKZF 1 1377 | chr7:50358218-50358245 | IKZF1 2988 | chr7:50402475-50402502 |
| IKZF 1 1378 | chr7:50358219-50358246 | IKZF1 2989 | chr7:50402494-50402521 |
| IKZF 1 1379 | chr7:50358220-50358247 | IKZF1 2990 | chr7:50402506-50402533 |
| IKZF 1 1380 | chr7:50358225-50358252 | IKZF1 2991 | chr7:50402513-50402540 |
| IKZF 1 1381 | chr7:50358230-50358257 | IKZF1 2992 | chr7:50402514-50402541 |
| IKZF 1 1382 | chr7:50358277-50358304 | IKZF1 2993 | chr7:50402518-50402545 |
| IKZF 1 1383 | chr7:50358278-50358305 | IKZF1 2994 | chr7:50402519-50402546 |
| IKZF 1 1384 | chr7:50358319-50358346 | IKZF1 2995 | chr7:50402530-50402557 |
| IKZF 1 1385 | chr7:50358331-50358358 | IKZF1 2996 | chr7:50402550-50402577 |
| IKZF 1 1386 | chr7:50358338-50358365 | IKZF1 2997 | chr7:50402551-50402578 |
| IKZF 1 1387 | chr7:50358348-50358375 | IKZF1 2998 | chr7:50402554-50402581 |
| IKZF 1 1388 | chr7:50358361-50358388 | IKZF1 2999 | chr7:50402576-50402603 |
| IKZF 1 1389 | chr7:50358362-50358389 | IKZF1 3000 | chr7:50402590-50402617 |
| IKZF 1 1390 | chr7:50358363-50358390 | IKZF1 3001 | chr7:50402591-50402618 |
| IKZF 1 1391 | chr7:50358369-50358396 | IKZF1 3002 | chr7:50402599-50402626 |
| IKZF 1 1392 | chr7:50358380-50358407 | IKZF1 3003 | chr7:50402600-50402627 |
| IKZF 1 1393 | chr7:50358386-50358413 | IKZF1 3004 | chr7:50402610-50402637 |
| IKZF 1 1394 | chr7:50358387-50358414 | IKZF1 3005 | chr7:50402649-50402676 |
| IKZF 1 1395 | chr7:50358391-50358418 | IKZF1 3006 | chr7:50402668-50402695 |
| IKZF 1 1396 | chr7:50358392-50358419 | IKZF1 3007 | chr7:50402669-50402696 |
| IKZF 1 1397 | chr7:50358393-50358420 | IKZF1 3008 | chr7:50402670-50402697 |
| IKZF 1 1398 | chr7:50358397-50358424 | IKZF1 3009 | chr7:50402693-50402720 |
| IKZF 1 1399 | chr7:50358402-50358429 | IKZF1 3010 | chr7:50402699-50402726 |
| IKZF 1 1400 | chr7:50358403-50358430 | IKZF1 3011 | chr7:50402730-50402757 |
| IKZF 1 1401 | chr7:50358404-50358431 | IKZF1 3012 | chr7:50402741-50402768 |
| IKZF 1 1402 | chr7:50358435-50358462 | IKZF1 3013 | chr7:50402750-50402777 |
| IKZF 1 1403 | chr7:50358438-50358465 | IKZF1 3014 | chr7:50402751-50402778 |
| IKZF 1 1404 | chr7:50358439-50358466 | IKZF1 3015 | chr7:50402756-50402783 |
| IKZF 1 1405 | chr7:50358440-50358467 | IKZF1 3016 | chr7:50402763-50402790 |
| IKZF 1 1406 | chr7:50358444-50358471 | IKZF1 3017 | chr7:50402769-50402796 |
| IKZF 1 1407 | chr7:50358446-50358473 | IKZF1 3018 | chr7:50402770-50402797 |
| IKZF 1 1408 | chr7:50358452-50358479 | IKZF1 3019 | chr7:50402808-50402835 |
| IKZF 1 1409 | chr7:50358469-50358496 | IKZF1 3020 | chr7:50402817-50402844 |
| IKZF 1 1410 | chr7:50358476-50358503 | IKZF1 3021 | chr7:50402822-50402849 |
| IKZF 1 1411 | chr7:50358478-50358505 | IKZF1 3022 | chr7:50402829-50402856 |
| IKZF 1 1412 | chr7:50358488-50358515 | IKZF1 3023 | chr7:50402830-50402857 |
| IKZF 1 1413 | chr7:50358492-50358519 | IKZF1 3024 | chr7:50402872-50402899 |
| IKZF 1 1414 | chr7:50358494-50358521 | IKZF1 3025 | chr7:50402901-50402928 |
| IKZF 1 1415 | chr7:50358504-50358531 | IKZF1 3026 | chr7:50402903-50402930 |
| IKZF 1 1416 | chr7:50358518-50358545 | IKZF1 3027 | chr7:50402909-50402936 |
| IKZF 1 1417 | chr7:50358527-50358554 | IKZF1 3028 | chr7:50402935-50402962 |
| IKZF 1 1418 | chr7:50358532-50358559 | IKZF1 3029 | chr7:50402966-50402993 |
| IKZF 1 1419 | chr7:50358549-50358576 | IKZF1 3030 | chr7:50402979-50403006 |
| IKZF 1 1420 | chr7:50358554-50358581 | IKZF1 3031 | chr7:50402982-50403009 |
| IKZF 1 1421 | chr7:50358566-50358593 | IKZF1 3032 | chr7:50402986-50403013 |
| IKZF 1 1422 | chr7:50358574-50358601 | IKZF1 3033 | chr7:50402987-50403014 |
| IKZF 1 1423 | chr7:50358580-50358607 | IKZF1 3034 | chr7:50402990-50403017 |
| IKZF 1 1424 | chr7:50358585-50358612 | IKZF1 3035 | chr7:50402991-50403018 |
| IKZF 1 1425 | chr7:50358601-50358628 | IKZF1 3036 | chr7:50402992-50403019 |
| IKZF 1 1426 | chr7:50358604-50358631 | IKZF1 3037 | chr7:50402993-50403020 |
| IKZF 1 1427 | chr7:50358608-50358635 | IKZF1 3038 | chr7:50403005-50403032 |
| IKZF 1 1428 | chr7:50358611-50358638 | IKZF1 3039 | chr7:50403050-50403077 |
| IKZF 1 1429 | chr7:50358615-50358642 | IKZF1 3040 | chr7:50403052-50403079 |
| IKZF 1 1430 | chr7:50358616-50358643 | IKZF1 3041 | chr7:50403100-50403127 |
| IKZF 1 1431 | chr7:50358621-50358648 | IKZF1 3042 | chr7:50403101-50403128 |
| IKZF 1 1432 | chr7:50358651-50358678 | IKZF1 3043 | chr7:50403106-50403133 |
| IKZF 1 1433 | chr7:50358652-50358679 | IKZF1 3044 | chr7:50403116-50403143 |
| IKZF 1 1434 | chr7:50358664-50358691 | IKZF1 3045 | chr7:50403126-50403153 |
| IKZF 1 1435 | chr7:50358665-50358692 | IKZF1 3046 | chr7:50403135-50403162 |
| IKZF 1 1436 | chr7:50358673-50358700 | IKZF1 3047 | chr7:50403150-50403177 |
| IKZF 1 1437 | chr7:50358705-50358732 | IKZF1 3048 | chr7:50403178-50403205 |
| IKZF 1 1438 | chr7:50358706-50358733 | IKZF1 3049 | chr7:50403186-50403213 |
| IKZF 1 1439 | chr7:50358707-50358734 | IKZF1 3050 | chr7:50403228-50403255 |
| IKZF 1 1440 | chr7:50358712-50358739 | IKZF1 3051 | chr7:50403231-50403258 |
| IKZF 1 1441 | chr7:50358716-50358743 | IKZF1 3052 | chr7:50403259-50403286 |
| IKZF 1 1442 | chr7:50358736-50358763 | IKZF1 3053 | chr7:50403271-50403298 |
| IKZF 1 1443 | chr7:50358741-50358768 | IKZF1 3054 | chr7:50403275-50403302 |
| IKZF1 1444 | chr7:50358744-50358771 | IKZF1 3055 | chr7:50403284-50403311 |
| IKZF 1 1445 | chr7:50358747-50358774 | IKZF1 3056 | chr7:50403336-50403363 |
| IKZF1 1446 | chr7:50358758-50358785 | IKZF1 3057 | chr7:50403354-50403381 |
| IKZF 1 1447 | chr7:50358759-50358786 | IKZF1 3058 | chr7:50403355-50403382 |
| IKZF 1 1448 | chr7:50358810-50358837 | IKZF1 3059 | chr7:50403385-50403412 |
| IKZF 1 1449 | chr7:50358827-50358854 | IKZF1 3060 | chr7:50403405-50403432 |
| IKZF 1 1450 | chr7:50358843-50358870 | IKZF1 3061 | chr7:50403413-50403440 |
| IKZF 1 1451 | chr7:50358849-50358876 | IKZF1 3062 | chr7:50403420-50403447 |
| IKZF 1 1452 | chr7:50358865-50358892 | IKZF1 3063 | chr7:50403427-50403454 |
| IKZF1 1453 | chr7:50358866-50358893 | IKZF1 3064 | chr7:50403430-50403457 |
| IKZF 1 1454 | chr7:50358875-50358902 | IKZF1 3065 | chr7:50403431-50403458 |
| IKZF 1 1455 | chr7:50358913-50358940 | IKZF1 3066 | chr7:50403438-50403465 |
| IKZF 1 1456 | chr7:50358921-50358948 | IKZF1 3067 | chr7:50403442-50403469 |
| IKZF1 1457 | chr7:50358925-50358952 | IKZF1 3068 | chr7:50403451-50403478 |
| IKZF1 1458 | chr7:50358943-50358970 | IKZF1 3069 | chr7:50403470-50403497 |
| IKZF1 1459 | chr7:50358952-50358979 | IKZF1 3070 | chr7:50403506-50403533 |
| IKZF 1 1460 | chr7:50358962-50358989 | IKZF1 3071 | chr7:50403509-50403536 |
| IKZF 1 1461 | chr7:50358980-50359007 | IKZF1 3072 | chr7:50403510-50403537 |
| IKZF 1 1462 | chr7:503 59002-503 59029 | IKZF1 3073 | chr7:50403511-50403538 |
| IKZF 1 1463 | chr7:50359005-50359032 | IKZF1 3074 | chr7:50403512-50403539 |
| IKZF 1 1464 | chr7:503 59007-503 59034 | IKZF1 3075 | chr7:50403515-50403542 |
| IKZF 1 1465 | chr7:50359008-50359035 | IKZF1 3076 | chr7:50403516-50403543 |
| IKZF 1 1466 | chr7:50359009-50359036 | IKZF1 3077 | chr7 :50403 523-50403 550 |
| IKZF 1 1467 | chr7:50359025-50359052 | IKZF1 3078 | chr7:50403524-50403551 |
| IKZF1 1468 | chr7:50359044-50359071 | IKZF1 3079 | chr7:50403525-50403552 |
| IKZF 1 1469 | chr7:50359052-50359079 | IKZF1 3080 | chr7:50403532-50403559 |
| IKZF 1 1470 | chr7:50359062-50359089 | IKZF1 3081 | chr7:50403533-50403560 |
| IKZF 1 1471 | chr7:50359066-50359093 | IKZF1 3082 | chr7:50403548-50403575 |
| IKZF 1 1472 | chr7:50359085-50359112 | IKZF1 3083 | chr7:50403562-50403589 |
| IKZF 1 1473 | chr7:50359086-50359113 | IKZF1 3084 | chr7:50403567-50403594 |
| IKZF 1 1474 | chr7:50359092-50359119 | IKZF1 3085 | chr7:50403568-50403595 |
| IKZF 1 1475 | chr7:50359093-50359120 | IKZF1 3086 | chr7:50403569-50403596 |
| IKZF 1 1476 | chr7:50359098-50359125 | IKZF1 3087 | chr7:50403575-50403602 |
| IKZF 1 1477 | chr7:50359101-50359128 | IKZF1 3088 | chr7:50403576-50403603 |
| IKZF 1 1478 | chr7:50359121-50359148 | IKZF1 3089 | chr7:50403592-50403619 |
| IKZF 1 1479 | chr7:50359128-50359155 | IKZF1 3090 | chr7:50403610-50403637 |
| IKZF 1 1480 | chr7:50359129-50359156 | IKZF1 3091 | chr7:50403636-50403663 |
| IKZF 1 1481 | chr7:50359132-50359159 | IKZF1 3092 | chr7:50403637-50403664 |
| IKZF 1 1482 | chr7:50359135-50359162 | IKZF1 3093 | chr7:50403671-50403698 |
| IKZF 1 1483 | chr7:50359142-50359169 | IKZF1 3094 | chr7:50403678-50403705 |
| IKZF 1 1484 | chr7:50359144-50359171 | IKZF1 3095 | chr7:50403694-50403721 |
| IKZF1 1485 | chr7:50359145-50359172 | IKZF1 3096 | chr7:50403704-50403731 |
| IKZF 1 1486 | chr7:50359164-50359191 | IKZF1 3097 | chr7:50403710-50403737 |
| IKZF 1 1487 | chr7:50359183-50359210 | IKZF1 3098 | chr7:50403725-50403752 |
| IKZF 1 1488 | chr7:50359184-50359211 | IKZF1 3099 | chr7:50403750-50403777 |
| IKZF 1 1489 | chr7:50359210-50359237 | IKZF1 3100 | chr7:50403763-50403790 |
| IKZF 1 1490 | chr7:50359211-50359238 | IKZF1 3101 | chr7:50403766-50403793 |
| IKZF 1 1491 | chr7:50359219-50359246 | IKZF1 3102 | chr7:50403793-50403820 |
| IKZF 1 1492 | chr7:50359233-50359260 | IKZF1 3103 | chr7:50403794-50403821 |
| IKZF 1 1493 | chr7:50359239-50359266 | IKZF1 3104 | chr7:50403795-50403822 |
| IKZF 1 1494 | chr7:50359253-50359280 | IKZF1 3105 | chr7:50403798-50403825 |
| IKZF 1 1495 | chr7:50359301-50359328 | IKZF1 3106 | chr7:50403802-50403829 |
| IKZF 1 1496 | chr7:50359306-50359333 | IKZF1 3107 | chr7:50403863-50403890 |
| IKZF 1 1497 | chr7:50359324-50359351 | IKZF1 3108 | chr7:50403870-50403897 |
| IKZF 1 1498 | chr7:50359327-50359354 | IKZF1 3109 | chr7:50403873-50403900 |
| IKZF 1 1499 | chr7:50359370-50359397 | IKZF1 3110 | chr7:50403921-50403948 |
| IKZF 1 1500 | chr7:50359394-50359421 | IKZF1 3111 | chr7:50403930-50403957 |
| IKZF 1 1501 | chr7:50359405-50359432 | IKZF1 3112 | chr7:50403934-50403961 |
| IKZF 1 1502 | chr7:50359406-50359433 | IKZF1 3113 | chr7:50403936-50403963 |
| IKZF 1 1503 | chr7:50359409-50359436 | IKZF1 3114 | chr7:50403947-50403974 |
| IKZF 1 1504 | chr7:50359412-50359439 | IKZF1 3115 | chr7:50404026-50404053 |
| IKZF 1 1505 | chr7:50359413-50359440 | IKZF1 3116 | chr7:50404031-50404058 |
| IKZF 1 1506 | chr7:50359414-50359441 | IKZF1 3117 | chr7:50404063-50404090 |
| IKZF 1 1507 | chr7:50359415-50359442 | IKZF1 3118 | chr7:50404071-50404098 |
| IKZF 1 1508 | chr7:50359420-50359447 | IKZF1 3119 | chr7:50404121-50404148 |
| IKZF 1 1509 | chr7:50359421-50359448 | IKZF1 3120 | chr7:50404147-50404174 |
| IKZF 1 1510 | chr7:50359433-50359460 | IKZF1 3121 | chr7:50404160-50404187 |
| IKZF 1 1511 | chr7:50359436-50359463 | IKZF1 3122 | chr7:50404172-50404199 |
| IKZF 1 1512 | chr7:50359462-50359489 | IKZF1 3123 | chr7:50404203-50404230 |
| IKZF 1 1513 | chr7:50359479-50359506 | IKZF1 3124 | chr7:50404215-50404242 |
| IKZF 1 1514 | chr7:50359480-50359507 | IKZF1 3125 | chr7:50404222-50404249 |
| IKZF 1 1515 | chr7:50359499-50359526 | IKZF1 3126 | chr7:50404238-50404265 |
| IKZF 1 1516 | chr7:50359504-50359531 | IKZF1 3127 | chr7:50404241-50404268 |
| IKZF 1 1517 | chr7:50359508-50359535 | IKZF1 3128 | chr7:50404242-50404269 |
| IKZF 1 1518 | chr7:50359513-50359540 | IKZF1 3129 | chr7:50404294-50404321 |
| IKZF 1 1519 | chr7:50359520-50359547 | IKZF1 3130 | chr7:50404302-50404329 |
| IKZF 1 1520 | chr7:50359525-50359552 | IKZF1 3131 | chr7:50404319-50404346 |
| IKZF 1 1521 | chr7:50359561-50359588 | IKZF1 3132 | chr7:50404320-50404347 |
| IKZF 1 1522 | chr7:50359584-50359611 | IKZF1 3133 | chr7:50404332-50404359 |
| IKZF 1 1523 | chr7:50359585-50359612 | IKZF1 3134 | chr7:50404335-50404362 |
| IKZF 1 1524 | chr7:50359586-50359613 | IKZF1 3135 | chr7:50404353-50404380 |
| IKZF 1 1525 | chr7:50359642-50359669 | IKZF1 3136 | chr7:50404373-50404400 |
| IKZF 1 1526 | chr7:50359695-50359722 | IKZF1 3137 | chr7:50404441-50404468 |
| IKZF 1 1527 | chr7:50359703-50359730 | IKZF1 3138 | chr7:50404444-50404471 |
| IKZF 1 1528 | chr7:50359708-50359735 | IKZF1 3139 | chr7:50404445-50404472 |
| IKZF 1 1529 | chr7:50359717-50359744 | IKZF1 3140 | chr7:50404450-50404477 |
| IKZF 1 1530 | chr7:50359718-50359745 | IKZF1 3141 | chr7:50404451-50404478 |
| IKZF 1 1531 | chr7:50359721-50359748 | IKZF1 3142 | chr7:50404453-50404480 |
| IKZF 1 1532 | chr7:50359722-50359749 | IKZF1 3143 | chr7:50404460-50404487 |
| IKZF 1 1533 | chr7:50359728-50359755 | IKZF1 3144 | chr7:50404467-50404494 |
| IKZF 1 1534 | chr7:50359735-50359762 | IKZF1 3145 | chr7:50404481-50404508 |
| IKZF 1 1535 | chr7:50359741-50359768 | IKZF1 3146 | chr7:50404482-50404509 |
| IKZF 1 1536 | chr7:50359742-50359769 | IKZF1 3147 | chr7:50404501-50404528 |
| IKZF 1 1537 | chr7:50359743-50359770 | IKZF1 3148 | chr7:50404510-50404537 |
| IKZF 1 1538 | chr7:50359752-50359779 | IKZF1 3149 | chr7:50404511-50404538 |
| IKZF 1 1539 | chr7:50359765-50359792 | IKZF1 3150 | chr7:50404513-50404540 |
| IKZF 1 1540 | chr7:50359785-50359812 | IKZF1 3151 | chr7:50404514-50404541 |
| IKZF 1 1541 | chr7:50359793-50359820 | IKZF1 3152 | chr7:50404522-50404549 |
| IKZF 1 1542 | chr7:50359811-50359838 | IKZF1 3153 | chr7:50404523-50404550 |
| IKZF 1 1543 | chr7:50359817-50359844 | IKZF1 3154 | chr7:50404524-50404551 |
| IKZF 1 1544 | chr7:50359834-50359861 | IKZF1 3155 | chr7:50404525-50404552 |
| IKZF 1 1545 | chr7:50359835-50359862 | IKZF1 3156 | chr7:50404530-50404557 |
| IKZF 1 1546 | chr7:50359838-50359865 | IKZF1 3157 | chr7:50404550-50404577 |
| IKZF 1 1547 | chr7:50359841-50359868 | IKZF1 3158 | chr7:50404551-50404578 |
| IKZF 1 1548 | chr7:50359842-50359869 | IKZF1 3159 | chr7:50404562-50404589 |
| IKZF 1 1549 | chr7:50359851-50359878 | IKZF1 3160 | chr7:50404570-50404597 |
| IKZF 1 1550 | chr7:50359860-50359887 | IKZF1 3161 | chr7:50404575-50404602 |
| IKZF 1 1551 | chr7:50359864-50359891 | IKZF1 3162 | chr7:50404603-50404630 |
| IKZF 1 1552 | chr7:50359882-50359909 | IKZF1 3163 | chr7:50404624-50404651 |
| IKZF 1 1553 | chr7:50359893-50359920 | IKZF1 3164 | chr7:50404629-50404656 |
| IKZF 1 1554 | chr7:50359894-50359921 | IKZF1 3165 | chr7:50404630-50404657 |
| IKZF 1 1555 | chr7:50359895-50359922 | IKZF1 3166 | chr7:50404631-50404658 |
| IKZF 1 1556 | chr7:50359900-50359927 | IKZF1 3167 | chr7:50404632-50404659 |
| IKZF 1 1557 | chr7:50359905-50359932 | IKZF1 3168 | chr7:50404633-50404660 |
| IKZF 1 1558 | chr7:50359908-50359935 | IKZF1 3169 | chr7:50404654-50404681 |
| IKZF 1 1559 | chr7:50359910-50359937 | IKZF1 3170 | chr7:50404655-50404682 |
| IKZF 1 1560 | chr7:50359911-50359938 | IKZF1 3171 | chr7:50404658-50404685 |
| IKZF 1 1561 | chr7:50359914-50359941 | IKZF1 3172 | chr7:50404659-50404686 |
| IKZF 1 1562 | chr7:50359927-50359954 | IKZF1 3173 | chr7:50404662-50404689 |
| IKZF 1 1563 | chr7:50359931-50359958 | IKZF1 3174 | chr7:50404668-50404695 |
| IKZF 1 1564 | chr7:50359948-50359975 | IKZF1 3175 | chr7:50404669-50404696 |
| IKZF 1 1565 | chr7:50359949-50359976 | IKZF1 3176 | chr7:50404670-50404697 |
| IKZF 1 1566 | chr7:50359950-50359977 | IKZF1 3177 | chr7:50404671-50404698 |
| IKZF 1 1567 | chr7:50359982-50360009 | IKZF1 3178 | chr7:50404672-50404699 |
| IKZF1 1568 | chr7:50359983-50360010 | IKZF1 3179 | chr7:50404717-50404744 |
| IKZF1 1569 | chr7:50359984-50360011 | IKZF1 3180 | chr7:50404725-50404752 |
| IKZF 1 1570 | chr7:50359988-50360015 | IKZF1 3181 | chr7:50404731-50404758 |
| IKZF 1 1571 | chr7:50360002-50360029 | IKZF1 3182 | chr7:50404735-50404762 |
| IKZF 1 1572 | chr7:50360004-50360031 | IKZF1 3183 | chr7:50404736-50404763 |
| IKZF 1 1573 | chr7:50360015-50360042 | IKZF1 3184 | chr7:50404742-50404769 |
| IKZF 1 1574 | chr7:50360025-50360052 | IKZF1 3185 | chr7:50404743-50404770 |
| IKZF 1 1575 | chr7:50360028-50360055 | IKZF1 3186 | chr7:50404759-50404786 |
| IKZF 1 1576 | chr7:50360029-50360056 | IKZF1 3187 | chr7:50404764-50404791 |
| IKZF 1 1577 | chr7:50360036-50360063 | IKZF1 3188 | chr7:50404765-50404792 |
| IKZF 1 1578 | chr7:50360037-50360064 | IKZF1 3189 | chr7:50404771-50404798 |
| IKZF 1 1579 | chr7:50360038-50360065 | IKZF1 3190 | chr7:50404772-50404799 |
| IKZF 1 1580 | chr7:50360039-50360066 | IKZF1 3191 | chr7:50404788-50404815 |
| IKZF 1 1581 | chr7:50360043-50360070 | IKZF1 3192 | chr7:50404818-50404845 |
| IKZF 1 1582 | chr7:50360083-50360110 | IKZF1 3193 | chr7:50404829-50404856 |
| IKZF 1 1583 | chr7:50360084-50360111 | IKZF1 3194 | chr7:50404854-50404881 |
| IKZF 1 1584 | chr7:50360088-50360115 | IKZF1 3195 | chr7:50404863-50404890 |
| IKZF 1 1585 | chr7:50360089-50360116 | IKZF1 3196 | chr7:50404864-50404891 |
| IKZF 1 1586 | chr7:50360092-50360119 | IKZF1 3197 | chr7:50404877-50404904 |
| IKZF 1 1587 | chr7:50360095-50360122 | IKZF1 3198 | chr7:50404891-50404918 |
| IKZF 1 1588 | chr7:50360107-50360134 | IKZF1 3199 | chr7:50404893-50404920 |
| IKZF 1 1589 | chr7:50360108-50360135 | IKZF1 3200 | chr7:50404895-50404922 |
| IKZF 1 1590 | chr7:50360111-50360138 | IKZF1 3201 | chr7:50404898-50404925 |
| IKZF 1 1591 | chr7:50360117-50360144 | IKZF1 3202 | chr7:50404899-50404926 |
| IKZF 1 1592 | chr7:50360118-50360145 | IKZF1 3203 | chr7:50404900-50404927 |
| IKZF 1 1593 | chr7:50360125-50360152 | IKZF1 3204 | chr7:50404914-50404941 |
| IKZF1 1594 | chr7:50360129-50360156 | IKZF1 3205 | chr7:50404915-50404942 |
| IKZF 1 1595 | chr7:50360130-50360157 | IKZF1 3206 | chr7:50404928-50404955 |
| IKZF 1 1596 | chr7:50360133-50360160 | IKZF1 3207 | chr7:50404954-50404981 |
| IKZF 1 1597 | chr7:50360134-50360161 | IKZF1 3208 | chr7:50404955-50404982 |
| IKZF 1 1598 | chr7:50360137-50360164 | IKZF1 3209 | chr7:50404969-50404996 |
| IKZF 1 1599 | chr7:50360138-50360165 | IKZF1 3210 | chr7:50404974-50405001 |
| IKZF 1 1600 | chr7:50360144-50360171 | IKZF1 3211 | chr7:50404975-50405002 |
| IKZF 1 1601 | chr7:50360166-50360193 | IKZF1 3212 | chr7:50404976-50405003 |
| IKZF 1 1602 | chr7:50360167-50360194 | IKZF1 3213 | chr7:50404981-50405008 |
| IKZF1 1603 | chr7:50360168-50360195 | IKZF1 3214 | chr7:50404982-50405009 |
| IKZF 1 1604 | chr7:50360169-50360196 | IKZF1 3215 | chr7:50405013-50405040 |
| IKZF 1 1605 | chr7:50360174-50360201 | IKZF1 3216 | chr7:50405014-50405041 |
| IKZF1 1606 | chr7:50360177-50360204 | IKZF1 3217 | chr7:50405028-50405055 |
| IKZF1 1607 | chr7:50360178-50360205 | IKZF1 3218 | chr7:50405029-50405056 |
| IKZF1 1608 | chr7:50360186-50360213 | IKZF1 3219 | chr7:50405052-50405079 |
| IKZF1 1609 | chr7:50360189-50360216 | IKZF1 3220 | chr7:50405055-50405082 |
| IKZF1 1610 | chr7:50360201-50360228 | IKZF1 3221 | chr7:50405069-50405096 |
| IKZF 1 1611 | chr7:50360202-50360229 | IKZF1 3222 | chr7:50405091-50405118 |

**Tabel 2G. Genomic Coordinates of Human ADNP in the Current Invention**

| Index | Chromosomal coordinate range | Index | Chromosomal coordinate range |
|---|---|---|---|
| ADNP 1 | chr20:50888894-50888921 | ADNP 623 | chr20:50928632-50928659 |
| ADNP 2 | chr20:50888903-50888930 | ADNP 624 | chr20:50928644-50928671 |
| ADNP 3 | chr20:50888941-50888968 | ADNP 625 | chr20:50928646-50928673 |
| ADNP 4 | chr20:50888955-50888982 | ADNP 626 | chr20:50928647-50928674 |
| ADNP 5 | chr20:50888956-50888983 | ADNP 627 | chr20:50928650-50928677 |
| ADNP 6 | chr20:50888959-50888986 | ADNP 628 | chr20:50928658-50928685 |
| ADNP 7 | chr20:50888997-50889024 | ADNP 629 | chr20:50928662-50928689 |
| ADNP 8 | chr20:50889003-50889030 | ADNP 630 | chr20:50928668-50928695 |
| ADNP 9 | chr20:50889013-50889040 | ADNP 631 | chr20:50928673-50928700 |
| ADNP 10 | chr20:50889034-50889061 | ADNP 632 | chr20:50928688-50928715 |
| ADNP 11 | chr20:50889056-50889083 | ADNP 633 | chr20:50928697-50928724 |
| ADNP 12 | chr20:50889057-50889084 | ADNP 634 | chr20:50928698-50928725 |
| ADNP 13 | chr20:50889079-50889106 | ADNP 635 | chr20:50928699-50928726 |
| ADNP 14 | chr20:50889080-50889107 | ADNP 636 | chr20:50928705-50928732 |
| ADNP 15 | chr20:50889099-50889126 | ADNP 637 | chr20:50928711-50928738 |
| ADNP 16 | chr20:50889103-50889130 | ADNP 638 | chr20:50928731-50928758 |
| ADNP 17 | chr20:50889104-50889131 | ADNP 639 | chr20:50928739-50928766 |
| ADNP 18 | chr20:50889107-50889134 | ADNP 640 | chr20:50928742-50928769 |
| ADNP 19 | chr20:50889119-50889146 | ADNP 641 | chr20:50928756-50928783 |
| ADNP 20 | chr20:50889134-50889161 | ADNP 642 | chr20:50928768-50928795 |
| ADNP 21 | chr20:50889140-50889167 | ADNP 643 | chr20:50928769-50928796 |
| ADNP 22 | chr20:50889141-50889168 | ADNP 644 | chr20:50928770-50928797 |
| ADNP 23 | chr20:50889154-50889181 | ADNP 645 | chr20:50928776-50928803 |
| ADNP 24 | chr20:50889158-50889185 | ADNP 646 | chr20:50928798-50928825 |
| ADNP 25 | chr20:50889172-50889199 | ADNP 647 | chr20:50928804-50928831 |
| ADNP 26 | chr20:50889175-50889202 | ADNP 648 | chr20:50928817-50928844 |
| ADNP 27 | chr20:50889196-50889223 | ADNP 649 | chr20:50928825-50928852 |
| ADNP 28 | chr20:50889197-50889224 | ADNP 650 | chr20:50928831-50928858 |
| ADNP 29 | chr20:50889203-50889230 | ADNP 651 | chr20:50928853-50928880 |
| ADNP 30 | chr20:50889209-50889236 | ADNP 652 | chr20:50928856-50928883 |
| ADNP 31 | chr20:50889216-50889243 | ADNP 653 | chr20:50928857-50928884 |
| ADNP 32 | chr20:50889219-50889246 | ADNP 654 | chr20:50928870-50928897 |
| ADNP 33 | chr20:50889226-50889253 | ADNP 655 | chr20:50928871-50928898 |
| ADNP 34 | chr20:50889232-50889259 | ADNP 656 | chr20:50928874-50928901 |
| ADNP 35 | chr20:50889233-50889260 | ADNP 657 | chr20:50928894-50928921 |
| ADNP 36 | chr20:50889234-50889261 | ADNP 658 | chr20:50928897-50928924 |
| ADNP 37 | chr20:50889239-50889266 | ADNP 659 | chr20:50928901-50928928 |
| ADNP 38 | chr20:50889240-50889267 | ADNP 660 | chr20:50928920-50928947 |
| ADNP 39 | chr20:50889241-50889268 | ADNP 661 | chr20:50928941-50928968 |
| ADNP 40 | chr20:50889270-50889297 | ADNP 662 | chr20:50928944-50928971 |
| ADNP 41 | chr20:50889273-50889300 | ADNP 663 | chr20:50928945-50928972 |
| ADNP 42 | chr20:50889276-50889303 | ADNP 664 | chr20:50928959-50928986 |
| ADNP 43 | chr20:50889277-50889304 | ADNP 665 | chr20:50928999-50929026 |
| ADNP 44 | chr20:50889280-50889307 | ADNP 666 | chr20:50929000-50929027 |
| ADNP 45 | chr20:50889281-50889308 | ADNP 667 | chr20:50929017-50929044 |
| ADNP 46 | chr20:50889291-50889318 | ADNP 668 | chr20:50929018-50929045 |
| ADNP 47 | chr20:50889306-50889333 | ADNP 669 | chr20:50929020-50929047 |
| ADNP 48 | chr20:50889315-50889342 | ADNP 670 | chr20:50929039-50929066 |
| ADNP 49 | chr20:50889321-50889348 | ADNP 671 | chr20:50929065-50929092 |
| ADNP 50 | chr20:50889334-50889361 | ADNP 672 | chr20:50929066-50929093 |
| ADNP 51 | chr20:50889336-50889363 | ADNP 673 | chr20:50929083-50929110 |
| ADNP 52 | chr20:50889341-50889368 | ADNP 674 | chr20:50929097-50929124 |
| ADNP 53 | chr20:50889359-50889386 | ADNP 675 | chr20:50929107-50929134 |
| ADNP 54 | chr20:50889360-50889387 | ADNP 676 | chr20:50929120-50929147 |
| ADNP 55 | chr20:50889361-50889388 | ADNP 677 | chr20:50929121-50929148 |
| ADNP 56 | chr20:50889382-50889409 | ADNP 678 | chr20:50929135-50929162 |
| ADNP 57 | chr20:50889386-50889413 | ADNP 679 | chr20:50929155-50929182 |
| ADNP 58 | chr20:50889393-50889420 | ADNP 680 | chr20:50929164-50929191 |
| ADNP 59 | chr20:50889398-50889425 | ADNP 681 | chr20:50929165-50929192 |
| ADNP 60 | chr20:50889436-50889463 | ADNP 682 | chr20:50929166-50929193 |
| ADNP 61 | chr20:50889463-50889490 | ADNP 683 | chr20:50929167-50929194 |
| ADNP 62 | chr20:50889464-50889491 | ADNP 684 | chr20:50929172-50929199 |
| ADNP 63 | chr20:50889469-50889496 | ADNP 685 | chr20:50929189-50929216 |
| ADNP 64 | chr20:50889473-50889500 | ADNP 686 | chr20:50929191-50929218 |
| ADNP 65 | chr20:50889474-50889501 | ADNP 687 | chr20:50929197-50929224 |
| ADNP 66 | chr20:50889475-50889502 | ADNP 688 | chr20:50929199-50929226 |
| ADNP 67 | chr20:50889482-50889509 | ADNP 689 | chr20:50929203-50929230 |
| ADNP 68 | chr20:50889515-50889542 | ADNP 690 | chr20:50929206-50929233 |
| ADNP 69 | chr20:50889520-50889547 | ADNP 691 | chr20:50929220-50929247 |
| ADNP 70 | chr20:50889521-50889548 | ADNP 692 | chr20:50929223-50929250 |
| ADNP 71 | chr20:50889526-50889553 | ADNP 693 | chr20:50929237-50929264 |
| ADNP 72 | chr20:50889530-50889557 | ADNP 694 | chr20:50929241-50929268 |
| ADNP 73 | chr20:50889549-50889576 | ADNP 695 | chr20:50929242-50929269 |
| ADNP 74 | chr20:50889579-50889606 | ADNP 696 | chr20:50929256-50929283 |
| ADNP 75 | chr20:50889600-50889627 | ADNP 697 | chr20:50929258-50929285 |
| ADNP 76 | chr20:50889627-50889654 | ADNP 698 | chr20:50929267-50929294 |
| ADNP 77 | chr20:50889635-50889662 | ADNP 699 | chr20:50929273-50929300 |
| ADNP 78 | chr20:50889642-50889669 | ADNP 700 | chr20:50929278-50929305 |
| ADNP 79 | chr20:50889655-50889682 | ADNP 701 | chr20:50929289-50929316 |
| ADNP 80 | chr20:50889662-50889689 | ADNP 702 | chr20:50929290-50929317 |
| ADNP 81 | chr20:50889664-50889691 | ADNP 703 | chr20:50929295-50929322 |
| ADNP 82 | chr20:50889669-50889696 | ADNP 704 | chr20:50929296-50929323 |
| ADNP 83 | chr20:50889677-50889704 | ADNP 705 | chr20:50929297-50929324 |
| ADNP 84 | chr20:50889681-50889708 | ADNP 706 | chr20:50929298-50929325 |
| ADNP 85 | chr20:50889684-50889711 | ADNP 707 | *c*hr20:50929323-50929350 |
| ADNP 86 | chr20:50889697-50889724 | ADNP 708 | chr20:50929326-50929353 |
| ADNP 87 | chr20:50889702-50889729 | ADNP 709 | chr20:50929344-50929371 |
| ADNP 88 | chr20:50889703-50889730 | ADNP 710 | chr20:50929345-50929372 |
| ADNP 89 | chr20:50889706-50889733 | ADNP 711 | chr20:50929348-50929375 |
| ADNP 90 | chr20:50889734-50889761 | ADNP 712 | chr20:50929349-50929376 |
| ADNP 91 | chr20:50889747-50889774 | ADNP 713 | chr20:50929419-50929446 |
| ADNP 92 | chr20:50889757-50889784 | ADNP 714 | chr20:50929425-50929452 |
| ADNP 93 | chr20:50889758-50889785 | ADNP 715 | chr20:50929444-50929471 |
| ADNP 94 | chr20:50889771-50889798 | ADNP 716 | chr20:50929446-50929473 |
| ADNP 95 | chr20:50889807-50889834 | ADNP 717 | chr20:50929447-50929474 |
| ADNP 96 | chr20:50889813-50889840 | ADNP 718 | chr20:50929462-50929489 |
| ADNP 97 | chr20:50889817-50889844 | ADNP 719 | chr20:50929473-50929500 |
| ADNP 98 | chr20:50889829-50889856 | ADNP 720 | chr20:50929475-50929502 |
| ADNP 99 | chr20:50889830-50889857 | ADNP 721 | chr20:50929476-50929503 |
| ADNP 100 | chr20:50889849-50889876 | ADNP 722 | chr20:50929483-50929510 |
| ADNP 101 | chr20:50889850-50889877 | ADNP 723 | chr20:50929484-50929511 |
| ADNP 102 | chr20:50889855-50889882 | ADNP 724 | chr20:50929503-50929530 |
| ADNP 103 | chr20:50889860-50889887 | ADNP 725 | chr20:50929504-50929531 |
| ADNP 104 | chr20:50889861-50889888 | ADNP 726 | chr20:50929505-50929532 |
| ADNP 105 | chr20:50889870-50889897 | ADNP 727 | chr20:50929506-50929533 |
| ADNP 106 | chr20:50889878-50889905 | ADNP 728 | chr20:50929516-50929543 |
| ADNP 107 | chr20:50889903-50889930 | ADNP 729 | chr20:50929534-50929561 |
| ADNP 108 | chr20:50889906-50889933 | ADNP 730 | chr20:50929535-50929562 |
| ADNP 109 | chr20:50889907-50889934 | ADNP 731 | chr20:50929539-50929566 |
| ADNP 110 | chr20:50889923-50889950 | ADNP 732 | chr20:50929540-50929567 |
| ADNP 111 | chr20:50889924-50889951 | ADNP 733 | chr20:50929541-50929568 |
| ADNP 112 | chr20:50889963-50889990 | ADNP 734 | chr20:50929542-50929569 |
| ADNP 113 | chr20:50889982-50890009 | ADNP 735 | chr20:50929543-50929570 |
| ADNP 114 | chr20:50889983-50890010 | ADNP 736 | chr20:50929552-50929579 |
| ADNP 115 | chr20:50890019-50890046 | ADNP 737 | chr20:50929557-50929584 |
| ADNP 116 | chr20:50890024-50890051 | ADNP 738 | chr20:50929558-50929585 |
| ADNP 117 | chr20:50890048-50890075 | ADNP 739 | chr20:50929575-50929602 |
| ADNP 118 | chr20:50890068-50890095 | ADNP 740 | chr20:50929576-50929603 |
| ADNP 119 | chr20:50890083-50890110 | ADNP 741 | chr20:50929577-50929604 |
| ADNP 120 | chr20:50890087-50890114 | ADNP 742 | chr20:50929578-50929605 |
| ADNP 121 | chr20:50890088-50890115 | ADNP 743 | chr20:50929588-50929615 |
| ADNP 122 | chr20:50890156-50890183 | ADNP 744 | chr20:50929589-50929616 |
| ADNP 123 | chr20:50890179-50890206 | ADNP 745 | chr20:50929595-50929622 |
| ADNP 124 | chr20:50890186-50890213 | ADNP 746 | chr20:50929601-50929628 |
| ADNP 125 | chr20:50890199-50890226 | ADNP 747 | chr20:50929605-50929632 |
| ADNP 126 | chr20:50890200-50890227 | ADNP 748 | chr20:50929606-50929633 |
| ADNP 127 | chr20:50890229-50890256 | ADNP 749 | chr20:50929608-50929635 |
| ADNP 128 | chr20:50890232-50890259 | ADNP 750 | chr20:50929609-50929636 |
| ADNP 129 | chr20:50890242-50890269 | ADNP 751 | chr20:50929610-50929637 |
| ADNP 130 | chr20:50890256-50890283 | ADNP 752 | chr20:50929611-50929638 |
| ADNP 131 | chr20:50890260-50890287 | ADNP 753 | chr20:50929612-50929639 |
| ADNP 132 | chr20:50890266-50890293 | ADNP 754 | chr20:50929616-50929643 |
| ADNP 133 | chr20:50890281-50890308 | ADNP 755 | chr20:50929630-50929657 |
| ADNP 134 | chr20:50890290-50890317 | ADNP 756 | chr20:50929652-50929679 |
| ADNP 135 | chr20:50890370-50890397 | ADNP 757 | chr20:50929658-50929685 |
| ADNP 136 | chr20:50890371-50890398 | ADNP 758 | chr20:50929665-50929692 |
| ADNP 137 | chr20:50890426-50890453 | ADNP 759 | chr20:50929710-50929737 |
| ADNP 138 | chr20:50890449-50890476 | ADNP 760 | chr20:50929714-50929741 |
| ADNP 139 | chr20:50890465-50890492 | ADNP 761 | chr20:50929727-50929754 |
| ADNP 140 | chr20:50890468-50890495 | ADNP 762 | chr20:50929743-50929770 |
| ADNP 141 | chr20:50890497-50890524 | ADNP 763 | chr20:50929744-50929771 |
| ADNP 142 | chr20:50890504-50890531 | ADNP 764 | chr20:50929748-50929775 |
| ADNP 143 | chr20:50890540-50890567 | ADNP 765 | chr20:50929749-50929776 |
| ADNP 144 | chr20:50890646-50890673 | ADNP 766 | chr20:50929755-50929782 |
| ADNP 145 | chr20:50890652-50890679 | ADNP 767 | chr20:50929756-50929783 |
| ADNP 146 | chr20:50890700-50890727 | ADNP 768 | chr20:50929763-50929790 |
| ADNP 147 | chr20:50890711-50890738 | ADNP 769 | chr20:50929764-50929791 |
| ADNP 148 | chr20:50890715-50890742 | ADNP 770 | chr20:50929765-50929792 |
| ADNP 149 | chr20:50890718-50890745 | ADNP 771 | chr20:50929769-50929796 |
| ADNP 150 | chr20:50890739-50890766 | ADNP 772 | chr20:50929804-50929831 |
| ADNP 151 | chr20:50890743-50890770 | ADNP 773 | chr20:50929840-50929867 |
| ADNP 152 | chr20:50890790-50890817 | ADNP 774 | chr20:50929841-50929868 |
| ADNP 153 | chr20:50890799-50890826 | ADNP 775 | chr20:50929842-50929869 |
| ADNP 154 | chr20:50890803-50890830 | ADNP 776 | chr20:50929844-50929871 |
| ADNP 155 | chr20:50890836-50890863 | ADNP 777 | chr20:50929848-50929875 |
| ADNP 156 | chr20:50890837-50890864 | ADNP 778 | chr20:50929850-50929877 |
| ADNP 157 | chr20:50890865-50890892 | ADNP 779 | chr20:50929871-50929898 |
| ADNP 158 | chr20:50890866-50890893 | ADNP 780 | chr20:50929886-50929913 |
| ADNP 159 | chr20:50890880-50890907 | ADNP 781 | chr20:50929906-50929933 |
| ADNP 160 | chr20:50890903-50890930 | ADNP 782 | chr20:50929907-50929934 |
| ADNP 161 | chr20:50890997-50891024 | ADNP 783 | chr20:50929908-50929935 |
| ADNP 162 | chr20:50890998-50891025 | ADNP 784 | chr20:50929909-50929936 |
| ADNP 163 | chr20:50891013-50891040 | ADNP 785 | chr20:50929910-50929937 |
| ADNP 164 | chr20:50891041-50891068 | ADNP 786 | chr20:50929923-50929950 |
| ADNP 165 | chr20:50891042-50891069 | ADNP 787 | chr20:50929932-50929959 |
| ADNP 166 | chr20:50891051-50891078 | ADNP 788 | chr20:50929953-50929980 |
| ADNP 167 | chr20:50891079-50891106 | ADNP 789 | chr20:50929959-50929986 |
| ADNP 168 | chr20:50891080-50891107 | ADNP 790 | chr20:50929960-50929987 |
| ADNP 169 | chr20:50891089-50891116 | ADNP 791 | chr20:50929968-50929995 |
| ADNP 170 | chr20:50891098-50891125 | ADNP 792 | chr20:50929969-50929996 |
| ADNP 171 | chr20:50891139-50891166 | ADNP 793 | chr20:50930003-50930030 |
| ADNP 172 | chr20:50891150-50891177 | ADNP 794 | chr20:50930004-50930031 |
| ADNP 173 | chr20:50891156-50891183 | ADNP 795 | chr20:50930011-50930038 |
| ADNP 174 | chr20:50891172-50891199 | ADNP 796 | chr20:50930013-50930040 |
| ADNP 175 | chr20:50891192-50891219 | ADNP 797 | chr20:50930015-50930042 |
| ADNP 176 | chr20:50891201-50891228 | ADNP 798 | chr20:50930016-50930043 |
| ADNP 177 | chr20:50891237-50891264 | ADNP 799 | chr20:50930017-50930044 |
| ADNP 178 | chr20:50891242-50891269 | ADNP 800 | chr20:50930018-50930045 |
| ADNP 179 | chr20:50891243-50891270 | ADNP 801 | chr20:50930019-50930046 |
| ADNP 180 | chr20:50891254-50891281 | ADNP 802 | chr20:50930020-50930047 |
| ADNP 181 | chr20:50891264-50891291 | ADNP 803 | chr20:50930023-50930050 |
| ADNP 182 | chr20:50891282-50891309 | ADNP 804 | chr20:50930024-50930051 |
| ADNP 183 | chr20:50891289-50891316 | ADNP 805 | chr20:50930025-50930052 |
| ADNP 184 | chr20:50891290-50891317 | ADNP 806 | chr20:50930029-50930056 |
| ADNP 185 | chr20:50891291-50891318 | ADNP 807 | chr20:50930033-50930060 |
| ADNP 186 | chr20:50891298-50891325 | ADNP 808 | chr20:50930037-50930064 |
| ADNP 187 | chr20:50891302-50891329 | ADNP 809 | chr20:50930048-50930075 |
| ADNP 188 | chr20:50891322-50891349 | ADNP 810 | chr20:50930058-50930085 |
| ADNP 189 | chr20:50891326-50891353 | ADNP 811 | chr20:50930065-50930092 |
| ADNP 190 | chr20:50891342-50891369 | ADNP 812 | chr20:50930068-50930095 |
| ADNP 191 | chr20:50891365-50891392 | ADNP 813 | chr20:50930069-50930096 |
| ADNP 192 | chr20:50891366-50891393 | ADNP 814 | chr20:50930114-50930141 |
| ADNP 193 | chr20:50891367-50891394 | ADNP 815 | chr20:50930115-50930142 |
| ADNP 194 | chr20:50891374-50891401 | ADNP 816 | chr20:50930116-50930143 |
| ADNP 195 | chr20:50891382-50891409 | ADNP 817 | chr20:50930130-50930157 |
| ADNP 196 | chr20:50891383-50891410 | ADNP 818 | chr20:50930162-50930189 |
| ADNP 197 | chr20:50891389-50891416 | ADNP 819 | chr20:50930164-50930191 |
| ADNP 198 | chr20:50891390-50891417 | ADNP 820 | chr20:50930165-50930192 |
| ADNP 199 | chr20:50891397-50891424 | ADNP 821 | chr20:50930166-50930193 |
| ADNP 200 | chr20:50891409-50891436 | ADNP 822 | chr20:50930172-50930199 |
| ADNP 201 | chr20:50891410-50891437 | ADNP 823 | chr20:50930175-50930202 |
| ADNP 202 | chr20:50891427-50891454 | ADNP 824 | chr20:50930186-50930213 |
| ADNP 203 | chr20:50891428-50891455 | ADNP 825 | chr20:50930197-50930224 |
| ADNP 204 | chr20:50891433-50891460 | ADNP 826 | chr20:50930210-50930237 |
| ADNP 205 | chr20:50891445-50891472 | ADNP 827 | chr20:50930211-50930238 |
| ADNP 206 | chr20:50891466-50891493 | ADNP 828 | chr20:50930212-50930239 |
| ADNP 207 | chr20:50891491-50891518 | ADNP 829 | chr20:50930218-50930245 |
| ADNP 208 | chr20:50891492-50891519 | ADNP 830 | chr20:50930219-50930246 |
| ADNP 209 | chr20:50891493-50891520 | ADNP 831 | chr20:50930220-50930247 |
| ADNP 210 | chr20:50891497-50891524 | ADNP 832 | chr20:50930223-50930250 |
| ADNP 211 | chr20:50891507-50891534 | ADNP 833 | chr20:50930226-50930253 |
| ADNP 212 | chr20:50891508-50891535 | ADNP 834 | chr20:50930236-50930263 |
| ADNP 213 | chr20:50891509-50891536 | ADNP 835 | chr20:50930241-50930268 |
| ADNP 214 | chr20:50891510-50891537 | ADNP 836 | chr20:50930249-50930276 |
| ADNP 215 | chr20:50891522-50891549 | ADNP 837 | chr20:50930257-50930284 |
| ADNP 216 | chr20:50891558-50891585 | ADNP 838 | chr20:50930261-50930288 |
| ADNP 217 | chr20:50891573-50891600 | ADNP 839 | chr20:50930265-50930292 |
| ADNP 218 | chr20:50891587-50891614 | ADNP 840 | chr20:50930266-50930293 |
| ADNP 219 | chr20:50891592-50891619 | ADNP 841 | chr20:50930267-50930294 |
| ADNP 220 | chr20:50891594-50891621 | ADNP 842 | chr20:50930274-50930301 |
| ADNP 221 | chr20:50891600-50891627 | ADNP 843 | chr20:50930284-50930311 |
| ADNP 222 | chr20:50891601-50891628 | ADNP 844 | chr20:50930285-50930312 |
| ADNP 223 | chr20:50891613-50891640 | ADNP 845 | chr20:50930286-50930313 |
| ADNP 224 | chr20:50891630-50891657 | ADNP 846 | chr20:50930289-50930316 |
| ADNP 225 | chr20:50891634-50891661 | ADNP 847 | chr20:50930329-50930356 |
| ADNP 226 | chr20:50891649-50891676 | ADNP 848 | chr20:50930334-50930361 |
| ADNP 227 | chr20:50891652-50891679 | ADNP 849 | chr20:50930801-50930828 |
| ADNP 228 | chr20:50891656-50891683 | ADNP 850 | chr20:50930808-50930835 |
| ADNP 229 | chr20:50891665-50891692 | ADNP 851 | chr20:50930816-50930843 |
| ADNP 230 | chr20:50891684-50891711 | ADNP 852 | chr20:50930825-50930852 |
| ADNP 231 | chr20:50891685-50891712 | ADNP 853 | chr20:50930826-50930853 |
| ADNP 232 | chr20:50891693-50891720 | ADNP 854 | chr20:50930827-50930854 |
| ADNP 233 | chr20:50891703-50891730 | ADNP 855 | chr20:50930831-50930858 |
| ADNP 234 | chr20:50891710-50891737 | ADNP 856 | chr20:50930836-50930863 |
| ADNP 235 | chr20:50891724-50891751 | ADNP 857 | chr20:50930840-50930867 |
| ADNP 236 | chr20:50891726-50891753 | ADNP 858 | chr20:50930841-50930868 |
| ADNP 237 | chr20:50891733-50891760 | ADNP 859 | chr20:50930842-50930869 |
| ADNP 238 | chr20:50891753-50891780 | ADNP 860 | chr20:50930843-50930870 |
| ADNP 239 | chr20:50891754-50891781 | ADNP 861 | chr20:50930847-50930874 |
| ADNP 240 | chr20:50891758-50891785 | ADNP 862 | chr20:50930854-50930881 |
| ADNP 241 | chr20:50891761-50891788 | ADNP 863 | chr20:50930855-50930882 |
| ADNP 242 | chr20:50891776-50891803 | ADNP 864 | chr20:50930857-50930884 |
| ADNP 243 | chr20:50891784-50891811 | ADNP 865 | chr20:50930860-50930887 |
| ADNP 244 | chr20:50891789-50891816 | ADNP 866 | chr20:50930863-50930890 |
| ADNP 245 | chr20:50891790-50891817 | ADNP 867 | chr20:50930866-50930893 |
| ADNP 246 | chr20:50891811-50891838 | ADNP 868 | chr20:50930867-50930894 |
| ADNP 247 | chr20:50891813-50891840 | ADNP 869 | chr20:50930870-50930897 |
| ADNP 248 | chr20:50891819-50891846 | ADNP 870 | chr20:50930871-50930898 |
| ADNP 249 | chr20:50891845-50891872 | ADNP 871 | chr20:50930874-50930901 |
| ADNP 250 | chr20:50891850-50891877 | ADNP 872 | chr20:50930877-50930904 |
| ADNP 251 | chr20:50891854-50891881 | ADNP 873 | chr20:50930880-50930907 |
| ADNP 252 | chr20:50891884-50891911 | ADNP 874 | chr20:50930885-50930912 |
| ADNP 253 | chr20:50891903-50891930 | ADNP 875 | chr20:50930888-50930915 |
| ADNP 254 | chr20:50891916-50891943 | ADNP 876 | chr20:50930891-50930918 |
| ADNP 255 | chr20:50891920-50891947 | ADNP 877 | chr20:50930894-50930921 |
| ADNP 256 | chr20:50891938-50891965 | ADNP 878 | chr20:50930900-50930927 |
| ADNP 257 | chr20:50891941-50891968 | ADNP 879 | chr20:50930901-50930928 |
| ADNP 258 | chr20:50891959-50891986 | ADNP 880 | chr20:50930902-50930929 |
| ADNP 259 | chr20:50891965-50891992 | ADNP 881 | chr20:50930905-50930932 |
| ADNP 260 | chr20:50891966-50891993 | ADNP 882 | chr20:50930906-50930933 |
| ADNP 261 | chr20:50891971-50891998 | ADNP 883 | chr20:50930915-50930942 |
| ADNP 262 | chr20:50891986-50892013 | ADNP 884 | chr20:50930916-50930943 |
| ADNP 263 | chr20:50892004-50892031 | ADNP 885 | chr20:50930917-50930944 |
| ADNP 264 | chr20:50892005-50892032 | ADNP 886 | chr20:50930933-50930960 |
| ADNP 265 | chr20:50892013-50892040 | ADNP 887 | chr20:50930938-50930965 |
| ADNP 266 | chr20:50892014-50892041 | ADNP 888 | chr20:50930939-50930966 |
| ADNP 267 | chr20:50892030-50892057 | ADNP 889 | chr20:50930941-50930968 |
| ADNP 268 | chr20:50892045-50892072 | ADNP 890 | chr20:50930945-50930972 |
| ADNP 269 | chr20:50892063-50892090 | ADNP 891 | chr20:50930948-50930975 |
| ADNP 270 | chr20:50892067-50892094 | ADNP 892 | chr20:50930957-50930984 |
| ADNP 271 | chr20:50892086-50892113 | ADNP 893 | chr20:50930960-50930987 |
| ADNP 272 | chr20:50892103-50892130 | ADNP 894 | chr20:50930966-50930993 |
| ADNP 273 | chr20:50892107-50892134 | ADNP 895 | chr20:50930967-50930994 |
| ADNP 274 | chr20:50892108-50892135 | ADNP 896 | chr20:50930972-50930999 |
| ADNP 275 | chr20:50892126-50892153 | ADNP 897 | chr20:50930976-50931003 |
| ADNP 276 | chr20:50892157-50892184 | ADNP 898 | chr20:50930977-50931004 |
| ADNP 277 | chr20:50892182-50892209 | ADNP 899 | chr20:50930978-50931005 |
| ADNP 278 | chr20:50892199-50892226 | ADNP 900 | chr20:50930979-50931006 |
| ADNP 279 | chr20:50892229-50892256 | ADNP 901 | chr20:50930980-50931007 |
| ADNP 280 | chr20:50892239-50892266 | ADNP 902 | chr20:50930982-50931009 |
| ADNP 281 | chr20:50892240-50892267 | ADNP 903 | chr20:50930985-50931012 |
| ADNP 282 | chr20:50892241-50892268 | ADNP 904 | chr20:50930988-50931015 |
| ADNP 283 | chr20:50892251-50892278 | ADNP 905 | chr20:50930989-50931016 |
| ADNP 284 | chr20:50892252-50892279 | ADNP 906 | chr20:50931000-50931027 |
| ADNP 285 | chr20:50892281-50892308 | ADNP 907 | chr20:50931002-50931029 |
| ADNP 286 | chr20:50892282-50892309 | ADNP 908 | chr20:50931005-50931032 |
| ADNP 287 | chr20:50892290-50892317 | ADNP 909 | chr20:50931006-50931033 |
| ADNP 288 | chr20:50892315-50892342 | ADNP 910 | chr20:50931009-50931036 |
| ADNP 289 | chr20:50892326-50892353 | ADNP 911 | chr20:50931012-50931039 |
| ADNP 290 | chr20:50892332-50892359 | ADNP 912 | chr20:50931015-50931042 |
| ADNP 291 | chr20:50892342-50892369 | ADNP 913 | chr20:50931028-50931055 |
| ADNP 292 | chr20:50892345-50892372 | ADNP 914 | chr20:50931040-50931067 |
| ADNP 293 | chr20:50892346-50892373 | ADNP 915 | chr20:50931043-50931070 |
| ADNP 294 | chr20:50892351-50892378 | ADNP 916 | chr20:50931044-50931071 |
| ADNP 295 | chr20:50892352-50892379 | ADNP 917 | chr20:50931045-50931072 |
| ADNP 296 | chr20:50892365-50892392 | ADNP 918 | chr20:50931048-50931075 |
| ADNP 297 | chr20:50892390-50892417 | ADNP 919 | chr20:50931051-50931078 |
| ADNP 298 | chr20:50892399-50892426 | ADNP 920 | chr20:50931052-50931079 |
| ADNP 299 | chr20:50892413-50892440 | ADNP 921 | chr20:50931055-50931082 |
| ADNP 300 | chr20:50892420-50892447 | ADNP 922 | chr20:50931061-50931088 |
| ADNP 301 | chr20:50892421-50892448 | ADNP 923 | chr20:50931064-50931091 |
| ADNP 302 | chr20:50892422-50892449 | ADNP 924 | chr20:50931065-50931092 |
| ADNP 303 | chr20:50892441-50892468 | ADNP 925 | chr20:50931070-50931097 |
| ADNP 304 | chr20:50892442-50892469 | ADNP 926 | chr20:50931071-50931098 |
| ADNP 305 | chr20:50892451-50892478 | ADNP 927 | chr20:50931076-50931103 |
| ADNP 306 | chr20:50892471-50892498 | ADNP 928 | chr20:50931079-50931106 |
| ADNP 307 | chr20:50892472-50892499 | ADNP 929 | chr20:50931088-50931115 |
| ADNP 308 | chr20:50892513-50892540 | ADNP 930 | chr20:50931093-50931120 |
| ADNP 309 | chr20:50892514-50892541 | ADNP 931 | chr20:50931094-50931121 |
| ADNP 310 | chr20:50892547-50892574 | ADNP 932 | chr20:50931097-50931124 |
| ADNP 311 | chr20:50892559-50892586 | ADNP 933 | chr20:50931100-50931127 |
| ADNP 312 | chr20:50892577-50892604 | ADNP 934 | chr20:50931103-50931130 |
| ADNP 313 | chr20:50892579-50892606 | ADNP 935 | chr20:50931112-50931139 |
| ADNP 314 | chr20:50892580-50892607 | ADNP 936 | chr20:50931131-50931158 |
| ADNP 315 | chr20:50892599-50892626 | ADNP 937 | chr20:50931132-50931159 |
| ADNP 316 | chr20:50892608-50892635 | ADNP 938 | chr20:50931133-50931160 |
| ADNP 317 | chr20:50892609-50892636 | ADNP 939 | chr20:50931138-50931165 |
| ADNP 318 | chr20:50892622-50892649 | ADNP 940 | chr20:50931144-50931171 |
| ADNP 319 | chr20:50892627-50892654 | ADNP 941 | chr20:50931145-50931172 |
| ADNP 320 | chr20:50892642-50892669 | ADNP 942 | chr20:50931147-50931174 |
| ADNP 321 | chr20:50892648-50892675 | ADNP 943 | chr20:50931162-50931189 |
| ADNP 322 | chr20:50892649-50892676 | ADNP 944 | chr20:50931192-50931219 |
| ADNP 323 | chr20:50892650-50892677 | ADNP 945 | chr20:50931193-50931220 |
| ADNP 324 | chr20:50892657-50892684 | ADNP 946 | chr20:50931196-50931223 |
| ADNP 325 | chr20:50892660-50892687 | ADNP 947 | chr20:50931197-50931224 |
| ADNP 326 | chr20:50892672-50892699 | ADNP 948 | chr20:50931198-50931225 |
| ADNP 327 | chr20:50892686-50892713 | ADNP 949 | chr20:50931199-50931226 |
| ADNP 328 | chr20:50892692-50892719 | ADNP 950 | chr20:50931200-50931227 |
| ADNP 329 | chr20:50892705-50892732 | ADNP 951 | chr20:50931201-50931228 |
| ADNP 330 | chr20:50892708-50892735 | ADNP 952 | chr20:50931202-50931229 |
| ADNP 331 | chr20:50892724-50892751 | ADNP 953 | chr20:50931203-50931230 |
| ADNP 332 | chr20:50892746-50892773 | ADNP 954 | chr20:50931204-50931231 |
| ADNP 333 | chr20:50892757-50892784 | ADNP 955 | chr20:50931205-50931232 |
| ADNP 334 | chr20:50892776-50892803 | ADNP 956 | chr20:50931213-50931240 |
| ADNP 335 | chr20:50892786-50892813 | ADNP 957 | chr20:50931215-50931242 |
| ADNP 336 | chr20:50892787-50892814 | ADNP 958 | chr20:50931218-50931245 |
| ADNP 337 | chr20:50892797-50892824 | ADNP 959 | chr20:50931221-50931248 |
| ADNP 338 | chr20:50892814-50892841 | ADNP 960 | chr20:50931225-50931252 |
| ADNP 339 | chr20:50892816-50892843 | ADNP 961 | chr20:50931226-50931253 |
| ADNP 340 | chr20:50892821-50892848 | ADNP 962 | chr20:50931227-50931254 |
| ADNP 341 | chr20:50892822-50892849 | ADNP 963 | chr20:50931228-50931255 |
| ADNP 342 | chr20:50892846-50892873 | ADNP 964 | chr20:50931229-50931256 |
| ADNP 343 | chr20:50892847-50892874 | ADNP 965 | chr20:50931230-50931257 |
| ADNP 344 | chr20:50892854-50892881 | ADNP 966 | chr20:50931231-50931258 |
| ADNP 345 | chr20:50892855-50892882 | ADNP 967 | chr20:50931232-50931259 |
| ADNP 346 | chr20:50892862-50892889 | ADNP 968 | chr20:50931233-50931260 |
| ADNP 347 | chr20:50892877-50892904 | ADNP 969 | chr20:50931234-50931261 |
| ADNP 348 | chr20:50892878-50892905 | ADNP 970 | chr20:50931235-50931262 |
| ADNP 349 | chr20:50892904-50892931 | ADNP 971 | chr20:50931236-50931263 |
| ADNP 350 | chr20:50892910-50892937 | ADNP 972 | chr20:50931239-50931266 |
| ADNP 351 | chr20:50892913-50892940 | ADNP 973 | chr20:50931240-50931267 |
| ADNP 352 | chr20:50892916-50892943 | ADNP 974 | chr20:50931249-50931276 |
| ADNP 353 | chr20:50892919-50892946 | ADNP 975 | chr20:50931254-50931281 |
| ADNP 354 | chr20:50892925-50892952 | ADNP 976 | chr20:50931261-50931288 |
| ADNP 355 | chr20:50892928-50892955 | ADNP 977 | chr20:50931264-50931291 |
| ADNP 356 | chr20:50892938-50892965 | ADNP 978 | chr20:50931265-50931292 |
| ADNP 357 | chr20:50892939-50892966 | ADNP 979 | chr20:50931268-50931295 |
| ADNP 358 | chr20:50892944-50892971 | ADNP 980 | chr20:50931271-50931298 |
| ADNP 359 | chr20:50892964-50892991 | ADNP 981 | chr20:50931274-50931301 |
| ADNP 360 | chr20:50892965-50892992 | ADNP 982 | chr20:50931275-50931302 |
| ADNP 361 | chr20:50892966-50892993 | ADNP 983 | chr20:50931276-50931303 |
| ADNP 362 | chr20:50892995-50893022 | ADNP 984 | chr20:50931283-50931310 |
| ADNP 363 | chr20:50892997-50893024 | ADNP 985 | chr20:50931284-50931311 |
| ADNP 364 | chr20:50892998-50893025 | ADNP 986 | chr20:50931285-50931312 |
| ADNP 365 | chr20:50893001-50893028 | ADNP 987 | chr20:50931288-50931315 |
| ADNP 366 | chr20:50893018-50893045 | ADNP 988 | chr20:50931291-50931318 |
| ADNP 367 | chr20:50893044-50893071 | ADNP 989 | chr20:50931294-50931321 |
| ADNP 368 | chr20:50893045-50893072 | ADNP 990 | chr20:50931297-50931324 |
| ADNP 369 | chr20:50893063-50893090 | ADNP 991 | chr20:50931300-50931327 |
| ADNP 370 | chr20:50893092-50893119 | ADNP 992 | chr20:50931303-50931330 |
| ADNP 371 | chr20:50893093-50893120 | ADNP 993 | chr20:50931307-50931334 |
| ADNP 372 | chr20:50893104-50893131 | ADNP 994 | chr20:50931309-50931336 |
| ADNP 373 | chr20:50893114-50893141 | ADNP 995 | chr20:50931310-50931337 |
| ADNP 374 | chr20:50893118-50893145 | ADNP 996 | chr20:50931312-50931339 |
| ADNP 375 | chr20:50893132-50893159 | ADNP 997 | chr20:50931313-50931340 |
| ADNP 376 | chr20:50893136-50893163 | ADNP 998 | chr20:50931314-50931341 |
| ADNP 377 | chr20:50893141-50893168 | ADNP 999 | chr20:50931315-50931342 |
| ADNP 378 | chr20:50893144-50893171 | ADNP 1000 | chr20:50931316-50931343 |
| ADNP 379 | chr20:50893172-50893199 | ADNP 1001 | chr20:50931319-50931346 |
| ADNP 380 | chr20:50893182-50893209 | ADNP 1002 | chr20:50931320-50931347 |
| ADNP 381 | chr20:50893191-50893218 | ADNP 1003 | chr20:50931321-50931348 |
| ADNP 382 | chr20:50893192-50893219 | ADNP 1004 | chr20:50931322-50931349 |
| ADNP 383 | chr20:50893214-50893241 | ADNP 1005 | chr20:50931355-50931382 |
| ADNP 384 | chr20:50893254-50893281 | ADNP 1006 | chr20:50931356-50931383 |
| ADNP 385 | chr20:50893264-50893291 | ADNP 1007 | chr20:50931364-50931391 |
| ADNP 386 | chr20:50893265-50893292 | ADNP 1008 | chr20:50931365-50931392 |
| ADNP 387 | chr20:50893318-50893345 | ADNP 1009 | chr20:50931366-50931393 |
| ADNP 388 | chr20:50893365-50893392 | ADNP 1010 | chr20:50931387-50931414 |
| ADNP 389 | chr20:50893376-50893403 | ADNP 1011 | chr20:50931394-50931421 |
| ADNP 390 | chr20:50893378-50893405 | ADNP 1012 | chr20:50931398-50931425 |
| ADNP 391 | chr20:50893379-50893406 | ADNP 1013 | chr20:50931400-50931427 |
| ADNP 392 | chr20:50893380-50893407 | ADNP 1014 | chr20:50931401-50931428 |
| ADNP 393 | chr20:50893381-50893408 | ADNP 1015 | chr20:50931402-50931429 |
| ADNP 394 | chr20:50893384-50893411 | ADNP 1016 | chr20:50931407-50931434 |
| ADNP 395 | chr20:50893385-50893412 | ADNP 1017 | chr20:50931411-50931438 |
| ADNP 396 | chr20:50893392-50893419 | ADNP 1018 | chr20:50931412-50931439 |
| ADNP 397 | chr20:50893401-50893428 | ADNP 1019 | chr20:50931415-50931442 |
| ADNP 398 | chr20:50893405-50893432 | ADNP 1020 | chr20:50931417-50931444 |
| ADNP 399 | chr20:50893410-50893437 | ADNP 1021 | chr20:50931429-50931456 |
| ADNP 400 | chr20:50893413-50893440 | ADNP 1022 | chr20:50931447-50931474 |
| ADNP 401 | chr20:50893416-50893443 | ADNP 1023 | chr20:50931457-50931484 |
| ADNP 402 | chr20:50893434-50893461 | ADNP 1024 | chr20:50931469-50931496 |
| ADNP 403 | chr20:50893449-50893476 | ADNP 1025 | chr20:50931470-50931497 |
| ADNP 404 | chr20:50893454-50893481 | ADNP 1026 | chr20:50931471-50931498 |
| ADNP 405 | chr20:50893455-50893482 | ADNP 1027 | chr20:50931472-50931499 |
| ADNP 406 | chr20:50893459-50893486 | ADNP 1028 | chr20:50931473-50931500 |
| ADNP 407 | chr20:50893465-50893492 | ADNP 1029 | chr20:50931474-50931501 |
| ADNP 408 | chr20:50893472-50893499 | ADNP 1030 | chr20:50931475-50931502 |
| ADNP 409 | chr20:50893491-50893518 | ADNP 1031 | chr20:50931481-50931508 |
| ADNP 410 | chr20:50893500-50893527 | ADNP 1032 | chr20:50931482-50931509 |
| ADNP 411 | chr20:50893501-50893528 | ADNP 1033 | chr20:50931483-50931510 |
| ADNP 412 | chr20:50893508-50893535 | ADNP 1034 | chr20:50931484-50931511 |
| ADNP 413 | chr20:50893509-50893536 | ADNP 1035 | chr20:50931512-50931539 |
| ADNP 414 | chr20:50893522-50893549 | ADNP 1036 | chr20:50931513-50931540 |
| ADNP 415 | chr20:50893529-50893556 | ADNP 1037 | chr20:50931514-50931541 |
| ADNP 416 | chr20:50893530-50893557 | ADNP 1038 | chr20:50931515-50931542 |
| ADNP 417 | chr20:50893552-50893579 | ADNP 1039 | chr20:50931516-50931543 |
| ADNP 418 | chr20:50893559-50893586 | ADNP 1040 | chr20:50931517-50931544 |
| ADNP 419 | chr20:50893571-50893598 | ADNP 1041 | chr20:50931518-50931545 |
| ADNP 420 | chr20:50893572-50893599 | ADNP 1042 | chr20:50931519-50931546 |
| ADNP 421 | chr20:50893576-50893603 | ADNP 1043 | chr20:50931524-50931551 |
| ADNP 422 | chr20:50893577-50893604 | ADNP 1044 | chr20:50931525-50931552 |
| ADNP 423 | chr20:50893578-50893605 | ADNP 1045 | chr20:50931526-50931553 |
| ADNP 424 | chr20:50893586-50893613 | ADNP 1046 | chr20:50931527-50931554 |
| ADNP 425 | chr20:50893590-50893617 | ADNP 1047 | chr20:50931533-50931560 |
| ADNP 426 | chr20:50893596-50893623 | ADNP 1048 | chr20:50931534-50931561 |
| ADNP 427 | chr20:50893609-50893636 | ADNP 1049 | chr20:50931535-50931562 |
| ADNP 428 | chr20:50893614-50893641 | ADNP 1050 | chr20:50931547-50931574 |
| ADNP 429 | chr20:50893621-50893648 | ADNP 1051 | chr20:50931556-50931583 |
| ADNP 430 | chr20:50893656-50893683 | ADNP 1052 | chr20:50931557-50931584 |
| ADNP 431 | chr20:50893659-50893686 | ADNP 1053 | chr20:50931560-50931587 |
| ADNP 432 | chr20:50893665-50893692 | ADNP 1054 | chr20:50931561-50931588 |
| ADNP 433 | chr20:50893666-50893693 | ADNP 1055 | chr20:50931562-50931589 |
| ADNP 434 | chr20:50893673-50893700 | ADNP 1056 | chr20:50931565-50931592 |
| ADNP 435 | chr20:50893683-50893710 | ADNP 1057 | chr20:50931566-50931593 |
| ADNP 436 | chr20:50893695-50893722 | ADNP 1058 | chr20:50931572-50931599 |
| ADNP 437 | chr20:50893696-50893723 | ADNP 1059 | chr20:50931582-50931609 |
| ADNP 438 | chr20:50893701-50893728 | ADNP 1060 | chr20:50931596-50931623 |
| ADNP 439 | chr20:50893716-50893743 | ADNP 1061 | chr20:50931597-50931624 |
| ADNP 440 | chr20:50893719-50893746 | ADNP 1062 | chr20:50931605-50931632 |
| ADNP 441 | chr20:50893753-50893780 | ADNP 1063 | chr20:50931606-50931633 |
| ADNP 442 | chr20:50893759-50893786 | ADNP 1064 | chr20:50931607-50931634 |
| ADNP 443 | chr20:50893761-50893788 | ADNP 1065 | chr20:50931608-50931635 |
| ADNP 444 | chr20:50893792-50893819 | ADNP 1066 | chr20:50931611-50931638 |
| ADNP 445 | chr20:50893802-50893829 | ADNP 1067 | chr20:50931612-50931639 |
| ADNP 446 | chr20:50893804-50893831 | ADNP 1068 | chr20:50931613-50931640 |
| ADNP 447 | chr20:50893820-50893847 | ADNP 1069 | chr20:50931614-50931641 |
| ADNP 448 | chr20:50893821-50893848 | ADNP 1070 | chr20:50931620-50931647 |
| ADNP 449 | chr20:50893826-50893853 | ADNP 1071 | chr20:50931621-50931648 |
| ADNP 450 | chr20:50893834-50893861 | ADNP 1072 | chr20:50931622-50931649 |
| ADNP 451 | chr20:50893836-50893863 | ADNP 1073 | chr20:50931623-50931650 |
| ADNP 452 | chr20:50893837-50893864 | ADNP 1074 | chr20:50931624-50931651 |
| ADNP 453 | chr20:50893838-50893865 | ADNP 1075 | chr20:50931637-50931664 |
| ADNP 454 | chr20:50893851-50893878 | ADNP 1076 | chr20:50931638-50931665 |
| ADNP 455 | chr20:50893856-50893883 | ADNP 1077 | chr20:50931639-50931666 |
| ADNP 456 | chr20:50893864-50893891 | ADNP 1078 | chr20:50931643-50931670 |
| ADNP 457 | chr20:50893869-50893896 | ADNP 1079 | chr20:50931646-50931673 |
| ADNP 458 | chr20:50893870-50893897 | ADNP 1080 | chr20:50931666-50931693 |
| ADNP 459 | chr20:50893887-50893914 | ADNP 1081 | chr20:50931667-50931694 |
| ADNP 460 | chr20:50893888-50893915 | ADNP 1082 | chr20:50931670-50931697 |
| ADNP 461 | chr20:50893893-50893920 | ADNP 1083 | chr20:50931678-50931705 |
| ADNP 462 | chr20:50893898-50893925 | ADNP 1084 | chr20:50931679-50931706 |
| ADNP 463 | chr20:50893899-50893926 | ADNP 1085 | chr20:50931685-50931712 |
| ADNP 464 | chr20:50893900-50893927 | ADNP 1086 | chr20:50931686-50931713 |
| ADNP 465 | chr20:50893929-50893956 | ADNP 1087 | chr20:50931687-50931714 |
| ADNP 466 | chr20:50893930-50893957 | ADNP 1088 | chr20:50931700-50931727 |
| ADNP 467 | chr20:50893946-50893973 | ADNP 1089 | chr20:50931702-50931729 |
| ADNP 468 | chr20:50893947-50893974 | ADNP 1090 | chr20:50931703-50931730 |
| ADNP 469 | chr20:50893958-50893985 | ADNP 1091 | chr20:50931704-50931731 |
| ADNP 470 | chr20:50893963-50893990 | ADNP 1092 | chr20:50931713-50931740 |
| ADNP 471 | chr20:50893971-50893998 | ADNP 1093 | chr20:50931717-50931744 |
| ADNP 472 | chr20:50893992-50894019 | ADNP 1094 | chr20:50931720-50931747 |
| ADNP 473 | chr20:50893999-50894026 | ADNP 1095 | chr20:50931721-50931748 |
| ADNP 474 | chr20:50894005-50894032 | ADNP 1096 | chr20:50931725-50931752 |
| ADNP 475 | chr20:50894009-50894036 | ADNP 1097 | chr20:50931726-50931753 |
| ADNP 476 | chr20:50894042-50894069 | ADNP 1098 | chr20:50931732-50931759 |
| ADNP 477 | chr20:50894043-50894070 | ADNP 1099 | chr20:50931733-50931760 |
| ADNP 478 | chr20:50894058-50894085 | ADNP 1100 | chr20:50931734-50931761 |
| ADNP 479 | chr20:50894059-50894086 | ADNP 1101 | chr20:50931746-50931773 |
| ADNP 480 | chr20:50894060-50894087 | ADNP 1102 | chr20:50931749-50931776 |
| ADNP 481 | chr20:50894079-50894106 | ADNP 1103 | chr20:50931751-50931778 |
| ADNP 482 | chr20:50894092-50894119 | ADNP 1104 | chr20:50931752-50931779 |
| ADNP 483 | chr20:50894093-50894120 | ADNP 1105 | chr20:50931755-50931782 |
| ADNP 484 | chr20:50894094-50894121 | ADNP 1106 | chr20:50931762-50931789 |
| ADNP 485 | chr20:50894104-50894131 | ADNP 1107 | chr20:50931767-50931794 |
| ADNP 486 | chr20:50894112-50894139 | ADNP 1108 | chr20:50931768-50931795 |
| ADNP 487 | chr20:50894116-50894143 | ADNP 1109 | chr20:50931770-50931797 |
| ADNP 488 | chr20:50894137-50894164 | ADNP 1110 | chr20:50931795-50931822 |
| ADNP 489 | chr20:50894155-50894182 | ADNP 1111 | chr20:50931796-50931823 |
| ADNP 490 | chr20:50894156-50894183 | ADNP 1112 | chr20:50931798-50931825 |
| ADNP 491 | chr20:50894164-50894191 | ADNP 1113 | chr20:50931806-50931833 |
| ADNP 492 | chr20:50894171-50894198 | ADNP 1114 | chr20:50931807-50931834 |
| ADNP 493 | chr20:50894227-50894254 | ADNP 1115 | chr20:50931808-50931835 |
| ADNP 494 | chr20:50894234-50894261 | ADNP 1116 | chr20:50931809-50931836 |
| ADNP 495 | chr20:50894245-50894272 | ADNP 1117 | chr20:50931821-50931848 |
| ADNP 496 | chr20:50894262-50894289 | ADNP 1118 | chr20:50931822-50931849 |
| ADNP 497 | chr20:50894270-50894297 | ADNP 1119 | chr20:50931826-50931853 |
| ADNP 498 | chr20:50894281-50894308 | ADNP 1120 | chr20:50931831-50931858 |
| ADNP 499 | chr20:50894289-50894316 | ADNP 1121 | chr20:50931832-50931859 |
| ADNP 500 | chr20:50894296-50894323 | ADNP 1122 | chr20:50931837-50931864 |
| ADNP 501 | chr20:50894349-50894376 | ADNP 1123 | chr20:50931839-50931866 |
| ADNP 502 | chr20:50894350-50894377 | ADNP 1124 | chr20:50931840-50931867 |
| ADNP 503 | chr20:50894358-50894385 | ADNP 1125 | chr20:50931848-50931875 |
| ADNP 504 | chr20:50894359-50894386 | ADNP 1126 | chr20:50931850-50931877 |
| ADNP 505 | chr20:50894360-50894387 | ADNP 1127 | chr20:50931851-50931878 |
| ADNP 506 | chr20:50894369-50894396 | ADNP 1128 | chr20:50931852-50931879 |
| ADNP 507 | chr20:50894399-50894426 | ADNP 1129 | chr20:50931855-50931882 |
| ADNP 508 | chr20:50894402-50894429 | ADNP 1130 | chr20:50931856-50931883 |
| ADNP 509 | chr20:50894408-50894435 | ADNP 1131 | chr20:50931857-50931884 |
| ADNP 510 | chr20:50894424-50894451 | ADNP 1132 | chr20:50931860-50931887 |
| ADNP 511 | chr20:50894442-50894469 | ADNP 1133 | chr20:50931864-50931891 |
| ADNP 512 | chr20:50894449-50894476 | ADNP 1134 | chr20:50931865-50931892 |
| ADNP 513 | chr20:50894470-50894497 | ADNP 1135 | chr20:50931866-50931893 |
| ADNP 514 | chr20:50894504-50894531 | ADNP 1136 | chr20:50931869-50931896 |
| ADNP 515 | chr20:50894512-50894539 | ADNP 1137 | chr20:50931874-50931901 |
| ADNP 516 | chr20:50901993-50902020 | ADNP 1138 | chr20:50931875-50931902 |
| ADNP 517 | chr20:50902003-50902030 | ADNP 1139 | chr20:50931876-50931903 |
| ADNP 518 | chr20:50902009-50902036 | ADNP 1140 | chr20:50931879-50931906 |
| ADNP 519 | chr20:50902010-50902037 | ADNP 1141 | chr20:50931897-50931924 |
| ADNP 520 | chr20:50902011-50902038 | ADNP 1142 | chr20:50931909-50931936 |
| ADNP 521 | chr20:50902016-50902043 | ADNP 1143 | chr20:50931911-50931938 |
| ADNP 522 | chr20:50902040-50902067 | ADNP 1144 | chr20:50931922-50931949 |
| ADNP 523 | chr20:50902041-50902068 | ADNP 1145 | chr20:50931924-50931951 |
| ADNP 524 | chr20:50902048-50902075 | ADNP 1146 | chr20:50931925-50931952 |
| ADNP 525 | chr20:50902055-50902082 | ADNP 1147 | chr20:50931926-50931953 |
| ADNP 526 | chr20:50902058-50902085 | ADNP 1148 | chr20:50931930-50931957 |
| ADNP 527 | chr20:50902059-50902086 | ADNP 1149 | chr20:50931933-50931960 |
| ADNP 528 | chr20:50902064-50902091 | ADNP 1150 | chr20:50931936-50931963 |
| ADNP 529 | chr20:50902109-50902136 | ADNP 1151 | chr20:50931937-50931964 |
| ADNP 530 | chr20:50903912-50903939 | ADNP 1152 | chr20:50931945-50931972 |
| ADNP 531 | chr20:50903916-50903943 | ADNP 1153 | chr20:50931949-50931976 |
| ADNP 532 | chr20:50903917-50903944 | ADNP 1154 | chr20:50931960-50931987 |
| ADNP 533 | chr20:50903925-50903952 | ADNP 1155 | chr20:50931968-50931995 |
| ADNP 534 | chr20:50903926-50903953 | ADNP 1156 | chr20:50931973-50932000 |
| ADNP 535 | chr20:50903949-50903976 | ADNP 1157 | chr20:50931980-50932007 |
| ADNP 536 | chr20:50903957-50903984 | ADNP 1158 | chr20:50931981-50932008 |
| ADNP 537 | chr20:50903964-50903991 | ADNP 1159 | chr20:50931982-50932009 |
| ADNP 538 | chr20:50903968-50903995 | ADNP 1160 | chr20:50931987-50932014 |
| ADNP 539 | chr20:50903981-50904008 | ADNP 1161 | chr20:50931988-50932015 |
| ADNP 540 | chr20:50904765-50904792 | ADNP 1162 | chr20:50931989-50932016 |
| ADNP 541 | chr20:50904771-50904798 | ADNP 1163 | chr20:50931992-50932019 |
| ADNP 542 | chr20:50904772-50904799 | ADNP 1164 | chr20:50931996-50932023 |
| ADNP 543 | chr20:50904780-50904807 | ADNP 1165 | chr20:50932016-50932043 |
| ADNP 544 | chr20:50904794-50904821 | ADNP 1166 | chr20:50932033-50932060 |
| ADNP 545 | chr20:50904795-50904822 | ADNP 1167 | chr20:50932052-50932079 |
| ADNP 546 | chr20:50904818-50904845 | ADNP 1168 | chr20:50932053-50932080 |
| ADNP 547 | chr20:50904829-50904856 | ADNP 1169 | chr20:50932054-50932081 |
| ADNP 548 | chr20:50904833-50904860 | ADNP 1170 | chr20:50932055-50932082 |
| ADNP 549 | chr20:50909486-50909513 | ADNP 1171 | chr20:50932056-50932083 |
| ADNP 550 | chr20:50909499-50909526 | ADNP 1172 | chr20:50932057-50932084 |
| ADNP 551 | chr20:50909541-50909568 | ADNP 1173 | chr20:50932064-50932091 |
| ADNP 552 | chr20:50909551-50909578 | ADNP 1174 | chr20:50932070-50932097 |
| ADNP 553 | chr20:50909565-50909592 | ADNP 1175 | chr20:50932071-50932098 |
| ADNP 554 | chr20:50909601-50909628 | ADNP 1176 | chr20:50932072-50932099 |
| ADNP 555 | chr20:50909602-50909629 | ADNP 1177 | chr20:50932073-50932100 |
| ADNP 556 | chr20:50909603-50909630 | ADNP 1178 | chr20:50932074-50932101 |
| ADNP 557 | chr20:50909614-50909641 | ADNP 1179 | chr20:50932075-50932102 |
| ADNP 558 | chr20:50909623-50909650 | ADNP 1180 | chr20:50932091-50932118 |
| ADNP 559 | chr20:50909634-50909661 | ADNP 1181 | chr20:50932102-50932129 |
| ADNP 560 | chr20:50909635-50909662 | ADNP 1182 | chr20:50932120-50932147 |
| ADNP 561 | chr20:50909643-50909670 | ADNP 1183 | chr20:50932123-50932150 |
| ADNP 562 | chr20:50909647-50909674 | ADNP 1184 | chr20:50932129-50932156 |
| ADNP 563 | chr20:50909648-50909675 | ADNP 1185 | chr20:50932130-50932157 |
| ADNP 564 | chr20:50909671-50909698 | ADNP 1186 | chr20:50932138-50932165 |
| ADNP 565 | chr20:50909673-50909700 | ADNP 1187 | chr20:50932149-50932176 |
| ADNP 566 | chr20:50909681-50909708 | ADNP 1188 | chr20:50932150-50932177 |
| ADNP 567 | chr20:50909685-50909712 | ADNP 1189 | chr20:50932171-50932198 |
| ADNP 568 | chr20:50909686-50909713 | ADNP 1190 | chr20:50932172-50932199 |
| ADNP 569 | chr20:50909690-50909717 | ADNP 1191 | chr20:50932178-50932205 |
| ADNP 570 | chr20:50909693-50909720 | ADNP 1192 | chr20:50932190-50932217 |
| ADNP 571 | chr20:50909697-50909724 | ADNP 1193 | chr20:50932202-50932229 |
| ADNP 572 | chr20:50909706-50909733 | ADNP 1194 | chr20:50932203-50932230 |
| ADNP 573 | chr20:50909712-50909739 | ADNP 1195 | chr20:50932210-50932237 |
| ADNP 574 | chr20:50909744-50909771 | ADNP 1196 | chr20:50932214-50932241 |
| ADNP 575 | chr20:50918659-50918686 | ADNP 1197 | chr20:50932218-50932245 |
| ADNP 576 | chr20:50918660-50918687 | ADNP 1198 | chr20:50932221-50932248 |
| ADNP 577 | chr20:50918714-50918741 | ADNP 1199 | chr20:50932222-50932249 |
| ADNP 578 | chr20:50918715-50918742 | ADNP 1200 | chr20:50932238-50932265 |
| ADNP 579 | chr20:50918738-50918765 | ADNP 1201 | chr20:50932239-50932266 |
| ADNP 580 | chr20:50918747-50918774 | ADNP 1202 | chr20:50932240-50932267 |
| ADNP 581 | chr20:50918748-50918775 | ADNP 1203 | chr20:50932246-50932273 |
| ADNP 582 | chr20:50918752-50918779 | ADNP 1204 | chr20:50932258-50932285 |
| ADNP 583 | chr20:50918758-50918785 | ADNP 1205 | chr20:50932287-50932314 |
| ADNP 584 | chr20:50918759-50918786 | ADNP 1206 | *c*hr20:50932288-50932315 |
| ADNP 585 | chr20:50918772-50918799 | ADNP 1207 | chr20:50932289-50932316 |
| ADNP 586 | chr20:50918781-50918808 | ADNP 1208 | chr20:50932302-50932329 |
| ADNP 587 | chr20:50918787-50918814 | ADNP 1209 | chr20:50932310-50932337 |
| ADNP 588 | chr20:50918826-50918853 | ADNP 1210 | chr20:50932318-50932345 |
| ADNP 589 | chr20:50918850-50918877 | ADNP 1211 | chr20:50932319-50932346 |
| ADNP 590 | chr20:50918885-50918912 | ADNP 1212 | chr20:50932334-50932361 |
| ADNP 591 | chr20:50918898-50918925 | ADNP 1213 | chr20:50932335-50932362 |
| ADNP 592 | chr20:50918901-50918928 | ADNP 1214 | chr20:50932339-50932366 |
| ADNP 593 | chr20:50918924-50918951 | ADNP 1215 | chr20:50932357-50932384 |
| ADNP 594 | chr20:50918941-50918968 | ADNP 1216 | chr20:50932390-50932417 |
| ADNP 595 | chr20:50918945-50918972 | ADNP 1217 | chr20:50932391-50932418 |
| ADNP 596 | chr20:50918986-50919013 | ADNP 1218 | chr20:50932395-50932422 |
| ADNP 597 | chr20:50919036-50919063 | ADNP 1219 | chr20:50932409-50932436 |
| ADNP 598 | chr20:50919040-50919067 | ADNP 1220 | chr20:50932415-50932442 |
| ADNP 599 | chr20:50919041-50919068 | ADNP 1221 | chr20:50932425-50932452 |
| ADNP 600 | chr20:50919113-50919140 | ADNP 1222 | chr20:50932429-50932456 |
| ADNP 601 | chr20:50919123-50919150 | ADNP 1223 | chr20:50932452-50932479 |
| ADNP 602 | chr20:50919128-50919155 | ADNP 1224 | chr20:50932456-50932483 |
| ADNP 603 | chr20:50919144-50919171 | ADNP 1225 | chr20:50932494-50932521 |
| ADNP 604 | chr20:50919150-50919177 | ADNP 1226 | chr20:50932528-50932555 |
| ADNP 605 | chr20:50919151-50919178 | ADNP 1227 | chr20:50932533-50932560 |
| ADNP 606 | chr20:50919165-50919192 | ADNP 1228 | chr20:50932540-50932567 |
| ADNP 607 | chr20:50919181-50919208 | ADNP 1229 | chr20:50932569-50932596 |
| ADNP 608 | chr20:50919192-50919219 | ADNP 1230 | chr20:50932616-50932643 |
| ADNP 609 | chr20:50919196-50919223 | ADNP 1231 | chr20:50932629-50932656 |
| ADNP 610 | chr20:50919202-50919229 | ADNP 1232 | chr20:50932643-50932670 |
| ADNP 611 | chr20:50919205-50919232 | ADNP 1233 | chr20:50932708-50932735 |
| ADNP 612 | chr20:50919206-50919233 | ADNP 1234 | chr20:50932709-50932736 |
| ADNP 613 | chr20:50919207-50919234 | ADNP 1235 | chr20:50932762-50932789 |
| ADNP 614 | chr20:50919208-50919235 | ADNP 1236 | chr20:50932763-50932790 |
| ADNP 615 | chr20:50919209-50919236 | ADNP 1237 | chr20:50932771-50932798 |
| ADNP 616 | chr20:50919247-50919274 | ADNP 1238 | chr20:50932786-50932813 |
| ADNP 617 | chr20:50919248-50919275 | ADNP 1239 | chr20:50932793-50932820 |
| ADNP 618 | chr20:50928624-50928651 | ADNP 1240 | chr20:50932794-50932821 |
| ADNP 619 | chr20:50928625-50928652 | ADNP 1241 | chr20:50932795-50932822 |
| ADNP 620 | chr20:50928626-50928653 | ADNP 1242 | chr20:50932799-50932826 |
| ADNP 621 | chr20:50928628-50928655 | ADNP 1243 | chr20:50932800-50932827 |
| ADNP 622 | chr20:50928629-50928656 | ADNP 1244 | chr20:50932801-50932828 |

**Tabel 2H. Genomic Coordinates of Human NFKBIA in the Current Invention**

| Index | Chromosomal coordinate range | Index | Chromosomal coordinate range |
|---|---|---|---|
| NFKBIA 1 | chr14:35401553-35401580 | NFKBIA 255 | chr14:35404706-35404733 |
| NFKBIA 2 | chr14:35401565-35401592 | NFKBIA 256 | chr14:35404709-35404736 |
| NFKBIA 3 | chr14:35401568-35401595 | NFKBIA 257 | chr14:35404714-35404741 |
| NFKBIA 4 | chr14:35401571-35401598 | NFKBIA 258 | chr14:35404724-35404751 |
| NFKBIA 5 | chr14:35401572-35401599 | NFKBIA 259 | chr14:35404725-35404752 |
| NFKBIA 6 | chr14:35401585-35401612 | NFKBIA 260 | chr14:35404744-35404771 |
| NFKBIA 7 | chr14:35401591-35401618 | NFKBIA 261 | chr14:35404761-35404788 |
| NFKBIA 8 | chr14:35401609-35401636 | NFKBIA 262 | chr14:35404763-35404790 |
| NFKBIA 9 | chr14:35401632-35401659 | NFKBIA 263 | chr14:35404765-35404792 |
| NFKBIA 10 | chr14:35401633-35401660 | NFKBIA 264 | chr14:35404766-35404793 |
| NFKBIA 11 | chr14:35401634-35401661 | NFKBIA 265 | chr14:35404767-35404794 |
| NFKBIA 12 | chr14:35401635-35401662 | NFKBIA 266 | chr14:35404774-35404801 |
| NFKBIA 13 | chr14:35401652-35401679 | NFKBIA 267 | chr14:35404775-35404802 |
| NFKBIA 14 | chr14:35401658-35401685 | NFKBIA 268 | chr14:35404777-35404804 |
| NFKBIA 15 | chr14:35401662-35401689 | NFKBIA 269 | chr14:35404778-35404805 |
| NFKBIA 16 | chr14:35401663-35401690 | NFKBIA 270 | chr14:35404779-35404806 |
| NFKBIA 17 | chr14:35401664-35401691 | NFKBIA 271 | chr14:35404782-35404809 |
| NFKBIA 18 | chr14:35401683-35401710 | NFKBIA 272 | chr14:35404783-35404810 |
| NFKBIA 19 | chr14:35401699-35401726 | NFKBIA 273 | chr14:35404784-35404811 |
| NFKBIA 20 | chr14:35401728-35401755 | NFKBIA 274 | chr14:35404789-35404816 |
| NFKBIA 21 | chr14:35401732-35401759 | NFKBIA 275 | chr14:35404794-35404821 |
| NFKBIA 22 | chr14:35401746-35401773 | NFKBIA 276 | chr14:35404795-35404822 |
| NFKBIA 23 | chr14:35401750-35401777 | NFKBIA 277 | chr14:35404796-35404823 |
| NFKBIA 24 | chr14:35401751-35401778 | NFKBIA 278 | chr14:35404829-35404856 |
| NFKBIA 25 | chr14:35401756-35401783 | NFKBIA 279 | chr14:35404833-35404860 |
| NFKBIA 26 | chr14:35401763-35401790 | NFKBIA 280 | chr14:35404834-35404861 |
| NFKBIA 27 | chr14:35401764-35401791 | NFKBIA 281 | chr14:35404843-35404870 |
| NFKBIA 28 | chr14:35401765-35401792 | NFKBIA 282 | chr14:35404850-35404877 |
| NFKBIA 29 | chr14:35401820-35401847 | NFKBIA 283 | chr14:35404851-35404878 |
| NFKBIA 30 | chr14:35401821-35401848 | NFKBIA 284 | chr14:35404852-35404879 |
| NFKBIA 31 | chr14:35401822-35401849 | NFKBIA 285 | chr14:35404853-35404880 |
| NFKBIA 32 | chr14:35401828-35401855 | NFKBIA 286 | chr14:35404867-35404894 |
| NFKBIA 33 | chr14:35401829-35401856 | NFKBIA 287 | chr14:35404879-35404906 |
| NFKBIA 34 | chr14:35401857-35401884 | NFKBIA 288 | chr14:35404880-35404907 |
| NFKBIA 35 | chr14:35401858-35401885 | NFKBIA 289 | chr14:35404881-35404908 |
| NFKBIA 36 | chr14:35401862-35401889 | NFKBIA 290 | chr14:35404889-35404916 |
| NFKBIA 37 | chr14:35401863-35401890 | NFKBIA 291 | chr14:35404890-35404917 |
| NFKBIA 38 | chr14:35401866-35401893 | NFKBIA 292 | chr14:35404896-35404923 |
| NFKBIA 39 | chr14:35401878-35401905 | NFKBIA 293 | chr14:35404897-35404924 |
| NFKBIA 40 | chr14:35401886-35401913 | NFKBIA 294 | chr14:35404916-35404943 |
| NFKBIA 41 | chr14:35401917-35401944 | NFKBIA 295 | chr14:35404917-35404944 |
| NFKBIA 42 | chr14:35401918-35401945 | NFKBIA 296 | chr14:35404923-35404950 |
| NFKBIA 43 | chr14:35401988-35402015 | NFKBIA 297 | chr14:35404924-35404951 |
| NFKBIA 44 | chr14:35402002-35402029 | NFKBIA 298 | chr14:35404925-35404952 |
| NFKBIA 45 | chr14:35402003-35402030 | NFKBIA 299 | chr14:35404928-35404955 |
| NFKBIA 46 | chr14:35402004-35402031 | NFKBIA 300 | chr14:35404929-35404956 |
| NFKBIA 47 | chr14:35402029-35402056 | NFKBIA 301 | chr14:35404936-35404963 |
| NFKBIA 48 | chr14:35402030-35402057 | NFKBIA 302 | chr14:35404953-35404980 |
| NFKBIA 49 | chr14:35402032-35402059 | NFKBIA 303 | chr14:35404957-35404984 |
| NFKBIA 50 | chr14:35402035-35402062 | NFKBIA 304 | chr14:35404973-35405000 |
| NFKBIA 51 | chr14:35402045-35402072 | NFKBIA 305 | chr14:35404975-35405002 |
| NFKBIA 52 | chr14:35402046-35402073 | NFKBIA 306 | chr14:35404989-35405016 |
| NFKBIA 53 | chr14:35402047-35402074 | NFKBIA 307 | chr14:35404990-35405017 |
| NFKBIA 54 | chr14:35402372-35402399 | NFKBIA 308 | chr14:35404994-35405021 |
| NFKBIA 55 | chr14:35402393-35402420 | NFKBIA 309 | chr14:35404998-35405025 |
| NFKBIA 56 | chr14:35402399-35402426 | NFKBIA 310 | chr14:35405003-35405030 |
| NFKBIA 57 | chr14:35402411-35402438 | NFKBIA 311 | chr14:35405004-35405031 |
| NFKBIA 58 | chr14:35402432-35402459 | NFKBIA 312 | chr14:35405005-35405032 |
| NFKBIA 59 | chr14:35402441-35402468 | NFKBIA 313 | chr14:35405006-35405033 |
| NFKBIA 60 | chr14:35402445-35402472 | NFKBIA 314 | chr14:35405009-35405036 |
| NFKBIA 61 | chr14:35402447-35402474 | NFKBIA 315 | chr14:35405010-35405037 |
| NFKBIA 62 | chr14:35402463-35402490 | NFKBIA 316 | chr14:35405011-35405038 |
| NFKBIA 63 | chr14:35402484-35402511 | NFKBIA 317 | chr14:35405012-35405039 |
| NFKBIA 64 | chr14:35402485-35402512 | NFKBIA 318 | chr14:35405013-35405040 |
| NFKBIA 65 | chr14:35402491-35402518 | NFKBIA 319 | chr14:35405022-35405049 |
| NFKBIA 66 | chr14:35402492-35402519 | NFKBIA 320 | chr14:35405023-35405050 |
| NFKBIA 67 | chr14:35402493-35402520 | NFKBIA 321 | chr14:35405030-35405057 |
| NFKBIA 68 | chr14:35402496-35402523 | NFKBIA 322 | chr14:35405036-35405063 |
| NFKBIA 69 | chr14:35402503-35402530 | NFKBIA 323 | chr14:35405037-35405064 |
| NFKBIA 70 | chr14:35402508-35402535 | NFKBIA 324 | chr14:35405045-35405072 |
| NFKBIA 71 | chr14:35402511-35402538 | NFKBIA 325 | chr14:35405050-35405077 |
| NFKBIA 72 | chr14:35402515-35402542 | NFKBIA 326 | chr14:35405051-35405078 |
| NFKBIA 73 | chr14:35402516-35402543 | NFKBIA 327 | chr14:35405052-35405079 |
| NFKBIA 74 | chr14:35402524-35402551 | NFKBIA 328 | chr14:35405053-35405080 |
| NFKBIA 75 | chr14:35402525-35402552 | NFKBIA 329 | chr14:35405065-35405092 |
| NFKBIA 76 | chr14:35402526-35402553 | NFKBIA 330 | chr14:35405073-35405100 |
| NFKBIA 77 | chr14:35402529-35402556 | NFKBIA 331 | chr14:35405074-35405101 |
| NFKBIA 78 | chr14:35402533-35402560 | NFKBIA 332 | chr14:35405077-35405104 |
| NFKBIA 79 | chr14:35402551-35402578 | NFKBIA 333 | chr14:35405078-35405105 |
| NFKBIA 80 | chr14:35402552-35402579 | NFKBIA 334 | chr14:35405079-35405106 |
| NFKBIA 81 | chr14:35402568-35402595 | NFKBIA 335 | chr14:35405084-35405111 |
| NFKBIA 82 | chr14:35402579-35402606 | NFKBIA 336 | chr14:35405085-35405112 |
| NFKBIA 83 | chr14:35402580-35402607 | NFKBIA 337 | chr14:35405086-35405113 |
| NFKBIA 84 | chr14:35402581-35402608 | NFKBIA 338 | chr14:35405103-35405130 |
| NFKBIA 85 | chr14:35402585-35402612 | NFKBIA 339 | chr14:35405118-35405145 |
| NFKBIA 86 | chr14:35402595-35402622 | NFKBIA 340 | chr14:35405119-35405146 |
| NFKBIA 87 | chr14:35402603-35402630 | NFKBIA 341 | chr14:35405122-35405149 |
| NFKBIA 88 | chr14:35402607-35402634 | NFKBIA 342 | chr14:35405124-35405151 |
| NFKBIA 89 | chr14:35402613-35402640 | NFKBIA 343 | chr14:35405125-35405152 |
| NFKBIA 90 | chr14:35402614-35402641 | NFKBIA 344 | chr14:35405133-35405160 |
| NFKBIA 91 | chr14:35402622-35402649 | NFKBIA 345 | chr14:35405134-35405161 |
| NFKBIA 92 | chr14:35402624-35402651 | NFKBIA 346 | chr14:35405135-35405162 |
| NFKBIA 93 | chr14:35402632-35402659 | NFKBIA 347 | chr14:35405138-35405165 |
| NFKBIA 94 | chr14:35402633-35402660 | NFKBIA 348 | chr14:35405151-35405178 |
| NFKBIA 95 | chr14:35402646-35402673 | NFKBIA 349 | chr14:35405152-35405179 |
| NFKBIA 96 | chr14:35402650-35402677 | NFKBIA 350 | chr14:35405167-35405194 |
| NFKBIA 97 | chr14:35402663-35402690 | NFKBIA 351 | chr14:35405171-35405198 |
| NFKBIA 98 | chr14:35402766-35402793 | NFKBIA 352 | chr14:35405175-35405202 |
| NFKBIA 99 | chr14:35402769-35402796 | NFKBIA 353 | chr14:35405180-35405207 |
| NFKBIA 100 | chr14:35402770-35402797 | NFKBIA 354 | chr14:35405185-35405212 |
| NFKBIA 101 | chr14:35402790-35402817 | NFKBIA 355 | chr14:35405190-35405217 |
| NFKBIA 102 | chr14:35402791-35402818 | NFKBIA 356 | chr14:35405199-35405226 |
| NFKBIA 103 | chr14:35402795-35402822 | NFKBIA 357 | chr14:35405203-35405230 |
| NFKBIA 104 | chr14:35402800-35402827 | NFKBIA 358 | chr14:35405204-35405231 |
| NFKBIA 105 | chr14:35402804-35402831 | NFKBIA 359 | chr14:35405210-35405237 |
| NFKBIA 106 | chr14:35402809-35402836 | NFKBIA 360 | chr14:35405211-35405238 |
| NFKBIA 107 | chr14:35402811-35402838 | NFKBIA 361 | chr14:35405236-35405263 |
| NFKBIA 108 | chr14:35402817-35402844 | NFKBIA 362 | chr14:35405237-35405264 |
| NFKBIA 109 | chr14:35402818-35402845 | NFKBIA 363 | chr14:35405238-35405265 |
| NFKBIA 110 | chr14:35402826-35402853 | NFKBIA 364 | chr14:35405242-35405269 |
| NFKBIA 111 | chr14:35402831-35402858 | NFKBIA 365 | chr14:35405248-35405275 |
| NFKBIA 112 | chr14:35402836-35402863 | NFKBIA 366 | chr14:35405249-35405276 |
| NFKBIA 113 | chr14:35402848-35402875 | NFKBIA 367 | chr14:35405250-35405277 |
| NFKBIA 114 | chr14:35402859-35402886 | NFKBIA 368 | chr14:35405253-35405280 |
| NFKBIA 115 | chr14:35403134-35403161 | NFKBIA 369 | chr14:35405258-35405285 |
| NFKBIA 116 | chr14:35403145-35403172 | NFKBIA 370 | chr14:35405259-35405286 |
| NFKBIA 117 | chr14:35403149-35403176 | NFKBIA 371 | chr14:35405260-35405287 |
| NFKBIA 118 | chr14:35403154-35403181 | NFKBIA 372 | chr14:35405261-35405288 |
| NFKBIA 119 | chr14:35403157-35403184 | NFKBIA 373 | chr14:35405268-35405295 |
| NFKBIA 120 | chr14:35403162-35403189 | NFKBIA 374 | chr14:35405277-35405304 |
| NFKBIA 121 | chr14:35403163-35403190 | NFKBIA 375 | chr14:35405296-35405323 |
| NFKBIA 122 | chr14:35403164-35403191 | NFKBIA 376 | chr14:35405301-35405328 |
| NFKBIA 123 | chr14:35403165-35403192 | NFKBIA 377 | chr14:35405312-35405339 |
| NFKBIA 124 | chr14:35403167-35403194 | NFKBIA 378 | chr14:35405313-35405340 |
| NFKBIA 125 | chr14:35403173-35403200 | NFKBIA 379 | chr14:35405319-35405346 |
| NFKBIA 126 | chr14:35403184-35403211 | NFKBIA 380 | chr14:35405354-35405381 |
| NFKBIA 127 | chr14:35403197-35403224 | NFKBIA 381 | chr14:35405355-35405382 |
| NFKBIA 128 | chr14:35403202-35403229 | NFKBIA 382 | chr14:35405356-35405383 |
| NFKBIA 129 | chr14:35403215-35403242 | NFKBIA 383 | chr14:35405357-35405384 |
| NFKBIA 130 | chr14:35403216-35403243 | NFKBIA 384 | chr14:35405360-35405387 |
| NFKBIA 131 | chr14:35403218-35403245 | NFKBIA 385 | chr14:35405361-35405388 |
| NFKBIA 132 | chr14:35403223-35403250 | NFKBIA 386 | chr14:35405362-35405389 |
| NFKBIA 133 | chr14:35403225-35403252 | NFKBIA 387 | chr14:35405363-35405390 |
| NFKBIA 134 | chr14:35403229-35403256 | NFKBIA 388 | chr14:35405364-35405391 |
| NFKBIA 135 | chr14:35403230-35403257 | NFKBIA 389 | chr14:35405365-35405392 |
| NFKBIA 136 | chr14:35403231-35403258 | NFKBIA 390 | chr14:35405372-35405399 |
| NFKBIA 137 | chr14:35403232-35403259 | NFKBIA 391 | chr14:35405373-35405400 |
| NFKBIA 138 | chr14:35403233-35403260 | NFKBIA 392 | chr14:35405374-35405401 |
| NFKBIA 139 | chr14:35403234-35403261 | NFKBIA 393 | chr14:35405375-35405402 |
| NFKBIA 140 | chr14:35403253-35403280 | NFKBIA 394 | chr14:35405376-35405403 |
| NFKBIA 141 | chr14:35403261-35403288 | NFKBIA 395 | chr14:35405378-35405405 |
| NFKBIA 142 | chr14:35403266-35403293 | NFKBIA 396 | chr14:35405379-35405406 |
| NFKBIA 143 | chr14:35403296-35403323 | NFKBIA 397 | chr14:35405401-35405428 |
| NFKBIA 144 | chr14:35403300-35403327 | NFKBIA 398 | chr14:35405416-35405443 |
| NFKBIA 145 | chr14:35403302-35403329 | NFKBIA 399 | chr14:35405423-35405450 |
| NFKBIA 146 | chr14:35403303-35403330 | NFKBIA 400 | chr14:35405425-35405452 |
| NFKBIA 147 | chr14:35403304-35403331 | NFKBIA 401 | chr14:35405426-35405453 |
| NFKBIA 148 | chr14:35403308-35403335 | NFKBIA 402 | chr14:35405447-35405474 |
| NFKBIA 149 | chr14:35403312-35403339 | NFKBIA 403 | chr14:35405448-35405475 |
| NFKBIA 150 | chr14:35403325-35403352 | NFKBIA 404 | chr14:35405449-35405476 |
| NFKBIA 151 | chr14:35403330-35403357 | NFKBIA 405 | chr14:35405450-35405477 |
| NFKBIA 152 | chr14:35403345-35403372 | NFKBIA 406 | chr14:35405453-35405480 |
| NFKBIA 153 | chr14:35403667-35403694 | NFKBIA 407 | chr14:35405454-35405481 |
| NFKBIA 154 | chr14:35403668-35403695 | NFKBIA 408 | chr14:35405455-35405482 |
| NFKBIA 155 | chr14:35403672-35403699 | NFKBIA 409 | chr14:35405462-35405489 |
| NFKBIA 156 | chr14:35403673-35403700 | NFKBIA 410 | chr14:35405463-35405490 |
| NFKBIA 157 | chr14:35403681-35403708 | NFKBIA 411 | chr14:35405473-35405500 |
| NFKBIA 158 | chr14:35403689-35403716 | NFKBIA 412 | chr14:35405474-35405501 |
| NFKBIA 159 | chr14:35403690-35403717 | NFKBIA 413 | chr14:35405475-35405502 |
| NFKBIA 160 | chr14:35403694-35403721 | NFKBIA 414 | chr14:35405490-35405517 |
| NFKBIA 161 | chr14:35403699-35403726 | NFKBIA 415 | chr14:35405491-35405518 |
| NFKBIA 162 | chr14:35403714-35403741 | NFKBIA 416 | chr14:35405492-35405519 |
| NFKBIA 163 | chr14:35403717-35403744 | NFKBIA 417 | chr14:35405519-35405546 |
| NFKBIA 164 | chr14:35403722-35403749 | NFKBIA 418 | chr14:35405525-35405552 |
| NFKBIA 165 | chr14:35403730-35403757 | NFKBIA 419 | chr14:35405537-35405564 |
| NFKBIA 166 | chr14:35403731-35403758 | NFKBIA 420 | chr14:35405538-35405565 |
| NFKBIA 167 | chr14:35403737-35403764 | NFKBIA 421 | chr14:35405543-35405570 |
| NFKBIA 168 | chr14:35403750-35403777 | NFKBIA 422 | chr14:35405546-35405573 |
| NFKBIA 169 | chr14:35403752-35403779 | NFKBIA 423 | chr14:35405552-35405579 |
| NFKBIA 170 | chr14:35403757-35403784 | NFKBIA 424 | chr14:35405583-35405610 |
| NFKBIA 171 | chr14:35403764-35403791 | NFKBIA 425 | chr14:35405641-35405668 |
| NFKBIA 172 | chr14:35403768-35403795 | NFKBIA 426 | chr14:35405642-35405669 |
| NFKBIA 173 | chr14:35403777-35403804 | NFKBIA 427 | chr14:35405655-35405682 |
| NFKBIA 174 | chr14:35403778-35403805 | NFKBIA 428 | chr14:35405656-35405683 |
| NFKBIA 175 | chr14:35403779-35403806 | NFKBIA 429 | chr14:35405660-35405687 |
| NFKBIA 176 | chr14:35403785-35403812 | NFKBIA 430 | chr14:35405661-35405688 |
| NFKBIA 177 | chr14:35403787-35403814 | NFKBIA 431 | chr14:35405676-35405703 |
| NFKBIA 178 | chr14:35403788-35403815 | NFKBIA 432 | chr14:35405677-35405704 |
| NFKBIA 179 | chr14:35403798-35403825 | NFKBIA 433 | chr14:35405691-35405718 |
| NFKBIA 180 | chr14:35404391-35404418 | NFKBIA 434 | chr14:35405692-35405719 |
| NFKBIA 181 | chr14:35404392-35404419 | NFKBIA 435 | chr14:35405694-35405721 |
| NFKBIA 182 | chr14:35404393-35404420 | NFKBIA 436 | chr14:35405733-35405760 |
| NFKBIA 183 | chr14:35404397-35404424 | NFKBIA 437 | chr14:35405742-35405769 |
| NFKBIA 184 | chr14:35404398-35404425 | NFKBIA 438 | chr14:35405745-35405772 |
| NFKBIA 185 | chr14:35404399-35404426 | NFKBIA 439 | chr14:35405751-35405778 |
| NFKBIA 186 | chr14:35404404-35404431 | NFKBIA 440 | chr14:35405762-35405789 |
| NFKBIA 187 | chr14:35404406-35404433 | NFKBIA 441 | chr14:35405766-35405793 |
| NFKBIA 188 | chr14:35404407-35404434 | NFKBIA 442 | chr14:35405771-35405798 |
| NFKBIA 189 | chr14:35404410-35404437 | NFKBIA 443 | chr14:35405776-35405803 |
| NFKBIA 190 | chr14:35404422-35404449 | NFKBIA 444 | chr14:35405777-35405804 |
| NFKBIA 191 | chr14:35404423-35404450 | NFKBIA 445 | chr14:35405780-35405807 |
| NFKBIA 192 | chr14:35404424-35404451 | NFKBIA 446 | chr14:35405787-35405814 |
| NFKBIA 193 | chr14:35404425-35404452 | NFKBIA 447 | chr14:35405789-35405816 |
| NFKBIA 194 | chr14:35404428-35404455 | NFKBIA 448 | chr14:35405797-35405824 |
| NFKBIA 195 | chr14:35404440-35404467 | NFKBIA 449 | chr14:35405819-35405846 |
| NFKBIA 196 | chr14:35404447-35404474 | NFKBIA 450 | chr14:35405820-35405847 |
| NFKBIA 197 | chr14:35404452-35404479 | NFKBIA 451 | chr14:35405824-35405851 |
| NFKBIA 198 | chr14:35404455-35404482 | NFKBIA 452 | chr14:35405828-35405855 |
| NFKBIA 199 | chr14:35404459-35404486 | NFKBIA 453 | chr14:35405829-35405856 |
| NFKBIA 200 | chr14:35404460-35404487 | NFKBIA 454 | chr14:35405830-35405857 |
| NFKBIA 201 | chr14:35404462-35404489 | NFKBIA 455 | chr14:35405831-35405858 |
| NFKBIA 202 | chr14:35404470-35404497 | NFKBIA 456 | chr14:35405832-35405859 |
| NFKBIA 203 | chr14:35404473-35404500 | NFKBIA 457 | chr14:35405833-35405860 |
| NFKBIA 204 | chr14:35404494-35404521 | NFKBIA 458 | chr14:35405834-35405861 |
| NFKBIA 205 | chr14:35404503-35404530 | NFKBIA 459 | chr14:35405837-35405864 |
| NFKBIA 206 | chr14:35404509-35404536 | NFKBIA 460 | chr14:35405845-35405872 |
| NFKBIA 207 | chr14:35404511-35404538 | NFKBIA 461 | chr14:35405851-35405878 |
| NFKBIA 208 | chr14:35404519-35404546 | NFKBIA 462 | chr14:35405864-35405891 |
| NFKBIA 209 | chr14:35404524-35404551 | NFKBIA 463 | chr14:35405865-35405892 |
| NFKBIA 210 | chr14:35404531-35404558 | NFKBIA 464 | chr14:35405873-35405900 |
| NFKBIA 211 | chr14:35404534-35404561 | NFKBIA 465 | chr14:35405874-35405901 |
| NFKBIA 212 | chr14:35404542-35404569 | NFKBIA 466 | chr14:35405875-35405902 |
| NFKBIA 213 | chr14:35404547-35404574 | NFKBIA 467 | chr14:35405881-35405908 |
| NFKBIA 214 | chr14:35404566-35404593 | NFKBIA 468 | chr14:35405884-35405911 |
| NFKBIA 215 | chr14:35404570-35404597 | NFKBIA 469 | chr14:35405887-35405914 |
| NFKBIA 216 | chr14:35404571-35404598 | NFKBIA 470 | chr14:35405896-35405923 |
| NFKBIA 217 | chr14:35404572-35404599 | NFKBIA 471 | chr14:35405929-35405956 |
| NFKBIA 218 | chr14:35404573-35404600 | NFKBIA 472 | chr14:35405954-35405981 |
| NFKBIA 219 | chr14:35404578-35404605 | NFKBIA 473 | chr14:35405959-35405986 |
| NFKBIA 220 | chr14:35404583-35404610 | NFKBIA 474 | chr14:35405962-35405989 |
| NFKBIA 221 | chr14:35404588-35404615 | NFKBIA 475 | chr14:35405965-35405992 |
| NFKBIA 222 | chr14:35404589-35404616 | NFKBIA 476 | chr14:35405968-35405995 |
| NFKBIA 223 | chr14:35404594-35404621 | NFKBIA 477 | chr14:35405971-35405998 |
| NFKBIA 224 | chr14:35404595-35404622 | NFKBIA 478 | chr14:35405976-35406003 |
| NFKBIA 225 | chr14:35404596-35404623 | NFKBIA 479 | chr14:35405981-35406008 |
| NFKBIA 226 | chr14:35404597-35404624 | NFKBIA 480 | chr14:35405984-35406011 |
| NFKBIA 227 | chr14:35404601-35404628 | NFKBIA 481 | chr14:35405990-35406017 |
| NFKBIA 228 | chr14:35404602-35404629 | NFKBIA 482 | chr14:35405996-35406023 |
| NFKBIA 229 | chr14:35404604-35404631 | NFKBIA 483 | chr14:35406016-35406043 |
| NFKBIA 230 | chr14:35404605-35404632 | NFKBIA 484 | chr14:35406043-35406070 |
| NFKBIA 231 | chr14:35404611-35404638 | NFKBIA 485 | chr14:35406046-35406073 |
| NFKBIA 232 | chr14:35404612-35404639 | NFKBIA 486 | chr14:35406065-35406092 |
| NFKBIA 233 | chr14:35404617-35404644 | NFKBIA 487 | chr14:35406066-35406093 |
| NFKBIA 234 | chr14:35404619-35404646 | NFKBIA 488 | chr14:35406073-35406100 |
| NFKBIA 235 | chr14:35404624-35404651 | NFKBIA 489 | chr14:35406074-35406101 |
| NFKBIA 236 | chr14:35404632-35404659 | NFKBIA 490 | chr14:35406104-35406131 |
| NFKBIA 237 | chr14:35404635-35404662 | NFKBIA 491 | chr14:35406122-35406149 |
| NFKBIA 238 | chr14:35404636-35404663 | NFKBIA 492 | chr14:35406132-35406159 |
| NFKBIA 239 | chr14:35404647-35404674 | NFKBIA 493 | chr14:35406143-35406170 |
| NFKBIA 240 | chr14:35404648-35404675 | NFKBIA 494 | chr14:35406148-35406175 |
| NFKBIA 241 | chr14:35404656-35404683 | NFKBIA 495 | chr14:35406152-35406179 |
| NFKBIA 242 | chr14:35404657-35404684 | NFKBIA 496 | chr14:35406153-35406180 |
| NFKBIA 243 | chr14:35404663-35404690 | NFKBIA 497 | chr14:35406154-35406181 |
| NFKBIA 244 | chr14:35404664-35404691 | NFKBIA 498 | chr14:35406169-35406196 |
| NFKBIA 245 | chr14:35404685-35404712 | NFKBIA 499 | chr14:35406172-35406199 |
| NFKBIA 246 | chr14:35404686-35404713 | NFKBIA 500 | chr14:35406173-35406200 |
| NFKBIA 247 | chr14:35404687-35404714 | NFKBIA 501 | chr14:35406174-35406201 |
| NFKBIA 248 | chr14:35404688-35404715 | NFKBIA 502 | chr14:35406177-35406204 |
| NFKBIA 249 | chr14:35404694-35404721 | NFKBIA 503 | chr14:35406178-35406205 |
| NFKBIA 250 | chr14:35404697-35404724 | NFKBIA 504 | chr14:35406179-35406206 |
| NFKBIA 251 | chr14:35404698-35404725 | NFKBIA 505 | chr 14:3 5406206-3 540623 3 |
| NFKBIA 252 | chr14:35404701-35404728 | NFKBIA 506 | chr14:35406207-35406234 |
| NFKBIA 253 | chr14:35404702-35404729 | NFKBIA 507 | chr14:35406221-35406248 |
| NFKBIA 254 | chr14:35404705-35404732 | | |

**Tabel 21. Genomic Coordinates of Human PTPN6 in the Current Invention**

| Index | Chromosomal coordinate range | Index | Chromosomal coordinate range |
|---|---|---|---|
| PTPN6 1 | chr12:6945086-6945113 | PTPN6 453 | chr12:6952145-6952172 |
| PTPN6 2 | chr12:6945088-6945115 | PTPN6 454 | chr12:6952146-6952173 |
| PTPN6 3 | chr12:6945089-6945116 | PTPN6 455 | chr12:6952151-6952178 |
| PTPN6 4 | chr12:6945090-6945117 | PTPN6 456 | chr12:6952156-6952183 |
| PTPN6 5 | chr12:6945091-6945118 | PTPN6 457 | chr12:6952157-6952184 |
| PTPN6 6 | chr12:6945094-6945121 | PTPN6 458 | chr12:6952159-6952186 |
| PTPN6 7 | chr12:6945095-6945122 | PTPN6 459 | chr12:6952164-6952191 |
| PTPN6 8 | chr12:6945096-6945123 | PTPN6 460 | chr12:6952165-6952192 |
| PTPN6 9 | chr12:6945097-6945124 | PTPN6 461 | chr12:6954778-6954805 |
| PTPN6 10 | chr12:6945098-6945125 | PTPN6 462 | chr12:6954780-6954807 |
| PTPN6 11 | chr12:6945107-6945134 | PTPN6 463 | chr12:6954781-6954808 |
| PTPN6 12 | chr12:6945118-6945145 | PTPN6 464 | chr12:6954784-6954811 |
| PTPN6 13 | chr12:6945125-6945152 | PTPN6 465 | chr12:6954785-6954812 |
| PTPN6 14 | chr12:6945135-6945162 | PTPN6 466 | chr12:6954789-6954816 |
| PTPN6 15 | chr12:6945140-6945167 | PTPN6 467 | chr12:6954795-6954822 |
| PTPN6 16 | chr12:6945155-6945182 | PTPN6 468 | chr12:6954801-6954828 |
| PTPN6 17 | chr12:6945159-6945186 | PTPN6 469 | chr12:6954804-6954831 |
| PTPN6 18 | chr12:6945191-6945218 | PTPN6 470 | chr12:6954805-6954832 |
| PTPN6 19 | chr12:6945205-6945232 | PTPN6 471 | chr12:6954809-6954836 |
| PTPN6 20 | chr12:6945217-6945244 | PTPN6 472 | chr12:6954811-6954838 |
| PTPN6 21 | chr12:6945221-6945248 | PTPN6 473 | chr12:6954817-6954844 |
| PTPN6 22 | chr12:6945247-6945274 | PTPN6 474 | chr12:6954827-6954854 |
| PTPN6 23 | chr12:6945292-6945319 | PTPN6 475 | chr12:6954833-6954860 |
| PTPN6 24 | chr12:6945295-6945322 | PTPN6 476 | chr12:6954834-6954861 |
| PTPN6 25 | chr12:6945300-6945327 | PTPN6 477 | chr12:6954844-6954871 |
| PTPN6 26 | chr12:6945316-6945343 | PTPN6 478 | chr12:6954855-6954882 |
| PTPN6 27 | chr12:6945317-6945344 | PTPN6 479 | chr12:6954866-6954893 |
| PTPN6 28 | chr12:6945339-6945366 | PTPN6 480 | chr12:6954867-6954894 |
| PTPN6 29 | chr12:6945340-6945367 | PTPN6 481 | chr12:6954879-6954906 |
| PTPN6 30 | chr12:6945341-6945368 | PTPN6 482 | chr12:6954892-6954919 |
| PTPN6 31 | chr12:6945342-6945369 | PTPN6 483 | chr12:6954898-6954925 |
| PTPN6 32 | chr12:6945343-6945370 | PTPN6 484 | chr12:6954902-6954929 |
| PTPN6 33 | chr12:6945344-6945371 | PTPN6 485 | chr12:6954917-6954944 |
| PTPN6 34 | chr12:6945345-6945372 | PTPN6 486 | chr12:6954921-6954948 |
| PTPN6 35 | chr12:6945346-6945373 | PTPN6 487 | chr12:6954927-6954954 |
| PTPN6 36 | chr12:6945350-6945377 | PTPN6 488 | chr12:6954934-6954961 |
| PTPN6 37 | chr12:6945353-6945380 | PTPN6 489 | chr12:6954938-6954965 |
| PTPN6 38 | chr12:6945365-6945392 | PTPN6 490 | chr12:6954939-6954966 |
| PTPN6 39 | chr12:6945376-6945403 | PTPN6 491 | chr12:6954940-6954967 |
| PTPN6 40 | chr12:6945389-6945416 | PTPN6 492 | chr12:6954941-6954968 |
| PTPN6 41 | chr12:6945399-6945426 | PTPN6 493 | chr12:6954949-6954976 |
| PTPN6 42 | chr12:6945408-6945435 | PTPN6 494 | chr12:6954950-6954977 |
| PTPN6 43 | chr12:6945409-6945436 | PTPN6 495 | chr12:6954956-6954983 |
| PTPN6 44 | chr12:6945410-6945437 | PTPN6 496 | chr12:6954957-6954984 |
| PTPN6 45 | chr12:6945440-6945467 | PTPN6 497 | chr12:6954958-6954985 |
| PTPN6 46 | chr12:6945448-6945475 | PTPN6 498 | chr12:6954959-6954986 |
| PTPN6 47 | chr12:6945449-6945476 | PTPN6 499 | chr12:6954968-6954995 |
| PTPN6 48 | chr12:6945450-6945477 | PTPN6 500 | chr12:6954972-6954999 |
| PTPN6 49 | chr12:6945452-6945479 | PTPN6 501 | chr12:6954973-6955000 |
| PTPN6 50 | chr12:6945460-6945487 | PTPN6 502 | chr12:6954981-6955008 |
| PTPN6 51 | chr12:6945468-6945495 | PTPN6 503 | chr12:6954984-6955011 |
| PTPN6 52 | chr12:6945469-6945496 | PTPN6 504 | chr12:6954987-6955014 |
| PTPN6 53 | chr12:6945470-6945497 | PTPN6 505 | chr12:6954988-6955015 |
| PTPN6 54 | chr12:6945471-6945498 | PTPN6 506 | chr12:6954989-6955016 |
| PTPN6 55 | chr12:6945496-6945523 | PTPN6 507 | chr12:6954990-6955017 |
| PTPN6 56 | chr12:6945500-6945527 | PTPN6 508 | chr12:6955124-6955151 |
| PTPN6 57 | chr12:6945528-6945555 | PTPN6 509 | chr12:6955125-6955152 |
| PTPN6 58 | chr12:6945529-6945556 | PTPN6 510 | chr12:6955128-6955155 |
| PTPN6 59 | chr12:6945550-6945577 | PTPN6 511 | chr12:6955131-6955158 |
| PTPN6 60 | chr12:6945576-6945603 | PTPN6 512 | chr12:6955142-6955169 |
| PTPN6 61 | chr12:6945577-6945604 | PTPN6 513 | chr12:6955143-6955170 |
| PTPN6 62 | chr12:6945590-6945617 | PTPN6 514 | chr12:6955144-6955171 |
| PTPN6 63 | chr12:6945591-6945618 | PTPN6 515 | chr12:6955145-6955172 |
| PTPN6 64 | chr12:6945600-6945627 | PTPN6 516 | chr12:6955146-6955173 |
| PTPN6 65 | chr12:6945601-6945628 | PTPN6 517 | chr12:6955147-6955174 |
| PTPN6 66 | chr12:6945608-6945635 | PTPN6 518 | chr12:6955151-6955178 |
| PTPN6 67 | chr12:6945615-6945642 | PTPN6 519 | chr12:6955172-6955199 |
| PTPN6 68 | chr12:6945620-6945647 | PTPN6 520 | chr12:6955178-6955205 |
| PTPN 6 69 | chr12:6945628-6945655 | PTPN6 521 | chr12:6955181-6955208 |
| PTPN6 70 | chr12:6945636-6945663 | PTPN6 522 | chr12:6955182-6955209 |
| PTPN6 71 | chr12:6945647-6945674 | PTPN6 523 | chr12:6955192-6955219 |
| PTPN6 72 | chr12:6945650-6945677 | PTPN6 524 | chr12:6955199-6955226 |
| PTPN6 73 | chr12:6945651-6945678 | PTPN6 525 | chr12:6955200-6955227 |
| PTPN6 74 | chr12:6945652-6945679 | PTPN6 526 | chr12:6955201-6955228 |
| PTPN6 75 | chr12:6945653-6945680 | PTPN6 527 | chr12:6955208-6955235 |
| PTPN6 76 | chr12:6945666-6945693 | PTPN6 528 | chr12:6955211-6955238 |
| PTPN6 77 | chr12:6945667-6945694 | PTPN6 529 | chr12:6955218-6955245 |
| PTPN6 78 | chr12:6945671-6945698 | PTPN6 530 | chr12:6955237-6955264 |
| PTPN6 79 | chr12:6945687-6945714 | PTPN6 531 | chr12:6955239-6955266 |
| PTPN6 80 | chr12:6945696-6945723 | PTPN6 532 | chr12:6955241-6955268 |
| PTPN6 81 | chr12:6945701-6945728 | PTPN6 533 | chr12:6955246-6955273 |
| PTPN6 82 | chr12:6945704-6945731 | PTPN6 534 | chr12:6955247-6955274 |
| PTPN6 83 | chr12:6945705-6945732 | PTPN6 535 | chr12:6955248-6955275 |
| PTPN6 84 | chr12:6945706-6945733 | PTPN6 536 | chr12:6955251-6955278 |
| PTPN6 85 | chr12:6945707-6945734 | PTPN6 537 | chr12:6955252-6955279 |
| PTPN6 86 | chr12:6945710-6945737 | PTPN6 538 | chr12:6955258-6955285 |
| PTPN6 87 | chr12:6945712-6945739 | PTPN6 539 | chr12:6955361-6955388 |
| PTPN6 88 | chr12:6945718-6945745 | PTPN6 540 | chr12:6955362-6955389 |
| PTPN6 89 | chr12:6945719-6945746 | PTPN6 541 | chr12:6955363-6955390 |
| PTPN6 90 | chr12:6945733-6945760 | PTPN6 542 | chr12:6955366-6955393 |
| PTPN6 91 | chr12:6945744-6945771 | PTPN6 543 | chr12:6955367-6955394 |
| PTPN6 92 | chr12:6945751-6945778 | PTPN6 544 | chr12:6955368-6955395 |
| PTPN6 93 | chr12:6945755-6945782 | PTPN6 545 | chr12:6955372-6955399 |
| PTPN6 94 | chr12:6945760-6945787 | PTPN6 546 | chr12:6955382-6955409 |
| PTPN6 95 | chr12:6945761-6945788 | PTPN6 547 | chr12:6955396-6955423 |
| PTPN6 96 | chr12:6945767-6945794 | PTPN6 548 | chr12:6955413-6955440 |
| PTPN6 97 | chr12:6945775-6945802 | PTPN6 549 | chr12:6955414-6955441 |
| PTPN6 98 | chr12:6945788-6945815 | PTPN6 550 | chr12:6955423-6955450 |
| PTPN6 99 | chr12:6945801-6945828 | PTPN6 551 | chr12:6955435-6955462 |
| PTPN6 100 | chr12:6945810-6945837 | PTPN6 552 | chr12:6955439-6955466 |
| PTPN6 101 | chr12:6945811-6945838 | PTPN6 553 | chr12:6955445-6955472 |
| PTPN6 102 | chr12:6945813-6945840 | PTPN6 554 | chr12:6955446-6955473 |
| PTPN6 103 | chr12:6945818-6945845 | PTPN6 555 | chr12:6955447-6955474 |
| PTPN6 104 | chr12:6945824-6945851 | PTPN6 556 | chr12:6955450-6955477 |
| PTPN6 105 | chr12:6945828-6945855 | PTPN6 557 | chr12:6955457-6955484 |
| PTPN6 106 | chr12:6945832-6945859 | PTPN6 558 | chr12:6955459-6955486 |
| PTPN6 107 | chr12:6945861-6945888 | PTPN6 559 | chr12:6955466-6955493 |
| PTPN6 108 | chr12:6945862-6945889 | PTPN6 560 | chr12:6955469-6955496 |
| PTPN6 109 | chr12:6945863-6945890 | PTPN6 561 | chr12:6955470-6955497 |
| PTPN6 110 | chr12:6945866-6945893 | PTPN6 562 | chr12:6955471-6955498 |
| PTPN6 111 | chr12:6945867-6945894 | PTPN6 563 | chr12:6955476-6955503 |
| PTPN6 112 | chr12:6945873-6945900 | PTPN6 564 | chr12:6955480-6955507 |
| PTPN6 113 | chr12:6945874-6945901 | PTPN6 565 | chr12:6955481-6955508 |
| PTPN6 114 | chr12:6945876-6945903 | PTPN6 566 | chr12:6955485-6955512 |
| PTPN6 115 | chr12:6945878-6945905 | PTPN6 567 | chr12:6955643-6955670 |
| PTPN6 116 | chr12:6945881-6945908 | PTPN6 568 | chr12:6955644-6955671 |
| PTPN6 117 | chr12:6945882-6945909 | PTPN6 569 | chr12:6955645-6955672 |
| PTPN6 118 | chr12:6945883-6945910 | PTPN6 570 | chr12:6955648-6955675 |
| PTPN6 119 | chr12:6945884-6945911 | PTPN6 571 | chr12:6955649-6955676 |
| PTPN6 120 | chr12:6945890-6945917 | PTPN6 572 | chr12:6955651-6955678 |
| PTPN6 121 | chr12:6945902-6945929 | PTPN6 573 | chr12:6955675-6955702 |
| PTPN6 122 | chr12:6945912-6945939 | PTPN6 574 | chr12:6955679-6955706 |
| PTPN6 123 | chr12:6945915-6945942 | PTPN6 575 | chr12:6955680-6955707 |
| PTPN6 124 | chr12:6945918-6945945 | PTPN6 576 | chr12:6955686-6955713 |
| PTPN6 125 | chr12:6945925-6945952 | PTPN6 577 | chr12:6955692-6955719 |
| PTPN6 126 | chr12:6945926-6945953 | PTPN6 578 | chr12:6955699-6955726 |
| PTPN6 127 | chr12:6945927-6945954 | PTPN6 579 | chr12:6955700-6955727 |
| PTPN6 128 | chr12:6945930-6945957 | PTPN6 580 | chr12:6955714-6955741 |
| PTPN6 129 | chr12:6945931-6945958 | PTPN6 581 | chr12:6955734-6955761 |
| PTPN6 130 | chr12:6945934-6945961 | PTPN6 582 | chr12:6955742-6955769 |
| PTPN6 131 | chr12:6945935-6945962 | PTPN6 583 | chr12:6955752-6955779 |
| PTPN6 132 | chr12:6945942-6945969 | PTPN6 584 | chr12:6955753-6955780 |
| PTPN6 133 | chr12:6945948-6945975 | PTPN6 585 | chr12:6955754-6955781 |
| PTPN6 134 | chr12:6945963-6945990 | PTPN6 586 | chr12:6956120-6956147 |
| PTPN6 135 | chr12:6945973-6946000 | PTPN6 587 | chr12:6956123-6956150 |
| PTPN6 136 | chr12:6945982-6946009 | PTPN6 588 | chr12:6956128-6956155 |
| PTPN6 137 | chr12:6945992-6946019 | PTPN6 589 | chr12:6956131-6956158 |
| PTPN6 138 | chr12:6945998-6946025 | PTPN6 590 | chr12:6956132-6956159 |
| PTPN6 139 | chr12:6946004-6946031 | PTPN6 591 | chr12:6956137-6956164 |
| PTPN6 140 | chr12:6946027-6946054 | PTPN6 592 | chr12:6956138-6956165 |
| PTPN6 141 | chr12:6946035-6946062 | PTPN6 593 | chr12:6956141-6956168 |
| PTPN6 142 | chr12:6946036-6946063 | PTPN6 594 | chr12:6956142-6956169 |
| PTPN6 143 | chr12:6946037-6946064 | PTPN6 595 | chr12:6956146-6956173 |
| PTPN6 144 | chr12:6946038-6946065 | PTPN6 596 | chr12:6956147-6956174 |
| PTPN6 145 | chr12:6946046-6946073 | PTPN6 597 | chr12:6956152-6956179 |
| PTPN6 146 | chr12:6946047-6946074 | PTPN6 598 | chr12:6956161-6956188 |
| PTPN6 147 | chr12:6946050-6946077 | PTPN6 599 | chr12:6956163-6956190 |
| PTPN6 148 | chr12:6946051-6946078 | PTPN6 600 | chr12:6956164-6956191 |
| PTPN6 149 | chr12:6946058-6946085 | PTPN6 601 | chr12:6956185-6956212 |
| PTPN6 150 | chr12:6946059-6946086 | PTPN6 602 | chr12:6956186-6956213 |
| PTPN6 151 | chr12:6946065-6946092 | PTPN6 603 | chr12:6956187-6956214 |
| PTPN6 152 | chr12:6946070-6946097 | PTPN6 604 | chr12:6956193-6956220 |
| PTPN6 153 | chr12:6946075-6946102 | PTPN6 605 | chr12:6956195-6956222 |
| PTPN6 154 | chr12:6946076-6946103 | PTPN6 606 | chr12:6956207-6956234 |
| PTPN6 155 | chr12:6946077-6946104 | PTPN6 607 | chr12:6956208-6956235 |
| PTPN6 156 | chr12:6946078-6946105 | PTPN6 608 | chr12:6956216-6956243 |
| PTPN6 157 | chr12:6946081-6946108 | PTPN6 609 | chr12:6956217-6956244 |
| PTPN6 158 | chr12:6946082-6946109 | PTPN6 610 | chr12:69 56220-6956247 |
| PTPN6 159 | chr12:6946083-6946110 | PTPN6 611 | chr12:6956403-6956430 |
| PTPN6 160 | chr12:6946084-6946111 | PTPN6 612 | chr12:6956405-6956432 |
| PTPN6 161 | chr12:6946088-6946115 | PTPN6 613 | chr12:6956415-6956442 |
| PTPN6 162 | chr12:6946089-6946116 | PTPN6 614 | chr12:6956421-6956448 |
| PTPN6 163 | chr12:6946095-6946122 | PTPN6 615 | chr12:6956433-6956460 |
| PTPN6 164 | chr12:6946098-6946125 | PTPN6 616 | chr12:6956434-6956461 |
| PTPN6 165 | chr12:6946099-6946126 | PTPN6 617 | chr12:6956443-6956470 |
| PTPN6 166 | chr12:6946106-6946133 | PTPN6 618 | chr12:6956444-6956471 |
| PTPN6 167 | chr12:6946107-6946134 | PTPN6 619 | chr12:6956452-6956479 |
| PTPN6 168 | chr12:6946117-6946144 | PTPN6 620 | chr12:6956453-6956480 |
| PTPN6 169 | chr12:6946122-6946149 | PTPN6 621 | chr12:6956455-6956482 |
| PTPN6 170 | chr12:6946123-6946150 | PTPN6 622 | chr12:6956461-6956488 |
| PTPN6 171 | chr12:6946124-6946151 | PTPN6 623 | chr12:6956462-6956489 |
| PTPN6 172 | chr12:6946127-6946154 | PTPN6 624 | chr12:6956466-6956493 |
| PTPN6 173 | chr12:6946128-6946155 | PTPN6 625 | chr12:6956475-6956502 |
| PTPN6 174 | chr12:6946129-6946156 | PTPN6 626 | chr12:6956482-6956509 |
| PTPN6 175 | chr12:6946135-6946162 | PTPN6 627 | chr12:6956483-6956510 |
| PTPN6 176 | chr12:6946140-6946167 | PTPN6 628 | chr12:6956487-6956514 |
| PTPN6 177 | chr12:6946141-6946168 | PTPN6 629 | chr12:6956491-6956518 |
| PTPN6 178 | chr12:6946142-6946169 | PTPN6 630 | chr12:6956526-6956553 |
| PTPN6 179 | chr12:6946143-6946170 | PTPN6 631 | chr12:6956527-6956554 |
| PTPN6 180 | chr12:6946144-6946171 | PTPN6 632 | chr12:6956530-6956557 |
| PTPN6 181 | chr12:6946145-6946172 | PTPN6 633 | chr12:6956537-6956564 |
| PTPN6 182 | chr12:6946148-6946175 | PTPN6 634 | chr12:6956541-6956568 |
| PTPN6 183 | chr12:6946154-6946181 | PTPN6 635 | chr12:6956542-6956569 |
| PTPN6 184 | chr12:6946156-6946183 | PTPN6 636 | chr12:6956543-6956570 |
| PTPN6 185 | chr12:6946157-6946184 | PTPN6 637 | chr12:6956546-6956573 |
| PTPN6 186 | chr12:6946158-6946185 | PTPN6 638 | chr12:6956547-6956574 |
| PTPN6 187 | chr12:6946159-6946186 | PTPN6 639 | chr12:6956565-6956592 |
| PTPN6 188 | chr12:6946160-6946187 | PTPN6 640 | chr12:6957630-6957657 |
| PTPN6 189 | chr12:6946163-6946190 | PTPN6 641 | chr12:6957631-6957658 |
| PTPN6 190 | chr12:6946164-6946191 | PTPN6 642 | chr12:6957632-6957659 |
| PTPN6 191 | chr12:6946165-6946192 | PTPN6 643 | chr12:6957635-6957662 |
| PTPN6 192 | chr12:6946166-6946193 | PTPN6 644 | chr12:6957636-6957663 |
| PTPN6 193 | chr12:6946182-6946209 | PTPN6 645 | chr12:6957640-6957667 |
| PTPN6 194 | chr12:6946183-6946210 | PTPN6 646 | chr12:6957641-6957668 |
| PTPN6 195 | chr12:6946186-6946213 | PTPN6 647 | chr12:6957644-6957671 |
| PTPN6 196 | chr12:6946187-6946214 | PTPN6 648 | chr12:6957645-6957672 |
| PTPN6 197 | chr12:6946190-6946217 | PTPN6 649 | chr12:6957647-6957674 |
| PTPN6 198 | chr12:6946191-6946218 | PTPN6 650 | chr12:6957650-6957677 |
| PTPN6 199 | chr12:6946195-6946222 | PTPN6 651 | chr12:6957654-6957681 |
| PTPN6 200 | chr12:6946196-6946223 | PTPN6 652 | chr12:6957657-6957684 |
| PTPN6 201 | chr12:6946201-6946228 | PTPN6 653 | chr12:6957658-6957685 |
| PTPN6 202 | chr12:6946202-6946229 | PTPN6 654 | chr12:6957665-6957692 |
| PTPN6 203 | chr12:6946203-6946230 | PTPN6 655 | chr12:6957666-6957693 |
| PTPN6 204 | chr12:6946204-6946231 | PTPN6 656 | chr12:6957675-6957702 |
| PTPN6 205 | chr12:6946205-6946232 | PTPN6 657 | chr12:6957676-6957703 |
| PTPN6 206 | chr12:6946219-6946246 | PTPN6 658 | chr12:6957677-6957704 |
| PTPN6 207 | chr12:6946221-6946248 | PTPN6 659 | chr12:6957700-6957727 |
| PTPN6 208 | chr12:6946227-6946254 | PTPN6 660 | chr12:6957701-6957728 |
| PTPN6 209 | chr12:6946228-6946255 | PTPN6 661 | chr12:6957702-6957729 |
| PTPN6 210 | chr12:6946234-6946261 | PTPN6 662 | chr12:6957705-6957732 |
| PTPN6 211 | chr12:6946236-6946263 | PTPN6 663 | chr12:6957706-6957733 |
| PTPN6 212 | chr12:6946237-6946264 | PTPN6 664 | chr12:6957718-6957745 |
| PTPN6 213 | chr12:6946249-6946276 | PTPN6 665 | chr12:6957741-6957768 |
| PTPN6 214 | chr12:6946257-6946284 | PTPN6 666 | chr12:6957742-6957769 |
| PTPN6 215 | chr12:6946270-6946297 | PTPN6 667 | chr12:6957753-6957780 |
| PTPN6 216 | chr12:6946275-6946302 | PTPN6 668 | chr12:6957755-6957782 |
| PTPN6 217 | chr12:6946277-6946304 | PTPN6 669 | chr12:6957760-6957787 |
| PTPN6 218 | chr12:6946278-6946305 | PTPN6 670 | chr12:6957766-6957793 |
| PTPN6 219 | chr12:6946288-6946315 | PTPN6 671 | chr12:6957772-6957799 |
| PTPN6 220 | chr12:6946291-6946318 | PTPN6 672 | chr12:6957773-6957800 |
| PTPN6 221 | chr12:6946306-6946333 | PTPN6 673 | chr12:6957774-6957801 |
| PTPN6 222 | chr12:6946307-6946334 | PTPN6 674 | chr12:6957892-6957919 |
| PTPN6 223 | chr12:6946308-6946335 | PTPN6 675 | chr12:6957893-6957920 |
| PTPN6 224 | chr12:6946309-6946336 | PTPN6 676 | chr12:6957900-6957927 |
| PTPN6 225 | chr12:6946319-6946346 | PTPN6 677 | chr12:6957901-6957928 |
| PTPN6 226 | chr12:6946324-6946351 | PTPN6 678 | chr12:6957906-6957933 |
| PTPN6 227 | chr12:6946330-6946357 | PTPN6 679 | chr12:6957907-6957934 |
| PTPN6 228 | chr12:6946342-6946369 | PTPN6 680 | chr12:6957908-6957935 |
| PTPN6 229 | chr12:6946353-6946380 | PTPN6 681 | chr12:6957915-6957942 |
| PTPN6 230 | chr12:6946354-6946381 | PTPN6 682 | chr12:6957924-6957951 |
| PTPN6 231 | chr12:6946355-6946382 | PTPN6 683 | chr12:6957936-6957963 |
| PTPN6 232 | chr12:6946359-6946386 | PTPN6 684 | chr12:6957947-6957974 |
| PTPN6 233 | chr12:6946360-6946387 | PTPN6 685 | chr12:6957948-6957975 |
| PTPN6 234 | chr12:6946366-6946393 | PTPN6 686 | chr12:6957949-6957976 |
| PTPN6 235 | chr12:6946367-6946394 | PTPN6 687 | chr12:6957954-6957981 |
| PTPN6 236 | chr12:6946368-6946395 | PTPN6 688 | chr12:6957964-6957991 |
| PTPN6 237 | chr12:6946371-6946398 | PTPN6 689 | chr12:6957965-6957992 |
| PTPN6 238 | chr12:6946380-6946407 | PTPN6 690 | chr12:6957966-6957993 |
| PTPN6 239 | chr12:6946383-6946410 | PTPN6 691 | chr12:6957967-6957994 |
| PTPN6 240 | chr12:6946391-6946418 | PTPN6 692 | chr12:6957968-6957995 |
| PTPN6 241 | chr12:6946398-6946425 | PTPN6 693 | chr12:6957969-6957996 |
| PTPN6 242 | chr12:6946399-6946426 | PTPN6 694 | chr12:6957978-6958005 |
| PTPN6 243 | chr12:6946400-6946427 | PTPN6 695 | chr12:6957979-6958006 |
| PTPN6 244 | chr12:6946401-6946428 | PTPN6 696 | chr12:6957980-6958007 |
| PTPN6 245 | chr12:6946403-6946430 | PTPN6 697 | chr12:6957985-6958012 |
| PTPN6 246 | chr12:6946405-6946432 | PTPN6 698 | chr12:6957988-6958015 |
| PTPN6 247 | chr12:6946408-6946435 | PTPN6 699 | chr12:6957999-6958026 |
| PTPN6 248 | chr12:6946413-6946440 | PTPN6 700 | chr12:6958002-6958029 |
| PTPN6 249 | chr12:6946414-6946441 | PTPN6 701 | chr12:6958006-6958033 |
| PTPN6 250 | chr12:6946416-6946443 | PTPN6 702 | chr12:6958008-6958035 |
| PTPN6 251 | chr12:6946421-6946448 | PTPN6 703 | chr12:6958014-6958041 |
| PTPN6 252 | chr12:6946422-6946449 | PTPN6 704 | chr12:6958023-6958050 |
| PTPN6 253 | chr12:6946423-6946450 | PTPN6 705 | chr12:6958025-6958052 |
| PTPN6 254 | chr12:6946426-6946453 | PTPN6 706 | chr12:6958026-6958053 |
| PTPN6 255 | chr12:6946427-6946454 | PTPN6 707 | chr12:6958042-6958069 |
| PTPN6 256 | chr12:6946436-6946463 | PTPN6 708 | chr12:6958047-6958074 |
| PTPN6 257 | chr12:6946437-6946464 | PTPN6 709 | chr12:6958052-6958079 |
| PTPN6 258 | chr12:6946443-6946470 | PTPN6 710 | chr12:6958054-6958081 |
| PTPN6 259 | chr12:6946448-6946475 | PTPN6 711 | chr12:6958055-6958082 |
| PTPN6 260 | chr12:6946449-6946476 | PTPN6 712 | chr12:6958068-6958095 |
| PTPN6 261 | chr12:6946450-6946477 | PTPN6 713 | chr12:6959905-6959932 |
| PTPN6 262 | chr12:6946455-6946482 | PTPN6 714 | chr12:6959910-6959937 |
| PTPN6 263 | chr12:6946457-6946484 | PTPN6 715 | chr12:6959911-6959938 |
| PTPN6 264 | chr12:6946458-6946485 | PTPN6 716 | chr12:6959912-6959939 |
| PTPN6 265 | chr12:6946465-6946492 | PTPN6 717 | chr12:6959913-6959940 |
| PTPN6 266 | chr12:6946466-6946493 | PTPN6 718 | chr12:6959920-6959947 |
| PTPN6 267 | chr12:6946467-6946494 | PTPN6 719 | chr12:6959921-6959948 |
| PTPN6 268 | chr12:6946474-6946501 | PTPN6 720 | chr12:6959922-6959949 |
| PTPN6 269 | chr12:6946475-6946502 | PTPN6 721 | chr12:6959923-6959950 |
| PTPN6 270 | chr12:6946478-6946505 | PTPN6 722 | chr12:6959929-6959956 |
| PTPN6 271 | chr12:6946484-6946511 | PTPN6 723 | chr12:6959939-6959966 |
| PTPN6 272 | chr12:6946485-6946512 | PTPN6 724 | chr12:6959949-6959976 |
| PTPN6 273 | chr12:6946493-6946520 | PTPN6 725 | chr12:6959950-6959977 |
| PTPN6 274 | chr12:6946513-6946540 | PTPN6 726 | chr12:6959967-6959994 |
| PTPN6 275 | chr12:6946515-6946542 | PTPN6 727 | chr12:6959968-6959995 |
| PTPN6 276 | chr12:6946517-6946544 | PTPN6 728 | chr12:6959973-6960000 |
| PTPN6 277 | chr12:6946518-6946545 | PTPN6 729 | chr12:6959974-6960001 |
| PTPN6 278 | chr12:6946519-6946546 | PTPN6 730 | chr12:6959975-6960002 |
| PTPN6 279 | chr12:6946522-6946549 | PTPN6 731 | chr12:6959982-6960009 |
| PTPN6 280 | chr12:6946523-6946550 | PTPN6 732 | chr12:6959983-6960010 |
| PTPN6 281 | chr12:6946524-6946551 | PTPN6 733 | chr12:6959984-6960011 |
| PTPN6 282 | chr12:6946527-6946554 | PTPN6 734 | chr12:6959985-6960012 |
| PTPN6 283 | chr12:6946531-6946558 | PTPN6 735 | chr12:6959986-6960013 |
| PTPN6 284 | chr12:6946532-6946559 | PTPN6 736 | chr12:6959989-6960016 |
| PTPN6 285 | chr12:6946538-6946565 | PTPN6 737 | chr12:6959990-6960017 |
| PTPN6 286 | chr12:6946554-6946581 | PTPN6 738 | chr12:6959991-6960018 |
| PTPN6 287 | chr12:6946559-6946586 | PTPN6 739 | chr12:6959992-6960019 |
| PTPN6 288 | chr12:6946563-6946590 | PTPN6 740 | chr12:6959993-6960020 |
| PTPN6 289 | chr12:6946565-6946592 | PTPN6 741 | chr12:6960061-6960088 |
| PTPN6 290 | chr12:6946566-6946593 | PTPN6 742 | chr12:6960066-6960093 |
| PTPN6 291 | chr12:6946567-6946594 | PTPN6 743 | chr12:6960076-6960103 |
| PTPN6 292 | chr12:6946574-6946601 | PTPN6 744 | chr12:6960077-6960104 |
| PTPN6 293 | chr12:6946575-6946602 | PTPN6 745 | chr12:6960078-6960105 |
| PTPN6 294 | chr12:6946577-6946604 | PTPN6 746 | chr12:6960079-6960106 |
| PTPN6 295 | chr12:6946582-6946609 | PTPN6 747 | chr12:6960080-6960107 |
| PTPN6 296 | chr12:6946583-6946610 | PTPN6 748 | chr12:6960083-6960110 |
| PTPN6 297 | chr12:6946598-6946625 | PTPN6 749 | chr12:6960084-6960111 |
| PTPN6 298 | chr12:6946613-6946640 | PTPN6 750 | chr12:6960089-6960116 |
| PTPN6 299 | chr12:6946618-6946645 | PTPN6 751 | chr12:6960102-6960129 |
| PTPN6 300 | chr12:6946619-6946646 | PTPN6 752 | chr12:6960107-6960134 |
| PTPN6 301 | chr12:6946631-6946658 | PTPN6 753 | chr12:6960108-6960135 |
| PTPN6 302 | chr12:6946636-6946663 | PTPN6 754 | chr12:6960110-6960137 |
| PTPN6 303 | chr12:6946644-6946671 | PTPN6 755 | chr12:6960118-6960145 |
| PTPN6 304 | chr12:6946647-6946674 | PTPN6 756 | chr12:6960120-6960147 |
| PTPN6 305 | chr12:6946664-6946691 | PTPN6 757 | chr12:6960121-6960148 |
| PTPN6 306 | chr12:6946665-6946692 | PTPN6 758 | chr12:6960122-6960149 |
| PTPN6 307 | chr12:6946677-6946704 | PTPN6 759 | chr12:6960126-6960153 |
| PTPN6 308 | chr12:6946678-6946705 | PTPN6 760 | chr12:6960135-6960162 |
| PTPN6 309 | chr12:6946679-6946706 | PTPN6 761 | chr12:6960138-6960165 |
| PTPN6 310 | chr12:6946682-6946709 | PTPN6 762 | chr12:6960162-6960189 |
| PTPN6 311 | chr12:6946683-6946710 | PTPN6 763 | chr12:6960190-6960217 |
| PTPN6 312 | chr12:6946684-6946711 | PTPN6 764 | chr12:6960196-6960223 |
| PTPN6 313 | chr12:6946685-6946712 | PTPN6 765 | chr12:6960197-6960224 |
| PTPN6 314 | chr12:6946692-6946719 | PTPN6 766 | chr12:6960201-6960228 |
| PTPN6 315 | chr12:6946693-6946720 | PTPN6 767 | chr12:6960204-6960231 |
| PTPN6 316 | chr12:6946699-6946726 | PTPN6 768 | chr12:6960211-6960238 |
| PTPN6 317 | chr12:6946708-6946735 | PTPN6 769 | chr12:6960213-6960240 |
| PTPN6 318 | chr12:6946709-6946736 | PTPN6 770 | chr12:6960228-6960255 |
| PTPN6 319 | chr12:6946710-6946737 | PTPN6 771 | chr12:6960229-6960256 |
| PTPN6 320 | chr12:6946711-6946738 | PTPN6 772 | chr12:6960233-6960260 |
| PTPN6 321 | chr12:6946712-6946739 | PTPN6 773 | chr12:6960234-6960261 |
| PTPN6 322 | chr12:6946713-6946740 | PTPN6 774 | chr12:6960235-6960262 |
| PTPN6 323 | chr12:6946714-6946741 | PTPN6 775 | chr12:6960236-6960263 |
| PTPN6 324 | chr12:6946715-6946742 | PTPN6 776 | chr12:6960237-6960264 |
| PTPN6 325 | chr12:6946723-6946750 | PTPN6 777 | chr12:6960238-6960265 |
| PTPN6 326 | chr12:6946724-6946751 | PTPN6 778 | chr12:6960239-6960266 |
| PTPN6 327 | chr12:6946733-6946760 | PTPN6 779 | chr12:6960318-6960345 |
| PTPN6 328 | chr12:6946734-6946761 | PTPN6 780 | chr12:6960322-6960349 |
| PTPN6 329 | chr12:6946735-6946762 | PTPN6 781 | chr12:6960323-6960350 |
| PTPN6 330 | chr12:6951330-6951357 | PTPN6 782 | chr12:6960326-6960353 |
| PTPN6 331 | chr12:6951331-6951358 | PTPN6 783 | chr12:6960327-6960354 |
| PTPN6 332 | chr12:6951332-6951359 | PTPN6 784 | chr12:6960330-6960357 |
| PTPN6 333 | chr12:6951333-6951360 | PTPN6 785 | chr12:6960331-6960358 |
| PTPN6 334 | chr12:6951335-6951362 | PTPN6 786 | chr12:6960332-6960359 |
| PTPN6 335 | chr12:6951336-6951363 | PTPN6 787 | chr12:6960337-6960364 |
| PTPN6 336 | chr12:6951338-6951365 | PTPN6 788 | chr12:6960338-6960365 |
| PTPN6 337 | chr12:6951340-6951367 | PTPN6 789 | chr12:6960345-6960372 |
| PTPN6 338 | chr12:6951347-6951374 | PTPN6 790 | chr12:6960346-6960373 |
| PTPN6 339 | chr12:6951348-6951375 | PTPN6 791 | chr12:6960355-6960382 |
| PTPN6 340 | chr12:6951349-6951376 | PTPN6 792 | chr12:6960381-6960408 |
| PTPN6 341 | chr12:6951353-6951380 | PTPN6 793 | chr12:6960386-6960413 |
| PTPN6 342 | chr12:6951354-6951381 | PTPN6 794 | chr12:6960387-6960414 |
| PTPN6 343 | chr12:6951362-6951389 | PTPN6 795 | chr12:6960388-6960415 |
| PTPN6 344 | chr12:6951363-6951390 | PTPN6 796 | chr12:6960389-6960416 |
| PTPN6 345 | chr12:6951364-6951391 | PTPN6 797 | chr12:6960393-6960420 |
| PTPN6 346 | chr12:6951365-6951392 | PTPN6 798 | chr12:6960405-6960432 |
| PTPN6 347 | chr12:6951368-6951395 | PTPN6 799 | chr12:6960406-6960433 |
| PTPN6 348 | chr12:6951369-6951396 | PTPN6 800 | chr12:6960411-6960438 |
| PTPN6 349 | chr12:6951370-6951397 | PTPN6 801 | chr12:6960416-6960443 |
| PTPN6 350 | chr12:6951371-6951398 | PTPN6 802 | chr12:6960417-6960444 |
| PTPN6 351 | chr12:6951373-6951400 | PTPN6 803 | chr12:6960420-6960447 |
| PTPN6 352 | chr12:6951376-6951403 | PTPN6 804 | chr12:6960421-6960448 |
| PTPN6 353 | chr12:6951381-6951408 | PTPN6 805 | chr12:6960426-6960453 |
| PTPN6 354 | chr12:6951383-6951410 | PTPN6 806 | chr12:6960432-6960459 |
| PTPN6 355 | chr12:6951386-6951413 | PTPN6 807 | chr12:6960636-6960663 |
| PTPN6 356 | chr12:6951387-6951414 | PTPN6 808 | chr12:6960643-6960670 |
| PTPN6 357 | chr12:6951388-6951415 | PTPN6 809 | chr12:6960649-6960676 |
| PTPN6 358 | chr12:6951402-6951429 | PTPN6 810 | chr12:6960656-6960683 |
| PTPN6 359 | chr12:6951404-6951431 | PTPN6 811 | chr12:6960657-6960684 |
| PTPN6 360 | chr12:6951405-6951432 | PTPN6 812 | chr12:6960664-6960691 |
| PTPN6 361 | chr12:6951436-6951463 | PTPN6 813 | chr12:6960684-6960711 |
| PTPN6 362 | chr12:6951437-6951464 | PTPN6 814 | chr12:6960685-6960712 |
| PTPN6 363 | chr12:6951450-6951477 | PTPN6 815 | chr12:6960686-6960713 |
| PTPN6 364 | chr12:6951454-6951481 | PTPN6 816 | chr12:6960689-6960716 |
| PTPN6 365 | chr12:6951455-6951482 | PTPN6 817 | chr12:6960696-6960723 |
| PTPN6 366 | chr12:6951469-6951496 | PTPN6 818 | chr12:6960697-6960724 |
| PTPN6 367 | chr12:6951472-6951499 | PTPN6 819 | chr12:6960698-6960725 |
| PTPN6 368 | chr12:6951474-6951501 | PTPN6 820 | chr12:6960700-6960727 |
| PTPN6 369 | chr12:6951479-6951506 | PTPN6 821 | chr12:6960712-6960739 |
| PTPN6 370 | chr12:6951480-6951507 | PTPN6 822 | chr12:6960713-6960740 |
| PTPN6 371 | chr12:6951485-6951512 | PTPN6 823 | chr12:6960717-6960744 |
| PTPN6 372 | chr12:6951486-6951513 | PTPN6 824 | chr12:6960730-6960757 |
| PTPN6 373 | chr12:6951487-6951514 | PTPN6 825 | chr12:6960736-6960763 |
| PTPN6 374 | chr12:6951488-6951515 | PTPN6 826 | chr12:6960742-6960769 |
| PTPN6 375 | chr12:6951489-6951516 | PTPN6 827 | chr12:6960743-6960770 |
| PTPN6 376 | chr12:6951493-6951520 | PTPN6 828 | chr12:6960744-6960771 |
| PTPN6 377 | chr12:6951494-6951521 | PTPN6 829 | chr12:6960745-6960772 |
| PTPN6 378 | chr12:6951498-6951525 | PTPN6 830 | chr12:6960757-6960784 |
| PTPN6 379 | chr12:6951499-6951526 | PTPN6 831 | chr12:6960762-6960789 |
| PTPN6 380 | chr12:6951500-6951527 | PTPN6 832 | chr12:6960768-6960795 |
| PTPN6 381 | chr12:6951505-6951532 | PTPN6 833 | chr12:6960772-6960799 |
| PTPN6 382 | chr12:6951506-6951533 | PTPN6 834 | chr12:6960775-6960802 |
| PTPN6 383 | chr12:6951507-6951534 | PTPN6 835 | chr12:6960785-6960812 |
| PTPN6 384 | chr12:6951508-6951535 | PTPN6 836 | chr12:6960786-6960813 |
| PTPN6 385 | chr12:6951514-6951541 | PTPN6 837 | chr12:6960787-6960814 |
| PTPN6 386 | chr12:6951582-6951609 | PTPN6 838 | chr12:6960788-6960815 |
| PTPN6 387 | chr12:6951585-6951612 | PTPN6 839 | chr12:6960789-6960816 |
| PTPN6 388 | chr12:6951590-6951617 | PTPN6 840 | chr12:6960795-6960822 |
| PTPN6 389 | chr12:6951591-6951618 | PTPN6 841 | chr12:6960796-6960823 |
| PTPN6 390 | chr12:6951595-6951622 | PTPN6 842 | chr12:6960824-6960851 |
| PTPN6 391 | chr12:6951601-6951628 | PTPN6 843 | chr12:6960825-6960852 |
| PTPN6 392 | chr12:6951604-6951631 | PTPN6 844 | chr12:6960828-6960855 |
| PTPN6 393 | chr12:6951605-6951632 | PTPN6 845 | chr12:6960830-6960857 |
| PTPN6 394 | chr12:6951606-6951633 | PTPN6 846 | chr12:6960848-6960875 |
| PTPN6 395 | chr12:6951610-6951637 | PTPN6 847 | chr12:6960861-6960888 |
| PTPN6 396 | chr12:6951618-6951645 | PTPN6 848 | chr12:6960873-6960900 |
| PTPN6 397 | chr12:6951624-6951651 | PTPN6 849 | chr12:6960874-6960901 |
| PTPN6 398 | chr12:6951631-6951658 | PTPN6 850 | chr12:6960887-6960914 |
| PTPN6 399 | chr12:6951632-6951659 | PTPN6 851 | chr12:6960897-6960924 |
| PTPN6 400 | chr12:6951638-6951665 | PTPN6 852 | chr12:6960904-6960931 |
| PTPN6 401 | chr12:6951647-6951674 | PTPN6 853 | chr12:6960905-6960932 |
| PTPN6 402 | chr12:6951648-6951675 | PTPN6 854 | chr12:6960910-6960937 |
| PTPN6 403 | chr12:6951658-6951685 | PTPN6 855 | chr12:6960913-6960940 |
| PTPN6 404 | chr12:6951660-6951687 | PTPN6 856 | chr12:6960919-6960946 |
| PTPN6 405 | chr12:6951663-6951690 | PTPN6 857 | chr12:6960931-6960958 |
| PTPN6 406 | chr12:6951669-6951696 | PTPN6 858 | chr12:6960937-6960964 |
| PTPN6 407 | chr12:6951681-6951708 | PTPN6 859 | chr12:6960938-6960965 |
| PTPN6 408 | chr12:6951682-6951709 | PTPN6 860 | chr12:6960941-6960968 |
| PTPN6 409 | chr12:6951683-6951710 | PTPN6 861 | chr12:6961103-6961130 |
| PTPN6 410 | chr12:6951691-6951718 | PTPN6 862 | chr12:6961104-6961131 |
| PTPN6 411 | chr12:6951692-6951719 | PTPN6 863 | chr12:6961111-6961138 |
| PTPN6 412 | chr12:6951705-6951732 | PTPN6 864 | chr12:6961112-6961139 |
| PTPN6 413 | chr12:6951709-6951736 | PTPN6 865 | chr12:6961118-6961145 |
| PTPN6 414 | chr12:6951712-6951739 | PTPN6 866 | chr12:6961126-6961153 |
| PTPN6 415 | chr12:6951713-6951740 | PTPN6 867 | chr12:6961132-6961159 |
| PTPN6 416 | chr12:6951725-6951752 | PTPN6 868 | chr12:6961133-6961160 |
| PTPN6 417 | chr12:6951729-6951756 | PTPN6 869 | chr12:6961134-6961161 |
| PTPN6 418 | chr12:6951730-6951757 | PTPN6 870 | chr12:6961138-6961165 |
| PTPN6 419 | chr12:6951956-6951983 | PTPN6 871 | chr12:6961139-6961166 |
| PTPN6 420 | chr12:6951957-6951984 | PTPN6 872 | chr12:6961158-6961185 |
| PTPN6 421 | chr 12:695 1960-6951987 | PTPN6 873 | chr12:6961174-6961201 |
| PTPN6 422 | chr12:6951961-6951988 | PTPN6 874 | chr12:6961180-6961207 |
| PTPN6 423 | chr12:6951962-6951989 | PTPN6 875 | chr12:6961187-6961214 |
| PTPN6 424 | chr12:6951963-6951990 | PTPN6 876 | chr12:6961190-6961217 |
| PTPN6 425 | chr12:6951965-6951992 | PTPN6 877 | chr12:6961191-6961218 |
| PTPN6 426 | chr12:6951968-6951995 | PTPN6 878 | chr12:6961192-6961219 |
| PTPN6 427 | chr12:6951969-6951996 | PTPN6 879 | chr12:6961221-6961248 |
| PTPN6 428 | chr12:6951975-6952002 | PTPN6 880 | chr12:6961222-6961249 |
| PTPN6 429 | chr12:6951976-6952003 | PTPN6 881 | chr12:6961223-6961250 |
| PTPN6 430 | chr12:6951983-6952010 | PTPN6 882 | chr12:6961224-6961251 |
| PTPN6 431 | chr12:6951997-6952024 | PTPN6 883 | chr12:6961225-6961252 |
| PTPN6 432 | chr12:6951998-6952025 | PTPN6 884 | chr12:6961226-6961253 |
| PTPN6 433 | chr12:6951999-6952026 | PTPN6 885 | chr12:6961229-6961256 |
| PTPN6 434 | chr12:6952000-6952027 | PTPN6 886 | chr12:6961234-6961261 |
| PTPN6 435 | chr12:6952001-6952028 | PTPN6 887 | chr12:6961235-6961262 |
| PTPN6 436 | chr12:6952013-6952040 | PTPN6 888 | chr12:6961240-6961267 |
| PTPN6 437 | chr12:6952018-6952045 | PTPN6 889 | chr12:6961241-6961268 |
| PTPN6 438 | chr12:6952021-6952048 | PTPN6 890 | chr12:6961245-6961272 |
| PTPN6 439 | chr12:6952022-6952049 | PTPN6 891 | chr12:6961246-6961273 |
| PTPN6 440 | chr12:6952023-6952050 | PTPN6 892 | chr12:6961252-6961279 |
| PTPN6 441 | chr12:6952037-6952064 | PTPN6 893 | chr12:6961253-6961280 |
| PTPN6 442 | chr12:6952050-6952077 | PTPN6 894 | chr12:6961257-6961284 |
| PTPN6 443 | chr12:6952053-6952080 | PTPN6 895 | chr12:6961262-6961289 |
| PTPN6 444 | chr12:6952074-6952101 | PTPN6 896 | chr12:6961263-6961290 |
| PTPN6 445 | chr12:6952075-6952102 | PTPN6 897 | chr12:6961264-6961291 |
| PTPN6 446 | chr12:6952086-6952113 | PTPN6 898 | chr12:6961274-6961301 |
| PTPN6 447 | chr12:6952096-6952123 | PTPN6 899 | chr12:6961278-6961305 |
| PTPN6 448 | chr12:6952106-6952133 | PTPN6 900 | chr12:6961279-6961306 |
| PTPN6 449 | chr12:6952115-6952142 | PTPN6 901 | chr12:6961280-6961307 |
| PTPN6 450 | chr12:6952126-6952153 | PTPN6 902 | chr12:6961285-6961312 |
| PTPN6 451 | chr12:6952133-6952160 | PTPN6 903 | chr12:6961301-6961328 |
| PTPN6 452 | chr12:6952136-6952163 | PTPN6 904 | chr12:6961302-6961329 |

**Tabel 2J. Genomic Coordinates of Human TNIP1 in the Current Invention**

| Index | Chromosomal coordinate range | Index | Chromosomal coordinate range |
|---|---|---|---|
| TNIP1 1 | chr5:151029936-151029963 | TNIP1 1166 | chr5:151071010-151071037 |
| TNIP1 2 | chr5:151029972-151029999 | TNIP1 1167 | chr5:151071011-151071038 |
| TNIP1 3 | chr5:151029973-151030000 | TNIP1 1168 | chr5:151071012-151071039 |
| TNIP1 4 | chr5:151029977-151030004 | TNIP1 1169 | chr5:151071020-151071047 |
| TNIP1 5 | chr5:151029995-151030022 | TNIP1 1170 | chr5:151071064-151071091 |
| TNIP1 6 | chr5:151029996-151030023 | TNIP1 1171 | chr5: 151071087-151071114 |
| TNIP1 7 | chr5:151030003-151030030 | TNIP1 1172 | chr5:151071088-151071115 |
| TNIP1 8 | chr5:151030009-151030036 | TNIP1 1173 | chr5:151071110-151071137 |
| TNIP1 9 | chr5:151030012-151030039 | TNIPI 1174 | chr5:151071116-151071143 |
| TNIP1 10 | chr5:151030013-151030040 | TNIP1 1175 | chr5:151071120-151071147 |
| TNIP1 11 | chr5:151030025-151030052 | TNIPl 1176 | chr5:151071121-151071148 |
| TNIP1 12 | chr5:151030026-151030053 | TNIP1 1177 | chr5:151071133-151071160 |
| TNIP1 13 | chr5:151030027-151030054 | TNIP1 1178 | chr5:151071139-151071166 |
| TNIP1 14 | chr5:151030033-151030060 | TNIPl 1179 | chr5:151071141-151071168 |
| TNIP1 15 | chr5:151030034-151030061 | TNIP1 1180 | chr5:151071153-151071180 |
| TNIP1 16 | chr5:151030040-151030067 | TNIP1 1181 | chr5: 151071158-151071185 |
| TNIP1 17 | chr5:151030043-151030070 | TNIP1 1182 | chr5:151071193-151071220 |
| TNIP1 18 | chr5:151030047-151030074 | TNIP1 1183 | chr5:151071205-151071232 |
| TNIP1 19 | chr5:151030048-151030075 | TNIPI 1184 | chr5:151071222-151071249 |
| TNIP1 20 | chr5:151030053-151030080 | TNIP1 1185 | chr5:151071227-151071254 |
| TNIP1 21 | chr5:151030056-151030083 | TNIP1 1186 | chr5:151071228-151071255 |
| TNIP1 22 | chr5:151030063-151030090 | TNIP1 1187 | chr5:151071236-151071263 |
| TNIP1 23 | chr5:151030067-151030094 | TNIP1 1188 | chr5:151071241-151071268 |
| TNIP1 24 | chr5:151030068-151030095 | TNIP1 1189 | chr5:151071252-151071279 |
| TNIP1 25 | chr5:151030076-151030103 | TNIP1 1190 | chr5:151071253-151071280 |
| TNIP1 26 | chr5:151030077-151030104 | TNIP1 1191 | chr5:151071265-151071292 |
| TNIP1 27 | chr5:151030080-151030107 | TNIP1 1192 | chr5:151071268-151071295 |
| TNIP1 28 | chr5:151030086-151030113 | TNIP1 1193 | chr5:151071272-151071299 |
| TNIP1 29 | chr5:151030090-151030117 | TNIPI 1194 | chr5:151071306-151071333 |
| TNIP1 30 | chr5:151030095-151030122 | TNIP1 1195 | chr5:151071307-151071334 |
| TNIP1 31 | chr5:151030097-151030124 | TNIP1 1196 | chr5:151071308-151071335 |
| TNIP1 32 | chr5:151030098-151030125 | TNIP1 1197 | chr5:151071322-151071349 |
| TNIP1 33 | chr5:151030099-151030126 | TNIP1 1198 | chr5:151071323-151071350 |
| TNIP1 34 | chr5:151030115-151030142 | TNIP1 1199 | chr5:151071335-151071362 |
| TNIP1 35 | chr5:151030123-151030150 | TNIP1 1200 | chr5:151071338-151071365 |
| TNIP1 36 | chr5:151030126-151030153 | TNIP1 1201 | chr5:151071344-151071371 |
| TNIP1 37 | chr5:151030129-151030156 | TNIP1 1202 | chr5:151071345-151071372 |
| TNIP1 38 | chr5:151030130-151030157 | TNIP1 1203 | chr5:151071368-151071395 |
| TNIP1 39 | chr5:151030139-151030166 | TNIP1 1204 | chr5:151071385-151071412 |
| TNIP1 40 | chr5:151030142-151030169 | TNIP1 1205 | chr5:151071386-151071413 |
| TNIP1 41 | chr5:151030145-151030172 | TNIP1 1206 | chr5:151071389-151071416 |
| TNIP1 42 | chr5:151030150-151030177 | TNIP1 1207 | chr5:151071390-151071417 |
| TNIP1 43 | chr5:151030151-151030178 | TNIP1 1208 | chr5:151071396-151071423 |
| TNIP1 44 | chr5:151030179-151030206 | TNIP1 1209 | chr5:151071405-151071432 |
| TNIP1 45 | chr5:151030180-151030207 | TNIP1 1210 | chr5:151071415-151071442 |
| TNIP1 46 | chr5:151030181-151030208 | TNIP1 1211 | chr5:151071416-151071443 |
| TNIP1 47 | chr5:151030187-151030214 | TNIP1 1212 | chr5:151071417-151071444 |
| TNIP1 48 | chr5:151030190-151030217 | TNIP1 1213 | chr5:151071418-151071445 |
| TNIP1 49 | chr5:151030196-151030223 | TNIP1 1214 | chr5:151071419-151071446 |
| TNIP1 50 | chr5:151030225-151030252 | TNIP1 1215 | chr5:151071427-151071454 |
| TNIP1 51 | chr5:151030226-151030253 | TNIP1 1216 | chr5:151071428-151071455 |
| TNIP1 52 | chr5:151030227-151030254 | TNIP1 1217 | chr5:151071441-151071468 |
| TNIP1 53 | chr5:151030228-151030255 | TNIP1 1218 | chr5:151071466-151071493 |
| TNIP1 54 | chr5:151030232-151030259 | TNIP1 1219 | chr5:151071469-151071496 |
| TNIP1 55 | chr5:151030233-151030260 | TNIP1 1220 | chr5:151071470-151071497 |
| TNIP1 56 | chr5:151030234-151030261 | TNIP1 1221 | chr5:151071471-151071498 |
| TNIP1 57 | chr5:151030235-151030262 | TNIP1 1222 | chr5:151071475-151071502 |
| TNIP1 58 | chr5:151030243-151030270 | TNIP1 1223 | chr5:151071516-151071543 |
| TNIP1 59 | chr5:151030244-151030271 | TNIP1 1224 | chr5:151071525-151071552 |
| TNIP1 60 | chr5:151030245-151030272 | TNIP1 1225 | chr5:151071533-151071560 |
| TNIP1 61 | chr5:151030249-151030276 | TNIP1 1226 | chr5:151071546-151071573 |
| TNIP1 62 | chr5:151030278-151030305 | TNIP1 1227 | chr5:151071547-151071574 |
| TNIP1 63 | chr5:151030296-151030323 | TNIP1 1228 | chr5:151071551-151071578 |
| TNIP1 64 | chr5:151030302-151030329 | TNIP1 1229 | chr5:151071552-151071579 |
| TNIP1 65 | chr5:151030312-151030339 | TNIP1 1230 | chr5:151071553-151071580 |
| TNIP1 66 | chr5:151030313-151030340 | TNIP1 1231 | chr5:151071556-151071583 |
| TNIP1 67 | chr5:151030330-151030357 | TNIP1 1232 | chr5:151071559-151071586 |
| TNIP1 68 | chr5:151030331-151030358 | TNIP1 1233 | chr5:151071564-151071591 |
| TNIP1 69 | chr5:151030334-151030361 | TNIP1 1234 | chr5:151071565-151071592 |
| TNIP1 70 | chr5:151030341-151030368 | TNIP1 1235 | chr5:151071575-151071602 |
| TNIP1 71 | chr5:151030343-151030370 | TNIP1 1236 | chr5:151071576-151071603 |
| TNIP1 72 | chr5:151030347-151030374 | TNIP1 1237 | chr5:151071588-151071615 |
| TNIP1 73 | chr5:151030348-151030375 | TNIP1 1238 | chr5:151071589-151071616 |
| TNIP1 74 | chr5:151030349-151030376 | TNIP1 1239 | chr5:151071594-151071621 |
| TNIP1 75 | chr5:151030350-151030377 | TNIP1 1240 | chr5:151071595-151071622 |
| TNIP1 76 | chr5:151030354-151030381 | TNIP1 1241 | chr5:151071598-151071625 |
| TNIP1 77 | chr5:151030356-151030383 | TNIP1 1242 | chr5:151071603-151071630 |
| TNIP1 78 | chr5:151030374-151030401 | TNIP1 1243 | chr5:151071606-151071633 |
| TNIP1 79 | chr5:151030387-151030414 | TNIP1 1244 | chr5:151071607-151071634 |
| TNIP1 80 | chr5:151030388-151030415 | TNIP1 1245 | chr5:151071617-151071644 |
| TNIP1 81 | chr5:151030390-151030417 | TNIP1 1246 | chr5:151071618-151071645 |
| TNIP1 82 | chr5:151030391-151030418 | TNIP1 1247 | chr5:151071632-151071659 |
| TNIP1 83 | chr5:151030403-151030430 | TNIP1 1248 | chr5:151071633-151071660 |
| TNIP1 84 | chr5:151030407-151030434 | TNIP1 1249 | chr5:151071634-151071661 |
| TNIP1 85 | chr5:151030410-151030437 | TNIP1 1250 | chr5:151071637-151071664 |
| TNIP1 86 | chr5:151030411-151030438 | TNIP1 1251 | chr5:151071638-151071665 |
| TNIP1 87 | chr5:151030421-151030448 | TNIP1 1252 | chr5:151071639-151071666 |
| TNIP1 88 | chr5:151030427-151030454 | TNIP1 1253 | chr5:151071656-151071683 |
| TNIP1 89 | chr5:151030445-151030472 | TNIPI 1254 | chr5:151071657-151071684 |
| TNIP1 90 | chr5:151030449-151030476 | TNIP1 1255 | chr5:151071660-151071687 |
| TNIP1 91 | chr5:151030450-151030477 | TNIP1 1256 | chr5:151071670-151071697 |
| TNIP1 92 | chr5:151030454-151030481 | TNIP1 1257 | chr5:151071676-151071703 |
| TNIP1 93 | chr5:151030455-151030482 | TNIP1 1258 | chr5:151071677-151071704 |
| TNIP1 94 | chr5:151030462-151030489 | TNIP1 1259 | chr5:151071683-151071710 |
| TNIP1 95 | chr5:151030489-151030516 | TNIP1 1260 | chr5:151071689-151071716 |
| TNIP1 96 | chr5:151030490-151030517 | TNIP1 1261 | chr5:151071692-151071719 |
| TNIP1 97 | chr5:151030499-151030526 | TNIP1 1262 | chr5:151071693-151071720 |
| TNIP1 98 | chr5:151030500-151030527 | TNIP1 1263 | chr5:151071694-151071721 |
| TNIP1 99 | chr5:151030501-151030528 | TNIP1 1264 | chr5:151071695-151071722 |
| TNIP1 100 | chr5:151030502-151030529 | TNIP1 1265 | chr5:151071697-151071724 |
| TNIP1 101 | chr5:151030505-151030532 | TNIP1 1266 | chr5:151071701-151071728 |
| TNIP1 102 | chr5:151030521-151030548 | TNIP1 1267 | chr5:151071712-151071739 |
| TNIP1 103 | chr5:151030535-151030562 | TNIP1 1268 | chr5:151071723-151071750 |
| TNIP1 104 | chr5:151030538-151030565 | TNIP1 1269 | chr5:151071724-151071751 |
| TNIP1 105 | chr5:151030539-151030566 | TNIP1 1270 | chr5:151071728-151071755 |
| TNIP1 106 | chr5:151030540-151030567 | TNIP1 1271 | chr5:151071741-151071768 |
| TNIP1 107 | chr5:151030545-151030572 | TNIP1 1272 | chr5:151071762-151071789 |
| TNIP1 108 | chr5:151030557-151030584 | TNIP1 1273 | chr5:151071763-151071790 |
| TNIP1 109 | chr5:151030585-151030612 | TNIP1 1274 | chr5:151071769-151071796 |
| TNIP1 110 | chr5:151030588-151030615 | TNIP1 1275 | chr5:151071776-151071803 |
| TNIP1 111 | chr5:151030589-151030616 | TNIP1 1276 | chr5:151071778-151071805 |
| TNIP1 112 | chr5:151030594-151030621 | TNIP1 1277 | chr5:151071779-151071806 |
| TNIP1 113 | chr5:151030598-151030625 | TNIP1 1278 | chr5:151071780-151071807 |
| TNIP1 114 | chr5:151030606-151030633 | TNIP1 1279 | chr5:151071781-151071808 |
| TNIP1 115 | chr5:151030611-151030638 | TNIP1 1280 | chr5:151071784-151071811 |
| TNIP1 116 | chr5:151030622-151030649 | TNIP1 1281 | chr5:151071785-151071812 |
| TNIP1 117 | chr5:151030626-151030653 | TNIP1 1282 | chr5:151071793-151071820 |
| TNIP1 118 | chr5:151030627-151030654 | TNIP1 1283 | chr5:151071794-151071821 |
| TNIP1 119 | chr5:151030644-151030671 | TNIP1 1284 | chr5:151071806-151071833 |
| TNIP1 120 | chr5:151030660-151030687 | TNIP1 1285 | chr5:151071807-151071834 |
| TNIP1 121 | chr5:151030663-151030690 | TNIP1 1286 | chr5:151071808-151071835 |
| TNIP1 122 | chr5:151030683-151030710 | TNIP1 1287 | chr5:151071817-151071844 |
| TNIP1 123 | chr5:151030693-151030720 | TNIP1 1288 | chr5:151071825-151071852 |
| TNIP1 124 | chr5:151030694-151030721 | TNIP1 1289 | chr5:151071828-151071855 |
| TNIP1 125 | chr5:151030704-151030731 | TNIP1 1290 | chr5:151071834-151071861 |
| TNIP1 126 | chr5:151030715-151030742 | TNIP1 1291 | chr5:151071835-151071862 |
| TNIP1 127 | chr5:151030722-151030749 | TNIP1 1292 | chr5:151071847-151071874 |
| TNIP1 128 | chr5:151030723-151030750 | TNIP1 1293 | chr5:151071850-151071877 |
| TNIP1 129 | chr5:151030724-151030751 | TNIP1 1294 | chr5:151071851-151071878 |
| TNIP1 130 | chr5:151030743-151030770 | TNIP1 1295 | chr5:151071861-151071888 |
| TNIP1 131 | chr5:151032262-151032289 | TNIP1 1296 | chr5:151071863-151071890 |
| TNIP1 132 | chr5:151032263-151032290 | TNIP1 1297 | chr5:151071867-151071894 |
| TNIP1 133 | chr5:151032272-151032299 | TNIP1 1298 | chr5:151071868-151071895 |
| TNIP1 134 | chr5:151032277-151032304 | TNIP1 1299 | chr5:151071888-151071915 |
| TNIP1 135 | chr5:151032283-151032310 | TNIP1 1300 | chr5:151071895-151071922 |
| TNIP1 136 | chr5:151032284-151032311 | TNIP1 1301 | chr5:151071896-151071923 |
| TNIP1 137 | chr5:151032286-151032313 | TNIP1 1302 | chr5:151071902-151071929 |
| TNIP1 138 | chr5:151032287-151032314 | TNIP1 1303 | chr5:151071911-151071938 |
| TNIP1 139 | chr5:151032288-151032315 | TNIP1 1304 | chr5:151071928-151071955 |
| TNIP1 140 | chr5:151032300-151032327 | TNIP1 1305 | chr5:151071934-151071961 |
| TNIP1 141 | chr5:151032301-151032328 | TNIP1 1306 | chr5:151071935-151071962 |
| TNIP1 142 | chr5:151032314-151032341 | TNIP1 1307 | chr5:151071944-151071971 |
| TNIP1 143 | chr5:151032317-151032344 | TNIP1 1308 | chr5:151071951-151071978 |
| TNIP1 144 | chr5:151032334-151032361 | TNIP1 1309 | chr5:151071963-151071990 |
| TNIP1 145 | chr5:151032335-151032362 | TNIP1 1310 | chr5:151071983-151072010 |
| TNIP1 146 | chr5:151032346-151032373 | TNIP1 1311 | chr5:151071985-151072012 |
| TNIP1 147 | chr5:151032350-151032377 | TNIP1 1312 | chr5:151071999-151072026 |
| TNIP1 148 | chr5:151032351-151032378 | TNIP1 1313 | chr5:151072013-151072040 |
| TNIP1 149 | chr5:151032352-151032379 | TNIP1 1314 | chr5:151072023-151072050 |
| TNIP1 150 | chr5:151032355-151032382 | TNIP1 1315 | chr5:151072032-151072059 |
| TNIP1 151 | chr5:151032356-151032383 | TNIP1 1316 | chr5:151072037-151072064 |
| TNIP1 152 | chr5:151032369-151032396 | TNIP1 1317 | chr5:151072043-151072070 |
| TNIP1 153 | chr5:151032371-151032398 | TNIP1 1318 | chr5:151072044-151072071 |
| TNIP1 154 | chr5:151032380-151032407 | TNIP1 1319 | chr5:151072091-151072118 |
| TNIP1 155 | chr5:151033587-151033614 | TNIP1 1320 | chr5:151072098-151072125 |
| TNIP1 156 | chr5:151033593-151033620 | TNIP1 1321 | chr5:151072100-151072127 |
| TNIP1 157 | chr5:151033603-151033630 | TNIP1 1322 | chr5:151072143-151072170 |
| TNIP1 158 | chr5:151033604-151033631 | TNIP1 1323 | chr5:151072160-151072187 |
| TNIP1 159 | chr5:151033605-151033632 | TNIP1 1324 | chr5:151072171-151072198 |
| TNIP1 160 | chr5:151033607-151033634 | TNIP1 1325 | chr5:151072172-151072199 |
| TNIP1 161 | chr5:151033610-151033637 | TNIP1 1326 | chr5:151072173-151072200 |
| TNIP1 162 | chr5:151033611-151033638 | TNIP1 1327 | chr5:151072176-151072203 |
| TNIP1 163 | chr5:151033612-151033639 | TNIP1 1328 | chr5:151072178-151072205 |
| TNIP1 164 | chr5:151033613-151033640 | TNIP1 1329 | chr5:151072179-151072206 |
| TNIP1 165 | chr5:151033616-151033643 | TNIP1 1330 | chr5:151072183-151072210 |
| TNIP1 166 | chr5:151033617-151033644 | TNIP1 1331 | chr5:151072194-151072221 |
| TNIP1 167 | chr5:151033627-151033654 | TNIP1 1332 | chr5:151072197-151072224 |
| TNIP1 168 | chr5:151033633-151033660 | TNIP1 1333 | chr5:151072200-151072227 |
| TNIP1 169 | chr5:151033634-151033661 | TNIP1 1334 | chr5:151072201-151072228 |
| TNIP1 170 | chr5:151033635-151033662 | TNIP1 1335 | chr5:151072232-151072259 |
| TNIP1 171 | chr5:151033636-151033663 | TNIP1 1336 | chr5:151072237-151072264 |
| TNIP1 172 | chr5:151033637-151033664 | TNIP1 1337 | chr5:151072245-151072272 |
| TNIP1 173 | chr5:151033640-151033667 | TNIP1 1338 | chr5:151072253-151072280 |
| TNIP1 174 | chr5:151033641-151033668 | TNIP1 1339 | chr5:151072263-151072290 |
| TNIP1 175 | chr5:151033642-151033669 | TNIP1 1340 | chr5:151072264-151072291 |
| TNIP1 176 | chr5:151033643-151033670 | TNIP1 1341 | chr5:151072265-151072292 |
| TNIP1 177 | chr5:151033644-151033671 | TNIP1 1342 | chr5:151072272-151072299 |
| TNIP1 178 | chr5:151033649-151033676 | TNIP1 1343 | chr5:151072277-151072304 |
| TNIP1 179 | chr5:151033650-151033677 | TNIP1 1344 | chr5:151072278-151072305 |
| TNIP1 180 | chr5:151033655-151033682 | TNIP1 1345 | chr5:151072279-151072306 |
| TNIP1 181 | chr5:151033656-151033683 | TNIP1 1346 | chr5:151072283-151072310 |
| TNIP1 182 | chr5:151033657-151033684 | TNIP1 1347 | chr5:151072289-151072316 |
| TNIP1 183 | chr5:151033677-151033704 | TNIP1 1348 | chr5:151072292-151072319 |
| TNIP1 184 | chr5:151033682-151033709 | TNIP1 1349 | chr5:151072312-151072339 |
| TNIP1 185 | chr5:151033686-151033713 | TNIP1 1350 | chr5:151072324-151072351 |
| TNIP1 186 | chr5:151033690-151033717 | TNIP1 1351 | chr5:151072325-151072352 |
| TNIP1 187 | chr5:151033691-151033718 | TNIP1 1352 | chr5:151072327-151072354 |
| TNIP1 188 | chr5:151033694-151033721 | TNIP1 1353 | chr5:151072328-151072355 |
| TNIP1 189 | chr5:151033699-151033726 | TNIP1 1354 | chr5:151072329-151072356 |
| TNIP1 190 | chr5:151033700-151033727 | TNIP1 1355 | chr5:151072337-151072364 |
| TNIP1 191 | chr5:151033703-151033730 | TNIP1 1356 | chr5:151072339-151072366 |
| TNIP1 192 | chr5:151033704-151033731 | TNIP1 1357 | chr5:151072343-151072370 |
| TNIP1 193 | chr5:151033708-151033735 | TNIP1 1358 | chr5:151072347-151072374 |
| TNIP1 194 | chr5:151033712-151033739 | TNIP1 1359 | chr5:151072348-151072375 |
| TNIP1 195 | chr5:151033714-151033741 | TNIP1 1360 | chr5:151072358-151072385 |
| TNIP1 196 | chr5:151033715-151033742 | TNIP1 1361 | chr5:151072391-151072418 |
| TNIP1 197 | chr5:151033716-151033743 | TNIP1 1362 | chr5:151072392-151072419 |
| TNIP1 198 | chr5:151033720-151033747 | TNIP1 1363 | chr5:151072395-151072422 |
| TNIP1 199 | chr5:151033739-151033766 | TNIP1 1364 | chr5:151072400-151072427 |
| TNIP1 200 | chr5:151033740-151033767 | TNIP1 1365 | chr5:151072401-151072428 |
| TNIP1 201 | chr5:151033743-151033770 | TNIP1 1366 | chr5:151072405-151072432 |
| TNIP1 202 | chr5:151033744-151033771 | TNIP1 1367 | chr5:151072406-151072433 |
| TNIP1 203 | chr5:151033745-151033772 | TNIP1 1368 | chr5:151072411-151072438 |
| TNIP1 204 | chr5:151033748-151033775 | TNIP1 1369 | chr5:151072412-151072439 |
| TNIP1 205 | chr5:151033749-151033776 | TNIP1 1370 | chr5:151072422-151072449 |
| TNIP1 206 | chr5:151033750-151033777 | TNIP1 1371 | chr5:151072423-151072450 |
| TNIP1 207 | chr5:151033755-151033782 | TNIP1 1372 | chr5:151072432-151072459 |
| TNIP1 208 | chr5:151033771-151033798 | TNIP1 1373 | chr5:151072440-151072467 |
| TNIP1 209 | chr5:151033775-151033802 | TNIP1 1374 | chr5:151072454-151072481 |
| TNIP1 210 | chr5:151033784-151033811 | TNIP1 1375 | chr5:151072464-151072491 |
| TNIP1 211 | chr5:151033792-151033819 | TNIP1 1376 | chr5:151072465-151072492 |
| TNIP1 212 | chr5:151033795-151033822 | TNIP1 1377 | chr5:151072466-151072493 |
| TNIP1 213 | chr5:151033799-151033826 | TNIP1 1378 | chr5:151072471-151072498 |
| TNIP1 214 | chr5:151034982-151035009 | TNIP1 1379 | chr5:151072474-151072501 |
| TNIP1 215 | chr5:151034985-151035012 | TNIP1 1380 | chr5:151072475-151072502 |
| TNIP1 216 | chr5:151034986-151035013 | TNIP1 1381 | chr5:151072476-151072503 |
| TNIP1 217 | chr5:151034993-151035020 | TNIP1 1382 | chr5:151072479-151072506 |
| TNIP1 218 | chr5:151034996-151035023 | TNIP1 1383 | chr5:151072487-151072514 |
| TNIP1 219 | chr5:151035001-151035028 | TNIP1 1384 | chr5:151072490-151072517 |
| TNIP1 220 | chr5:151035002-151035029 | TNIP1 1385 | chr5:151072497-151072524 |
| TNIP1 221 | chr5:151035003-151035030 | TNIP1 1386 | chr5:151072515-151072542 |
| TNIP1 222 | chr5:151035011-151035038 | TNIP1 1387 | chr5:151072523-151072550 |
| TNIP1 223 | chr5:151035040-151035067 | TNIP1 1388 | chr5:151072524-151072551 |
| TNIP1 224 | chr5:151035046-151035073 | TNIP1 1389 | chr5:151072535-151072562 |
| TNIP1 225 | chr5:151035054-151035081 | TNIP1 1390 | chr5:151072536-151072563 |
| TNIP1 226 | chr5:151035055-151035082 | TNIP1 1391 | chr5:151072543-151072570 |
| TNIP1 227 | chr5:151035058-151035085 | TNIP1 1392 | chr5:151072544-151072571 |
| TNIP1 228 | chr5:151035059-151035086 | TNIP1 1393 | chr5:151072550-151072577 |
| TNIP1 229 | chr5:151035556-151035583 | TNIP1 1394 | chr5:151072552-151072579 |
| TNIP1 230 | chr5:151035557-151035584 | TNIP1 1395 | chr5:151072553-151072580 |
| TNIP1 231 | chr5:151035558-151035585 | TNIP1 1396 | chr5:151072554-151072581 |
| TNIP1 232 | chr5:151035559-151035586 | TNIP1 1397 | chr5:151072564-151072591 |
| TNIP1 233 | chr5:151035561-151035588 | TNIP1 1398 | chr5:151072565-151072592 |
| TNIP1 234 | chr5:151035568-151035595 | TNIP1 1399 | chr5:151072566-151072593 |
| TNIP1 235 | chr5:151035570-151035597 | TNIP1 1400 | chr5:151072587-151072614 |
| TNIP1 236 | chr5:151035571-151035598 | TNIP1 1401 | chr5:151072588-151072615 |
| TNIP1 237 | chr5:151035579-151035606 | TNIP1 1402 | chr5:151072590-151072617 |
| TNIP1 238 | chr5:151035580-151035607 | TNIP1 1403 | chr5:151072598-151072625 |
| TNIP1 239 | chr5:151035581-151035608 | TNIP1 1404 | chr5:151072606-151072633 |
| TNIP1 240 | chr5:151035602-151035629 | TNIP1 1405 | chr5:151072610-151072637 |
| TNIP1 241 | chr5:151035608-151035635 | TNIP1 1406 | chr5:151072611-151072638 |
| TNIP1 242 | chr5:151035620-151035647 | TNIP1 1407 | chr5:151072628-151072655 |
| TNIP1 243 | chr5:151035641-151035668 | TNIP1 1408 | chr5:151072632-151072659 |
| TNIP1 244 | chr5:151035647-151035674 | TNIP1 1409 | chr5:151072633-151072660 |
| TNIP1 245 | chr5:151035657-151035684 | TNIP1 1410 | chr5:151072635-151072662 |
| TNIP1 246 | chr5:151035677-151035704 | TNIP1 1411 | chr5:151072651-151072678 |
| TNIP1 247 | chr5:151035678-151035705 | TNIP1 1412 | chr5:151072665-151072692 |
| TNIP1 248 | chr5:151035684-151035711 | TNIP1 1413 | chr5:151072666-151072693 |
| TNIP1 249 | chr5:151035689-151035716 | TNIP1 1414 | chr5:151072667-151072694 |
| TNIP1 250 | chr5:151035692-151035719 | TNIP1 1415 | chr5:151072668-151072695 |
| TNIP1 251 | chr5:151035693-151035720 | TNIP1 1416 | chr5:151072669-151072696 |
| TNIP1 252 | chr5:151035696-151035723 | TNIP1 1417 | chr5:151072670-151072697 |
| TNIP1 253 | chr5:151035697-151035724 | TNIP1 1418 | chr5:151072673-151072700 |
| TNIP1 254 | chr5:151035707-151035734 | TNIP1 1419 | chr5:151072678-151072705 |
| TNIP1 255 | chr5:151036765-151036792 | TNIP1 1420 | chr5:151072679-151072706 |
| TNIP1 256 | chr5:151036773-151036800 | TNIP1 1421 | chr5:151072680-151072707 |
| TNIP1 257 | chr5:151036776-151036803 | TNIP1 1422 | chr5:151072681-151072708 |
| TNIP1 258 | chr5:151036777-151036804 | TNIP1 1423 | chr5:151072684-151072711 |
| TNIP1 259 | chr5:151036784-151036811 | TNIP1 1424 | chr5:151072688-151072715 |
| TNIP1 260 | chr5:151036789-151036816 | TNIP1 1425 | chr5:151072689-151072716 |
| TNIP1 261 | chr5:151036811-151036838 | TNIP1 1426 | chr5:151072695-151072722 |
| TNIP1 262 | chr5:151036814-151036841 | TNIP1 1427 | chr5:151072696-151072723 |
| TNIP1 263 | chr5:151036815-151036842 | TNIP1 1428 | chr5:151072697-151072724 |
| TNIP1 264 | chr5:151036819-151036846 | TNIP1 1429 | chr5:151072700-151072727 |
| TNIP1 265 | chr5:151036825-151036852 | TNIP1 1430 | chr5:151072715-151072742 |
| TNIP1 266 | chr5:151036831-151036858 | TNIP1 1431 | chr5:151072720-151072747 |
| TNIP1 267 | chr5:151036832-151036859 | TNIP1 1432 | chr5:151072729-151072756 |
| TNIP1 268 | chr5:151036843-151036870 | TNIP1 1433 | chr5:151072730-151072757 |
| TNIP1 269 | chr5:151036844-151036871 | TNIP1 1434 | chr5:151072738-151072765 |
| TNIP1 270 | chr5:151036845-151036872 | TNIP1 1435 | chr5:151072740-151072767 |
| TNIP1 271 | chr5:151036849-151036876 | TNIP1 1436 | chr5:151072752-151072779 |
| TNIP1 272 | chr5:151036851-151036878 | TNIP1 1437 | chr5:151072757-151072784 |
| TNIP1 273 | chr5:151036852-151036879 | TNIP1 1438 | chr5:151072762-151072789 |
| TNIP1 274 | chr5:151036861-151036888 | TNIP1 1439 | chr5:151072774-151072801 |
| TNIP1 275 | chr5:151036862-151036889 | TNIP1 1440 | chr5:151072777-151072804 |
| TNIP1 276 | chr5:151036863-151036890 | TNIP1 1441 | chr5:151072780-151072807 |
| TNIP1 277 | chr5:151036864-151036891 | TNIP1 1442 | chr5:151072781-151072808 |
| TNIP1 278 | chr5:151036865-151036892 | TNIP1 1443 | chr5:151072782-151072809 |
| TNIP1 279 | chr5:151036866-151036893 | TNIP1 1444 | chr5:151072789-151072816 |
| TNIP1 280 | chr5:151036871-151036898 | TNIP1 1445 | chr5:151072790-151072817 |
| TNIP1 281 | chr5:151036892-151036919 | TNIP1 1446 | chr5:151072799-151072826 |
| TNIP1 282 | chr5:151036893-151036920 | TNIP1 1447 | chr5:151072812-151072839 |
| TNIP1 283 | chr5:151036898-151036925 | TNIP1 1448 | chr5:151072848-151072875 |
| TNIP1 284 | chr5:151036905-151036932 | TNIP1 1449 | chr5:151072863-151072890 |
| TNIP1 285 | chr5:151036912-151036939 | TNIP1 1450 | chr5:151072875-151072902 |
| TNIP1 286 | chr5:151036914-151036941 | TNIP1 1451 | chr5:151072882-151072909 |
| TNIP1 287 | chr5:151036915-151036942 | TNIP1 1452 | chr5:151072894-151072921 |
| TNIP1 288 | chr5:151036921-151036948 | TNIP1 1453 | chr5:151072913-151072940 |
| TNIP1 289 | chr5:151039070-151039097 | TNIP1 1454 | chr5:151072914-151072941 |
| TNIP1 290 | chr5:151039078-151039105 | TNIP1 1455 | chr5:151072918-151072945 |
| TNIP1 291 | chr5:151039079-151039106 | TNIP1 1456 | chr5:151072919-151072946 |
| TNIP1 292 | chr5:151039084-151039111 | TNIP1 1457 | chr5:151072924-151072951 |
| TNIP1 293 | chr5:151039085-151039112 | TNIP1 1458 | chr5:151072927-151072954 |
| TNIP1 294 | chr5:151039092-151039119 | TNIP1 1459 | chr5:151072954-151072981 |
| TNIP1 295 | chr5:151039093-151039120 | TNIP1 1460 | chr5:151072955-151072982 |
| TNIP1 296 | chr5:151039096-151039123 | TNIP1 1461 | chr5:151072958-151072985 |
| TNIP1 297 | chr5:151039100-151039127 | TNIP1 1462 | chr5:151072961-151072988 |
| TNIP1 298 | chr5:151039106-151039133 | TNIP1 1463 | chr5:151072964-151072991 |
| TNIP1 299 | chr5:151039115-151039142 | TNIP1 1464 | chr5:151072967-151072994 |
| TNIP1 300 | chr5:151039116-151039143 | TNIP1 1465 | chr5:151072968-151072995 |
| TNIP1 301 | chr5:151039117-151039144 | TNIP1 1466 | chr5:151072970-151072997 |
| TNIP1 302 | chr5:151039123-151039150 | TNIP1 1467 | chr5:151072971-151072998 |
| TNIP1 303 | chr5:151039124-151039151 | TNIP1 1468 | chr5:151072982-151073009 |
| TNIP1 304 | chr5:151039142-151039169 | TNIP1 1469 | chr5:151072986-151073013 |
| TNIP1 305 | chr5:151039157-151039184 | TNIP1 1470 | chr5:151072987-151073014 |
| TNIP1 306 | chr5:151039162-151039189 | TNIP1 1471 | chr5:151073003-151073030 |
| TNIP1 307 | chr5:151039170-151039197 | TNIP1 1472 | chr5:151073005-151073032 |
| TNIP1 308 | chr5:151039177-151039204 | TNIP1 1473 | chr5:151073014-151073041 |
| TNIP1 309 | chr5:151039192-151039219 | TNIP1 1474 | chr5:151073028-151073055 |
| TNIP1 310 | chr5:151039197-151039224 | TNIP1 1475 | chr5:151073033-151073060 |
| TNIP1 311 | chr5:151039198-151039225 | TNIP1 1476 | chr5:151073047-151073074 |
| TNIP1 312 | chr5:151039205-151039232 | TNIP1 1477 | chr5:151073048-151073075 |
| TNIP1 313 | chr5:151039206-151039233 | TNIP1 1478 | chr5:151073054-151073081 |
| TNIP1 314 | chr5:151039215-151039242 | TNIP1 1479 | chr5:151073059-151073086 |
| TNIP1 315 | chr5:151039216-151039243 | TNIP1 1480 | chr5:151073062-151073089 |
| TNIP1 316 | chr5:151039219-151039246 | TNIPI 1481 | chr5:151073065-151073092 |
| TNIP1 317 | chr5:151042523-151042550 | TNIP1 1482 | chr5:151073066-151073093 |
| TNIP1 318 | chr5:151042532-151042559 | TNIP1 1483 | chr5:151073077-151073104 |
| TNIP1 319 | chr5:151042538-151042565 | TNIP1 1484 | chr5:151073088-151073115 |
| TNIP1 320 | chr5:151042539-151042566 | TNIP1 1485 | chr5:151073089-151073116 |
| TNIP1 321 | chr5:151042542-151042569 | TNIP1 1486 | chr5:151073090-151073117 |
| TNIP1 322 | chr5:151042543-151042570 | TNIP1 1487 | chr5:151073093-151073120 |
| TNIP1 323 | chr5:151042545-151042572 | TNIP1 1488 | chr5:151073096-151073123 |
| TNIP1 324 | chr5:151042546-151042573 | TNIP1 1489 | chr5:151073099-151073126 |
| TNIP1 325 | chr5:151042555-151042582 | TNIP1 1490 | chr5:151073103-151073130 |
| TNIP1 326 | chr5:151042566-151042593 | TNIP1 1491 | chr5:151073104-151073131 |
| TNIP1 327 | chr5:151042586-151042613 | TNIP1 1492 | chr5:151073105-151073132 |
| TNIP1 328 | chr5:151042594-151042621 | TNIP1 1493 | chr5:151073121-151073148 |
| TNIP1 329 | chr5:151042605-151042632 | TNIP1 1494 | chr5:151073122-151073149 |
| TNIP1 330 | chr5:151042606-151042633 | TNIP1 1495 | chr5:151073125-151073152 |
| TNIP1 331 | chr5:151042614-151042641 | TNIP1 1496 | chr5:151073128-151073155 |
| TNIP1 332 | chr5:151042617-151042644 | TNIP1 1497 | chr5:151073169-151073196 |
| TNIP1 333 | chr5:151042629-151042656 | TNIP1 1498 | chr5:151073171-151073198 |
| TNIP1 334 | chr5:151042647-151042674 | TNIP1 1499 | chr5:151073172-151073199 |
| TNIP1 335 | chr5:151042648-151042675 | TNIP1 1500 | chr5:151073180-151073207 |
| TNIP1 336 | chr5:151042649-151042676 | TNIP1 1501 | chr5:151073183-151073210 |
| TNIP1 337 | chr5:151042657-151042684 | TNIP1 1502 | chr5:151073184-151073211 |
| TNIP1 338 | chr5:151042665-151042692 | TNIP1 1503 | chr5:151073190-151073217 |
| TNIP1 339 | chr5:151042878-151042905 | TNIP1 1504 | chr5:151073194-151073221 |
| TNIP1 340 | chr5:151042885-151042912 | TNIP1 1505 | chr5:151073215-151073242 |
| TNIP1 341 | chr5:151042886-151042913 | TNIP1 1506 | chr5:151073218-151073245 |
| TNIP1 342 | chr5:151042887-151042914 | TNIP1 1507 | chr5:151073219-151073246 |
| TNIP1 343 | chr5:151042894-151042921 | TNIP1 1508 | chr5:151073220-151073247 |
| TNIP1 344 | chr5:151042895-151042922 | TNIP1 1509 | chr5:151073229-151073256 |
| TNIP1 345 | chr5:151042899-151042926 | TNIP1 1510 | chr5:151073232-151073259 |
| TNIP1 346 | chr5:151042908-151042935 | TNIP1 1511 | chr5:151073248-151073275 |
| TNIP1 347 | chr5:151042923-151042950 | TNIP1 1512 | chr5:151073249-151073276 |
| TNIP1 348 | chr5:151042937-151042964 | TNIP1 1513 | chr5:151073250-151073277 |
| TNIP1 349 | chr5:151042938-151042965 | TNIP1 1514 | chr5:151073251-151073278 |
| TNIP1 350 | chr5:151042940-151042967 | TNIP1 1515 | chr5:151073269-151073296 |
| TNIP1 351 | chr5:151042941-151042968 | TNIP1 1516 | chr5:151073289-151073316 |
| TNIP1 352 | chr5:151042942-151042969 | TNIP1 1517 | chr5:151073306-151073333 |
| TNIP1 353 | chr5:151042951-151042978 | TNIP1 1518 | chr5:151073307-151073334 |
| TNIP1 354 | chr5:151042955-151042982 | TNIP1 1519 | chr5:151073325-151073352 |
| TNIP1 355 | chr5:151042961-151042988 | TNIP1 1520 | chr5:151073369-151073396 |
| TNIP1 356 | chr5:151045834-151045861 | TNIP1 1521 | chr5:151073394-151073421 |
| TNIP1 357 | chr5:151045835-151045862 | TNIP1 1522 | chr5:151073395-151073422 |
| TNIP1 358 | chr5:151045844-151045871 | TNIP1 1523 | chr5:151073400-151073427 |
| TNIP1 359 | chr5:151045847-151045874 | TNIP1 1524 | chr5:151073410-151073437 |
| TNIP1 360 | chr5:151045853-151045880 | TNIP1 1525 | chr5:151073427-151073454 |
| TNIP1 361 | chr5:151045856-151045883 | TNIP1 1526 | chr5:151073437-151073464 |
| TNIP1 362 | chr5:151045860-151045887 | TNIP1 1527 | chr5:151073440-151073467 |
| TNIP1 363 | chr5:151045874-151045901 | TNIP1 1528 | chr5:151073455-151073482 |
| TNIP1 364 | chr5:151045878-151045905 | TNIP1 1529 | chr5:151073456-151073483 |
| TNIP1 365 | chr5:151045886-151045913 | TNIP1 1530 | chr5:151073457-151073484 |
| TNIP1 366 | chr5:151045890-151045917 | TNIP1 1531 | chr5:151073467-151073494 |
| TNIP1 367 | chr5:151045899-151045926 | TNIP1 1532 | chr5:151073469-151073496 |
| TNIP1 368 | chr5:151045900-151045927 | TNIP1 1533 | chr5:151073515-151073542 |
| TNIP1 369 | chr5:151045907-151045934 | TNIP1 1534 | chr5:151073516-151073543 |
| TNIP1 370 | chr5:151045908-151045935 | TNIP1 1535 | chr5:151073531-151073558 |
| TNIP1 371 | chr5:151045914-151045941 | TNIP1 1536 | chr5:151073580-151073607 |
| TNIP1 372 | chr5:151045917-151045944 | TNIP1 1537 | chr5:151073605-151073632 |
| TNIP1 373 | chr5:151045920-151045947 | TNIP1 1538 | chr5:151073610-151073637 |
| TNIP1 374 | chr5:151045927-151045954 | TNIP1 1539 | chr5:151073626-151073653 |
| TNIP1 375 | chr5:151045928-151045955 | TNIP1 1540 | chr5:151073627-151073654 |
| TNIP1 376 | chr5:151045929-151045956 | TNIP1 1541 | chr5:151073630-151073657 |
| TNIP1 377 | chr5:151045934-151045961 | TNIP1 1542 | chr5:151073633-151073660 |
| TNIP1 378 | chr5:151045938-151045965 | TNIP1 1543 | chr5:151073636-151073663 |
| TNIP1 379 | chr5:151045942-151045969 | TNIP1 1544 | chr5:151073639-151073666 |
| TNIP1 380 | chr5:151045950-151045977 | TNIP1 1545 | chr5:151073640-151073667 |
| TNIP1 381 | chr5:151049816-151049843 | TNIP1 1546 | chr5:151073641-151073668 |
| TNIP1 382 | chr5:151049823-151049850 | TNIP1 1547 | chr5:151073642-151073669 |
| TNIP1 383 | chr5:151049834-151049861 | TNIP1 1548 | chr5:151073643-151073670 |
| TNIP1 384 | chr5:151049838-151049865 | TNIP1 1549 | chr5:151073654-151073681 |
| TNIP1 385 | chr5:151049844-151049871 | TNIP1 1550 | chr5:151073658-151073685 |
| TNIP1 386 | chr5:151049852-151049879 | TNIP1 1551 | chr5:151073659-151073686 |
| TNIP1 387 | chr5:151049853-151049880 | TNIP1 1552 | chr5:151073660-151073687 |
| TNIP1 388 | chr5:151049857-151049884 | TNIP1 1553 | chr5:151073661-151073688 |
| TNIP1 389 | chr5:151049858-151049885 | TNIP1 1554 | chr5:151073677-151073704 |
| TNIP1 390 | chr5:151049862-151049889 | TNIP1 1555 | chr5:151073678-151073705 |
| TNIP1 391 | chr5:151049876-151049903 | TNIP1 1556 | chr5:151073682-151073709 |
| TNIP1 392 | chr5:151049881-151049908 | TNIP1 1557 | chr5:151073696-151073723 |
| TNIP1 393 | chr5:151049884-151049911 | TNIP1 1558 | chr5:151073700-151073727 |
| TNIP1 394 | chr5:151049885-151049912 | TNIP1 1559 | chr5:151073737-151073764 |
| TNIP1 395 | chr5:151049894-151049921 | TNIP1 1560 | chr5:151073740-151073767 |
| TNIP1 396 | chr5:151049895-151049922 | TNIP1 1561 | chr5:151073751-151073778 |
| TNIP1 397 | chr5:151049906-151049933 | TNIP1 1562 | chr5:151073767-151073794 |
| TNIP1 398 | chr5:151049924-151049951 | TNIP1 1563 | chr5:151073774-151073801 |
| TNIP1 399 | chr5:151049925-151049952 | TNIP1 1564 | chr5:151073777-151073804 |
| TNIP1 400 | chr5:151049929-151049956 | TNIP1 1565 | chr5:151073780-151073807 |
| TNIP1 401 | chr5:151049932-151049959 | TNIP1 1566 | chr5:151073781-151073808 |
| TNIP1 402 | chr5:151049934-151049961 | TNIP1 1567 | chr5:151073782-151073809 |
| TNIP1 403 | chr5:151049943-151049970 | TNIP1 1568 | chr5:151073783-151073810 |
| TNIP1 404 | chr5:151049946-151049973 | TNIP1 1569 | chr5:151073784-151073 811 |
| TNIP1 405 | chr5:151049947-151049974 | TNIP1 1570 | chr5:151073808-151073835 |
| TNIP1 406 | chr5:151052138-151052165 | TNIP1 1571 | chr5:151073822-151073849 |
| TNIP1 407 | chr5:151052139-151052166 | TNIPl 1572 | chr5:151073823-151073850 |
| TNIP1 408 | chr5:151052140-151052167 | TNIP1 1573 | chr5:151073857-151073884 |
| TNIP1 409 | *chr5:151052150-151052177* | TNIP1 1574 | chr5:151073867-151073894 |
| TNIP1 410 | chr5:151052154-151052181 | TNIP1 1575 | chr5:151073871-151073898 |
| TNIP1 411 | chr5:151052164-151052191 | TNIP1 1576 | chr5:151073911-151073938 |
| TNIP1 412 | chr5:151052170-151052197 | TNIP1 1577 | chr5:151073946-151073973 |
| TNIP1 413 | chr5:151052173-151052200 | TNIP1 1578 | chr5:151073952-151073979 |
| TNIP1 414 | chr5:151052181-151052208 | TNIP1 1579 | chr5:151073959-151073986 |
| TNIP1 415 | chr5:151052187-151052214 | TNIPl 1580 | chr5:151073964-151073991 |
| TNIP1 416 | chr5:151052188-151052215 | TNIP1 1581 | chr5:151073981-151074008 |
| TNIP1 417 | chr5:151052189-151052216 | TNIP1 1582 | chr5:151074000-151074027 |
| TNIP1 418 | chr5:151052196-151052223 | TNIP1 1583 | chr5:151074020-151074047 |
| TNIP1 419 | chr5:151052201-151052228 | TNIPl 1584 | chr5:151074021-151074048 |
| TNIP1 420 | chr5:151052202-151052229 | TNIP1 1585 | chr5:151074058-151074085 |
| TNIP1 421 | chr5:151052208-151052235 | TNIP1 1586 | chr5:151074074-151074101 |
| TNIP1 422 | chr5:151052217-151052244 | TNIP1 1587 | chr5:151074075-151074102 |
| TNIP1 423 | chr5:151052226-151052253 | TNIP1 1588 | chr5:151074082-151074109 |
| TNIP1 424 | chr5:151052230-151052257 | TNIP1 1589 | chr5:151074086-151074113 |
| TNIP1 425 | chr5:151052231-151052258 | TNIP1 1590 | chr5:151074087-151074114 |
| TNIP1 426 | chr5:151052235-151052262 | TNIP1 1591 | chr5:151074088-151074115 |
| TNIP1 427 | chr5:151052238-151052265 | TNIP1 1592 | chr5:151074093-151074120 |
| TNIP1 428 | chr5:151052239-151052266 | TNIP1 1593 | chr5:151074097-151074124 |
| TNIP1 429 | chr5:151052240-151052267 | TNIPl 1594 | chr5:151074154-151074181 |
| TNIP1 430 | chr5:151052244-151052271 | TNIP1 1595 | chr5:151074155-151074182 |
| TNIP1 431 | chr5:151052245-151052272 | TNIP1 1596 | chr5:151074156-151074183 |
| TNIP1 432 | chr5:151052246-151052273 | TNIP1 1597 | chr5:151074157-151074184 |
| TNIP1 433 | chr5:151052256-151052283 | TNIP1 1598 | chr5:151074158-151074185 |
| TNIP1 434 | chr5:151052257-151052284 | TNIP1 1599 | chr5:151074187-151074214 |
| TNIP1 435 | chr5:151056747-151056774 | TNIP1 1600 | chr5:151074191-151074218 |
| TNIP1 436 | chr5:151056748-151056775 | TNIP1 1601 | chr5:151074192-151074219 |
| TNIP1 437 | chr5:151056749-151056776 | TNIP1 1602 | chr5:151074193-151074220 |
| TNIP1 438 | chr5:151056751-151056778 | TNIP1 1603 | chr5:151074203-151074230 |
| TNIP1 439 | chr5:151056752-151056779 | TNIP1 1604 | chr5:151074204-151074231 |
| TNIP1 440 | chr5:151056753-151056780 | TNIP1 1605 | chr5:151074253-151074280 |
| TNIP1 441 | chr5:151056760-151056787 | TNIP1 1606 | chr5:151074287-151074314 |
| TNIP1 442 | chr5:151056765-151056792 | TNIP1 1607 | chr5:151074294-151074321 |
| TNIP1 443 | chr5:151056779-151056806 | TNIP1 1608 | chr5:151074295-151074322 |
| TNIP1 444 | chr5:151056790-151056817 | TNIP1 1609 | chr5:151074296-151074323 |
| TNIP1 445 | chr5:151056801-151056828 | TNIP1 1610 | chr5:151074297-151074324 |
| TNIP1 446 | chr5:151056802-151056829 | TNIP1 1611 | chr5:151074301-151074328 |
| TNIP1 447 | chr5:151056803-151056830 | TNIP1 1612 | chr5:151074305-151074332 |
| TNIP1 448 | chr5:151056810-151056837 | TNIP1 1613 | chr5:151074329-151074356 |
| TNIP1 449 | chr5:151056811-151056838 | TNIPl 1614 | chr5:151074336-151074363 |
| TNIP1 450 | chr5:151056817-151056844 | TNIP1 1615 | chr5:151074350-151074377 |
| TNIP1 451 | chr5:151056820-151056847 | TNIP1 1616 | chr5:151074351-151074378 |
| TNIP1 452 | chr5:151056821-151056848 | TNIP1 1617 | chr5:151074352-151074379 |
| TNIP1 453 | chr5:151056825-151056852 | TNIP1 1618 | chr5:151074361-151074388 |
| TNIP1 454 | chr5:151056831-151056858 | TNIP1 1619 | chr5:151074362-151074389 |
| TNIP1 455 | chr5:151056832-151056859 | TNIP1 1620 | chr5:151074363-151074390 |
| TNIP1 456 | chr5:151056838-151056865 | TNIP1 1621 | chr5:151074367-151074394 |
| TNIP1 457 | chr5:151056839-151056866 | TNIP1 1622 | chr5:151074368-151074395 |
| TNIP1 458 | chr5:151056840-151056867 | TNIP1 1623 | chr5:151074369-151074396 |
| TNIP1 459 | chr5:151056843-151056870 | TNIP1 1624 | chr5:151074370-151074397 |
| TNIP1 460 | chr5:151056851-151056878 | TNIP1 1625 | chr5:151074385-151074412 |
| TNIP1 461 | chr5:151056860-151056887 | TNIP1 1626 | chr5:151074392-151074419 |
| TNIP1 462 | chr5:151056867-151056894 | TNIP1 1627 | chr5:151074395-151074422 |
| TNIP1 463 | chr5:151056869-151056896 | TNIP1 1628 | chr5:151074401-151074428 |
| TNIP1 464 | chr5:151056873-151056900 | TNIP1 1629 | chr5:151074402-151074429 |
| TNIP1 465 | chr5:151056877-151056904 | TNIP1 1630 | chr5:151074403-151074430 |
| TNIP1 466 | chr5:151056886-151056913 | TNIP1 1631 | chr5:151074424-151074451 |
| TNIP1 467 | chr5:151056898-151056925 | TNIP1 1632 | chr5:151074447-151074474 |
| TNIP1 468 | chr5:151056900-151056927 | TNIP1 1633 | chr5:151074451-151074478 |
| TNIP1 469 | chr5:151056913-151056940 | TNIPl 1634 | chr5:151074452-151074479 |
| TNIP1 470 | chr5:151056914-151056941 | TNIP1 1635 | chr5:151074456-151074483 |
| TNIP1 471 | chr5:151056915-151056942 | TNIP1 1636 | chr5:151074459-151074486 |
| TNIP1 472 | chr5:151056919-151056946 | TNIP1 1637 | chr5:151074460-151074487 |
| TNIP1 473 | chr5:151056920-151056947 | TNIP1 1638 | chr5:151074473-151074500 |
| TNIP1 474 | chr5:151056921-151056948 | TNIP1 1639 | chr5:151074474-151074501 |
| TNIP1 475 | chr5:151056922-151056949 | TNIP1 1640 | chr5:151074483-151074510 |
| TNIP1 476 | chr5:151056929-151056956 | TNIPl 1641 | chr5:151074484-151074511 |
| TNIP1 477 | chr5:151056933-151056960 | TNIP1 1642 | chr5:151074493-151074520 |
| TNIP1 478 | chr5:151056934-151056961 | TNIP1 1643 | chr5:151074496-151074523 |
| TNIP1 479 | chr5:151056939-151056966 | TNIP 1 1644 | chr5:151074503-151074530 |
| TNIP1 480 | chr5:151056940-151056967 | TNIPl 1645 | chr5:151074504-151074531 |
| TNIP1 481 | chr5:151056945-151056972 | TNIP1 1646 | chr5:151074506-151074533 |
| TNIP1 482 | chr5:151056946-151056973 | TNIP1 1647 | chr5:151074525-151074552 |
| TNIP1 483 | chr5:151056950-151056977 | TNIPl 1648 | chr5:151074536-151074563 |
| TNIP1 484 | chr5:151056951-151056978 | TNIP1 1649 | chr5:151074550-151074577 |
| TNIP1 485 | chr5:151056953-151056980 | TNIP1 1650 | chr5:151074555-151074582 |
| TNIP1 486 | chr5:151056957-151056984 | TNIP1 1651 | chr5:151074561-151074588 |
| TNIP1 487 | chr5:151060293-151060320 | TNIP1 1652 | chr5:151074568-151074595 |
| TNIP1 488 | chr5:151060294-151060321 | TNIP1 1653 | chr5:151074569-151074596 |
| TNIP1 489 | chr5:151060301-151060328 | TNIP1 1654 | chr5:151074570-151074597 |
| TNIP1 490 | chr5:151060305-151060332 | TNIP1 1655 | chr5:151074577-151074604 |
| TNIP1 491 | chr5:151060306-151060333 | TNIP1 1656 | chr5:151074581-151074608 |
| TNIP1 492 | chr5:151060310-151060337 | TNIP1 1657 | chr5:151074587-151074614 |
| TNIP1 493 | chr5:151060317-151060344 | TNIP1 1658 | chr5:151074588-151074615 |
| TNIP1 494 | chr5:151060320-151060347 | TNIP1 1659 | chr5:151074589-151074616 |
| TNIP1 495 | chr5:151060338-151060365 | TNIP1 1660 | chr5:151074592-151074619 |
| TNIP1 496 | chr5:151060359-151060386 | TNIP1 1661 | chr5:151074593-151074620 |
| TNIP1 497 | chr5:151060361-151060388 | TNIP1 1662 | chr5:151074598-151074625 |
| TNIP1 498 | chr5:151060364-151060391 | TNIP1 1663 | chr5:151074599-151074626 |
| TNIP1 499 | chr5:151060371-151060398 | TNIP1 1664 | chr5:151074600-151074627 |
| TNIP1 500 | chr5:151060381-151060408 | TNIP1 1665 | chr5:151074606-151074633 |
| TNIP1 501 | chr5:151060389-151060416 | TNIP1 1666 | chr5:151074648-151074675 |
| TNIP1 502 | chr5:151060394-151060421 | TNIP1 1667 | chr5:151074657-151074684 |
| TNIP1 503 | chr5:151060395-151060422 | TNIP1 1668 | chr5:151074688-151074715 |
| TNIP1 504 | chr5:151062100-151062127 | TNIP1 1669 | chr5:151074697-151074724 |
| TNIP1 505 | chr5:151062103-151062130 | TNIP1 1670 | chr5:151074712-151074739 |
| TNIP1 506 | chr5:151062104-151062131 | TNIP1 1671 | chr5:151074731-151074758 |
| TNIP1 507 | chr5:151062108-151062135 | TNIP1 1672 | chr5:151074753-151074780 |
| TNIP1 508 | chr5:151062111-151062138 | TNIP1 1673 | chr5:151074771-151074798 |
| TNIP1 509 | chr5:151062112-151062139 | TNIP1 1674 | chr5:151074777-151074804 |
| TNIP1 510 | chr5:151062118-151062145 | TNIP1 1675 | chr5:151074778-151074805 |
| TNIP1 511 | chr5:151062123-151062150 | TNIP1 1676 | chr5:151074797-151074824 |
| TNIP1 512 | chr5:151062129-151062156 | TNIP1 1677 | chr5:151074801-151074828 |
| TNIP1 513 | chr5:151062140-151062167 | TNIP1 1678 | chr5:151074811-151074838 |
| TNIP1 514 | chr5:151062141-151062168 | TNIP1 1679 | chr5:151074819-151074846 |
| TNIP1 515 | chr5:151062142-151062169 | TNIP1 1680 | chr5:151074820-151074847 |
| TNIP1 516 | chr5:151062150-151062177 | TNIP1 1681 | chr5:151074863-151074890 |
| TNIP1 517 | chr5:151062151-151062178 | TNIP1 1682 | chr5:151074866-151074893 |
| TNIP1 518 | chr5:151062152-151062179 | TNIP1 1683 | chr5:151074869-151074896 |
| TNIP1 519 | chr5:151062158-151062185 | TNIP1 1684 | chr5:151074870-151074897 |
| TNIP1 520 | chr5:151062159-151062186 | TNIP1 1685 | chr5:151074874-151074901 |
| TNIP1 521 | chr5:151062189-151062216 | TNIP1 1686 | chr5:151074875-151074902 |
| TNIP1 522 | chr5:151062207-151062234 | TNIP1 1687 | chr5:151074884-151074911 |
| TNIP1 523 | chr5:151062212-151062239 | TNIP1 1688 | chr5:151074885-151074912 |
| TNIP1 524 | chr5:151063595-151063622 | TNIP1 1689 | chr5:151074887-151074914 |
| TNIP1 525 | chr5:151063596-151063623 | TNIP1 1690 | chr5:151074888-151074915 |
| TNIP1 526 | chr5:151063602-151063629 | TNIP1 1691 | chr5:151074891-151074918 |
| TNIP1 527 | chr5:151063603-151063630 | TNIP1 1692 | chr5:151074894-151074921 |
| TNIP1 528 | chr5:151063604-151063631 | TNIP1 1693 | chr5:151074897-151074924 |
| TNIP1 529 | chr5:151063605-151063632 | TNIP1 1694 | chr5:151074928-151074955 |
| TNIP1 530 | chr5:151063612-151063639 | TNIP1 1695 | chr5:151074931-151074958 |
| TNIP1 531 | chr5:151063621-151063648 | TNIP1 1696 | chr5:151074936-151074963 |
| TNIP1 532 | chr5:151063624-151063651 | TNIP1 1697 | chr5:151074937-151074964 |
| TNIP1 533 | chr5:151063625-151063652 | TNIP1 1698 | chr5:151074941-151074968 |
| TNIP1 534 | chr5:151063631-151063658 | TNIP1 1699 | chr5:151074942-151074969 |
| TNIP1 535 | chr5:151063634-151063661 | TNIP1 1700 | chr5:151074948-151074975 |
| TNIP1 536 | chr5:151063637-151063664 | TNIP1 1701 | chr5:151074966-151074993 |
| TNIP1 537 | chr5:151063638-151063665 | TNIP1 1702 | chr5:151074976-151075003 |
| TNIP1 538 | chr5:151063642-151063669 | TNIP1 1703 | chr5:151074980-151075007 |
| TNIP1 539 | chr5:151063643-151063670 | TNIP1 1704 | chr5:151074981-151075008 |
| TNIP1 540 | chr5:151063679-151063706 | TNIP1 1705 | chr5:151074984-151075011 |
| TNIP1 541 | chr5:151063680-151063707 | TNIP1 1706 | chr5:151074985-151075012 |
| TNIP1 542 | chr5:151063687-151063714 | TNIP1 1707 | chr5:151075003-151075030 |
| TNIP1 543 | chr5:151063691-151063718 | TNIP1 1708 | chr5:151075019-151075046 |
| TNIP1 544 | chr5:151063697-151063724 | TNIP1 1709 | chr5:151075022-151075049 |
| TNIP1 545 | chr5:151063701-151063728 | TNIP1 1710 | chr5:151075026-151075053 |
| TNIP1 546 | chr5:151063702-151063729 | TNIP1 1711 | chr5:151075028-151075055 |
| TNIP1 547 | chr5:151063704-151063731 | TNIP1 1712 | chr5:151075035-151075062 |
| TNIP1 548 | chr5:151063710-151063737 | TNIP1 1713 | chr5: 151075038-151075065 |
| TNIP1 549 | chr5:151063721-151063748 | TNIP1 1714 | chr5:151075039-151075066 |
| TNIP1 550 | chr5:151063733-151063760 | TNIP1 1715 | chr5:151075047-151075074 |
| TNIP1 551 | chr5:151063734-151063761 | TNIP1 1716 | chr5:151075065-151075092 |
| TNIP1 552 | chr5:151063735-151063762 | TNIP1 1717 | chr5:151075075-151075102 |
| TNIP1 553 | chr5:151063736-151063763 | TNIP1 1718 | chr5:151075094-151075121 |
| TNIP1 554 | chr5:151063741-151063768 | TNIP1 1719 | chr5:151075095-151075122 |
| TNIP1 555 | chr5:151063745-151063772 | TNIP1 1720 | chr5:151075168-151075195 |
| TNIP1 556 | chr5:151063746-151063773 | TNIP1 1721 | chr5:151075169-151075196 |
| TNIP1 557 | chr5:151063747-151063774 | TNIP1 1722 | chr5:151075170-151075197 |
| TNIP1 558 | chr5:151064959-151064986 | TNIP1 1723 | chr5:151075174-151075201 |
| TNIP1 559 | chr5:151064970-151064997 | TNIP1 1724 | chr5:151075177-151075204 |
| TNIP1 560 | chr5:151064971-151064998 | TNIP1 1725 | chr5:151075187-151075214 |
| TNIP1 561 | chr5:151064973-151065000 | TNIP1 1726 | chr5:151075206-151075233 |
| TNIP1 562 | chr5:151064974-151065001 | TNIP1 1727 | chr5:151075246-151075273 |
| TNIP1 563 | chr5:151064987-151065014 | TNIP1 1728 | chr5:151075253-151075280 |
| TNIP1 564 | chr5:151064988-151065015 | TNIP1 1729 | chr5:151075266-151075293 |
| TNIP1 565 | chr5:151064994-151065021 | TNIP1 1730 | chr5:151075276-151075303 |
| TNIP1 566 | chr5:151065004-151065031 | TNIP1 1731 | chr5:151075287-151075314 |
| TNIP1 567 | chr5:151065005-151065032 | TNIP1 1732 | chr5:151075288-151075315 |
| TNIP1 568 | chr5:151065019-151065046 | TNIP1 1733 | chr5:151075307-151075334 |
| TNIP1 569 | chr5:151065020-151065047 | TNIP1 1734 | chr5:151075352-151075379 |
| TNIP1 570 | chr5:151065035-151065062 | TNIP1 1735 | chr5:151075353-151075380 |
| TNIP1 571 | chr5:151065036-151065063 | TNIP1 1736 | chr5:151075354-151075381 |
| TNIP1 572 | chr5:151065040-151065067 | TNIP1 1737 | chr5:151075355-151075382 |
| TNIP1 573 | chr5:151065048-151065075 | TNIP1 1738 | chr5:151075356-151075383 |
| TNIP1 574 | chr5:151065053-151065080 | TNIP1 1739 | chr5:151075368-151075395 |
| TNIP1 575 | chr5:151065055-151065082 | TNIP1 1740 | chr5:151075380-151075407 |
| TNIP1 576 | chr5:151065056-151065083 | TNIP1 1741 | chr5:151075381-151075408 |
| TNIP1 577 | chr5:151065057-151065084 | TNIP1 1742 | chr5:151075398-151075425 |
| TNIP1 578 | chr5:151065058-151065085 | TNIP1 1743 | chr5:151075399-151075426 |
| TNIP1 579 | chr5:151065072-151065099 | TNIP1 1744 | chr5:151075403-151075430 |
| TNIP1 580 | chr5:151065073-151065100 | TNIP1 1745 | chr5:151075404-151075431 |
| TNIP1 581 | chr5:151065082-151065109 | TNIP1 1746 | chr5:151075405-151075432 |
| TNIP1 582 | chr5:151065087-151065114 | TNIP1 1747 | chr5:151075421-151075448 |
| TNIP1 583 | chr5:151065088-151065115 | TNIP1 1748 | chr5:151075422-151075449 |
| TNIP1 584 | chr5:151065092-151065119 | TNIP1 1749 | chr5:151075437-151075464 |
| TNIP1 585 | chr5:151065093-151065120 | TNIP1 1750 | chr5: 151075438-151075465 |
| TNIP1 586 | chr5:151065104-151065131 | TNIP1 1751 | chr5:151075444-151075471 |
| TNIP1 587 | chr5:151065109-151065136 | TNIP1 1752 | chr5:151075464-151075491 |
| TNIP1 588 | chr5:151065110-151065137 | TNIP1 1753 | chr5:151075465-151075492 |
| TNIP1 589 | chr5:151065111-151065138 | TNIP1 1754 | chr5:151075485-151075512 |
| TNIP1 590 | chr5:151065112-151065139 | TNIP1 1755 | chr5:151075488-151075515 |
| TNIP1 591 | chr5:151065115-151065142 | TNIP1 1756 | chr5:151075489-151075516 |
| TNIP1 592 | chr5:151065123-151065150 | TNIP1 1757 | chr5:151075492-151075519 |
| TNIP1 593 | chr5:151065124-151065151 | TNIP1 1758 | chr5:151075493-151075520 |
| TNIP1 594 | chr5:151065127-151065154 | TNIP1 1759 | chr5:151075496-151075523 |
| TNIP1 595 | chr5:151067626-151067653 | TNIP1 1760 | chr5:151075497-151075524 |
| TNIP1 596 | chr5:151067633-151067660 | TNIP1 1761 | chr5:151075498-151075525 |
| TNIP1 597 | chr5:151067636-151067663 | TNIP1 1762 | chr5:151075511-151075538 |
| TNIP1 598 | chr5:151067639-151067666 | TNIP1 1763 | chr5:151075512-151075539 |
| TNIP1 599 | chr5:151067641-151067668 | TNIP1 1764 | chr5:151075513-151075540 |
| TNIP1 600 | chr5:151067642-151067669 | TNIP1 1765 | chr5:151075521-151075548 |
| TNIP1 601 | chr5:151067666-151067693 | TNIP1 1766 | chr5:151075529-151075556 |
| TNIP1 602 | chr5:151067667-151067694 | TNIP1 1767 | chr5:151075541-151075568 |
| TNIP1 603 | chr5:151067677-151067704 | TNIP1 1768 | chr5:151075550-151075577 |
| TNIP1 604 | chr5:151067681-151067708 | TNIP1 1769 | chr5:151075564-151075591 |
| TNIP1 605 | chr5:151067689-151067716 | TNIP1 1770 | chr5:151075567-151075594 |
| TNIP1 606 | chr5:151067692-151067719 | TNIP1 1771 | chr5:151075596-151075623 |
| TNIP1 607 | chr5:151067693-151067720 | TNIP1 1772 | chr5:151075604-151075631 |
| TNIP1 608 | chr5:151067699-151067726 | TNIP1 1773 | chr5:151075636-151075663 |
| TNIP1 609 | chr5:151067709-151067736 | TNIP1 1774 | chr5:151075650-151075677 |
| TNIP1 610 | chr5:151067710-151067737 | TNIP1 1775 | chr5:151075651-151075678 |
| TNIP1 611 | chr5:151067721-151067748 | TNIP1 1776 | chr5:151075657-151075684 |
| TNIP1 612 | chr5:151067731-151067758 | TNIP1 1777 | chr5:151075665-151075692 |
| TNIP1 613 | chr5:151067732-151067759 | TNIP1 1778 | chr5:151075677-151075704 |
| TNIP1 614 | chr5:151067733-151067760 | TNIP1 1779 | chr5:151075678-151075705 |
| TNIP1 615 | chr5:151067767-151067794 | TNIP1 1780 | chr5:151075682-151075709 |
| TNIP1 616 | chr5:151067768-151067795 | TNIP1 1781 | chr5:151075686-151075713 |
| TNIP1 617 | chr5:151067769-151067796 | TNIP1 1782 | chr5:151075687-151075714 |
| TNIP1 618 | chr5:151067774-151067801 | TNIP1 1783 | chr5:151075688-151075715 |
| TNIP1 619 | chr5:151067775-151067802 | TNIP1 1784 | chr5:151075694-151075721 |
| TNIP1 620 | chr5:151067776-151067803 | TNIP1 1785 | chr5:151075695-151075722 |
| TNIP1 621 | chr5:151067780-151067807 | TNIP1 1786 | chr5:151075717-151075744 |
| TNIP1 622 | chr5:151067781-151067808 | TNIP1 1787 | chr5:151075725-151075752 |
| TNIP1 623 | chr5:151067782-151067809 | TNIP1 1788 | chr5:151075732-151075759 |
| TNIP1 624 | chr5:151067785-151067812 | TNIP1 1789 | chr5: 151075738-151075765 |
| TNIP1 625 | chr5:151067788-151067815 | TNIP1 1790 | chr5:151075746-151075773 |
| TNIP1 626 | chr5:151067797-151067824 | TNIP1 1791 | chr5:151075754-151075781 |
| TNIP1 627 | chr5:151067803-151067830 | TNIP1 1792 | chr5:151075770-151075797 |
| TNIP1 628 | chr5:151067810-151067837 | TNIP1 1793 | chr5:151078173-151078200 |
| TNIP1 629 | chr5:151067811-151067838 | TNIP1 1794 | chr5:151078179-151078206 |
| TNIP1 630 | chr5:151067821-151067848 | TNIP1 1795 | chr5:151078185-151078212 |
| TNIP1 631 | chr5:151067826-151067853 | TNIP1 1796 | chr5:151078188-151078215 |
| TNIP1 632 | chr5:151067827-151067854 | TNIP1 1797 | chr5:151078191-151078218 |
| TNIP1 633 | chr5:151067832-151067859 | TNIP1 1798 | chr5:151078192-151078219 |
| TNIP1 634 | chr5:151067833-151067860 | TNIP1 1799 | chr5:151078198-151078225 |
| TNIP1 635 | chr5:151067840-151067867 | TNIP1 1800 | chr5:151078208-151078235 |
| TNIP1 636 | chr5:151067852-151067879 | TNIP1 1801 | chr5:151078221-151078248 |
| TNIP1 637 | chr5:151067856-151067883 | TNIP1 1802 | chr5:151078222-151078249 |
| TNIP1 638 | chr5:151067857-151067884 | TNIP1 1803 | chr5:151078233-151078260 |
| TNIP1 639 | chr5:151067858-151067885 | TNIP1 1804 | chr5:151078245-151078272 |
| TNIP1 640 | chr5:151067875-151067902 | TNIP1 1805 | chr5:151078272-151078299 |
| TNIP1 641 | chr5:151067880-151067907 | TNIP1 1806 | chr5:151078277-151078304 |
| TNIP1 642 | chr5:151067885-151067912 | TNIP1 1807 | chr5:151078280-151078307 |
| TNIP1 643 | chr5:151067889-151067916 | TNIP1 1808 | chr5:151078284-151078311 |
| TNIP1 644 | chr5:151067893-151067920 | TNIP1 1809 | chr5:151078300-151078327 |
| TNIP1 645 | chr5:151067896-151067923 | TNIP1 1810 | chr5:151078309-151078336 |
| TNIP1 646 | chr5:151067897-151067924 | TNIP1 1811 | chr5:151078327-151078354 |
| TNIP1 647 | chr5:151067899-151067926 | TNIP1 1812 | chr5:151078349-151078376 |
| TNIP1 648 | chr5:151067900-151067927 | TNIP1 1813 | chr5:151078365-151078392 |
| TNIP1 649 | chr5:151067908-151067935 | TNIP1 1814 | chr5:151078369-151078396 |
| TNIP1 650 | chr5:151067909-151067936 | TNIP1 1815 | chr5:151078372-151078399 |
| TNIP1 651 | chr5:151067910-151067937 | TNIP1 1816 | chr5:151078384-151078411 |
| TNIP1 652 | chr5:151067917-151067944 | TNIP1 1817 | chr5:151078386-151078413 |
| TNIP1 653 | chr5:151067923-151067950 | TNIP1 1818 | chr5:151078387-151078414 |
| TNIP1 654 | chr5:151067924-151067951 | TNIP1 1819 | chr5:151078391-151078418 |
| TNIP1 655 | chr5:151067960-151067987 | TNIP1 1820 | chr5:151078394-151078421 |
| TNIP1 656 | chr5:151067961-151067988 | TNIP1 1821 | chr5:151078421-151078448 |
| TNIP1 657 | chr5:151067964-151067991 | TNIP1 1822 | chr5:151078423-151078450 |
| TNIP1 658 | chr5:151067965-151067992 | TNIP1 1823 | chr5:151078424-151078451 |
| TNIP1 659 | chr5:151067966-151067993 | TNIP1 1824 | chr5:151078432-151078459 |
| TNIP1 660 | chr5:151067972-151067999 | TNIP1 1825 | chr5:151078433-151078460 |
| TNIP1 661 | chr5:151067975-151068002 | TNIP1 1826 | chr5:151078434-151078461 |
| TNIP1 662 | chr5:151067977-151068004 | TNIP1 1827 | chr5:151078435-151078462 |
| TNIP1 663 | chr5:151067983-151068010 | TNIP1 1828 | chr5:151078495-151078522 |
| TNIP1 664 | chr5:151067984-151068011 | TNIP1 1829 | chr5:151078579-151078606 |
| TNIP1 665 | chr5:151067985-151068012 | TNIP1 1830 | chr5:151078580-151078607 |
| TNIP1 666 | chr5:151067994-151068021 | TNIP1 1831 | chr5:151078581-151078608 |
| TNIP1 667 | chr5:151067995-151068022 | TNIP1 1832 | chr5:151078604-151078631 |
| TNIP1 668 | chr5:151068000-151068027 | TNIP1 1833 | chr5:151078605-151078632 |
| TNIP1 669 | chr5:151068001-151068028 | TNIP1 1834 | chr5:151078609-151078636 |
| TNIP1 670 | chr5:151068002-151068029 | TNIP1 1835 | chr5:151078620-151078647 |
| TNIP1 671 | chr5:151068009-151068036 | TNIP1 1836 | chr5:151078637-151078664 |
| TNIP1 672 | chr5:151068010-151068037 | TNIP1 1837 | chr5:151078638-151078665 |
| TNIP1 673 | chr5:151068023-151068050 | TNIP1 1838 | chr5:151078639-151078666 |
| TNIP1 674 | chr5:151068024-151068051 | TNIP1 1839 | chr5:151078654-151078681 |
| TNIP1 675 | chr5:151068059-151068086 | TNIP1 1840 | chr5:151078669-151078696 |
| TNIP1 676 | chr5:151068060-151068087 | TNIP1 1841 | chr5:151078670-151078697 |
| TNIP1 677 | chr5:151068061-151068088 | TNIP1 1842 | chr5:151078678-151078705 |
| TNIP1 678 | chr5:151068062-151068089 | TNIP1 1843 | chr5:151078679-151078706 |
| TNIP1 679 | chr5:151068068-151068095 | TNIP1 1844 | chr5:151078680-151078707 |
| TNIP1 680 | chr5:151068069-151068096 | TNIP1 1845 | chr5:151078687-151078714 |
| TNIP1 681 | chr5:151068070-151068097 | TNIP1 1846 | chr5:151078691-151078718 |
| TNIP1 682 | chr5:151068072-151068099 | TNIP1 1847 | chr5:151078693-151078720 |
| TNIP1 683 | chr5:151068073-151068100 | TNIP1 1848 | chr5:151078694-151078721 |
| TNIP1 684 | chr5:151068074-151068101 | TNIP1 1849 | chr5:151078700-151078727 |
| TNIP1 685 | chr5:151068080-151068107 | TNIP1 1850 | chr5:151078701-151078728 |
| TNIP1 686 | chr5:151068085-151068112 | TNIP1 1851 | chr5:151078716-151078743 |
| TNIP1 687 | chr5:151068091-151068118 | TNIP1 1852 | chr5:151078725-151078752 |
| TNIP1 688 | chr5:151068092-151068119 | TNIP1 1853 | chr5:151078741-151078768 |
| TNIP1 689 | chr5:151068098-151068125 | TNIP1 1854 | chr5:151078746-151078773 |
| TNIP1 690 | chr5:151068107-151068134 | TNIP1 1855 | chr5:151078749-151078776 |
| TNIP1 691 | chr5:151068125-151068152 | TNIP1 1856 | chr5:151078752-151078779 |
| TNIP1 692 | chr5:151068126-151068153 | TNIP1 1857 | chr5:151078753-151078780 |
| TNIP1 693 | chr5:151068140-151068167 | TNIP1 1858 | chr5:151078754-151078781 |
| TNIP1 694 | chr5:151068143-151068170 | TNIP1 1859 | chr5:151078755-151078782 |
| TNIP1 695 | chr5:151068150-151068177 | TNIP1 1860 | chr5:151078764-151078791 |
| TNIP1 696 | chr5:151068158-151068185 | TNIP1 1861 | chr5:151078773-151078800 |
| TNIP1 697 | chr5:151068159-151068186 | TNIP1 1862 | chr5:151078792-151078819 |
| TNIP1 698 | chr5:151068162-151068189 | TNIP1 1863 | chr5:151078793-151078820 |
| TNIP1 699 | chr5:151068165-151068192 | TNIP1 1864 | chr5:151078799-151078826 |
| TNIP1 700 | chr5:151068166-151068193 | TNIP1 1865 | chr5:151078806-151078833 |
| TNIP1 701 | chr5:151068171-151068198 | TNIP1 1866 | chr5:151078819-151078846 |
| TNIP1 702 | chr5:151068179-151068206 | TNIP1 1867 | chr5:151078820-151078847 |
| TNIP1 703 | chr5:151068180-151068207 | TNIP1 1868 | chr5:151078824-151078851 |
| TNIP1 704 | chr5:151068182-151068209 | TNIP1 1869 | chr5:151078833-151078860 |
| TNIP1 705 | chr5:151068183-151068210 | TNIP1 1870 | chr5:151078840-151078867 |
| TNIP1 706 | chr5:151068184-151068211 | TNIP1 1871 | chr5:151078872-151078899 |
| TNIP1 707 | chr5:151068185-151068212 | TNIP1 1872 | chr5:151078875-151078902 |
| TNIP1 708 | chr5:151068194-151068221 | TNIP1 1873 | chr5:151078879-151078906 |
| TNIP1 709 | chr5:151068199-151068226 | TNIP1 1874 | chr5:151078887-151078914 |
| TNIP1 710 | chr5:151068200-151068227 | TNIP1 1875 | chr5:151078894-151078921 |
| TNIP1 711 | chr5:151068207-151068234 | TNIP1 1876 | chr5:151078902-151078929 |
| TNIP1 712 | chr5:151068208-151068235 | TNIP1 1877 | chr5:151078903-151078930 |
| TNIP1 713 | chr5:151068241-151068268 | TNIP1 1878 | chr5:151078907-151078934 |
| TNIP1 714 | chr5:151068243-151068270 | TNIP1 1879 | chr5:151078912-151078939 |
| TNIP1 715 | chr5:151068250-151068277 | TNIP1 1880 | chr5:151078913-151078940 |
| TNIP1 716 | chr5:151068255-151068282 | TNIP1 1881 | chr5:151078917-151078944 |
| TNIP1 717 | chr5:151068260-151068287 | TNIP1 1882 | chr5:151078926-151078953 |
| TNIP1 718 | chr5:151068262-151068289 | TNIP1 1883 | chr5:151078927-151078954 |
| TNIP1 719 | chr5:151068263-151068290 | TNIP1 1884 | chr5:151078928-151078955 |
| TNIP1 720 | chr5:151068271-151068298 | TNIP1 1885 | chr5:151078929-151078956 |
| TNIP1 721 | chr5:151068272-151068299 | TNIP1 1886 | chr5:151078931-151078958 |
| TNIP1 722 | chr5:151068279-151068306 | TNIP1 1887 | chr5:151078932-151078959 |
| TNIP1 723 | chr5:151068280-151068307 | TNIP1 1888 | chr5:151078933-151078960 |
| TNIP1 724 | chr5:151068282-151068309 | TNIP1 1889 | chr5:151078934-151078961 |
| TNIP1 725 | chr5:151068283-151068310 | TNIP1 1890 | chr5:151078935-151078962 |
| TNIP1 726 | chr5:151068292-151068319 | TNIP1 1891 | chr5:151078962-151078989 |
| TNIP1 727 | chr5:151068303-151068330 | TNIP1 1892 | chr5:151078963-151078990 |
| TNIP1 728 | chr5:151068314-151068341 | TNIP1 1893 | chr5:151078964-151078991 |
| TNIP1 729 | chr5:151068315-151068342 | TNIP1 1894 | chr5:151078974-151079001 |
| TNIP1 730 | chr5:151068329-151068356 | TNIP1 1895 | *chr5:151078975-151079002* |
| TNIP1 731 | chr5:151068342-151068369 | TNIP1 1896 | chr5:151078976-151079003 |
| TNIP1 732 | chr5:151068345-151068372 | TNIP1 1897 | chr5:151078979-151079006 |
| TNIP1 733 | chr5:151068346-151068373 | TNIP1 1898 | chr5:151078982-151079009 |
| TNIP1 734 | chr5:151068355-151068382 | TNIP1 1899 | chr5:151078983-151079010 |
| TNIP1 735 | chr5: 151068358-151068385 | TNIP1 1900 | chr5:151078984-151079011 |
| TNIP1 736 | chr5:151068359-151068386 | TNIP1 1901 | chr5:151078985-151079012 |
| TNIP1 737 | chr5:151068360-151068387 | TNIP1 1902 | chr5:151078992-151079019 |
| TNIP1 738 | chr5:151068371-151068398 | TNIP1 1903 | chr5:151078993-151079020 |
| TNIP1 739 | chr5:151068372-151068399 | TNIP1 1904 | chr5:151078994-151079021 |
| TNIP1 740 | chr5:151068380-151068407 | TNIP1 1905 | chr5:151078995-151079022 |
| TNIP1 741 | chr5:151068393-151068420 | TNIP1 1906 | chr5:151078999-151079026 |
| TNIP1 742 | chr5:151068399-151068426 | TNIP1 1907 | chr5:151079016-151079043 |
| TNIP1 743 | chr5:151068402-151068429 | TNIP1 1908 | chr5:151079033-151079060 |
| TNIP1 744 | chr5:151068403-151068430 | TNIP1 1909 | chr5:151079037-151079064 |
| TNIP1 745 | chr5:151068413-151068440 | TNIP1 1910 | chr5:151079048-151079075 |
| TNIP1 746 | chr5:151068432-151068459 | TNIP1 1911 | chr5:151079052-151079079 |
| TNIP1 747 | chr5:151068438-151068465 | TNIP1 1912 | chr5:151079053-151079080 |
| TNIP1 748 | chr5:151068439-151068466 | TNIP1 1913 | chr5:151079058-151079085 |
| TNIP1 749 | chr5:151068440-151068467 | TNIP1 1914 | chr5:151079059-151079086 |
| TNIP1 750 | chr5:151068442-151068469 | TNIP1 1915 | chr5:151079065-151079092 |
| TNIP1 751 | chr5:151068443-151068470 | TNIP1 1916 | chr5:151079069-151079096 |
| TNIP1 752 | chr5:151068462-151068489 | TNIP1 1917 | chr5:151079070-151079097 |
| TNIP1 753 | chr5:151068497-151068524 | TNIP1 1918 | chr5:151079102-151079129 |
| TNIP1 754 | chr5:151068498-151068525 | TNIP1 1919 | chr5:151079115-151079142 |
| TNIP1 755 | chr5:151068499-151068526 | TNIP1 1920 | chr5:151079116-151079143 |
| TNIP1 756 | chr5:151068500-151068527 | TNIP1 1921 | chr5:151079117-151079144 |
| TNIP1 757 | chr5:151068510-151068537 | TNIP1 1922 | chr5:151079122-151079149 |
| TNIP1 758 | chr5:151068511-151068538 | TNIP1 1923 | chr5:151079123-151079150 |
| TNIP1 759 | chr5:151068512-151068539 | TNIP1 1924 | chr5:151079136-151079163 |
| TNIP1 760 | chr5:151068517-151068544 | TNIP1 1925 | chr5:151079160-151079187 |
| TNIP1 761 | chr5:151068518-151068545 | TNIP1 1926 | chr5:151079170-151079197 |
| TNIP1 762 | chr5:151068545-151068572 | TNIP1 1927 | chr5:151079171-151079198 |
| TNIP1 763 | chr5:151068555-151068582 | TNIP1 1928 | chr5:151079175-151079202 |
| TNIP1 764 | chr5:151068560-151068587 | TNIP1 1929 | chr5:151079176-151079203 |
| TNIP1 765 | chr5:151068561-151068588 | TNIP1 1930 | chr5:151079182-151079209 |
| TNIP1 766 | chr5:151068562-151068589 | TNIP1 1931 | chr5:151079193-151079220 |
| TNIP1 767 | chr5:151068565-151068592 | TNIP1 1932 | chr5:151079194-151079221 |
| TNIP1 768 | chr5:151068570-151068597 | TNIP1 1933 | chr5:151079195-151079222 |
| TNIP1 769 | chr5:151068579-151068606 | TNIP1 1934 | chr5:151079200-151079227 |
| TNIP1 770 | chr5:151068580-151068607 | TNIP1 1935 | chr5:151079201-151079228 |
| TNIP1 771 | chr5:151068592-151068619 | TNIP1 1936 | chr5:151079202-151079229 |
| TNIP1 772 | chr5:151068593-151068620 | TNIP1 1937 | chr5:151079205-151079232 |
| TNIP1 773 | chr5:151068601-151068628 | TNIP1 1938 | chr5:151079207-151079234 |
| TNIP1 774 | chr5:151068608-151068635 | TNIP1 1939 | chr5:151079208-151079235 |
| TNIP1 775 | chr5:151068614-151068641 | TNIP1 1940 | chr5:151079214-151079241 |
| TNIP1 776 | chr5:151068627-151068654 | TNIP1 1941 | chr5:151079215-151079242 |
| TNIP1 777 | chr5:151068632-151068659 | TNIP1 1942 | chr5:151079217-151079244 |
| TNIP1 778 | chr5:151068643-151068670 | TNIP1 1943 | chr5:151079221-151079248 |
| TNIP1 779 | chr5:151068644-151068671 | TNIP1 1944 | chr5:151079243-151079270 |
| TNIP1 780 | chr5:151068648-151068675 | TNIP1 1945 | chr5:151079244-151079271 |
| TNIP1 781 | chr5:151068654-151068681 | TNIP1 1946 | chr5:151079264-151079291 |
| TNIP1 782 | chr5:151068660-151068687 | TNIP1 1947 | chr5:151079265-151079292 |
| TNIP1 783 | chr5:151068661-151068688 | TNIP1 1948 | chr5:151079276-151079303 |
| TNIP1 784 | chr5:151068663-151068690 | TNIP1 1949 | chr5:151079282-151079309 |
| TNIP1 785 | chr5:151068670-151068697 | TNIP1 1950 | chr5:151079293-151079320 |
| TNIP1 786 | chr5:151068674-151068701 | TNIP1 1951 | chr5:151079302-151079329 |
| TNIP1 787 | chr5:151068676-151068703 | TNIP1 1952 | chr5:151079316-151079343 |
| TNIP1 788 | chr5:151068680-151068707 | TNIP1 1953 | chr5:151079320-151079347 |
| TNIP1 789 | chr5:151068681-151068708 | TNIP1 1954 | chr5:151079321-151079348 |
| TNIP1 790 | chr5:151068709-151068736 | TNIP1 1955 | chr5:151079324-151079351 |
| TNIP1 791 | chr5:151068710-151068737 | TNIP1 1956 | chr5:151079325-151079352 |
| TNIP1 792 | chr5:151068719-151068746 | TNIP1 1957 | chr5:151079326-151079353 |
| TNIP1 793 | chr5:151068720-151068747 | TNIP1 1958 | chr5:151079329-151079356 |
| TNIP1 794 | chr5:151068728-151068755 | TNIP1 1959 | chr5:151079344-151079371 |
| TNIP1 795 | chr5:151068729-151068756 | TNIP1 1960 | chr5:151079357-151079384 |
| TNIP1 796 | chr5:151068732-151068759 | TNIP1 1961 | chr5:151079360-151079387 |
| TNIP1 797 | chr5:151068740-151068767 | TNIP1 1962 | chr5:151079363-151079390 |
| TNIP1 798 | chr5:151068741-151068768 | TNIP1 1963 | chr5:151079369-151079396 |
| TNIP1 799 | chr5:151068747-151068774 | TNIP1 1964 | chr5:151079370-151079397 |
| TNIP1 800 | chr5:151068748-151068775 | TNIP1 1965 | chr5:151079371-151079398 |
| TNIP1 801 | chr5:151068752-151068779 | TNIP1 1966 | chr5:151079372-151079399 |
| TNIP1 802 | chr5:151068755-151068782 | TNIP1 1967 | chr5:151079373-151079400 |
| TNIP1 803 | chr5:151068758-151068785 | TNIP1 1968 | chr5:151079384-151079411 |
| TNIP1 804 | chr5:151068761-151068788 | TNIP1 1969 | chr5:151079389-151079416 |
| TNIP1 805 | chr5:151068762-151068789 | TNIP1 1970 | chr5:151079407-151079434 |
| TNIP1 806 | chr5:151068766-151068793 | TNIP1 1971 | chr5:151079416-151079443 |
| TNIP1 807 | chr5:151068769-151068796 | TNIP1 1972 | chr5:151079426-151079453 |
| TNIP1 808 | chr5:151068770-151068797 | TNIP1 1973 | chr5:151079430-151079457 |
| TNIP1 809 | chr5:151068771-151068798 | TNIP1 1974 | chr5:151079435-151079462 |
| TNIP1 810 | chr5:151068779-151068806 | TNIP1 1975 | chr5:151079455-151079482 |
| TNIP1 811 | chr5:151068780-151068807 | TNIP1 1976 | chr5:151079456-151079483 |
| TNIP1 812 | chr5:151068790-151068817 | TNIP1 1977 | chr5:151079457-151079484 |
| TNIP1 813 | chr5:151068797-151068824 | TNIP1 1978 | chr5:151079461-151079488 |
| TNIP1 814 | chr5:151068798-151068825 | TNIP1 1979 | chr5:151079464-151079491 |
| TNIP1 815 | chr5:151068799-151068826 | TNIP1 1980 | chr5:151079479-151079506 |
| TNIP1 816 | chr5:151068800-151068827 | TNIP1 1981 | chr5:151079491-151079518 |
| TNIP1 817 | chr5:151068803-151068830 | TNIP1 1982 | chr5:151079492-151079519 |
| TNIP1 818 | chr5:151068806-151068833 | TNIP1 1983 | chr5:151079495-151079522 |
| TNIP1 819 | chr5:151068811-151068838 | TNIP1 1984 | chr5:151079498-151079525 |
| TNIP1 820 | chr5:151068826-151068853 | TNIP1 1985 | chr5:151079501-151079528 |
| TNIP1 821 | chr5:151068829-151068856 | TNIP1 1986 | chr5:151079505-151079532 |
| TNIP1 822 | chr5:151068834-151068861 | TNIP1 1987 | chr5:151079506-151079533 |
| TNIP1 823 | chr5:151068835-151068862 | TNIP1 1988 | chr5:151079507-151079534 |
| TNIP1 824 | chr5:151068841-151068868 | TNIP1 1989 | chr5:151079523-151079550 |
| TNIP1 825 | chr5:151068852-151068879 | TNIP1 1990 | chr5:151079524-151079551 |
| TNIP1 826 | chr5:151068853-151068880 | TNIP1 1991 | chr5:151079527-151079554 |
| TNIP1 827 | chr5:151068867-151068894 | TNIP1 1992 | chr5:151079546-151079573 |
| TNIP1 828 | chr5:151068870-151068897 | TNIP1 1993 | chr5:151079562-151079589 |
| TNIP1 829 | chr5:151068884-151068911 | TNIP1 1994 | chr5:151079569-151079596 |
| TNIP1 830 | chr5:151068888-151068915 | TNIP1 1995 | chr5:151079571-151079598 |
| TNIP1 831 | chr5:151068889-151068916 | TNIP1 1996 | chr5:151079572-151079599 |
| TNIP1 832 | chr5:151068890-151068917 | TNIP1 1997 | chr5:151079580-151079607 |
| TNIP1 833 | chr5:151068892-151068919 | TNIP1 1998 | chr5:151079590-151079617 |
| TNIP1 834 | chr5:151068897-151068924 | TNIP1 1999 | chr5:151079594-151079621 |
| TNIP1 835 | chr5:151068898-151068925 | TNIP1 2000 | chr5:151079625-151079652 |
| TNIP1 836 | chr5:151068903-151068930 | TNIP1 2001 | chr5:151080126-151080153 |
| TNIP1 837 | chr5:151068931-151068958 | TNIP1 2002 | chr5:151080144-151080171 |
| TNIP1 838 | chr5:151068944-151068971 | TNIP1 2003 | chr5:151080163-151080190 |
| TNIP1 839 | chr5:151068945-151068972 | TNIP1 2004 | chr5:151080167-151080194 |
| TNIP1 840 | chr5:151068946-151068973 | TNIP1 2005 | chr5:151080173-151080200 |
| TNIP1 841 | chr5:151068948-151068975 | TNIP1 2006 | chr5:151080177-151080204 |
| TNIP1 842 | chr5:151068959-151068986 | TNIP1 2007 | chr5:151080178-151080205 |
| TNIP1 843 | chr5:151068965-151068992 | TNIP1 2008 | chr5:151080179-151080206 |
| TNIP1 844 | chr5:151068968-151068995 | TNIP1 2009 | chr5:151080190-151080217 |
| TNIP1 845 | chr5:151068969-151068996 | TNIP1 2010 | chr5:151080203-151080230 |
| TNIP1 846 | chr5:151068970-151068997 | TNIP1 2011 | chr5:151080207-151080234 |
| TNIP1 847 | chr5:151068974-151069001 | TNIP1 2012 | chr5:151080227-151080254 |
| TNIP1 848 | chr5:151068976-151069003 | TNIP1 2013 | chr5:151080230-151080257 |
| TNIP1 849 | chr5:151068977-151069004 | TNIP1 2014 | chr5:151080249-151080276 |
| TNIP1 850 | chr5:151068978-151069005 | TNIP1 2015 | chr5:151080257-151080284 |
| TNIP1 851 | chr5:151068983-151069010 | TNIP1 2016 | chr5:151080287-151080314 |
| TNIP1 852 | chr5:151068987-151069014 | TNIP1 2017 | chr5:151080294-151080321 |
| TNIP1 853 | chr5:151068988-151069015 | TNIP1 2018 | chr5:151080328-151080355 |
| TNIP1 854 | chr5:151068989-151069016 | TNIP1 2019 | chr5:151080334-151080361 |
| TNIP1 855 | chr5:151068998-151069025 | TNIP1 2020 | chr5:151080343-151080370 |
| TNIP1 856 | chr5:151068999-151069026 | TNIP1 2021 | chr5:151080348-151080375 |
| TNIP1 857 | chr5:151069000-151069027 | TNIP1 2022 | chr5:151080350-151080377 |
| TNIP1 858 | chr5:151069009-151069036 | TNIP1 2023 | chr5:151080359-151080386 |
| TNIP1 859 | chr5:151069010-151069037 | TNIP1 2024 | chr5:151080855-151080882 |
| TNIP1 860 | chr5:151069011-151069038 | TNIP1 2025 | chr5:151080856-151080883 |
| TNIP1 861 | chr5:151069012-151069039 | TNIP1 2026 | chr5:151080857-151080884 |
| TNIP1 862 | chr5:151069013-151069040 | TNIP1 2027 | chr5:151080858-151080885 |
| TNIP1 863 | chr5:151069019-151069046 | TNIP1 2028 | chr5:151080862-151080889 |
| TNIP1 864 | chr5:151069021-151069048 | TNIP1 2029 | chr5:151080863-151080890 |
| TNIP1 865 | chr5:151069022-151069049 | TNIP1 2030 | chr5:151080864-151080891 |
| TNIP1 866 | chr5:151069023-151069050 | TNIP1 2031 | chr5:151080867-151080894 |
| TNIP1 867 | chr5:151069028-151069055 | TNIP1 2032 | chr5:151080871-151080898 |
| TNIP1 868 | chr5:151069029-151069056 | TNIP1 2033 | chr5:151080872-151080899 |
| TNIP1 869 | chr5:151069036-151069063 | TNIP1 2034 | chr5:151080873-151080900 |
| TNIP1 870 | chr5:151069040-151069067 | TNIP1 2035 | chr5:151080879-151080906 |
| TNIP1 871 | chr5:151069045-151069072 | TNIP1 2036 | chr5:151080880-151080907 |
| TNIP1 872 | chr5:151069051-151069078 | TNIP1 2037 | chr5:151080909-151080936 |
| TNIP1 873 | chr5:151069052-151069079 | TNIP1 2038 | chr5:151080910-151080937 |
| TNIP1 874 | chr5:151069070-151069097 | TNIP1 2039 | chr5:151080911-151080938 |
| TNIP1 875 | chr5:151069079-151069106 | TNIP1 2040 | chr5:151080913-151080940 |
| TNIP1 876 | chr5:151069086-151069113 | TNIP1 2041 | chr5:151080914-151080941 |
| TNIP1 877 | chr5:151069094-151069121 | TNIP1 2042 | chr5:151080918-151080945 |
| TNIP1 878 | chr5:151069111-151069138 | TNIP1 2043 | chr5:151080919-151080946 |
| TNIP1 879 | chr5:151069115-151069142 | TNIP1 2044 | chr5:151080920-151080947 |
| TNIP1 880 | *chr5:151069124-151069151* | TNIP1 2045 | chr5:151080924-151080951 |
| TNIP1 881 | *chr5:151069125-151069152* | TNIP1 2046 | chr5:151080926-151080953 |
| TNIP1 882 | chr5:151069127-151069154 | TNIP1 2047 | chr5:151080928-151080955 |
| TNIP1 883 | chr5:151069128-151069155 | TNIP1 2048 | chr5:151080929-151080956 |
| TNIP1 884 | chr5:151069132-151069159 | TNIP1 2049 | chr5:151080930-151080957 |
| TNIP1 885 | chr5:151069133-151069160 | TNIP1 2050 | chr5:151080931-151080958 |
| TNIP1 886 | chr5:151069136-151069163 | TNIP1 2051 | chr5:151080932-151080959 |
| TNIP1 887 | chr5:151069150-151069177 | TNIP1 2052 | chr5:151080936-151080963 |
| TNIP1 888 | chr5:151069151-151069178 | TNIP1 2053 | chr5:151080937-151080964 |
| TNIP1 889 | chr5:151069171-151069198 | TNIP1 2054 | chr5:151080943-151080970 |
| TNIP1 890 | chr5:151069172-151069199 | TNIP1 2055 | chr5:151080954-151080981 |
| TNIP1 891 | chr5:151069173-151069200 | TNIP1 2056 | chr5:151080955-151080982 |
| TNIP1 892 | chr5:151069183-151069210 | TNIP1 2057 | chr5:151080963-151080990 |
| TNIP1 893 | chr5:151069184-151069211 | TNIP1 2058 | chr5:151080964-151080991 |
| TNIP1 894 | chr5:151069185-151069212 | TNIP1 2059 | chr5:151080967-151080994 |
| TNIP1 895 | chr5:151069191-151069218 | TNIP1 2060 | chr5:151080971-151080998 |
| TNIP1 896 | chr5:151069196-151069223 | TNIP1 2061 | chr5:151080972-151080999 |
| TNIP1 897 | chr5:151069197-151069224 | TNIP1 2062 | chr5:151080973-151081000 |
| TNIP1 898 | chr5:151069198-151069225 | TNIP1 2063 | chr5:151080974-151081001 |
| TNIP1 899 | chr5:151069247-151069274 | TNIP1 2064 | chr5:151080975-151081002 |
| TNIP1 900 | chr5:151069282-151069309 | TNIP1 2065 | chr5:151080976-151081003 |
| TNIP1 901 | chr5:151069285-151069312 | TNIP1 2066 | chr5:151080977-151081004 |
| TNIP1 902 | chr5:151069289-151069316 | TNIP1 2067 | chr5:151080978-151081005 |
| TNIP1 903 | chr5:151069290-151069317 | TNIP1 2068 | chr5:151080982-151081009 |
| TNIP1 904 | chr5:151069291-151069318 | TNIP1 2069 | chr5:151080983-151081010 |
| TNIP1 905 | chr5:151069292-151069319 | TNIP1 2070 | chr5:151080985-151081012 |
| TNIP1 906 | chr5:151069294-151069321 | TNIP1 2071 | chr5:151080990-151081017 |
| TNIP1 907 | chr5:151069295-151069322 | TNIP1 2072 | chr5:151080991-151081018 |
| TNIP1 908 | chr5:151069296-151069323 | TNIP1 2073 | chr5:151080994-151081021 |
| TNIP1 909 | chr5:151069297-151069324 | TNIP1 2074 | chr5:151080997-151081024 |
| TNIP1 910 | chr5:151069298-151069325 | TNIP1 2075 | chr5:151081003-151081030 |
| TNIP1 911 | chr5:151069301-151069328 | TNIP1 2076 | chr5:151081012-151081039 |
| TNIP1 912 | chr5:151069330-151069357 | TNIP1 2077 | chr5:151081013-151081040 |
| TNIP1 913 | chr5:151069331-151069358 | TNIP1 2078 | chr5:151081014-151081041 |
| TNIP1 914 | chr5:151069336-151069363 | TNIP1 2079 | chr5:151081015-151081042 |
| TNIP1 915 | chr5:151069337-151069364 | TNIP1 2080 | chr5:151081019-151081046 |
| TNIP1 916 | chr5:151069338-151069365 | TNIP1 2081 | chr5:151081020-151081047 |
| TNIP1 917 | chr5:151069339-151069366 | TNIP1 2082 | chr5:151081024-151081051 |
| TNIP1 918 | chr5:151069340-151069367 | TNIP1 2083 | chr5:151081025-151081052 |
| TNIP1 919 | chr5:151069363-151069390 | TNIP1 2084 | chr5:151087058-151087085 |
| TNIP1 920 | chr5:151069394-151069421 | TNIP1 2085 | chr5:151087059-151087086 |
| TNIP1 921 | chr5:151069395-151069422 | TNIP1 2086 | chr5:151087061-151087088 |
| TNIP1 922 | chr5:151069408-151069435 | TNIP1 2087 | chr5:151087062-151087089 |
| TNIP1 923 | chr5:151069409-151069436 | TNIP1 2088 | chr5:151087063-151087090 |
| TNIP1 924 | chr5:151069410-151069437 | TNIP1 2089 | chr5:151087066-151087093 |
| TNIP1 925 | chr5:151069416-151069443 | TNIP1 2090 | chr5:151087067-151087094 |
| TNIP1 926 | chr5:151069420-151069447 | TNIP1 2091 | chr5:151087068-151087095 |
| TNIP1 927 | chr5:151069421-151069448 | TNIP1 2092 | chr5:151087069-151087096 |
| TNIP1 928 | chr5:151069430-151069457 | TNIP1 2093 | chr5:151087072-151087099 |
| TNIP1 929 | chr5:151069431-151069458 | TNIP1 2094 | chr5:151087083-151087110 |
| TNIP1 930 | chr5:151069439-151069466 | TNIP1 2095 | chr5:151087084-151087111 |
| TNIP1 931 | chr5:151069445-151069472 | TNIP1 2096 | chr5:151087097-151087124 |
| TNIP1 932 | chr5:151069455-151069482 | TNIP1 2097 | chr5:151087098-151087125 |
| TNIP1 933 | chr5:151069459-151069486 | TNIP1 2098 | chr5:151087099-151087126 |
| TNIP1 934 | chr5:151069464-151069491 | TNIP1 2099 | chr5:151087102-151087129 |
| TNIP1 935 | chr5:151069465-151069492 | TNIP1 2100 | chr5:151087117-151087144 |
| TNIP1 936 | chr5:151069466-151069493 | TNIP1 2101 | chr5:151087120-151087147 |
| TNIP1 937 | chr5:151069483-151069510 | TNIP1 2102 | chr5:151087125-151087152 |
| TNIP1 938 | chr5:151069495-151069522 | TNIP1 2103 | chr5:151087134-151087161 |
| TNIP1 939 | chr5:151069502-151069529 | TNIP1 2104 | chr5:151087135-151087162 |
| TNIP1 940 | chr5:151069504-151069531 | TNIP1 2105 | chr5:151087144-151087171 |
| TNIP1 941 | chr5:151069505-151069532 | TNIP1 2106 | chr5:151087147-151087174 |
| TNIP1 942 | chr5:151069513-151069540 | TNIP1 2107 | chr5:151087148-151087175 |
| TNIP1 943 | chr5:151069514-151069541 | TNIP1 2108 | chr5:151087151-151087178 |
| TNIP1 944 | chr5:151069515-151069542 | TNIP1 2109 | chr5:151087152-151087179 |
| TNIP1 945 | chr5:151069516-151069543 | TNIP1 2110 | chr5:151087156-151087183 |
| TNIP1 946 | chr5:151069536-151069563 | TNIP1 2111 | chr5:151087157-151087184 |
| TNIP1 947 | chr5:151069537-151069564 | TNIP1 2112 | chr5:151087567-151087594 |
| TNIP1 948 | chr5:151069543-151069570 | TNIP1 2113 | chr5:151087570-151087597 |
| TNIP1 949 | chr5:151069551-151069578 | TNIP1 2114 | chr5:151087571-151087598 |
| TNIP1 950 | chr5:151069552-151069579 | TNIP1 2115 | chr5:151087574-151087601 |
| TNIP1 951 | chr5:151069558-151069585 | TNIP1 2116 | chr5:151087575-151087602 |
| TNIP1 952 | chr5:151069559-151069586 | TNIP1 2117 | chr5:151087576-151087603 |
| TNIP1 953 | chr5:151069560-151069587 | TNIP1 2118 | chr5:151087581-151087608 |
| TNIP1 954 | chr5:151069566-151069593 | TNIP1 2119 | chr5:151087586-151087613 |
| TNIP1 955 | chr5:151069567-151069594 | TNIP1 2120 | chr5:151087590-151087617 |
| TNIP1 956 | chr5:151069576-151069603 | TNIP1 2121 | chr5:151087597-151087624 |
| TNIP1 957 | chr5:151069577-151069604 | TNIP1 2122 | chr5:151087610-151087637 |
| TNIP1 958 | chr5:151069580-151069607 | TNIP1 2123 | chr5:151087611-151087638 |
| TNIP1 959 | chr5:151069581-151069608 | TNIP1 2124 | chr5:151087612-151087639 |
| TNIP1 960 | chr5:151069586-151069613 | TNIP1 2125 | chr5:151087619-151087646 |
| TNIP1 961 | chr5:151069587-151069614 | TNIP1 2126 | chr5:151087623-151087650 |
| TNIP1 962 | chr5:151069589-151069616 | TNIP1 2127 | chr5:151087628-151087655 |
| TNIP1 963 | chr5:151069595-151069622 | TNIP1 2128 | chr5:151087640-151087667 |
| TNIP1 964 | chr5:151069596-151069623 | TNIP1 2129 | chr5:151087641-151087668 |
| TNIP1 965 | chr5:151069599-151069626 | TNIP1 2130 | chr5:151087646-151087673 |
| TNIP1 966 | chr5:151069602-151069629 | TNIP1 2131 | chr5:151087649-151087676 |
| TNIP1 967 | chr5:151069606-151069633 | TNIP1 2132 | chr5:151087650-151087677 |
| TNIP1 968 | chr5:151069615-151069642 | TNIP1 2133 | chr5:151087662-151087689 |
| TNIP1 969 | chr5:151069640-151069667 | TNIP1 2134 | chr5:151087684-151087711 |
| TNIP1 970 | chr5:151069643-151069670 | TNIP1 2135 | chr5:151087690-151087717 |
| TNIP1 971 | chr5:151069645-151069672 | TNIP1 2136 | chr5:151087699-151087726 |
| TNIP1 972 | chr5:151069646-151069673 | TNIP1 2137 | chr5:151087705-151087732 |
| TNIP1 973 | chr5:151069653-151069680 | TNIP1 2138 | chr5:151087706-151087733 |
| TNIP1 974 | chr5:151069654-151069681 | TNIP1 2139 | chr5:151087725-151087752 |
| TNIP1 975 | chr5:151069655-151069682 | TNIP1 2140 | chr5:151087737-151087764 |
| TNIP1 976 | chr5:151069656-151069683 | TNIP1 2141 | chr5:151087750-151087777 |
| TNIP1 977 | chr5:151069657-151069684 | TNIP1 2142 | chr5:151087759-151087786 |
| TNIP1 978 | chr5:151069658-151069685 | TNIP1 2143 | chr5:151087765-151087792 |
| TNIP1 979 | chr5:151069662-151069689 | TNIP1 2144 | chr5:151087766-151087793 |
| TNIP1 980 | chr5:151069666-151069693 | TNIP1 2145 | chr5:151087777-151087804 |
| TNIP1 981 | chr5:151069669-151069696 | TNIP1 2146 | chr5:151087791-151087818 |
| TNIP1 982 | chr5:151069670-151069697 | TNIP1 2147 | chr5:151087794-151087821 |
| TNIP1 983 | chr5:151069674-151069701 | TNIP1 2148 | chr5:151087800-151087827 |
| TNIP1 984 | chr5:151069677-151069704 | TNIP1 2149 | chr5:151087807-151087834 |
| TNIP1 985 | chr5:151069680-151069707 | TNIP1 2150 | chr5:151087811-151087838 |
| TNIP1 986 | chr5:151069681-151069708 | TNIP1 2151 | chr5:151087814-151087841 |
| TNIP1 987 | chr5:151069682-151069709 | TNIP1 2152 | chr5:151087815-151087842 |
| TNIP1 988 | chr5:151069683-151069710 | TNIP1 2153 | chr5:151087819-151087846 |
| TNIP1 989 | chr5:151069689-151069716 | TNIP1 2154 | chr5:151087827-151087854 |
| TNIP1 990 | chr5:151069690-151069717 | TNIP1 2155 | chr5:151087843-151087870 |
| TNIP1 991 | chr5:151069694-151069721 | TNIP1 2156 | chr5:151087847-151087874 |
| TNIP1 992 | chr5:151069695-151069722 | TNIP1 2157 | chr5:151087849-151087876 |
| TNIP1 993 | chr5:151069696-151069723 | TNIP1 2158 | chr5:151087859-151087886 |
| TNIP1 994 | chr5:151069704-151069731 | TNIP1 2159 | chr5:151087861-151087888 |
| TNIP1 995 | chr5:151069712-151069739 | TNIP1 2160 | chr5:151087866-151087893 |
| TNIP1 996 | chr5:151069727-151069754 | TNIP1 2161 | chr5:151087870-151087897 |
| TNIP1 997 | chr5:151069735-151069762 | TNIP1 2162 | chr5:151087882-151087909 |
| TNIP1 998 | chr5:151069766-151069793 | TNIP1 2163 | chr5:151087887-151087914 |
| TNIP1 999 | chr5:151069774-151069801 | TNIP1 2164 | chr5:151087904-151087931 |
| TNIP1 1000 | chr5:151069775-151069802 | TNIP1 2165 | chr5:151087909-151087936 |
| TNIP1 1001 | chr5:151069796-151069823 | TNIP1 2166 | chr5:151087919-151087946 |
| TNIP1 1002 | chr5:151069797-151069824 | TNIP1 2167 | chr5:151087923-151087950 |
| TNIP1 1003 | chr5:151069798-151069825 | TNIP1 2168 | chr5:151087949-151087976 |
| TNIP1 1004 | chr5:151069801-151069828 | TNIP1 2169 | chr5:151087950-151087977 |
| TNIP1 1005 | chr5:151069808-151069835 | TNIP1 2170 | chr5:151087951-151087978 |
| TNIP1 1006 | chr5:151069809-151069836 | TNIP1 2171 | chr5:151087955-151087982 |
| TNIP1 1007 | chr5:151069810-151069837 | TNIP1 2172 | chr5:151087958-151087985 |
| TNIP1 1008 | chr5:151069824-151069851 | TNIP1 2173 | chr5:151087985-151088012 |
| TNIP1 1009 | chr5:151069825-151069852 | TNIP1 2174 | chr5:151087986-151088013 |
| TNIP1 1010 | chr5:151069831-151069858 | TNIP1 2175 | chr5:151087989-151088016 |
| TNIP1 1011 | chr5:151069837-151069864 | TNIP1 2176 | chr5:151087992-151088019 |
| TNIP1 1012 | chr5:151069841-151069868 | TNIP1 2177 | chr5:151087995-151088022 |
| TNIP1 1013 | chr5:151069848-151069875 | TNIP1 2178 | chr5:151087998-151088025 |
| TNIP1 1014 | chr5:151069869-151069896 | TNIP1 2179 | chr5:151087999-151088026 |
| TNIP1 1015 | chr5:151069871-151069898 | TNIP1 2180 | chr5:151088000-151088027 |
| TNIP1 1016 | chr5:151069886-151069913 | TNIP1 2181 | chr5:151088001-151088028 |
| TNIP1 1017 | chr5:151069887-151069914 | TNIP1 2182 | chr5:151088018-151088045 |
| TNIP1 1018 | chr5:151069890-151069917 | TNIP1 2183 | chr5:151088021-151088048 |
| TNIP1 1019 | chr5:151069914-151069941 | TNIP1 2184 | chr5:151088027-151088054 |
| TNIP1 1020 | chr5:151069930-151069957 | TNIP1 2185 | chr5:151088040-151088067 |
| TNIP1 1021 | chr5:151069931-151069958 | TNIP1 2186 | chr5:151088041-151088068 |
| TNIP1 1022 | chr5:151069934-151069961 | TNIP1 2187 | chr5:151088046-151088073 |
| TNIP1 1023 | chr5:151069936-151069963 | TNIP1 2188 | chr5:151088065-151088092 |
| TNIP1 1024 | chr5:151069938-151069965 | TNIP1 2189 | chr5:151088067-151088094 |
| .TNIP1 1025 | chr5:151069946-151069973 | TNIP1 2190 | chr5:151088068-151088095 |
| TNIP1 1026 | chr5:151069953-151069980 | TNIP1 2191 | chr5:151088076-151088103 |
| TNIP1 1027 | chr5:151069956-151069983 | TNIP1 2192 | chr5:151088086-151088113 |
| TNIP1 1028 | chr5:151069957-151069984 | TNIP1 2193 | chr5:151088090-151088117 |
| TNIP1 1029 | chr5:151069970-151069997 | TNIP1 2194 | chr5:151088109-151088136 |
| TNIP1 1030 | chr5:151069975-151070002 | TNIP1 2195 | chr5:151088110-151088137 |
| TNIP1 1031 | chr5:151069990-151070017 | TNIP1 2196 | chr5:151088164-151088191 |
| TNIP1 1032 | chr5:151069998-151070025 | TNIP1 2197 | chr5:151088205-151088232 |
| TNIP1 1033 | chr5:151069999-151070026 | TNIP1 2198 | chr5:151088206-151088233 |
| TNIP1 1034 | chr5:151070000-151070027 | TNIP1 2199 | chr5:151088207-151088234 |
| TNIP1 1035 | chr5:151070001-151070028 | TNIP1 2200 | chr5:151088208-151088235 |
| TNIP1 1036 | chr5:151070002-151070029 | TNIP1 2201 | chr5:151088210-151088237 |
| TNIP1 1037 | chr5:151070018-151070045 | TNIP1 2202 | chr5:151088211-151088238 |
| TNIP1 1038 | chr5:151070030-151070057 | TNIP1 2203 | chr5:151088212-151088239 |
| TNIP1 1039 | chr5:151070035-151070062 | TNIP1 2204 | chr5:151088214-151088241 |
| TNIP1 1040 | chr5:151070040-151070067 | TNIP1 2205 | chr5:151088215-151088242 |
| TNIP1 1041 | chr5:151070041-151070068 | TNIP1 2206 | chr5:151088216-151088243 |
| TNIP1 1042 | chr5:151070045-151070072 | TNIP1 2207 | chr5:151088221-151088248 |
| TNIP1 1043 | chr5:151070055-151070082 | TNIP1 2208 | chr5:151088222-151088249 |
| TNIP1 1044 | chr5:151070057-151070084 | TNIP1 2209 | chr5:151088228-151088255 |
| TNIP1 1045 | chr5:151070060-151070087 | TNIP1 2210 | chr5:151088230-151088257 |
| TNIP1 1046 | chr5:151070061-151070088 | TNIP1 2211 | chr5:151088236-151088263 |
| TNIP1 1047 | chr5:151070077-151070104 | TNIP1 2212 | chr5:151088241-151088268 |
| TNIP1 1048 | chr5:151070078-151070105 | TNIP1 2213 | chr5:151088250-151088277 |
| TNIP1 1049 | chr5:151070085-151070112 | TNIP1 2214 | chr5:151088261-151088288 |
| TNIP1 1050 | chr5:151070088-151070115 | TNIP1 2215 | chr5:151088269-151088296 |
| TNIP1 1051 | chr5:151070095-151070122 | TNIP1 2216 | chr5:151088284-151088311 |
| TNIP1 1052 | chr5:151070100-151070127 | TNIP1 2217 | chr5:151088286-151088313 |
| TNIP1 1053 | chr5:151070105-151070132 | TNIP1 2218 | chr5:151088291-151088318 |
| TNIP1 1054 | chr5:151070106-151070133 | TNIP1 2219 | chr5:151088298-151088325 |
| TNIP1 1055 | chr5:151070115-151070142 | TNIP1 2220 | chr5:151088300-151088327 |
| TNIP1 1056 | chr5:151070146-151070173 | TNIP1 2221 | chr5:151088308-151088335 |
| TNIP1 1057 | chr5:151070150-151070177 | TNIP1 2222 | chr5:151088320-151088347 |
| TNIP1 1058 | chr5:151070171-151070198 | TNIP1 2223 | chr5:151088327-151088354 |
| TNIP1 1059 | chr5:151070175-151070202 | TNIP1 2224 | chr5:151088358-151088385 |
| TNIP1 1060 | chr5:151070176-151070203 | TNIP1 2225 | chr5:151088359-151088386 |
| TNIP1 1061 | chr5:151070198-151070225 | TNIP1 2226 | chr5:151088365-151088392 |
| TNIP1 1062 | chr5:151070234-151070261 | TNIP1 2227 | chr5:151088368-151088395 |
| TNIP1 1063 | chr5:151070285-151070312 | TNIP1 2228 | chr5:151088369-151088396 |
| TNIP1 1064 | chr5:151070309-151070336 | TNIP1 2229 | chr5:151088378-151088405 |
| TNIP1 1065 | chr5:151070323-151070350 | TNIP1 2230 | chr5:151088379-151088406 |
| TNIP1 1066 | chr5:151070329-151070356 | TNIP1 2231 | chr5:151088380-151088407 |
| TNIP1 1067 | chr5:151070360-151070387 | TNIP1 2232 | chr5:151088398-151088425 |
| TNIP1 1068 | chr5:151070361-151070388 | TNIP1 2233 | chr5:151088462-151088489 |
| TNIP1 1069 | chr5:151070362-151070389 | TNIP1 2234 | chr5:151088499-151088526 |
| TNIP1 1070 | chr5:151070368-151070395 | TNIP1 2235 | chr5:151088508-151088535 |
| TNIP1 1071 | chr5:151070373-151070400 | TNIP1 2236 | chr5:151088540-151088567 |
| TNIP1 1072 | chr5:151070374-151070401 | TNIP1 2237 | chr5:151088543-151088570 |
| TNIP1 1073 | chr5:151070390-151070417 | TNIP1 2238 | chr5:151088555-151088582 |
| TNIP1 1074 | chr5:151070397-151070424 | TNIP1 2239 | chr5:151088556-151088583 |
| TNIP1 1075 | chr5:151070398-151070425 | TNIP1 2240 | chr5:151088557-151088584 |
| TNIP1 1076 | chr5:151070400-151070427 | TNIP1 2241 | chr5:151088558-151088585 |
| TNIP1 1077 | chr5:151070401-151070428 | TNIP1 2242 | chr5:151088565-151088592 |
| TNIP1 1078 | chr5:151070402-151070429 | TNIP1 2243 | chr5:151088574-151088601 |
| TNIP1 1079 | chr5:151070406-151070433 | TNIP1 2244 | chr5:151088577-151088604 |
| TNIP1 1080 | chr5:151070415-151070442 | TNIP1 2245 | chr5:151088597-151088624 |
| TNIP1 1081 | chr5:151070432-151070459 | TNIP1 2246 | chr5:151088612-151088639 |
| TNIP1 1082 | chr5:151070438-151070465 | TNIP1 2247 | chr5:151088616-151088643 |
| TNIP1 1083 | chr5:151070439-151070466 | TNIP1 2248 | chr5:151088627-151088654 |
| TNIP1 1084 | chr5:151070466-151070493 | TNIP1 2249 | chr5:151088628-151088655 |
| TNIP1 1085 | chr5:151070469-151070496 | TNIP1 2250 | chr5:151088629-151088656 |
| TNIP1 1086 | chr5:151070484-151070511 | TNIP1 2251 | chr5: 151088630-151088657 |
| TNIP1 1087 | chr5:151070487-151070514 | TNIP1 2252 | chr5:151088636-151088663 |
| TNIP1 1088 | chr5:151070493-151070520 | TNIP1 2253 | chr5:151088637-151088664 |
| TNIP1 1089 | chr5:151070494-151070521 | TNIP1 2254 | chr5:151088644-151088671 |
| TNIP1 1090 | chr5:151070498-151070525 | TNIP1 2255 | chr5:151088660-151088687 |
| TNIP1 1091 | chr5:151070501-151070528 | TNIP1 2256 | chr5:151088665-151088692 |
| TNIP1 1092 | chr5:151070524-151070551 | TNIP1 2257 | chr5:151088667-151088694 |
| TNIP1 1093 | chr5:151070534-151070561 | TNIP1 2258 | chr5:151088671-151088698 |
| TNIP1 1094 | chr5:151070565-151070592 | TNIP1 2259 | chr5:151088672-151088699 |
| TNIP1 1095 | chr5:151070566-151070593 | TNIP1 2260 | chr5:151088673-151088700 |
| TNIP1 1096 | chr5:151070573-151070600 | TNIP1 2261 | chr5:151088679-151088706 |
| TNIP1 1097 | chr5:151070574-151070601 | TNIP1 2262 | chr5:151088680-151088707 |
| TNIP1 1098 | chr5:151070581-151070608 | TNIP1 2263 | chr5:151088692-151088719 |
| TNIP1 1099 | chr5:151070590-151070617 | TNIP1 2264 | chr5:151088695-151088722 |
| TNIP1 1100 | chr5:151070605-151070632 | TNIP1 2265 | chr5:151088715-151088742 |
| TNIP1 1101 | chr5:151070606-151070633 | TNIP1 2266 | chr5:151088731-151088758 |
| TNIP1 1102 | chr5:151070610-151070637 | TNIP1 2267 | chr5:151088743-151088770 |
| TNIP1 1103 | chr5:151070618-151070645 | TNIP1 2268 | chr5:151088744-151088771 |
| TNIP1 1104 | chr5:151070631-151070658 | TNIP1 2269 | chr5:151088754-151088781 |
| TNIP1 1105 | chr5:151070639-151070666 | TNIP1 2270 | chr5:151088764-151088791 |
| TNIP1 1106 | chr5:151070640-151070667 | TNIP1 2271 | chr5:151088765-151088792 |
| TNIP1 1107 | chr5:151070649-151070676 | TNIP1 2272 | chr5:151088783-151088810 |
| TNIP1 1108 | chr5:151070652-151070679 | TNIP1 2273 | chr5:151088857-151088884 |
| TNIP1 1109 | chr5:151070653-151070680 | TNIP1 2274 | chr5:151088860-151088887 |
| TNIP1 1110 | chr5:151070677-151070704 | TNIP1 2275 | chr5:151088861-151088888 |
| TNIP1 1111 | chr5:151070714-151070741 | TNIP1 2276 | chr5:151088869-151088896 |
| TNIP1 1112 | chr5:151070736-151070763 | TNIP1 2277 | chr5:151088877-151088904 |
| TNIP1 1113 | chr5:151070737-151070764 | TNIP1 2278 | chr5:151088878-151088905 |
| TNIP1 1114 | chr5:151070739-151070766 | TNIP1 2279 | chr5:151088879-151088906 |
| TNIP1 1115 | chr5:151070741-151070768 | TNIP1 2280 | chr5:151088880-151088907 |
| TNIP1 1116 | chr5:151070742-151070769 | TNIP1 2281 | chr5:151088882-151088909 |
| TNIP1 1117 | chr5:151070743-151070770 | TNIP1 2282 | chr5:151088892-151088919 |
| TNIP1 1118 | chr5:151070744-151070771 | TNIP1 2283 | chr5:151088901-151088928 |
| TNIP1 1119 | chr5:151070747-151070774 | TNIP1 2284 | chr5:151088914-151088941 |
| TNIP1 1120 | chr5:151070748-151070775 | TNIP1 2285 | chr5:151088920-151088947 |
| TNIP1 1121 | chr5:151070749-151070776 | TNIP1 2286 | chr5:151088924-151088951 |
| TNIP1 1122 | chr5:151070753-151070780 | TNIP1 2287 | chr5:151088928-151088955 |
| TNIP1 1123 | chr5:151070759-151070786 | TNIP1 2288 | chr5:151088929-151088956 |
| TNIP1 1124 | chr5:151070760-151070787 | TNIP1 2289 | chr5:151088930-151088957 |
| TNIP1 1125 | chr5:151070774-151070801 | TNIP1 2290 | chr5:151088943-151088970 |
| TNIP1 1126 | chr5:151070778-151070805 | TNIP1 2291 | chr5:151088950-151088977 |
| TNIP1 1127 | chr5:151070779-151070806 | TNIP1 2292 | chr5:151088953-151088980 |
| TNIP1 1128 | chr5:151070780-151070807 | TNIP1 2293 | chr5:151088958-151088985 |
| TNIP1 1129 | chr5:151070783-151070810 | TNIP1 2294 | chr5:151088968-151088995 |
| TNIP1 1130 | chr5:151070788-151070815 | TNIP1 2295 | chr5:151088970-151088997 |
| TNIP1 1131 | chr5:151070792-151070819 | TNIP1 2296 | chr5:151088973-151089000 |
| TNIP1 1132 | chr5:151070793-151070820 | TNIP1 2297 | chr5:151088974-151089001 |
| TNIP1 1133 | chr5:151070802-151070829 | TNIP1 2298 | chr5:151088975-151089002 |
| TNIP1 1134 | chr5:151070825-151070852 | TNIP1 2299 | chr5:151088976-151089003 |
| TNIP1 1135 | chr5:151070828-151070855 | TNIP1 2300 | chr5:151088979-151089006 |
| TNIP1 1136 | chr5:151070859-151070886 | TNIP1 2301 | chr5:151088985-151089012 |
| TNIP1 1137 | chr5:151070863-151070890 | TNIP1 2302 | chr5:151088986-151089013 |
| TNIP1 1138 | chr5:151070868-151070895 | TNIP1 2303 | chr5:151089015-151089042 |
| TNIP1 1139 | chr5:151070878-151070905 | TNIP1 2304 | chr5:151089024-151089051 |
| TNIP1 1140 | chr5:151070881-151070908 | TNIP1 2305 | chr5:151089025-151089052 |
| TNIP1 1141 | chr5:151070882-151070909 | TNIP1 2306 | chr5:151089029-151089056 |
| TNIP1 1142 | chr5:151070883-151070910 | TNIP1 2307 | chr5:151089030-151089057 |
| TNIP1 1143 | chr5:151070884-151070911 | TNIP1 2308 | chr5:151089039-151089066 |
| TNIP1 1144 | chr5:151070898-151070925 | TNIP1 2309 | chr5:151089040-151089067 |
| TNIP1 1145 | chr5:151070901-151070928 | TNIP1 2310 | chr5:151089041-151089068 |
| TNIP1 1146 | chr5:151070902-151070929 | TNIP1 2311 | chr5:151089044-151089071 |
| TNIP1 1147 | chr5:151070909-151070936 | TNIP1 2312 | chr5:151089045-151089072 |
| TNIP1 1148 | chr5:151070910-151070937 | TNIP1 2313 | chr5:151089065-151089092 |
| TNIP1 1149 | chr5:151070919-151070946 | TNIP1 2314 | chr5:151089080-151089107 |
| TNIP1 1150 | chr5:151070924-151070951 | TNIP1 2315 | chr5:151089084-151089111 |
| TNIP1 1151 | chr5:151070933-151070960 | TNIP1 2316 | chr5:151089085-151089112 |
| TNIP1 1152 | chr5:151070962-151070989 | TNIP1 2317 | chr5:151089086-151089113 |
| TNIP1 1153 | chr5:151070965-151070992 | TNIP1 2318 | chr5:151089090-151089117 |
| TNIP1 1154 | chr5:151070968-151070995 | TNIP1 2319 | chr5:151089093-151089120 |
| TNIP1 1155 | chr5:151070981-151071008 | TNIP1 2320 | chr5:151089102-151089129 |
| TNIP1 1156 | chr5:151070982-151071009 | TNIP1 2321 | chr5:151089103-151089130 |
| TNIP1 1157 | chr5:151070983-151071010 | TNIP1 2322 | chr5:151089104-151089131 |
| TNIP1 1158 | chr5:151070984-151071011 | TNIP1 2323 | chr5:151089121-151089148 |
| TNIP1 1159 | chr5:151071001-151071028 | TNIP1 2324 | chr5:151089133-151089160 |
| TNIP1 1160 | chr5:151071002-151071029 | TNIP1 2325 | chr5:151089134-151089161 |
| TNIP1 1161 | chr5:151071003-151071030 | TNIP1 2326 | chr5:151089142-151089169 |
| TNIP1 1162 | chr5:151071006-151071033 | TNIP1 2327 | chr5:151089166-151089193 |
| TNIP1 1163 | chr5:151071007-151071034 | TNIP1 2328 | chr5:151089173-151089200 |
| TNIP1 1164 | chr5:151071008-151071035 | TNIP1 2329 | chr5:151089179-151089206 |
| TNIP1 1165 | chr5:151071009-151071036 | | |

## Claims

1. A method for culturing cells, the method comprising: reducing the expression and/or attenuating the activity of GTPase activating protein 1 family members and/or functionally active fragments thereof in the cells.

2. The method of claim 1, wherein the cells comprise immune cells.

3. The method of claim 2, wherein the immune cells comprise phagocytes, lymphocytes, neutrophils, eosinophils and/or basophils.

4. The method according to any one of claims 2-3, wherein the immune cells comprise monocytes, macrophages and/or dendritic cells.

5. The method according to any one of claims 2-4, wherein the immune cells are derived from immune cells differentiated from stem cells.

6. The method of claim 5, wherein the stem cells comprise induced pluripotent stem cells (iPSCs).

7. The method according to any one of claims 2-6, wherein the immune cells comprise B cells, T cells, natural killer cells and/or natural killer-like T cells (NKT).

8. The method according to any one of claims 2-7, wherein the immune cells comprise αβ T cells and/or γδ T cells.

9. The method of any one of claims 2-8, wherein the immune cells comprise tumor infiltrating lymphocytes (TILs).

10. The method according to claim 9, wherein the TILs are TILs derived from tumor tissue fragments, pleural effusion and/or peritoneal effusion and/or TILs derived from recovery after cryopreservation.

11. The method of claim 10, wherein the volume of the fragments is from about 1 cubic millimeter to about 27 cubic millimeters.

12. The method of any one of claims 2-11, wherein the immune cell comprises an engineered immune receptor displayed on a cell surface.

13. The method of claim 12, wherein the engineered immune receptor specifically binds to an antigen expressed on a target cell.

14. The method of any one of claims 2-13, wherein the immune cell comprises a chimeric antigen receptor and/or a T cell receptor.

15. The method according to any one of claims 1-14, wherein said reducing the expression and/or attenuating the activity of GTPase activating protein 1 family members in said cell comprises inhibiting the function of the GTPase.

16. The method according to any one of claims 1-15, wherein the cells obtained by reducing the expression and/or attenuating the activity of GTPase activating protein 1 family members show improved cellular properties compared to cells in which the expression and/or activity of the GTPase activating protein 1 family member is not altered.

17. The method according to claim 16, wherein the improved cellular properties comprise one or more selected from the following group: improved cell proliferation ability, increased proportion of live cells, improved proportion of cell subpopulations, increased cytokine secretion ability and increased tumor cell killing ability.

18. The method according to claim 17, wherein the improved cell subpopulation ratio comprises one or more selected from the following group: an increased ratio of activated cells, a decreased ratio of regulatory cells, a decreased ratio of exhausted cells, an increased ratio of central memory cells and/or naive cells, a decreased ratio of apoptotic cells, and an increased ratio of stem-like cells.

19. The method of any one of claims 1-18, wherein the GTPase activating protein 1 family member comprises a GTPase activating domain.

20. The method of any one of claims 1-19, wherein the GTPase activating protein 1 family member comprises RASA2.

21. The method according to any one of claims 1-20, wherein said reducing the expression and/or attenuating the activity of GTPase activating protein 1 family members in said cell comprises introducing a gene regulatory system into said cell.

22. The method of claim 21, wherein the gene regulatory system is capable of disrupting the GTPase activating protein 1 family member at the DNA level.

23. The method of any one of claims 21-22, wherein the gene regulatory system comprises a guide nucleic acid molecule and an enzyme protein.

24. The method of claim 23, wherein said reducing the expression and/or attenuating the activity of GTPase activating protein 1 family members comprises: introducing a complex comprising the guide nucleic acid molecule and the enzyme protein, or a complex comprising the guide nucleic acid molecule and a nucleic acid encoding the enzyme protein into the cell.

25. The method of any one of claims 23-24, wherein the enzyme protein comprises a Cas protein, a Cas protein homolog, or a functionally active fragment thereof.

26. The method of any one of claims 23-25, wherein the guide nucleic acid molecule comprises a guide RNA (gRNA).

27. The method of any one of claims 23-26, wherein the guide nucleic acid molecule is capable of binding to the sequence of the GTPase activating protein 1 family member.

28. The method of any one of claims 23-27, wherein the guide nucleic acid molecule is capable of binding to a region defined by the genomic coordinates selected from those shown in Table 1A or Table 2A, or a fragment thereof.

29. The method of any one of claims 23-28, wherein the guide nucleic acid molecule is capable of binding to a region or a fragment thereof selected from the following group: SEQ ID NOs: 1093-2184.

30. The method of any one of claims 23-29, wherein the guide nucleic acid molecule is capable of binding to a sequence of about 15 to about 25 nucleotides upstream of the 5' end of the protospacer adjacent motif (PAM) selected from the following group: AGG, TGG, CGG, and GGG.

31. The method of any one of claims 23-30, wherein the guide nucleic acid molecule comprises a targeting domain comprising a sequence as shown in any one of SEQ ID NOs: 1-1092, 29247-29248, 29292-29314.

32. The method according to any one of claims 1 to 31, wherein in the cells obtained by reducing the expression and/or attenuating the activity of GTPase activating protein 1 family members, the proportion of cells expressing the product of the target gene is reduced and/or the expression level of the target gene in individual cells is decreased, compared to cells in which the expression and/or activity of the GTPase activating protein 1 family member is not changed.

33. The method according to any one of claims 1-32, wherein among the cells obtained by reducing the expression and/or attenuating the activity of GTPase activating protein 1 family members, the proportion of cells expressing the target gene is about 95% or less.

34. A cell obtained by the method of any one of claims 1-33.

35. A pharmaceutical composition comprising the cell of claim 34, and optionally a pharmaceutically acceptable carrier.

36. A method of influencing cell growth, comprising administering the cell of claim 34 and/or the pharmaceutical composition of claim 35.

37. Use of the cell according to claim 34 and/or the pharmaceutical composition according to claim 35 in the preparation of a medicament for preventing and/or treating a disease and/or symptom.

38. A medicament for preventing and/or treating a disease and/or symptom, comprising the cell according to claim 34 and/or the pharmaceutical composition according to claim 35 as an active ingredient.

39. A method for preventing and/or treating a disease and/or a symptom, comprising administering the cell of claim 34 and/or the pharmaceutical composition of claim 35 to a subject in need.

40. The cell of claim 34 and/or the pharmaceutical composition of claim 35, for use in preventing and/or treating a disease and/or symptom.

41. The use according to claim 37, the medicament according to claim 38, the method according to claim 39, and/or the cell and/or the pharmaceutical composition for use according to claim 40, wherein the disease and/or symptom comprises a tumor.

42. The use according to claim 37, the medicament according to claim 38, the method according to claim 39, and/or the cell and/or the pharmaceutical composition for use according to claim 40, wherein the disease and/or symptom comprises a solid tumor.

43. The use according to claim 37, the medicament according to claim 38, the method according to claim 39, and/or the cell and/or the pharmaceutical composition for use according to claim 40, wherein the disease and/or symptom comprises one or more selected from the following group: melanoma, ovarian cancer, cervical cancer, lung cancer, bladder cancer, breast cancer, head and neck cancer, pancreatic cancer, liver cancer, gastric cancer, colorectal cancer and kidney cancer.
